# EUROPEAN PATENT SPECIFICATION

(11) **EP 4 048 796 B1**
(45) Date of publication and mention of the grant of the patent: **27.08.2025**
(21) Application number: 20891848.2
(22) Date of filing: 23.10.2020
(51) Int. Cl.: C12N 15/63, C12N 15/79, C12N 9/22, C40B 40/02, C12N 15/10

(54) **COMPOSITIONS AND METHODS COMPRISING VIRAL VECTOR SYSTEMS FOR MULTIPLEXED ACTIVATION OF ENDOGENOUS GENES AS IMMUNOTHERAPY AND VIRAL-BASED IMMUNE-GENE THERAPY**
ZUSAMMENSETZUNGEN UND VERFAHREN MIT VIRALEN VEKTORSYSTEMEN ZUR MULTIPLEXIERTEN AKTIVIERUNG ENDOGENER GENE ALS IMMUNTHERAPIE UND IMMUN-GENTHERAPIE AUF VIRALER BASIS
COMPOSITIONS ET PROCÉDÉS COMPRENANT DES SYSTÈMES DE VECTEURS VIRAUX POUR L'ACTIVATION MULTIPLEXÉE DE GÈNES ENDOGÈNES EN TANT QU'IMMUNOTHÉRAPIE ET THÉRAPIE GÉNIQUE IMMUNITAIRE À BASE VIRALE

(30) Priority: 23.10.2019 US 201962924973 P
(43) Date of publication of application: 31.08.2022
(73) Proprietor: Yale University, New Haven, CT 06510 (US)
(72) Inventor: CHEN, Sidi, Milford, Connecticut 06461 (US); WANG, Guangchuan, West Haven, Connecticut 06511 (US); CHOW, Ryan D., San Jose, California 95120 (US); ZHANG, Feifei, New Haven, Connecticut 06511 (US)
(74) Representative: Grünecker Patent- und Rechtsanwälte PartG mbB
(86) International application number: PCT/US2020/057113
(87) International publication number: WO 2021/108050

(56) References cited:
- WO-A1-2019/204503
- US-A1- 2002 098 542
- US-A1- 2003 223 971
- US-A1- 2019 194 653
- S. T. HAILE ET AL: "Soluble CD80 Restores T Cell Activation and Overcomes Tumor Cell Programmed Death Ligand 1-Mediated Immune Suppression", THE JOURNAL OF IMMUNOLOGY, vol. 191, no. 5, 5 August 2013 (2013-08-05), US, pages 2829 - 2836, XP055334763, ISSN: 0022-1767, DOI: 10.4049/jimmunol.1202777
- WANG GUANGCHUAN ET AL: "Multiplexed activation of endogenous genes by CRISPRa elicits potent antitumor immunity", NATURE IMMULOGY, NATURE PUBLISHING GROUP US, NEW YORK, vol. 20, no. 11, 14 October 2019 (2019-10-14), pages 1494 - 1505, XP036912475, ISSN: 1529-2908, [retrieved on 20191014], DOI: 10.1038/S41590-019-0500-4
- WANGENSTEEN KIRK J. ET AL: "Combinatorial genetics in liver repopulation and carcinogenesis with a in vivo CRISPR activation platform", HEPATOLOGY, vol. 68, no. 2, 1 August 2018 (2018-08-01), US, pages 663 - 676, XP055876179, ISSN: 0270-9139, DOI: 10.1002/hep.29626
- CODINA ET AL.: "Convergent Identification and Interrogation of Tumor-Intrinsic Factors that Modulate Cancer Immunity In Vivo", CELL SYST., vol. 8, no. 2, 27 February 2019 (2019-02-27), pages 136 - 151, XP055857053

## Description

### BACKGROUND OF THE INVENTION

Immunotherapy has transformed cancer treatment by leveraging the patient's own immune system against the tumor, thereby turning several previously lethal cancers into manageable diseases for a subset of patients. Major types of immunotherapy include checkpoint blockade, adoptive cell transfer, human recombinant cytokines, and cancer vaccines. Although antigen recognition is a key process in the anti-tumor immune response, there has been limited success using cancer vaccines in the clinic over the past several decades. Traditional cancer vaccines are often dendritic cell-based vaccines such as sipuleucel-T. Recent advances of peptide- and RNA-based vaccines showed that targeted delivery of multiple mutated neoantigens can generate a strong anti-tumor effect, providing direct clinical evidence of effective cancer vaccines against late-stage melanoma. Tumor cells harbor a multitude of mutations that frequently encode mutated, partially truncated, or amplified genes that are immunogenic. However, many of these mutations might not be expressed at levels sufficient to elicit an effective T-cell-mediated response. Synthesis of these peptides or transcripts is possible with parallel protein- or RNA-synthesis, but the cost of this approach is proportional to the number of mutations identified.

Guangchuan Wang et al. (Nature Immunology, vol. 20, no. 11, Oct. 2019, pages 1494-1505) report that multiplexed activation of endogenous genes by CRISPRa elicits potent antitumor immunity. K.J. Wangensteen et al. (Hepatology, vol. 68, no. 2, 1 August 2018, pages 663-676) report on combinatorial genetics in liver repopulation and carcinogenesis with a novel *in vivo* CRISPR activation platform.

A need exists for new types of cancer vaccines that can elicit strong, durable, and specific immune responses against cancer antigens, while maintaining efficacy, versatility, and cost-effectiveness. The present invention addresses this need.

### SUMMARY OF THE INVENTION

As described herein, the present invention relates to methods comprising viral vector systems for multiplexed activation of endogenous genes as immunotherapy and viral-based immune-gene therapy as further described in the appended claims.

In one aspect, the invention comprises a method of developing a cancer immunotherapy as described in the appended claims. The method comprises a) administering a CRISPR activation (CRISPRa) system comprising an sgRNA library to a cancer cell, thereby generating a modified cell, b) administering the modified cell to an immunocompetent non-human mammal whereby the mammal develops cancer, c) determining the sgRNAs and thereby the targeted genes that are depleted in the cancer, and d) designing a cancer immunotherapy that targets the depleted genes.

In certain embodiments, the sgRNA library comprises the nucleotide sequences set forth in SEQ ID NOs. 86-192 or SEQ ID NOs. 193-411.

In certain embodiments, the mammal is a mouse.

In certain embodiments, the CRISPRa system comprises a vector comprising the nucleotide sequence set forth in SEQ ID NO: 1.

In certain embodiments, determining the genes that are depleted in the cancer comprises nucleotide sequencing and analysis.

In certain embodiments, designing a cancer therapy that targets the depleted genes comprises packaging the open reading frames (ORFs) of the depleted genes in a vector.

In certain embodiments, the vector is an adeno-associated viral (AAV) vector or an adenoviral vector.

In another unclaimed aspect, the present disclosure includes a treatment of cancer in a subject in need thereof. This comprises administering a therapeutically effective amount of the cancer immunotherapy generated by any of the methods contemplated herein.

In another unclaimed aspect, the present disclosure includes a composition comprising a vector comprising an open reading frame (ORF) of at least one gene selected from the group consisting of CD80, Light, CXCL10, 4-1BBL, GITRL, IL2, IL-23, and IFNg.

In certain unclaimed aspects, the vector comprises the nucleotide sequence set forth in any one of SEQ ID NOs: 2, 3, and 24-58.

In another unclaimed aspect, the present disclosure includes a vector comprising a nucleic acid sequence that is 85%, 90%, 95%, 96%, 97%, 98%, 99%, or 100% identical to any one of SEQ ID NOs: 2-18 or 24-58.

In another unclaimed aspect, the present disclosure includes a vector comprising at least one nucleic acid sequence that is 85%, 90%, 95%, 96%, 97%, 98%, 99%, or 100% identical to any one of SEQ ID NOs: 73-84.

In another unclaimed aspect, the present disclosure includes a composition comprising any of the vectors contemplated herein.

In another unclaimed aspect, the present disclosure includes a treatment of cancer in a subject in need therof. This comprises administering to the subject a therapeutically effective amount of any of the compositions contemplated herein.

In certain unclaimed aspects, the cancer is selected from the group consisting of triple-negative breast cancer, melanoma, pancreatic cancer, or a solid tumor.

In certain unclaimed apects, the treatment further comprises administering an additional treatment to the subject.

### BRIEF DESCRIPTION OF THE DRAWINGS

The following detailed description of specific embodiments of the invention will be better understood when read in conjunction with the appended drawings. For the purpose of illustrating the invention, there are shown in the drawings exemplary embodiments. It should be understood, however, that the invention is not limited to the precise arrangements and instrumentalities of the embodiments shown in the drawings.

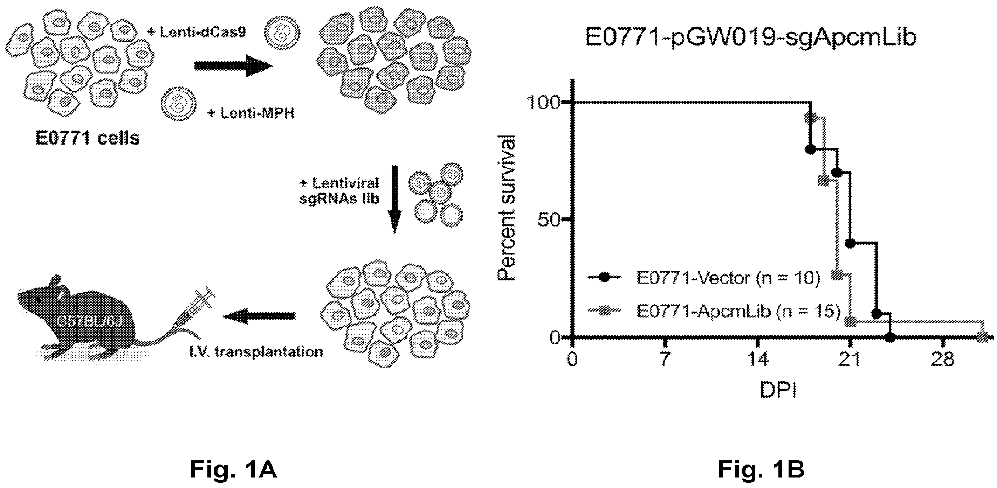

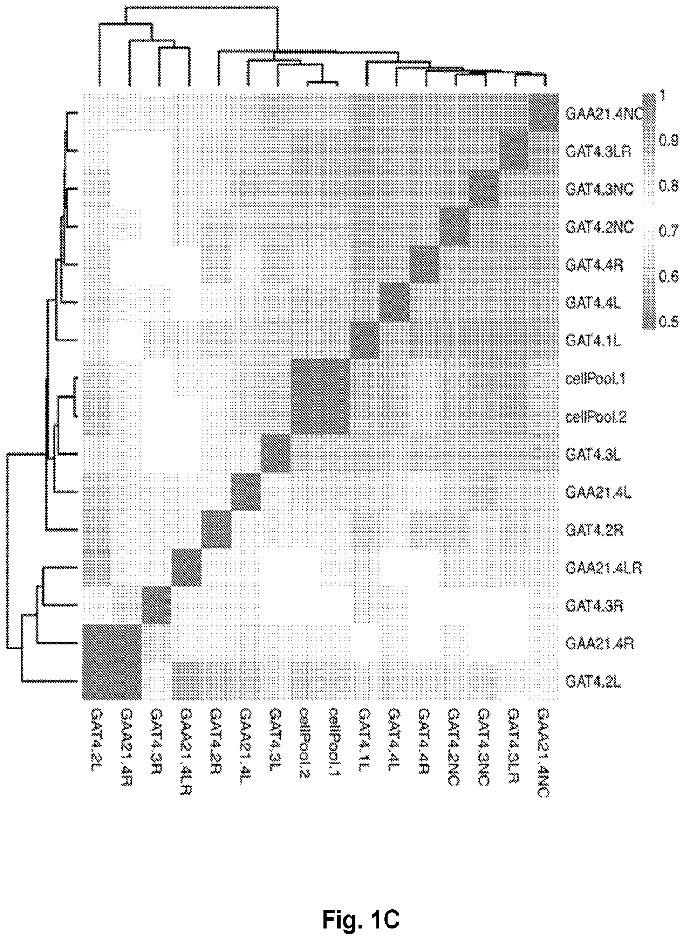

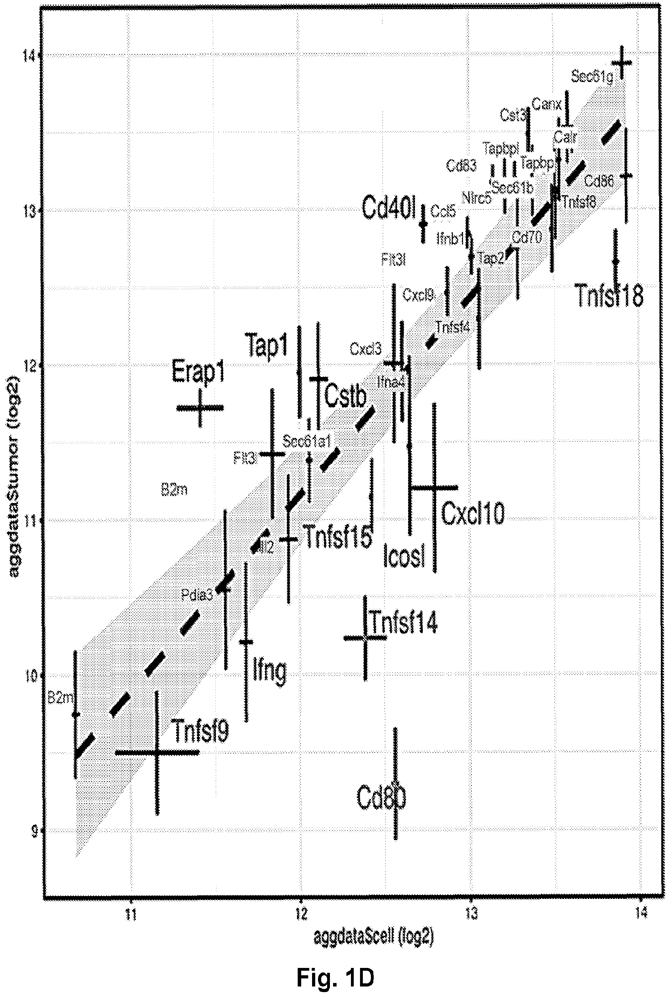

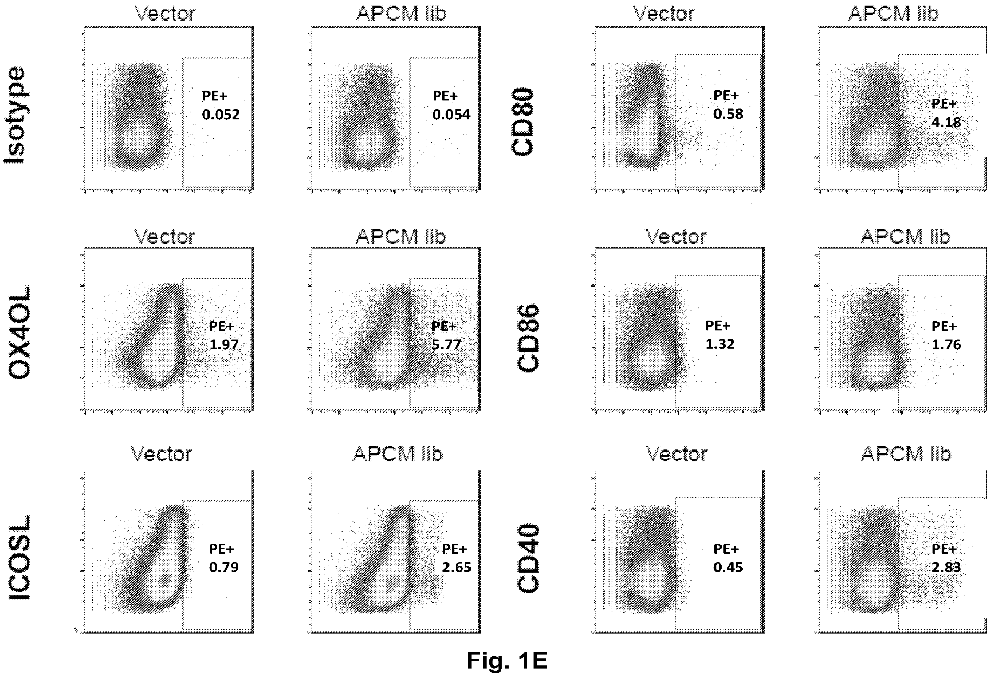

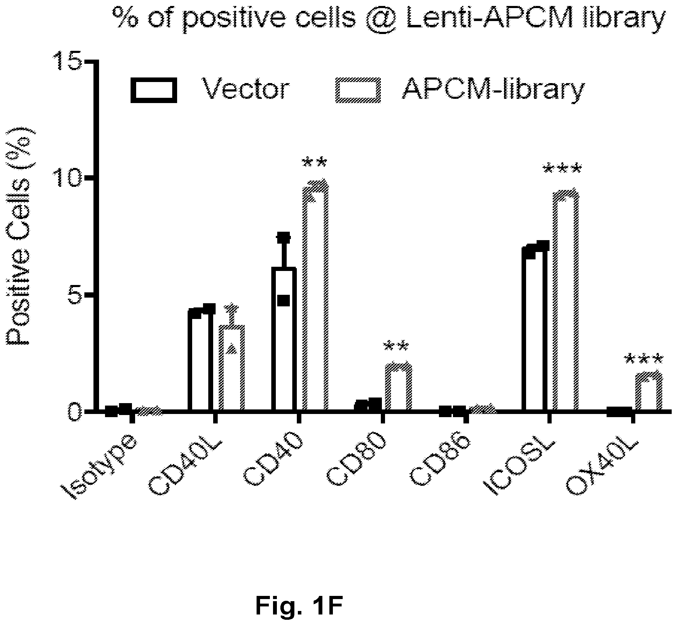

Figs. 1A-1D illustrate an *in vivo* CRISPRa screen to pinpoint the genetic factors that can enhance host immune surveillance and tumor destruction. Fig. 1A is a schematic of the experiment design. The APCM sgRNA library was introduced into E0771, a triple-negative breast cancer cell line, by infection with lentiviruses at M.O.I = 0.2-0.3. After 7 days of *in vitro* culture under puromycin selection, 2*10⁶ of APCM library-transduced cells were intravenously injected into immunocompetent C57BL/6J mice. The resulting metastatic tumors in the lungs were collected, and the immune-mediated sgRNA depletion and enrichment was readout by illumina sequencing. Fig. 1B shows Kaplan-Meier survival curves of mice transplanted with E0771-Vector (n = 10) or E0771-APCM library (n = 15) formed metastatic tumors. Log-rank test, E0771-APCM vs. E0771-Vector, p = 0.2000. Fig. 1C depicts the correlations within tumor samples and cell samples. Fig. 1D is a scatter plot of genes whose activation may lead to elevated immune destruction. Average sgRNA representation across metastatic tumors in C57BL/6J mice (n = 14) was plotted against average sgRNA library representation in cultured cell pool (n = 2). Screen was performed in immunocompetent C57BL/6J mice by intravenously injecting library-transduced E0771 to form metastatic tumors. The linearized sgRNA population regression was used to call depletion under immune pressure. Error bars: Data points in this figure are presented as mean ± s.e.m. Asterisks: * p < 0.05, ** p < 0.01, *** p < 0.001. Fig. 1E depicts representative flow cytometry plots demonstrating CRISPRa-mediated expression of several representative costimulatory molecules, such as OX40L, ICOSL, CD80, CD86, and CD40 s in APCM sgRNA library transduced E0771 cells. Fig. 1F shows the percentage of CD40, CD40L, CD80, CD86, OX40L, and ICOSL expressing cells in APCM sgRNA library transduced E0771 cells.

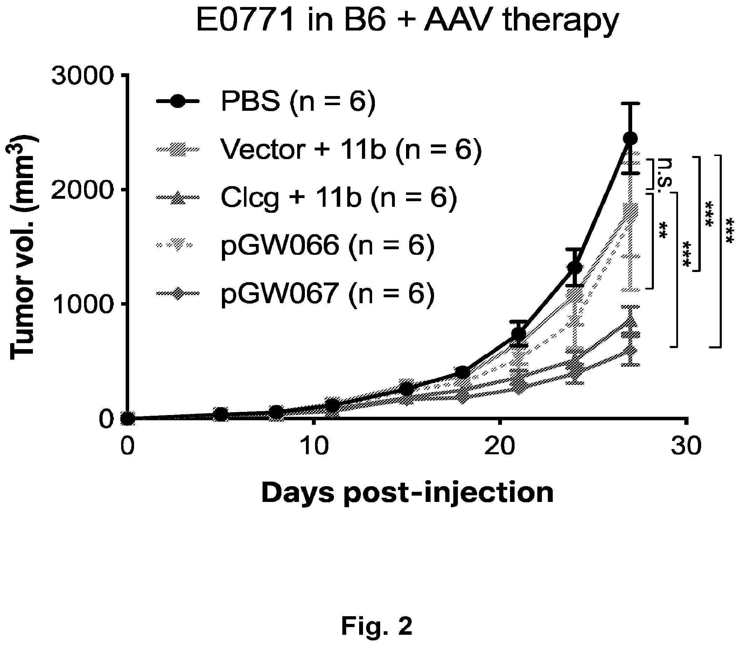

Fig. 2 illustrates *in vivo* efficacy of AAV-o-MAEGI-CLC4G, AAV-ORF-CLC4 and AAV-ORF-CLC4I in a syngeneic triple-negative breast cancer model. Therapeutic effects of intratumoral activation of CLC4I (Cd80, light/Tnfsf14, Cxcl10, 4-1BBL/Tnfsf9, Ifng or Gitrl) are shown. Tumor growth curves of E0771 tumors treated with PBS (n = 6), AAV-dCas9 + Vector (n = 6), AAV-dCas9 + CLC4G (dual AAV delivered CRISPRa of sgRNA library targeting CLC4G, i.e. o-MAEGI-CLC4G, n = 6), AAV-orf-CLC4 (pGW066: AAV delivered open reading frame expression of CLC4, n = 6), or AAV-orf-CLC4I (pGW067: AAV delivered open reading frame expression of Clcg and Ifng, n = 6). Two-way ANOVA: AAV-dCas9 + Vector vs. PBS, *p* = 0.9675; AAV-dCas9 + CLC4 vs. PBS, *p* < 0.0001; AAV-orf-CLC4 vs. PBS, *p =* 0.04; AAV-orf-CLC4I vs. PBS, p < 0.0001; AAV-dCas9 + CLC4G vs. AAV-dCas9 + Vector, *p* < 0.0001. Error bars: Data points in this figure are presented as mean ± s.e.m. Asterisks: * p < 0.05, ** p < 0.01, *** p < 0.001.

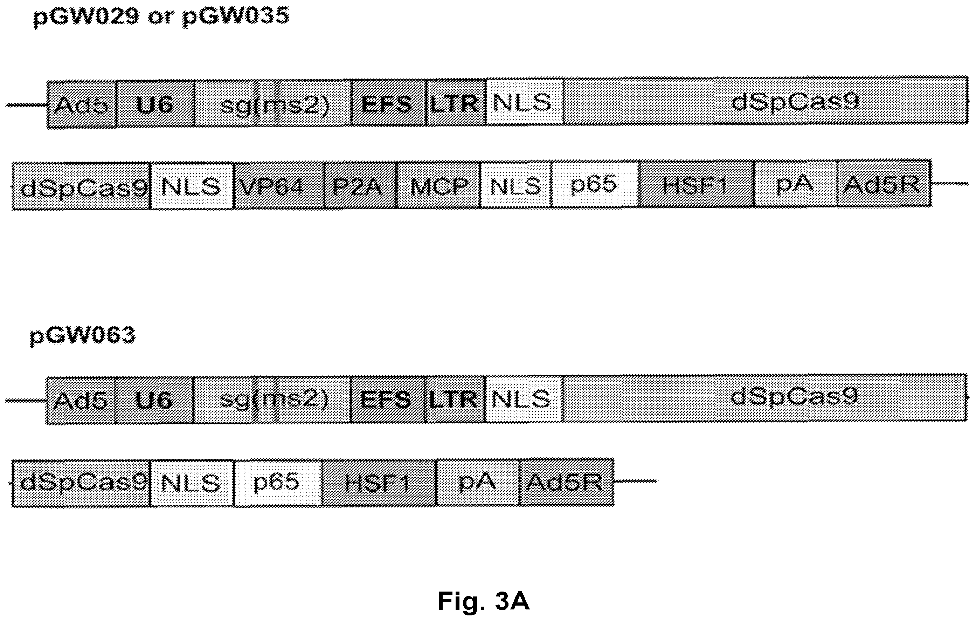

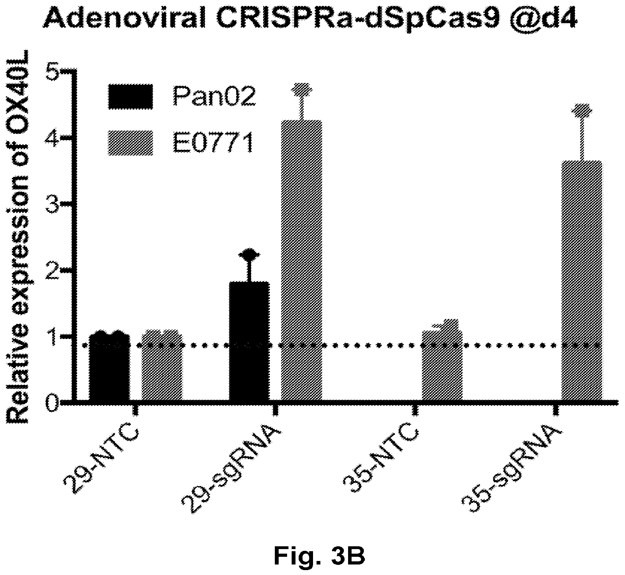

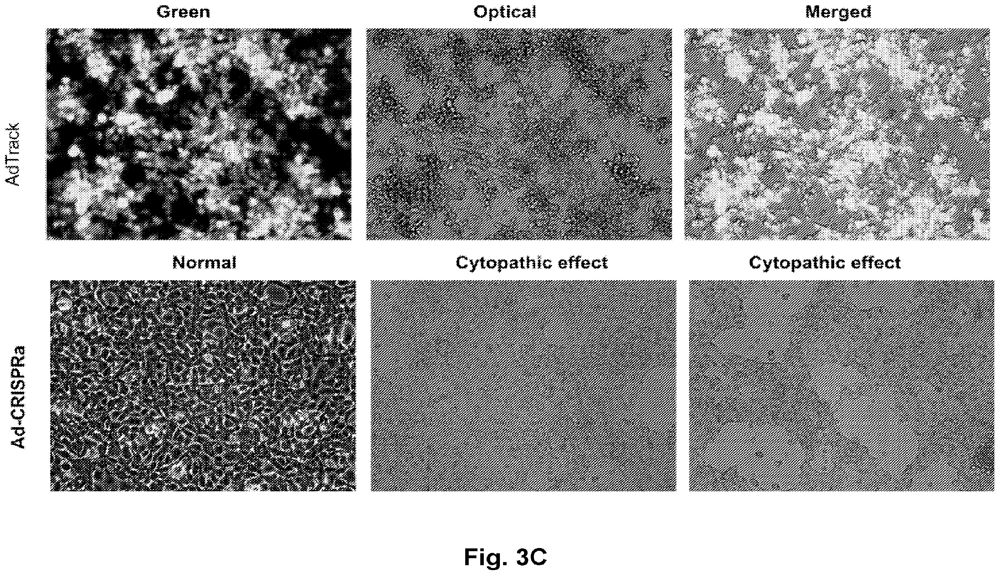

Figs. 3A-3C illustrate the design and characterization of an adenoviral CRISPRa system. Fig. 3A is a schematic of the designs of adenoviral dSpCas9-based CRISPRa systems, that use a single construct to deliver all CRISPRa components. Depicted are pGW029 (SEQ ID NO: 4), pGW035 (SEQ ID NO: 5), and pGW063 (SEQ ID NO: 6). Fig. 3B shows results from an experiment wherein Ox40l-targeted sgRNA was cloned into a construct from Fig. 3A and the activation efficency tested *in vitro.* 4 days post-transfection, expression levels of Ox40l were determined by quantitative RT-PCR. Fig. 3C shows the cytopathic effects in HEK293FT cells caused by the adenoviral CRISPRa system. Upper panel: AdTrack (adenoviral CMV-EGFP) were rescued, and the expression of GFP and cytopathic effects were observed, demonstrating the robustness of the adenoviral engineering and rescue system. Lower panel: the CRISPRa system was cloned into adenoviral constructs (U6-sgRNA-EFS-LTR-dSpCas9-p65-HSF1-sPA) and the adenoviruses were rescued. 4-6 days post-infection of the adenovirus, cytopathic effects on HEK293FT were observed.

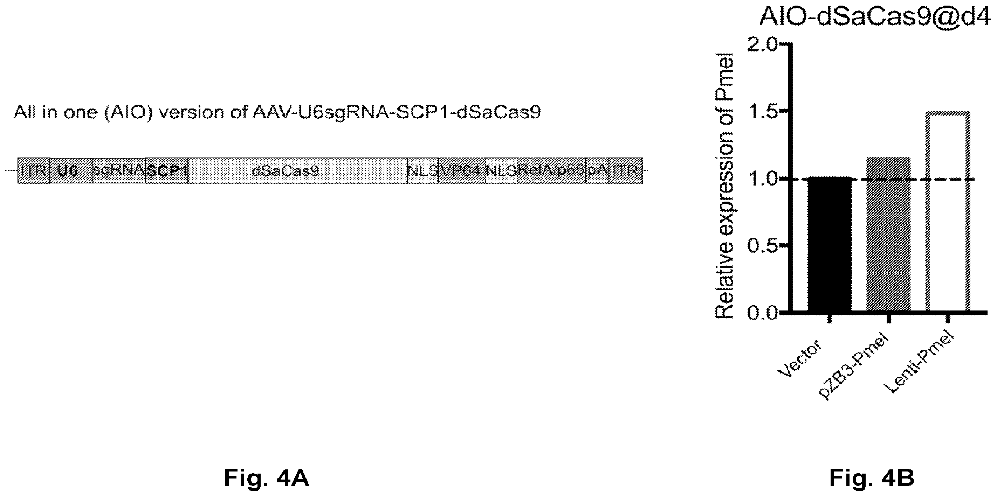

Figs. 4A-4B illustrate an AAV based one-vector system with dSaCas9. Fig. 4A is a diagram illustrating a dSaCas9-based AIO (all-in-one) CRISPRa system, using a single AAV vector to deliver all CRISPRa components. Fig. 4B shows *in vitro* activation of the Pmel gene with the AIO CRISPRa system. Pmel was targeted with the AIO CRISPRa system and 4 days post-infection expression levels of Pmel were determined by quantitative RT-PCR.

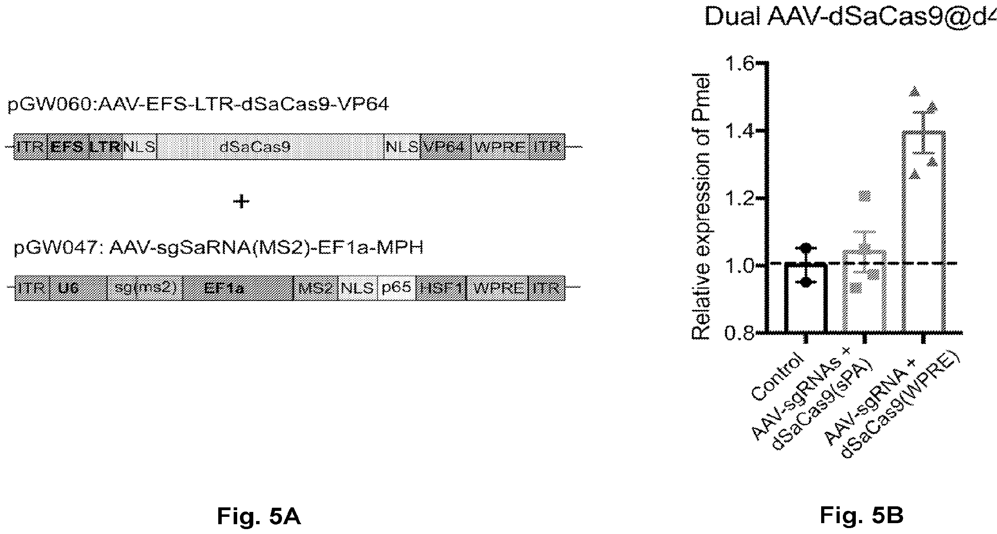

Figs. 5A-5B illustrate an AAV based two-vector system with dSaCas9. Fig 5A is a schematic of the design of a dSaCas9-based dual-AAV delivery CRISPRa system. Fig. 5B shows results from *in vitro* testing of the dSaCas9-based dual-AAV CRISPRa system. Pmel targeted sgRNA was cloned into with the dual AAV-CRISPRa system. Four days post-infection, expression levels of Pmel were determined by quantitative RT-PCR.

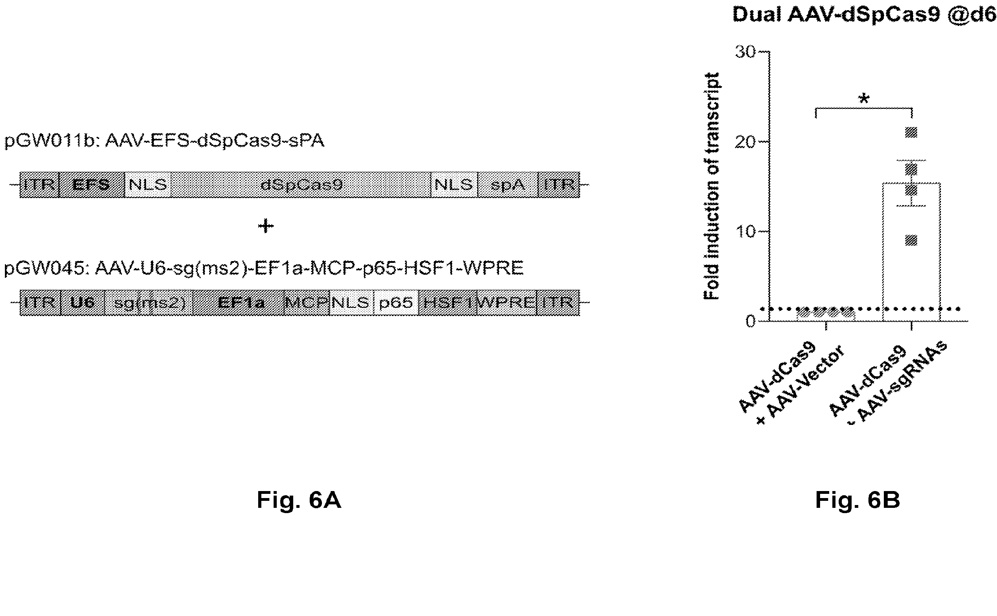

Figs. 6A-6B illustrate an AAV based two-vector system with dSpCas9. Fig. 6A is a schematic of the design of a dSpCas9-based dual-AAV delivery of CRISPRa system. Fig. 6B shows results of in vitro testing. Pmel targeted sgRNA was cloned into with the dual AAV-CRISPRa system. 6 days post-infection, expression levels of Pmel were determined by quantitative RT-PCR.

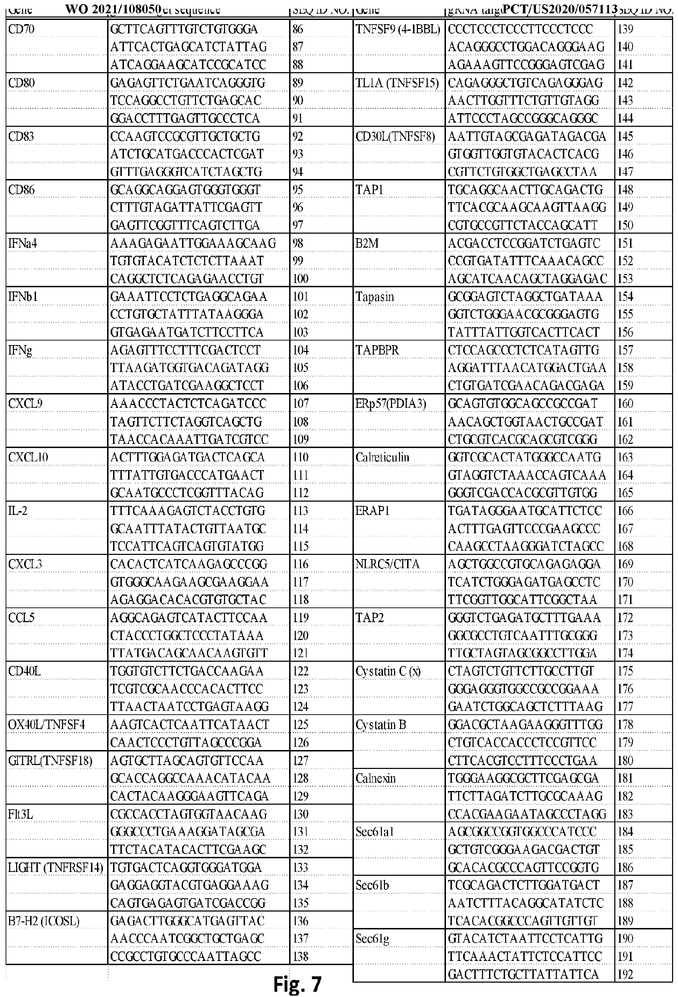

Fig. 7 is a table illustrating sgRNA sequences for an AAV-APCM library (SEQ ID NOs: 86-192).

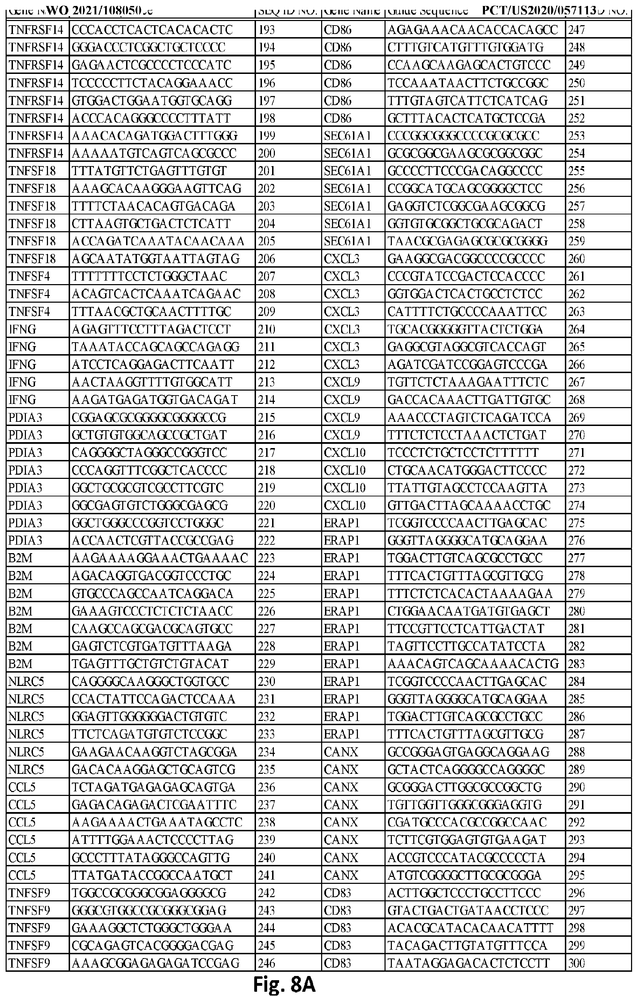

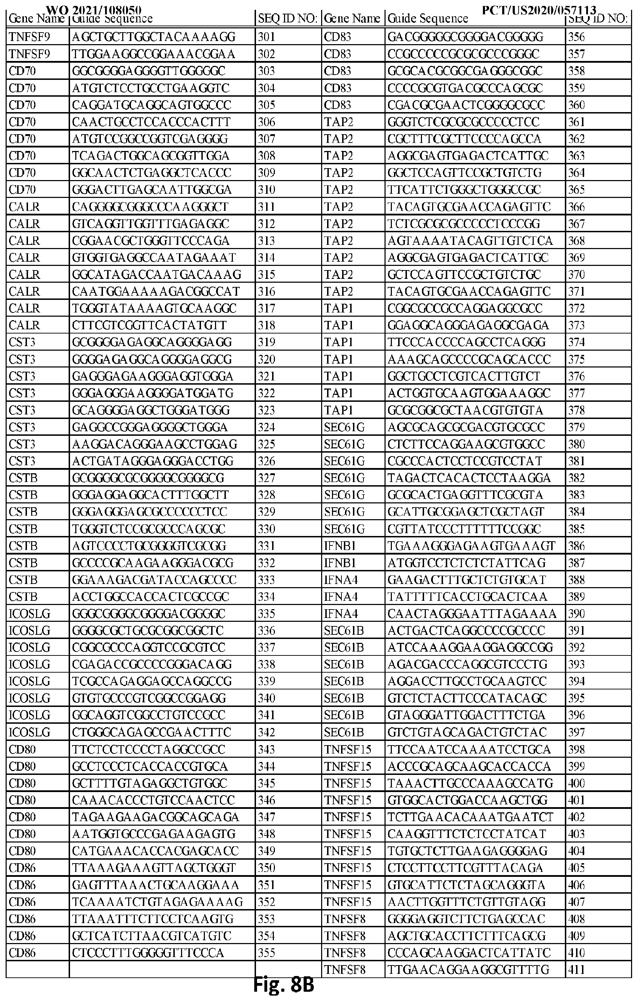

Figs. 8A-8B illustrate sgRNA sequences for an AAV-APCM library designed/optimized specifically for human genes (SEQ ID NOs: 193-411).

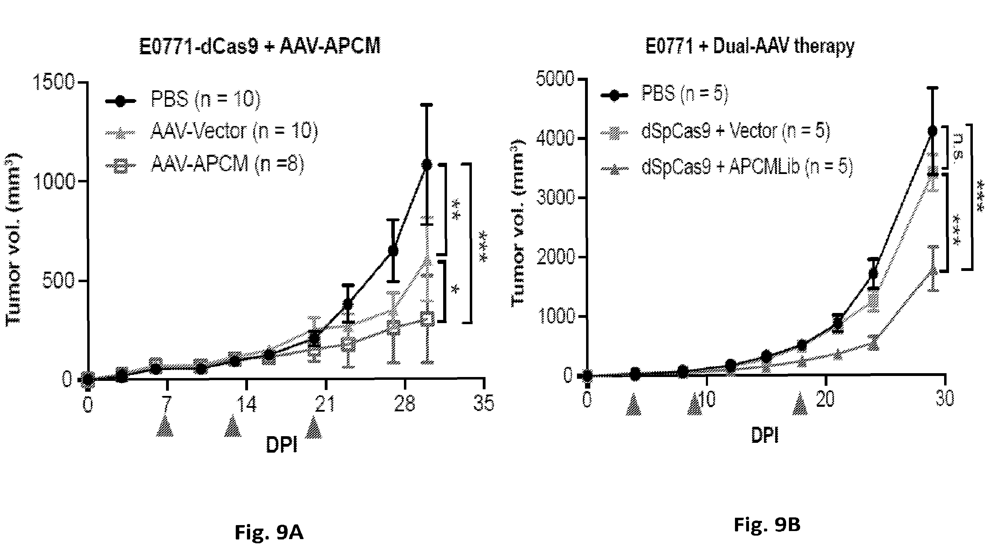

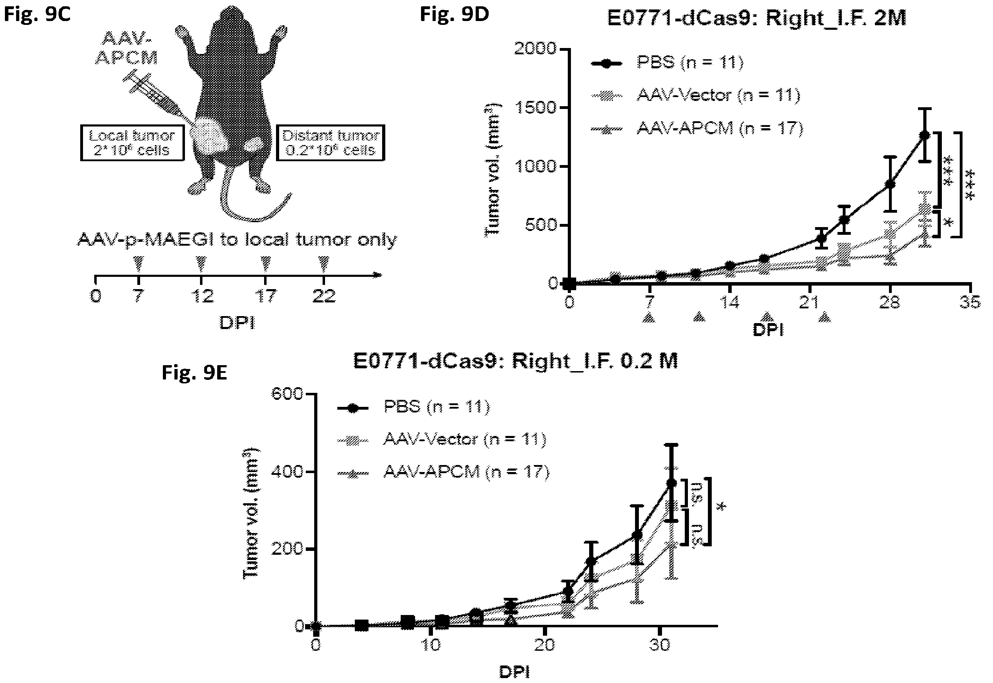

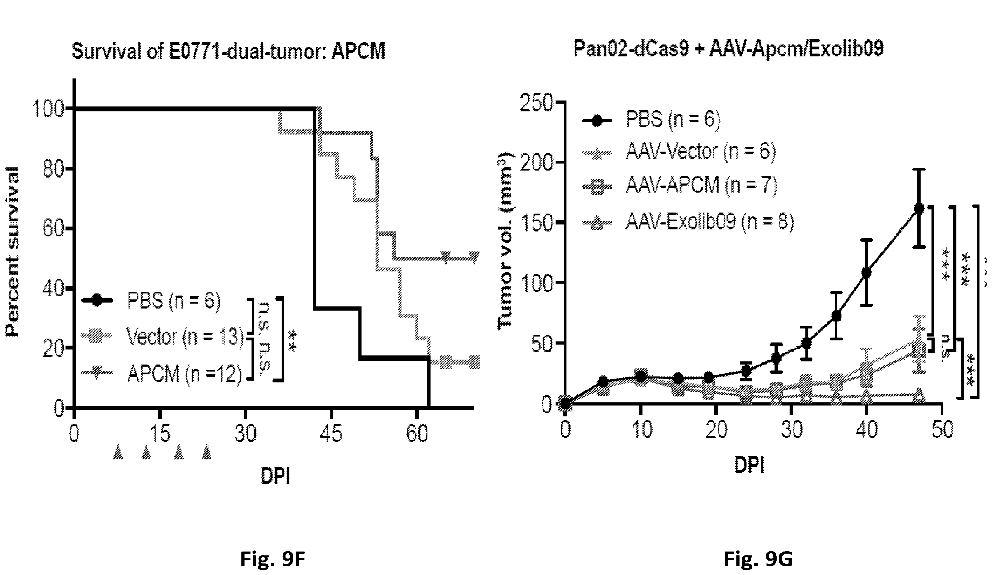

Figs. 9A-9G illustrate multiplexed activation of endogenous genes as immunotherapy, by activating immune genes (APCM) or mutant genes (p-MAEGI). Fig. 9A shows tumor growth curves of orthotopic breast tumor formed by E0771-dCas9-VP64 in mice treated with PBS (n = 10 mice), AAV-Vector (n = 10), or AAV-APCM (n = 8) by intratumoral administration at indicated times (arrows). Two-way ANOVA: AAV-Vector vs. PBS, *p* = 0.0054; AAV- APCM vs. PBS, *p <* 0.0001; AAV- APCM vs. AAV-Vector, *p* = 0.0139. Fig. 9B illustrates the therapeutic effects of dual-AAV delivered APCM CRISPRa into tumor-bearing mice. The growth curves of E0771 syngeneic tumors in mice treated by intratumoral injection of PBS (n = 5 mice), AAV-dCas9 + Vector (n = 5), or AAV-dCas9 + APCM (n = 5) at the indicated time points (arrows). Two-way ANOVA: AAV-dCas9 + Vector vs. PBS, p = 0.1586; AAV-dCas9 + APCM vs. PBS, p < 0.0001; AAV-dCas9 + APCM vs. AAV-dCas9 + Vector, p < 0.0001. Fig. 9C shows an experimental design for evaluating the systemic anti-tumor effects of AAV-APCM. 2*10⁶ or 0.2*10⁶ E0771-dCas9-VP64 tumor cells were transplanted into the left or right flank of C57BL/6J mice respectively to model local and distant tumors. AAV-p-MAEGI was administered only into the local tumors at the indicated times (arrows). Figs. 9D-9E are growth curves of E0771-dCas9-VP64 local (Fig. 9D) and distant (Fig. 9E) tumors in mice treated with PBS (n = 11), AAV-Vector (n = 11), or AAV-APCM (n = 17). Fig. 9D: Local tumors, two-way ANOVA: AAV-Vector vs. PBS, *p* = 0.0001; AAV- APCM vs. PBS, *p* < 0.0001; AAV- APCM vs. AAV-Vector, *p =* 0.0130. Fig. 9E: Distant tumor: two-way ANOVA: AAV-Vector vs. PBS, *p* = 0.2308; AAV- APCM vs. PBS, *p* = 0.011; AAV- APCM vs. AAV-Vector, *p* = 0.1407. Fig. 9F shows survival curves of the E0771 breast tumor bearing mice treated with PBS (n = 6), AAV-Vector (n = 13), or AAV-APCM (n = 12). Log-rank test, AAV-Vector vs. PBS, *p* = 0.1115; AAV- APCM vs. PBS, *p* = 0.0022; AAV- APCM vs. AAV-Vector, *p* = 0.1178. Fig. 9G show growth curves of Pan02-dCas9-VP64 tumors in C57BL/6J mice treated with PBS (n = 6 mice), AAV-Vector (n = 6), AAV-APCM (n =7), or AAV-p-MAEGI (n = 8) at indicated times (blue arrows). Two-way ANOVA: AAV-Vector vs. PBS, *p* < 0.0001; AAV- APCM vs. PBS, *p* < 0.0001; AAV-p-MAEGI vs. *PBS, p* < 0.0001; AAV- APCM vs. AAV-Vector, *p =* 0.4432; AAV-p-MAEGI vs. AAV-Vector, *p* < 0.0001. Error bars: Data points in this figure are presented as mean ± s.e.m. Asterisks: * p < 0.05, ** p < 0.01, *** p < 0.001.

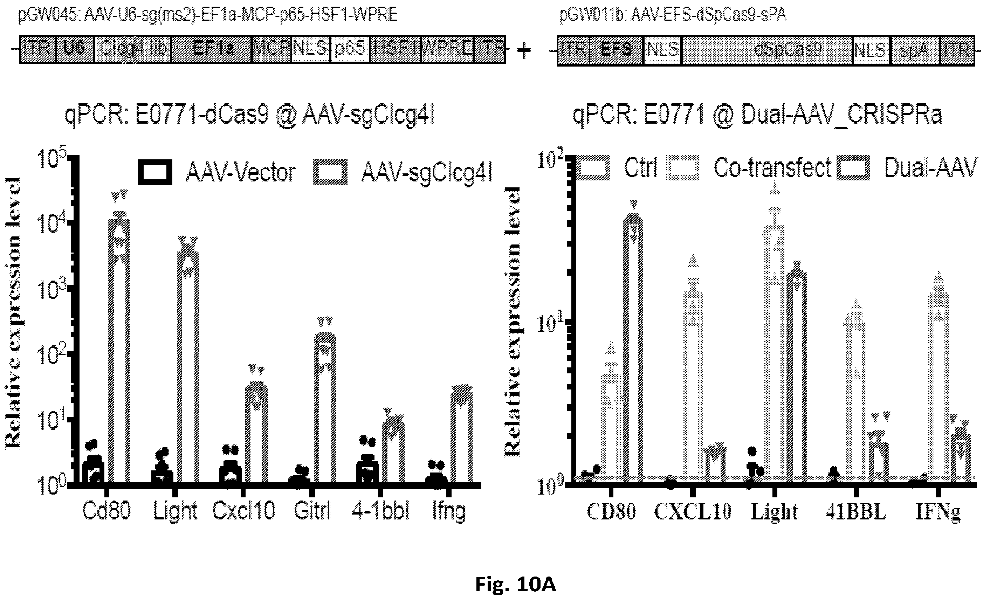

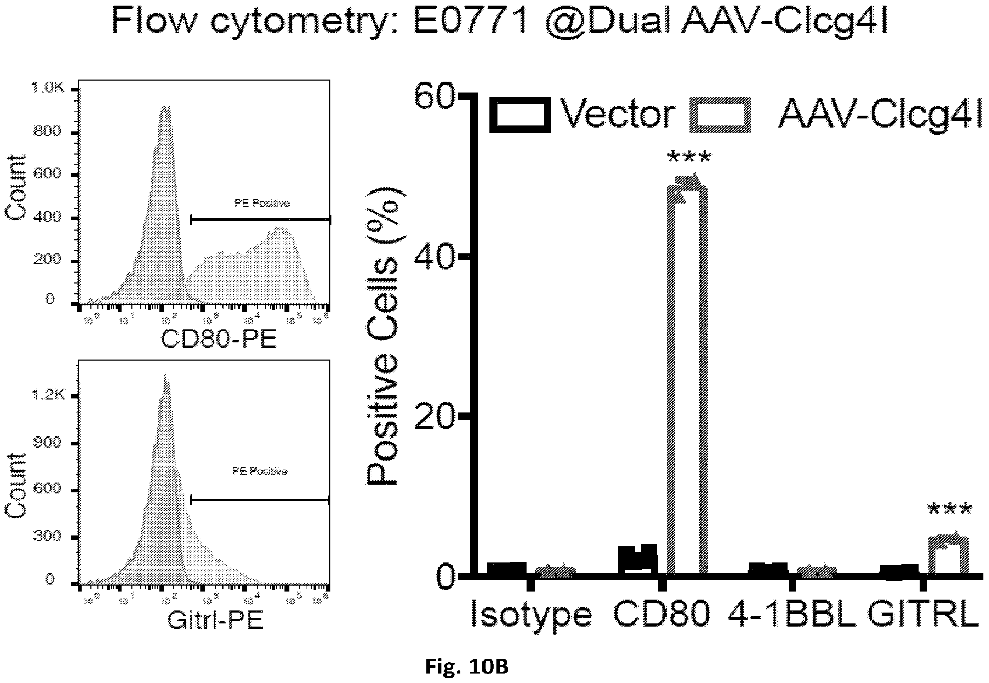

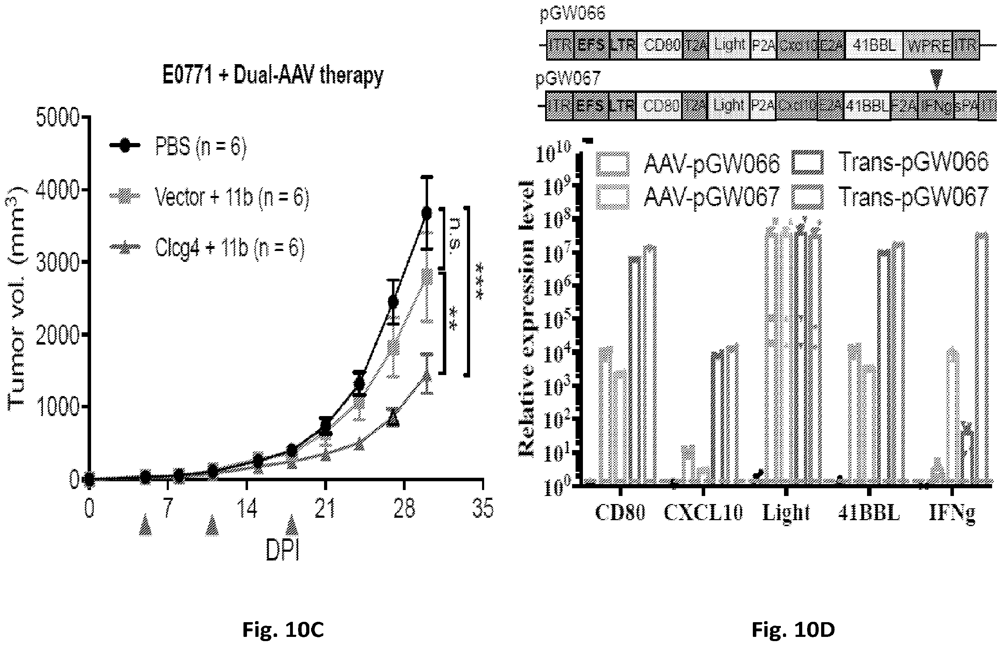

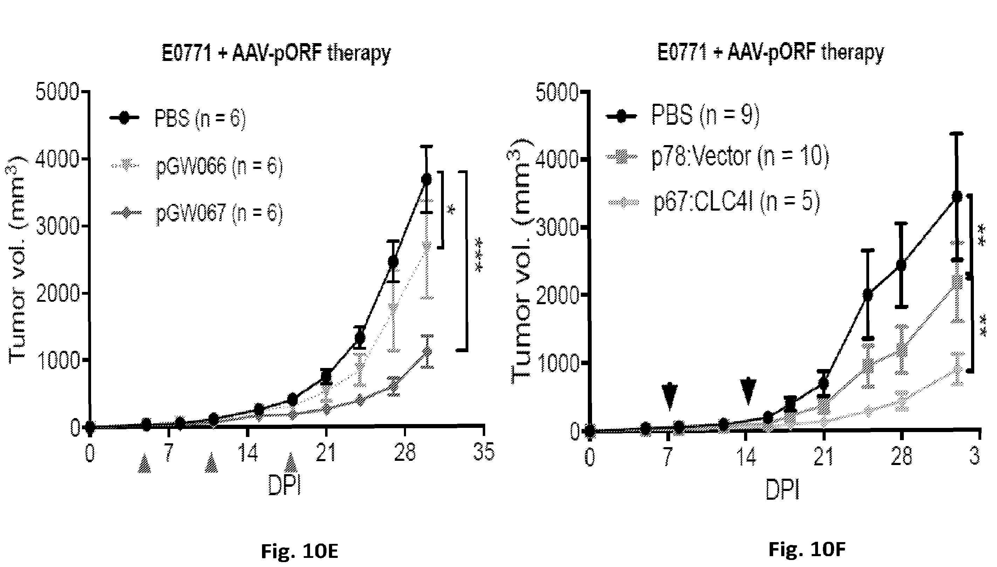

Figs. 10A-10F illustrate therapeutic effects by multiplexed activation of Cd80, Light, Cxcl10, Gitrl, 41bbl, and IFNg in tumor cells by AAV-delivered CRISPRa or AAV-ORFs (polycistronic open-reading-frames). Fig. 10A left: AAV-CRISPRa-mediated transcriptional activation of Cd80, Light, Cxcl10, Gitrl, 41bbl, and IFNg, normalized to vector-transduced controls (n = 8, mutiple t-test, AAV-sgRNAs versus vector Cd80, P= 0.01; Light, P = 0.00001; Cxcl10, P = 0.0003; Gitrl, P = 0.0004; 41bbl, P = 0.00002; Ifny, P < 0.0001); Fig. 10A right: Co-transfection of Dual AAV-CRISPRa-mediated transcriptional activation of Cd80, Light, Cxcl10, Gitrl, 41bbl, and IFNg, normalized to control (n = 4). Fig. 10B shows the expression of CD80, 41BBL, and Gitrl in dual-AAV-CRISPRa infected cells as assessed by flow cytometry. Fig. 10C shows the therapeutic effects of intratumoral injected AAV-CRISPRa. Dual-AAV mediated activation of Cd80, light/Tnfsf14, Cxcl10, 4-1BBL/Tnfsf9, and Ifng (CLC4I). Growth curves of E0771 tumors treated with PBS (n = 6), AAV-dCas9 + Vector (n = 6), AAV-dCas9 + CLCG4 (dual AAV delivered CRISRPa of Clcg (n = 6). Two-way ANOVA: AAV-dCas9 + Vector vs. PBS, *p* = 0.9675; AAV-dCas9 + Clcg4 vs. PBS, *p* < 0.0001; AAV-dCas9 + Clcg4 vs. AAV-dCas9 + Vector, *p* = 0.0028. Fig. 10D illustrates express Cd80, Light, Cxcl10, 41bbl, or/and IFNg using AAV polycistronic ORFs. Both transfection and AAV infection lead to high levels' expression of these molecules. Fig. 10E illustrates the therapeutic effects of intratumoral injected AAV-ORFs expressing Cd80, Light, Cxcl10, 41bbl (orfClc4, n = 6), or AAV expressing Cd80, Light, Cxcl10, 41bbl, Ifng (orfClc4I, n = 6). Fig. 10F illustrates the therapeutic effects of intratumoral injected AAV-ORFs expressing Cd80, Light, Cxcl10, 41bbl, Ifng (orfClc4I, n = 5). Two-way ANOVA: AAV-orfCLC4 vs. PBS, *p* < 0.0001; AAV-Vector vs. PBS, p = 0.0041; AAV-orfCLC4 vs. AAV-Vector, *p* = 0.0038. Two-way ANOVA: AAV-orfCLC4 vs. PBS, *p =* 0.6182; AAV-orfCLC4I vs. PBS, p < 0.0001. Error bars: Data points in this figure are presented as mean ± s.e.m. Asterisks: * p < 0.05, ** p < 0.01, *** p < 0.001.

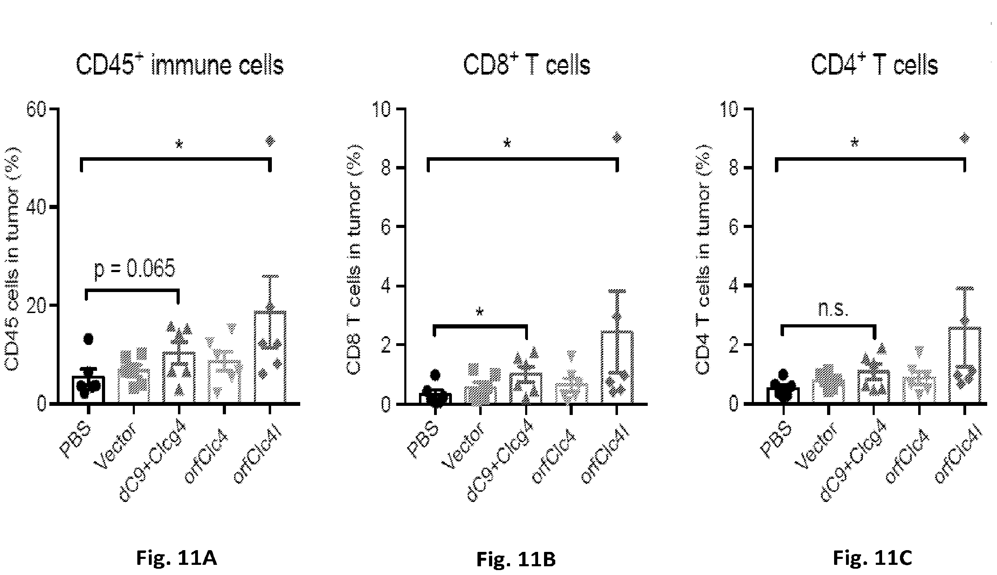

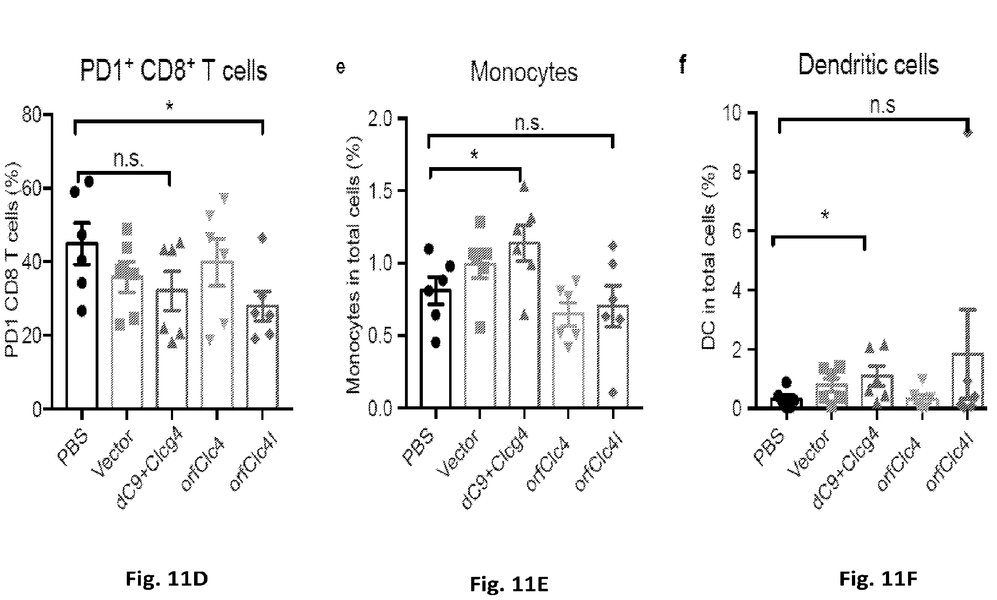

Figs. 11A-11F illustrate the finding that AAV-mediated costimulatory molecule expression promotes tumor immune infiltration. Fig. 11A shows flow cytometry quantification of CD45+ immune cells in tumor microenvironment at DPI = 30. Percentage of CD45+ immune cells out of total cells in tumors treated with PBS, dual-AAV-vector, dual-AAV-Clcg4, AAV-orfClc4, AAV-orfClc4I (n = 5 mice per group; two-tailed Mann-Whitney test: AAV-dCas9 + Clcg4 vs. PBS, P = 0.0931; AAV-orfClc4I vs. PBS, P = 0.0411). Fig. 11B shows percentage of CD8+ T cells out of total cells in tumors treated with PBS, dual-AAV-vector, dual-AAV-Clcg4, AAV-orfClc4, AAV-orfClc4I (n = 5 mice per group; two-tailed Mann-Whitney test: AAV-dCas9 + Clcg4 vs. PBS, P = 0.0411; AAV-orfClc4I vs. PBS, P = 0.0411). Fig. 11C shows percentage of CD4+ T cells out of total cells in tumors treated with PBS, dual-AAV-vector, dual-AAV-Clcg4, AAV-orfClc4, AAV-orfClc4I (n = 5 mice per group; two-tailed Mann-Whitney test: AAV-dCas9 + Clcg4 vs. PBS, P = 0.1320; AAV-orfClc4I vs. PBS, P = 0.0152). Fig. 11D shows flow cytometry quantification of PD1+CD8+ T cells out of total CD8+ T cells in tumors treated with PBS, dual-AAV-vector, dual-AAV-Clcg4, AAV-orfClc4, AAV-orfClc4I (n = 5 mice per group; two-tailed Mann-Whitney test: AAV-dCas9 + Clcg4 vs. PBS, P = 0.1797; AAV-orfClc4I vs. PBS, P = 0.026). Fig. 11E shows flow cytometry quantification of monocytes out of total cells in tumors treated with PBS, dual-AAV-vector, dual-AAV-Clcg4, AAV-orfClc4, AAV-orfClc4I (n = 5 mice per group; two-tailed Mann-Whitney test: AAV-dCas9 + Clcg4 vs. PBS, P = 0.0411; AAV-orfClc4I vs. PBS, P = 0.6991). Fig. 11F shows flow cytometry quantification of dendritic cells out of total cells in tumors treated with PBS, dual-AAV-vector, dual-AAV-Clcg4, AAV-orfClc4, AAV-orfClc4I (n = 5 mice per group; two-tailed Mann-Whitney test: AAV-dCas9 + Clcg4 vs. PBS, P = 0.0411; AAV-orfClc4I vs. PBS, P = 0.5887). Error bars: Data points in this figure are presented as mean ± s.e.m. Asterisks: * p < 0.05, ** p < 0.01, *** p < 0.001.

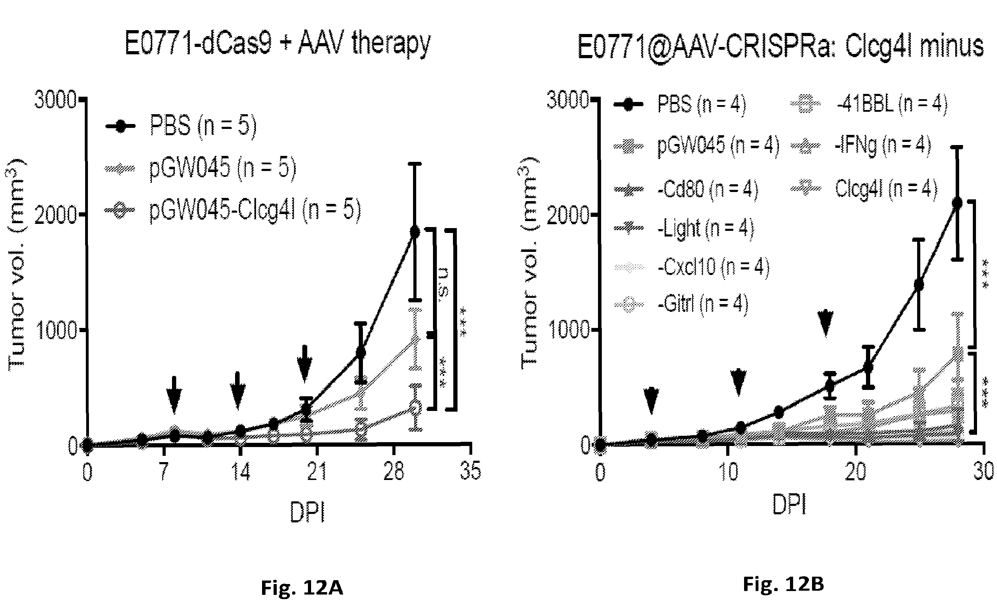

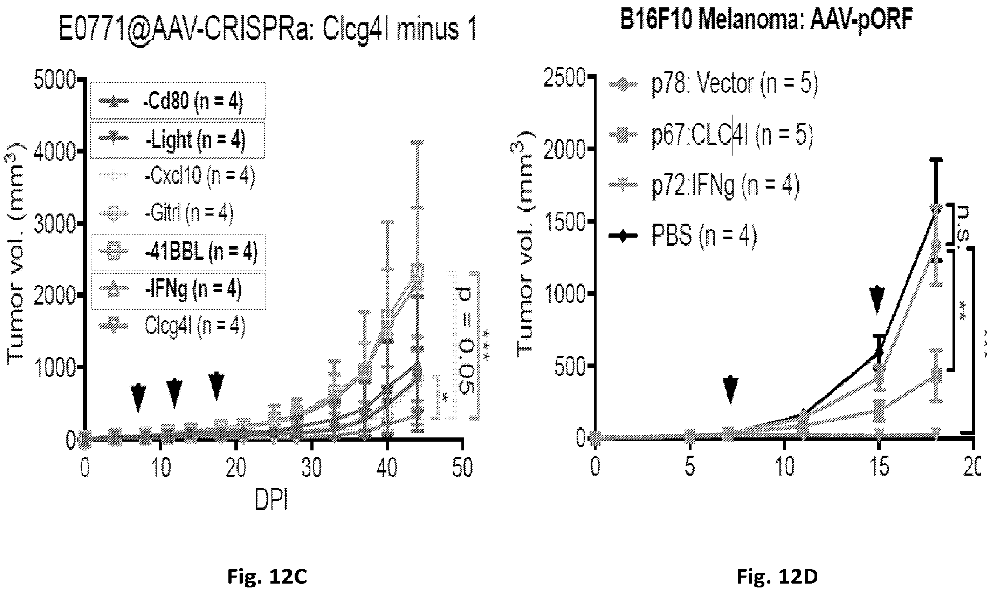

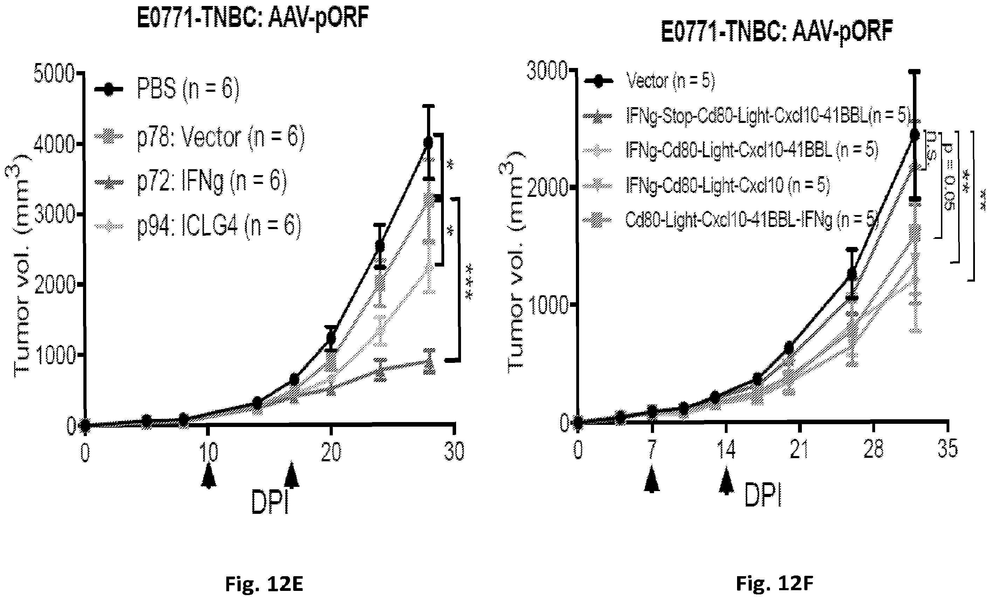

Figs. 12A-12F illustrate optimized combinations of immune-stimulating molecules for tumor therapy. Fig. 12A illustrates the therapeutic effects of intra-tumoral injected AAV-CRISPR activating Cd80, light, Cxcl10, 4-1BBL, Gitrl and Ifng (Clcg4I). Growth curves of E0771 tumors treated with PBS (n = 5), AAV-Vector (pGW045; n = 5), or AAV-Clcg4I (pGW045-Clcg4I). Two-way ANOVA: AAV- Vector vs. PBS, *p* = 0.0871; AAV-Clcg4 vs. PBS, *p* < 0.0001; AAV- Clcg4 vs. AAV-Vector, *p =* 0.0009. Figs. 12B-12C illustrate further optimization of the combinatorial genes pool by removing one gene in each pool. Fig. 12B shows growth curves of E0771 tumors treated with PBS (n = 4), AAV-Vector (n = 4), AAV-Clcg4I (n = 4), or AAV-Cleg4Iminus1 (n = 4). Two-way ANOVA: AAV-Vector vs. PBS, *p* = 0.0002; AAV- Clcg4I vs. AAV-Vector, *p* = 0.0002. Fig. 12C shows growth curves of E0771 tumors treated with AAV-Clcg4I (n = 4), or AAV-Clcg4I minus1 (n = 4). Two-way ANOVA: AAV- Clcg4I vs. 41BBL, *p <* 0.0001; AAV- Clcg4I vs. -IFNg, *p* = 0.05; AAV- Clcg4I vs. -CD80, *p =* 0.095; AAV- Clcg4I vs. -Light, *p =* 0.05; AAV- Clcg4I vs. -Gitrl, *p =* 0.0116. Fig. 12D shows growth curves of B16F10 melanoma treated with PBS (n = 4), AAV-Vector (n = 5), AAV-p67:Clc4I (Cd80, Light, Cxcl10, 41bbl, Ifng; n = 5), AAV-P72:IFNg (n = 4). Two-way ANOVA: AAV-Vector vs. PBS, *p* = 0.3495; AAV-p67:Clc4I vs. Vector, *p =* 0.0012; AAV-p72:IFNg vs. PBS, p < 0.0001. Fig. 12E shows growth curves of E0771 tumors treated with PBS (n = 6), AAV-Vector (n = 6), AAV-P72:IFNg (n = 6), AAV-p94:IClG4 (Ifng, Cd80, Light, Gitrl, 41bbl; n = 6). Fig. 12F shows growth curves of E0771 tumors treated with AAV-Vector (n = 5), AAV-polycistronic P119:IFNg-STOP-CD80-Light-Cxcl10-41BBL (n = 5), AAV-polycistronic P118:IFNg-CD80-Light-Cxcl10-41BBL (n = 5), AAV-polycistronic P100:IFNg-CD80-Light-Cxcl10 (n = 5), AAV-P67: CD80-Light-Cxcl10-41BBL-IFNg (n = 5). Two-way ANOVA: AAV-Vector vs. PBS, *p* = 0.0455; AAV-p94:ICLG4 vs. Vector, *p* = 0.0111; AAV-p72:IFNg vs. PBS, p < 0.0001. Error bars: Data points in this figure are presented as mean ± s.e.m. Asterisks: * p < 0.05, ** p < 0.01, *** p < 0.001

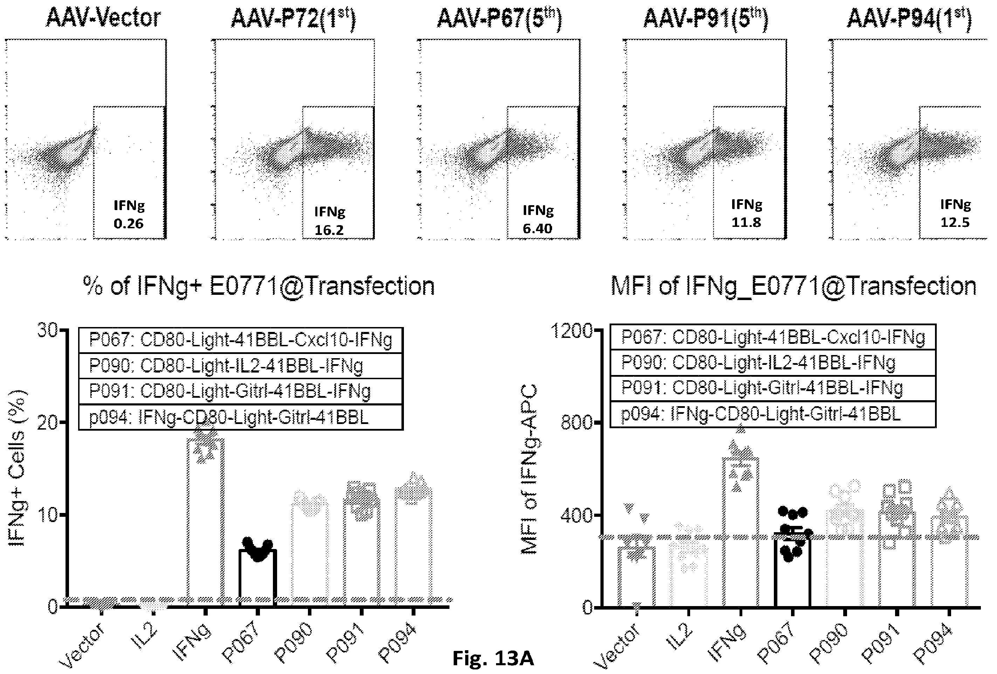

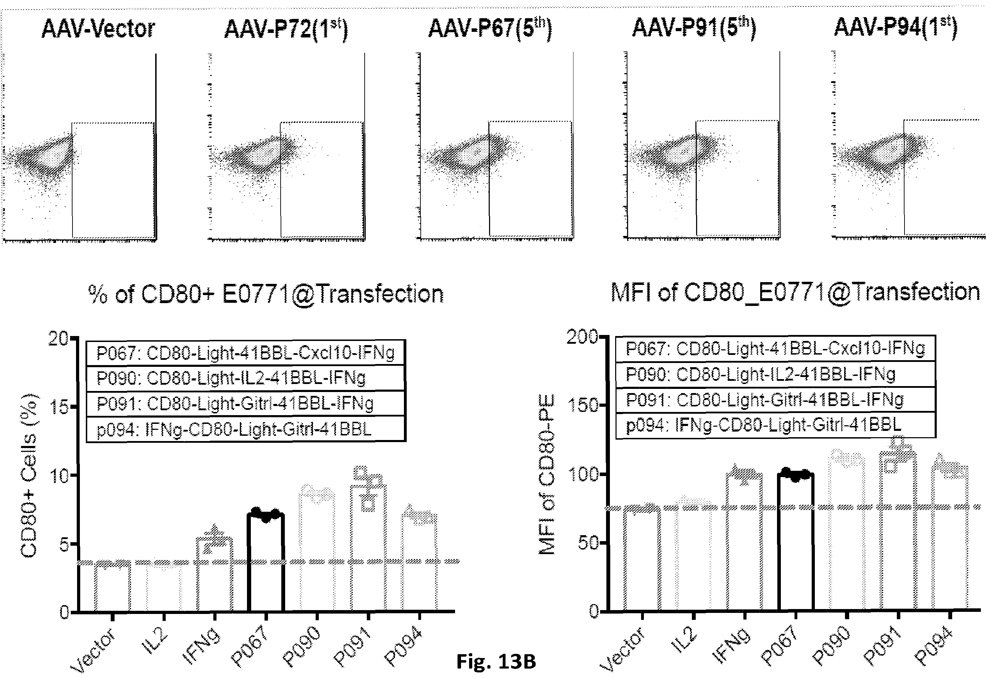

Figs. 13A-13B illustrate the expression levels of IFNg and CD80 in transfected cells using different polycistronic AAV-ORFs. Fig. 13A Upper panel: Representative flow cytometry plots demonstrating AAV-ORFs-mediated expression of IFNg in different AAV constructs transfected E0771 cells; low panel (left): the percentage of IFNg expressing cells in different constructs-transfected E0771 cells; low panel (right): the median fluorescent intensity (MFI) of IFNg-APC in different constructs-transfected E0771 cells. Fig. 13B Upper panel: Representative flow cytometry plots demonstrating AAV-ORFs-mediated expression of CD80 in different AAV constructs transfected E0771 cells; low panel (left): the percentage of CD80+ cells in different constructs-transfected E0771 cells; low panel (right): the median fluorescent intensity (MFI) of CD80-PE in different constructs-transfected E0771 cells.

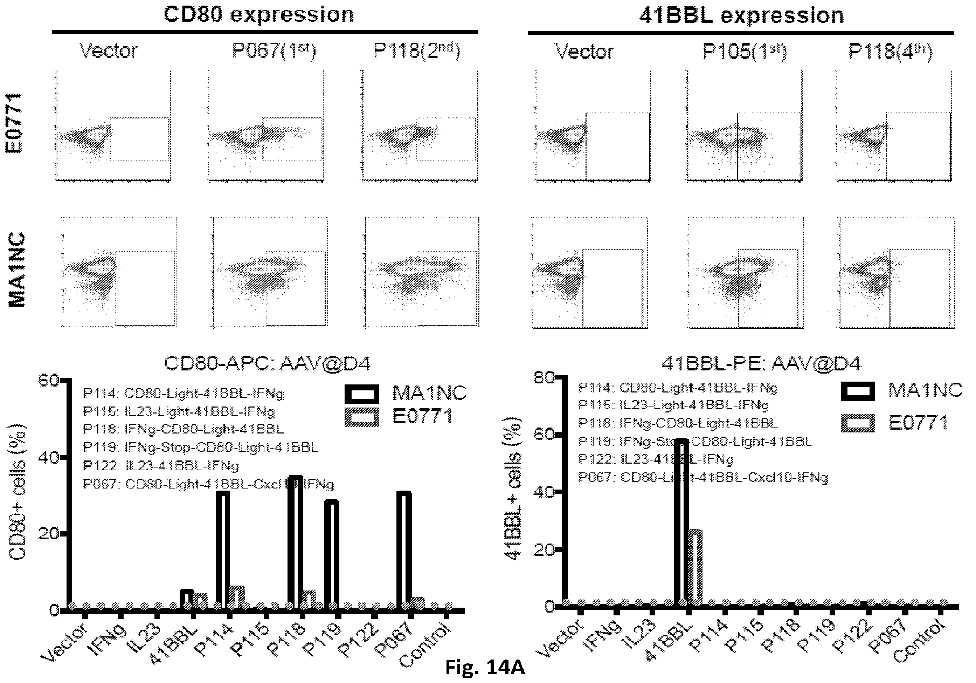

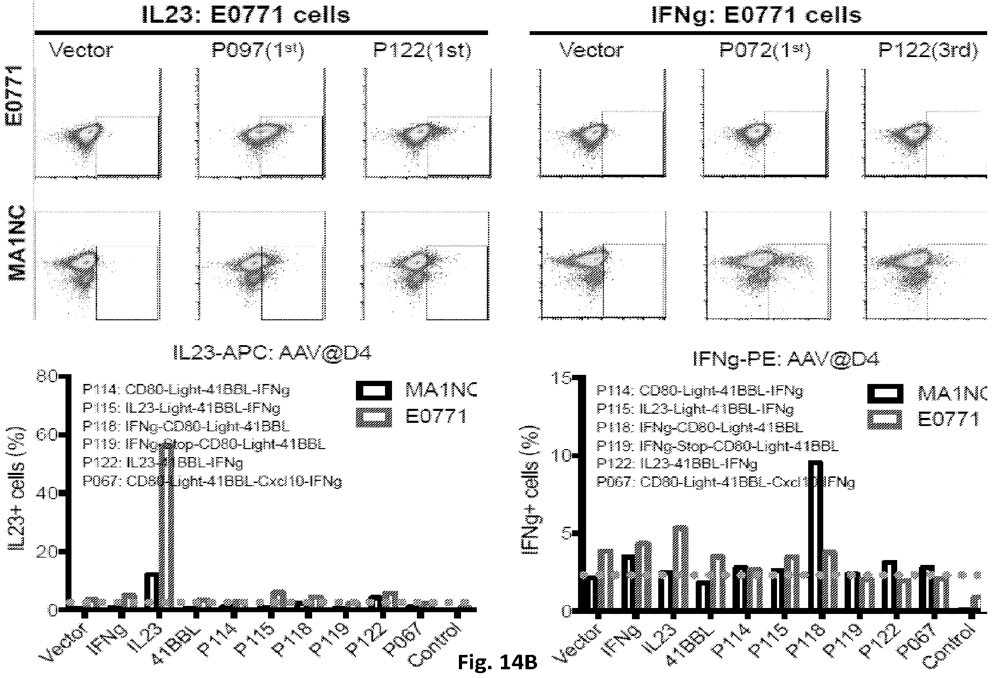

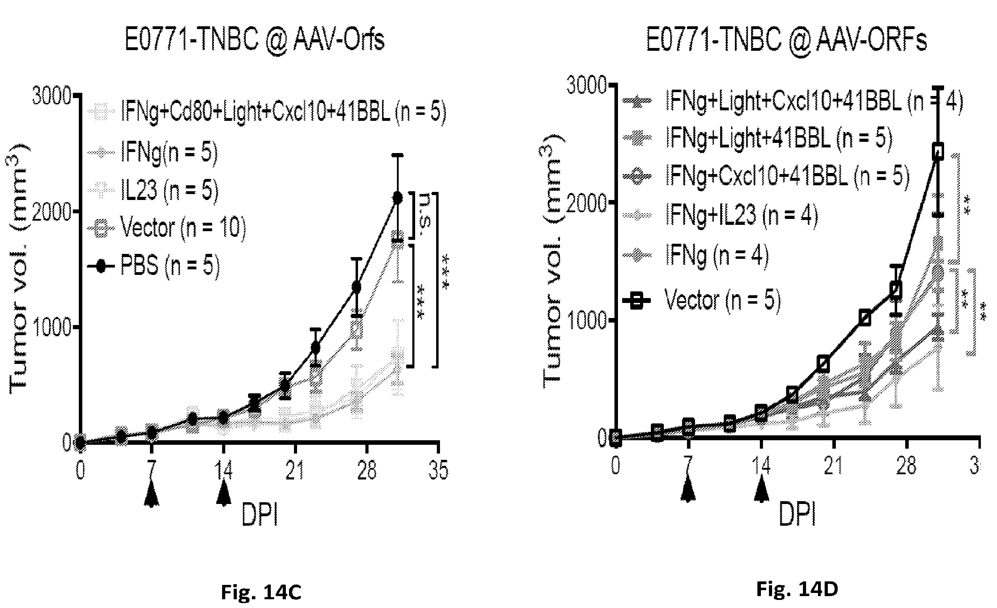

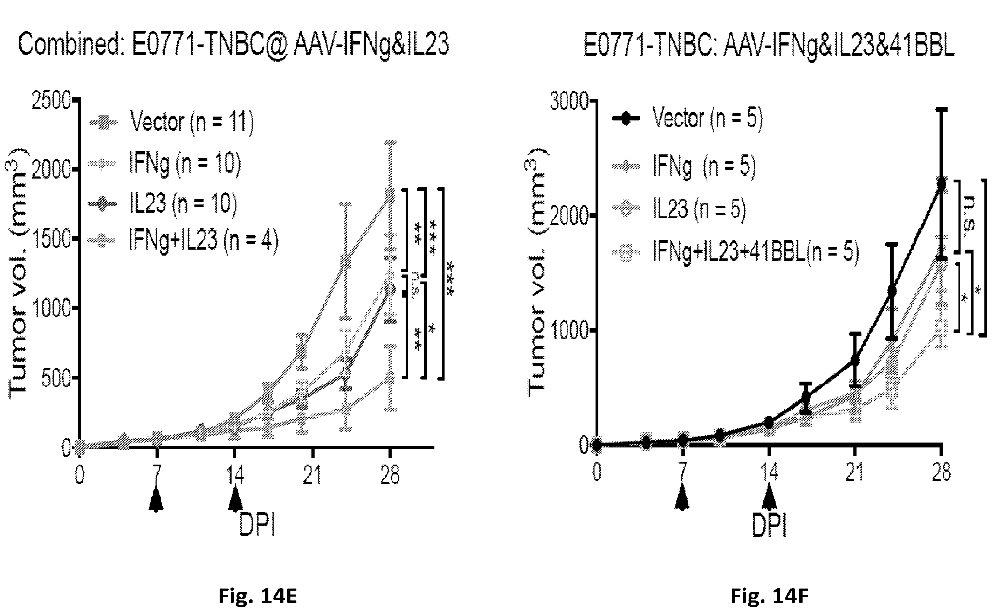

Figs. 14A-14F illustrate optimizing costimulatory molecule combinations for tumor therapy. Fig. 14A Upper panel: Representative flow cytometry plots demonstrating AAV-ORFs-mediated expression of CD80 and 41BBL in AAV-infected E0771 or MAINC cells; lower panel: the percentage of CD80 and 41BBL positive cells in different AAV-infected E0771 and MAINC cells. Fig. 14B Upper panel: Representative flow cytometry plots demonstrating AAV-ORFs-mediated expression of IL23 and IFNg in AAV-infected E0771 or MAINC cells; lower panel: the percentage of IL23 and IFNg positive cells in different AAV-infected E0771 and MAINC cells. Fig. 14C shows tumor growth curves of E0771 tumors treated with PBS (n = 5), AAV-Vector (n = 10), AAV-IL23 (n = 5), AAV-IFNg (n = 6), Pooled AAVs(IFNg + CD80 + LIGHT + CXCL10 + 41BBL; n = 5). Fig. 14D shows tumor growth curves of E0771 tumors treated AAV-Vector (n =5), AAV-IFNg (n = 4), AAV-IFNg+AAV-IL23 (n = 5), pooled AAVs (IFNg + CXCL10 + 41BBL; n = 5), pooled AAVs (IFNg + LIGHT + 41BBL; n = 5), pooled AAV(IFNg + LIGHT + CXCL10 + 41BBL; n = 5). Fig. 14E shows tumor growth curves of E0771 tumors treated AAV-Vector (n =11), AAV-IFNg (n = 10), AAV-IL23 (n = 10), AAV-IFNg+AAV-IL23 (n = 4). Fig. 14F shows tumor growth curves of E0771 tumors treated AAV-Vector (n = 5), AAV-IFNg (n = 5), AAV-IL23 (n = 5), AAV-IFNg+AAV-IL23+AAV-41BBL (n = 5). Error bars: Data points in this figure are presented as mean ± s.e.m. Asterisks: * p < 0.05, ** p < 0.01, *** p < 0.001.

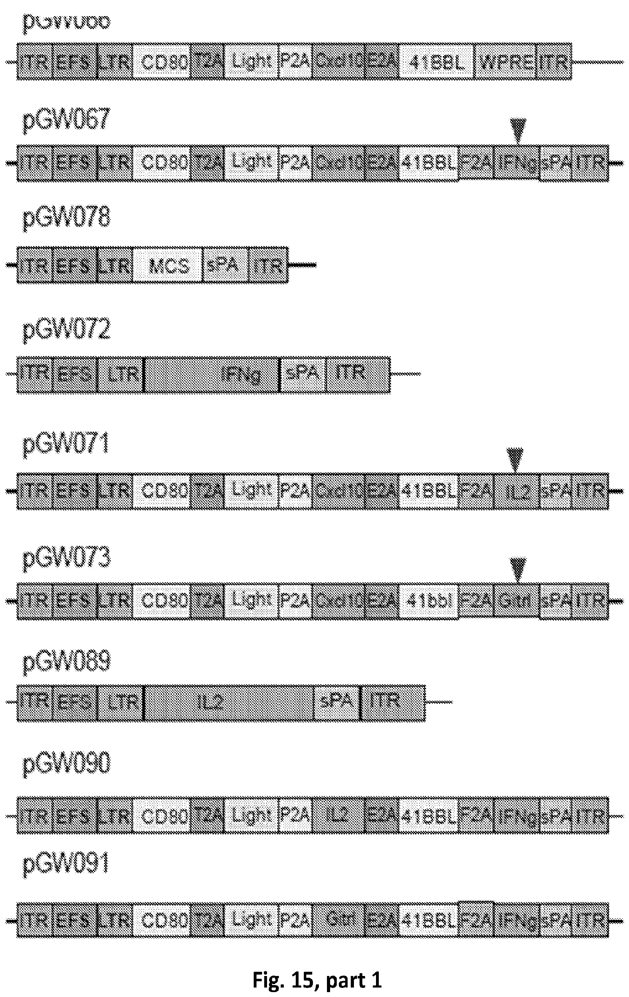

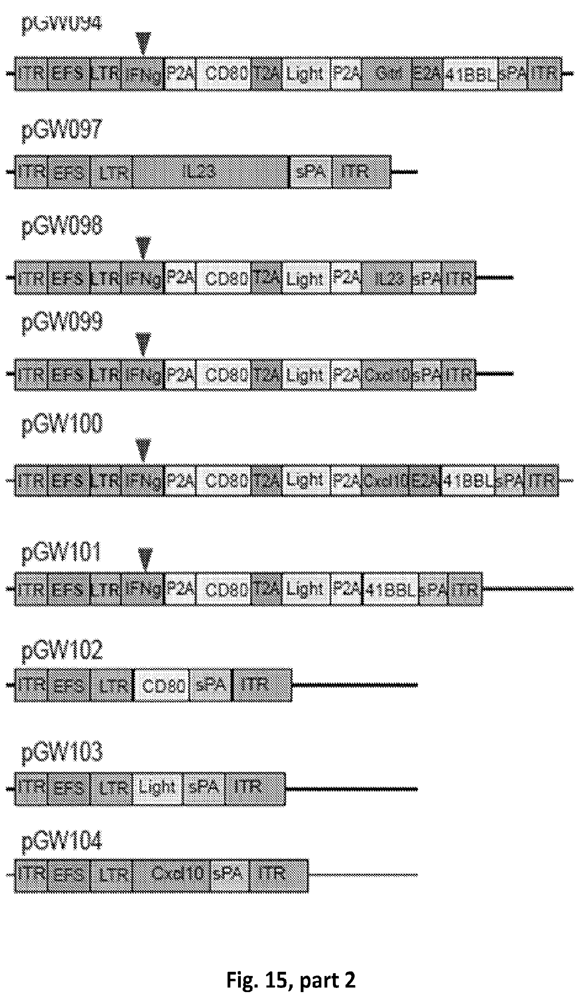

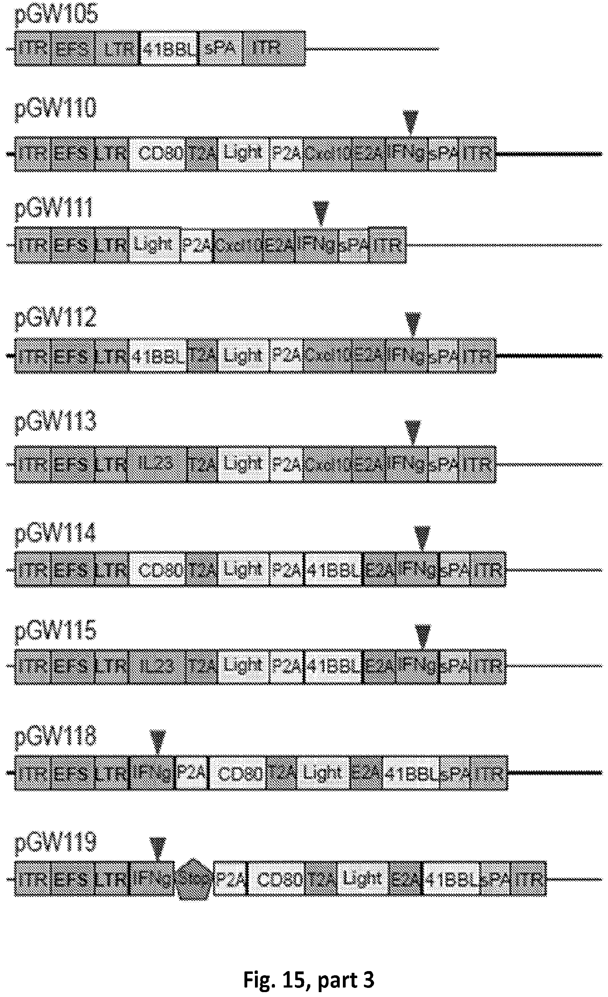

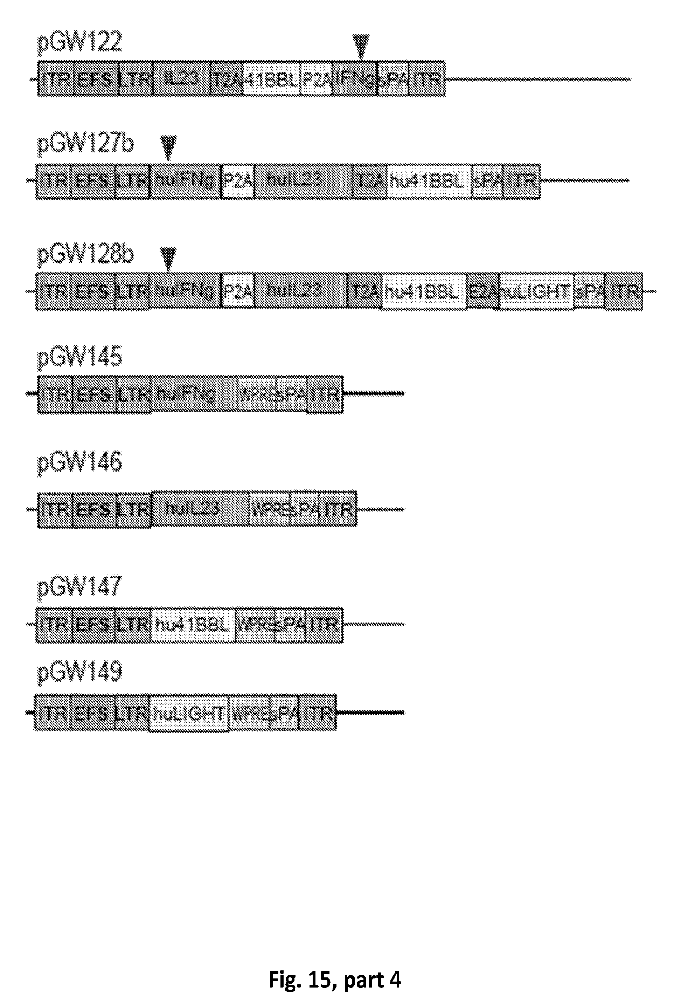

Fig. 15 depicts schematics of the construct designs for AAV-ORFs. Constructs include: pGW066 inserts(including ITR): 4542 bp (SEQ ID NO: 2); pGW067 inserts(including ITR): 4513 bp (SEQ ID NO: 3); pGW071 inserts(including ITR): 4556 bp (SEQ ID NO: 24); pGW072 inserts(including ITR): 1360 bp (SEQ ID NO: 25); pGW073 inserts(including ITR): 4567 bp (SEQ ID NO: 26); pGW078 inserts(including ITR): 895 bp (SEQ ID NO: 27); pGW071 inserts(including ITR): 4556 bp (SEQ ID NO: 24); pGW089 inserts(including ITR): 1401 bp (SEQ ID NO: 57); pGW090 inserts(including ITR): 4726 bp (SEQ ID NO: 28); pGW091 inserts(including ITR): 4738 bp (SEQ ID NO: 29); pGW094 inserts(including ITR): 4723 bp (SEQ ID NO:58); pGW096 inserts(including ITR): 5209 bp (SEQ ID NO: 30); pGW097 inserts(including ITR): 1899 bp (SEQ ID NO: 31); pGW098 inserts(including ITR): 4207 bp (SEQ ID NO: 32); pGW099 inserts(including ITR): 3496 bp (SEQ ID NO: 33); pGW100 inserts(including ITR): 4498 bp (SEQ ID NO: 34); pGW101 inserts(including ITR): 4132 bp (SEQ ID NO: 35); pGW102 inserts(including ITR): 1816 bp (SEQ ID NO: 36); pGW103 inserts(including ITR): 1612 bp (SEQ ID NO: 37); pGW104 inserts(including ITR): 1189 bp (SEQ ID NO: 38); pGW105 inserts(including ITR): 1822 bp (SEQ ID NO: 39); pGW110 inserts(including ITR): 4080 bp (SEQ ID NO: 40); pGW111 inserts(including ITR): 3093 bp (SEQ ID NO: 41); pGW112 inserts(including ITR): 4083 bp (SEQ ID NO: 42); pGW113 inserts(including ITR): 4161 bp (SEQ ID NO: 43); pGW114 inserts(including ITR): 4713 bp (SEQ ID NO: 44); pGW115 inserts(including ITR): 4794 bp (SEQ ID NO: 45); pGW118 inserts(including ITR): 4133 bp (SEQ ID NO: 46); pGW119 inserts(including ITR): 4133 bp (SEQ ID NO: 47); pGW122 inserts(including ITR): 4002 bp (SEQ ID NO: 48); pGW127b inserts(including ITR): 3852 bp (SEQ ID NO: 49); pGW128b inserts(including ITR): 4671 bp (SEQ ID NO: 50); pGW145 inserts(including ITR): 2023 bp (SEQ ID NO: 51); pGW146 inserts(including ITR): 2509 bp (SEQ ID NO: 52); pGW147 inserts(including ITR): 2287 bp (SEQ ID NO: 53); pGW149 inserts(including ITR): 2260 bp (SEQ ID NO: 54)

### DETAILED DESCRIPTION

### Definitions

Unless defined otherwise, all technical and scientific terms used herein have the same meaning as commonly understood by one of ordinary skill in the art to which the invention pertains. Although any methods and materials similar or equivalent to those described herein can be used in the practice for testing of the present invention, the preferred materials and methods are described herein. In describing and claiming the present invention, the following terminology will be used.

It is also to be understood that the terminology used herein is for the purpose of describing particular embodiments only, and is not intended to be limiting.

The articles "a" and "an" are used herein to refer to one or to more than one (*i.e.,* to at least one) of the grammatical object of the article. By way of example, "an element" means one element or more than one element.

"About" as used herein when referring to a measurable value such as an amount, a temporal duration, and the like, is meant to encompass variations of ±20% or ± 10%, more preferably ±5%, even more preferably ±1%, and still more preferably 0.1% from the
As used herein, the term "autologous" is meant to refer to any material derived from the same individual to which it is later to be re-introduced into the individual.

"Allogeneic" refers to any material derived from a different animal of the same species.

As used herein, the term "bp" refers to base pair.

The term "complementary" refers to the degree of anti-parallel alignment between two nucleic acid strands. Complete complementarity requires that each nucleotide be across from its opposite. No complementarity requires that each nucleotide is not across from its opposite. The degree of complementarity determines the stability of the sequences to be together or anneal/hybridize. Furthermore, various DNA repair functions as well as regulatory functions are based on base pair complementarity.

The term "CRISPR/Cas" or "clustered regularly interspaced short palindromic repeats" or "CRISPR" refers to DNA loci containing short repetitions of base sequences followed by short segments of spacer DNA from previous exposures to a virus or plasmid. Bacteria and archaea have evolved adaptive immune defenses termed CRISPR/CRISPR-associated (Cas) systems that use short RNA to direct degradation of foreign nucleic acids. In bacteria, the CRISPR system provides acquired immunity against invading foreign DNA via RNA-guided DNA cleavage.

The "CRISPR/Cas9" system or "CRISPR/Cas9-mediated gene editing" refers to a type II CRISPR/Cas system that has been modified for genome editing/engineering. It is typically comprised of a "guide" RNA (gRNA) and a non-specific CRISPR-associated endonuclease (Cas9). "Guide RNA (gRNA)" is used interchangeably herein with "short guide RNA (sgRNA)" or "single guide RNA (sgRNA). The sgRNA is a short synthetic RNA composed of a "scaffold" sequence necessary for Cas9-binding and a user-defined ~20 nucleotide "spacer" or "targeting" sequence which defines the genomic target to be modified. The genomic target of Cas9 can be changed by changing the targeting sequence present in the sgRNA.

"CRISPRa" system refers to a modification of the CRISPR-Cas9 system that functions to activate or increase gene expression. In certain embodiments, the CRISPRa system is comprised of a catalytically dead RNA/DNA guided endonuclease, such as dCas9, dCas12a/dCpf1, dCas12b/dC2c1, dCas12c/dC2c3, dCas12d/dCasY, dCas12e/dCasX, dCas13a/dC2c2, dCas13b, dCas13c, dCas14, dead Cascade complex, or others; at least one transcriptional activator; and at least one sgRNA that functions to increase expression of at least one gene of interest. The term "activation" as used herein refers to an increase in gene expression of one or more genes.

"dCas9" as used herein refers to a catalytically dead Cas9 protein that lacks endonuclease activity. "dSaCas9" refers to dCas9 derived from *Staphylococcus aureus.* dSpCas9" refers to dCas9 derived from *Streptococcus pyogenes.*

A "disease" is a state of health of an animal wherein the animal cannot maintain homeostasis, and wherein if the disease is not ameliorated then the animal's health continues to deteriorate. In contrast, a "disorder" in an animal is a state of health in which the animal is able to maintain homeostasis, but in which the animal's state of health is less favorable than it would be in the absence of the disorder. Left untreated, a disorder does not necessarily cause a further decrease in the animal's state of health.

The term "downregulation" as used herein refers to the decrease or elimination of gene expression of one or more genes.

"Effective amount" or "therapeutically effective amount" are used interchangeably herein, and refer to an amount of a compound, formulation, material, or composition, as described herein effective to achieve a particular biological result or provides a therapeutic or prophylactic benefit. Such results may include, but are not limited to, anti-tumor activity as determined by any means suitable in the art.

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (*i.e.,* rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

As used herein "endogenous" refers to any material from or produced inside an organism, cell, tissue or system.

As used herein, the term "exogenous" refers to any material introduced from or produced outside an organism, cell, tissue or system.

The term "expression" as used herein is defined as the transcription and/or translation of a particular nucleotide sequence driven by its promoter.

"Expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an *in vitro* expression system. Expression vectors include all those known in the art, such as cosmids, plasmids (*e.g.,* naked or contained in liposomes) and viruses (*e.g.,* Sendai viruses, lentiviruses, retroviruses, adenoviruses, and adeno-associated viruses) that incorporate the recombinant polynucleotide.

"Homologous" as used herein, refers to the subunit sequence identity between two polymeric molecules, *e.g.,* between two nucleic acid molecules, such as, two DNA molecules or two RNA molecules, or between two polypeptide molecules. When a subunit position in both of the two molecules is occupied by the same monomeric subunit; *e.g.,* if a position in each of two DNA molecules is occupied by adenine, then they are homologous at that position. The homology between two sequences is a direct function of the number of matching or homologous positions; *e.g.,* if half *(e.g.,* five positions in a polymer ten subunits in length) of the positions in two sequences are homologous, the two sequences are 50% homologous; if 90% of the positions (*e.g.,* 9 of 10), are matched or homologous, the two sequences are 90% homologous.

"Identity" as used herein refers to the subunit sequence identity between two polymeric molecules particularly between two amino acid molecules, such as, between two polypeptide molecules. When two amino acid sequences have the same residues at the same positions; *e.g.,* if a position in each of two polypeptide molecules is occupied by an Arginine, then they are identical at that position. The identity or extent to which two amino acid sequences have the same residues at the same positions in an alignment is often expressed as a percentage. The identity between two amino acid sequences is a direct function of the number of matching or identical positions; *e.g.,* if half (*e.g.,* five positions in a polymer ten amino acids in length) of the positions in two sequences are identical, the two sequences are 50% identical; if 90% of the positions (*e.g.,* 9 of 10), are matched or identical, the two amino acids sequences are 90% identical.

As used herein, an "instructional material" includes a publication, a recording, a diagram, or any other medium of expression which can be used to communicate the usefulness of the compositions and methods of the invention. The instructional material of the kit of the invention may, for example, be affixed to a container which contains the nucleic acid, peptide, and/or composition of the invention or be shipped together with a container which contains the nucleic acid, peptide, and/or composition. Alternatively, the instructional material may be shipped separately from the container with the intention that the instructional material and the compound be used cooperatively by the recipient.

"Isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

The term "knockdown" as used herein refers to a decrease in gene expression of one or more genes.

The term "knockout" as used herein refers to the ablation of gene expression of one or more genes.

A "lentivirus" as used herein refers to a genus of the Retroviridae family. Lentiviruses are unique among the retroviruses in being able to infect non-dividing cells; they can deliver a significant amount of genetic information into the DNA of the host cell, so they are one of the most efficient vectors for gene delivery. HIV, SIV, and FIV are all examples of lentiviruses. Vectors derived from lentiviruses offer the means to achieve significant levels of gene transfer *in vivo.*

By the term "modified" as used herein, is meant a changed state or structure of a molecule or cell of the invention. Molecules may be modified in many ways, including chemically, structurally, and functionally. Cells may be modified through the introduction of nucleic acids.

By the term "modulating," as used herein, is meant mediating a detectable increase or decrease in the level of a response in a subject compared with the level of a response in the subject in the absence of a treatment or compound, and/or compared with the level of a response in an otherwise identical but untreated subject. The term encompasses perturbing and/or affecting a native signal or response thereby mediating a beneficial therapeutic response in a subject, preferably, a human.

A "mutation" as used herein is a change in a DNA sequence resulting in an alteration from a given reference sequence (which may be, for example, an earlier collected DNA sample from the same subject). The mutation can comprise deletion and/or insertion and/or duplication and/or substitution of at least one deoxyribonucleic acid base such as a purine (adenine and/or thymine) and/or a pyrimidine (guanine and/or cytosine). Mutations may or may not produce discernible changes in the observable characteristics (phenotype) of an organism (subject).

By "nucleic acid" is meant any nucleic acid, whether composed of deoxyribonucleosides or ribonucleosides, and whether composed of phosphodiester linkages or modified linkages such as phosphotriester, phosphoramidate, siloxane, carbonate, carboxymethyl ester, acetamidate, carbamate, thioether, bridged phosphoramidate, bridged methylene phosphonate, phosphorothioate, methylphosphonate, phosphorodithioate, bridged phosphorothioate or sulfone linkages, and combinations of such linkages. The term nucleic acid also specifically includes nucleic acids composed of bases other than the five biologically occurring bases (adenine, guanine, thymine, cytosine and uracil). In the context of the present invention, the following abbreviations for the commonly occurring nucleic acid bases are used. "A" refers to adenosine, "C" refers to cytosine, "G" refers to guanosine, "T" refers to thymidine, and "U" refers to uridine.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or an RNA may also include introns to the extent that the nucleotide sequence encoding the protein may in some version contain an intron(s).

The term "oligonucleotide" typically refers to short polynucleotides, generally no greater than about 60 nucleotides. It will be understood that when a nucleotide sequence is represented by a DNA sequence (*i.e.,* A, T, G, C), this also includes an RNA sequence (*i.e.,* A, U, G, C) in which "U" replaces "T".

As used herein, the terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. The polypeptides include natural peptides, recombinant peptides, synthetic peptides, or a combination thereof.

"Parenteral" administration of an immunogenic composition includes, *e.g.,* subcutaneous (s.c.), intravenous (i.v.), intramuscular (i.m.), or intrasternal injection, or infusion techniques.

As used herein, the terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. The polypeptides include natural peptides, recombinant peptides, synthetic peptides, or a combination thereof.

The term "polynucleotide" as used herein is defined as a chain of nucleotides. Furthermore, nucleic acids are polymers of nucleotides. Thus, nucleic acids and polynucleotides as used herein are interchangeable. One skilled in the art has the general knowledge that nucleic acids are polynucleotides, which can be hydrolyzed into the monomeric "nucleotides." The monomeric nucleotides can be hydrolyzed into nucleosides. As used herein polynucleotides include, but are not limited to, all nucleic acid sequences which are obtained by any means available in the art, including, without limitation, recombinant means, i.e., the cloning of nucleic acid sequences from a recombinant library or a cell genome, using ordinary cloning technology and PCR^{™}, and the like, and by synthetic means. Conventional notation is used herein to describe polynucleotide sequences: the left-hand end of a single-stranded polynucleotide sequence is the 5'- end; the left-hand direction of a double-stranded polynucleotide sequence is referred to as the 5'-direction.

A "sample" or "biological sample" as used herein means a biological material from a subject, including but is not limited to organ, tissue, exosome, blood, plasma, saliva, urine and other body fluid. A sample can be any source of material obtained from a subject.

The term "subject" is intended to include living organisms in which an immune response can be elicited (e.g., mammals). A "subject" or "patient," as used therein, may be a human or non-human mammal. Non-human mammals include, for example, livestock and pets, such as ovine, bovine, porcine, canine, feline and murine mammals. Preferably, the subject is human.

As used herein, a "substantially purified" cell is a cell that is essentially free of other cell types. A substantially purified cell also refers to a cell which has been separated from other cell types with which it is normally associated in its naturally occurring state. In some instances, a population of substantially purified cells refers to a homogenous population of cells. In other instances, this term refers simply to cell that have been separated from the cells with which they are naturally associated in their natural state. In some embodiments, the cells are cultured *in vitro.* In other embodiments, the cells are not cultured *in vitro.*

A "target site" or "target sequence" refers to a genomic nucleic acid sequence that defines a portion of a nucleic acid to which a binding molecule may specifically bind under conditions sufficient for binding to occur.

The term "therapeutic" as used herein means a treatment and/or prophylaxis. A therapeutic effect is obtained by suppression, remission, or eradication of a disease state.

The term "transfected" or "transformed" or "transduced" as used herein refers to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny.

To "treat" a disease as the term is used herein, means to reduce the frequency or severity of at least one sign or symptom of a disease or disorder experienced by a subject.

As used herein, "vaccinating" means administering a substance to a subject that induces an immune response against a disease.

A "vector" is a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid or a virus. The term should also be construed to include non-plasmid and non-viral compounds which facilitate transfer of nucleic acid into cells, such as, for example, polylysine compounds, liposomes, and the like. Examples of viral vectors include, but are not limited to, Sendai viral vectors, adenoviral vectors, adeno-associated virus vectors, retroviral vectors, lentiviral vectors, and the like.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

### Description

The subject matter of the invention is as set out in the appended claims. Recitations of methods of treatment in the following description are to be understood as relating to the substance in question for use in a method of treatment.

The present disclosure refers to compositions and methods for treating cancer. In certain unclaimed aspects, the present disclosure includes methods for treating cancer using a viral vector based (such as Adenovirus or AAV-based ) composition that utilizes the CRISPR activation (CRISPRa) system to activate endogenous genes. In certain unclaimed aspects, the present diclosure includes use of the CRISPRa system to identify key factors for generating direct ORF-based immune-gene therapies.

It was discovered herein that direct activation of endogenous genes using a single vector system is an effective means to amplify tumor-associated antigens to enhance anti-tumor immune responses. Highly multiplexed and customizable endogenous neoantigen activation using a single vector system has not yet been demonstrated prior to this disclosure. The present invention demonstrates the feasibility of using CRISPR technologies with RNA-guided precise genetic manipulations. The CRISPR activation (CRISPRa) system, based on dead Cas9 without nuclease activity (dCas9) (Qi et al.(20131) Cell 152, 1173-1183), enables simple and flexible regulation of gene expression with dCas9 - transcriptional activation domain fusion (Gilbert et al. (2013) Cell 154, 442-451), which is further augmented by the recruitment of synergistic activation mediators (SAM) (Konermann, et al. (2015) Nature 517, 583-588; Chavez, et al. (2015) Nat Methods 12, 326-328; Tanenbaum, et al. (2014) Cell 159, 635-646). Herein, the CRISPRa system was harnessed to manipulate endogenous gene expression to magnify anti-tumor immune responses. The approach was developed into multiplexed tumor vaccination strategies.

Another unclaimed aspect includes compositions and methods comprising off-the-shelf endogenous gene vaccination cancer vaccines. The off-the-shelf versions have fixed components, which can have multiple forms, including single component or multi-component vaccines. The compositions of these forms of cancer vaccines and their pre-clinical efficacy showed that they can be an effective means of prophylactic and therapeutic agents.

### CRISPR activation (CRISPRa) system

The CRISPR activation (CRISPRa) system is comprised of a catalytically inactive RNA-guided endonuclease or other endonucleases, such as but not limited to dCas9, dCas12a/dCpf1, dCas12b/dC2c1, dCas12c/dC2c3, dCas12d/dCasY, dCas12e/dCasX, dCas13a/dC2c2, dCas13b, dCas13c, dCas14, dead Cascade complex, or others. The CRISPRa system also comprises at least one transcriptional activator, and at least one sgRNA that functions to increase expression of at least one gene of interest. Like a standard CRISPR-Cas9 system, CRISPRa systems rely on sgRNAs to guide Cas9 to intended targets. However, while a standard CRISPR-Cas9 system creates breaks in DNA through the endonuclease activity of Cas9 and then manipulates DNA repair mechanisms for gene editing, CRISPRa systems are modified and employ transcriptional activators to increase expression of genes of interest.

"dCas9" refers to a catalytically dead Cas9 protein that lacks endonuclease activity. This can be accomplished by introducing point mutations in the two catalytic residues (D10A and H840A) of the gene encoding Cas9. In doing so, dCas9 is unable to cleave dsDNA but retains the ability to target and bind DNA. This alone is often enough to attenuate if not outright block transcription of the targeted gene if the gRNA positions dCas9 in a way that prevents transcriptional factors and RNA polymerase from accessing the DNA. However, this ability to bind DNA can also be exploited for activation since dCas9 has modifiable regions, typically the N and C terminus of the protein, that can be used to attach transcriptional activators. dCas9 can be derived, for example, from S. *pyogenes* (dSpCas9), *S. aureus* (dSaCas9) *N. meningiditis, S. thermopilus, F. novicida, C. jejuni, B. laterosporus,* or from other species.

Targeting specificity of the CRISPRa system is determined by complementary base-pairing of a small guide RNA (sgRNA) to the genomic loci. sgRNA is a chimeric noncoding RNA that can be subdivided into three regions: a base-pairing sequence, a dCas9-binding hairpin and a terminator. When designing a synthetic sgRNA, only the base-pairing sequence is modified. Secondary variables must also be considered: off-target effects (for which a simple BLAST run of the base-pairing sequence is required), maintenance of the dCas9-binding hairpin structure, and ensuring that no restriction sites are present in the modified sgRNA, as this may pose a problem in downstream cloning steps. Due to the simplicity of sgRNA design, this technology is amenable to genome-wide scaling.

Transcriptional activators are protein domains or whole proteins that can be linked to dCas9 or sgRNAs and assist in the recruitment of important co-factors as well as RNA Polymerase for transcription of the gene(s) targeted by the system. Transcriptional activators have a DNA binding domain and a domain for activation of transcription. The activation domain can recruit general transcription factors or RNA polymerase to the gene sequence. Activation domains can also function by facilitating transcription by stalled RNA polymerases, and in eukaryotes can act to move nucleosomes on the DNA or modify histones to increase gene expression. These activators can be introduced into the system through attachment to dCas9 or to the sgRNA. Transcriptional activators can be either mammalian cellular endogenous proteins that have activator function, activators from other species such as viruses, microbials or plants, their partial or mutant variants, engineered activators, or other forms of activators that can increase gene expression. A list of applicable viral activators include but are not limited to: VP16, VP32, VP64, VP160, HBx, NS proteins, and VMW65. A list of applicable microbial activators include but are not limited to: Lac operons and GAL4. A list of applicable mammalian cellular transcriptional activators include but are not limited to: CAP, ACTN1, ACTN2, ACTN2, ACTN4, ACTN4, ANKRD1, APEX1, ARID5B, ARL2BP, ASCC1, ASXL1, ATN1, ATXN7L3, ATXN7L3, ATXN7L3, BCL9, BCL9L, BCL10, BCL10, BICRA, BIRC2, BRCA1, BRD7, CALCOCO1, CALCOCO1, CALCOCO1, CALCOCO1, CARM1, CARM1, CARM1, CBFB, CCAR1, CCAR1, CCAR1, CCAR1, CCAR2, CCDC62, CEBPA, CENPJ, CITED1, CITED1, CITED2, CITED2, CITED2, CITED2, CITED2, CITED4, CITED4, CITED4, COPS5, CREBBP, CREBBP, CREBBP, CTBP2, CTNNB1, CTNNB1, CTNNB1, CTNNB1, CTNNB1, DAXX, DAXX, DCAF6, DCC, DDX17, DHX9, DR1, DYRK1B, EDF1, ELF3, ELOB, ENY2, ENY2, ENY2, EP300, EP300, EP300, FAM129B, FGF2, FHL5, FOXC1, GATA3, GATA3, GATA3, GATA4, GM20517, GMEB1, GMEB2, GPS2, GPS2, GTF2A2, GTF2A2, HAND1, HCFC1, HCFC1, HELZ2, HIF3A, HINFP, HIPK2, HMGA1, HMGA1, HMGA1B, HMGB2, HYAL2, ING4, ISL1, JADE1, JMJD6, JMY, JMY, JUN, JUN, JUNB, JUND, JUP, JUP, KAT2A, KAT2B, KAT2B, KAT5, KAT5, KAT6A, KDM1A, KDM5A, KMT2C, KMT2D, LPIN1, LPIN1, LPIN2, LPIN2, LPIN3, MAGED1, MAK, MAML1, MAML1, MAML1, MAML1, MAML2, MAML2, MAML3, MAML3, MAML3, MCIDAS, MED1, MED1, MED1, MED1, MED1, MED1, MED6, MED12, MED 12, MED 12, MED12L, MED13, MED14, MED16, MED17, MED17, MED20, MED21, MED24, MED27, MED31, MEF2A, MMS19, MRTFA, MRTFB, MRTFB, MTA1, MTA1, MTA1, MTA1, MTA2, MTA3, MTDH, MYCBP, MYOCD, MYOD1, MYSM1, MYT1L, NACA, NCOA1, NCOA1, NCOA1, NCOA1, NCOA1, NCOA2, NCOA2, NCOA2, NCOA2, NCOA2, NCOA2, NCOA3, NCOA3, NCOA3, NCOA3, NCOA3, NCOA6, NCOA6, NCOA7, NEUROD1, NEUROG3, NFE2L1, NKX2-2, NME2, NPAT, NPM1, NR1D1, NR1D2, NR1H2, NR1H3, NR1H3, NR1H4, NR1H5, NR1I2, NR1I3, NR3C1, NRBF2, NRIP1, NRIP1, NRIP1, NRL, NSD3, NUP98, NUPR1, PARK7, PCBD1, PDLIM1, PER2, PHF2, PKN1, PMF1, PMF1, PML, PML, PML, POU2AF1, POU3F1, POU3F2, POU3F2, POU4F1, POU4F2, POU5F1, PPARA, PPARD, PPARD, PPARG, PPARG, PPARG, PPARGC1A, PPARGC1A, PPARGC1A, PPARGC1A, PPARGC1A, PPARGC1A, PPARGC1B, PPARGC1B, PPRC1, PRDM16, PRKCB, PRMT2, PRPF6, PRRX1, PSIP1, PSMC3IP, PSMC3IP, PSMD9, PSMD9, PUS1, RAP2C, RARA, RARA, RARB, RARG, RBM14, RBM14, RBM39, RBPMS, RERE, REXO4, RNF20, RRP1B, RUVBL1, RXRB, SCAND1, SERTAD2, SETD3, SFR1, SFR1, SIX3, SLC30A9, SLC30A9, SMARCA2, SMARCA4, SMARCB1, SMARCB1, SMARCD3, SNW1, SNW1, SOX4, SOX11, SOX11, SOX12, SOX17, SP4, SRA1, SRA1, SRA1, SRA1, SRA1, SRA1, SS18, SS18, SS18L1, SS18L2, SUB1, SUB1, SUPT3, SUPT7L, TADA1, TADA1, TADA2A, TADA2B, TADA3, TADA3, TADA3, TAF1, TAF5L, TAF6L, TAF6L, TAF7, TAF7, TAF7L, TAF9, TAF11, TAF11, TAF12, TCF3, TDRD3, TFAP2A, TFAP2A, TFAP2A, TFAP2B, TFAP2B, TFAP2B, TGFB1I1, THRA, THRAP3, THRAP3, THRAP3, THRB, TRIM24, TRIM24, TRIM28, TRIP4, TRIP4, TRRAP, TSG101, UBE2L3, UBE3A, USP16, USP21, USP22, USP22, UTF1, UTF1, VDR, VGLL2, WBP2, WBP2, WBP2NL, WDR77, WNT3A, WWC1, WWOX, WWTR1, WWTR1, YAF2, YAP1, YAP1, ZBTB18, ZCCHC12, ZCCHC12, ZCCHC18, ZMIZ2.

Applicable CRISPRa systems demonstrated to be capable of activating transcription in mammalian species include but are not limited to: VP64-p65-Rta (VPR), Synergistic Activation Mediator (SAM), Suntag, p300, and VP160.

One example of a transcriptional activator (or transactivator domain) is VP64. VP64 is made up of four copies of VP16, a viral protein sequence of 16 amino acids that is used for transcriptional activation. Embodiments of the invention include various forms of VP64, for example a nucleic acid comprising dCas9 and/or VP64, or plasmids or vectors that encode the dCas9 and/or VP64 genes. One non-limiting example includes pcDNA-dCas9-VP64 (Plasmid #47107, from Addgene). Additional elements can be present in the nucleic acid encoding dCas9 and/or VP64, as in for example lenti vector EF1a-NLS-dCas9(N863)-VP64-2A-Blast-WPRE (Plasmid #61425 from Addgene), which additionally encodes a 2A Blast resistance marker. Another non-limiting example includes plasmid pLV hUbC-VP64 dCas9 VP64-T2A-GFP (Plasmid #59791 from Addgene) that co-expresses human optimized *S. pyogenes* dCas9 fused to two copies of VP64 and GFP.

Certain embodiments of the invention utilize the VP64-p65-Rta, or VPR, in which a VP64 transcriptional activator is joined to the C terminus of dCas9. In the dCas9-VPR protein, the transcription factors p65 and Rta are added to the C terminus of dCas9-VP64. Therefore, all three transcription factors are targeted to the same gene. The use of three transcription factors, as opposed to solely VP64, results in increased expression of targeted genes. dCas9-VPR can be used to increase expression of multiple genes within the same cell by putting multiple sgRNAs into the same cell.

In certain embodiments, the invention utilizes the Synergistic Activation Mediator (SAM) system. SAM makes use of not only VP64 but also sgRNA 2.0, which contains a sequence to recruit a viral protein fused to even more effectors (p65-hsf1). In one embodiment the SAM complex comprises dCas9-VP64, sgRNA, MS2-p65HSF-1. In one embodiment, the CRISPRa system comprises a nucleic acid encoding dCas9-VP64, a nucleic acid encoding MS2-p65-HSF1, and a genome-scale lentiviral SAM CRISPRa sgRNA library. Administering this type of CRISPRa system to a plurality of cells results in a highly diverse population of cells encompassing the entire sgRNA library.

The invention should be construed to work with any alternative activator, such as VP16, VP160, p65AD, p300 or any other transcriptional activator.

The invention should also be construed to work with any dCas9/CRISPRa system or any other adaptor system known in the art, including but not limited to: 1) RNA Scaffolds, which also utilizes sgRNA 2.0 and recruits 3 viral proteins fused to VP64, 2) Suntag, which sports a protruding chain of 10 peptide epitopes that are recognized by an entourage of antibodies fused to VP64, 3) The epigenetic editor p300, which deposits activating H3K27ac. 4) VP160, which is also known as CRISPR-on and has ten times the VPs of VP16, and 5) VP64-dCas9-BFP-VP64, which makes use of that much neglected N-terminus.

The CRISPRa systems disclosed herein can be used with an sgRNA library. With regard to any and all sgRNA libraries disclosed herein, it should be understood by one of ordinary skill in the art that when it is stated that the library comprises at least one sgRNA, it should be construed that the library can comprise one or more sgRNAs, all sgRNAs in the library, and and all integer values and numerical ranges of sgRNAs there between. For example, an sgRNA library comprising a total of 219 sgRNAs (e.g. SEQ ID NOs. 193-411) can comprise one sgRNA, all 219 sgRNAs, or and any and all integer values between 1 and 219. In other words, the library could include 1, 10, 20, 50, 100, 150, 200, or 219 sgRNAs and any and all values in between.

### CRISPRa screen assisted rational design of off-the-shelf viral immune gene therapies

Provided herein is a method of developing an off-the-shelf viral immune gene therapy. The method utilizes an *in vivo* CRISPRa screen to unbiasedly pinpoint the genetic factors that enhance host immune surveillance and tumor destruction ("rational design", or "immune gene therapy rationalization"). In one embodiment, the method comprises administering an APCM sgRNA library (*e.g.* SEQ ID NOs: 86-192) to a cell *(e.g.* a cancer cell) or population of cells. "APCM" refers to an approach of multiplexed immune gene therapy (abbreviated for Antigen Presentation CRISPR Manipulation; Antigen Presentation and Co-stimulation Manipulation; and/or Antigen Presentation, Co-stimulation and Migration) wherein a plurality of APCM genes are activited by the CRISPRa system using sgRNAs specific for these antigen presentation genes, or by overexpression with cDNA expressing ORFs, or by other gene activation / expression / delivery approaches, or by the combination(s) of above. The cell(s) are cultured *in vitro,* thereby generating a modified cell or population of cells, then injected into a non-human mammal (*e.g.* a mouse). Tumor cells are collected from the mammal and analyzed to determine which genes are depleted (*e.g.* sgRNA depletion and enrichment readout by Illumina sequencing). Specifically, sgRNAs that that are depleted by the mammal's immune system in the resulting tumors, identifying the genes that confer immune recognition of tumor cells. This novel screening approach is based on immune rejection, e.g. identifies the strongest immune factors. By using this method, one can identifiy which combination of genes should be targeted, *e.g.* those that are rejected by the immune system, rather than guessing. Genes that are depleted by the method are highly immunogenic and work the best. This method can be used for different tumor/cancer types, as each tumor/cancer type may involve a different set of genes that are immunogenic. Thus, different therapies can be designed to target different combinations of genes from different types of cancers.

In one aspect, the invention provides a method of developing a cancer therapy (e.g. a rationalized immune gene therapy). The method comprises administering a CRISPR activation (CRISPRa) system comprising a sgRNA library to a cancer cell, thereby generating a modified cell, administering the modified cell to a non-human mammal whereby the mammal develops cancer, determining the genes that are depleted in the cancer cell from the mammal, and designing a cancer therapy that targets the depleted genes (e.g immune promoting genes).

In certain embodiments, the sgRNA library comprises the nucleotide sequences set forth in SEQ ID NOs. 86-192 (Fig. 7). In certain embodiments, the sgRNA library comprises an AAV-APCM library (AAV-Apcm) designed/optimized specifically for human genes. Such gRNAs were designed to target the same set of immune genes as above, yielding a library of 219 sgRNAs (SEQ ID NOs: 193-411) (Figs. 8A-8B).

In certain embodiments, the mammal is a mouse. Other non-human mammals can be used include but are not limited to dogs, cats, and pigs.

In certain embodiments, the CRISPRa system comprises a vector comprising the nucleotide sequence set forth in SEQ ID NO: 1.

Determining the genes that are depleted in the cancer cell can be achieved by any means known to one of ordingary skill in the art, for example, nucleotide sequencing and analysis.

In certain embodiments, designing a cancer therapy that targets the depleted genes comprises packaging the open reading frames (ORFs) of the depleted genes in a vector. In certain embodiments, the vector is an adeno-associated viral (AAV) vector.

### Compositions

Certain unclaimed aspects of the present disclosure comprise vectors and compositions comprising vectors. In certain aspects, the vectors and compositons are useful for treating cancer.

In one unclaimed aspect, the present disclosure provides an off-the-shelf therapy useful for treating cancer (*e.g.* triple negative breast cancer). In one unclaimed aspects, the therapy comprises CLC4, which targets CD80, Light, CXCL10, and 4-1BBL genes. In one unclaimed aspect, the therapy comprises CLC4I, which targets CD80, Light, CXCL10, 4-1BBL, and IFNG. In one unclaimed aspect, the therapy comprises CLC4G, which targets CD80, Light, CXCL10, 4-1BBL, and GITRL. These therapies can be in the form of MAEGI (CRISPRa-based pooled activation), direct ORF-based viral immune gene therapy (AAV expressing concatenated ORFs), or other forms.

In one unclaimed aspect, the present disclosure provides a composition comprising a vector comprising an open reading frame (ORF) of at least one gene selected from the group consisting of CD80, Light, CXCL10, 4-1BBL, GITRL, IL2, IL-23, and IFNg. The disclosure should be construed to include ORFs from any number or any combination of the genes selected from the group consisting of CD80, Light, CXCL10, 4-1BBL, GITRL, IL2, IL-23, and IFNg. For example, the vector can comprise one or more ORFs from one or more of the aforementioned genes. The vector can comprise one or more ORFs from any combination of the gene selected from the group consisting of CD80, Light, CXCL10, 4-1BBL, GITRL, IL2,IL-23, and IFNg. For example, the vector can comprise ORFs from one, two, three, four, five, six, seven, or eight genes selected from the group consisting of CD80, Light, CXCL10, 4-1BBL, GITRL, IL2, IL-23, and IFNg.

In one unclaimed aspect, the present disclosure provides a composition comprising a vector comprising the open reading frames (ORFs) of CD80, Light, CXCL10, 4-1BBL and IFNG genes. In another unclaimed aspect, the disclosure provides a vector comprising the open reading frames (ORFs) of CD80, Light, CXCL10, and 4-1BBL. In certain embodiments, vector is an AAV vector. In certain unclaimed aspects, the vector comprises the nucleotide sequence set forth in SEQ ID NO: 2 or SEQ ID NO: 3.

In one unclaimed aspect, the present disclosure provides a composition comprising a vector comprising the open reading frames (ORFs) of IL-23, 4-1BBL, and IFNg genes. In one unclaimed aspect, the present disclosure invention provides a composition comprising a vector comprising the open reading frames (ORFs) of IL-23, Light, CXCL10, 4-1BBL and IFNg genes. In one unclaimed aspect, the present disclosure provides a composition comprising a vector comprising the open reading frames (ORFs) of IFNg, CD80, Light, and 4-1BBL and genes. In one unclaimed aspect, the present disclosure provides a composition comprising a vector comprising the open reading frames (ORFs) of IL-23, 4-1BBL, and IFNg genes. In one unclaimed aspect, the present disclosure invention provides a composition comprising a vector comprising the open reading frames (ORFs) of CD80, 41BBL, IL23 and IFNg genes.

In one unclaimed aspect, the present disclosure provides a composition comprising an adenoviral-based dSpCas9 CRISPRa system that utilizes a single vector to deliver all CRISPRa components. In one unclaimed aspect, the vector comprises pGW029 (SEQ ID NO: 4). In one unclaimed, the vector comprises pGW035 (SEQ ID NO: 5), In one unclaimed aspect, the vector comprises pGW063 (SEQ ID NO: 6),

In another unclaimed aspect, the present disclosure provides a composition comprising a dSaCas9-based AIO (all-in-one) CRISPRa system, which uses a single AAV vector to deliver all CRISPRa components. In one unclaimed aspect, the vector comprises pZB3 (SEQ ID NO: 10).

In another unclaimed aspect, the present disclosure provides a composition comprising an AAV based two-vector system with dSaCas9. In this system, the dSaCas9 enzyme is on a separate vector from the other CRISPRa components. In one unclaimed aspect, the vector which contains the dSaCas9 is pGW060 (SEQ ID NO: 12) and the vector that contains the other CRISPRa components is pGW047 (SEQ ID NO:11).

In another unclaimed aspect, the present disclosure provides a composition comprising an AAV based two-vector system with dSpCas9. In this system, the dSpCas9 enzyme is on a separate vector from the other CRISPRa components. In one unclaimed aspect, the vector which contains the dSpCas9 is pGW011b (SEQ ID NO: 56) and the vector that contains the other CRISPRa components is pGW045 (SEQ ID NO: 13).

In another unclaimed aspect, the present disclosure provides a composition comprising an AAV based two-vector system with dCas12/dCpf1. In certain unclaimed aspects, the composition comprises one or more vectors selected from the group consisting of pRC119 (SEQ ID NO: 14), pRC120 (SEQ ID NO: 15), pRC121b(SEQ ID NO: 16), pRC124(SEQ ID NO: 17), and pRC126 (SEQ ID NO: 18).

In one aspect, the invention includes a vector comprising SEQ ID NO: 1. In an unclaimed aspect, the invention includes a vector comprising SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, or SEQ ID NO: 56.

In certain unclaimed aspects, the present disclosure includes an AAV-ORF immune gene therapy construct. In certain unclaimed aspects, the present disclosure includes any of the contstructs/ vectors depicted in Fig. 15. In certain unclaimed aspects, the present disclosure includes a vector comprising a nucleotide sequence encoding at least one of the following components: Ori, f1Ori, AmpR promoter, AmpR, AAV-ITR, EFS core promoter, LTR promoter, EFS-LTR promoter, T2A, P2A, E2A, WPRE, short-PolyA, ORF-Cd80, ORF-Light, ORF-Cxcl10, ORF-41BBL, ORF-IFNg, ORF-Il2, ORF-Gitrl, ORF-Il23, ORF-hIFNg, ORF-hIL23, ORF-h41BBL, ORF-hLIGHT(remove-EQLI), CGKRK. In certain unclaimed aspects, the present disclosure includes a vector comprising the nucleotide sequence set forth in any one of SEQ ID NOs: 59-85.

In certain unclaimed aspects, the present disclosure includes a vector comprising a nucleotide sequence encoding at least one ORF selected from the group consisting of ORF-Light, ORF-Cxcl10, ORF-41BBL,ORF-IFNg, ORF-Il2, ORF-Gitrl, ORF-Il23, ORF-hIFNg, ORF-hIL23, ORF-h41BBL, ORF-hLIGHT, or any combination thereof. In certain unclaimed aspects, the vector comprises one, two, three, four, five, six, seven, eight, nine, ten, or eleven ORF sequences selected from the group consisting of ORF-Light, ORF-Cxcl10, ORF-41BBL,ORF-IFNg, ORF-Il2, ORF-Gitrl, ORF-Il23, ORF-hIFNg, ORF-hIL23, ORF-h41BBL, and ORF-hLIGHT. In certain unclaimed apects, the vector comprises at least one nucleotide sequence selected from the group consisting of SEQ ID NOs: 73-84. In certain unclaimed aspects, the vector comprises one, two, three, four, five, six, seven, eight, nine, ten, or eleven nucleotide sequences selected from the group consisting of SEQ ID NOs: 73-84.

Tolerable variations of any one of the vectors or component parts will be known to those of skill in the art. For example, in some unclaimed aspects the vector or component part comprises a nucleic acid sequence that has at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to any one of the nucleic acid sequence set forth in SEQ ID NO: 2-18 or 24-85.

### Methods of Treatment

Recitations of methods of treatment are to be understood as relating to the substance in question for use in a method of treatment. The present disclosure includes methods for treating or preventing cancer in a subject comprising administering to the subject a therapeutically effective amount of any of the compositions disclosed herein. This composition can be utilized as a prophylactic treatment, a therapeutic treatment, a personalized, subject-specific treatment, and/or a method of turning a 'cold' tumor into a 'hot' tumor, thus making it more susceptible to immunotherapy.

One unclaimed aspect of the disclosure includes a method of treating cancer in a subject in need thereof comprising administering to the subject a therapeutically effective amount of a composition comprising a vector comprising a CRISPRa system, wherein the CRISPRa system increases expression of at least one endogenous gene, thus treating the cancer in the subject.

The vector can be any vector that can carry the gene, including but not limited to, standard viral vectors, chimeric viral vectors, other viral vectors, bacterial vectors, yeast vectors, DNA vectors, mRNA, protein carriers, nanomaterials, or other delivery vehicles. Applicable standard viral vectors for delivery include the vectors from the following types of viruses: dsDNA viruses (*e.g.* Adenoviruses, Herpesviruses, Poxviruses), ssDNA viruses (+ strand or "sense") DNA (*e.g.* Parvoviruses), dsRNA viruses (*e.g.* Reoviruses), (+)ssRNA viruses (+ strand or sense) RNA (*e.g.* Picornaviruses, Togaviruses), (-)ssRNA viruses (- strand or antisense) RNA (e.g. Orthomyxoviruses, Rhabdoviruses), ssRNA-RT viruses (+ strand or sense) RNA with DNA intermediate in life-cycle (*e.g.* Retroviruses), and dsDNA-RT viruses DNA with RNA intermediate in life-cycle (*e.g.* Hepadnaviruses).

In certain unclaimed aspects of the disclosure, the cells are packaged into an AAV vector. Applicable AAV serotypes include, but are not limited to, AAV1, AAV2, AAV3, AAV4, AAV5, AAV6, AAV7, AAV8, AAV9, AAV10, AAV11, AAV12, artificial variants such as AAV.rhlO, AAV.rh32/33, AAV.rh43, AAV.rh64Rl, rAAV2-retro, AAV-DJ, AAV-PHP.B, AAV-PHP.S, AAV-PHP.eB, or other engineered versions of AAV. In one unclaimed aspect, the AAV vector is AAV9. In one unclaimed aspect, the CRISPRa system is cloned into an AAV vector.

The cell or cells utilized in the invention can be from any source known to one of ordinary skill in the art. The cells of the invention may be autologous, allogeneic or xenogeneic with respect to the subject undergoing treatment. In some embodiments, the cell is from a cancer cell line. In some embodiments, the cell is from the subject. In some embodiments, the cell from the subject is a cancer cell. In further embodiments, the cancer cell is from a tumor. In some embodiments, the subject is a mammal. In further unclaimed aspescts, the subject is a human.

The cells or vectors may be administered in a manner appropriate to the disease to be treated or prevented. The quantity and frequency of administration will be determined by such factors as the condition of the patient, and the type and severity of the patient's disease, although appropriate dosages may be determined by clinical trials. Cells or vectors can be administered in dosages and routes and at times to be determined in appropriate pre-clinical and clinical experimentation and trials. Cell and vector compositions may be administered multiple times at various dosages. Administration of the cells or vectors may be combined with other methods useful to treat the desired disease or condition as determined by those of skill in the art. In one embodiment, administering the therapeutically effective amount of the composition comprises a one dose, a two dose, a three dose, a four dose, or a multi-dose treatment. The administration of the modified cells or vectors may be carried out in any convenient manner known to those of skill in the art. In one unclaimed aspect, the cells are administered intratumorally.

The present disclosure includes compositions and methods for treating cancer which are not part of the claims. Types of cancer that can be treated include, but are not limited to, Acute Lymphoblastic Leukemia (ALL), Acute Myeloid Leukemia (AML), Adrenocortical Carcinoma, AIDS-Related Cancers, Kaposi Sarcoma, AIDS-Related Lymphoma, Primary CNS Lymphoma, Anal Cancer, Appendix Cancer (Gastrointestinal Carcinoid Tumors), Astrocytomas, Atypical Teratoid/Rhabdoid Tumor, Brain Cancer, Basal Cell Carcinoma of the Skin, Bile Duct Cancer, Bladder Cancer, Bone Cancer (includes Ewing Sarcoma and Osteosarcoma and Malignant Fibrous Histiocytoma), Brain Tumors, Breast Cancer, Bronchial Tumors, Burkitt Lymphoma, Non-Hodgkin Lymphoma, Carcinoid Tumors, Carcinoma of Unknown Primary, Cardiac (Heart) Tumors, Embryonal Tumors, Germ Cell Tumor, Primary CNS Lymphoma, Cervical Cancer, Cholangiocarcinoma, Chordoma, Chronic Lymphocytic Leukemia (CLL), Chronic Myelogenous Leukemia (CML), Chronic Myeloproliferative Neoplasms, Colorectal Cancer, Craniopharyngioma, Cutaneous T-Cell Lymphoma (Mycosis Fungoides and Sézary Syndrome), Ductal Carcinoma In Situ (DCIS), Endometrial Cancer, Ependymoma, Esophageal Cancer, Esthesioneuroblastoma, Ewing Sarcoma, Extracranial Germ Cell Tumor, Eye Cancer, Intraocular Melanoma, Fallopian Tube Cancer, Fibrous Histiocytoma of Bone, Osteosarcoma, Gallbladder Cancer, Gastric Cancer, Stomach Cancer, Gastrointestinal Carcinoid Tumor, Gastrointestinal Stromal Tumors (GIST), Central Nervous System Germ Cell Tumors, Extracranial Germ Cell Tumors, Extragonadal Germ Cell Tumors, Ovarian Germ Cell Tumors, Testicular Cancer, Gestational Trophoblastic Disease, Hairy Cell Leukemia, Head and Neck Cancer, Heart Tumors, Hepatocellular (Liver) Cancer, Histiocytosis (Langerhans Cell), Hodgkin Lymphoma, Hypopharyngeal Cancer, Intraocular Melanoma, Islet Cell Tumors, Pancreatic Neuroendocrine Tumors, Kidney Cancer, Renal Cell Cancer, Langerhans Cell Histiocytosis, Laryngeal Cancer, Leukemia, Lip and Oral Cavity Cancer, Liver Cancer, Lung Cancer (Non-Small Cell and Small Cell), Lymphoma, Male Breast Cancer, Malignant Fibrous Histiocytoma of Bone and Osteosarcoma, Melanoma, Intraocular (Eye) Melanoma, Merkel Cell Carcinoma (Skin Cancer), Malignant Mesothelioma, Metastatic Cancer, Metastatic Squamous Neck Cancer with Occult Primary, Midline Tract Carcinoma With NUT Gene Changes, Mouth Cancer, Multiple Endocrine Neoplasia Syndromes, Multiple Myeloma/Plasma Cell Neoplasms, Mycosis Fungoides (Lymphoma), Myelodysplastic Syndromes, Myelodysplastic/ Myeloproliferative Neoplasms, Nasal Cavity and Paranasal Sinus Cancer, Nasopharyngeal Cancer, Neuroblastoma, Non-Small Cell Lung Cancer, Oral Cancer, and Oropharyngeal Cancer, Ovarian Cancer, Pancreatic Cancer, Papillomatosis, Paraganglioma, Paranasal Sinus and Nasal Cavity Cancer, Parathyroid Cancer, Penile Cancer, Pharyngeal Cancer, Pheochromocytoma, Pituitary Tumor, Plasma Cell Neoplasm/Multiple Myeloma, Pleuropulmonary Blastoma, Primary Central Nervous System (CNS) Lymphoma, Primary Peritoneal Cancer, Prostate Cancer, Rectal Cancer Recurrent Cancer, Retinoblastoma, Rhabdomyosarcoma, Salivary Gland Cancer, Sarcoma, Vascular Tumors, Uterine Sarcoma, Sézary Syndrome (Lymphoma), Small Cell Lung Cancer, Small Intestine Cancer, Soft Tissue Sarcoma, Squamous Cell Carcinoma, Stomach (Gastric) Cancer, Throat Cancer, Thymoma, Thymic Carcinoma, Thyroid Cancer, Transitional Cell Cancer of the Renal Pelvis and Ureter, Carcinoma of Unknown Primary, Ureter and Renal Pelvis, Transitional Cell Cancer, Urethral Cancer, Uterine Cancer, Vaginal Cancer, Vulvar Cancer, Wilms Tumor, and combinations thereof.

Certain unclaimed aspects of the present disclosure further comprise administering an additional treatment to the subject. Certain unclaimed aspects of the invention include treating the subject with a combination of a composition of the present invention and an additional treatment. Examples of additional treatments include but are not limited to, chemotherapy, radiation, surgery, medication, immune checkpoint inhibitors, immune checkpoint blockade (ICB) antibodies, immune checkpoint inhibitors that block CTLA-4 or PD1, anti-CTLA4 monoclonal antibody, anti-PD1 monoclonal antibody, anti-PD-L1 monoclonal antibody, adoptive cell transfer, human recombinant cytokines, cancer vaccines, immunotherapy, targeted therapy, hormone therapy, stem cell transplant, precision medicine, non-specific immunotherapy (*e.g.* cytokines and chemokines, such as IL-2, IL-7, IL-12, IL-15, IL-18, IL-23, IFNa, IFNb, IFNg, TNFa), oncolytic virus therapy, cell therapy (*e*.*g*. adoptive transfer of TILs, TCR-T, CAR-T, CAR-NK, CAR-macrophage, or other forms of naive, patient-isolated or engineered primary cells), cancer vaccines (*e.g.* conventional DC vaccine), Ipilimumab (Yervoy), Nivolumab (Opdivo), Pembrolizumab (Keytruda), Atezolizumab (Tecentriq), Avelumab (Bavencio), Durvalumab (Imfinzi), Anti-LAG-3, anti-TIM1, Anti-TIM3, Anti-CSF-R, IDO inhibitor, OX-40 agonist, GITR agonist, CD80 agonist, CD86 agonist, ICOS agonist, ICOSLG agonist, CD276 agonist, VTCN1 agonist, TNFSF14 agonist, TNFSF9 agonist, TNFSF4 agonist, CD70 agonist, CD40 agonist, LGALS9 agonist, CD80 inhibitor, CD86 inhibitor, ICOS inhibitor, ICOSLG inhibitor, CD276 inhibitor, VTCN1 inhibitor, TNFSF14 inhibitor, TNFSF9 inhibitor, TNFSF4 inhibitor, CD70 inhibitor, CD40 inhibitor, LGALS9 inhibitor, TLR9 agonist, CD20 antibody, CD80 antibody, TIGIT antibody, B7-H1 antibody, B7-H2 antibody, B7-H3 antibody, B7-H4 antibody, CD28 antibody, CD47 antibody, anti-BTLA, anti-Galetin9, anti-IL15R, anti-GD2. In some embodiments the monoclonal antibody is fully human, humanized or chimeric.

In certain unclaimed apects, administering a composition of the present invention alters the tumor microenvironment. In certain unclaimed aspects, administering the composition augments host immune responses against established tumors.

### Introduction of Nucleic Acids

In certain embodiments an expression system is used for the introduction of gRNAs and (d)Cas9 proteins into the cells of interest. Typically employed options include but are not limited to plasmids and viral vectors such as adeno-associated virus (AAV) vector, adenoviral vector or lentivirus vector.

Methods of introducing nucleic acids into a cell include physical, biological and chemical methods. Physical methods for introducing a polynucleotide, such as RNA, into a host cell include calcium phosphate precipitation, lipofection, particle bombardment, microinjection, electroporation, and the like. RNA can be introduced into target cells using commercially available methods which include electroporation (Amaxa Nucleofector-II (Amaxa Biosystems, Cologne, Germany)), (ECM 830 (BTX) (Harvard Instruments, Boston, Mass.) or the Gene Pulser II (BioRad, Denver, Colo.), Multiporator (Eppendort, Hamburg Germany). RNA can also be introduced into cells using cationic liposome mediated transfection using lipofection, using polymer encapsulation, using peptide mediated transfection, or using biolistic particle delivery systems such as "gene guns" (see, for example, Nishikawa, et al. Hum Gene Ther., 12(8):861-70 (2001).

Biological methods for introducing a polynucleotide of interest into a host cell include the use of DNA and RNA vectors. Viral vectors have become the most widely used method for introducing genes into mammalian, e.g., human cells. Other viral vectors can include as listed above. See, for example, U.S. Pat. Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g., an artificial membrane vesicle).

Lipids suitable for use can be obtained from commercial sources. For example, dimyristyl phosphatidylcholine ("DMPC") can be obtained from Sigma, St. Louis, MO; dicetyl phosphate ("DCP") can be obtained from K & K Laboratories (Plainview, NY); cholesterol ("Choi") can be obtained from Calbiochem-Behring; dimyristyl phosphatidylglycerol ("DMPG") and other lipids may be obtained from Avanti Polar Lipids, Inc. (Birmingham, AL). Stock solutions of lipids in chloroform or chloroform/methanol can be stored at about -20°C. Chloroform is used as the only solvent since it is more readily evaporated than methanol. "Liposome" is a generic term encompassing a variety of single and multilamellar lipid vehicles formed by the generation of enclosed lipid bilayers or aggregates. Liposomes can be characterized as having vesicular structures with a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh et al., 1991 Glycobiology 5: 505-10). However, compositions that have different structures in solution than the normal vesicular structure are also encompassed. For example, the lipids may assume a micellar structure or merely exist as nonuniform aggregates of lipid molecules. Also contemplated are lipofectamine-nucleic acid complexes.

Regardless of the method used to introduce exogenous nucleic acids into a host cell or otherwise expose a cell to the inhibitor of the present invention, in order to confirm the presence of the nucleic acids in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, e.g., by immunological means (ELISAs and Western blots) or by assays described herein to identify agents falling within the scope of the invention.

Moreover, the nucleic acids may be introduced by any means, such as transducing the cells, transfecting the cells, and electroporating the cells. One nucleic acid may be introduced by one method and another nucleic acid may be introduced into the cell by a different method.

### RNA

In one embodiment, the nucleic acids introduced into the cell are RNA. In another embodiment, the RNA is mRNA that comprises in vitro transcribed RNA or synthetic RNA. The RNA is produced by in vitro transcription using a polymerase chain reaction (PCR)-generated template. DNA of interest from any source can be directly converted by PCR into a template for in vitro mRNA synthesis using appropriate primers and RNA polymerase. The source of the DNA can be, for example, genomic DNA, plasmid DNA, phage DNA, cDNA, synthetic DNA sequence or any other appropriate source of DNA.

PCR can be used to generate a template for *in vitro* transcription of mRNA which is then introduced into cells. Methods for performing PCR are well known in the art. Primers for use in PCR are designed to have regions that are substantially complementary to regions of the DNA to be used as a template for the PCR. "Substantially complementary", as used herein, refers to sequences of nucleotides where a majority or all of the bases in the primer sequence are complementary, or one or more bases are non-complementary, or mismatched. Substantially complementary sequences are able to anneal or hybridize with the intended DNA target under annealing conditions used for PCR. The primers can be designed to be substantially complementary to any portion of the DNA template. For example, the primers can be designed to amplify the portion of a gene that is normally transcribed in cells (the open reading frame), including 5' and 3' UTRs. The primers can also be designed to amplify a portion of a gene that encodes a particular domain of interest. In one embodiment, the primers are designed to amplify the coding region of a human cDNA, including all or portions of the 5' and 3' UTRs. Primers useful for PCR are generated by synthetic methods that are well known in the art. "Forward primers" are primers that contain a region of nucleotides that are substantially complementary to nucleotides on the DNA template that are upstream of the DNA sequence that is to be amplified. "Upstream" is used herein to refer to a location 5, to the DNA sequence to be amplified relative to the coding strand. "Reverse primers" are primers that contain a region of nucleotides that are substantially complementary to a double-stranded DNA template that are downstream of the DNA sequence that is to be amplified. "Downstream" is used herein to refer to a location 3' to the DNA sequence to be amplified relative to the coding strand.

Chemical structures that have the ability to promote stability and/or translation efficiency of the RNA may also be used. The RNA preferably has 5' and 3' UTRs. In one embodiment, the 5' UTR is between zero and 3000 nucleotides in length. The length of 5' and 3' UTR sequences to be added to the coding region can be altered by different methods, including, but not limited to, designing primers for PCR that anneal to different regions of the UTRs. Using this approach, one of ordinary skill in the art can modify the 5' and 3' UTR lengths required to achieve optimal translation efficiency following transfection of the transcribed RNA.

The 5' and 3' UTRs can be the naturally occurring, endogenous 5' and 3' UTRs for the gene of interest. Alternatively, UTR sequences that are not endogenous to the gene of interest can be added by incorporating the UTR sequences into the forward and reverse primers or by any other modifications of the template. The use of UTR sequences that are not endogenous to the gene of interest can be useful for modifying the stability and/or translation efficiency of the RNA. For example, it is known that AU-rich elements in 3' UTR sequences can decrease the stability of mRNA. Therefore, 3' UTRs can be selected or designed to increase the stability of the transcribed RNA based on properties of UTRs that are well known in the art.

In one embodiment, the 5' UTR can contain the Kozak sequence of the endogenous gene. Alternatively, when a 5' UTR that is not endogenous to the gene of interest is being added by PCR as described above, a consensus Kozak sequence can be redesigned by adding the 5' UTR sequence. Kozak sequences can increase the efficiency of translation of some RNA transcripts, but does not appear to be required for all RNAs to enable efficient translation. The requirement for Kozak sequences for many mRNAs is known in the art. In other embodiments the 5' UTR can be derived from an RNA virus whose RNA genome is stable in cells. In other embodiments various nucleotide analogues can be used in the 3' or 5' UTR to impede exonuclease degradation of the mRNA.

To enable synthesis of RNA from a DNA template, a promoter of transcription should be attached to the DNA template upstream of the sequence to be transcribed. When a sequence that functions as a promoter for an RNA polymerase is added to the 5' end of the forward primer, the RNA polymerase promoter becomes incorporated into the PCR product upstream of the open reading frame that is to be transcribed. In one embodiment, the promoter is a T7 polymerase promoter, as described elsewhere herein. Other useful promoters include, but are not limited to, T3 and SP6 RNA polymerase promoters. Consensus nucleotide sequences for T7, T3 and SP6 promoters are known in the art.

In one embodiment, the mRNA has a cap on the 5' end and a 3' poly(A) tail which determine ribosome binding, initiation of translation and stability mRNA in the cell. On a circular DNA template, for instance, plasmid DNA, RNA polymerase produces a long concatameric product which may not be suitable for expression in eukaryotic cells. The transcription of plasmid DNA linearized at the end of the 3' UTR results in normal sized mRNA which may not be effective in eukaryotic transfection even if it is polyadenylated after transcription.

On a linear DNA template, phage T7 RNA polymerase can extend the 3' end of the transcript beyond the last base of the template (Schenborn and Mierendorf, Nuc Acids Res., 13:6223-36 (1985); Nacheva and Berzal-Herranz, Eur. J. Biochem., 270:1485-65 (2003).

The conventional method of integration of polyA/T stretches into a DNA template is by molecular cloning. However polyA/T sequence integrated into plasmid DNA can cause plasmid instability, which is why plasmid DNA templates obtained from bacterial cells are often highly contaminated with deletions and other aberrations. This makes cloning procedures not only laborious and time consuming but often not reliable. That is why a method which allows construction of DNA templates with polyA/T 3' stretch without cloning highly desirable.

The polyA/T segment of the transcriptional DNA template can be produced during PCR by using a reverse primer containing a polyT tail, such as 100T tail (size can be 50-5000 T), or after PCR by any other method, including, but not limited to, DNA ligation or in vitro recombination. Poly(A) tails also provide stability to RNAs and reduce their degradation. Generally, the length of a poly(A) tail positively correlates with the stability of the transcribed RNA. In one embodiment, the poly(A) tail is between 100 and 5000 adenosines.

Poly(A) tails of RNAs can be further extended following *in vitro* transcription with the use of a poly(A) polymerase, such as *E. coli* polyA polymerase (E-PAP). In one embodiment, increasing the length of a poly(A) tail from 100 nucleotides to between 300 and 400 nucleotides results in about a two-fold increase in the translation efficiency of the RNA. Additionally, the attachment of different chemical groups to the 3' end can increase mRNA stability. Such attachment can contain modified/artificial nucleotides, aptamers and other compounds. For example, ATP analogs can be incorporated into the poly(A) tail using poly(A) polymerase. ATP analogs can further increase the stability of the RNA.

5' caps also provide stability to RNA molecules. In a preferred embodiment, RNAs produced by the methods disclosed herein include a 5' cap. The 5' cap is provided using techniques known in the art and described herein (Cougot, et al., Trends in Biochem. Sci., 29:436-444 (2001); Stepinski, et al., RNA, 7:1468-95 (2001); Elango, et al., Biochim. Biophys. Res. Commun., 330:958-966 (2005)).

The RNAs produced by the methods disclosed herein can also contain an internal ribosome entry site (IRES) sequence. The IRES sequence may be any viral, chromosomal or artificially designed sequence which initiates cap-independent ribosome binding to mRNA and facilitates the initiation of translation. Any solutes suitable for cell electroporation, which can contain factors facilitating cellular permeability and viability such as sugars, peptides, lipids, proteins, antioxidants, and surfactants can be included.

In some embodiments, the RNA is electroporated into the cells, such as *in vitro* transcribed RNA.

The methods also provide the ability to control the level of expression over a wide range by changing, for example, the promoter or the amount of input RNA, making it possible to individually regulate the expression level. Furthermore, the PCR-based technique of mRNA production greatly facilitates the design of the mRNAs with different structures and combination of their domains.

One advantage of RNA transfection methods of the invention is that RNA transfection is essentially transient and vector-free. A RNA transgene can be delivered to a lymphocyte and expressed therein following a brief *in vitro* cell activation, as a minimal expressing cassette without the need for any additional viral sequences. Under these conditions, integration of the transgene into the host cell genome is unlikely. Cloning of cells is not necessary because of the efficiency of transfection of the RNA and its ability to uniformly modify the entire lymphocyte population.

Genetic modification of cells with *in vitro*-transcribed RNA (IVT-RNA) makes use of two different strategies both of which have been successively tested in various animal models. Cells are transfected with *in vitro*-transcribed RNA by means of lipofection or electroporation. It is desirable to stabilize IVT-RNA using various modifications in order to achieve prolonged expression of transferred IVT-RNA.

Some IVT vectors are known in the literature which are utilized in a standardized manner as template for in vitro transcription and which have been genetically modified in such a way that stabilized RNA transcripts are produced. Currently protocols used in the art are based on a plasmid vector with the following structure: a 5' RNA polymerase promoter enabling RNA transcription, followed by a gene of interest which is flanked either 3' and/or 5' by untranslated regions (UTR), and a 3' polyadenyl cassette containing 50-70 A nucleotides. Prior to *in vitro* transcription, the circular plasmid is linearized downstream of the polyadenyl cassette by type II restriction enzymes (recognition sequence corresponds to cleavage site). The polyadenyl cassette thus corresponds to the later poly(A) sequence in the transcript. As a result of this procedure, some nucleotides remain as part of the enzyme cleavage site after linearization and extend or mask the poly(A) sequence at the 3' end. It is not clear, whether this nonphysiological overhang affects the amount of protein produced intracellularly from such a construct.

RNA has several advantages over more traditional plasmid or viral approaches. Gene expression from an RNA source does not require transcription and the protein product is produced rapidly after the transfection. Further, since the RNA has to only gain access to the cytoplasm, rather than the nucleus, and therefore typical transfection methods result in an extremely high rate of transfection. In addition, plasmid based approaches require that the promoter driving the expression of the gene of interest be active in the cells under study.

In another aspect, the RNA construct is delivered into the cells by electroporation. See, e.g., the formulations and methodology of electroporation of nucleic acid constructs into mammalian cells as taught in US 2004/0014645, US 2005/0052630A1, US 2005/0070841A1, US 2004/0059285A1, US 2004/0092907A1. The various parameters including electric field strength required for electroporation of any known cell type are generally known in the relevant research literature as well as numerous patents and applications in the field. See e.g., U.S. Pat. No. 6,678,556, U.S. Pat. No. 7,171,264, and U.S. Pat. No. 7,173,116. Apparatus for therapeutic application of electroporation are available commercially, e.g., the MedPulser^{™} DNA Electroporation Therapy System (Inovio/Genetronics, San Diego, Calif.), and are described in patents such as U.S. Pat. No. 6,567,694; U.S. Pat. No. 6,516,223, U.S. Pat. No. 5,993,434, U.S. Pat. No. 6,181,964, U.S. Pat. No. 6,241,701, and U.S. Pat. No. 6,233,482; electroporation may also be used for transfection of cells in vitro as described e.g. in US20070128708A1. Electroporation may also be utilized to deliver nucleic acids into cells in vitro. Accordingly, electroporation-mediated administration into cells of nucleic acids including expression constructs utilizing any of the many available devices and electroporation systems known to those of skill in the art presents an exciting new means for delivering an RNA of interest to a target cell.

### Sources of Cells

In one embodiment, cells are obtained from a subject. Non-limiting examples of subjects include humans, dogs, cats, mice, rats, pigs and transgenic species thereof. Preferably, the subject is a human. Cells can be obtained from a number of sources, including peripheral blood mononuclear cells, bone marrow, lymph node tissue, spleen tissue, umbilical cord, cancer cells and tumors. In certain embodiments, any number of cell lines available in the art, may be used. In certain embodiments, cells can be obtained from a unit of blood collected from a subject using any number of techniques known to the skilled artisan, such as Ficoll separation. In one embodiment, cells from the circulating blood of an individual are obtained by apheresis or leukapheresis. The apheresis product typically contains lymphocytes, including T cells, monocytes, granulocytes, B cells, other nucleated white blood cells, red blood cells, and platelets. The cells collected by apheresis may be washed to remove the plasma fraction and to place the cells in an appropriate buffer or media, such as phosphate buffered saline (PBS) or wash solution lacks calcium and may lack magnesium or may lack many if not all divalent cations, for subsequent processing steps. After washing, the cells may be resuspended in a variety of biocompatible buffers, such as, for example, Ca-free, Mg-free PBS. Alternatively, the undesirable components of the apheresis sample may be removed and the cells directly resuspended in culture media.

In another embodiment, cells are isolated from peripheral blood. Alternatively, cells can be isolated from umbilical cord. In any event, a specific subpopulation of cells can be further isolated by positive or negative selection techniques.

Cells can also be frozen. While many freezing solutions and parameters are known in the art and will be useful in this context, in a non-limiting example, one method involves using PBS containing 20% DMSO and 8% human serum albumin, or other suitable cell freezing media. The cells are then frozen to -80°C at a rate of 1° per minute and stored in the vapor phase of a liquid nitrogen storage tank. Other methods of controlled freezing may be used as well as uncontrolled freezing immediately at -20°C or in liquid nitrogen.

### Pharmaceutical compositions

Pharmaceutical compositions of the present disclosure may comprise the vector or modified cell as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions are preferably formulated for intravenous administration.

Pharmaceutical compositions may be administered in a manner appropriate to the disease to be treated (or prevented). The quantity and frequency of administration will be determined by such factors as the condition of the patient, and the type and severity of the patient's disease, although appropriate dosages may be determined by clinical trials.

It can generally be stated that a pharmaceutical composition comprising the modified cells described herein may be administered at a dosage of 10⁴ to 10⁹ cells/kg body weight, in some instances 10⁵ to 10⁶ cells/kg body weight, including all integer values within those ranges. Compositions may also be administered multiple times at these dosages. The cells or vectors can be administered by using infusion techniques that are commonly known in immunotherapy (see, e.g., Rosenberg et al., New Eng. J. of Med. 319:1676, 1988). The optimal dosage and treatment regime for a particular patient can readily be determined by one skilled in the art of medicine by monitoring the patient for signs of disease and adjusting the treatment accordingly.

The administration of the modified cells or vectors may be carried out in any convenient manner known to those of skill in the art. The cells or vectors may be administered to a subject by aerosol inhalation, injection, ingestion, transfusion, implantation or transplantation. The compositions described herein may be administered to a patient transarterially, subcutaneously, intradermally, intratumorally, intranodally, intramedullarly, intracystically intramuscularly, by intravenous (i.v.) injection, parenterally or intraperitoneally. In other instances, the cells are injected directly into a site of inflammation in the subject, a local disease site in the subject, a lymph node, an organ, a tumor, and the like.

It should be understood that the method and compositions that would be useful in the present invention are not limited to the particular formulations set forth in the examples. The following examples are put forth so as to provide those of ordinary skill in the art with a complete disclosure and description of how to make and use the cells, expansion and culture methods, and therapeutic methods of the invention.

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, Sambrook et al. (2012) Molecular Cloning: A Laboratory Manual, fourth edition. Cold Spring Harbor NY, Cold Spring Harbor Lab Press; Freshney, R.I. (2010) Culture of Animal Cells: A Manual of Basic Technique and Specialized Applications, sixth edition. John Wiley & Sons; Ausubel et al. (2002) Short Protocols in Molecular Biology. John Wiley & Sons; Coligan et al. (2002) Current Protocols in Immunology. John Wiley & Sons. These techniques are applicable to the production of the polynucleotides and polypeptides of the invention, and, as such, may be considered in making and practicing the invention. Particularly useful techniques for particular embodiments will be discussed in the sections that follow.

### EXPERIMENTAL EXAMPLES

The invention is now described with reference to the following Examples. These Examples are provided for the purpose of illustration only, and the invention is not limited to these Examples, but rather encompasses all variations that are evident as a result of the teachings provided herein.

The materials and methods employed in these experiments are now described.

*APCM sgRNA library:* The APCM library was designed by first selecting a small set of genes that elicit immune responses: B2M, B7-H2 (ICOSL), Calnexin, Calreticulin, CCL5, CD30L(TNFSF8), CD40L, CD70, CD80, CD83, CD86, CXCL10, CXCL3, CXCL9, Cystatin B, Cystatin C (x), ERAP1, ERp57(PDIA3), Flt3L, GITRL(TNFSF18), IFNa4, IFNb1, IFNg, IL-2, LIGHT (TNFRSF14), NLRC5/CITA, OX40L/TNFSF4, Sec61a1, Sec61b, Sec61g, TAP1, TAP2, Tapasin, TAPBPR, TL1A (TNFSF15), and TNFSF9 (4-1BBL). SgRNA sequences were designed to target these genes in mice, yielding a library of 107 sgRNAs (SEQ ID NOs. 86-192) (Fig. 7). The sgRNAs were cloned into a viral vector and packaged.

***CLC4 (CD80-Light-CXCL10-4-1BBL), CLC4G (CD80-Light-CXCL10-4-1BBL-GITRL) and CLC4I (CD80-Light-CXCL10-4-1BBL-IFNG):*** AAV-ORF-CLC4 was generated by concatenating ORFs of the CD80, Light, CXCL10, and 4-1BBL genes and expressing in an AAV vector (SEQ ID NO: 2). AAV-ORF-CLC4I was generated by concatenating ORFs of the CD80, Light, CXCL10, 4-1BBL, and IFNG genes expressing in an AAV vector (SEQ ID NO: 3). AAV-o-MAEGI-CLC4G was generated by cloning sgRNAs targeting the 5 genes into the AAV-CRISPRa vector (pGW059), and packaging into an AAV virus as a small pool: CD80-sg2 (NM_009855): TCCAGGCCTGTTCTGA GCAC (SEQ ID NO: 19), LIGHT (TNFRSF14)-sg2 (NM_019418): GAGGAGGTACGT GAGGAAAG (SEQ ID NO: 20), CXCL10-sg3 (NM_021274): GCAATGCCCTCGGTT TACAG (SEQ ID NO: 21), TNFSF9 (4-1BBL)-sg2 (NM_009404): ACAGGGCCTGG ACAGGGAAG (SEQ ID NO: 22), GITRL(TNFSF18)-sg1 (NM_183391): AGTGCTTAGCAGTGTTCCAA (SEQ ID NO: 23).

**Table 1: Vectors used in the invention**

| **Vector name** | **Function** | **SEQ ID NO:** |
|---|---|---|
| pGW059 | Lenti CRISPRa screen vector | 1 |
| pGW066 | AAV-ORF-CLC4 | 2 |
| pGW067 | AAV-ORF-CLC4I | 3 |
| pGW029 | Adenovirus one vector dSpCas9 | 4 |
| pGW035 | Adenovirus one vector dSpCas9 | 5 |
| pGW063 | Adenovirus one vector dSpCas9 | 6 |
| pAdEasy-1 | Adenovirus packaging or helper | 7 |
| pAD_Track8509 | Adenovirus packaging or helper | 8 |
| pAdTrack-CMV Sequences_190476 | Adenovirus packaging or helper | 9 |
| pZB3 | AAV dSaCas9 one vector | 10 |
| pGW047 | AAV dSaCas9 two vector | 11 |
| pGW060 | AAV dSaCas9 two vector | 12 |
| pGW045 | AAV dSpCas9 two vector | 13 |
| pRC119 | AAV two vector dCas12a/dCpf1 | 14 |
| pRC120 | AAV two vector dCas12a/dCpf1 | 15 |
| pRC121b | AAV two vector dCas12a/dCpf1 | 16 |
| pRC124 | AAV two vector dCas12a/dCpf1 | 17 |
| pRC126 | AAV two vector dCas12a/dCpf1 | 18 |
| pGW071 | AAV vector expressing Cd80-T2A-light-P2A-cxcl10-E2A-41bb-F2A-Il2 | 24 |
| pGW072 | AAV vector expressing IFNg | 25 |
| pGW073 | AAV vector expressing Cd80-T2A-light-P2A-cxcl10-E2A-41bb-F2A-Gitrl | 26 |
| pGW078 | AAV vector (base) | 27 |
| pGW089 | AAV vector expressing Il2 | 57 |
| pGW090 | AAV vector expressing Cd80-T2A-light-P2A-IL2-E2A-41bb-F2A-Ifng | 28 |
| pGW091 | AAV vector expressing Cd80-T2A-light-P2A-GITRL-E2A-41bb-F2A-Ifng | 29 |
| pGW094 | AAV vector expressing Ifng-P2A-Cd80-T2A-light-P2A-Gitrl-E2A-41BBL | 58 |
| pGW096 | AAV vector expressing Ifng-P2A-Cd80-T2A-light-P2AIL23-E2A-41BBL-sPA | 30 |
| pGW097 | AAV vector expressing IL23 | 31 |
| pGW098 | AAV vector expressing Ifng-P2A-Cd80-T2A-light-P2AIL23 | 32 |
| pGW099 | AAV vector expressing IFNg-P2A-Cd80-T2A-light-P2A-cxcl10 | 33 |
| pGW100 | AAV vector expressing Ifng-P2A-Cd80-T2A-light-P2A-Cxcl10-E2A-41BBL | 34 |
| pGW101 | AAV vector expressing Ifng-P2A-Cd80-T2A-light-E2A-41BBL | 35 |
| pGW102 | AAV vector expressing Cd80 | 36 |
| pGW103 | AAV vector expressing Light | 37 |
| pGW104 | AAV vector expressing Cxcl10 | 38 |
| pGW105 | AAV vector expressing 41bbL | 39 |
| pGW110 | AAV vector expressing Cd80-T2A-Light-P2A-Cxcl10-E2A-IFNg | 40 |
| pGW111 | AAV vector expressing Light-P2A-Cxcl10-E2A-IFNg | 41 |
| pGW112 | AAV vector expressing 41BBL-T2A-Light-P2A-Cxcl10-E2A-IFNg | 42 |
| pGW113 | AAV vector expressing IL23-T2A-Light-P2A-Cxcl10-E2A-IFNg | 43 |
| pGW114 | AAV vector expressing Cd80-T2A-Light-P2A-41BBL-E2A-IFNg | 44 |
| pGW115 | AAV vector expressing IL23-T2A-Light-P2A-41BBL-E2A-IFNg | 45 |
| pGW118 | AAV vector expressing Ifng-P2A-Cd80-T2A-light-E2A-41BBL | 46 |
| pGW119 | AAV vector expressing Ifng-STOP-P2A-Cd80-T2Alight-P2A-E2A-41BBL | 47 |
| pGW122 | AAV vector expressing IL23-T2A-41BBL-P2A-IFNg | 48 |
| pGW127b | AAV vector expressing hIFNg-P2A-hIL23-T2Ah41BBL | 49 |
| pGW128b | AAV vector expressing hIFNg-P2A-hIL23-T2Ah41BBL-E2A-hLight | 50 |
| pGW145 | AAV vector expressing hIFNg | 51 |
| pGW146 | AAV vector expressing hIL23 | 52 |
| pGW147 | AAV vector expressing h41BBL | 53 |
| pGW149 | AAV vector expressing hLIGHT(Remove-EQLI)-RSR | 54 |
| pGW036 | AAV vector expressing dSaCas9-VP64 | 55 |
| pGW011b | AAV vector expressing EFS-dCAS9-spA | 56 |

**pGW059 Lenti CRISPRa Screen Vector (SEQ ID NO: 1)** 
ttaatgtagtcttatgcaatactcttgtagtcttgcaacatggtaacgatgagttagcaacatgccttacaaggagagaaaaagcacc
gtgcatgccgattggtggaagtaaggtggtacgatcgtgccttattaggaaggcaacagacgggtctgacatggattggacgaa
ccactgaattgccgcattgcagagatattgtatttaagtgcctagctcgatacataaacgggtctctctggttagaccagatctgagc
ctgggagctctctggctaactagggaacccactgcttaagcctcaataaagcttgccttgagtgcttcaagtagtgtgtgcccgtct
gttgtgtgactctggtaactagagatccctcagacccttttagtcagtgtggaaaatctctagcagtggcgcccgaacagggactt
gaaagcgaaagggaaaccagaggagctctctcgacgcaggactcggcttgctgaagcgcgcacggcaagaggcgaggggc
ggcgactggtgagtacgccaaaaattttgactagcggaggctagaaggagagagatgggtgcgagagcgtcagtattaagcg
ggggagaattagatcgcgatgggaaaaaattcggttaaggccagggggaaagaaaaaatataaattaaaacatatagtatgggc
aagcagggagctagaacgattcgcagttaatcctggcctgttagaaacatcagaaggctgtagacaaatactgggacagctaca
accatcccttcagacaggatcagaagaacttagatcattatataatacagtagcaaccctctattgtgtgcatcaaaggatagagat
aaaagacaccaaggaagctttagacaagatagaggaagagcaaaacaaaagtaagaccaccgcacagcaagcggccgctga
tcttcagacctggaggaggagatatgagggacaattggagaagtgaattatataaatataaagtagtaaaaattgaaccattagga
gtagcacccaccaaggcaaagagaagagtggtgcagagagaaaaaagagcagtgggaataggagctttgttccttgggttctt
gggagcagcaggaagcactatgggcgcagcgtcaatgacgctgacggtacaggccagacaattattgtctggtatagtgcagc
 
agcagaacaatttgctgagggctattgaggcgcaacagcatctgttgcaactcacagtctggggcatcaagcagctccaggcaa
gaatcctggctgtggaaagatacctaaaggatcaacagctcctggggatttggggttgctctggaaaactcatttgcaccactgct
gtgccttggaatgctagttggagtaataaatctctggaacagatttggaatcacacgacctggatggagtgggacagagaaattaa
caattacacaagcttaatacactccttaattgaagaatcgcaaaaccagcaagaaaagaatgaacaagaattattggaattagata
aatgggcaagtttgtggaattggtttaacataacaaattggctgtggtatataaaattattcataatgatagtaggaggcttggtaggt
ttaagaatagtttttgctgtactttctatagtgaatagagttaggcagggatattcaccattatcgtttcagacccacctcccaaccccg
aggggaccctctagagatccgacgcgccatctctaggcccgcgccggccccctcgcacggacttgtgggagaagctcggcta
ctcccctgccccggttaatttgcatataatatttcctagtaactatagaggcttaatgtgcgataaaagacagataatctgttctttttaa
tactagctacattttacatgataggcttggatttctataacttcgtatagcatacattatacgaagttataaacagcacaaaaggaaact
caccctaactgtaaagtaattgtgtgttttgagactataaGtatcccttggagaaCCAcctTGTTGgGAGACGggata
cCGTCTCtgtttAagagctaAGCTGgccAACATGAGGATCACCCATGTCTGCAGggcCAG
CTtagcaagttaaaataaggctagtccgttatcaacttggccAACATGAGGATCACCCATGTCTGCAGg
gccaagtggcaccgagtcggtgcTTTTTTGGATCCaagcttggcgtaactagatcttgagacaaatggcagtattcatc
cacaattttaaaagaaaaggggggattggggggtacagtgcaggggaaagaatagtagacataatagcaacagacatacaaac
taaagaattacaaaaacaaattacaaaaattcaaaattttcgggtttattacagggacagcagagatccactttggcgccggctcga
gggggcccgggtgcaaagatggataaagttttaaacagagaggaatctttgcagctaatggaccttctaggtcttgaaaggagtg
ggaattggctccggtgcccgtcagtgggcagagcgcacatcgcccacagtccccgagaagttggggggaggggtcggcaatt
gatccggtgcctagagaaggtggcgcggggtaaactgggaaagtgatgtcgtgtactggctccgcctttttcccgagggtgggg
gagaaccgtatataagtgcagtagtcgccgtgaacgttctttttcgcaacgggtttgccgccagaacacaggtaagtgccgtgtgt
ggttcccgcgggcctggcctctttacgggttatggcccttgcgtgccttgaattacttccactggctgcagtacgtgattcttgatcc
cgagcttcgggttggaagtgggtgggagagttcgaggccttgcgcttaaggagccccttcgcctcgtgcttgagttgaggcctg
gcctgggcgctggggccgccgcgtgcgaatctggtggcaccttcgcgcctgtctcgctgctttcgataagtctctagccatttaaa
atttttgatgacctgctgcgacgctttttttctggcaagatagtcttgtaaatgcgggccaagatctgcacactggtatttcggtttttgg
ggccgcgggcggcgacggggcccgtgcgtcccagcgcacatgttcggcgaggcggggcctgcgagcgcggccaccgaga
atcggacgggggtagtctcaagctggccggcctgctctggtgcctggcctcgcgccgccgtgtatcgccccgccctgggcggc
aaggctggcccggtcggcaccagttgcgtgagcggaaagatggccgcttcccggccctgctgcagggagctcaaaatggag
gacgcggcgctcgggagagcgggcgggtgagtcacccacacaaaggaaaagggcctttccgtcctcagccgtcgcttcatgt
gactccacggagtaccgggcgccgtccaggcacctcgattagttctcgagcttttggagtacgtcgtctttaggttggggggagg
ggttttatgcgatggagtttccccacactgagtgggtggagactgaagttaggccagcttggcacttgatgtaattctccttggaatt
tgccctttttgagtttggatcttggttcattctcaagcctcagacagtggttcaaagtttttttcttccatttcaggtgtcgtgacgtacgg
ccaccatgaccgagtacaagcccacggtgcgcctcgccacccgcgacgacgtccccagggccgtacgcaccctcgccgccg
cgttcgccgactaccccgccacgcgccacaccgtcgatccggaccgccacatcgagcgggtcaccgagctgcaagaactctt
cctcacgcgcgtcgggctcgacatcggcaaggtgtgggtcgcggacgacggcgccgccgtggcggtctggaccacgccgg
agagcgtcgaagcgggggcggtgttcgccgagatcggcccgcgcatggccgagttgagcggttcccggctggccgcgcagc
aacagatggaaggcctcctggcgccgcaccggcccaaggagcccgcgtggttcctggccaccgtcggagtctcgcccgacc
accagggcaagggtctgggcagcgccgtcgtgctccccggagtggaggcggccgagcgcgccggggtgcccgccttcctg
gagacctccgcgccccgcaacctccccttctacgagcggctcggcttcaccgtcaccgccgacgtcgaggtgcccgaaggac
cgcgcacctggtgcatgacccgcaagcccggtgccGGATCCGGCGCAACAAACTTCTCTCTGCTG
AAACAAGCCGGAGATGTCGAAGAGAATCCTGGACCGatggaagacgccaaaaacataaag
aaaggcccggcgccattctatccgctggaagatggaaccgctggagagcaactgcataaggctatgaagagatacgccctggt
tcctggaacaattgcttttacagatgcacatatcgaggtggacatcacttacgctgagtacttcgaaatgtccgttcggttggcagaa
gctatgaaacgatatgggctgaatacaaatcacagaatcgtcgtatgcagtgaaaactctcttcaattctttatgccggtgttgggcg
cgttatttatcggagttgcagttgcgcccgcgaacgacatttataatgaacgtgaattgctcaacagtatgggcatttcgcagcctac
cgtggtgttcgtttccaaaaaggggttgcaaaaaattttgaacgtgcaaaaaaagctcccaatcatccaaaaaattattatcatggat
tctaaaacggattaccagggatttcagtcgatgtacacgttcgtcacatctcatctacctcccggttttaatgaatacgattttgtgcca
gagtccttcgatagggacaagacaattgcactgatcatgaactcctctggatctactggtctgcctaaaggtgtcgctctgcctcat
agaactgcctgcgtgagattctcgcatgccagagatcctatttttggcaatcaaatcattccggatactgcgattttaagtgttgttcc
attccatcacggttttggaatgtttactacactcggatatttgatatgtggatttcgagtcgtcttaatgtatagatttgaagaGgagct
gtttctgaggagccttcaggattacaagattcaaagtgcgctgctggtgccaaccctattctccttcttcgccaaaagcactctgatt
gacaaatacgatttatctaatttacacgaaattgcttctggtggcgctcccctctctaaggaagtcggggaagcggttgccaagag
 
gttccatctgccaggtatcaggcaaggatatgggctcactgagactacatcagctattctgattacacccgagggggatgataaac
cgggcgcggtcggtaaagttgttccattttttgaagcgaaggttgtggatctggataccgggaaaacgctgggcgttaatcaaag
aggcgaactgtgtgtgagaggtcctatgattatgtccggttatgtaaacaatccggaagcgaccaacgccttgattgacaaggatg
gatggctacattctggagacatagcttactgggacgaagacgaacacttcttcatcgttgaccgcctgaagtctctgattaagtaca
aaggctatcaggtggctcccgctgaattggaatccatcttgctccaacaccccaacatcttcgacgcaggtgtcgcaggtcttccc
gacgatgacgccggtgaacttcccgccgccgttgttgttttggagcacggaaagacgatgacggaaaaagagatcgtggattac
gtcgccagtcaagtaacaaccgcgaaaaagttgcgcggaggagttgtgtttgtggacgaagtaccgaaaggtcttaccggaaaa
ctcgacgcaagaaaaatcagagagatcctcataaaggccaagaagggcggaaagatcgccgtgTAAgaattcacgcgttaa
gtcgacaatcaacctctggattacaaaatttgtgaaagattgactggtattcttaactatgttgctccttttacgctatgtggatacgctg
ctttaatgcctttgtatcatgctattgcttcccgtatggctttcattttctcctccttgtataaatcctggttgctgtctctttatgaggagttg
tggcccgttgtcaggcaacgtggcgtggtgtgcactgtgtttgctgacgcaacccccactggttggggcattgccaccacctgtc
agctcctttccgggactttcgctttccccctccctattgccacggcggaactcatcgccgcctgccttgcccgctgctggacaggg
gctcggctgttgggcactgacaattccgtggtgttgtcggggaaatcatcgtcctttccttggctgctcgcctgtgttgccacctgga
ttctgcgcgggacgtccttctgctacgtcccttcggccctcaatccagcggaccttccttcccgcggcctgctgccggctctgcgg
cctcttccgcgtcttcgccttcgccctcagacgagtcggatctccctttgggccgcctccccgcgtcgactttaagaccaatgactt
acaaggcagctgtagatcttagccactttttaaaagaaaaggggggactggaagggctaattcactcccaacgaagacaagatct
gctttttgcttgtactgggtctctctggttagaccagatctgagcctgggagctctctggctaactagggaacccactgcttaagcct
caataaagcttgccttgagtgcttcaagtagtgtgtgcccgtctgttgtgtgactctggtaactagagatccctcagacccttttagtc
agtgtggaaaatctctagcagtacgtatagtagttcatgtcatcttattattcagtatttataacttgcaaagaaatgaatatcagagag
tgagaggaacttgtttattgcagcttataatggttacaaataaagcaatagcatcacaaatttcacaaataaagcatttttttcactgca
ttctagttgtggtttgtccaaactcatcaatgtatcttatcatgtctggctctagctatcccgcccctaactccgcccatcccgccccta
actccgcccagttccgcccattctccgccccatggctgactaattttttttatttatgcagaggccgaggccgcctcggcctctgag
ctattccagaagtagtgaggaggcttttttggaggcctagggacgtacccaattcgccctatagtgagtcgtattacgcgcgctca
ctggccgtcgttttacaacgtcgtgactgggaaaaccctggcgttacccaacttaatcgccttgcagcacatccccctttcgccag
ctggcgtaatagcgaagaggcccgcaccgatcgcccttcccaacagttgcgcagcctgaatggcgaatgggacgcgccctgta
gcggcgcattaagcgcggcgggtgtggtggttacgcgcagcgtgaccgctacacttgccagcgccctagcgcccgctcctttc
gctttcttcccttcctttctcgccacgttcgccggctttccccgtcaagctctaaatcgggggctccctttagggttccgatttagtgct
ttacggcacctcgaccccaaaaaacttgattagggtgatggttcacgtagtgggccatcgccctgatagacggtttttcgccctttg
acgttggagtccacgttctttaatagtggactcttgttccaaactggaacaacactcaaccctatctcggtctattcttttgatttataag
ggattttgccgatttcggcctattggttaaaaaatgagctgatttaacaaaaatttaacgcgaattttaacaaaatattaacgcttacaa
tttaggtggcacttttcggggaaatgtgcgcggaacccctatttgtttatttttctaaatacattcaaatatgtatccgctcatgagacaa
taaccctgataaatgcttcaataatattgaaaaaggaagagtatgagtattcaacatttccgtgtcgcccttattcccttttttgcggcat
tttgccttcctgtttttgctcacccagaaacgctggtgaaagtaaaagatgctgaagatcagttgggtgcacgagtgggttacatcg
aactggatctcaacagcggtaagatccttgagagttttcgccccgaagaacgttttccaatgatgagcacttttaaagttctgctatgt
ggcgcggtattatcccgtattgacgccgggcaagagcaactcggtcgccgcatacactattctcagaatgacttggttgagtactc
accagtcacagaaaagcatcttacggatggcatgacagtaagagaattatgcagtgctgccataaccatgagtgataacactgcg
gccaacttacttctgacaacgatcggaggaccgaaggagctaaccgcttttttgcacaacatgggggatcatgtaactcgccttga
tcgttgggaaccggagctgaatgaagccataccaaacgacgagcgtgacaccacgatgcctgtagcaatggcaacaacgttgc
gcaaactattaactggcgaactacttactctagcttcccggcaacaattaatagactggatggaggcggataaagttgcaggacc
acttctgcgctcggcccttccggctggctggtttattgctgataaatctggagccggtgagcgtgggtctcgcggtatcattgcagc
actggggccagatggtaagccctcccgtatcgtagttatctacacgacggggagtcaggcaactatggatgaacgaaatagaca
gatcgctgagataggtgcctcactgattaagcattggtaactgtcagaccaagtttactcatatatactttagattgatttaaaacttca
tttttaatttaaaaggatctaggtgaagatcctttttgataatctcatgaccaaaatcccttaacgtgagttttcgttccactgagcgtca
gaccccgtagaaaagatcaaaggatcttcttgagatcctttttttctgcgcgtaatctgctgcttgcaaacaaaaaaaccaccgctac
cagcggtggtttgtttgccggatcaagagctaccaactctttttccgaaggtaactggcttcagcagagcgcagataccaaatact
gttcttctagtgtagccgtagttaggccaccacttcaagaactctgtagcaccgcctacatacctcgctctgctaatcctgttaccagt
ggctgctgccagtggcgataagtcgtgtcttaccgggttggactcaagacgatagttaccggataaggcgcagcggtcgggctg
aacggggggttcgtgcacacagcccagcttggagcgaacgacctacaccgaactgagatacctacagcgtgagctatgagaa
agcgccacgcttcccgaagggagaaaggcggacaggtatccggtaagcggcagggtcggaacaggagagcgcacgaggg
agcttccagggggaaacgcctggtatctttatagtcctgtcgggtttcgccacctctgacttgagcgtcgatttttgtgatgctcgtca
 
gggggggcggagcctatggaaaaacgccagcaacgcggcctttttacggttcctggccttttgctggccttttgctcacatgttcttt
cctgcgttatcccctgattctgtggataaccgtattaccgcctttgagtgagctgataccgctcgccgcagccgaacgaccgagcg
cagcgagtcagtgagcgaggaagcggaagagcgcccaatacgcaaaccgcctctccccgcgcgttggccgattcattaatgc
agctggcacgacaggtttcccgactggaaagcgggcagtgagcgcaacgcaattaatgtgagttagctcactcattaggcaccc
caggctttacactttatgcttccggctcgtatgttgtgtggaattgtgagcggataacaatttcacacaggaaacagctatgaccatg
attacgccaagcgcgcaattaaccctcactaaagggaacaaaagctggagctgcaagc
**pGW066 AAV-ORF-CLC4 (SEQ ID NO: 2)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atggcttgcaattgtcagttgatgcaggatacaccactcctcaagtttccatgtccaaggctcattcttctctttgtgctgctgattcgtc
tttcacaagtgtcttcagatgttgatgaacaactgtccaagtcagtgaaagataaggtattgctgccttgccgttacaactctcctcat
gaagatgagtctgaagaccgaatctactggcaaaaacatgacaaagtggtgctgtctgtcattgctgggaaactaaaagtgtggc
ccgagtataagaaccggactttatatgacaacactacctactctcttatcatcctgggcctggtcctttcagaccggggcacataca
gctgtgtcgttcaaaagaaggaaagaggaacgtatgaagttaaacacttggctttagtaaagttgtccatcaaagctgacttctcta
cccccaacataactgagtctggaaacccatctgcagacactaaaaggattacctgctttgcttccgggggtttcccaaagcctcgc
ttctcttggttggaaaatggaagagaattacctggcatcaatacgacaatttcccaggatcctgaatctgaattgtacaccattagta
gccaactagatttcaatacgactcgcaaccacaccattaagtgtctcattaaatatggagatgctcacgtgtcagaggacttcacct
gggaaaaacccccagaagaccctcctgatagcaagaacacacttgtgctctttggggcaggattcggcgcagtaataacagtcg
tcgtcatcgttgtcatcatcaaatgcttctgtaagcacagaagctgtttcagaagaaatgaggcaagcagagaaacaaacaacag
ccttaccttcgggcctgaagaagcattagctgaacagaccgtcttccttcgtacgGGCAGTGGAGAGGGCAGA
GGAAGTCTGCTAACATGCGGTGACGTCGAGGAGAATCCTGGCCCAatggagagtgt
ggtacagccttcagtgtttgtggtggatggacagacggacatcccattcaggcggctggaacagaaccaccggagacggcgct
gtggcactgtccaggtcagcctggccctggtgctgctgctaAgtgctgggctggccactcagggctggtttctcctgagactgc
atcaacgtcttggagacatagtagctcatctgccagatggaggcaaaggctcctgggagaagctgatacaagatcaacgatctc
accaggccaacccagcagcacatcttacaggagccaacgccagcttgataggtattggtggacctctgttatgggagacacgac
ttggcctggccttcttgaggggcttgacgtatcatgatggggccctggtgaccatggagcccggttactactatgtgtactccaaa
gtgcagctgagcggcgtgggctgcccccaggggctggccaatggcctccccatcacccatggactatacaagcgcacatccc
gctacccgaaggagttagaactgctggtcagtcggcggtcaccctgtggccgggccaacagctcccgagtctggtgggacag
cagcttcctgggcggcgtggtacatctggaggctggggaagaggtggtggtccgcgtgcctggaaaccgcctggtcagacca
cgtgacggcaccaggtcctatttcggagctttcatggtcactagtGGATCCGGCGCAACAAACTTCTCTCT
GCTGAAACAAGCCGGAGATGTCGAAGAGAATCCTGGACCGatgaacccaagtgctgccg
tcattttctgcctcatcctgctgggtctgagtgggactcaagggatccctctcgcaaggacggtccgctgcaactgcatccatatcg
atgacgggccagtgagaatgagggccatagggaagcttgaaatcatccctgcgagcctatcctgcccacgtgttgagatcattg
ccacgatgaaaaagaatgatgagcagagatgtctgaatccggaatctaagaccatcaagaatttaatgaaagcgtttagccaaaa
aaggtctaaaagggctcctgctagcagcggtacccagtgcaccaactacgccctgctgaagctggccggcgatgtggagagc
aaccccgggcccatggaccagcacacacttgatgtggaggataccgcggatgccagacatccagcaggtacttcgtgcccctc
ggatgcggcgctcctcagagataccgggctcctcgcggacgctgcgctcctctcagatactgtgcgccccacaaatgccgcgc
tccccacggatgctgcctaccctgcggttaatgttcgggatcgcgaggccgcgtggccgcctgcactgaacttctgttcccgcca
 
cccaaagctctatggcctagtcgctttggttttgctgcttctgatcgccgcctgtgttcctatcttcacccgcaccgagcctcggcca
gcgctcacaatcaccacctcgcccaacctgggtacccgagagaataatgcagaccaggtcacccctgtttcccacattggctgc
cccaacactacacaacagggctctcctgtgttcgccaagctactggctaaaaaccaagcatcgttgtgcaatacaactctgaactg
gcacagccaagatggagctgggagctcatacctatctcaaggtctgaggtacgaagaagacaaaaaggagttggtggtagaca
gtcccgggctctactacgtatttttggaactgaagctcagtccaacattcacaaacacaggccacaaggtgcagggctgggtctct
cttgttttgcaagcaaagcctcaggtagatgactttgacaacttggccctgacagtggaactgttcccttgctccatggagaacaag
ttagtggaccgttcctggagtcaactgttgctcctgaaggctggccaccgcctcagtgtgggtctgagggcttatctgcatggagc
ccaggatgcatacagagactgggagctgtcttatcccaacaccaccagctttggactctttcttAtgaaacccgacaacccatgg
gaatgaGaattcacgcgttaagtcgacaatcaacctctggattacaaaatttgtgaaagattgactggtattcttaactatgttgctcc
ttttacgctatgtggatacgctgctttaatgcctttgtatcatgctattgcttcccgtatggctttcattttctcctccttgtataaatcctggt
tgctgtctctttatgaggagttgtggcccgttgtcaggcaacgtggcgtggtgtgcactgtgtttgctgacgcaacccccactggtt
ggggcattgccaccacctgtcagctcctttccgggactttcgctttccccctccctattgccacggcggaactcatcgccgcctgc
cttgcccgctgctggacaggggctcggctgttgggcactgacaattccgtggtgttgtcggggaaatcatcgtcctttccttggctg
ctcgcctgtgttgccacctggattctgcgcgggacgtccttctgctacgtcccttcggccctcaatccagcggaccttccttcccgc
ggcctgctgccggctctgcggcctcttccgcgtcttcgccttcgccctcagacgagtcggatctccctttgggccgcctccccgc
gtcgactttaagaccaatgacGGCCGCAGGAACCCCTAGTGATGGAGTTGGCCACTCCCTC
TCTGCGCGCTCGCTCGCTCACTGAGGCCGGGCGACCAAAGGTCGCCCGACGC
CCGGGCTTTGCCCGGGCGGCCTCAGTGAGCGAGCGAGCGCGCAGCTGCCTGC
AGGGGCGCCTGATGCGGTATTTTCTCCTTACGCATCTGTGCGGTATTTCACAC
CGCATACGTCAAAGCAACCATAGTACGCGCCCTGTAGCGGCGCATTAAGCGC
GGCGGGTGTGGTGGTTACGCGCAGCGTGACCGCTACACTTGCCAGCGCCTTA
GCGCCCGCTCCTTTCGCTTTCTTCCCTTCCTTTCTCGCCACGTTCGCCGGCTTT
CCCCGTCAAGCTCTAAATCGGGGGCTCCCTTTAGGGTTCCGATTTAGTGCTTT
ACGGCACCTCGACCCCAAAAAACTTGATTTGGGTGATGGTTCACGTAGTGGG
CCATCGCCCTGATAGACGGTTTTTCGCCCTTTGACGTTGGAGTCCACGTTCTTT
AATAGTGGACTCTTGTTCCAAACTGGAACAACACTCAACTCTATCTCGGGCTA
TTCTTTTGATTTATAAGGGATTTTGCCGATTTCGGTCTATTGGTTAAAAAATGA
GCTGATTTAACAAAAATTTAACGCGAATTTTAACAAAATATTAACGTTTACAA
TTTTATGGTGCACTCTCAGTACAATCTGCTCTGATGCCGCATAGTTAAGCCAG
CCCCGACACCCGCCAACACCCGCTGACGCGCCCTGACGGGCTTGTCTGCTCCC
GGCATCCGCTTACAGACAAGCTGTGACCGTCTCCGGGAGCTGCATGTGTCAG
AGGTTTTCACCGTCATCACCGAAACGCGCGAGACGAAAGGGCCTCGTGATAC
GCCTATTTTTATAGGTTAATGTCATGATAATAATGGTTTCTTAGACGTCAGGT
GGCACTTTTCGGGGAAATGTGCGCGGAACCCCTATTTGTTTATTTTTCTAAAT
ACATTCAAATATGTATCCGCTCATGAGACAATAACCCTGATAAATGCTTCAAT
AATATTGAAAAAGGAAGAGTATGAGTATTCAACATTTCCGTGTCGCCCTTATT
CCCTTTTTTGCGGCATTTTGCCTTCCTGTTTTTGCTCACCCAGAAACGCTGGTG
AAAGTAAAAGATGCTGAAGATCAGTTGGGTGCACGAGTGGGTTACATCGAAC
TGGATCTCAACAGCGGTAAGATCCTTGAGAGTTTTCGCCCCGAAGAACGTTTT
CCAATGATGAGCACTTTTAAAGTTCTGCTATGTGGCGCGGTATTATCCCGTAT
TGACGCCGGGCAAGAGCAACTCGGTCGCCGCATACACTATTCTCAGAATGAC
TTGGTTGAGTACTCACCAGTCACAGAAAAGCATCTTACGGATGGCATGACAG
TAAGAGAATTATGCAGTGCTGCCATAACCATGAGTGATAACACTGCGGCCAA
CTTACTTCTGACAACGATCGGAGGACCGAAGGAGCTAACCGCTTTTTTGCACA
ACATGGGGGATCATGTAACTCGCCTTGATCGTTGGGAACCGGAGCTGAATGA
AGCCATACCAAACGACGAGCGTGACACCACGATGCCTGTAGCAATGGCAACA
ACGTTGCGCAAACTATTAACTGGCGAACTACTTACTCTAGCTTCCCGGCAACA
ATTAATAGACTGGATGGAGGCGGATAAAGTTGCAGGACCACTTCTGCGCTCG
GCCCTTCCGGCTGGCTGGTTTATTGCTGATAAATCTGGAGCCGGTGAGCGTGG
AAGCCGCGGTATCATTGCAGCACTGGGGCCAGATGGTAAGCCCTCCCGTATC
 
GTAGTTATCTACACGACGGGGAGTCAGGCAACTATGGATGAACGAAATAGAC
AGATCGCTGAGATAGGTGCCTCACTGATTAAGCATTGGTAACTGTCAGACCA
AGTTTACTCATATATACTTTAGATTGATTTAAAACTTCATTTTTAATTTAAAAG
GATCTAGGTGAAGATCCTTTTTGATAATCTCATGACCAAAATCCCTTAACGTG
AGTTTTCGTTCCACTGAGCGTCAGACCCCGTAGAAAAGATCAAAGGATCTTCT
TGAGATCCTTTTTTTCTGCGCGTAATCTGCTGCTTGCAAACAAAAAAACCACC
GCTACCAGCGGTGGTTTGTTTGCCGGATCAAGAGCTACCAACTCTTTTTCCGA
AGGTAACTGGCTTCAGCAGAGCGCAGATACCAAATACTGTTCTTCTAGTGTAG
CCGTAGTTAGGCCACCACTTCAAGAACTCTGTAGCACCGCCTACATACCTCGC
TCTGCTAATCCTGTTACCAGTGGCTGCTGCCAGTGGCGATAAGTCGTGTCTTA
CCGGGTTGGACTCAAGACGATAGTTACCGGATAAGGCGCAGCGGTCGGGCTG
AACGGGGGGTTCGTGCACACAGCCCAGCTTGGAGCGAACGACCTACACCGAA
CTGAGATACCTACAGCGTGAGCTATGAGAAAGCGCCACGCTTCCCGAAGGGA
GAAAGGCGGACAGGTATCCGGTAAGCGGCAGGGTCGGAACAGGAGAGCGCA
CGAGGGAGCTTCCAGGGGGAAACGCCTGGTATCTTTATAGTCCTGTCGGGTTT
CGCCACCTCTGACTTGAGCGTCGATTTTTGTGATGCTCGTCAGGGGGGCGGAG
CCTATGGAAAAACGCCAGCAACGCGGCCTTTTTACGGTTCCTGGCCTTTTGCT
GGCCTTTTGCTCACATGT
**pGW067 AAV-ORF-CLC4I (SEQ ID NO: 3)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atggcttgcaattgtcagttgatgcaggatacaccactcctcaagtttccatgtccaaggctcattcttctctttgtgctgctgattcgtc
tttcacaagtgtcttcagatgttgatgaacaactgtccaagtcagtgaaagataaggtattgctgccttgccgttacaactctcctcat
gaagatgagtctgaagaccgaatctactggcaaaaacatgacaaagtggtgctgtctgtcattgTtgggaaactaaaagtgtggc
ccgagtataagaaccggactttatatgacaacactacctactctcttatcatcctgggcctggtcctttcagaccggggcacataca
gctgtgtcgttcaaaagaaggaaagaggaacgtatgaagttaaacacttggctttagtaaagttgtccatcaaagctgacttctcta
cccccaacataactgagtctggaaacccatctgcagacactaaaaggattacctgctttgcttccgggggtttcccaaagcctcgc
ttctcttggttggaaaatggaagagaattacctggcatcaatacgacaatttcccaggatcctgaatctgaattgtacaccattagta
gccaactagatttcaatacgactcgcaaccacaccattaagtgtctcattaaatatggagatgctcacgtgtcagaggacttcacct
gggaaaaacccccagaagaccctcctgatagcaagaacacacttgtgctctttggggcaggattcggcgcagtaataacagtcg
tcgtcatcgttgtcatcatcaaatgcttctgtaagcacagaagctgtttcagaagaaatgaggcaagcagagaaacaaacaacag
ccttaccttcgggcctgaagaagcattagctgaacagaccgtcttccttcgtacgGGCAGTGGAGAGGGCAGA
GGAAGTCTGCTAACATGCGGTGACGTCGAGGAGAATCCTGGCCCAatggagagtgt
ggtacagccttcagtgtttgtggtggatggacagacggacatcccattcaggcggctggaacagaaccaccggagacggcgct
gtggcactgtccaggtcagcctggccctggtgctgctgctaggtgctgggctggccactcagggctggtttctcctgagactgca
tcaacgtcttggagacatagtagctcatctgccagatggaggcaaaggctcctgggagaagctgatacaagatcaacgatctca
ccaggccaacccagcagcacatcttacaggagccaacgccagcttgataggtattggtggacctctgttatgggagacacgact
tggcctggccttcttgaggggcttgacgtatcatgatggggccctggtgaccatggagcccggttactactatgtgtactccaaag
 
tgcagctgagcggcgtgggctgcccccaggggctggccaatggcctccccatcacccatggactatacaagcgcacatcccg
ctacccgaaggagttagaactgctggtcagtcggcggtcaccctgtggccgggccaacagctcccgagtctggtgggacagca
gcttcctgggcggcgtggtacatctggaggctggggaagaggtggtggtccgcgtgcctggaaaccgcctggtcagaccacgt
gacggcaccaggtcctatttcggagctttcatggtcactagtGGATCCGGCGCAACAAACTTCTCTCTG
CTGAAACAAGCCGGAGATGTCGAAGAGAATCCTGGACCGatgaacccaagtgctgccgtc
attttctgcctcatcctgctgggtctgagtgggactcaagggatccctctcgcaaggacggtccgctgcaactgcatccatatcgat
gacgggccagtgagaatgagggccatagggaagcttgaaatcatccctgcgagcctatcctgcccacgtgttgagatcattgcc
acgatgaaaaagaatgatgagcagagatgtctgaatccggaatctaagaccatcaagaatttaatgaaagcgtttagccaaaaaa
ggtctaaaagggctcctgctagcagcggtacccagtgcaccaactacgccctgctgaagctggccggcgatgtggagagcaa
ccccgggcccatggaccagcacacacttgatgtggaggataccgcggatgccagacatccagcaggtacttcgtgcccctcgg
atgcggcgctcctcagagataccgggctcctcgcggacgctgcgctcctctcagatactgtgcgccccacaaatgccgcgctcc
ccacggatgctgcctaccctgcggttaatgttcgggatcgcgaggccgcgtggccgcctgcactgaacttctgttcccgccaccc
aaagctctatggcctagtcgctttggttttgctgcttctgatcgccgcctgtgttcctatcttcacccgcaccgagcctcggccagcg
ctcacaatcaccacctcgcccaacctgggtacccgagagaataatgcagaccaggtcacccctgtttcccacattggctgcccca
acactacacaacagggctctcctgtgttcgccaagctactggctaaaaaccaagcatcgttgtgcaatacaactctgaactggcac
agccaagatggagctgggagctcatacctatctcaaggtctgaggtacgaagaagacaaaaaggagttggtggtagacagtcc
cgggctctactacgtatttttggaactgaagctcagtccaacattcacaaacacaggccacaaggtgcagggctgggtctctcttg
ttttgcaagcaaagcctcaggtagatgactttgacaacttggccctgacagtggaactgttcccttgctccatggagaacaagttag
tggaccgttcctggagtcaactgttgctcctgaaggctggccaccgcctcagtgtgggtctgagggcttatctgcatggagccca
ggatgcatacagagactgggagctgtcttatcccaacaccaccagctttggactctttcttgtgaaacccgacaacccatgggaa
ggcatatgcggtaccgtgaagcagaccctgaacttcgatctgctgaagctggccggcgatgtggagagcaaccccgggcccat
gaacgctacacactgcatcttggctttgcagctcttcctcatggctgtttctggctgttactgccacggcacagtcattgaaagccta
gaaagtctgaataactattttaactcaagtggcatagatgtggaagaaaagagtctcttcttggatatctggaggaactggcaaaag
gatggtgacatgaaaatcctgcagagccagattatctctttctacctcagactctttgaagtcttgaaagacaatcaggccatcagc
aacaacataagcgtcattgaatcacacctgattactaccttcttcagcaacagcaaggcgaaaaaggatgcattcatgagtattgc
caagtttgaggtcaacaacccacaggtccagcgccaagcattcaatgagctcatccgagtggtccaccagctgttgccggaatc
cagcctcaggaagcggaaaaggagtcgctgctgaGAATTCAATAAAAGATCTTTATTTTCATTAG
ATCTGTGTGTTGGTTTTTTGTGTGCGGCCGCAGGAACCCCTAGTGATGGAGTT
GGCCACTCCCTCTCTGCGCGCTCGCTCGCTCACTGAGGCCGGGCGACCAAAG
GTCGCCCGACGCCCGGGCTTTGCCCGGGCGGCCTCAGTGAGCGAGCGAGCGC
GCAGCTGCCTGCAGGGGCGCCTGATGCGGTATTTTCTCCTTACGCATCTGTGC
GGTATTTCACACCGCATACGTCAAAGCAACCATAGTACGCGCCCTGTAGCGG
CGCATTAAGCGCGGCGGGTGTGGTGGTTACGCGCAGCGTGACCGCTACACTT
GCCAGCGCCTTAGCGCCCGCTCCTTTCGCTTTCTTCCCTTCCTTTCTCGCCACG
TTCGCCGGCTTTCCCCGTCAAGCTCTAAATCGGGGGCTCCCTTTAGGGTTCCG
ATTTAGTGCTTTACGGCACCTCGACCCCAAAAAACTTGATTTGGGTGATGGTT
CACGTAGTGGGCCATCGCCCTGATAGACGGTTTTTCGCCCTTTGACGTTGGAG
TCCACGTTCTTTAATAGTGGACTCTTGTTCCAAACTGGAACAACACTCAACTC
TATCTCGGGCTATTCTTTTGATTTATAAGGGATTTTGCCGATTTCGGTCTATTG
GTTAAAAAATGAGCTGATTTAACAAAAATTTAACGCGAATTTTAACAAAATA
TTAACGTTTACAATTTTATGGTGCACTCTCAGTACAATCTGCTCTGATGCCGC
ATAGTTAAGCCAGCCCCGACACCCGCCAACACCCGCTGACGCGCCCTGACGG
GCTTGTCTGCTCCCGGCATCCGCTTACAGACAAGCTGTGACCGTCTCCGGGAG
CTGCATGTGTCAGAGGTTTTCACCGTCATCACCGAAACGCGCGAGACGAAAG
GGCCTCGTGATACGCCTATTTTTATAGGTTAATGTCATGATAATAATGGTTTCT
TAGACGTCAGGTGGCACTTTTCGGGGAAATGTGCGCGGAACCCCTATTTGTTT
ATTTTTCTAAATACATTCAAATATGTATCCGCTCATGAGACAATAACCCTGAT
AAATGCTTCAATAATATTGAAAAAGGAAGAGTATGAGTATTCAACATTTCCG
TGTCGCCCTTATTCCCTTTTTTGCGGCATTTTGCCTTCCTGTTTTTGCTCACCCA
GAAACGCTGGTGAAAGTAAAAGATGCTGAAGATCAGTTGGGTGCACGAGTGG
 
GTTACATCGAACTGGATCTCAACAGCGGTAAGATCCTTGAGAGTTTTCGCCCC
GAAGAACGTTTTCCAATGATGAGCACTTTTAAAGTTCTGCTATGTGGCGCGGT
ATTATCCCGTATTGACGCCGGGCAAGAGCAACTCGGTCGCCGCATACACTATT
CTCAGAATGACTTGGTTGAGTACTCACCAGTCACAGAAAAGCATCTTACGGA
TGGCATGACAGTAAGAGAATTATGCAGTGCTGCCATAACCATGAGTGATAAC
ACTGCGGCCAACTTACTTCTGACAACGATCGGAGGACCGAAGGAGCTAACCG
CTTTTTTGCACAACATGGGGGATCATGTAACTCGCCTTGATCGTTGGGAACCG
GAGCTGAATGAAGCCATACCAAACGACGAGCGTGACACCACGATGCCTGTAG
CAATGGCAACAACGTTGCGCAAACTATTAACTGGCGAACTACTTACTCTAGCT
TCCCGGCAACAATTAATAGACTGGATGGAGGCGGATAAAGTTGCAGGACCAC
TTCTGCGCTCGGCCCTTCCGGCTGGCTGGTTTATTGCTGATAAATCTGGAGCC
GGTGAGCGTGGAAGCCGCGGTATCATTGCAGCACTGGGGCCAGATGGTAAGC
CCTCCCGTATCGTAGTTATCTACACGACGGGGAGTCAGGCAACTATGGATGA
ACGAAATAGACAGATCGCTGAGATAGGTGCCTCACTGATTAAGCATTGGTAA
CTGTCAGACCAAGTTTACTCATATATACTTTAGATTGATTTAAAACTTCATTTT
TAATTTAAAAGGATCTAGGTGAAGATCCTTTTTGATAATCTCATGACCAAAAT
CCCTTAACGTGAGTTTTCGTTCCACTGAGCGTCAGACCCCGTAGAAAAGATCA
AAGGATCTTCTTGAGATCCTTTTTTTCTGCGCGTAATCTGCTGCTTGCAAACAA
AAAAACCACCGCTACCAGCGGTGGTTTGTTTGCCGGATCAAGAGCTACCAAC
TCTTTTTCCGAAGGTAACTGGCTTCAGCAGAGCGCAGATACCAAATACTGTTC
TTCTAGTGTAGCCGTAGTTAGGCCACCACTTCAAGAACTCTGTAGCACCGCCT
ACATACCTCGCTCTGCTAATCCTGTTACCAGTGGCTGCTGCCAGTGGCGATAA
GTCGTGTCTTACCGGGTTGGACTCAAGACGATAGTTACCGGATAAGGCGCAG
CGGTCGGGCTGAACGGGGGGTTCGTGCACACAGCCCAGCTTGGAGCGAACGA
CCTACACCGAACTGAGATACCTACAGCGTGAGCTATGAGAAAGCGCCACGCT
TCCCGAAGGGAGAAAGGCGGACAGGTATCCGGTAAGCGGCAGGGTCGGAAC
AGGAGAGCGCACGAGGGAGCTTCCAGGGGGAAACGCCTGGTATCTTTATAGT
CCTGTCGGGTTTCGCCACCTCTGACTTGAGCGTCGATTTTTGTGATGCTCGTCA
GGGGGGCGGAGCCTATGGAAAAACGCCAGCAACGCGGCCTTTTTACGGTTCC
TGGCCTTTTGCTGGCCTTTTGCTCACATGT
**pGW029 Adenovirus One Vector dSpCas9 (SEQ ID NO: 4)** 
CTACAGGGCGCGTCCATTCGCCATTCAGGATCGAATTAATTCTTAATTAACAT
CATCAATAATATACCTTAttttggattgaagccaatatgataatgagggggtggagtttgtgacgtggcgcgggg
cgtgggaacggggcgggtgacgtagtagtgtggcggaagtgtgatgttgcaagtgtggcggaacacatgtaagcgacggatgt
ggcaaaagtgacgtttttggtgtgcgccggtgtacacaggaagtgacaattttcgcgcggttttaggcggatgttgtagtaaatttg
ggcgtaaccgagtaagatttggccattttcgcgggaaaactgaataagaggaagtgaaatctgaataattttgtgttactcatagcg
cgtaaTACTggtaccgcggccgcctcgagtCTAGAAAGAgggcctatttcccatgattccttcatatttgcatatacga
tacaaggctgttagagagataattagaattaatttgactgtaaacacaaagatattagtacaaaatacgtgacgtagaaagtaataat
ttcttgggtagtttgcagttttaaaattatgttttaaaatggactatcatatgcttaccgtaacttgaaagtatttcgatttcttggctttatat
atcttGTGGAAAGGACGAAACACCggaagagcgagctcttctgttttagagctaggccAACATGAGG
ATCACCCATGTCTGCAGggcctagcaagttaaaataaggctagtccgttatcaacttggccAACATGAGG
ATCACCCATGTCTGCAGggccaagtggcaccgagtcggtgcTTTTTTGctagcGTCAGTGGGC
AGAGCGCACATCGCCCACAGTCCCCGAGAAGTTGGGGGGAGGGGTCGGCAAT
TGAACGGGTGCCTAGAGAAGGTGGCGCGGGGTAAACTGGGAAAGTGATGTC
GTGTACTGGCTCCGCCTTTTTCCCGAGGGTGGGGGAGAACCGTATATAAGTGC
AGTAGTCGCCGTGAACGTTCTTTTTCGCAACGGGTTTGCCGCCAGAACACAGC
TGAAGCTTCGAGGGGCTCGCATCTCTCCTTCACGCGCCCGCCGCCCTACCTGA
GGCCGCCATCCACGCCGGTTGAGTCGCGTTCTGCCGCCTCCCGCCTGTGGTGC
CTCCTGAACTGCGTCCGCCGTCTAGGTAAGTTTAAAGCTCAGGTCGAGACCGG
 
GCCTTTGTCCGGCGCTCCCTTGGAGCCTACCTAGACTCAGCCGGCTCTCCACG
CTTTGCCTGACCCTGCTTGCTCAACTCTACGTCTTTGTTTCGTTTTCTGTTCTGC
GCCGTTACAGATCCAAGctcGTACGgccaccATGAAAAGGCCGGCGGCCACGAAA
AAGGCCGGCCAGGCAAAAAAGAAAAAGGACAAGAAGTACAGCATCGGCCTG
GCCATCGGCACCAACTCTGTGGGCTGGGCCGTGATCACCGACGAGTACAAGG
TGCCCAGCAAGAAATTCAAGGTGCTGGGCAACACCGACCGGCACAGCATCAA
GAAGAACCTGATCGGAGCCCTGCTGTTCGACAGCGGCGAAACAGCCGAGGCC
ACCCGGCTGAAGAGAACCGCCAGAAGAAGATACACCAGACGGAAGAACCGG
ATCTGCTATCTGCAAGAGATCTTCAGCAACGAGATGGCCAAGGTGGACGACA
GCTTCTTCCACAGACTGGAAGAGTCCTTCCTGGTGGAAGAGGATAAGAAGCA
CGAGCGGCACCCCATCTTCGGCAACATCGTGGACGAGGTGGCCTACCACGAG
AAGTACCCCACCATCTACCACCTGAGAAAGAAACTGGTGGACAGCACCGACA
AGGCCGACCTGCGGCTGATCTATCTGGCCCTGGCCCACATGATCAAGTTCCGG
GGCCACTTCCTGATCGAGGGCGACCTGAACCCCGACAACAGCGACGTGGACA
AGCTGTTCATCCAGCTGGTGCAGACCTACAACCAGCTGTTCGAGGAAAACCC
CATCAACGCCAGCGGCGTGGACGCCAAGGCCATCCTGTCTGCCAGACTGAGC
AAGAGCAGACGGCTGGAAAATCTGATCGCCCAGCTGCCCGGCGAGAAGAAG
AATGGCCTGTTCGGCAACCTGATTGCCCTGAGCCTGGGCCTGACCCCCAACTT
CAAGAGCAACTTCGACCTGGCCGAGGATGCCAAACTGCAGCTGAGCAAGGAC
ACCTACGACGACGACCTGGACAACCTGCTGGCCCAGATCGGCGACCAGTACG
CCGACCTGTTTCTGGCCGCCAAGAACCTGTCCGACGCCATCCTGCTGAGCGAC
ATCCTGAGAGTGAACACCGAGATCACCAAGGCCCCCCTGAGCGCCTCTATGA
TCAAGAGATACGACGAGCACCACCAGGACCTGACCCTGCTGAAAGCTCTCGT
GCGGCAGCAGCTGCCTGAGAAGTACAAAGAGATTTTCTTCGACCAGAGCAAG
AACGGCTACGCCGGCTACATTGACGGCGGAGCCAGCCAGGAAGAGTTCTACA
AGTTCATCAAGCCCATCCTGGAAAAGATGGACGGCACCGAGGAACTGCTCGT
GAAGCTGAACAGAGAGGACCTGCTGCGGAAGCAGCGGACCTTCGACAACGG
CAGCATCCCCCACCAGATCCACCTGGGAGAGCTGCACGCCATTCTGCGGCGG
CAGGAAGATTTTTACCCATTCCTGAAGGACAACCGGGAAAAGATCGAGAAGA
TCCTGACCTTCCGCATCCCCTACTACGTGGGCCCTCTGGCCAGGGGAAACAGC
AGATTCGCCTGGATGACCAGAAAGAGCGAGGAAACCATCACCCCCTGGAACT
TCGAGGAAGTGGTGGACAAGGGCGCTTCCGCCCAGAGCTTCATCGAGCGGAT
GACCAACTTCGATAAGAACCTGCCCAACGAGAAGGTGCTGCCCAAGCACAGC
CTGCTGTACGAGTACTTCACCGTGTATAACGAGCTGACCAAAGTGAAATACG
TGACCGAGGGAATGAGAAAGCCCGCCTTCCTGAGCGGCGAGCAGAAAAAGG
CCATCGTGGACCTGCTGTTCAAGACCAACCGGAAAGTGACCGTGAAGCAGCT
GAAAGAGGACTACTTCAAGAAAATCGAGTGCTTCGACTCCGTGGAAATCTCC
GGCGTGGAAGATCGGTTCAACGCCTCCCTGGGCACATACCACGATCTGCTGA
AAATTATCAAGGACAAGGACTTCCTGGACAATGAGGAAAACGAGGACATTCT
GGAAGATATCGTGCTGACCCTGACACTGTTTGAGGACAGAGAGATGATCGAG
GAACGGCTGAAAACCTATGCCCACCTGTTCGACGACAAAGTGATGAAGCAGC
TGAAGCGGCGGAGATACACCGGCTGGGGCAGGCTGAGCCGGAAGCTGATCA
ACGGCATCCGGGACAAGCAGTCCGGCAAGACAATCCTGGATTTCCTGAAGTC
CGACGGCTTCGCCAACAGAAACTTCATGCAGCTGATCCACGACGACAGCCTG
ACCTTTAAAGAGGACATCCAGAAAGCCCAGGTGTCCGGCCAGGGCGATAGCC
TGCACGAGCACATTGCCAATCTGGCCGGCAGCCCCGCCATTAAGAAGGGCAT
CCTGCAGACAGTGAAGGTGGTGGACGAGCTCGTGAAAGTGATGGGCCGGCAC
AAGCCCGAGAACATCGTGATCGAAATGGCCAGAGAGAACCAGACCACCCAG
AAGGGACAGAAGAACAGCCGCGAGAGAATGAAGCGGATCGAAGAGGGCATC
AAAGAGCTGGGCAGCCAGATCCTGAAAGAACACCCCGTGGAAAACACCCAG
 
CTGCAGAACGAGAAGCTGTACCTGTACTACCTGCAGAATGGGCGGGATATGT
ACGTGGACCAGGAACTGGACATCAACCGGCTGTCCGACTACGATGTGGACCA
CATCGTGCCTCAGAGCTTTCTGAAGGACGACTCCATCGACAACAAGGTGCTG
ACCAGAAGCGACAAGGCCCGGGGCAAGAGCGACAACGTGCCCTCCGAAGAG
GTCGTGAAGAAGATGAAGAACTACTGGCGGCAGCTGCTGAACGCCAAGCTGA
TTACCCAGAGAAAGTTCGACAATCTGACCAAGGCCGAGAGAGGCGGCCTGAG
CGAACTGGATAAGGCCGGCTTCATCAAGAGACAGCTGGTGGAAACCCGGCAG
ATCACAAAGCACGTGGCACAGATCCTGGACTCCCGGATGAACACTAAGTACG
ACGAGAATGACAAGCTGATCCGGGAAGTGAAAGTGATCACCCTGAAGTCCAA
GCTGGTGTCCGATTTCCGGAAGGATTTCCAGTTTTACAAAGTGCGCGAGATCA
ACAACTACCACCACGCCCACGACGCCTACCTGAACGCCGTCGTGGGAACCGC
CCTGATCAAAAAGTACCCTAAGCTGGAAAGCGAGTTCGTGTACGGCGACTAC
AAGGTGTACGACGTGCGGAAGATGATCGCCAAGAGCGAGCAGGAAATCGGC
AAGGCTACCGCCAAGTACTTCTTCTACAGCAACATCATGAACTTTTTCAAGAC
CGAGATTACCCTGGCCAACGGCGAGATCCGGAAGCGGCCTCTGATCGAGACA
AACGGCGAAACCGGGGAGATCGTGTGGGATAAGGGCCGGGATTTTGCCACCG
TGCGGAAAGTGCTGAGCATGCCCCAAGTGAATATCGTGAAAAAGACCGAGGT
GCAGACAGGCGGCTTCAGCAAAGAGTCTATCCTGCCCAAGAGGAACAGCGAT
AAGCTGATCGCCAGAAAGAAGGACTGGGACCCTAAGAAGTACGGCGGCTTCG
ACAGCCCCACCGTGGCCTATTCTGTGCTGGTGGTGGCCAAAGTGGAAAAGGG
CAAGTCCAAGAAACTGAAGAGTGTGAAAGAGCTGCTGGGGATCACCATCATG
GAAAGAAGCAGCTTCGAGAAGAATCCCATCGACTTTCTGGAAGCCAAGGGCT
ACAAAGAAGTGAAAAAGGACCTGATCATCAAGCTGCCTAAGTACTCCCTGTT
CGAGCTGGAAAACGGCCGGAAGAGAATGCTGGCCTCTGCCGGCGAACTGCAG
AAGGGAAACGAACTGGCCCTGCCCTCCAAATATGTGAACTTCCTGTACCTGG
CCAGCCACTATGAGAAGCTGAAGGGCTCCCCCGAGGATAATGAGCAGAAACA
GCTGTTTGTGGAACAGCACAAGCACTACCTGGACGAGATCATCGAGCAGATC
AGCGAGTTCTCCAAGAGAGTGATCCTGGCCGACGCTAATCTGGACAAAGTGC
TGTCCGCCTACAACAAGCACCGGGATAAGCCCATCAGAGAGCAGGCCGAGA
ATATCATCCACCTGTTTACCCTGACCAATCTGGGAGCCCCTGCCGCCTTCAAG
TACTTTGACACCACCATCGACCGGAAGAGGTACACCAGCACCAAAGAGGTGC
TGGACGCCACCCTGATCCACCAGAGCATCACCGGCCTGTACGAGACACGGAT
CGACCTGTCTCAGCTGGGAGGCGACagcgctGGAggacctaagaaaaagaggaaggtgggatccG
GACGGGCTGACGCATTGGACGATTTTGATCTGGATATGCTGGGAAGTGACGC
CCTCGATGATTTTGACCTTGACATGCTTGGTTCGGATGCCCTTGATGACTTTGA
CCTCGACATGCTCGGCAGTGACGCCCTTGATGATTTCGACCTGGACATGCTGA
TTAACtgtacaggaagcggagccactaacttctccctgttgaaacaagcaggggatgtcgaagagaatcccgggccaGC
CACCATGGCTTCAAACTTTACTCAGTTCGTGCTCGTGGACAATGGTGGGACAG
GGGATGTGACAGTGGCTCCTTCTAATTTCGCTAATGGGGTGGCAGAGTGGATC
AGCTCCAACTCACGGAGCCAGGCCTACAAGGTGACATGCAGCGTCAGGCAGT
CTAGTGCCCAGAAGAGAAAGTATACCATCAAGGTGGAGGTCCCCAAAGTGGC
TACCCAGACAGTGGGCGGAGTCGAACTGCCTGTCGCCGCTTGGAGGTCCTAC
CTGAACATGGAGCTCACTATCCCAATTTTCGCTACCAATTCTGACTGTGAACT
CATCGTGAAGGCAATGCAGGGGCTCCTCAAAGACGGTAATCCTATCCCTTCC
GCCATCGCCGCTAACTCAGGTATCTACAGCGCTGGAGGAGGTGGAAGCGGAG
GAGGAGGAAGCGGAGGAGGAGGTAGCGGACCTAAGAAAAAGAGGAAGGTG
GCGGCCGCTGGATCCCCTTCAGGGCAGATCAGCAACCAGGCCCTGGCTCTGG
CCCCTAGCTCCGCTCCAGTGCTGGCCCAGACTATGGTGCCCTCTAGTGCTATG
GTGCCTCTGGCCCAGCCACCTGCTCCAGCCCCTGTGCTGACCCCAGGACCACC
CCAGTCACTGAGCGCTCCAGTGCCCAAGTCTACACAGGCCGGCGAGGGGACT
 
CTGAGTGAAGCTCTGCTGCACCTGCAGTTCGACGCTGATGAGGACCTGGGAG
CTCTGCTGGGGAACAGCACCGATCCCGGAGTGTTCACAGATCTGGCCTCCGTG
GACAACTCTGAGTTTCAGCAGCTGCTGAATCAGGGCGTGTCCATGTCTCATAG
TACAGCCGAACCAATGCTGATGGAGTACCCCGAAGCCATTACCCGGCTGGTG
ACCGGCAGCCAGCGGCCCCCCGACCCCGCTCCAACTCCCCTGGGAACCAGCG
GCCTGCCTAATGGGCTGTCCGGAGATGAAGACTTCTCAAGCATCGCTGATATG
GACTTTAGTGCCCTGCTGTCACAGATTTCCTCTAGTGGGCAGGGAGGAGGTGG
AAGCGGCTTCAGCGTGGACACCAGTGCCCTGCTGGACCTGTTCAGCCCCTCG
GTGACCGTGCCCGACATGAGCCTGCCTGACCTTGACAGCAGCCTGGCCAGTA
TCCAAGAGCTCCTGTCTCCCCAGGAGCCCCCCAGGCCTCCCGAGGCAGAGAA
CAGCAGCCCGGATTCAGGGAAGCAGCTGGTGCACTACACAGCGCAGCCGCTG
TTCCTGCTGGACCCCGGCTCCGTGGACACCGGGAGCAACGACCTGCCGGTGC
TGTTTGAGCTGGGAGAGGGCTCCTACTTCTCCGAAGGGGACGGCTTCGCCGA
GGACCCCACCATCTCCCTGCTGACAGGCTCGGAGCCTCCCAAAGCCAAGGAC
CCCACTGTCTCCTAAGAATTCgatatcaagcttAATAAAAGATCTTTATTTTCATTAG
ATCTGTGTGTTGGTTTTTTGTGTagatctGGGCGTGGTtaagggtgggaaagaatatataaggtgg
gggtcttatgtagttttgtatctgttttgcagcagccgccgccgccatgagcaccaactcgtttgatggaagcattgtgagctcatatt
tgacaacgcgcatgcccccatgggccggggtgcgtcagaatgtgatgggctccagcattgatggtcgccccgtcctgcccgca
aactctactaccttgacctacgagaccgtgtctggaacgccgttggagactgcagcctccgccgccgcttcagccgctgcagcc
accgcccgcgggattgtgactgactttgctttcctgagcccgcttgcaagcagtgcagcttcccgttcatccgcccgcgatgacaa
gttgacggctcttttggcacaattggattctttgacccgggaacttaatgtcgtttctcagcagctgttggatctgcgccagcaggttt
ctgccctgaaggcttcctcccctcccaatgcggtttaaaacataaataaaaaaccagactctgtttggatttggatcaagcaagtgt
cttgctgtctttatttaggggttttgcgcgcgcggtaggcccgggaccagcggtctcggtcgttgagggtcctgtgtattttttccag
gacgtggtaaaggtgactctggatgttcagatacatgggcataagcccgtctctggggtggaggtagcaccactgcagagcttc
atgctgcggggtggtgttgtagatgatccagtcgtagcaggagcgctgggcgtggtgcctaaaaatgtctttcagtagcaagctg
attgccaggggcaggcccttggtgtaagtgtttacaaagcggttaagctgggatgggtgcatacgtggggatatgagatgcatctt
ggactgtatttttaggttggctatgttcccagccatatccctccggggattcatgttgtgcagaaccaccagcacagtgtatccggtg
cacttgggaaatttgtcatgtagcttagaaggaaatgcgtggaagaacttggagacgcccttgtgacctccaagattttccatgcat
tcgtccataatgatggcaatgggcccacgggcggcggcctgggcgaagatatttctgggatcactaacgtcatagttgtgttcca
ggatgagatcgtcataggccatttttacaaagcgcgggcggagggtgccagactgcggtataatggttccatccggcccaggg
gcgtagttaccctcacagatttgcatttcccacgctttgagttcagatggggggatcatgtctacctgcggggcgatgaagaaaac
ggtttccggggtaggggagatcagctgggaagaaagcaggttcctgagcagctgcgacttaccgcagccggtgggcccgtaa
atcacacctattaccgggtgcaactggtagttaagagagctgcagctgccgtcatccctgagcaggggggccacttcgttaagca
tgtccctgactcgcatgttttccctgaccaaatccgccagaaggcgctcgccgcccagcgatagcagttcttgcaaggaagcaaa
gtttttcaacggtttgagaccgtccgccgtaggcatgcttttgagcgtttgaccaagcagttccaggcggtcccacagctcggtca
cctgctctacggcatctcgatccagcatatctcctcgtttcgcgggttggggcggctttcgctgtacggcagtagtcggtgctcgtc
cagacgggccagggtcatgtctttccacgggcgcagggtcctcgtcagcgtagtctgggtcacggtgaaggggtgcgctccgg
gctgcgcgctggccagggtgcgcttgaggctggtcctgctggtgctgaagcgctgccggtcttcgccctgcgcgtcggccagg
tagcatttgaccatggtgtcatagtccagcccctccgcggcgtggcccttggcgcgcagcttgcccttggaggaggcgccgcac
gaggggcagtgcagacttttgagggcgtagagcttgggcgcgagaaataccgattccggggagtaggcatccgcgccgcag
gccccgcagacggtctcgcattccacgagccaggtgagctctggccgttcggggtcaaaaaccaggtttcccccatgctttttga
tgcgtttcttacctctggtttccatgagccggtgtccacgctcggtgacgaaaaggctgtccgtgtccccgtatacagactnnngtt
taaacgaattcnnntataaaatgcaaggtgctgctcaaaaaatcaggcaaagcctcgcgcaaaaaagaaagcacatcgtagtca
tgctcatgcagataaaggcaggtaagctccggaaccaccacagaaaaagacaccatttttctctcaaacatgtctgcgggtttctg
cataaacacaaaataaaataacaaaaaaacatttaaacattagaagcctgtcttacaacaggaaaaacaacccttataagcataag
acggactacggccatgccggcgtgaccgtaaaaaaactggtcaccgtgattaaaaagcaccaccgacagctcctcggtcatgtc
cggagtcataatgtaagactcggtaaacacatcaggttgattcatcggtcagtgctaaaaagcgaccgaaatagcccgggggaa
tacatacccgcaggcgtagagacaacattacagcccccataggaggtataacaaaattaataggagagaaaaacacataaaca
cctgaaaaaccctcctgcctaggcaaaatagcaccctcccgctccagaacaacatacagcgcttcacagcggcagcctaacagt
cagccttaccagtaaaaaagaaaacctattaaaaaaacaccactcgacacggcaccagctcaatcagtcacagtgtaaaaaagg
 
gccaagtgcagagcgagtatatataggactaaaaaatgacgtaacggttaaagtccacaaaaaacacccagaaaaccgcacgc
gaacctacgcccagaaacgaaagccaaaaaacccacaacttcctcaaatcgtcacttccgttttcccacgttacgtaacttcccatt
ttaagaaaactacaattcccaacacatacaagttactccgccctaaaacctacgtcacccgccccgttcccacgccccgcgccac
gtcacaaactccaccccctcattatcatattggcttcaatccaaaataaggtatattattgatgattaattaattaaggatcccggtgtg
aaataccgcacagatgcgtaaggagaaaataccgcatcaggcgctcttacgcttcctcgctcactgactcgctgcgctcggtcgt
tcggctgcggcgagcggtatcagctcactcaaaggcggtaatacggttatccacagaatcaggggataacgcaggaaagaaca
tgtgagcaaaaggccagcaaaaggccaggaaccgtaaaaaggccgcgttgctggcgtttttccataggctccgcccccctgac
gagcatcacaaaaatcgacgctcaagtcagaggtggcgaaacccgacaggactataaagataccaggcgtttccccctggaag
ctccctcgtgcgctctcctgttccgaccctgccgcttaccggatacctgtccgcctttctcccttcgggaagcgtggcgctttctcat
agctcacgctgtaggtatctcagttcggtgtaggtcgttcgctccaagctgggctgtgtgcacgaaccccccgttcagcccgacc
gctgcgccttatccggtaactatcgtcttgagtccaacccggtaagacacgacttatcgccactggcagcagccactggtaacag
gattagcagagcgaggtatgtaggcggtgctacagagttcttgaagtggtggcctaactacggctacactagaaggacagtattt
ggtatctgcgctctgctgaagccagttaccttcggaaaaagagttggtagctcttgatccggcaaacaaaccaccgctggtagcg
gtggtttttttgtttgcaagcagcagattacgcgcagaaaaaaaggatctcaagaagatcctttgatcttttctacggggtctgacgct
cagtggaacgaaaactcacgttaagggattttggtcatgagattatcaaaaaggatcttcacctagatccttttaaattaaaaatgaa
gttttaaatcaatctaaagtatatatgagtaaacttggtctgacagttaccaatgcttaatcagtgaggcacctatctcagcgatctgtc
tatttcgttcatccatagttgcctgactccccgtcgtgtagataactacgatacgggagggcttaccatctggccccagtgctgcaat
gataccgcgagacccacgctcaccggctccagatttatcagcaataaaccagccagccggaagggccgagcgcagaagtggt
cctgcaactttatccgcctccatccagtctattaattgttgccgggaagctagagtaagtagttcgccagttaatagtttgcgcaacgt
tgttgnnnnnnaaaaaggatcttcacctagatccttttcacgtagaaagccagtccgcagaaacggtgctgaccccggatgaat
gtcagctactgggctatctggacaagggaaaacgcaagcgcaaagagaaagcaggtagcttgcagtgggcttacatggcgata
gctagactgggcggttttatggacagcaagcgaaccggaattgccagctggggcgccctctggtaaggttgggaagccctgca
aagtaaactggatggctttctcgccgccaaggatctgatggcgcaggggatcaagctctgatcaagagacaggatgaggatcgt
ttcgcatgattgaacaagatggattgcacgcaggttctccggccgcttgggtggagaggctattcggctatgactgggcacaaca
gacaatcggctgctctgatgccgccgtgttccggctgtcagcgcaggggcgcccggttctttttgtcaagaccgacctgtccggt
gccctgaatgaactgcaagacgaggcagcgcggctatcgtggctggccacgacgggcgttccttgcgcagctgtgctcgacgt
tgtcactgaagcgggaagggactggctgctattgggcgaagtgccggggcaggatctcctgtcatctcaccttgctcctgccga
gaaagtatccatcatggctgatgcaatgcggcggctgcatacgcttgatccggctacctgcccattcgaccaccaagcgaaacat
cgcatcgagcgagcacgtactcggatggaagccggtcttgtcgatcaggatgatctggacgaggagcatcaggggctcgcgc
cagccgaactgttcgccaggctcaaggcgagcatgcccgacggcgaggatctcgtcgtgacccatggcgatgcctgcttgccg
aatatcatggtggaaaatggccgcttttctggattcatcgactgtggccggctgggtgtggcggaccgctatcaggacatagcgtt
ggctacccgtgatattgctgaggagcttggcggcgaatgggctgaccgcttcctcgtgctttacggtatcgccgctcccgattcgc
agcgcatcgccttctatcgccttcttgacgagttcttctgaattttgttaaaatttttgttaaatcagctcattttttaaccaataggccgaa
atcggcaacatcccttataaatcaaaagaatagaccgcgatagggttgagtgttgttccagtttggaacaagagtccactattaaag
aacgtggactccaacgtcaaagggcgaaaaaccgtctatcagggcgatggcccactacgtgaaccatcacccaaatcaagtttt
ttgcggtcgaggtgccgtaaagctctaaatcggaaccctaaagggagcccccgatttagagcttgacggggaaagccggcgaa
cgtggcgagaaaggaagggaagaaagcgaaaggagcgggcgctagggcgctggcaagtgtagcggtcacgctgcgcgta
accaccacacccgcgcgcttaatgcgccg
**pGW035 Adenovirus One Vector dSpCas9 (SEQ ID NO: 5)** 
CTACAGGGCGCGTCCATTCGCCATTCAGGATCGAATTAATTCTTAATTAACAT
CATCAATAATATACCTTAttttggattgaagccaatatgataatgagggggtggagtttgtgacgtggcgcgggg
cgtgggaacggggcgggtgacgtagtagtgtggcggaagtgtgatgttgcaagtgtggcggaacacatgtaagcgacggatgt
ggcaaaagtgacgtttttggtgtgcgccggtgtacacaggaagtgacaattttcgcgcggttttaggcggatgttgtagtaaatttg
ggcgtaaccgagtaagatttggccattttcgcgggaaaactgaataagaggaagtgaaatctgaataattttgtgttactcatagcg
cgtaaTACTggtaccgcggccgcctcgagtCTAGAAAGAgggcctatttcccatgattccttcatatttgcatatacga
tacaaggctgttagagagataattagaattaatttgactgtaaacacaaagatattagtacaaaatacgtgacgtagaaagtaataat
ttcttgggtagtttgcagttttaaaattatgttttaaaatggactatcatatgcttaccgtaacttgaaagtatttcgatttcttggctttatat
atcttGTGGAAAGGACGAAACACCggaagagcgagctcttctgttttagagctaggccAACATGAGG
ATCACCCATGTCTGCAGggcctagcaagttaaaataaggctagtccgttatcaacttggccAACATGAGG
 
ATCACCCATGTCTGCAGggccaagtggcaccgagtcggtgcTTTTTTGctagcGTCAGTGGGC
AGAGCGCACATCGCCCACAGTCCCCGAGAAGTTGGGGGGAGGGGTCGGCAAT
TGAACGGGTGCCTAGAGAAGGTGGCGCGGGGTAAACTGGGAAAGTGATGTC
GTGTACTGGCTCCGCCTTTTTCCCGAGGGTGGGGGAGAACCGTATATAAGTGC
AGTAGTCGCCGTGAACGTTCTTTTTCGCAACGGGTTTGCCGCCAGAACACAGC
TGAAGCTTCGAGGGGCTCGCATCTCTCCTTCACGCGCCCGCCGCCCTACCTGA
GGCCGCCATCCACGCCGGTTGAGTCGCGTTCTGCCGCCTCCCGCCTGTGGTGC
CTCCTGAACTGCGTCCGCCGTCTAGGTAAGTTTAAAGCTCAGGTCGAGACCGG
GCCTTTGTCCGGCGCTCCCTTGGAGCCTACCTAGACTCAGCCGGCTCTCCACG
CTTTGCCTGACCCTGCTTGCTCAACTCTACGTCTTTGTTTCGTTTTCTGTTCTGC
GCCGTTACAGATCCAAGctcGTACGgccaccATGAAAAGGCCGGCGGCCACGAAA
AAGGCCGGCCAGGCAAAAAAGAAAAAGGACAAGAAGTACAGCATCGGCCTG
GCCATCGGCACCAACTCTGTGGGCTGGGCCGTGATCACCGACGAGTACAAGG
TGCCCAGCAAGAAATTCAAGGTGCTGGGCAACACCGACCGGCACAGCATCAA
GAAGAACCTGATCGGAGCCCTGCTGTTCGACAGCGGCGAAACAGCCGAGGCC
ACCCGGCTGAAGAGAACCGCCAGAAGAAGATACACCAGACGGAAGAACCGG
ATCTGCTATCTGCAAGAGATCTTCAGCAACGAGATGGCCAAGGTGGACGACA
GCTTCTTCCACAGACTGGAAGAGTCCTTCCTGGTGGAAGAGGATAAGAAGCA
CGAGCGGCACCCCATCTTCGGCAACATCGTGGACGAGGTGGCCTACCACGAG
AAGTACCCCACCATCTACCACCTGAGAAAGAAACTGGTGGACAGCACCGACA
AGGCCGACCTGCGGCTGATCTATCTGGCCCTGGCCCACATGATCAAGTTCCGG
GGCCACTTCCTGATCGAGGGCGACCTGAACCCCGACAACAGCGACGTGGACA
AGCTGTTCATCCAGCTGGTGCAGACCTACAACCAGCTGTTCGAGGAAAACCC
CATCAACGCCAGCGGCGTGGACGCCAAGGCCATCCTGTCTGCCAGACTGAGC
AAGAGCAGACGGCTGGAAAATCTGATCGCCCAGCTGCCCGGCGAGAAGAAG
AATGGCCTGTTCGGCAACCTGATTGCCCTGAGCCTGGGCCTGACCCCCAACTT
CAAGAGCAACTTCGACCTGGCCGAGGATGCCAAACTGCAGCTGAGCAAGGAC
ACCTACGACGACGACCTGGACAACCTGCTGGCCCAGATCGGCGACCAGTACG
CCGACCTGTTTCTGGCCGCCAAGAACCTGTCCGACGCCATCCTGCTGAGCGAC
ATCCTGAGAGTGAACACCGAGATCACCAAGGCCCCCCTGAGCGCCTCTATGA
TCAAGAGATACGACGAGCACCACCAGGACCTGACCCTGCTGAAAGCTCTCGT
GCGGCAGCAGCTGCCTGAGAAGTACAAAGAGATTTTCTTCGACCAGAGCAAG
AACGGCTACGCCGGCTACATTGACGGCGGAGCCAGCCAGGAAGAGTTCTACA
AGTTCATCAAGCCCATCCTGGAAAAGATGGACGGCACCGAGGAACTGCTCGT
GAAGCTGAACAGAGAGGACCTGCTGCGGAAGCAGCGGACCTTCGACAACGG
CAGCATCCCCCACCAGATCCACCTGGGAGAGCTGCACGCCATTCTGCGGCGG
CAGGAAGATTTTTACCCATTCCTGAAGGACAACCGGGAAAAGATCGAGAAGA
TCCTGACCTTCCGCATCCCCTACTACGTGGGCCCTCTGGCCAGGGGAAACAGC
AGATTCGCCTGGATGACCAGAAAGAGCGAGGAAACCATCACCCCCTGGAACT
TCGAGGAAGTGGTGGACAAGGGCGCTTCCGCCCAGAGCTTCATCGAGCGGAT
GACCAACTTCGATAAGAACCTGCCCAACGAGAAGGTGCTGCCCAAGCACAGC
CTGCTGTACGAGTACTTCACCGTGTATAACGAGCTGACCAAAGTGAAATACG
TGACCGAGGGAATGAGAAAGCCCGCCTTCCTGAGCGGCGAGCAGAAAAAGG
CCATCGTGGACCTGCTGTTCAAGACCAACCGGAAAGTGACCGTGAAGCAGCT
GAAAGAGGACTACTTCAAGAAAATCGAGTGCTTCGACTCCGTGGAAATCTCC
GGCGTGGAAGATCGGTTCAACGCCTCCCTGGGCACATACCACGATCTGCTGA
AAATTATCAAGGACAAGGACTTCCTGGACAATGAGGAAAACGAGGACATTCT
GGAAGATATCGTGCTGACCCTGACACTGTTTGAGGACAGAGAGATGATCGAG
GAACGGCTGAAAACCTATGCCCACCTGTTCGACGACAAAGTGATGAAGCAGC
TGAAGCGGCGGAGATACACCGGCTGGGGCAGGCTGAGCCGGAAGCTGATCA
 
ACGGCATCCGGGACAAGCAGTCCGGCAAGACAATCCTGGATTTCCTGAAGTC
CGACGGCTTCGCCAACAGAAACTTCATGCAGCTGATCCACGACGACAGCCTG
ACCTTTAAAGAGGACATCCAGAAAGCCCAGGTGTCCGGCCAGGGCGATAGCC
TGCACGAGCACATTGCCAATCTGGCCGGCAGCCCCGCCATTAAGAAGGGCAT
CCTGCAGACAGTGAAGGTGGTGGACGAGCTCGTGAAAGTGATGGGCCGGCAC
AAGCCCGAGAACATCGTGATCGAAATGGCCAGAGAGAACCAGACCACCCAG
AAGGGACAGAAGAACAGCCGCGAGAGAATGAAGCGGATCGAAGAGGGCATC
AAAGAGCTGGGCAGCCAGATCCTGAAAGAACACCCCGTGGAAAACACCCAG
CTGCAGAACGAGAAGCTGTACCTGTACTACCTGCAGAATGGGCGGGATATGT
ACGTGGACCAGGAACTGGACATCAACCGGCTGTCCGACTACGATGTGGACCA
CATCGTGCCTCAGAGCTTTCTGAAGGACGACTCCATCGACAACAAGGTGCTG
ACCAGAAGCGACAAGGCCCGGGGCAAGAGCGACAACGTGCCCTCCGAAGAG
GTCGTGAAGAAGATGAAGAACTACTGGCGGCAGCTGCTGAACGCCAAGCTGA
TTACCCAGAGAAAGTTCGACAATCTGACCAAGGCCGAGAGAGGCGGCCTGAG
CGAACTGGATAAGGCCGGCTTCATCAAGAGACAGCTGGTGGAAACCCGGCAG
ATCACAAAGCACGTGGCACAGATCCTGGACTCCCGGATGAACACTAAGTACG
ACGAGAATGACAAGCTGATCCGGGAAGTGAAAGTGATCACCCTGAAGTCCAA
GCTGGTGTCCGATTTCCGGAAGGATTTCCAGTTTTACAAAGTGCGCGAGATCA
ACAACTACCACCACGCCCACGACGCCTACCTGAACGCCGTCGTGGGAACCGC
CCTGATCAAAAAGTACCCTAAGCTGGAAAGCGAGTTCGTGTACGGCGACTAC
AAGGTGTACGACGTGCGGAAGATGATCGCCAAGAGCGAGCAGGAAATCGGC
AAGGCTACCGCCAAGTACTTCTTCTACAGCAACATCATGAACTTTTTCAAGAC
CGAGATTACCCTGGCCAACGGCGAGATCCGGAAGCGGCCTCTGATCGAGACA
AACGGCGAAACCGGGGAGATCGTGTGGGATAAGGGCCGGGATTTTGCCACCG
TGCGGAAAGTGCTGAGCATGCCCCAAGTGAATATCGTGAAAAAGACCGAGGT
GCAGACAGGCGGCTTCAGCAAAGAGTCTATCCTGCCCAAGAGGAACAGCGAT
AAGCTGATCGCCAGAAAGAAGGACTGGGACCCTAAGAAGTACGGCGGCTTCG
ACAGCCCCACCGTGGCCTATTCTGTGCTGGTGGTGGCCAAAGTGGAAAAGGG
CAAGTCCAAGAAACTGAAGAGTGTGAAAGAGCTGCTGGGGATCACCATCATG
GAAAGAAGCAGCTTCGAGAAGAATCCCATCGACTTTCTGGAAGCCAAGGGCT
ACAAAGAAGTGAAAAAGGACCTGATCATCAAGCTGCCTAAGTACTCCCTGTT
CGAGCTGGAAAACGGCCGGAAGAGAATGCTGGCCTCTGCCGGCGAACTGCAG
AAGGGAAACGAACTGGCCCTGCCCTCCAAATATGTGAACTTCCTGTACCTGG
CCAGCCACTATGAGAAGCTGAAGGGCTCCCCCGAGGATAATGAGCAGAAACA
GCTGTTTGTGGAACAGCACAAGCACTACCTGGACGAGATCATCGAGCAGATC
AGCGAGTTCTCCAAGAGAGTGATCCTGGCCGACGCTAATCTGGACAAAGTGC
TGTCCGCCTACAACAAGCACCGGGATAAGCCCATCAGAGAGCAGGCCGAGA
ATATCATCCACCTGTTTACCCTGACCAATCTGGGAGCCCCTGCCGCCTTCAAG
TACTTTGACACCACCATCGACCGGAAGAGGTACACCAGCACCAAAGAGGTGC
TGGACGCCACCCTGATCCACCAGAGCATCACCGGCCTGTACGAGACACGGAT
CGACCTGTCTCAGCTGGGAGGCGACagcgctGGAGGAGGTGGAAGCGGAGGAG
GAGGAAGCGGAGGAGGAGGTAGCggacctaagaaaaagaggaaggtggcggccgctggatccGGA
CGGGCTGACGCATTGGACGATTTTGATCTGGATATGCTGGGAAGTGACGCCCT
CGATGATTTTGACCTTGACATGCTTGGTTCGGATGCCCTTGATGACTTTGACCT
CGACATGCTCGGCAGTGACGCCCTTGATGATTTCGACCTGGACATGCTGATTA
ACtgtacaggaagcggagccactaacttctccctgttgaaacaagcaggggatgtcgaagagaatcccgggccaGCCAC
CATGGCTTCAAACTTTACTCAGTTCGTGCTCGTGGACAATGGTGGGACAGGGG
ATGTGACAGTGGCTCCTTCTAATTTCGCTAATGGGGTGGCAGAGTGGATCAGC
TCCAACTCACGGAGCCAGGCCTACAAGGTGACATGCAGCGTCAGGCAGTCTA
GTGCCCAGAAGAGAAAGTATACCATCAAGGTGGAGGTCCCCAAAGTGGCTAC
 
CCAGACAGTGGGCGGAGTCGAACTGCCTGTCGCCGCTTGGAGGTCCTACCTG
AACATGGAGCTCACTATCCCAATTTTCGCTACCAATTCTGACTGTGAACTCAT
CGTGAAGGCAATGCAGGGGCTCCTCAAAGACGGTAATCCTATCCCTTCCGCC
ATCGCCGCTAACTCAGGTATCTACAGCGCTGGAGGAGGTGGAAGCGGAGGAG
GAGGAAGCGGAGGAGGAGGTAGCGGACCTAAGAAAAAGAGGAAGGTGGCG
GCCGCTGGATCCCCTTCAGGGCAGATCAGCAACCAGGCCCTGGCTCTGGCCC
CTAGCTCCGCTCCAGTGCTGGCCCAGACTATGGTGCCCTCTAGTGCTATGGTG
CCTCTGGCCCAGCCACCTGCTCCAGCCCCTGTGCTGACCCCAGGACCACCCCA
GTCACTGAGCGCTCCAGTGCCCAAGTCTACACAGGCCGGCGAGGGGACTCTG
AGTGAAGCTCTGCTGCACCTGCAGTTCGACGCTGATGAGGACCTGGGAGCTC
TGCTGGGGAACAGCACCGATCCCGGAGTGTTCACAGATCTGGCCTCCGTGGA
CAACTCTGAGTTTCAGCAGCTGCTGAATCAGGGCGTGTCCATGTCTCATAGTA
CAGCCGAACCAATGCTGATGGAGTACCCCGAAGCCATTACCCGGCTGGTGAC
CGGCAGCCAGCGGCCCCCCGACCCCGCTCCAACTCCCCTGGGAACCAGCGGC
CTGCCTAATGGGCTGTCCGGAGATGAAGACTTCTCAAGCATCGCTGATATGG
ACTTTAGTGCCCTGCTGTCACAGATTTCCTCTAGTGGGCAGGGAGGAGGTGGA
AGCGGCTTCAGCGTGGACACCAGTGCCCTGCTGGACCTGTTCAGCCCCTCGGT
GACCGTGCCCGACATGAGCCTGCCTGACCTTGACAGCAGCCTGGCCAGTATC
CAAGAGCTCCTGTCTCCCCAGGAGCCCCCCAGGCCTCCCGAGGCAGAGAACA
GCAGCCCGGATTCAGGGAAGCAGCTGGTGCACTACACAGCGCAGCCGCTGTT
CCTGCTGGACCCCGGCTCCGTGGACACCGGGAGCAACGACCTGCCGGTGCTG
TTTGAGCTGGGAGAGGGCTCCTACTTCTCCGAAGGGGACGGCTTCGCCGAGG
ACCCCACCATCTCCCTGCTGACAGGCTCGGAGCCTCCCAAAGCCAAGGACCC
CACTGTCTCCTAAGAATTCgatatcaagcttAATAAAAGATCTTTATTTTCATTAGAT
CTGTGTGTTGGTTTTTTGTGTagatctGGGCGTGGTtaagggtgggaaagaatatataaggtgggggt
cttatgtagttttgtatctgttttgcagcagccgccgccgccatgagcaccaactcgtttgatggaagcattgtgagctcatatttgac
aacgcgcatgcccccatgggccggggtgcgtcagaatgtgatgggctccagcattgatggtcgccccgtcctgcccgcaaact
ctactaccttgacctacgagaccgtgtctggaacgccgttggagactgcagcctccgccgccgcttcagccgctgcagccaccg
cccgcgggattgtgactgactttgctttcctgagcccgcttgcaagcagtgcagcttcccgttcatccgcccgcgatgacaagttg
acggctcttttggcacaattggattctttgacccgggaacttaatgtcgtttctcagcagctgttggatctgcgccagcaggtttctgc
cctgaaggcttcctcccctcccaatgcggtttaaaacataaataaaaaaccagactctgtttggatttggatcaagcaagtgtcttgc
tgtctttatttaggggttttgcgcgcgcggtaggcccgggaccagcggtctcggtcgttgagggtcctgtgtattttttccaggacgt
ggtaaaggtgactctggatgttcagatacatgggcataagcccgtctctggggtggaggtagcaccactgcagagcttcatgctg
cggggtggtgttgtagatgatccagtcgtagcaggagcgctgggcgtggtgcctaaaaatgtctttcagtagcaagctgattgcc
aggggcaggcccttggtgtaagtgtttacaaagcggttaagctgggatgggtgcatacgtggggatatgagatgcatcttggact
gtatttttaggttggctatgttcccagccatatccctccggggattcatgttgtgcagaaccaccagcacagtgtatccggtgcactt
gggaaatttgtcatgtagcttagaaggaaatgcgtggaagaacttggagacgcccttgtgacctccaagattttccatgcattcgtc
cataatgatggcaatgggcccacgggcggcggcctgggcgaagatatttctgggatcactaacgtcatagttgtgttccaggatg
agatcgtcataggccatttttacaaagcgcgggcggagggtgccagactgcggtataatggttccatccggcccaggggcgtag
ttaccctcacagatttgcatttcccacgctttgagttcagatggggggatcatgtctacctgcggggcgatgaagaaaacggtttcc
ggggtaggggagatcagctgggaagaaagcaggttcctgagcagctgcgacttaccgcagccggtgggcccgtaaatcacac
ctattaccgggtgcaactggtagttaagagagctgcagctgccgtcatccctgagcaggggggccacttcgttaagcatgtccct
gactcgcatgttttccctgaccaaatccgccagaaggcgctcgccgcccagcgatagcagttcttgcaaggaagcaaagtttttc
aacggtttgagaccgtccgccgtaggcatgcttttgagcgtttgaccaagcagttccaggcggtcccacagctcggtcacctgct
ctacggcatctcgatccagcatatctcctcgtttcgcgggttggggcggctttcgctgtacggcagtagtcggtgctcgtccagac
gggccagggtcatgtctttccacgggcgcagggtcctcgtcagcgtagtctgggtcacggtgaaggggtgcgctccgggctgc
gcgctggccagggtgcgcttgaggctggtcctgctggtgctgaagcgctgccggtcttcgccctgcgcgtcggccaggtagca
tttgaccatggtgtcatagtccagcccctccgcggcgtggcccttggcgcgcagcttgcccttggaggaggcgccgcacgagg
ggcagtgcagacttttgagggcgtagagcttgggcgcgagaaataccgattccggggagtaggcatccgcgccgcaggcccc
gcagacggtctcgcattccacgagccaggtgagctctggccgttcggggtcaaaaaccaggtttcccccatgctttttgatgcgtt
 
tcttacctctggtttccatgagccggtgtccacgctcggtgacgaaaaggctgtccgtgtccccgtatacagactnnngtttaaac
gaattcnnntataaaatgcaaggtgctgctcaaaaaatcaggcaaagcctcgcgcaaaaaagaaagcacatcgtagtcatgctc
atgcagataaaggcaggtaagctccggaaccaccacagaaaaagacaccatttttctctcaaacatgtctgcgggtttctgcataa
acacaaaataaaataacaaaaaaacatttaaacattagaagcctgtcttacaacaggaaaaacaacccttataagcataagacgg
actacggccatgccggcgtgaccgtaaaaaaactggtcaccgtgattaaaaagcaccaccgacagctcctcggtcatgtccgga
gtcataatgtaagactcggtaaacacatcaggttgattcatcggtcagtgctaaaaagcgaccgaaatagcccgggggaatacat
acccgcaggcgtagagacaacattacagcccccataggaggtataacaaaattaataggagagaaaaacacataaacacctga
aaaaccctcctgcctaggcaaaatagcaccctcccgctccagaacaacatacagcgcttcacagcggcagcctaacagtcagc
cttaccagtaaaaaagaaaacctattaaaaaaacaccactcgacacggcaccagctcaatcagtcacagtgtaaaaaagggcca
agtgcagagcgagtatatataggactaaaaaatgacgtaacggttaaagtccacaaaaaacacccagaaaaccgcacgcgaac
ctacgcccagaaacgaaagccaaaaaacccacaacttcctcaaatcgtcacttccgttttcccacgttacgtaacttcccattttaag
aaaactacaattcccaacacatacaagttactccgccctaaaacctacgtcacccgccccgttcccacgccccgcgccacgtcac
aaactccaccccctcattatcatattggcttcaatccaaaataaggtatattattgatgattaattaattaaggatcccggtgtgaaata
ccgcacagatgcgtaaggagaaaataccgcatcaggcgctcttacgcttcctcgctcactgactcgctgcgctcggtcgttcggc
tgcggcgagcggtatcagctcactcaaaggcggtaatacggttatccacagaatcaggggataacgcaggaaagaacatgtga
gcaaaaggccagcaaaaggccaggaaccgtaaaaaggccgcgttgctggcgtttttccataggctccgcccccctgacgagc
atcacaaaaatcgacgctcaagtcagaggtggcgaaacccgacaggactataaagataccaggcgtttccccctggaagctcc
ctcgtgcgctctcctgttccgaccctgccgcttaccggatacctgtccgcctttctcccttcgggaagcgtggcgctttctcatagct
cacgctgtaggtatctcagttcggtgtaggtcgttcgctccaagctgggctgtgtgcacgaaccccccgttcagcccgaccgctg
cgccttatccggtaactatcgtcttgagtccaacccggtaagacacgacttatcgccactggcagcagccactggtaacaggatta
gcagagcgaggtatgtaggcggtgctacagagttcttgaagtggtggcctaactacggctacactagaaggacagtatttggtat
ctgcgctctgctgaagccagttaccttcggaaaaagagttggtagctcttgatccggcaaacaaaccaccgctggtagcggtggt
ttttttgtttgcaagcagcagattacgcgcagaaaaaaaggatctcaagaagatcctttgatcttttctacggggtctgacgctcagt
ggaacgaaaactcacgttaagggattttggtcatgagattatcaaaaaggatcttcacctagatccttttaaattaaaaatgaagtttt
aaatcaatctaaagtatatatgagtaaacttggtctgacagttaccaatgcttaatcagtgaggcacctatctcagcgatctgtctattt
cgttcatccatagttgcctgactccccgtcgtgtagataactacgatacgggagggcttaccatctggccccagtgctgcaatgat
accgcgagacccacgctcaccggctccagatttatcagcaataaaccagccagccggaagggccgagcgcagaagtggtcct
gcaactttatccgcctccatccagtctattaattgttgccgggaagctagagtaagtagttcgccagttaatagtttgcgcaacgttgt
tgnnnnnnaaaaaggatcttcacctagatccttttcacgtagaaagccagtccgcagaaacggtgctgaccccggatgaatgtc
agctactgggctatctggacaagggaaaacgcaagcgcaaagagaaagcaggtagcttgcagtgggcttacatggcgatagct
agactgggcggttttatggacagcaagcgaaccggaattgccagctggggcgccctctggtaaggttgggaagccctgcaaag
taaactggatggctttctcgccgccaaggatctgatggcgcaggggatcaagctctgatcaagagacaggatgaggatcgtttcg
catgattgaacaagatggattgcacgcaggttctccggccgcttgggtggagaggctattcggctatgactgggcacaacagac
aatcggctgctctgatgccgccgtgttccggctgtcagcgcaggggcgcccggttctttttgtcaagaccgacctgtccggtgcc
ctgaatgaactgcaagacgaggcagcgcggctatcgtggctggccacgacgggcgttccttgcgcagctgtgctcgacgttgtc
actgaagcgggaagggactggctgctattgggcgaagtgccggggcaggatctcctgtcatctcaccttgctcctgccgagaaa
gtatccatcatggctgatgcaatgcggcggctgcatacgcttgatccggctacctgcccattcgaccaccaagcgaaacatcgca
tcgagcgagcacgtactcggatggaagccggtcttgtcgatcaggatgatctggacgaggagcatcaggggctcgcgccagc
cgaactgttcgccaggctcaaggcgagcatgcccgacggcgaggatctcgtcgtgacccatggcgatgcctgcttgccgaatat
catggtggaaaatggccgcttttctggattcatcgactgtggccggctgggtgtggcggaccgctatcaggacatagcgttggct
acccgtgatattgctgaggagcttggcggcgaatgggctgaccgcttcctcgtgctttacggtatcgccgctcccgattcgcagc
gcatcgccttctatcgccttcttgacgagttcttctgaattttgttaaaatttttgttaaatcagctcattttttaaccaataggccgaaatc
ggcaacatcccttataaatcaaaagaatagaccgcgatagggttgagtgttgttccagtttggaacaagagtccactattaaagaa
cgtggactccaacgtcaaagggcgaaaaaccgtctatcagggcgatggcccactacgtgaaccatcacccaaatcaagttttttg
cggtcgaggtgccgtaaagctctaaatcggaaccctaaagggagcccccgatttagagcttgacggggaaagccggcgaacg
tggcgagaaaggaagggaagaaagcgaaaggagcgggcgctagggcgctggcaagtgtagcggtcacgctgcgcgtaacc
accacacccgcgcgcttaatgcgccg
**pGW063 Adenovirus One Vector dSpCas9 (SEQ ID NO: 6)** 
AATTAATTAAGCTAGCATCATCAATAATATACCTTATTTTGGATTGAAGCCAA
TATGATAATGAGGGGGTGGAGTTTGTGACGTGGCGCGGGGCGTGGGAACGGG
GCGGGTGACGTAGTAGTGTGGCGGAAGTGTGATGTTGCAAGTGTGGCGGAAC
ACATGTAAGCGACGGATGTGGCAAAAGTGACGTTTTTGGTGTGCGCCGGTGT
ACACAGGAAGTGACAATTTTCGCGCGGTTTTAGGCGGATGTTGTAGTAAATTT
GGGCGTAACCGAGTAAGATTTGGCCATTTTCGCGGGAAAACTGAATAAGAGG
AAGTGAAATCTGAATAATTTTGTGTTACTCATAGCGCGTAATATTTGTCTAGG
GAGATCCGGTACcgcggccgcctcgagtCTAGAAAGAgggcctatttcccatgattccttcatatttgcatata
cgatacaaggctgttagagagataattagaattaatttgactgtaaacacaaagatattagtacaaaatacgtgacgtagaaagtaa
taatttcttgggtagtttgcagttttaaaattatgttttaaaatggactatcatatgcttaccgtaacttgaaagtatttcgatttcttggcttt
atatatcttGTGGAAAGGACGAAACACCggaagagcgagctcttctgttttagagctaggccAACATGA
GGATCACCCATGTCTGCAGggcctagcaagttaaaataaggctagtccgttatcaacttggccAACATGA
GGATCACCCATGTCTGCAGggccaagtggcaccgagtcggtgcTTTTTTGctagcGTCAGTGG
GCAGAGCGCACATCGCCCACAGTCCCCGAGAAGTTGGGGGGAGGGGTCGGC
AATTGAACGGGTGCCTAGAGAAGGTGGCGCGGGGTAAACTGGGAAAGTGAT
GTCGTGTACTGGCTCCGCCTTTTTCCCGAGGGTGGGGGAGAACCGTATATAAG
TGCAGTAGTCGCCGTGAACGTTCTTTTTCGCAACGGGTTTGCCGCCAGAACAC
AGCTGAAGCTTCGAGGGGCTCGCATCTCTCCTTCACGCGCCCGCCGCCCTACC
TGAGGCCGCCATCCACGCCGGTTGAGTCGCGTTCTGCCGCCTCCCGCCTGTGG
TGCCTCCTGAACTGCGTCCGCCGTCTAGGTAAGTTTAAAGCTCAGGTCGAGAC
CGGGCCTTTGTCCGGCGCTCCCTTGGAGCCTACCTAGACTCAGCCGGCTCTCC
ACGCTTTGCCTGACCCTGCTTGCTCAACTCTACGTCTTTGTTTCGTTTTCTGTTC
TGCGCCGTTACAGATCCAAGctcGTACGgccaccATGAAAAGGCCGGCGGCCACG
AAAAAGGCCGGCCAGGCAAAAAAGAAAAAGGACAAGAAGTACAGCATCGGC
CTGGCCATCGGCACCAACTCTGTGGGCTGGGCCGTGATCACCGACGAGTACA
AGGTGCCCAGCAAGAAATTCAAGGTGCTGGGCAACACCGACCGGCACAGCAT
CAAGAAGAACCTGATCGGAGCCCTGCTGTTCGACAGCGGCGAAACAGCCGAG
GCCACCCGGCTGAAGAGAACCGCCAGAAGAAGATACACCAGACGGAAGAAC
CGGATCTGCTATCTGCAAGAGATCTTCAGCAACGAGATGGCCAAGGTGGACG
ACAGCTTCTTCCACAGACTGGAAGAGTCCTTCCTGGTGGAAGAGGATAAGAA
GCACGAGCGGCACCCCATCTTCGGCAACATCGTGGACGAGGTGGCCTACCAC
GAGAAGTACCCCACCATCTACCACCTGAGAAAGAAACTGGTGGACAGCACCG
ACAAGGCCGACCTGCGGCTGATCTATCTGGCCCTGGCCCACATGATCAAGTTC
CGGGGCCACTTCCTGATCGAGGGCGACCTGAACCCCGACAACAGCGACGTGG
ACAAGCTGTTCATCCAGCTGGTGCAGACCTACAACCAGCTGTTCGAGGAAAA
CCCCATCAACGCCAGCGGCGTGGACGCCAAGGCCATCCTGTCTGCCAGACTG
AGCAAGAGCAGACGGCTGGAAAATCTGATCGCCCAGCTGCCCGGCGAGAAG
AAGAATGGCCTGTTCGGCAACCTGATTGCCCTGAGCCTGGGCCTGACCCCCA
ACTTCAAGAGCAACTTCGACCTGGCCGAGGATGCCAAACTGCAGCTGAGCAA
GGACACCTACGACGACGACCTGGACAACCTGCTGGCCCAGATCGGCGACCAG
TACGCCGACCTGTTTCTGGCCGCCAAGAACCTGTCCGACGCCATCCTGCTGAG
CGACATCCTGAGAGTGAACACCGAGATCACCAAGGCCCCCCTGAGCGCCTCT
ATGATCAAGAGATACGACGAGCACCACCAGGACCTGACCCTGCTGAAAGCTC
TCGTGCGGCAGCAGCTGCCTGAGAAGTACAAAGAGATTTTCTTCGACCAGAG
CAAGAACGGCTACGCCGGCTACATTGACGGCGGAGCCAGCCAGGAAGAGTTC
TACAAGTTCATCAAGCCCATCCTGGAAAAGATGGACGGCACCGAGGAACTGC
TCGTGAAGCTGAACAGAGAGGACCTGCTGCGGAAGCAGCGGACCTTCGACAA
CGGCAGCATCCCCCACCAGATCCACCTGGGAGAGCTGCACGCCATTCTGCGG
CGGCAGGAAGATTTTTACCCATTCCTGAAGGACAACCGGGAAAAGATCGAGA
AGATCCTGACCTTCCGCATCCCCTACTACGTGGGCCCTCTGGCCAGGGGAAAC
 
AGCAGATTCGCCTGGATGACCAGAAAGAGCGAGGAAACCATCACCCCCTGGA
ACTTCGAGGAAGTGGTGGACAAGGGCGCTTCCGCCCAGAGCTTCATCGAGCG
GATGACCAACTTCGATAAGAACCTGCCCAACGAGAAGGTGCTGCCCAAGCAC
AGCCTGCTGTACGAGTACTTCACCGTGTATAACGAGCTGACCAAAGTGAAAT
ACGTGACCGAGGGAATGAGAAAGCCCGCCTTCCTGAGCGGCGAGCAGAAAA
AGGCCATCGTGGACCTGCTGTTCAAGACCAACCGGAAAGTGACCGTGAAGCA
GCTGAAAGAGGACTACTTCAAGAAAATCGAGTGCTTCGACTCCGTGGAAATC
TCCGGCGTGGAAGATCGGTTCAACGCCTCCCTGGGCACATACCACGATCTGCT
GAAAATTATCAAGGACAAGGACTTCCTGGACAATGAGGAAAACGAGGACATT
CTGGAAGATATCGTGCTGACCCTGACACTGTTTGAGGACAGAGAGATGATCG
AGGAACGGCTGAAAACCTATGCCCACCTGTTCGACGACAAAGTGATGAAGCA
GCTGAAGCGGCGGAGATACACCGGCTGGGGCAGGCTGAGCCGGAAGCTGAT
CAACGGCATCCGGGACAAGCAGTCCGGCAAGACAATCCTGGATTTCCTGAAG
TCCGACGGCTTCGCCAACAGAAACTTCATGCAGCTGATCCACGACGACAGCC
TGACCTTTAAAGAGGACATCCAGAAAGCCCAGGTGTCCGGCCAGGGCGATAG
CCTGCACGAGCACATTGCCAATCTGGCCGGCAGCCCCGCCATTAAGAAGGGC
ATCCTGCAGACAGTGAAGGTGGTGGACGAGCTCGTGAAAGTGATGGGCCGGC
ACAAGCCCGAGAACATCGTGATCGAAATGGCCAGAGAGAACCAGACCACCC
AGAAGGGACAGAAGAACAGCCGCGAGAGAATGAAGCGGATCGAAGAGGGC
ATCAAAGAGCTGGGCAGCCAGATCCTGAAAGAACACCCCGTGGAAAACACC
CAGCTGCAGAACGAGAAGCTGTACCTGTACTACCTGCAGAATGGGCGGGATA
TGTACGTGGACCAGGAACTGGACATCAACCGGCTGTCCGACTACGATGTGGA
CCACATCGTGCCTCAGAGCTTTCTGAAGGACGACTCCATCGACAACAAGGTG
CTGACCAGAAGCGACAAGGCCCGGGGCAAGAGCGACAACGTGCCCTCCGAA
GAGGTCGTGAAGAAGATGAAGAACTACTGGCGGCAGCTGCTGAACGCCAAG
CTGATTACCCAGAGAAAGTTCGACAATCTGACCAAGGCCGAGAGAGGCGGCC
TGAGCGAACTGGATAAGGCCGGCTTCATCAAGAGACAGCTGGTGGAAACCCG
GCAGATCACAAAGCACGTGGCACAGATCCTGGACTCCCGGATGAACACTAAG
TACGACGAGAATGACAAGCTGATCCGGGAAGTGAAAGTGATCACCCTGAAGT
CCAAGCTGGTGTCCGATTTCCGGAAGGATTTCCAGTTTTACAAAGTGCGCGAG
ATCAACAACTACCACCACGCCCACGACGCCTACCTGAACGCCGTCGTGGGAA
CCGCCCTGATCAAAAAGTACCCTAAGCTGGAAAGCGAGTTCGTGTACGGCGA
CTACAAGGTGTACGACGTGCGGAAGATGATCGCCAAGAGCGAGCAGGAAAT
CGGCAAGGCTACCGCCAAGTACTTCTTCTACAGCAACATCATGAACTTTTTCA
AGACCGAGATTACCCTGGCCAACGGCGAGATCCGGAAGCGGCCTCTGATCGA
GACAAACGGCGAAACCGGGGAGATCGTGTGGGATAAGGGCCGGGATTTTGCC
ACCGTGCGGAAAGTGCTGAGCATGCCCCAAGTGAATATCGTGAAAAAGACCG
AGGTGCAGACAGGCGGCTTCAGCAAAGAGTCTATCCTGCCCAAGAGGAACAG
CGATAAGCTGATCGCCAGAAAGAAGGACTGGGACCCTAAGAAGTACGGCGG
CTTCGACAGCCCCACCGTGGCCTATTCTGTGCTGGTGGTGGCCAAAGTGGAAA
AGGGCAAGTCCAAGAAACTGAAGAGTGTGAAAGAGCTGCTGGGGATCACCA
TCATGGAAAGAAGCAGCTTCGAGAAGAATCCCATCGACTTTCTGGAAGCCAA
GGGCTACAAAGAAGTGAAAAAGGACCTGATCATCAAGCTGCCTAAGTACTCC
CTGTTCGAGCTGGAAAACGGCCGGAAGAGAATGCTGGCCTCTGCCGGCGAAC
TGCAGAAGGGAAACGAACTGGCCCTGCCCTCCAAATATGTGAACTTCCTGTA
CCTGGCCAGCCACTATGAGAAGCTGAAGGGCTCCCCCGAGGATAATGAGCAG
AAACAGCTGTTTGTGGAACAGCACAAGCACTACCTGGACGAGATCATCGAGC
AGATCAGCGAGTTCTCCAAGAGAGTGATCCTGGCCGACGCTAATCTGGACAA
AGTGCTGTCCGCCTACAACAAGCACCGGGATAAGCCCATCAGAGAGCAGGCC
GAGAATATCATCCACCTGTTTACCCTGACCAATCTGGGAGCCCCTGCCGCCTT
 
CAAGTACTTTGACACCACCATCGACCGGAAGAGGTACACCAGCACCAAAGAG
GTGCTGGACGCCACCCTGATCCACCAGAGCATCACCGGCCTGTACGAGACAC
GGATCGACCTGTCTCAGCTGGGAGGCGACagcgctGGAGGAGGTGGAAGCGGAG
GAGGAGGAAGCGGAGGAGGAGGTAGCggacctaagaaaaagaggaaggtggcggccgctgGAT
CCCCTTCAGGGCAGATCAGCAACCAGGCCCTGGCTCTGGCCCCTAGCTCCGCT
CCAGTGCTGGCCCAGACTATGGTGCCCTCTAGTGCTATGGTGCCTCTGGCCCA
GCCACCTGCTCCAGCCCCTGTGCTGACCCCAGGACCACCCCAGTCACTGAGC
GCTCCAGTGCCCAAGTCTACACAGGCCGGCGAGGGGACTCTGAGTGAAGCTC
TGCTGCACCTGCAGTTCGACGCTGATGAGGACCTGGGAGCTCTGCTGGGGAA
CAGCACCGATCCCGGAGTGTTCACAGATCTGGCCTCCGTGGACAACTCTGAGT
TTCAGCAGCTGCTGAATCAGGGCGTGTCCATGTCTCATAGTACAGCCGAACCA
ATGCTGATGGAGTACCCCGAAGCCATTACCCGGCTGGTGACCGGCAGCCAGC
GGCCCCCCGACCCCGCTCCAACTCCCCTGGGAACCAGCGGCCTGCCTAATGG
GCTGTCCGGAGATGAAGACTTCTCAAGCATCGCTGATATGGACTTTAGTGCCC
TGCTGTCACAGATTTCCTCTAGTGGGCAGGGAGGAGGTGGAAGCGGCTTCAG
CGTGGACACCAGTGCCCTGCTGGACCTGTTCAGCCCCTCGGTGACCGTGCCCG
ACATGAGCCTGCCTGACCTTGACAGCAGCCTGGCCAGTATCCAAGAGCTCCT
GTCTCCCCAGGAGCCCCCCAGGCCTCCCGAGGCAGAGAACAGCAGCCCGGAT
TCAGGGAAGCAGCTGGTGCACTACACAGCGCAGCCGCTGTTCCTGCTGGACC
CCGGCTCCGTGGACACCGGGAGCAACGACCTGCCGGTGCTGTTTGAGCTGGG
AGAGGGCTCCTACTTCTCCGAAGGGGACGGCTTCGCCGAGGACCCCACCATC
TCCCTGCTGACAGGCTCGGAGCCTCCCAAAGCCAAGGACCCCACTGTCTCCTA
AGAATTCCTGCAGCCCGGGGGATCCACTAGTTCTAGAGCGGCCGCCACCGCG
GGGAGATCCAGACATGATAAGATACATTGATGAGTTTGGACAAACCACAACT
AGAATGCAGTGAAAAAAATGCTTTATTTGTGAAATTTGTGATGCTATTGCTTT
ATTTGTAACCATTATAAGCTGCAATAAACAAGTTAACAACAACAATTGCATTC
ATTTTATGTTTCAGGTTCAGGGGGAGGTGTGGGAGGTTTTTTAAAGCAAGTAA
AACCTCTACAAATGTGGTATGGCTGATTATGATCCCGGCTGCCTCGCGCGTTT
CGGTGATGACGGTGAAAACCTCTTGACACATGCAGCTCCCGGAGACGGTCAC
AGCTTGTCTGTAAGCGGATGCCGGGAGCAGACAAGCCCGTCAGGGCGCGTCA
GCGGGTGTTGGCGGGTGTCGGGGCGCAGCCATGAGGTCGACTCTAGTCCCCG
CGGTGGCAGATCTGGAAGGTGCTGAGGTACGATGAGACCCGCACCAGGTGCA
GACCCTGCGAGTGTGGCGGTAAACATATTAGGAACCAGCCTGTGATGCTGGA
TGTGACCGAGGAGCTGAGGCCCGATCACTTGGTGCTGGCCTGCACCCGCGCT
GAGTTTGGCTCTAGCGATGAAGATACAGATTGAGGTACTGAAATGTGTGGGC
GTGGCTTAAGGGTGGGAAAGAATATATAAGGTGGGGGTCTTATGTAGTTTTG
TATCTGTTTTGCAGCAGCCGCCGCCGCCATGAGCACCAACTCGTTTGATGGAA
GCATTGTGAGCTCATATTTGACAACGCGCATGCCCCCATGGGCCGGGGTGCGT
CAGAATGTGATGGGCTCCAGCATTGATGGTCGCCCCGTCCTGCCCGCAAACTC
TACTACCTTGACCTACGAGACCGTGTCTGGAACGCCGTTGGAGACTGCAGCCT
CCGCCGCCGCTTCAGCCGCTGCAGCCACCGCCCGCGGGATTGTGACTGACTTT
GCTTTCCTGAGCCCGCTTGCAAGCAGTGCAGCTTCCCGTTCATCCGCCCGCGA
TGACAAGTTGACGGCTCTTTTGGCACAATTGGATTCTTTGACCCGGGAACTTA
ATGTCGTTTCTCAGCAGCTGTTGGATCTGCGCCAGCAGGTTTCTGCCCTGAAG
GCTTCCTCCCCTCCCAATGCGGTTTAAAACATAAATAAAAAACCAGACTCTGT
TTGGATTTGGATCAAGCAAGTGTCTTGCTGTCTTTATTTAGGGGTTTTGCGCGC
GCGGTAGGCCCGGGACCAGCGGTCTCGGTCGTTGAGGGTCCTGTGTATTTTTT
CCAGGACGTGGTAAAGGTGACTCTGGATGTTCAGATACATGGGCATAAGCCC
GTCTCTGGGGTGGAGGTAGCACCACTGCAGAGCTTCATGCTGCGGGGTGGTG
TTGTAGATGATCCAGTCGTAGCAGGAGCGCTGGGCGTGGTGCCTAAAAATGT
 
CTTTCAGTAGCAAGCTGATTGCCAGGGGCAGGCCCTTGGTGTAAGTGTTTACA
AAGCGGTTAAGCTGGGATGGGTGCATACGTGGGGATATGAGATGCATCTTGG
ACTGTATTTTTAGGTTGGCTATGTTCCCAGCCATATCCCTCCGGGGATTCATGT
TGTGCAGAACCACCAGCACAGTGTATCCGGTGCACTTGGGAAATTTGTCATGT
AGCTTAGAAGGAAATGCGTGGAAGAACTTGGAGACGCCCTTGTGACCTCCAA
GATTTTCCATGCATTCGTCCATAATGATGGCAATGGGCCCACGGGCGGCGGCC
TGGGCGAAGATATTTCTGGGATCACTAACGTCATAGTTGTGTTCCAGGATGAG
ATCGTCATAGGCCATTTTTACAAAGCGCGGGCGGAGGGTGCCAGACTGCGGT
ATAATGGTTCCATCCGGCCCAGGGGCGTAGTTACCCTCACAGATTTGCATTTC
CCACGCTTTGAGTTCAGATGGGGGGATCATGTCTACCTGCGGGGCGATGAAG
AAAACGGTTTCCGGGGTAGGGGAGATCAGCTGGGAAGAAAGCAGGTTCCTGA
GCAGCTGCGACTTACCGCAGCCGGTGGGCCCGTAAATCACACCTATTACCGG
GTGCAACTGGTAGTTAAGAGAGCTGCAGCTGCCGTCATCCCTGAGCAGGGGG
GCCACTTCGTTAAGCATGTCCCTGACTCGCATGTTTTCCCTGACCAAATCCGC
CAGAAGGCGCTCGCCGCCCAGCGATAGCAGTTCTTGCAAGGAAGCAAAGTTT
TTCAACGGTTTGAGACCGTCCGCCGTAGGCATGCTTTTGAGCGTTTGACCAAG
CAGTTCCAGGCGGTCCCACAGCTCGGTCACCTGCTCTACGGCATCTCGATCCA
GCATATCTCCTCGTTTCGCGGGTTGGGGCGGCTTTCGCTGTACGGCAGTAGTC
GGTGCTCGTCCAGACGGGCCAGGGTCATGTCTTTCCACGGGCGCAGGGTCCTC
GTCAGCGTAGTCTGGGTCACGGTGAAGGGGTGCGCTCCGGGCTGCGCGCTGG
CCAGGGTGCGCTTGAGGCTGGTCCTGCTGGTGCTGAAGCGCTGCCGGTCTTCG
CCCTGCGCGTCGGCCAGGTAGCATTTGACCATGGTGTCATAGTCCAGCCCCTC
CGCGGCGTGGCCCTTGGCGCGCAGCTTGCCCTTGGAGGAGGCGCCGCACGAG
GGGCAGTGCAGACTTTTGAGGGCGTAGAGCTTGGGCGCGAGAAATACCGATT
CCGGGGAGTAGGCATCCGCGCCGCAGGCCCCGCAGACGGTCTCGCATTCCAC
GAGCCAGGTGAGCTCTGGCCGTTCGGGGTCAAAAACCAGGTTTCCCCCATGC
TTTTTGATGCGTTTCTTACCTCTGGTTTCCATGAGCCGGTGTCCACGCTCGGTG
ACGAAAAGGCTGTCCGTGTCCCCGTATACAGACTTGAGAGGCCTGTCCTCGA
CCGATGCCCTTGAGAGCCTTCAACCCAGTCAGCTCCTTCCGGTGGGCGCGGG
GCATGACTATCGTCGCCGCACTTATGACTGTCTTCTTTATCATGCAACTCGTA
GGACAGGTGCCGGCAGCGCTCTGGGTCATTTTCGGCGAGGACCGCTTTCGCTG
GAGCGCGACGATGATCGGCCTGTCGCTTGCGGTATTCGGAATCTTGCACGCCC
TCGCTCAAGCCTTCGTCACTGGTCCCGCCACCAAACGTTTCGGCGAGAAGCA
GGCCATTATCGCCGGCATGGCGGCCGACGCGCTGGGCTACGTCTTGCTGGCGT
TCGCGACGCGAGGCTGGATGGCCTTCCCCATTATGATTCTTCTCGCTTCCGGC
GGCATCGGGATGCCCGCGTTGCAGGCCATGCTGTCCAGGCAGGTAGATGACG
ACCATCAGGGACAGCTTCAAGGCCAGCAAAAGGCCAGGAACCGTAAAAAGG
CCGCGTTGCTGGCGTTTTTCCATAGGCTCCGCCCCCCTGACGAGCATCACAAA
AATCGACGCTCAAGTCAGAGGTGGCGAAACCCGACAGGACTATAAAGATACC
AGGCGTTTCCCCCTGGAAGCTCCCTCGTGCGCTCTCCTGTTCCGACCCTGCCG
CTTACCGGATACCTGTCCGCCTTTCTCCCTTCGGGAAGCGTGGCGCTTTCTCAT
AGCTCACGCTGTAGGTATCTCAGTTCGGTGTAGGTCGTTCGCTCCAAGCTGGG
CTGTGTGCACGAACCCCCCGTTCAGCCCGACCGCTGCGCCTTATCCGGTAACT
ATCGTCTTGAGTCCAACCCGGTAAGACACGACTTATCGCCACTGGCAGCAGC
CACTGGTAACAGGATTAGCAGAGCGAGGTATGTAGGCGGTGCTACAGAGTTC
TTGAAGTGGTGGCCTAACTACGGCTACACTAGAAGGACAGTATTTGGTATCTG
CGCTCTGCTGAAGCCAGTTACCTTCGGAAAAAGAGTTGGTAGCTCTTGATCCG
GCAAACAAACCACCGCTGGTAGCGGTGGTTTTTTTGTTTGCAAGCAGCAGATT
ACGCGCAGAAAAAAAGGATCTCAAGAAGATCCTTTGATCTTTTCTACGGGGT
CTGACGCTCAGTGGAACGAAAACTCACGTTAAGGGATTTTGGTCATGAGATT
 
ATCAAAAAGGATCTTCACCTAGATCCTTTTAAATTAAAAATGAAGTTTTAAAT
CAATCTAAAGTATATATGAGTAAACTTGGTCTGACAGTTACCAATGCTTAATC
AGTGAGGCACCTATCTCAGCGATCTGTCTATTTCGTTCATCCATAGTTGCCTG
ACTCCCCGTCGTGTAGATAACTACGATACGGGAGGGCTTACCATCTGGCCCCA
GTGCTGCAATGATACCGCGAGACCCACGCTCACCGGCTCCAGATTTATCAGC
AATAAACCAGCCAGCCGGAAGGGCCGAGCGCAGAAGTGGTCCTGCAACTTTA
TCCGCCTCCATCCAGTCTATTAATTGTTGCCGGGAAGCTAGAGTAAGTAGTTC
GCCAGTTAATAGTTTGCGCAACGTTGTTGCCATTGCTGCAGGCATCGTGGTGT
CACGCTCGTCGTTTGGTATGGCTTCATTCAGCTCCGGTTCCCAACGATCAAGG
CGAGTTACATGATCCCCCATGTTGTGCAAAAAAGCGGTTAGCTCCTTCGGTCC
TCCGATCGTTGTCAGAAGTAAGTTGGCCGCAGTGTTATCACTCATGGTTATGG
CAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAG
TTGCTCTTGCCCGGCGTCAACACGGGATAATACCGCGCCACATAGCAGAACTT
TAAAAGTGCTCATCATTGGAAAACGTTCTTCGGGGCGAAAACTCTCAAGGAT
CTTACCGCTGTTGAGATCCAGTTCGATGTAACCCACTCGTGCACCCAACTGAT
CTTCAGCATCTTTTACTTTCACCAGCGTTTCTGGGTGAGCAAAAACAGGAAGG
CAAAATGCCGCAAAAAAGGGAATAAGGGCGACACGGAAATGTTGAATACTC
ATACTCTTCCTTTTTCAATATTATTGAAGCATTTATCAGGGTTATTGTCTCATG
AGCGGATACATATTTGAATGTATTTAGAAAAATAAACAAATAGGGGTTCCGC
GCACATTTCCCCGAAAAGTGCCACCTGACGTCTAAGAAACCATTATTATCATG
ACATTAACCTATAAAAATAGGCGTATCACGAGGCCCTTTCGTCTTCAAGAA
**pAdEasy-1 Adenovirus Packaging or Helper (SEQ ID NO: 7)** 
attgtgagctcatatttgacaacgcgcatgcccccatgggccggggtgcgtcagaatgtgatgggctccagcattgatggtcgcc
ccgtcctgcccgcaaactctactaccttgacctacgagaccgtgtctggaacgccgttggagactgcagcctccgccgccgcttc
agccgctgcagccaccgcccgcgggattgtgactgactttgctttcctgagcccgcttgcaagcagtgcagcttcccgttcatcc
gcccgcgatgacaagttgacggctcttttggcacaattggattctttgacccgggaacttaatgtcgtttctcagcagctgttggatc
tgcgccagcaggtttctgccctgaaggcttcctcccctcccaatgcggtttaaaacataaataaaaaaccagactctgtttggatttg
gatcaagcaagtgtcttgctgtctttatttaggggttttgcgcgcgcggtaggcccgggaccagcggtctcggtcgttgagggtcc
tgtgtattttttccaggacgtggtaaaggtgactctggatgttcagatacatgggcataagcccgtctctggggtggaggtagcacc
actgcagagcttcatgctgcggggtggtgttgtagatgatccagtcgtagcaggagcgctgggcgtggtgcctaaaaatgtctttc
agtagcaagctgattgccaggggcaggcccttggtgtaagtgtttacaaagcggttaagctgggatgggtgcatacgtggggat
atgagatgcatcttggactgtatttttaggttggctatgttcccagccatatccctccggggattcatgttgtgcagaaccaccagca
cagtgtatccggtgcacttgggaaatttgtcatgtagcttagaaggaaatgcgtggaagaacttggagacgcccttgtgacctcca
agattttccatgcattcgtccataatgatggcaatgggcccacgggcggcggcctgggcgaagatatttctgggatcactaacgtc
atagttgtgttccaggatgagatcgtcataggccatttttacaaagcgcgggcggagggtgccagactgcggtataatggttccat
ccggcccaggggcgtagttaccctcacagatttgcatttcccacgctttgagttcagatggggggatcatgtctacctgcggggc
gatgaagaaaacggtttccggggtaggggagatcagctgggaagaaagcaggttcctgagcagctgcgacttaccgcagccg
gtgggcccgtaaatcacacctattaccggctgcaactggtagttaagagagctgcagctgccgtcatccctgagcaggggggcc
acttcgttaagcatgtccctgactcgcatgttttccctgaccaaatccgccagaaggcgctcgccgcccagcgatagcagttcttg
caaggaagcaaagtttttcaacggtttgagaccgtccgccgtaggcatgcttttgagcgtttgaccaagcagttccaggcggtccc
acagctcggtcacctgctctacggcatctcgatccagcatatctcctcgtttcgcgggttggggcggctttcgctgtacggcagta
gtcggtgctcgtccagacgggccagggtcatgtctttccacgggcgcagggtcctcgtcagcgtagtctgggtcacggtgaagg
ggtgcgctccgggctgcgcgctggccagggtgcgcttgaggctggtcctgctggtgctgaagcgctgccggtcttcgccctgc
gcgtcggccaggtagcatttgaccatggtgtcatagtccagcccctccgcggcgtggcccttggcgcgcagcttgcccttggag
gaggcgccgcacgaggggcagtgcagacttttgagggcgtagagcttgggcgcgagaaataccgattccggggagtaggca
tccgcgccgcaggccccgcagacggtctcgcattccacgagccaggtgagctctggccgttcggggtcaaaaaccaggtttcc
cccatgctttttgatgcgtttcttacctctggtttccatgagccggtgtccacgctcggtgacgaaaaggctgtccgtgtccccgtata
cagacttgagaggcctgtcctcgagcggtgttccgcggtcctcctcgtatagaaactcggaccactctgagacaaaggctcgcgt
 
ccaggccagcacgaaggaggctaagtgggaggggtagcggtcgttgtccactagggggtccactcgctccagggtgtgaaga
cacatgtcgccctcttcggcatcaaggaaggtgattggtttgtaggtgtaggccacgtgaccgggtgttcctgaaggggggctat
aaaagggggtgggggcgcgttcgtcctcactctcttccgcatcgctgtctgcgagggccagctgttggggtgagtactccctctg
aaaagcgggcatgacttctgcgctaagattgtcagtttccaaaaacgaggaggatttgatattcacctggcccgcggtgatgccttt
gagggtggccgcatccatctggtcagaaaagacaatctttttgttgtcaagcttggtggcaaacgacccgtagagggcgttggac
agcaacttggcgatggagcgcagggtttggtttttgtcgcgatcggcgcgctccttggccgcgatgtttagctgcacgtattcgcg
cgcaacgcaccgccattcgggaaagacggtggtgcgctcgtcgggcaccaggtgcacgcgccaaccgcggttgtgcagggt
gacaaggtcaacgctggtggctacctctccgcgtaggcgctcgttggtccagcagaggcggccgcccttgcgcgagcagaat
ggcggtagggggtctagctgcgtctcgtccggggggtctgcgtccacggtaaagaccccgggcagcaggcgcgcgtcgaag
tagtctatcttgcatccttgcaagtctagcgcctgctgccatgcgcgggcggcaagcgcgcgctcgtatgggttgagtgggggac
cccatggcatggggtgggtgagcgcggaggcgtacatgccgcaaatgtcgtaaacgtagaggggctctctgagtattccaaga
tatgtagggtagcatcttccaccgcggatgctggcgcgcacgtaatcgtatagttcgtgcgagggagcgaggaggtcgggacc
gaggttgctacgggcgggctgctctgctcggaagactatctgcctgaagatggcatgtgagttggatgatatggttggacgctgg
aagacgttgaagctggcgtctgtgagacctaccgcgtcacgcacgaaggaggcgtaggagtcgcgcagcttgttgaccagctc
ggcggtgacctgcacgtctagggcgcagtagtccagggtttccttgatgatgtcatacttatcctgtcccttttttttccacagctcgc
ggttgaggacaaactcttcgcggtctttccagtactcttggatcggaaacccgtcggcctccgaacggtaagagcctagcatgta
gaactggttgacggcctggtaggcgcagcatcccttttctacgggtagcgcgtatgcctgcgcggccttccggagcgaggtgtg
ggtgagcgcaaaggtgtccctgaccatgactttgaggtactggtatttgaagtcagtgtcgtcgcatccgccctgctcccagagca
aaaagtccgtgcgctttttggaacgcggatttggcagggcgaaggtgacatcgttgaagagtatctttcccgcgcgaggcataaa
gttgcgtgtgatgcggaagggtcccggcacctcggaacggttgttaattacctgggcggcgagcacgatctcgtcaaagccgtt
gatgttgtggcccacaatgtaaagttccaagaagcgcgggatgcccttgatggaaggcaattttttaagttcctcgtaggtgagctc
ttcaggggagctgagcccgtgctctgaaagggcccagtctgcaagatgagggttggaagcgacgaatgagctccacaggtca
cgggccattagcatttgcaggtggtcgcgaaaggtcctaaactggcgacctatggccattttttctggggtgatgcagtagaaggt
aagcgggtcttgttcccagcggtcccatccaaggttcgcggctaggtctcgcgcggcagtcactagaggctcatctccgccgaa
cttcatgaccagcatgaagggcacgagctgcttcccaaaggcccccatccaagtataggtctctacatcgtaggtgacaaagag
acgctcggtgcgaggatgcgagccgatcgggaagaactggatctcccgccaccaattggaggagtggctattgatgtggtgaa
agtagaagtccctgcgacgggccgaacactcgtgctggcttttgtaaaaacgtgcgcagtactggcagcggtgcacgggctgta
catcctgcacgaggttgacctgacgaccgcgcacaaggaagcagagtgggaatttgagcccctcgcctggcgggtttggctgg
tggtcttctacttcggctgcttgtccttgaccgtctggctgctcgaggggagttacggtggatcggaccaccacgccgcgcgagc
ccaaagtccagatgtccgcgcgcggcggtcggagcttgatgacaacatcgcgcagatgggagctgtccatggtctggagctcc
cgcggcgtcaggtcaggcgggagctcctgcaggtttacctcgcatagacgggtcagggcgcgggctagatccaggtgatacct
aatttccaggggctggttggtggcggcgtcgatggcttgcaagaggccgcatccccgcggcgcgactacggtaccgcgcggc
gggcggtgggccgcgggggtgtccttggatgatgcatctaaaagcggtgacgcgggcgagcccccggaggtagggggggc
tccggacccgccgggagagggggcaggggcacgtcggcgccgcgcgcgggcaggagctggtgctgcgcgcgtaggttgc
tggcgaacgcgacgacgcggcggttgatctcctgaatctggcgcctctgcgtgaagacgacgggcccggtgagcttgaacctg
aaagagagttcgacagaatcaatttcggtgtcgttgacggcggcctggcgcaaaatctcctgcacgtctcctgagttgtcttgata
ggcgatctcggccatgaactgctcgatctcttcctcctggagatctccgcgtccggctcgctccacggtggcggcgaggtcgttg
gaaatgcgggccatgagctgcgagaaggcgttgaggcctccctcgttccagacgcggctgtagaccacgcccccttcggcatc
gcgggcgcgcatgaccacctgcgcgagattgagctccacgtgccgggcgaagacggcgtagtttcgcaggcgctgaaagag
gtagttgagggtggtggcggtgtgttctgccacgaagaagtacataacccagcgtcgcaacgtggattcgttgatatcccccaag
gcctcaaggcgctccatggcctcgtagaagtccacggcgaagttgaaaaactgggagttgcgcgccgacacggttaactcctc
ctccagaagacggatgagctcggcgacagtgtcgcgcacctcgcgctcaaaggctacaggggcctcttcttcttcttcaatctcct
cttccataagggcctccccttcttcttcttctggcggcggtgggggaggggggacacggcggcgacgacggcgcaccgggag
gcggtcgacaaagcgctcgatcatctccccgcggcgacggcgcatggtctcggtgacggcgcggccgttctcgcgggggcg
cagttggaagacgccgcccgtcatgtcccggttatgggttggcggggggctgccatgcggcagggatacggcgctaacgatg
catctcaacaattgttgtgtaggtactccgccgccgagggacctgagcgagtccgcatcgaccggatcggaaaacctctcgaga
aaggcgtctaaccagtcacagtcgcaaggtaggctgagcaccgtggcgggcggcagcgggcggcggtcggggttgtttctgg
cggaggtgctgctgatgatgtaattaaagtaggcggtcttgagacggcggatggtcgacagaagcaccatgtccttgggtccgg
cctgctgaatgcgcaggcggtcggccatgccccaggcttcgttttgacatcggcgcaggtctttgtagtagtcttgcatgagccttt
ctaccggcacttcttcttctccttcctcttgtcctgcatctcttgcatctatcgctgcggcggcggcggagtttggccgtaggtggcg
 
ccctcttcctcccatgcgtgtgaccccgaagcccctcatcggctgaagcagggctaggtcggcgacaacgcgctcggctaatat
ggcctgctgcacctgcgtgagggtagactggaagtcatccatgtccacaaagcggtggtatgcgcccgtgttgatggtgtaagtg
cagttggccataacggaccagttaacggtctggtgacccggctgcgagagctcggtgtacctgagacgcgagtaagccctcga
gtcaaatacgtagtcgttgcaagtccgcaccaggtactggtatcccaccaaaaagtgcggcggcggctggcggtagaggggcc
agcgtagggtggccggggctccgggggcgagatcttccaacataaggcgatgatatccgtagatgtacctggacatccaggtg
atgccggcggcggtggtggaggcgcgcggaaagtcgcggacgcggttccagatgttgcgcagcggcaaaaagtgctccatg
gtcgggacgctctggccggtcaggcgcgcgcaatcgttgacgctctagcgtgcaaaaggagagcctgtaagcgggcactcttc
cgtggtctggtggataaattcgcaagggtatcatggcggacgaccggggttcgagccccgtatccggccgtccgccgtgatcca
tgcggttaccgcccgcgtgtcgaacccaggtgtgcgacgtcagacaacgggggagtgctccttttggcttccttccaggcgcgg
cggctgctgcgctagcttttttggccactggccgcgcgcagcgtaagcggttaggctggaaagcgaaagcattaagtggctcgc
tccctgtagccggagggttattttccaagggttgagtcgcgggacccccggttcgagtctcggaccggccggactgcggcgaa
cgggggtttgcctccccgtcatgcaagaccccgcttgcaaattcctccggaaacagggacgagccccttttttgcttttcccagat
gcatccggtgctgcggcagatgcgcccccctcctcagcagcggcaagagcaagagcagcggcagacatgcagggcaccctc
ccctcctcctaccgcgtcaggaggggcgacatccgcggttgacgcggcagcagatggtgattacgaacccccgcggcgccg
ggcccggcactacctggacttggaggagggcgagggcctggcgcggctaggagcgccctctcctgagcggcacccaaggg
tgcagctgaagcgtgatacgcgtgaggcgtacgtgccgcggcagaacctgtttcgcgaccgcgagggagaggagcccgagg
agatgcgggatcgaaagttccacgcagggcgcgagctgcggcatggcctgaatcgcgagcggttgctgcgcgaggaggactt
tgagcccgacgcgcgaaccgggattagtcccgcgcgcgcacacgtggcggccgccgacctggtaaccgcatacgagcaga
cggtgaaccaggagattaactttcaaaaaagctttaacaaccacgtgcgtacgcttgtggcgcgcgaggaggtggctataggac
tgatgcatctgtgggactttgtaagcgcgctggagcaaaacccaaatagcaagccgctcatggcgcagctgttccttatagtgca
gcacagcagggacaacgaggcattcagggatgcgctgctaaacatagtagagcccgagggccgctggctgctcgatttgataa
acatcctgcagagcatagtggtgcaggagcgcagcttgagcctggctgacaaggtggccgccatcaactattccatgcttagcct
gggcaagttttacgcccgcaagatataccataccccttacgttcccatagacaaggaggtaaagatcgaggggttctacatgcgc
atggcgctgaaggtgcttaccttgagcgacgacctgggcgtttatcgcaacgagcgcatccacaaggccgtgagcgtgagccg
gcggcgcgagctcagcgaccgcgagctgatgcacagcctgcaaagggccctggctggcacgggcagcggcgatagagag
gccgagtcctactttgacgcgggcgctgacctgcgctgggccccaagccgacgcgccctggaggcagctggggccggacct
gggctggcggtggcacccgcgcgcgctggcaacgtcggcggcgtggaggaatatgacgaggacgatgagtacgagccaga
ggacggcgagtactaagcggtgatgtttctgatcagatgatgcaagacgcaacggacccggcggtgcgggcggcgctgcaga
gccagccgtccggccttaactccacggacgactggcgccaggtcatggaccgcatcatgtcgctgactgcgcgcaatcctgac
gcgttccggcagcagccgcaggccaaccggctctccgcaattctggaagcggtggtcccggcgcgcgcaaaccccacgcac
gagaaggtgctggcgatcgtaaacgcgctggccgaaaacagggccatccggcccgacgaggccggcctggtctacgacgc
gctgcttcagcgcgtggctcgttacaacagcggcaacgtgcagaccaacctggaccggctggtgggggatgtgcgcgaggcc
gtggcgcagcgtgagcgcgcgcagcagcagggcaacctgggctccatggttgcactaaacgccttcctgagtacacagcccg
ccaacgtgccgcggggacaggaggactacaccaactttgtgagcgcactgcggctaatggtgactgagacaccgcaaagtga
ggtgtaccagtctgggccagactattttttccagaccagtagacaaggcctgcagaccgtaaacctgagccaggctttcaaaaac
ttgcaggggctgtggggggtgcgggctcccacaggcgaccgcgcgaccgtgtctagcttgctgacgcccaactcgcgcctgtt
gctgctgctaatagcgcccttcacggacagtggcagcgtgtcccgggacacatacctaggtcacttgctgacactgtaccgcga
ggccataggtcaggcgcatgtggacgagcatactttccaggagattacaagtgtcagccgcgcgctggggcaggaggacacg
ggcagcctggaggcaaccctaaactacctgctgaccaaccggcggcagaagatcccctcgttgcacagtttaaacagcgagga
ggagcgcattttgcgctacgtgcagcagagcgtgagccttaacctgatgcgcgacggggtaacgcccagcgtggcgctggac
atgaccgcgcgcaacatggaaccgggcatgtatgcctcaaaccggccgtttatcaaccgcctaatggactacttgcatcgcgcg
gccgccgtgaaccccgagtatttcaccaatgccatcttgaacccgcactggctaccgccccctggtttctacaccgggggattcg
aggtgcccgagggtaacgatggattcctctgggacgacatagacgacagcgtgttttccccgcaaccgcagaccctgctagagt
tgcaacagcgcgagcaggcagaggcggcgctgcgaaaggaaagcttccgcaggccaagcagcttgtccgatctaggcgctg
cggccccgcggtcagatgctagtagcccatttccaagcttgatagggtctcttaccagcactcgcaccacccgcccgcgcctgct
gggcgaggaggagtacctaaacaactcgctgctgcagccgcagcgcgaaaaaaacctgcctccggcatttcccaacaacggg
atagagagcctagtggacaagatgagtagatggaagacgtacgcgcaggagcacagggacgtgccaggcccgcgcccgcc
cacccgtcgtcaaaggcacgaccgtcagcggggtctggtgtgggaggacgatgactcggcagacgacagcagcgtcctggat
ttgggagggagtggcaacccgtttgcgcaccttcgccccaggctggggagaatgttttaaaaaaaaaaaagcatgatgcaaaat
aaaaaactcaccaaggccatggcaccgagcgttggttttcttgtattccccttagtatgcggcgcgcggcgatgtatgaggaaggt
 
cctcctccctcctacgagagtgtggtgagcgcggcgccagtggcggcgctgggttctcccttcgatgctcccctggacccg
ccgtttgtgcctccgcggtacctgcggcctaccggggggagaaacagcatccgttactctgagttggcacccctattcgacacca
cccgtgtgtacctggtggacaacaagtcaacggatgtggcatccctgaactaccagaacgaccacagcaactttctgaccacgg
tcattcaaaacaatgactacagcccgggggaggcaagcacacagaccatcaatcttgacgaccggtcgcactggggcggcga
cctgaaaaccatcctgcataccaacatgccaaatgtgaacgagttcatgtttaccaataagtttaaggcgcgggtgatggtgtcgc
gcttgcctactaaggacaatcaggtggagctgaaatacgagtgggtggagttcacgctgcccgagggcaactactccgagacc
atgaccatagaccttatgaacaacgcgatcgtggagcactacttgaaagtgggcagacagaacggggttctggaaagcgacat
cggggtaaagtttgacacccgcaacttcagactggggtttgaccccgtcactggtcttgtcatgcctggggtatatacaaacgaag
ccttccatccagacatcattttgctgccaggatgcggggtggacttcacccacagccgcctgagcaacttgttgggcatccgcaa
gcggcaacccttccaggagggctttaggatcacctacgatgatctggagggtggtaacattcccgcactgttggatgtggacgcc
taccaggcgagcttgaaagatgacaccgaacagggcgggggtggcgcaggcggcagcaacagcagtggcagcggcgcgg
aagagaactccaacgcggcagccgcggcaatgcagccggtggaggacatgaacgatcatgccattcgcggcgacacctttgc
cacacgggctgaggagaagcgcgctgaggccgaagcagcggccgaagctgccgcccccgctgcgcaacccgaggtcgag
aagcctcagaagaaaccggtgatcaaacccctgacagaggacagcaagaaacgcagttacaacctaataagcaatgacagca
ccttcacccagtaccgcagctggtaccttgcatacaactacggcgaccctcagaccggaatccgctcatggaccctgctttgcac
tcctgacgtaacctgcggctcggagcaggtctactggtcgttgccagacatgatgcaagaccccgtgaccttccgctccacgcg
ccagatcagcaactttccggtggtgggcgccgagctgttgcccgtgcactccaagagcttctacaacgaccaggccgtctactc
ccaactcatccgccagtttacctctctgacccacgtgttcaatcgctttcccgagaaccagattttggcgcgcccgccagccccca
ccatcaccaccgtcagtgaaaacgttcctgctctcacagatcacgggacgctaccgctgcgcaacagcatcggaggagtccag
cgagtgaccattactgacgccagacgccgcacctgcccctacgtttacaaggccctgggcatagtctcgccgcgcgtcctatcg
agccgcactttttgagcaagcatgtccatccttatatcgcccagcaataacacaggctggggcctgcgcttcccaagcaagatgtt
tggcggggccaagaagcgctccgaccaacacccagtgcgcgtgcgcgggcactaccgcgcgccctggggcgcgcacaaac
gcggccgcactgggcgcaccaccgtcgatgacgccatcgacgcggtggtggaggaggcgcgcaactacacgcccacgccg
ccaccagtgtccacagtggacgcggccattcagaccgtggtgcgcggagcccggcgctatgctaaaatgaagagacggcgg
aggcgcgtagcacgtcgccaccgccgccgacccggcactgccgcccaacgcgcggcggcggccctgcttaaccgcgcacg
tcgcaccggccgacgggcggccatgcgggccgctcgaaggctggccgcgggtattgtcactgtgccccccaggtccaggcg
acgagcggccgccgcagcagccgcggccattagtgctatgactcagggtcgcaggggcaacgtgtattgggtgcgcgactcg
gttagcggcctgcgcgtgcccgtgcgcacccgccccccgcgcaactagattgcaagaaaaaactacttagactcgtactgttgta
tgtatccagcggcggcggcgcgcaacgaagctatgtccaagcgcaaaatcaaagaagagatgctccaggtcatcgcgccgga
gatctatggccccccgaagaaggaagagcaggattacaagccccgaaagctaaagcgggtcaaaaagaaaaagaaagatga
tgatgatgaacttgacgacgaggtggaactgctgcacgctaccgcgcccaggcgacgggtacagtggaaaggtcgacgcgta
aaacgtgttttgcgacccggcaccaccgtagtctttacgcccggtgagcgctccacccgcacctacaagcgcgtgtatgatgag
gtgtacggcgacgaggacctgcttgagcaggccaacgagcgcctcggggagtttgcctacggaaagcggcataaggacatgc
tggcgttgccgctggacgagggcaacccaacacctagcctaaagcccgtaacactgcagcaggtgctgcccgcgcttgcacc
gtccgaagaaaagcgcggcctaaagcgcgagtctggtgacttggcacccaccgtgcagctgatggtacccaagcgccagcga
ctggaagatgtcttggaaaaaatgaccgtggaacctgggctggagcccgaggtccgcgtgcggccaatcaagcaggtggcgc
cgggactgggcgtgcagaccgtggacgttcagatacccactaccagtagcaccagtattgccaccgccacagagggcatgga
gacacaaacgtccccggttgcctcagcggtggcggatgccgcggtgcaggcggtcgctgcggccgcgtccaagacctctacg
gaggtgcaaacggacccgtggatgtttcgcgtttcagccccccggcgcccgcgccgttcgaggaagtacggcgccgccagcg
cgctactgcccgaatatgccctacatccttccattgcgcctacccccggctatcgtggctacacctaccgccccagaagacgagc
aactacccgacgccgaaccaccactggaacccgccgccgccgtcgccgtcgccagcccgtgctggccccgatttccgtgcgc
agggtggctcgcgaaggaggcaggaccctggtgctgccaacagcgcgctaccaccccagcatcgtttaaaagccggtctttgt
ggttcttgcagatatggccctcacctgccgcctccgtttcccggtgccgggattccgaggaagaatgcaccgtaggaggggcat
ggccggccacggcctgacgggcggcatgcgtcgtgcgcaccaccggcggcggcgcgcgtcgcaccgtcgcatgcgcggc
ggtatcctgcccctccttattccactgatcgccgcggcgattggcgccgtgcccggaattgcatccgtggccttgcaggcgcaga
gacactgattaaaaacaagttgcatgtggaaaaatcaaaataaaaagtctggactctcacgctcgcttggtcctgtaactattttgta
gaatggaagacatcaactttgcgtctctggccccgcgacacggctcgcgcccgttcatgggaaactggcaagatatcggcacca
gcaatatgagcggtggcgccttcagctggggctcgctgtggagcggcattaaaaatttcggttccaccgttaagaactatggcag
caaggcctggaacagcagcacaggccagatgctgagggataagttgaaagagcaaaatttccaacaaaaggtggtagatggc
ctggcctctggcattagcggggtggtggacctggccaaccaggcagtgcaaaataagattaacagtaagcttgatccccgccct
 
cccgtagaggagcctccaccggccgtggagacagtgtctccagaggggcgtggcgaaaagcgtccgcgccccgacaggga
agaaactctggtgacgcaaatagacgagcctccctcgtacgaggaggcactaaagcaaggcctgcccaccacccgtcccatc
gcgcccatggctaccggagtgctgggccagcacacacccgtaacgctggacctgcctccccccgccgacacccagcagaaa
cctgtgctgccaggcccgaccgccgttgttgtaacccgtcctagccgcgcgtccctgcgccgcgccgccagcggtccgcgatc
gttgcggcccgtagccagtggcaactggcaaagcacactgaacagcatcgtgggtctgggggtgcaatccctgaagcgccga
cgatgcttctgatagctaacgtgtcgtatgtgtgtcatgtatgcgtccatgtcgccgccagaggagctgctgagccgccgcgcgc
ccgctttccaagatggctaccccttcgatgatgccgcagtggtcttacatgcacatctcgggccaggacgcctcggagtacctga
gccccgggctggtgcagtttgcccgcgccaccgagacgtacttcagcctgaataacaagtttagaaaccccacggtggcgccta
cgcacgacgtgaccacagaccggtcccagcgtttgacgctgcggttcatccctgtggaccgtgaggatactgcgtactcgtaca
aggcgcggttcaccctagctgtgggtgataaccgtgtgctggacatggcttccacgtactttgacatccgcggcgtgctggacag
gggccctacttttaagccctactctggcactgcctacaacgccctggctcccaagggtgccccaaatccttgcgaatgggatgaa
gctgctactgctcttgaaataaacctagaagaagaggacgatgacaacgaagacgaagtagacgagcaagctgagcagcaaa
aaactcacgtatttgggcaggcgccttattctggtataaatattacaaaggagggtattcaaataggtgtcgaaggtcaaacaccta
aatatgccgataaaacatttcaacctgaacctcaaataggagaatctcagtggtacgaaacagaaattaatcatgcagctgggag
agtcctaaaaaagactaccccaatgaaaccatgttacggttcatatgcaaaacccacaaatgaaaatggagggcaaggcattctt
gtaaagcaacaaaatggaaagctagaaagtcaagtggaaatgcaatttttctcaactactgaggcagccgcaggcaatggtgata
acttgactcctaaagtggtattgtacagtgaagatgtagatatagaaaccccagacactcatatttcttacatgcccactattaagga
aggtaactcacgagaactaatgggccaacaatctatgcccaacaggcctaattacattgcttttagggacaattttattggtctaatg
tattacaacagcacgggtaatatgggtgttctggcgggccaagcatcgcagttgaatgctgttgtagatttgcaagacagaaacac
agagctttcataccagcttttgcttgattccattggtgatagaaccaggtacttttctatgtggaatcaggctgttgacagctatgatcc
agatgttagaattattgaaaatcatggaactgaagatgaacttccaaattactgctttccactgggaggtgtgattaatacagagact
cttaccaaggtaaaacctaaaacaggtcaggaaaatggatgggaaaaagatgctacagaattttcagataaaaatgaaataagag
ttggaaataattttgccatggaaatcaatctaaatgccaacctgtggagaaatttcctgtactccaacatagcgctgtatttgcccgac
aagctaaagtacagtccttccaacgtaaaaatttctgataacccaaacacctacgactacatgaacaagcgagtggtggctcccg
ggctagtggactgctacattaaccttggagcacgctggtcccttgactatatggacaacgtcaacccatttaaccaccaccgcaat
gctggcctgcgctaccgctcaatgttgctgggcaatggtcgctatgtgcccttccacatccaggtgcctcagaagttctttgccatt
aaaaacctccttctcctgccgggctcatacacctacgagtggaacttcaggaaggatgttaacatggttctgcagagctccctagg
aaatgacctaagggttgacggagccagcattaagtttgatagcatttgcctttacgccaccttcttccccatggcccacaacaccgc
ctccacgcttgaggccatgcttagaaacgacaccaacgaccagtcctttaacgactatctctccgccgccaacatgctctacccta
tacccgccaacgctaccaacgtgcccatatccatcccctcccgcaactgggcggctttccgcggctgggccttcacgcgccttaa
gactaaggaaaccccatcactgggctcgggctacgacccttattacacctactctggctctataccctacctagatggaacctttta
cctcaaccacacctttaagaaggtggccattacctttgactcttctgtcagctggcctggcaatgaccgcctgcttacccccaacga
gtttgaaattaagcgctcagttgacggggagggttacaacgttgcccagtgtaacatgaccaaagactggttcctggtacaaatgc
tagctaactataacattggctaccagggcttctatatcccagagagctacaaggaccgcatgtactccttctttagaaacttccagcc
catgagccgtcaggtggtggatgatactaaatacaaggactaccaacaggtgggcatcctacaccaacacaacaactctggattt
gttggctaccttgcccccaccatgcgcgaaggacaggcctaccctgctaacttcccctatccgcttataggcaagaccgcagttg
acagcattacccagaaaaagtttctttgcgatcgcaccctttggcgcatcccattctccagtaactttatgtccatgggcgcactcac
agacctgggccaaaaccttctctacgccaactccgcccacgcgctagacatgacttttgaggtggatcccatggacgagcccac
ccttctttatgttttgtttgaagtctttgacgtggtccgtgtgcaccagccgcaccgcggcgtcatcgaaaccgtgtacctgcgcacg
cccttctcggccggcaacgccacaacataaagaagcaagcaacatcaacaacagctgccgccatgggctccagtgagcagga
actgaaagccattgtcaaagatcttggttgtgggccatattttttgggcacctatgacaagcgctttccaggctttgtttctccacaca
agctcgcctgcgccatagtcaatacggccggtcgcgagactgggggcgtacactggatggcctttgcctggaacccgcactca
aaaacatgctacctctttgagccctttggcttttctgaccagcgactcaagcaggtttaccagtttgagtacgagtcactcctgcgcc
gtagcgccattgcttcttcccccgaccgctgtataacgctggaaaagtccacccaaagcgtacaggggcccaactcggccgcct
gtggactattctgctgcatgtttctccacgcctttgccaactggccccaaactcccatggatcacaaccccaccatgaaccttattac
cggggtacccaactccatgctcaacagtccccaggtacagcccaccctgcgtcgcaaccaggaacagctctacagcttcctgg
agcgccactcgccctacttccgcagccacagtgcgcagattaggagcgccacttctttttgtcacttgaaaaacatgtaaaaataat
gtactagagacactttcaataaaggcaaatgcttttatttgtacactctcgggtgattatttacccccacccttgccgtctgcgccgttt
aaaaatcaaaggggttctgccgcgcatcgctatgcgccactggcagggacacgttgcgatactggtgtttagtgctccacttaaa
ctcaggcacaaccatccgcggcagctcggtgaagttttcactccacaggctgcgcaccatcaccaacgcgtttagcaggtcggg
 
cgccgatatcttgaagtcgcagttggggcctccgccctgcgcgcgcgagttgcgatacacagggttgcagcactggaacactat
cagcgccgggtggtgcacgctggccagcacgctcttgtcggagatcagatccgcgtccaggtcctccgcgttgctcagggcga
acggagtcaactttggtagctgccttcccaaaaagggcgcgtgcccaggctttgagttgcactcgcaccgtagtggcatcaaaag
gtgaccgtgcccggtctgggcgttaggatacagcgcctgcataaaagccttgatctgcttaaaagccacctgagcctttgcgcctt
cagagaagaacatgccgcaagacttgccggaaaactgattggccggacaggccgcgtcgtgcacgcagcaccttgcgtcggt
gttggagatctgcaccacatttcggccccaccggttcttcacgatcttggccttgctagactgctccttcagcgcgcgctgcccgttt
tcgctcgtcacatccatttcaatcacgtgctccttatttatcataatgcttccgtgtagacacttaagctcgccttcgatctcagcgcag
cggtgcagccacaacgcgcagcccgtgggctcgtgatgcttgtaggtcacctctgcaaacgactgcaggtacgcctgcaggaa
tcgccccatcatcgtcacaaaggtcttgttgctggtgaaggtcagctgcaacccgcggtgctcctcgttcagccaggtcttgcata
cggccgccagagcttccacttggtcaggcagtagtttgaagttcgcctttagatcgttatccacgtggtacttgtccatcagcgcgc
gcgcagcctccatgcccttctcccacgcagacacgatcggcacactcagcgggttcatcaccgtaatttcactttccgcttcgctg
ggctcttcctcttcctcttgcgtccgcataccacgcgccactgggtcgtcttcattcagccgccgcactgtgcgcttacctcctttgc
catgcttgattagcaccggtgggttgctgaaacccaccatttgtagcgccacatcttctctttcttcctcgctgtccacgattacctctg
gtgatggcgggcgctcgggcttgggagaagggcgcttctttttcttcttgggcgcaatggccaaatccgccgccgaggtcgatg
gccgcgggctgggtgtgcgcggcaccagcgcgtcttgtgatgagtcttcctcgtcctcggactcgatacgccgcctcatccgctt
ttttgggggcgcccggggaggcggcggcgacggggacggggacgacacgtcctccatggttgggggacgtcgcgccgcac
cgcgtccgcgctcgggggtggtttcgcgctgctcctcttcccgactggccatttccttctcctataggcagaaaaagatcatggag
tcagtcgagaagaaggacagcctaaccgccccctctgagttcgccaccaccgcctccaccgatgccgccaacgcgcctacca
ccttccccgtcgaggcacccccgcttgaggaggaggaagtgattatcgagcaggacccaggttttgtaagcgaagacgacgag
gaccgctcagtaccaacagaggataaaaagcaagaccaggacaacgcagaggcaaacgaggaacaagtcgggcgggggg
acgaaaggcatggcgactacctagatgtgggagacgacgtgctgttgaagcatctgcagcgccagtgcgccattatctgcgac
gcgttgcaagagcgcagcgatgtgcccctcgccatagcggatgtcagccttgcctacgaacgccacctattctcaccgcgcgta
ccccccaaacgccaagaaaacggcacatgcgagcccaacccgcgcctcaacttctaccccgtatttgccgtgccagaggtgctt
gccacctatcacatctttttccaaaactgcaagatacccctatcctgccgtgccaaccgcagccgagcggacaagcagctggcct
tgcggcagggcgctgtcatacctgatatcgcctcgctcaacgaagtgccaaaaatctttgagggtcttggacgcgacgagaagc
gcgcggcaaacgctctgcaacaggaaaacagcgaaaatgaaagtcactctggagtgttggtggaactcgagggtgacaacgc
gcgcctagccgtactaaaacgcagcatcgaggtcacccactttgcctacccggcacttaacctaccccccaaggtcatgagcac
agtcatgagtgagctgatcgtgcgccgtgcgcagcccctggagagggatgcaaatttgcaagaacaaacagaggagggccta
cccgcagttggcgacgagcagctagcgcgctggcttcaaacgcgcgagcctgccgacttggaggagcgacgcaaactaatga
tggccgcagtgctcgttaccgtggagcttgagtgcatgcagcggttctttgctgacccggagatgcagcgcaagctagaggaaa
cattgcactacacctttcgacagggctacgtacgccaggcctgcaagatctccaacgtggagctctgcaacctggtctcctacctt
ggaattttgcacgaaaaccgccttgggcaaaacgtgcttcattccacgctcaagggcgaggcgcgccgcgactacgtccgcga
ctgcgtttacttatttctatgctacacctggcagacggccatgggcgtttggcagcagtgcttggaggagtgcaacctcaaggagc
tgcagaaactgctaaagcaaaacttgaaggacctatggacggccttcaacgagcgctccgtggccgcgcacctggcggacatc
attttccccgaacgcctgcttaaaaccctgcaacagggtctgccagacttcaccagtcaaagcatgttgcagaactttaggaacttt
atcctagagcgctcaggaatcttgcccgccacctgctgtgcacttcctagcgactttgtgcccattaagtaccgcgaatgccctcc
gccgctttggggccactgctaccttctgcagctagccaactaccttgcctaccactctgacataatggaagacgtgagcggtgac
ggtctactggagtgtcactgtcgctgcaacctatgcaccccgcaccgctccctggtttgcaattcgcagctgcttaacgaaagtca
aattatcggtacctttgagctgcagggtccctcgcctgacgaaaagtccgcggctccggggttgaaactcactccggggctgtgg
acgtcggcttaccttcgcaaatttgtacctgaggactaccacgcccacgagattaggttctacgaagaccaatcccgcccgccta
atgcggagcttaccgcctgcgtcattacccagggccacattcttggccaattgcaagccatcaacaaagcccgccaagagtttct
gctacgaaagggacggggggtttacttggacccccagtccggcgaggagctcaacccaatccccccgccgccgcagccctat
cagcagcagccgcgggcccttgcttcccaggatggcacccaaaaagaagctgcagctgccgccgccacccacggacgagg
aggaatactgggacagtcaggcagaggaggttttggacgaggaggaggaggacatgatggaagactgggagagcctagacg
aggaagcttccgaggtcgaagaggtgtcagacgaaacaccgtcaccctcggtcgcattcccctcgccggcgccccagaaatc
ggcaaccggttccagcatggctacaacctccgctcctcaggcgccgccggcactgcccgttcgccgacccaaccgtagatgg
gacaccactggaaccagggccggtaagtccaagcagccgccgccgttagcccaagagcaacaacagcgccaaggctaccg
ctcatggcgcgggcacaagaacgccatagttgcttgcttgcaagactgtgggggcaacatctccttcgcccgccgctttcttctct
accatcacggcgtggccttcccccgtaacatcctgcattactaccgtcatctctacagcccatactgcaccggcggcagcggcag
caacagcagcggccacacagaagcaaaggcgaccggatagcaagactctgacaaagcccaagaaatccacagcggcgga
 
gcagcaggaggaggagcgctgcgtctggcgcccaacgaacccgtatcgacccgcgagcttagaaacaggatttttcccactct
gtatgctatatttcaacagagcaggggccaagaacaagagctgaaaataaaaaacaggtctctgcgatccctcacccgcagctg
cctgtatcacaaaagcgaagatcagcttcggcgcacgctggaagacgcggaggctctcttcagtaaatactgcgcgctgactctt
aaggactagtttcgcgccctttctcaaatttaagcgcgaaaactacgtcatctccagcggccacacccggcgccagcacctgttgt
cagcgccattatgagcaaggaaattcccacgccctacatgtggagttaccagccacaaatgggacttgcggctggagctgccca
agactactcaacccgaataaactacatgagcgcgggaccccacatgatatcccgggtcaacggaatacgcgcccaccgaaac
cgaattctcctggaacaggcggctattaccaccacacctcgtaataaccttaatccccgtagttggcccgctgccctggtgtacca
ggaaagtcccgctcccaccactgtggtacttcccagagacgcccaggccgaagttcagatgactaactcaggggcgcagcttg
cgggcggctttcgtcacagggtgcggtcgcccgggcagggtataactcacctgacaatcagagggcgaggtattcagctcaac
gacgagtcggtgagctcctcgcttggtctccgtccggacgggacatttcagatcggcggcgccggccgctcttcattcacgcctc
gtcaggcaatcctaactctgcagacctcgtcctctgagccgcgctctggaggcattggaactctgcaatttattgaggagtttgtgc
catcggtctactttaaccccttctcgggacctcccggccactatccggatcaatttattcctaactttgacgcggtaaaggactcggc
ggacggctacgactgaatgttaagtggagaggcagagcaactgcgcctgaaacacctggtccactgtcgccgccacaagtgct
ttgcccgcgactccggtgagttttgctactttgaattgcccgaggatcatatcgagggcccggcgcacggcgtccggcttaccgc
ccagggagagcttgcccgtagcctgattcgggagtttacccagcgccccctgctagttgagcgggacaggggaccctgtgttct
cactgtgatttgcaactgtcctaaccctggattacatcaagatcctctagttaatgtcaggtcgcctaagtcgattaactagagtaccc
ggggatcttattccctttaactaataaaaaaaaataataaagcatcacttacttaaaatcagttagcaaatttctgtccagtttattcagc
agcacctccttgccctcctcccagctctggtattgcagcttcctcctggctgcaaactttctccacaatctaaatggaatgtcagtttc
ctcctgttcctgtccatccgcacccactatcttcatgttgttgcagatgaagcgcgcaagaccgtctgaagataccttcaaccccgt
gtatccatatgacacggaaaccggtcctccaactgtgccttttcttactcctccctttgtatcccccaatgggtttcaagagagtcccc
ctggggtactctctttgcgcctatccgaacctctagttacctccaatggcatgcttgcgctcaaaatgggcaacggcctctctctgg
acgaggccggcaaccttacctcccaaaatgtaaccactgtgagcccacctctcaaaaaaaccaagtcaaacataaacctggaaa
tatctgcacccctcacagttacctcagaagccctaactgtggctgccgccgcacctctaatggtcgcgggcaacacactcaccat
gcaatcacaggccccgctaaccgtgcacgactccaaacttagcattgccacccaaggacccctcacagtgtcagaaggaaagc
tagccctgcaaacatcaggccccctcaccaccaccgatagcagtacccttactatcactgcctcaccccctctaactactgccact
ggtagcttgggcattgacttgaaagagcccatttatacacaaaatggaaaactaggactaaagtacggggctcctttgcatgtaac
agacgacctaaacactttgaccgtagcaactggtccaggtgtgactattaataatacttccttgcaaactaaagttactggagccttg
ggttttgattcacaaggcaatatgcaacttaatgtagcaggaggactaaggattgattctcaaaacagacgccttatacttgatgtta
gttatccgtttgatgctcaaaaccaactaaatctaagactaggacagggccctctttttataaactcagcccacaacttggatattaac
tacaacaaaggcctttacttgtttacagcttcaaacaattccaaaaagcttgaggttaacctaagcactgccaaggggttgatgtttg
acgctacagccatagccattaatgcaggagatgggcttgaatttggttcacctaatgcaccaaacacaaatcccctcaaaacaaa
aattggccatggcctagaatttgattcaaacaaggctatggttcctaaactaggaactggccttagttttgacagcacaggtgccatt
acagtaggaaacaaaaataatgataagctaactttgtggaccacaccagctccatctcctaactgtagactaaatgcagagaaag
atgctaaactcactttggtcttaacaaaatgtggcagtcaaatacttgctacagtttcagttttggctgttaaaggcagtttggctccaa
tatctggaacagttcaaagtgctcatcttattataagatttgacgaaaatggagtgctactaaacaattccttcctggacccagaatat
tggaactttagaaatggagatcttactgaaggcacagcctatacaaacgctgttggatttatgcctaacctatcagcttatccaaaat
ctcacggtaaaactgccaaaagtaacattgtcagtcaagtttacttaaacggagacaaaactaaacctgtaacactaaccattacac
taaacggtacacaggaaacaggagacacaactccaagtgcatactctatgtcattttcatgggactggtctggccacaactacatt
aatgaaatatttgccacatcctcttacactttttcatacattgcccaagaataaagaatcgtttgtgttatgtttcaacgtgtttatttttcaa
ttgcagaaaatttcaagtcatttttcattcagtagtatagccccaccaccacatagcttatacagatcaccgtaccttaatcaaactca
cagaaccctagtattcaacctgccacctccctcccaacacacagagtacacagtcctttctccccggctggccttaaaaagcatca
tatcatgggtaacagacatattcttaggtgttatattccacacggtttcctgtcgagccaaacgctcatcagtgatattaataaactcc
ccgggcagctcacttaagttcatgtcgctgtccagctgctgagccacaggctgctgtccaacttgcggttgcttaacgggcggcg
aaggagaagtccacgcctacatgggggtagagtcataatcgtgcatcaggatagggcggtggtgctgcagcagcgcgcgaat
aaactgctgccgccgccgctccgtcctgcaggaatacaacatggcagtggtctcctcagcgatgattcgcaccgcccgcagcat
aaggcgccttgtcctccgggcacagcagcgcaccctgatctcacttaaatcagcacagtaactgcagcacagcaccacaatatt
gttcaaaatcccacagtgcaaggcgctgtatccaaagctcatggcggggaccacagaacccacgtggccatcataccacaagc
gcaggtagattaagtggcgacccctcataaacacgctggacataaacattacctcttttggcatgttgtaattcaccacctcccggt
accatataaacctctgattaaacatggcgccatccaccaccatcctaaaccagctggccaaaacctgcccgccggctatacactg
cagggaaccgggactggaacaatgacagtggagagcccaggactcgtaaccatggatcatcatgctcgtcatgatatcaatgtt
 
ggcacaacacaggcacacgtgcatacacttcctcaggattacaagctcctcccgcgttagaaccatatcccagggaacaaccca
ttcctgaatcagcgtaaatcccacactgcagggaagacctcgcacgtaactcacgttgtgcattgtcaaagtgttacattcgggca
gcagcggatgatcctccagtatggtagcgcgggtttctgtctcaaaaggaggtagacgatccctactgtacggagtgcgccgag
acaaccgagatcgtgttggtcgtagtgtcatgccaaatggaacgccggacgtagtcatatttcctgaagcaaaaccaggtgcgg
gcgtgacaaacagatctgcgtctccggtctcgccgcttagatcgctctgtgtagtagttgtagtatatccactctctcaaagcatcca
ggcgccccctggcttcgggttctatgtaaactccttcatgcgccgctgccctgataacatccaccaccgcagaataagccacacc
cagccaacctacacattcgttctgcgagtcacacacgggaggagcgggaagagctggaagaaccatgtttttttttttattccaaaa
gattatccaaaacctcaaaatgaagatctattaagtgaacgcgctcccctccggtggcgtggtcaaactctacagccaaagaaca
gataatggcatttgtaagatgttgcacaatggcttccaaaaggcaaacggccctcacgtccaagtggacgtaaaggctaaaccct
tcagggtgaatctcctctataaacattccagcaccttcaaccatgcccaaataattctcatctcgccaccttctcaatatatctctaagc
aaatcccgaatattaagtccggccattgtaaaaatctgctccagagcgccctccaccttcagcctcaagcagcgaatcatgattgc
aaaaattcaggttcctcacagacctgtataagattcaaaagcggaacattaacaaaaataccgcgatcccgtaggtcccttcgcag
ggccagctgaacataatcgtgcaggtctgcacggaccagcgcggccacttccccgccaggaaccatgacaaaagaacccaca
ctgattatgacacgcatactcggagctatgctaaccagcgtagccccgatgtaagcttgttgcatgggcggcgatataaaatgcaa
ggtgctgctcaaaaaatcaggcaaagcctcgcgcaaaaaagaaagcacatcgtagtcatgctcatgcagataaaggcaggtaa
gctccggaaccaccacagaaaaagacaccatttttctctcaaacatgtctgcgggtttctgcataaacacaaaataaaataacaaa
aaaacatttaaacattagaagcctgtcttacaacaggaaaaacaacccttataagcataagacggactacggccatgccggcgtg
accgtaaaaaaactggtcaccgtgattaaaaagcaccaccgacagctcctcggtcatgtccggagtcataatgtaagactcggta
aacacatcaggttgattcacatcggtcagtgctaaaaagcgaccgaaatagcccgggggaatacatacccgcaggcgtagaga
caacattacagcccccataggaggtataacaaaattaataggagagaaaaacacataaacacctgaaaaaccctcctgcctagg
caaaatagcaccctcccgctccagaacaacatacagcgcttccacagcggcagccataacagtcagccttaccagtaaaaaag
aaaacctattaaaaaaacaccactcgacacggcaccagctcaatcagtcacagtgtaaaaaagggccaagtgcagagcgagta
tatataggactaaaaaatgacgtaacggttaaagtccacaaaaaacacccagaaaaccgcacgcgaacctacgcccagaaacg
aaagccaaaaaacccacaacttcctcaaatcgtcacttccgttttcccacgttacgtcacttcccattttaagaaaactacaattccca
acacatacaagttactccgccctaaaacctacgtcacccgccccgttcccacgccccgcgccacgtcacaaactccaccccctc
attatcatattggcttcaatccaaaataaggtatattattgatgatgttaattaacatgcatggatcctacgtctcgaccgatgcccttga
gagccttcaacccagtcagctccttccggtgggcgcggggcatgactatcgtcgccgcacttatgactgtcttctttatcatgcaac
tcgtaggacaggtgccggcagcgctctgggtcattttcggcgaggaccgctttcgctggagcgcgacgatgatcggcctgtcgc
ttgcggtattcggaatcttgcacgccctcgctcaagccttcgtcactggtcccgccaccaaacgtttcggcgagaagcaggccatt
atcgccggcatggcggccgacgcgctgggctacgtcttgctggcgttcgcgacgcgaggctggatggccttccccattatgatt
cttctcgcttccggcggcatcgggatgcccgcgttgcaggccatgctgtccaggcaggtagatgacgaccatcagggacagctt
caaggatcgctcgcggctcttaccagcctaacttcgatcattggaccgctgatcgtcacggcgatttatgccgcctcggcgagca
catggaacgggttggcatggattgtaggcgccgccctataccttgtctgcctccccgcgttgcgtcgcggtgcatggagccggg
ccacctcgacctgaatggaagccggcggcacctcgctaacggattcaccactccaagaattggagccaatcaattcttgcggag
aactgtgaatgcgcaaaccaacccttggcagaacatatccatcgcgtccgccatctccagcagccgcacgcggcgcatctcgg
gcagcgttgggtcctggccacgggtgcgcatgatcgtgctcctgtcgttgaggacccggctaggctggcggggttgccttactg
gttagcagaatgaatcaccgatacgcgagcgaacgtgaagcgactgctgctgcaaaacgtctgcgacctgagcaacaacatga
atggtcttcggtttccgtgtttcgtaaagtctggaaacgcggaagtcagcgccctgcaccattatgttccggatctgcatcgcagga
tgctgctggctaccctgtggaacacctacatctgtattaacgaagcgctggcattgaccctgagtgatttttctctggtcccgccgca
tccataccgccagttgtttaccctcacaacgttccagtaaccgggcatgttcatcatcagtaacccgtatcgtgagcatcctctctcg
tttcatcggtatcattacccccatgaacagaaatcccccttacacggaggcatcagtgaccaaacaggaaaaaaccgcccttaac
atggcccgctttatcagaagccagacattaacgcttctggagaaactcaacgagctggacgcggatgaacaggcagacatctgt
gaatcgcttcacgaccacgctgatgagctttaccgcagctgcctcgcgcgtttcggtgatgacggtgaaaacctctgacacatgc
agctcccggagacggtcacagcttgtctgtaagcggatgccgggagcagacaagcccgtcagggcgcgtcagcgggtgttgg
cgggtgtcggggcgcagccatgacccagtcacgtagcgatagcggagtgtatactggcttaactatgcggcatcagagcagatt
gtactgagagtgcaccatatgcggtgtgaaataccgcacagatgcgtaaggagaaaataccgcatcaggcgctcttccgcttcct
cgctcactgactcgctgcgctcggtcgttcggctgcggcgagcggtatcagctcactcaaaggcggtaatacggttatccacag
aatcaggggataacgcaggaaagaacatgtgagcaaaaggccagcaaaaggccaggaaccgtaaaaaggccgcgttgctgg
cgtttttccataggctccgcccccctgacgagcatcacaaaaatcgacgctcaagtcagaggtggcgaaacccgacaggactat
aaagataccaggcgtttccccctggaagctccctcgtgcgctctcctgttccgaccctgccgcttaccggatacctgtccgcctttc
 
tcccttcgggaagcgtggcgctttctcatagctcacgctgtaggtatctcagttcggtgtaggtcgttcgctccaagctgggctgtgt
gcacgaaccccccgttcagcccgaccgctgcgccttatccggtaactatcgtcttgagtccaacccggtaagacacgacttatcg
ccactggcagcagccactggtaacaggattagcagagcgaggtatgtaggcggtgctacagagttcttgaagtggtggcctaac
tacggctacactagaaggacagtatttggtatctgcgctctgctgaagccagttaccttcggaaaaagagttggtagctcttgatcc
ggcaaacaaaccaccgctggtagcggtggtttttttgtttgcaagcagcagattacgcgcagaaaaaaaggatctcaagaagatc
ctttgatcttttctacggggtctgacgctcagtggaacgaaaactcacgttaagggattttggtcatgagattatcaaaaaggatcttc
acctagatccttttaaattaaaaatgaagttttaaatcaatctaaagtatatatgagtaaacttggtctgacagttaccaatgcttaatca
gtgaggcacctatctcagcgatctgtctatttcgttcatccatagttgcctgactccccgtcgtgtagataactacgatacgggagg
gcttaccatctggccccagtgctgcaatgataccgcgagacccacgctcaccggctccagatttatcagcaataaaccagccag
ccggaagggccgagcgcagaagtggtcctgcaactttatccgcctccatccagtctattaattgttgccgggaagctagagtaag
tagttcgccagttaatagtttgcgcaacgttgttgccattgctgcaggcatcgtggtgtcacgctcgtcgtttggtatggcttcattca
gctccggttcccaacgatcaaggcgagttacatgatcccccatgttgtgcaaaaaagcggttagctccttcggtcctccgatcgtt
gtcagaagtaagttggccgcagtgttatcactcatggttatggcagcactgcataattctcttactgtcatgccatccgtaagatgctt
ttctgtgactggtgagtactcaaccaagtcattctgagaatagtgtatgcggcgaccgagttgctcttgcccggcgtcaacacggg
ataataccgcgccacatagcagaactttaaaagtgctcatcattggaaaacgttcttcggggcgaaaactctcaaggatcttaccg
ctgttgagatccagttcgatgtaacccactcgtgcacccaactgatcttcagcatcttttactttcaccagcgtttctgggtgagcaaa
aacaggaaggcaaaatgccgcaaaaaagggaataagggcgacacggaaatgttgaatactcatactcttcctttttcaatattatt
gaagcatttatcagggttattgtctcatgagcggatacatatttgaatgtatttagaaaaataaacaaataggggttccgcgcacattt
ccccgaaaagtgccacctgtctagctacgaattcttcgacagcttcgaaactagaatcgatttcgaaactagcttaagggtgggaa
agaatatataaggtgggggtcttatgtagttttgtatctgttttgcagcagccgccgccgccatgagcaccaactcgtttgatggaa
gc
**pAD_Track8509 Adenovirus Packaging or Helper (SEQ ID NO: 8)** 
ttaattaannntcccttccagctctctgccccttttggattgaagccaatatgataatgagggggtggagtttgtgacgtggcgcgg
ggcgtgggaacggggcgggtgacgtagtagtgtggcggaagtgtgatgttgcaagtgtggcggaacacatgtaagcgacgga
tgtggcaaaagtgacgtttttggtgtgcgccggtgtacacaggaagtgacaattttcgcgcggttttaggcggatgttgtagtaaatt
tgggcgtaaccgagtaagatttggccattttcgcgggaaaactgaataagaggaagtgaaatctgaataattttgtgttactcatag
cgcgtaannncgcgttaagatacattgatgagtttggacaaaccacaactagaatgcagtgaaaaaaatgctttatttgtgaaattt
gtgatgctattgctttatttgtaaccattataagctgcaataaacaagttaacaacaacaattgcattcattttatgtttcaggttcaggg
ggaggtgtgggaggttttttaaagcaagtaaaacctctacaaatgtggtatggctgattatgatcagttatctagatccggtggatct
gagtccggacttgtacagctcgtccatgccgagagtgatcccggcggcggtcacgaactccagcaggaccatgtgatcgcgctt
ctcgttggggtctttgctcagggcggactgggtgctcaggtagtggttgtcgggcagcagcacggggccgtcgccgatggggg
tgttctgctggtagtggtcggcgagctgcacgctgccgtcctcgatgttgtggcggatcttgaagttcaccttgatgccgttcttctg
cttgtcggccatgatatagacgttgtggctgttgtagttgtactccagcttgtgccccaggatgttgccgtcctccttgaagtcgatgc
ccttcagctcgatgcggttcaccagggtgtcgccctcgaacttcacctcggcgcgggtcttgtagttgccgtcgtccttgaagaag
atggtgcgctcctggacgtagccttcgggcatggcggacttgaagaagtcgtgctgcttcatgtggtcggggtagcggctgaag
cactgcacgccgtaggtcagggtggtcacgagggtgggccagggcacgggcagcttgccggtggtgcagatgaacttcagg
gtcagcttgccgtaggtggcatcgccctcgccctcgccggacacgctgaacttgtggccgtttacgtcgccgtccagctcgacca
ggatgggcaccaccccggtgaacagctcctcgcccttgctcaccatggtggcgaccggtagcgctagcggatctgacggttca
ctaaaccagctctgcttatatagacctcccaccgtacacgcctaccgcccatttgcgtcaatggggcggagttgttacgacattttg
gaaagtcccgttgattttggtgccaaaacaaactcccattgacgtcaatggggtggagacttggaaatccccgtgagtcaaaccg
ctatccacgcccattgatgtactgccaaaaccgcatcaccatggtaatagcgatgactaatacgtagatgtactgccaagtaggaa
agtcccataaggtcatgtactgggcataatgccaggcgggccatttaccgtcattgacgtcaatagggggcgtacttggcatatga
tacacttgatgtactgccaagtgggcagtttaccgtaaatactccacccattgacgtcaatggaaagtccctattggcgttactatgg
gaacatacgtcattattgacgtcaatgggcgggggtcgttgggcggtcagccaggcgggccatttaccgtaagttatgtaacgcg
gaactccatatatgggctatgaactaatgaccccgtaattgattactattannnctagcagatctggtaccgtcgacgcggccgcg
atatcctcgagaagctttctagagnnntaagggtgggaaagaatatataaggtgggggtcttatgtagttttgtatctgttttgcagc
agccgccgccgccatgagcaccaactcgtttgatggaagcattgtgagctcatatttgacaacgcgcatgcccccatgggccgg
ggtgcgtcagaatgtgatgggctccagcattgatggtcgccccgtcctgcccgcaaactctactaccttgacctacgagaccgtg
tctggaacgccgttggagactgcagcctccgccgccgcttcagccgctgcagccaccgcccgcgggattgtgactgactttgct
 
ttcctgagcccgcttgcaagcagtgcagcttcccgttcatccgcccgcgatgacaagttgacggctcttttggcacaattggattctt
tgacccgggaacttaatgtcgtttctcagcagctgttggatctgcgccagcaggtttctgccctgaaggcttcctcccctcccaatg
cggtttaaaacataaataaaaaaccagactctgtttggatttggatcaagcaagtgtcttgctgtctttatttaggggttttgcgcgcg
cggtaggcccgggaccagcggtctcggtcgttgagggtcctgtgtattttttccaggacgtggtaaaggtgactctggatgttcag
atacatgggcataagcccgtctctggggtggaggtagcaccactgcagagcttcatgctgcggggtggtgttgtagatgatccag
tcgtagcaggagcgctgggcgtggtgcctaaaaatgtctttcagtagcaagctgattgccaggggcaggcccttggtgtaagtgt
ttacaaagcggttaagctgggatgggtgcatacgtggggatatgagatgcatcttggactgtatttttaggttggctatgttcccagc
catatccctccggggattcatgttgtgcagaaccaccagcacagtgtatccggtgcacttgggaaatttgtcatgtagcttagaagg
aaatgcgtggaagaacttggagacgcccttgtgacctccaagattttccatgcattcgtccataatgatggcaatgggcccacgg
gcggcggcctgggcgaagatatttctgggatcactaacgtcatagttgtgttccaggatgagatcgtcataggccatttttacaaag
cgcgggcggagggtgccagactgcggtataatggttccatccggcccaggggcgtagttaccctcacagatttgcatttcccac
gctttgagttcagatggggggatcatgtctacctgcggggcgatgaagaaaacggtttccggggtaggggagatcagctggga
agaaagcaggttcctgagcagctgcgacttaccgcagccggtgggcccgtaaatcacacctattaccgggtgcaactggtagtt
aagagagctgcagctgccgtcatccctgagcaggggggccacttcgttaagcatgtccctgactcgcatgttttccctgaccaaat
ccgccagaaggcgctcgccgcccagcgatagcagttcttgcaaggaagcaaagtttttcaacggtttgagaccgtccgccgtag
gcatgcttttgagcgtttgaccaagcagttccaggcggtcccacagctcggtcacctgctctacggcatctcgatccagcatatctc
ctcgtttcgcgggttggggcggctttcgctgtacggcagtagtcggtgctcgtccagacgggccagggtcatgtctttccacggg
cgcagggtcctcgtcagcgtagtctgggtcacggtgaaggggtgcgctccgggctgcgcgctggccagggtgcgcttgaggc
tggtcctgctggtgctgaagcgctgccggtcttcgccctgcgcgtcggccaggtagcatttgaccatggtgtcatagtccagccc
ctccgcggcgtggcccttggcgcgcagcttgcccttggaggaggcgccgcacgaggggcagtgcagacttttgagggcgtag
agcttgggcgcgagaaataccgattccggggagtaggcatccgcgccgcaggccccgcagacggtctcgcattccacgagcc
aggtgagctctggccgttcggggtcaaaaaccaggtttcccccatgctttttgatgcgtttcttacctctggtttccatgagccggtgt
ccacgctcggtgacgaaaaggctgtccgtgtccccgtatacagactnnngtttaaacgaattcnnntataaaatgcaaggtgctg
ctcaaaaaatcaggcaaagcctcgcgcaaaaaagaaagcacatcgtagtcatgctcatgcagataaaggcaggtaagctccgg
aaccaccacagaaaaagacaccatttttctctcaaacatgtctgcgggtttctgcataaacacaaaataaaataacaaaaaaacatt
taaacattagaagcctgtcttacaacaggaaaaacaacccttataagcataagacggactacggccatgccggcgtgaccgtaa
aaaaactggtcaccgtgattaaaaagcaccaccgacagctcctcggtcatgtccggagtcataatgtaagactcggtaaacacat
caggttgattcatcggtcagtgctaaaaagcgaccgaaatagcccgggggaatacatacccgcaggcgtagagacaacattac
agcccccataggaggtataacaaaattaataggagagaaaaacacataaacacctgaaaaaccctcctgcctaggcaaaatagc
accctcccgctccagaacaacatacagcgcttcacagcggcagcctaacagtcagccttaccagtaaaaaagaaaacctattaa
aaaaacaccactcgacacggcaccagctcaatcagtcacagtgtaaaaaagggccaagtgcagagcgagtatatataggacta
aaaaatgacgtaacggttaaagtccacaaaaaacacccagaaaaccgcacgcgaacctacgcccagaaacgaaagccaaaa
aacccacaacttcctcaaatcgtcacttccgttttcccacgttacgtaacttcccattttaagaaaactacaattcccaacacatacaa
gttactccgccctaaaacctacgtcacccgccccgttcccacgccccgcgccacgtcacaaactccaccccctcattatcatattg
gcttcaatccaaaataaggtatattattgatgatnnnttaattaaggatccnnncggtgtgaaataccgcacagatgcgtaaggag
aaaataccgcatcaggcgctcttccgcttcctcgctcactgactcgctgcgctcggtcgttcggctgcggcgagcggtatcagct
cactcaaaggcggtaatacggttatccacagaatcaggggataacgcaggaaagaacatgtgagcaaaaggccagcaaaagg
ccaggaaccgtaaaaaggccgcgttgctggcgtttttccataggctccgcccccctgacgagcatcacaaaaatcgacgctcaa
gtcagaggtggcgaaacccgacaggactataaagataccaggcgtttccccctggaagctccctcgtgcgctctcctgttccga
ccctgccgcttaccggatacctgtccgcctttctcccttcgggaagcgtggcgctttctcatagctcacgctgtaggtatctcagttc
ggtgtaggtcgttcgctccaagctgggctgtgtgcacgaaccccccgttcagcccgaccgctgcgccttatccggtaactatcgt
cttgagtccaacccggtaagacacgacttatcgccactggcagcagccactggtaacaggattagcagagcgaggtatgtagg
cggtgctacagagttcttgaagtggtggcctaactacggctacactagaaggacagtatttggtatctgcgctctgctgaagccag
ttaccttcggaaaaagagttggtagctcttgatccggcaaacaaaccaccgctggtagcggtggtttttttgtttgcaagcagcaga
ttacgcgcagaaaaaaaggatctcaagaagatcctttgatcttttctacggggtctgacgctcagtggaacgaaaactcacgttaa
gggattttggtcatgagattatcaaaaaggatcttcacctagatccttttaaattaaaaatgaagttttaaatcaatctaaagtatatatg
agtaaacttggtctgacagttaccaatgcttaatcagtgaggcacctatctcagcgatctgtctatttcgttcatccatagttgcctgac
tccccgtcgtgtagataactacgatacgggagggcttaccatctggccccagtgctgcaatgataccgcgagacccacgctcac
cggctccagatttatcagcaataaaccagccagccggaagggccgagcgcagaagtggtcctgcaactttatccgcctccatcc
agtctattaattgttgccgggaagctagagtaagtagttcgccagttaatagtttgcgcaacgttgttgnnnnnnaaaaaggatctt
 
cacctagatccttttcacgtagaaagccagtccgcagaaacggtgctgaccccggatgaatgtcagctactgggctatctggaca
agggaaaacgcaagcgcaaagagaaagcaggtagcttgcagtgggcttacatggcgatagctagactgggcggttttatggac
agcaagcgaaccggaattgccagctggggcgccctctggtaaggttgggaagccctgcaaagtaaactggatggctttctcgc
cgccaaggatctgatggcgcaggggatcaagctctgatcaagagacaggatgaggatcgtttcgcatgattgaacaagatggat
tgcacgcaggttctccggccgcttgggtggagaggctattcggctatgactgggcacaacagacaatcggctgctctgatgccg
ccgtgttccggctgtcagcgcaggggcgcccggttctttttgtcaagaccgacctgtccggtgccctgaatgaactgcaagacga
ggcagcgcggctatcgtggctggccacgacgggcgttccttgcgcagctgtgctcgacgttgtcactgaagcgggaagggact
ggctgctattgggcgaagtgccggggcaggatctcctgtcatctcaccttgctcctgccgagaaagtatccatcatggctgatgca
atgcggcggctgcatacgcttgatccggctacctgcccattcgaccaccaagcgaaacatcgcatcgagcgagcacgtactcg
gatggaagccggtcttgtcgatcaggatgatctggacgaagagcatcaggggctcgcgccagccgaactgttcgccaggctca
aggcgagcatgcccgacggcgaggatctcgtcgtgacccatggcgatgcctgcttgccgaatatcatggtggaaaatggccgc
ttttctggattcatcgactgtggccggctgggtgtggcggaccgctatcaggacatagcgttggctacccgtgatattgctgaaga
gcttggcggcgaatgggctgaccgcttcctcgtgctttacggtatcgccgctcccgattcgcagcgcatcgccttctatcgccttct
tgacgagttcttctgaattttgttaaaatttttgttaaatcagctcattttttaaccaataggccgaaatcggcaacatcccttataaatca
aaagaatagaccgcgatagggttgagtgttgttccagtttggaacaagagtccactattaaagaacgtggactccaacgtcaaag
ggcgaaaaaccgtctatcagggcgatggcccactacgtgaaccatcacccaaatcaagttttttgcggtcgaggtgccgtaaag
ctctaaatcggaaccctaaagggagcccccgatttagagcttgacggggaaagccggcgaacgtggcgagaaaggaaggga
agaaagcgaaaggagcgggcgctagggcgctggcaagtgtagcggtcacgctgcgcgtaaccaccacacccgcgcgcttaa
tgcgccg
**pAdTrack-CMV Sequence190476 Adenovirus Packaging or Helper (SEQ ID NO: 9)** 
tgttaacttgtttattgcagcttataatggttacaaataaagcaatagcatcacaaatttcacaaataaagcatttttttcactgcattcta
gttgtggtttgtccaaactcatcaatgtatcttaacgcggaggtttatcgacgatctgctagtgattaatagtaatcaattacggggtc
attagttcatagcccatatatggagttccgcgttacataacttacggtaaatggcccgcctggctgaccgcccaacgacccccgcc
cattgacgtcaataatgacgtatgttcccatagtaacgccaatagggactttccattgacgtcaatgggtggagtatttacggtaaac
tgcccacttggcagtacatcaagtgtatcatatgccaagtacgccccctattgacgtcaatgacggtaaatggcccgcctggcatt
atgcccagtacatgaccttatgggactttcctacttggcagtacatctacgtattagtcatcgctattaccatggtgatgcggttttggc
agtacatcaatgggcgtggatagcggtttgactcacggggatttccaagtctccaccccattgacgtcaatgggagtttgttttggc
accaaaatcaacgggactttccaaaatgtcgtaacaactccgccccattgacgcaaatgggcggtaggcgtgtacggtgggag
gtctatataagcagagctggtttagtgaaccgtcagatccgctagcgctaccggtcgccaccatggtgagcaagggcgaggag
ctgttcaccggggtggtgcccatcctggtcgagctggacggcgacgtaaacggccacaagttcagcgtgtccggcgagggcg
agggcgatgccacctacggcaagctgaccctgaagttcatctgcaccaccggcaagctgcccgtgccctggcccaccctcgtg
accaccctgacctacggcgtgcagtgcttcagccgctaccccgaccacatgaagcagcacgacttcttcaagtccgccatgccc
gaaggctacgtccaggagcgcaccatcttcttcaaggacgacggcaactacaagacccgcgccgaggtgaagttcgagggcg
acaccctggtgaaccgcatcgagctgaagggcatcgacttcaaggaggacggcaacatcctggggcacaagctggagtacaa
ctacaacagccacaacgtctatatcatggccgacaagcagaagaacggcatcaaggtgaacttcaagatccgccacaacatcg
aggacggcagcgtgcagctcgccgaccactaccagcagaacacccccatcggcgacggccccgtgctgctgcccgacaacc
actacctgagcacccagtccgccctgagcaaagaccccaacgagaagcgcgatcacatggtcctgctggagttcgtgaccgcc
gccgggatcactctcggcatggacgagctgtacaagtccggactcagatccaccggatctagataactgatcataatcagccata
ccacatttgtagaggttttacttgctttaaaaaacctcccacacctccccctgaacctgaaacataaaatgaatgcaattgttgttgtta
acttgtttattgcagcttataatggttacaaataaagcaatagcatcacaaatttcacaaataaagcatttttttcactgcattctagttgt
ggtttgtccaaactcatcaatgtatcttaacgcgatcaagctagcttgctagactcgactgactataataataaaacgccaactttga
cccggaacgcggaaaacacctgagaaaaacacctgggcgagtctccacgtaaacggtcaaagtccccgcggccctagacaa
atattacgcgctatgagtaacacaaaattattcagatttcacttcctcttattcagttttcccgcgaaaatggccaaatcttactcggtta
cgcccaaatttactacaacatccgcctaaaaccgcgcgaaaattgtcacttcctgtgtacaccggcgcacaccaaaaacgtcactt
ttgccacatccgtcgcttacatgtgttccgccacacttgcaacatcacacttccgccacactactacgtcacccgccccgttcccac
gccccgcgccacgtcacaaactccaccccctcattatcatattggcttcaatccaaaataaggtatattattgatgatgttaattaaga
attaattcgatcctgaatggcgaatggacgcgccctgtagcggcgcattaagcgcggcgggtgtggtggttacgcgcagcgtga
ccgctacacttgccagcgccctagcgcccgctcctttcgctttcttcccttcctttctcgccacgttcgccggctttccccgtcaagct
 
ctaaatcgggggctccctttagggttccgatttagtgctttacggcacctcgaccccaaaaaacttgattagggtgatggttcacgt
agtgggccatcgccctgatagacggtttttcgccctttgacgttggagtccacgttctttaatagtggactcttgttccaaactggaac
aacactcaaccctatctcggtctattcttttgatttataagggattttgccgatttcggcctattggttaaaaaatgagctgatttaacaa
aaattttaacaaaattcagaagaactcgtcaagaaggcgatagaaggcgatgcgctgcgaatcgggagcggcgataccgtaaa
gcacgaggaagcggtcagcccattcgccgccaagctcttcagcaatatcacgggtagccaacgctatgtcctgatagcggtccg
ccacacccagccggccacagtcgatgaatccagaaaagcggccattttccaccatgatattcggcaagcaggcatcgccatgg
gtcacgacgagatcctcgccgtcgggcatgctcgccttgagcctggcgaacagttcggctggcgcgagcccctgatgctcttcg
tccagatcatcctgatcgacaagaccggcttccatccgagtacgtgctcgctcgatgcgatgtttcgcttggtggtcgaatgggca
ggtagccggatcaagcgtatgcagccgccgcattgcatcagccatgatggatactttctcggcaggagcaaggtgagatgaca
ggagatcctgccccggcacttcgcccaatagcagccagtcccttcccgcttcagtgacaacgtcgagcacagctgcgcaagga
acgcccgtcgtggccagccacgatagccgcgctgcctcgtcttgcagttcattcagggcaccggacaggtcggtcttgacaaaa
agaaccgggcgcccctgcgctgacagccggaacacggcggcatcagagcagccgattgtctgttgtgcccagtcatagccga
atagcctctccacccaagcggccggagaacctgcgtgcaatccatcttgttcaatcatgcgaaacgatcctcatcctgtctcttgat
cagagcttgatcccctgcgccatcagatccttggcggcaagaaagccatccagtttactttgcagggcttcccaaccttaccagag
ggcgccccagctggcaattccggttcgcttgctgtccataaaaccgcccagtctagctatcgccatgtaagcccactgcaagcta
cctgctttctctttgcgcttgcgttttcccttgtccagatagcccagtagctgacattcatccggggtcagcaccgtttctgcggactg
gctttctacgtgaaaaggatctaggtgaagatcctttttgataatctcatggctgcagcaatggcaacaacgttgcgcaaactattaa
ctggcgaactacttactctagcttcccggcaacaattaatagactggatggaggcggataaagttgcaggaccacttctgcgctcg
gcccttccggctggctggtttattgctgataaatctggagccggtgagcgtgggtctcgcggtatcattgcagcactggggccag
atggtaagccctcccgtatcgtagttatctacacgacggggagtcaggcaactatggatgaacgaaatagacagatcgctgagat
aggtgcctcactgattaagcattggtaactgtcagaccaagtttactcatatatactttagattgatttaaaacttcatttttaatttaaaa
ggatctaggtgaagatcctttttgataatctcatgaccaaaatcccttaacgtgagttttcgttccactgagcgtcagaccccgtaga
aaagatcaaaggatcttcttgagatcctttttttctgcgcgtaatctgctgcttgcaaacaaaaaaaccaccgctaccagcggtggtt
tgtttgccggatcaagagctaccaactctttttccgaaggtaactggcttcagcagagcgcagataccaaatactgtccttctagtgt
agccgtagttaggccaccacttcaagaactctgtagcaccgcctacatacctcgctctgctaatcctgttaccagtggctgctgcca
gtggcgataagtcgtgtcttaccgggttggactcaagacgatagttaccggataaggcgcagcggtcgggctgaacggggggt
tcgtgcacacagcccagcttggagcgaacgacctacaccgaactgagatacctacagcgtgagctatgagaaagcgccacgct
tcccgaagggagaaaggcggacaggtatccggtaagcggcagggtcggaacaggagagcgcacgagggagcttccaggg
ggaaacgcctggtatctttatagtcctgtcgggtttcgccacctctgacttgagcgtcgatttttgtgatgctcgtcaggggggcgg
agcctatggaaaaacgccagcaacgcggcctttttacggttcctggccttttgctggccttttgctcacatgttctttcctgcgttatcc
cctgattctgtggataaccgtattaccgcctttgagtgagctgataccgctcgccgcagccgaacgaccgagcgcagcgagtca
gtgagcgaggaagcggaagagcgcctgatgcggtattttctccttacgcatctgtgcggtatttcacaccgcatatggatccatgc
atgttaattaacatcatcaataatataccttattttggattgaagccaatatgataatgagggggtggagtttgtgacgtggcgcggg
gcgtgggaacggggcgggtgacgtaggttttagggcggagtaacttgtatgtgttgggaattgtagttttcttaaaatgggaagtg
acgtaacgtgggaaaacggaagtgacgatttgaggaagttgtgggttttttggctttcgtttctgggcgtaggttcgcgtgcggtttt
ctgggtgttttttgtggactttaaccgttacgtcattttttagtcctatatatactcgctctgcacttggcccttttttacactgtgactgatt
gagctggtgccgtgtcgagtggtgtttttttaataggttttcttttttactggtaaggctgactgttatggctgccgctgtggaagcgct
gtatgttgttctggagcgggagggtgctattttgcctaggcaggagggtttttcaggtgtttatgtgtttttctctcctattaattttgttat
acctcctatgggggctgtaatgttgtctctacgcctgcgggtatgtattcccccgggctatttcggtcgctttttagcactgaccgatg
tgaatcaacctgatgtgtttaccgagtcttacattatgactccggacatgaccgaggagctgtcggtggtgctttttaatcacggtga
ccagtttttttacggtcacgccggcatggccgtagtccgtcttatgcttataagggttgtttttcctgttgtaagacaggcttctaatgttt
aaatgtttttttgttattttattttgtgtttatgcagaaacccgcagacatgtttgagagaaaaatggtgtctttttctgtggtggttccgga
gcttacctgcctttatctgcatgagcatgactacgatgtgctttcttttttgcgcgaggctttgcctgattttttgagcagcaccttgcatt
ttatatcgccgcccatgcaacaagctattgaattcgtttaaactccctctcaagtctgtatacggggacacggacagccttttcgtca
ccgagcgtggacaccggctcatggaaaccagaggtaagaaacgcatcaaaaagcatgggggaaacctggtttttgaccccga
acggccagagctcacctggctcgtggaatgcgagaccgtctgcggggcctgcggcgcggatgcctactccccggaatcggta
tttctcgcgcccaagctctacgccctcaaaagtctgcactgcccctcgtgcggcgcctcctccaagggcaagctgcgcgccaag
ggccacgccgcggaggggctggactatgacaccatggtcaaatgctacctggccgacgcgcagggcgaagaccggcagcg
cttcagcaccagcaggaccagcctcaagcgcaccctggccagcgcgcagcccggagcgcaccccttcaccgtgacccagac
tacgctgacgaggaccctgcgcccgtggaaagacatgaccctggcccgtctggacgagcaccgactactgccgtacagcgaa
 
agccgccccaacccgcgaaacgaggagatatgctggatcgagatgccgtagagcaggtgaccgagctgtgggaccgcctgg
aactgcttggtcaaacgctcaaaagcatgcctacggcggacggtctcaaaccgttgaaaaactttgcttccttgcaagaactgcta
tcgctgggcggcgagcgccttctggcggatttggtcagggaaaacatgcgagtcagggacatgcttaacgaagtggcccccct
gctcagggatgacggcagctgcagctctcttaactaccagttgcagccggtaataggtgtgatttacgggcccaccggctgcggt
aagtcgcagctgctcaggaacctgctttcttcccagctgatctcccctaccccggaaaccgttttcttcatcgccccgcaggtagac
atgatccccccatctgaactcaaagcgtgggaaatgcaaatctgtgagggtaactacgcccctgggccggatggaaccattatac
cgcagtctggcaccctccgcccgcgctttgtaaaaatggcctatgacgatctcatcctggaacacaactatgacgttagtgatccc
agaaatatcttcgcccaggccgccgcccgtgggcccattgccatcattatggacgaatgcatggaaaatcttggaggtcacaag
ggcgtctccaagttcttccacgcatttccttctaagctacatgacaaatttcccaagtgcaccggatacactgtgctggtggttctgc
acaacatgaatccccggagggatatggctgggaacatagccaacctaaaaatacagtccaagatgcatctcatatccccacgtat
gcacccatcccagcttaaccgctttgtaaacacttacaccaagggcctgcccctggcaatcagcttgctactgaaagacatttttag
gcaccacgcccagcgctcctgctacgactggatcatctacaacaccaccccgcagcatgaagctctgcagtggtgctacctcca
ccccagagacgggcttatgcccatgtatctgaacatccagagtcacctttaccacgtcctggaaaaaatacacaggaccctcaac
gaccgagaccgctggtcccgggcctaccgcgcgcgcaaaacccctaaataaagacagcaagacacttgcttgatccaaatcca
aacagagtctggttttttatttatgttttaaaccgcattgggaggggaggaagccttcagggcagaaacctgctggcgcagatcca
acagctgctgagaaacgacattaagttcccgggtcaaagaatccaattgtgccaaaagagccgtcaacttgtcatcgcgggcgg
atgaacgggaagctgcactgcttgcaagcgggctcaggaaagcaaagtcagtcacaatcccgcgggcggtggctgcagcgg
ctgaagcggcggcggaggctgcagtctccaacggcgttccagacacggtctcgtaggtcaaggtagtagagtttgcgggcag
gacggggcgaccatcaatgctggagcccatcacattctgacgcaccccggcccatgggggcatgcgcgttgtcaaatatgagc
tcacaatgcttccatcaaacgagttggtgctcatggcggcggcggctgctgcaaaacagatacaaaactacataagacccccac
cttatatattctttcccacccttaaccacgcccagatcctctagcagtgataaacgtctaatagtaatcaattacggggtcattagttca
tagcccatatatggagttccgcgttacataacttacggtaaatggcccgcctggctgaccgcccaacgacccccgcccattgacg
tcaataatgacgtatgttcccatagtaacgccaatagggactttccattgacgtcaatgggtggagtatttacggtaaactgcccact
tggcagtacatcaagtgtatcatatgccaagtacgccccctattgacgtcaatgacggtaaatggcccgcctggcattatgcccag
tacatgaccttatgggactttcctacttggcagtacatctacgtattagtcatcgctattaccatggtgatgcggttttggcagtacatc
aatgggcgtggatagcggtttgactcacggggatttccaagtctccaccccattgacgtcaatgggagtttgttttggcaccaaaat
caacgggactttccaaaatgtcgtaacaactccgccccattgacgcaaatgggcggtaggcgtgtacggtgggaggtctatataa
gcagagctggtttagtgaaccgtcagatccgctagagatctggtaccgtcgacgcggccgctcgagcctaagcttctagataag
atatccgatccaccggatctagataactgatcataatcagccataccacatttgtagaggttttacttgctttaaaaaacctcccacac
ctccccctgaacctgaaacataaaatgaatgcaattgttgt
**pZB3 AAV dSaCas9 One Vector (SEQ ID NO: 10)** 
aaaacctctgacacatgcagctcccggagacggtcacagcttgtctgtaagcggatgccgggagcagacaagcccgtcaggg
cgcgtcagcgggtgttggcgggtgtcggggctggcttaactatgcggcatcagagcagattgtactgagagtgcaccataaaatt
gtaaacgttaatattttgttaaaattcgcgttaaatttttgttaaatcagctcattttttaaccaataggccgaaatcggcaaaatcccttat
aaatcaaaagaatagcccgagatagagttgagtgttgttccagtttggaacaagagtccactattaaagaacgtggactccaacgt
caaagggcgaaaaaccgtctatcagggcgatggcccactacgtgaaccatcacccaaatcaagttttttggggtcgaggtgccg
taaagcactaaatcggaaccctaaagggagcccccgatttagagcttgacggggaaagccggcgaacgtggcgagaaagga
agggaagaaagcgaaaggagcgggcgctaaggcgctggcaagtgtagcggtcacgctgcgcgtaaccaccacacccgccg
cgcttaatgcgccgctacagggcgcgtactatggttgctttgacgtatgcggtgtgaaataccgcacagatgcgtaaggagaaaa
taccgcatcaggcgcccctgcaggcagctgcgcgctcgctcgctcactgaggccgcccgggcaaagcccgggcgtcgggcg
acctttggtcgcccggcctcagtgagcgagcgagcgcgcagagagggagtggccaactccatcactaggggttcctgcggcc
gcgagggcctatttcccatgattccttcatatttgcatatacgatacaaggctgttagagagataattggaattaatttgactgtaaac
acaaagatattagtacaaaatacgtgacgtagaaagtaataatttcttgggtagtttgcagttttaaaattatgttttaaaatggactatc
atatgcttaccgtaacttgaaagtatttcgatttcttggctttatatatcttgtggaaaggacgaaacaccgggagaccacggcaggt
ctcagttttagtactctggaggccAACATGAGGATCACCCATGTCTGCAGggccaacagaatctactaaa
acaaggcaaaatgccgtgtttatctcgtcaacttgttggcgagatttttttggccgcctcgaggtacttatataagggggtgggggc
gcgttcgtcctcagtcgcgatcgaacactcgagccgagcagacgtgcctacggaccgaccggtgccaccatggccccaaaga
agaagcggaaggtcggtatccacggagtcccagcagccaagcggaactacatcctgggcctggccatcggcatcaccagcgt
gggctacggcatcatcgactacgagacacgggacgtgatcgatgccggcgtgcggctgttcaaagaggccaacgtggaaaac
 
aacgagggcaggcggagcaagagaggcgccagaaggctgaagcggcggaggcggcatagaatccagagagtgaagaag
ctgctgttcgactacaacctgctgaccgaccacagcgagctgagcggcatcaacccctacgaggccagagtgaagggcctga
gccagaagctgagcgaggaagagttctctgccgccctgctgcacctggccaagagaagaggcgtgcacaacgtgaacgagg
tggaagaggacaccggcaacgagctgtccaccaaagagcagatcagccggaacagcaaggccctggaagagaaatacgtg
gccgaactgcagctggaacggctgaagaaagacggcgaagtgcggggcagcatcaacagattcaagaccagcgactacgtg
aaagaagccaaacagctgctgaaggtgcagaaggcctaccaccagctggaccagagcttcatcgacacctacatcgacctgct
ggaaacccggcggacctactatgagggacctggcgagggcagccccttcggctggaaggacatcaaagaatggtacgagat
gctgatgggccactgcacctacttccccgaggaactgcggagcgtgaagtacgcctacaacgccgacctgtacaacgccctga
acgacctgaacaatctcgtgatcaccagggacgagaacgagaagctggaatattacgagaagttccagatcatcgagaacgtgt
tcaagcagaagaagaagcccaccctgaagcagatcgccaaagaaatcctcgtgaacgaagaggatattaagggctacagagt
gaccagcaccggcaagcccgagttcaccaacctgaaggtgtaccacgacatcaaggacattaccgcccggaaagagattattg
agaacgccgagctgctggatcagattgccaagatcctgaccatctaccagagcagcgaggacatccaggaagaactgaccaat
ctgaactccgagctgacccaggaagagatcgagcagatctctaatctgaagggctataccggcacccacaacctgagcctgaa
ggccatcaacctgatcctggacgagctgtggcacaccaacgacaaccagatcgctatcttcaaccggctgaagctggtgcccaa
gaaggtggacctgtcccagcagaaagagatccccaccaccctggtggacgacttcatcctgagccccgtcgtgaagagaagct
tcatccagagcatcaaagtgatcaacgccatcatcaagaagtacggcctgcccaacgacatcattatcgagctggcccgcgaga
agaactccaaggacgcccagaaaatgatcaacgagatgcagaagcggaaccggcagaccaacgagcggatcgaggaaatc
atccggaccaccggcaaagagaacgccaagtacctgatcgagaagatcaagctgcacgacatgcaggaaggcaagtgcctgt
acagcctggaagccatccctctggaagatctgctgaacaaccccttcaactatgaggtggaccacatcatccccagaagcgtgtc
cttcgacaacagcttcaacaacaaggtgctcgtgaagcaggaagaagccagcaagaagggcaaccggaccccattccagtac
ctgagcagcagcgacagcaagatcagctacgaaaccttcaagaagcacatcctgaatctggccaagggcaagggcagaatca
gcaagaccaagaaagagtatctgctggaagaacgggacatcaacaggttctccgtgcagaaagacttcatcaaccggaacctg
gtggataccagatacgccaccagaggcctgatgaacctgctgcggagctacttcagagtgaacaacctggacgtgaaagtgaa
gtccatcaatggcggcttcaccagctttctgcggcggaagtggaagtttaagaaagagcggaacaaggggtacaagcaccacg
ccgaggacgccctgatcattgccaacgccgatttcatcttcaaagagtggaagaaactggacaaggccaaaaaagtgatggaa
aaccagatgttcgaggaaaagcaggccgagagcatgcccgagatcgaaaccgagcaggagtacaaagagatcttcatcaccc
cccaccagatcaagcacattaaggacttcaaggactacaagtacagccaccgggtggacaagaagcctaatagagagctgatt
aacgacaccctgtactccacccggaaggacgacaagggcaacaccctgatcgtgaacaatctgaacggcctgtacgacaagg
acaatgacaagctgaaaaagctgatcaacaagagccccgaaaagctgctgatgtaccaccacgacccccagacctaccagaa
actgaagctgattatggaacagtacggcgacgagaagaatcccctgtacaagtactacgaggaaaccgggaactacctgacca
agtactccaaaaaggacaacggccccgtgatcaagaagattaagtattacggcaacaaactgaacgcccatctggacatcaccg
acgactaccccaacagcagaaacaaggtcgtgaagctgtccctgaagccctacagattcgacgtgtacctggacaatggcgtgt
acaagttcgtgaccgtgaagaatctggatgtgatcaaaaaagaaaactactacgaagtgaatagcaagtgctatgaggaagctaa
gaagctgaagaagatcagcaaccaggccgagtttatcgcctccttctacaacaacgatctgatcaagatcaacggcgagctgtat
agagtgatcggcgtgaacaacgacctgctgaaccggatcgaagtgaacatgatcgacatcacctaccgcgagtacctggaaaa
catgaacgacaagaggccccccaggatcattaagacaatcgcctccaagacccagagcattaagaagtacagcacagacattc
tgggcaacctgtatgaagtgaaatctaagaagcaccctcagatcatcaaaaagggcaaaaggccggcggccacgaaaaaggc
cggccaggcaaaaaagaaaaagggatccgaggccagcggttccggacgggctgacgcattggacgattttgatctggatatgc
tgggaagtgacgccctcgatgattttgaccttgacatgcttggttcggatgcccttgatgactttgacctcgacatgctcggcagtg
acgcccttgatgatttcgacctggacatgctgattaactctagaagttccggatctccgaaaaagaaacgcaaagttggttcggga
ggtggttcgggtggctctggatcagtgctgcctcaggctcctgctcctgcaccagctccagccatggtgtctgcactggctcagg
caccagcacccgtgcctgtgctggctcctggacctccacaggctgtggctccaccagcccctaaacctacacaggccggcgag
ggcacactgtctgaagctctgctgcagctgcagttcgacgacgaggatctgggagccctgctgggaaacagcaccgatcctgc
cgtgttcaccgacctggccagcgtggacaacagcgagttccagcagctgctgaaccagggcatccctgtggcccctcacacca
ccgagcccatgctgatggaataccccgaggccatcacccggctcgtgacaggcgctcagaggcctcctgatccagctcctgcc
cctctgggagcaccaggcctgcctaatggactgctgtctggcgacgaggacttcagctctatcgccgatatggatttctcagcctt
gctgtcaggcggtggtagtggtgggagcggtagtgacctttcccatccgcccccaaggggccatctggatgagctgacaacca
cacttgagtccatgaccgaggatctgaacctggactcacccctgaccccggaattgaacgagattctggataccttcctgaacga
cgagtgcctcttgcatgccatgcatatcagcacaggactgtccatcttcgacacatctctgttttaggaattcaaataaaatacgaaa
tgaaataaaatacgaaatgcaattgggccgcaggaacccctagtgatggagttggccactccctctctgcgcgctcgctcgctca
 
ctgaggccgggcgaccaaaggtcgcccgacgcccgggctttgcccgagcggcctcagtgagcgagcgagcgcgcagctgc
ctgcaggacatgtgagcaaaaggccagcaaaaggccaggaaccgtaaaaaggccgcgttgctggcgtttttccataggctccg
cccccctgacgagcatcacaaaaatcgacgctcaagtcagaggtggcgaaacccgacaggactataaagataccaggcgtttc
cccctggaagctccctcgtgcgctctcctgttccgaccctgccgcttaccggatacctgtccgcctttctcccttcgggaagcgtg
gcgctttctcatagctcacgctgtaggtatctcagttcggtgtaggtcgttcgctccaagctgggctgtgtgcacgaaccccccgtt
cagcccgaccgctgcgccttatccggtaactatcgtcttgagtccaacccggtaagacacgacttatcgccactggcagcagcc
actggtaacaggattagcagagcgaggtatgtaggcggtgctacagagttcttgaagtggtggcctaactacggctacactagaa
gaacagtatttggtatctgcgctctgctgaagccagttaccttcggaaaaagagttggtagctcttgatccggcaaacaaaccacc
gctggtagcggtggtttttttgtttgcaagcagcagattacgcgcagaaaaaaaggatctcaagaagatcctttgatcttttctacgg
ggtctgacgctcagtggaacgaaaactcacgttaagggattttggtcatgagattatcaaaaaggatcttcacctagatccttttaaa
ttaaaaatgaagttttaaatcaatctaaagtatatatgagtaaacttggtctgacagttaccaatgcttaatcagtgaggcacctatctc
agcgatctgtctatttcgttcatccatagttgcctgactccccgtcgtgtagataactacgatacgggagggcttaccatctggcccc
agtgctgcaatgataccgcggcttccacgctcaccggctccagatttatcagcaataaaccagccagccggaagggccgagcg
cagaagtggtcctgcaactttatccgcctccatccagtctattaattgttgccgggaagctagagtaagtagttcgccagttaatagt
ttgcgcaacgttgttgccattgctacaggcatcgtggtgtcacgctcgtcgtttggtatggcttcattcagctccggttcccaacgat
caaggcgagttacatgatcccccatgttgtgcaaaaaagcggttagctccttcggtcctccgatcgttgtcagaagtaagttggcc
gcagtgttatcactcatggttatggcagcactgcataattctcttactgtcatgccatccgtaagatgcttttctgtgactggtgagtac
tcaaccaagtcattctgagaatagtgtatgcggcgaccgagttgctcttgcccggcgtcaatacgggataataccgcgccacata
gcagaactttaaaagtgctcatcattggaaaacgttcttcggggcgaaaactctcaaggatcttaccgctgttgagatccagttcga
tgtaacccactcgtgcacccaactgatcttcagcatcttttactttcaccagcgtttctgggtgagcaaaaacaggaaggcaaaatg
ccgcaaaaaagggaataagggcgacacggaaatgttgaatactcatactcttcctttttcaatattattgaagcatttatcagggttat
tgtctcatgagcggatacatatttgaatgtatttagaaaaataaacaaataggggttccgcgcacatttccccgaaaagtgccacct
gacgtctaagaaaccattattatcatgacattaacctataaaaataggcgtatcacgaggccctttcgtctcgcgcgtttcggtgatg
acggtg
**pGW047 AAV dSaCas9 Two Vector (SEQ ID NO: 11)** 
cctgcaggcagctgcgcgctcgctcgctcactgaggccgcccgggcaaagcccgggcgtcgggcgacctttggtcgcccgg
cctcagtgagcgagcgagcgcgcagagagggagtggccaactccatcactaggggttcctgcggccgcacgcgttctagaaa
gagggcctatttcccatgattccttcatatttgcatatacgatacaaggctgttagagagataattggaattaatttgactgtaaacaca
aagatattagtacaaaatacgtgacgtagaaagtaataatttcttgggtagtttgcagttttaaaattatgttttaaaatggactatcatat
gcttaccgtaacttgaaagtatttcgatttcttggctttatatatcttgtggaaaggacgaaacaccggaagagcgagctcttctgtttt
agtactctggaggccaacatgaggatcacccatgtctgcagggccaacagaatctactaaaacaaggcaaaatgccgtgtttatc
tcgtcaactggccaacatgaggatcacccatgtctgcagggcctgttggcgagatttttttgctagctgcaaagatggataaagtttt
aaacagagaggaatctttgcagctaatggaccttctaggtcttgaaaggagtgggaattggctccggtgcccgtcagtgggcag
agcgcacatcgcccacagtccccgagaagttggggggaggggtcggcaattgaaccggtgcctagagaaggtggcgcggg
gtaaactgggaaagtgatgtcgtgtactggctccgcctttttcccgagggtgggggagaaccgtatataagtgcagtagtcgccg
tgaacgttctttttcgcaacgggtttgccgccagaacacaggtaagtgccgtgtgtggttcccgcgggcctggcctctttacgggtt
atggcccttgcgtgccttgaattacttccacctggctgcagtacgtgattcttgatcccgagcttcgggttggaagtgggtgggaga
gttcgaggccttgcgcttaaggagccccttcgcctcgtgcttgagttgaggcctggcctgggcgctggggccgccgcgtgcgaa
tctggtggcaccttcgcgcctgtctcgctgctttcgataagtctctagccatttaaaatttttgatgacctgctgcgacgctttttttctg
gcaagatagtcttgtaaatgcgggccaagatctgcacactggtatttcggtttttggggccgcgggcggcgacggggcccgtgc
gtcccagcgcacatgttcggcgaggcggggcctgcgagcgcggccaccgagaatcggacgggggtagtctcaagctggcc
ggcctgctctggtgcctggcctcgcgccgccgtgtatcgccccgccctgggcggcaaggctggcccggtcggcaccagttgc
gtgagcggaaagatggccgcttcccggccctgctgcagggagctcaaaatgaaggacgcggcgctcgggagagcgggcgg
gtgagtcacccacacaaaggaaaagggcctttccgtcctcagccgtcgcttcatgtgactccacggagtaccgggcgccgtcca
ggcacctcgattagttctcgagcttttggagtacgtcgtctttaggttggggggaggggttttatgcgatggagtttccccacactga
gtgggtggagactgaagttaggccagcttggcacttgatgtaattctccttggaatttgccctttttgagtttggatcttggttcattctc
aagcctcagacagtggttcaaagtttttttcttccatttcaggtgtcgtgacgtacggccaccatggcttcaaactttactcagttcgtg
ctcgtggacaatggtgggacaggggatgtgacagtggctccttctaatttcgctaatggggtggcagagtggatcagctccaact
cacggagccaggcctacaaggtgacatgcagcgtcaggcagtctagtgcccagaagagaaagtataccatcaaggtggaggt
 
ccccaaagtggctacccagacagtgggcggagtcgaactgcctgtcgccgcttggaggtcctacctgaacatggagctcactat
cccaattttcgctaccaattctgactgtgaactcatcgtgaaggcaatgcaggggctcctcaaagacggtaatcctatcccttccgc
catcgccgctaactcaggtatctacagcgctggaggaggtggaagcggaggaggaggaagcggaggaggaggtagcggac
ctaagaaaaagaggaaggtggcggccgctggatccccttcagggcagatcagcaaccaggccctggctctggcccctagctc
cgctccagtgctggcccagactatggtgccctctagtgctatggtgcctctggcccagccacctgctccagcccctgtgctgacc
ccaggaccaccccagtcactgagcgctccagtgcccaagtctacacaggccggcgaggggactctgagtgaagctctgctgc
acctgcagttcgacgctgatgaggacctgggagctctgctggggaacagcaccgatcccggagtgttcacagatctggcctcc
gtggacaactctgagtttcagcagctgctgaatcagggcgtgtccatgtctcatagtacagccgaaccaatgctgatggagtacc
ccgaagccattacccggctggtgaccggcagccagcggccccccgaccccgctccaactcccctgggaaccagcggcctgc
ctaatgggctgtccggagatgaagacttctcaagcatcgctgatatggactttagtgccctgctgtcacagatttcctctagtgggc
agggaggaggtggaagcggcttcagcgtggacaccagtgccctgctggacctgttcagcccctcggtgaccgtgcccgacat
gagcctgcctgaccttgacagcagcctggccagtatccaagagctcctgtctccccaggagccccccaggcctcccgaggcag
agaacagcagcccggattcagggaagcagctggtgcactacacagcgcagccgctgttcctgctggaccccggctccgtgga
caccgggagcaacgacctgccggtgctgtttgagctgggagagggctcctacttctccgaaggggacggcttcgccgaggac
cccaccatctccctgctgacaggctcggagcctcccaaagccaaggaccccactgtctcctaagaattcacgcgttaagtcgac
aatcaacctctggattacaaaatttgtgaaagattgactggtattcttaactatgttgctccttttacgctatgtggatacgctgctttaat
gcctttgtatcatgctattgcttcccgtatggctttcattttctcctccttgtataaatcctggttgctgtctctttatgaggagttgtggcc
cgttgtcaggcaacgtggcgtggtgtgcactgtgtttgctgacgcaacccccactggttggggcattgccaccacctgtcagctc
ctttccgggactttcgctttccccctccctattgccacggcggaactcatcgccgcctgccttgcccgctgctggacaggggctcg
gctgttgggcactgacaattccgtggtgttgtcggggaaatcatcgtcctttccttggctgctcgcctgtgttgccacctggattctg
cgcgggacgtccttctgctacgtcccttcggccctcaatccagcggaccttccttcccgcggcctgctgccggctctgcggcctct
tccgcgtcttcgccttcgccctcagacgagtcggatctccctttgggccgcctccccgcgtcgactttaagaccaatgacgtgcgg
accgagcggccgcaggaacccctagtgatggagttggccactccctctctgcgcgctcgctcgctcactgaggccgggcgac
caaaggtcgcccgacgcccgggctttgcccgggcggcctcagtgagcgagcgagcgcgcagctgcctgcaggggcgcctg
atgcggtattttctccttacgcatctgtgcggtatttcacaccgcatacgtcaaagcaaccatagtacgcgccctgtagcggcgcat
taagcgcggcgggtgtggtggttacgcgcagcgtgaccgctacacttgccagcgccctagcgcccgctcctttcgctttcttccct
tcctttctcgccacgttcgccggctttccccgtcaagctctaaatcgggggctccctttagggttccgatttagtgctttacggcacct
cgaccccaaaaaacttgatttgggtgatggttcacgtagtgggccatcgccctgatagacggtttttcgccctttgacgttggagtc
cacgttctttaatagtggactcttgttccaaactggaacaacactcaaccctatctcgggctattcttttgatttataagggattttgccg
atttcggcctattggttaaaaaatgagctgatttaacaaaaatttaacgcgaattttaacaaaatattaacgtttacaattttatggtgca
ctctcagtacaatctgctctgatgccgcatagttaagccagccccgacacccgccaacacccgctgacgcgccctgacgggctt
gtctgctcccggcatccgcttacagacaagctgtgaccgtctccgggagctgcatgtgtcagaggttttcaccgtcatcaccgaaa
cgcgcgagacgaaagggcctcgtgatacgcctatttttataggttaatgtcatgataataatggtttcttagacgtcaggtggcactt
ttcggggaaatgtgcgcggaacccctatttgtttatttttctaaatacattcaaatatgtatccgctcatgagacaataaccctgataaa
tgcttcaataatattgaaaaaggaagagtatgagtattcaacatttccgtgtcgcccttattcccttttttgcggcattttgccttcctgttt
ttgctcacccagaaacgctggtgaaagtaaaagatgctgaagatcagttgggtgcacgagtgggttacatcgaactggatctcaa
cagcggtaagatccttgagagttttcgccccgaagaacgttttccaatgatgagcacttttaaagttctgctatgtggcgcggtattat
cccgtattgacgccgggcaagagcaactcggtcgccgcatacactattctcagaatgacttggttgagtactcaccagtcacaga
aaagcatcttacggatggcatgacagtaagagaattatgcagtgctgccataaccatgagtgataacactgcggccaacttacttc
tgacaacgatcggaggaccgaaggagctaaccgcttttttgcacaacatgggggatcatgtaactcgccttgatcgttgggaacc
ggagctgaatgaagccataccaaacgacgagcgtgacaccacgatgcctgtagcaatggcaacaacgttgcgcaaactattaa
ctggcgaactacttactctagcttcccggcaacaattaatagactggatggaggcggataaagttgcaggaccacttctgcgctcg
gcccttccggctggctggtttattgctgataaatctggagccggtgagcgtgggtctcgcggtatcattgcagcactggggccag
atggtaagccctcccgtatcgtagttatctacacgacggggagtcaggcaactatggatgaacgaaatagacagatcgctgagat
aggtgcctcactgattaagcattggtaactgtcagaccaagtttactcatatatactttagattgatttaaaacttcatttttaatttaaaa
ggatctaggtgaagatcctttttgataatctcatgaccaaaatcccttaacgtgagttttcgttccactgagcgtcagaccccgtaga
aaagatcaaaggatcttcttgagatcctttttttctgcgcgtaatctgctgcttgcaaacaaaaaaaccaccgctaccagcggtggtt
tgtttgccggatcaagagctaccaactctttttccgaaggtaactggcttcagcagagcgcagataccaaatactgtccttctagtgt
agccgtagttaggccaccacttcaagaactctgtagcaccgcctacatacctcgctctgctaatcctgttaccagtggctgctgcca
gtggcgataagtcgtgtcttaccgggttggactcaagacgatagttaccggataaggcgcagcggtcgggctgaacggggggt
 
tcgtgcacacagcccagcttggagcgaacgacctacaccgaactgagatacctacagcgtgagctatgagaaagcgccacgct
tcccgaagggagaaaggcggacaggtatccggtaagcggcagggtcggaacaggagagcgcacgagggagcttccaggg
ggaaacgcctggtatctttatagtcctgtcgggtttcgccacctctgacttgagcgtcgatttttgtgatgctcgtcaggggggcgg
agcctatggaaaaacgccagcaacgcggcctttttacggttcctggccttttgctggccttttgctcacatgt
**pGW060 AAV dSaCas9 Two Vector (SEQ ID NO: 12)** 
cctgcaggcagctgcgcgctcgctcgctcactgaggccgcccgggcgtcgggcgacctttggtcgcccggcctcagtgagcg
agcgagcgcgcagagagggagtggccaactccatcactaggggttcctgcggcctctagaaaggatctgcgatcgctccggt
gcccgtcagtgggcagagcgcacatcgcccacagtccccgagaagttggggggaggggtcggcaattgaacgggtgcctag
agaaggtggcgcggggtaaactgggaaagtgatgtcgtgtactggctccgcctttttcccgagggtgggggagaaccgtatata
agtgcagtagtcgccgtgaacgttctttttcgcaacgggtttgccgccagaacacagctgaagcttcgaggggctcgcatctctcc
ttcacgcgcccgccgccctacctgaggccgccatccacgccggttgagtcgcgttctgccgcctcccgcctgtggtgcctcctg
aactgcgtccgccgtctaggtaagtttaaagctcaggtcgagaccgggcctttgtccggcgctcccttggagcctacctagactc
agccggctctccacgctttgcctgaccctgcttgctcaactctacgtctttgtttcgttttctgttctgcgccgttacagatctggctaac
taccggtgccaccatggccccaaagaagaagcggaaggtcggtatccacggagtcccagcagccaagcggaactacatcctg
ggcctggccatcggcatcaccagcgtgggctacggcatcatcgactacgagacacgggacgtgatcgatgccggcgtgcggc
tgttcaaagaggccaacgtggaaaacaacgagggcaggcggagcaagagaggcgccagaaggctgaagcggcggaggcg
gcatagaatccagagagtgaagaagctgctgttcgactacaacctgctgaccgaccacagcgagctgagcggcatcaacccct
acgaggccagagtgaagggcctgagccagaagctgagcgaggaagagttctctgccgccctgctgcacctggccaagagaa
gaggcgtgcacaacgtgaacgaggtggaagaggacaccggcaacgagctgtccaccaaagagcagatcagccggaacagc
aaggccctggaagagaaatacgtggccgaactgcagctggaacggctgaagaaagacggcgaagtgcggggcagcatcaa
cagattcaagaccagcgactacgtgaaagaagccaaacagctgctgaaggtgcagaaggcctaccaccagctggaccagag
cttcatcgacacctacatcgacctgctggaaacccggcggacctactatgagggacctggcgagggcagccccttcggctgga
aggacatcaaagaatggtacgagatgctgatgggccactgcacctacttccccgaggaactgcggagcgtgaagtacgcctac
aacgccgacctgtacaacgccctgaacgacctgaacaatctcgtgatcaccagggacgagaacgagaagctggaatattacga
gaagttccagatcatcgagaacgtgttcaagcagaagaagaagcccaccctgaagcagatcgccaaagaaatcctcgtgaacg
aagaggatattaagggctacagagtgaccagcaccggcaagcccgagttcaccaacctgaaggtgtaccacgacatcaagga
cattaccgcccggaaagagattattgagaacgccgagctgctggatcagattgccaagatcctgaccatctaccagagcagcga
ggacatccaggaagaactgaccaatctgaactccgagctgacccaggaagagatcgagcagatctctaatctgaagggctata
ccggcacccacaacctgagcctgaaggccatcaacctgatcctggacgagctgtggcacaccaacgacaaccagatcgctatc
ttcaaccggctgaagctggtgcccaagaaggtggacctgtcccagcagaaagagatccccaccaccctggtggacgacttcat
cctgagccccgtcgtgaagagaagcttcatccagagcatcaaagtgatcaacgccatcatcaagaagtacggcctgcccaacg
acatcattatcgagctggcccgcgagaagaactccaaggacgcccagaaaatgatcaacgagatgcagaagcggaaccggc
agaccaacgagcggatcgaggaaatcatccggaccaccggcaaagagaacgccaagtacctgatcgagaagatcaagctgc
acgacatgcaggaaggcaagtgcctgtacagcctggaagccatccctctggaagatctgctgaacaaccccttcaactatgagg
tggaccacatcatccccagaagcgtgtccttcgacaacagcttcaacaacaaggtgctcgtgaagcaggaagaagccagcaag
aagggcaaccggaccccattccagtacctgagcagcagcgacagcaagatcagctacgaaaccttcaagaagcacatcctga
atctggccaagggcaagggcagaatcagcaagaccaagaaagagtatctgctggaagaacgggacatcaacaggttctccgt
gcagaaagacttcatcaaccggaacctggtggataccagatacgccaccagaggcctgatgaacctgctgcggagctacttca
gagtgaacaacctggacgtgaaagtgaagtccatcaatggcggcttcaccagctttctgcggcggaagtggaagtttaagaaag
agcggaacaaggggtacaagcaccacgccgaggacgccctgatcattgccaacgccgatttcatcttcaaagagtggaagaaa
ctggacaaggccaaaaaagtgatggaaaaccagatgttcgaggaaaagcaggccgagagcatgcccgagatcgaaaccgag
caggagtacaaagagatcttcatcaccccccaccagatcaagcacattaaggacttcaaggactacaagtacagccaccgggtg
gacaagaagcctaatagagagctgattaacgacaccctgtactccacccggaaggacgacaagggcaacaccctgatcgtga
acaatctgaacggcctgtacgacaaggacaatgacaagctgaaaaagctgatcaacaagagccccgaaaagctgctgatgtac
caccacgacccccagacctaccagaaactgaagctgattatggaacagtacggcgacgagaagaatcccctgtacaagtacta
cgaggaaaccgggaactacctgaccaagtactccaaaaaggacaacggccccgtgatcaagaagattaagtattacggcaaca
aactgaacgcccatctggacatcaccgacgactaccccaacagcagaaacaaggtcgtgaagctgtccctgaagccctacaga
ttcgacgtgtacctggacaatggcgtgtacaagttcgtgaccgtgaagaatctggatgtgatcaaaaaagaaaactactacgaagt
gaatagcaagtgctatgaggaagctaagaagctgaagaagatcagcaaccaggccgagtttatcgcctccttctacaacaacga
 
tctgatcaagatcaacggcgagctgtatagagtgatcggcgtgaacaacgacctgctgaaccggatcgaagtgaacatgatcga
catcacctaccgcgagtacctggaaaacatgaacgacaagaggccccccaggatcattaagacaatcgcctccaagacccaga
gcattaagaagtacagcacagacattctgggcaacctgtatgaagtgaaatctaagaagcaccctcagatcatcaaaaagggca
aaaggccggcggccacgaaaaaggccggccaggcaaaaaagaaaaagggatccggacgggctgacgcattggacgatttt
gatctggatatgctgggaagtgacgccctcgatgattttgaccttgacatgcttggttcggatgcccttgatgactttgacctcgaca
tgctcggcagtgacgcccttgatgatttcgacctggacatgctgatttaagaattcacgcgttaagtcgacaatcaacctctggatta
caaaatttgtgaaagattgactggtattcttaactatgttgctccttttacgctatgtggatacgctgctttaatgcctttgtatcatgctat
tgcttcccgtatggctttcattttctcctccttgtataaatcctggttgctgtctctttatgaggagttgtggcccgttgtcaggcaacgtg
gcgtggtgtgcactgtgtttgctgacgcaacccccactggttggggcattgccaccacctgtcagctcctttccgggactttcgcttt
ccccctccctattgccacggcggaactcatcgccgcctgccttgcccgctgctggacaggggctcggctgttgggcactgacaa
ttccgtggtgttgtcggggaaatcatcgtcctttccttggctgctcgcctgtgttgccacctggattctgcgcgggacgtccttctgct
acgtcccttcggccctcaatccagcggaccttccttcccgcggcctgctgccggctctgcggcctcttccgcgtcttcgccttcgc
cctcagacgagtcggatctccctttgggccgcctccccgcgtcgactttaagaccaatgacggccgcaggaacccctagtgatg
gagttggccactccctctctgcgcgctcgctcgctcactgaggccgggcgaccaaaggtcgcccgacgcccgggctttgcccg
ggcggcctcagtgagcgagcgagcgcgcagctgcctgcaggggcgcctgatgcggtattttctccttacgcatctgtgcggtatt
tcacaccgcatacgtcaaagcaaccatagtacgcgccctgtagcggcgcattaagcgcggcgggtgtggtggttacgcgcagc
gtgaccgctacacttgccagcgccttagcgcccgctcctttcgctttcttcccttcctttctcgccacgttcgccggctttccccgtca
agctctaaatcgggggctccctttagggttccgatttagtgctttacggcacctcgaccccaaaaaacttgatttgggtgatggttca
cgtagtgggccatcgccctgatagacggtttttcgccctttgacgttggagtccacgttctttaatagtggactcttgttccaaactgg
aacaacactcaactctatctcgggctattcttttgatttataagggattttgccgatttcggtctattggttaaaaaatgagctgatttaac
aaaaatttaacgcgaattttaacaaaatattaacgtttacaattttatggtgcactctcagtacaatctgctctgatgccgcatagttaa
gccagccccgacacccgccaacacccgctgacgcgccctgacgggcttgtctgctcccggcatccgcttacagacaagctgtg
accgtctccgggagctgcatgtgtcagaggttttcaccgtcatcaccgaaacgcgcgagacgaaagggcctcgtgatacgccta
tttttataggttaatgtcatgataataatggtttcttagacgtcaggtggcacttttcggggaaatgtgcgcggaacccctatttgtttatt
tttctaaatacattcaaatatgtatccgctcatgagacaataaccctgataaatgcttcaataatattgaaaaaggaagagtatgagta
ttcaacatttccgtgtcgcccttattcccttttttgcggcattttgccttcctgtttttgctcacccagaaacgctggtgaaagtaaaagat
gctgaagatcagttgggtgcacgagtgggttacatcgaactggatctcaacagcggtaagatccttgagagttttcgccccgaag
aacgttttccaatgatgagcacttttaaagttctgctatgtggcgcggtattatcccgtattgacgccgggcaagagcaactcggtc
gccgcatacactattctcagaatgacttggttgagtactcaccagtcacagaaaagcatcttacggatggcatgacagtaagagaa
ttatgcagtgctgccataaccatgagtgataacactgcggccaacttacttctgacaacgatcggaggaccgaaggagctaaccg
cttttttgcacaacatgggggatcatgtaactcgccttgatcgttgggaaccggagctgaatgaagccataccaaacgacgagcg
tgacaccacgatgcctgtagcaatggcaacaacgttgcgcaaactattaactggcgaactacttactctagcttcccggcaacaat
taatagactggatggaggcggataaagttgcaggaccacttctgcgctcggcccttccggctggctggtttattgctgataaatctg
gagccggtgagcgtggaagccgcggtatcattgcagcactggggccagatggtaagccctcccgtatcgtagttatctacacga
cggggagtcaggcaactatggatgaacgaaatagacagatcgctgagataggtgcctcactgattaagcattggtaactgtcag
accaagtttactcatatatactttagattgatttaaaacttcatttttaatttaaaaggatctaggtgaagatcctttttgataatctcatgac
caaaatcccttaacgtgagttttcgttccactgagcgtcagaccccgtagaaaagatcaaaggatcttcttgagatcctttttttctgc
gcgtaatctgctgcttgcaaacaaaaaaaccaccgctaccagcggtggtttgtttgccggatcaagagctaccaactctttttccga
aggtaactggcttcagcagagcgcagataccaaatactgttcttctagtgtagccgtagttaggccaccacttcaagaactctgtag
caccgcctacatacctcgctctgctaatcctgttaccagtggctgctgccagtggcgataagtcgtgtcttaccgggttggactcaa
gacgatagttaccggataaggcgcagcggtcgggctgaacggggggttcgtgcacacagcccagcttggagcgaacgaccta
caccgaactgagatacctacagcgtgagctatgagaaagcgccacgcttcccgaagggagaaaggcggacaggtatccggta
agcggcagggtcggaacaggagagcgcacgagggagcttccagggggaaacgcctggtatctttatagtcctgtcgggtttcg
ccacctctgacttgagcgtcgatttttgtgatgctcgtcaggggggcggagcctatggaaaaacgccagcaacgcggcctttttac
ggttcctggccttttgctggccttttgctcacatgt
**pGW045 AAV dSpCas9 Two Vector (SEQ ID NO: 13)** 
cctgcaggcagctgcgcgctcgctcgctcactgaggccgcccgggcaaagcccgggcgtcgggcgacctttggtcgcccgg
cctcagtgagcgagcgagcgcgcagagagggagtggccaactccatcactaggggttcctgcggccgcaCGCGTTCT
AGAAAGAgggcctatttcccatgattccttcatatttgcatatacgatacaaggctgttagagagataattggaattaatttgac
 
tgtaaacacaaagatattagtacaaaatacgtgacgtagaaagtaataatttcttgggtagtttgcagttttaaaattatgttttaaaatg
gactatcatatgcttaccgtaacttgaaagtatttcgatttcttggctttatatatcttGTGGAAAGGACGAAACACC
ggaagagcgagctcttctgttttagagctaggccAACATGAGGATCACCCATGTCTGCAGggcctagca
agttaaaataaggctagtccgttatcaacttggccAACATGAGGATCACCCATGTCTGCAGggccaagt
ggcaccgagtcggtgcTTTTTTGGATCCaagcttggcgtaactagatcttgagacaaatggGACAGCAGAG
ATCCAGTTTGGTTAATTAGCTAGCTGCAAAGATGGATAAAGTTTTAAACAGA
GAGGAATCTTTGCAGCTAATGGACCTTCTAGGTCTTGAAAGGAGTGGGAATT
GGCTCCGGTGCCCGTCAGTGGGCAGAGCGCACATCGCCCACAGTCCCCGAGA
AGTTGGGGGGAGGGGTCGGCAATTGAACCGGTGCCTAGAGAAGGTGGCGCG
GGGTAAACTGGGAAAGTGATGTCGTGTACTGGCTCCGCCTTTTTCCCGAGGGT
GGGGGAGAACCGTATATAAGTGCAGTAGTCGCCGTGAACGTTCTTTTTCGCA
ACGGGTTTGCCGCCAGAACACAGGTAAGTGCCGTGTGTGGTTCCCGCGGGCC
TGGCCTCTTTACGGGTTATGGCCCTTGCGTGCCTTGAATTACTTCCACCTGGCT
GCAGTACGTGATTCTTGATCCCGAGCTTCGGGTTGGAAGTGGGTGGGAGAGT
TCGAGGCCTTGCGCTTAAGGAGCCCCTTCGCCTCGTGCTTGAGTTGAGGCCTG
GCCTGGGCGCTGGGGCCGCCGCGTGCGAATCTGGTGGCACCTTCGCGCCTGT
CTCGCTGCTTTCGATAAGTCTCTAGCCATTTAAAATTTTTGATGACCTGCTGCG
ACGCTTTTTTTCTGGCAAGATAGTCTTGTAAATGCGGGCCAAGATCTGCACAC
TGGTATTTCGGTTTTTGGGGCCGCGGGCGGCGACGGGGCCCGTGCGTCCCAGC
GCACATGTTCGGCGAGGCGGGGCCTGCGAGCGCGGCCACCGAGAATCGGAC
GGGGGTAGTCTCAAGCTGGCCGGCCTGCTCTGGTGCCTGGCCTCGCGCCGCC
GTGTATCGCCCCGCCCTGGGCGGCAAGGCTGGCCCGGTCGGCACCAGTTGCG
TGAGCGGAAAGATGGCCGCTTCCCGGCCCTGCTGCAGGGAGCTCAAAATGAA
GGACGCGGCGCTCGGGAGAGCGGGCGGGTGAGTCACCCACACAAAGGAAAA
GGGCCTTTCCGTCCTCAGCCGTCGCTTCATGTGACTCCACGGAGTACCGGGCG
CCGTCCAGGCACCTCGATTAGTTCTCGAGCTTTTGGAGTACGTCGTCTTTAGG
TTGGGGGGAGGGGTTTTATGCGATGGAGTTTCCCCACACTGAGTGGGTGGAG
ACTGAAGTTAGGCCAGCTTGGCACTTGATGTAATTCTCCTTGGAATTTGCCCT
TTTTGAGTTTGGATCTTGGTTCATTCTCAAGCCTCAGACAGTGGTTCAAAGTTT
TTTTCTTCCATTTCAGGTGTCGTGACGTACGGCCACCATGGCTTCAAACTTTAC
TCAGTTCGTGCTCGTGGACAATGGTGGGACAGGGGATGTGACAGTGGCTCCT
TCTAATTTCGCTAATGGGGTGGCAGAGTGGATCAGCTCCAACTCACGGAGCC
AGGCCTACAAGGTGACATGCAGCGTCAGGCAGTCTAGTGCCCAGAAGAGAAA
GTATACCATCAAGGTGGAGGTCCCCAAAGTGGCTACCCAGACAGTGGGCGGA
GTCGAACTGCCTGTCGCCGCTTGGAGGTCCTACCTGAACATGGAGCTCACTAT
CCCAATTTTCGCTACCAATTCTGACTGTGAACTCATCGTGAAGGCAATGCAGG
GGCTCCTCAAAGACGGTAATCCTATCCCTTCCGCCATCGCCGCTAACTCAGGT
ATCTACAGCGCTGGAGGAGGTGGAAGCGGAGGAGGAGGAAGCGGAGGAGGA
GGTAGCGGACCTAAGAAAAAGAGGAAGGTGGCGGCCGCTGGATCCCCTTCAG
GGCAGATCAGCAACCAGGCCCTGGCTCTGGCCCCTAGCTCCGCTCCAGTGCT
GGCCCAGACTATGGTGCCCTCTAGTGCTATGGTGCCTCTGGCCCAGCCACCTG
CTCCAGCCCCTGTGCTGACCCCAGGACCACCCCAGTCACTGAGCGCTCCAGTG
CCCAAGTCTACACAGGCCGGCGAGGGGACTCTGAGTGAAGCTCTGCTGCACC
TGCAGTTCGACGCTGATGAGGACCTGGGAGCTCTGCTGGGGAACAGCACCGA
TCCCGGAGTGTTCACAGATCTGGCCTCCGTGGACAACTCTGAGTTTCAGCAGC
TGCTGAATCAGGGCGTGTCCATGTCTCATAGTACAGCCGAACCAATGCTGATG
GAGTACCCCGAAGCCATTACCCGGCTGGTGACCGGCAGCCAGCGGCCCCCCG
ACCCCGCTCCAACTCCCCTGGGAACCAGCGGCCTGCCTAATGGGCTGTCCGG
AGATGAAGACTTCTCAAGCATCGCTGATATGGACTTTAGTGCCCTGCTGTCAC
AGATTTCCTCTAGTGGGCAGGGAGGAGGTGGAAGCGGCTTCAGCGTGGACAC
 
CAGTGCCCTGCTGGACCTGTTCAGCCCCTCGGTGACCGTGCCCGACATGAGCC
TGCCTGACCTTGACAGCAGCCTGGCCAGTATCCAAGAGCTCCTGTCTCCCCAG
GAGCCCCCCAGGCCTCCCGAGGCAGAGAACAGCAGCCCGGATTCAGGGAAG
CAGCTGGTGCACTACACAGCGCAGCCGCTGTTCCTGCTGGACCCCGGCTCCGT
GGACACCGGGAGCAACGACCTGCCGGTGCTGTTTGAGCTGGGAGAGGGCTCC
TACTTCTCCGAAGGGGACGGCTTCGCCGAGGACCCCACCATCTCCCTGCTGAC
AGGCTCGGAGCCTCCCAAAGCCAAGGACCCCACTGTCTCCTAAGaattcacgcgttaa
gtcgacaatcaacctctggattacaaaatttgtgaaagattgactggtattcttaactatgttgctccttttacgctatgtggatacgctg
ctttaatgcctttgtatcatgctattgcttcccgtatggctttcattttctcctccttgtataaatcctggttgctgtctctttatgaggagttg
tggcccgttgtcaggcaacgtggcgtggtgtgcactgtgtttgctgacgcaacccccactggttggggcattgccaccacctgtc
agctcctttccgggactttcgctttccccctccctattgccacggcggaactcatcgccgcctgccttgcccgctgctggacaggg
gctcggctgttgggcactgacaattccgtggtgttgtcggggaaatcatcgtcctttccttggctgctcgcctgtgttgccacctgga
ttctgcgcgggacgtccttctgctacgtcccttcggccctcaatccagcggaccttccttcccgcggcctgctgccggctctgcgg
cctcttccgcgtcttcgccttcgccctcagacgagtcggatctccctttgggccgcctccccgcgtcgactttaagaccaatgacgt
gcggaccgagcggccgcaggaacccctagtgatggagttggccactccctctctgcgcgctcgctcgctcactgaggccggg
cgaccaaaggtcgcccgacgcccgggctttgcccgggcggcctcagtgagcgagcgagcgcgcagctgcctgcaggggcg
cctgatgcggtattttctccttacgcatctgtgcggtatttcacaccgcatacgtcaaagcaaccatagtacgcgccctgtagcggc
gcattaagcgcggcgggtgtggtggttacgcgcagcgtgaccgctacacttgccagcgccctagcgcccgctcctttcgctttct
tcccttcctttctcgccacgttcgccggctttccccgtcaagctctaaatcgggggctccctttagggttccgatttagtgctttacgg
cacctcgaccccaaaaaacttgatttgggtgatggttcacgtagtgggccatcgccctgatagacggtttttcgccctttgacgttg
gagtccacgttctttaatagtggactcttgttccaaactggaacaacactcaaccctatctcgggctattcttttgatttataagggattt
tgccgatttcggcctattggttaaaaaatgagctgatttaacaaaaatttaacgcgaattttaacaaaatattaacgtttacaattttatg
gtgcactctcagtacaatctgctctgatgccgcatagttaagccagccccgacacccgccaacacccgctgacgcgccctgacg
ggcttgtctgctcccggcatccgcttacagacaagctgtgaccgtctccgggagctgcatgtgtcagaggttttcaccgtcatcac
cgaaacgcgcgagacgaaagggcctcgtgatacgcctatttttataggttaatgtcatgataataatggtttcttagacgtcaggtg
gcacttttcggggaaatgtgcgcggaacccctatttgtttatttttctaaatacattcaaatatgtatccgctcatgagacaataaccct
gataaatgcttcaataatattgaaaaaggaagagtatgagtattcaacatttccgtgtcgcccttattcccttttttgcggcattttgcctt
cctgtttttgctcacccagaaacgctggtgaaagtaaaagatgctgaagatcagttgggtgcacgagtgggttacatcgaactgga
tctcaacagcggtaagatccttgagagttttcgccccgaagaacgttttccaatgatgagcacttttaaagttctgctatgtggcgcg
gtattatcccgtattgacgccgggcaagagcaactcggtcgccgcatacactattctcagaatgacttggttgagtactcaccagtc
acagaaaagcatcttacggatggcatgacagtaagagaattatgcagtgctgccataaccatgagtgataacactgcggccaact
tacttctgacaacgatcggaggaccgaaggagctaaccgcttttttgcacaacatgggggatcatgtaactcgccttgatcgttgg
gaaccggagctgaatgaagccataccaaacgacgagcgtgacaccacgatgcctgtagcaatggcaacaacgttgcgcaaac
tattaactggcgaactacttactctagcttcccggcaacaattaatagactggatggaggcggataaagttgcaggaccacttctgc
gctcggcccttccggctggctggtttattgctgataaatctggagccggtgagcgtgggtctcgcggtatcattgcagcactgggg
ccagatggtaagccctcccgtatcgtagttatctacacgacggggagtcaggcaactatggatgaacgaaatagacagatcgct
gagataggtgcctcactgattaagcattggtaactgtcagaccaagtttactcatatatactttagattgatttaaaacttcatttttaattt
aaaaggatctaggtgaagatcctttttgataatctcatgaccaaaatcccttaacgtgagttttcgttccactgagcgtcagaccccg
tagaaaagatcaaaggatcttcttgagatcctttttttctgcgcgtaatctgctgcttgcaaacaaaaaaaccaccgctaccagcggt
ggtttgtttgccggatcaagagctaccaactctttttccgaaggtaactggcttcagcagagcgcagataccaaatactgtccttcta
gtgtagccgtagttaggccaccacttcaagaactctgtagcaccgcctacatacctcgctctgctaatcctgttaccagtggctgct
gccagtggcgataagtcgtgtcttaccgggttggactcaagacgatagttaccggataaggcgcagcggtcgggctgaacggg
gggttcgtgcacacagcccagcttggagcgaacgacctacaccgaactgagatacctacagcgtgagctatgagaaagcgcc
acgcttcccgaagggagaaaggcggacaggtatccggtaagcggcagggtcggaacaggagagcgcacgagggagcttcc
agggggaaacgcctggtatctttatagtcctgtcgggtttcgccacctctgacttgagcgtcgatttttgtgatgctcgtcagggggg
cggagcctatggaaaaacgccagcaacgcggcctttttacggttcctggccttttgctggccttttgctcacatgt
**pRC119 AAV Two Vector dCas12a/dCpf1 (SEQ ID NO: 14)** 
cctgcaggcagctgcgcgctcgctcgctcactgaggccgcccgggcaaagcccgggcgtcgggcgacctttggtcgcccgg
cctcagtgagcgagcgagcgcgcagagagggagtggccaactccatcactaggggttcctgcggccgcacgcgttctaggtc
 
ttgaaaggagtgggaattggctccggtgcccgtcagtgggcagagcgcacatcgcccacagtccccgagaagttggggggag
gggtcggcaattgatccggtgcctagagaaggtggcgcggggtaaactgggaaagtgatgtcgtgtactggctccgcctttttcc
cgagggtgggggagaaccgtatataagtgcagtagtcgccgtgaacgttctttttcgcaacgggtttgccgccagaacacaggT
GTCGTGACGCGcGTACGgccaccatgacacagttcgagggctttaccaacctgtatcaggtgagcaagacactg
cggtttgagctgatcccacagggcaagaccctgaagcacatccaggagcagggcttcatcgaggaggacaaggcccgcaatg
atcactacaaggagctgaagcccatcatcgatcggatctacaagacctatgccgaccagtgcctgcagctggtgcagctggatt
gggagaacctgagcgccgccatcgactcctatagaaaggagaaaaccgaggagacaaggaacgccctgatcgaggagcag
gccacatatcgcaatgccatccacgactacttcatcggccggacagacaacctgaccgatgccatcaataagagacacgccga
gatctacaagggcctgttcaaggccgagctgtttaatggcaaggtgctgaagcagctgggcaccgtgaccacaaccgagcacg
agaacgccctgctgcggagcttcgacaagtttacaacctacttctccggcttttatagaaacaggaagaacgtgttcagcgccga
ggatatcagcacagccatcccacaccgcatcgtgcaggacaacttccccaagtttaaggagaattgtcacatcttcacacgcctg
atcaccgccgtgcccagcctgcgggagcactttgagaacgtgaagaaggccatcggcatcttcgtgagcacctccatcgagga
ggtgttttccttccctttttataaccagctgctgacacagacccagatcgacctgtataaccagctgctgggaggaatctctcggga
ggcaggcaccgagaagatcaagggcctgaacgaggtgctgaatctggccatccagaagaatgatgagacagcccacatcatc
gcctccctgccacacagattcatccccctgtttaagcagatcctgtccgataggaacaccctgtctttcatcctggaggagtttaag
agcgacgaggaagtgatccagtccttctgcaagtacaagacactgctgagaaacgagaacgtgctggagacagccgaggccc
tgtttaacgagctgaacagcatcgacctgacacacatcttcatcagccacaagaagctggagacaatcagcagcgccctgtgcg
accactgggatacactgaggaatgccctgtatgagcggagaatctccgagctgacaggcaagatcaccaagtctgccaaggag
aaggtgcagcgcagcctgaagcacgaggatatcaacctgcaggagatcatctctgccgcaggcaaggagctgagcgaggcct
tcaagcagaaaaccagcgagatcctgtcccacgcacacgccgccctggatcagccactgcctacaaccctgaagaagcagga
ggagaaggagatcctgaagtctcagctggacagcctgctgggcctgtaccacctgctggactggtttgccgtggatgagtccaa
cgaggtggaccccgagttctctgcccggctgaccggcatcaagctggagatggagccttctctgagcttctacaacaaggccag
aaattatgccaccaagaagccctactccgtggagaagttcaagctgaactttcagatgcctacactggccagaggctgggacgt
gaatagagagaagaacaatggcgccatcctgtttgtgaagaacggcctgtactatctgggcatcatgccaaagcagaagggca
ggtataaggccctgagcttcgagcccacagagaaaaccagcgagggctttgataagatgtactatgactacttccctgatgccgc
caagatgatcccaaagtgcagcacccagctgaaggccgtgacagcccactttcagacccacacaacccccatcctgctgtcca
acaatttcatcgagcctctggagatcacaaaggagatctacgacctgaacaatcctgagaaggagccaaagaagtttcagacag
cctacgccaagaaaaccggcgaccagaagggctacagagaggccctgtgcaagtggatcgacttcacaagggattttctgtcc
aagtataccaagacaacctctatcgatctgtctagcctgcggccatcctctcagtataaggacctgggcgagtactatgccgagct
gaatcccctgctgtaccacatcagcttccagagaatcgccgagaaggagatcatggatgccgtggagacaggcaagctgtacct
gttccagatctataacaaggactttgccaagggccaccacggcaagcctaatctgcacacactgtattggaccggcctgttttctc
cagagaacctggccaagacaagcatcaagctgaatggccaggccgagctgttctaccgccctaagtccaggatgaagaggat
ggcacaccggctgggagagaagatgctgaacaagaagctgaaggatcagaaaaccccaatccccgacaccctgtaccagga
gctgtacgactatgtgaatcacagactgtcccacgacctgtctgatgaggccagggccctgctgcccaacgtgatcaccaagga
ggtgtctcacgagatcatcaaggataggcgctttaccagcgacaagttctttttccacgtgcctatcacactgaactatcaggccgc
caattccccatctaagttcaaccagagggtgaatgcctacctgaaggagcaccccgagacacctatcatcggcatcgcccgggg
cgagagaaacctgatctatatcacagtgatcgactccaccggcaagatcctggagcagcggagcctgaacaccatccagcagtt
tgattaccagaagaagctggacaacagggagaaggagagggtggcagcaaggcaggcctggtctgtggtgggcacaatcaa
ggatctgaagcagggctatctgagccaggtcatccacgagatcgtggacctgatgatccactaccaggccgtggtggtgctgga
gaacctgaatttcggctttaagagcaagaggaccggcatcgccgagaaggccgtgtaccagcagttcgagaagatgctgatcg
ataagctgaattgcctggtgctgaaggactatccagcagagaaagtgggaggcgtgctgaacccataccagctgacagaccag
ttcacctcctttgccaagatgggcacccagtctggcttcctgttttacgtgcctgccccatatacatctaagatcgatcccctgaccg
gcttcgtggaccccttcgtgtggaaaaccatcaagaatcacgagagccgcaagcacttcctggagggcttcgactttctgcacta
cgacgtgaaaaccggcgacttcatcctgcactttaagatgaacagaaatctgtccttccagaggggcctgcccggctttatgcctg
catgggatatcgtgttcgagaagaacgagacacagtttgacgccaagggcacccctttcatcgccggcaagagaatcgtgcca
gtgatcgagaatcacagattcaccggcagataccgggacctgtatcctgccaacgagctgatcgccctgctggaggagaaggg
catcgtgttcagggatggctccaacatcctgccaaagctgctggagaatgacgattctcacgccatcgacacgatggtggccctg
atccgcagcgtgctgcagatgcggaactccaatgccgccacaggcgaggactatatcaacagccccgtgcgcgatctgaatgg
cgtgtgcttcgactcccggtttcagaacccagagtggccaatggacgccgatgccaatggcgcctaccacatcgccctgaagg
gccagctgctgctgaatcacctgaaggagagcaaggatctgaagctgcagaacggcatctccaatcaggactggctggcctac
 
atccaggagctgcgcaacaaaaggccggcggccacgaaaaaggccggccaggcaaaaaagaaaaagGGCAGTGG
TAGTGGAGAAGAGCTGTTGTCAAAAAATTATCACTTGGAGAATGAGGTAGCC
CGCCTTAAGAAAGGCAGCGGATCAGGCGAGGAACTTCTGAGCAAGAATTACC
ATCTCGAAAACGAAGTGGCACGCTTGAAGAAGGGTTCAGGTTCCGGGGAGGA
GCTCCTCTCCAAGAACTACCACCTGGAGAACGAAGTCGCTCGCCTGAAGAAAt
gaAATAAAAGATCTTTATTTTCATTAGATCTGTGTGTTGGTTTTTTGTGTGcggac
cgagcggccgcaggaacccctagtgatggagttggccactccctctctgcgcgctcgctcgctcactgaggccgggcgacca
aaggtcgcccgacgcccgggctttgcccgggcggcctcagtgagcgagcgagcgcgcagctgcctgcaggggcgcctgat
gcggtattttctccttacgcatctgtgcggtatttcacaccgcatacgtcaaagcaaccatagtacgcgccctgtagcggcgcatta
agcgcggcgggtgtggtggttacgcgcagcgtgaccgctacacttgccagcgccctagcgcccgctcctttcgctttcttcccttc
ctttctcgccacgttcgccggctttccccgtcaagctctaaatcgggggctccctttagggttccgatttagtgctttacggcacctc
gaccccaaaaaacttgatttgggtgatggttcacgtagtgggccatcgccctgatagacggtttttcgccctttgacgttggagtcc
acgttctttaatagtggactcttgttccaaactggaacaacactcaaccctatctcgggctattcttttgatttataagggattttgccga
tttcggcctattggttaaaaaatgagctgatttaacaaaaatttaacgcgaattttaacaaaatattaacgtttacaattttatggtgcact
ctcagtacaatctgctctgatgccgcatagttaagccagccccgacacccgccaacacccgctgacgcgccctgacgggcttgt
ctgctcccggcatccgcttacagacaagctgtgaccgtctccgggagctgcatgtgtcagaggttttcaccgtcatcaccgaaac
gcgcgagacgaaagggcctcgtgatacgcctatttttataggttaatgtcatgataataatggtttcttagacgtcaggtggcactttt
cggggaaatgtgcgcggaacccctatttgtttatttttctaaatacattcaaatatgtatccgctcatgagacaataaccctgataaat
gcttcaataatattgaaaaaggaagagtatgagtattcaacatttccgtgtcgcccttattcccttttttgcggcattttgccttcctgttt
ttgctcacccagaaacgctggtgaaagtaaaagatgctgaagatcagttgggtgcacgagtgggttacatcgaactggatctcaac
agcggtaagatccttgagagttttcgccccgaagaacgttttccaatgatgagcacttttaaagttctgctatgtggcgcggtattatc
ccgtattgacgccgggcaagagcaactcggtcgccgcatacactattctcagaatgacttggttgagtactcaccagtcacagaa
aagcatcttacggatggcatgacagtaagagaattatgcagtgctgccataaccatgagtgataacactgcggccaacttacttct
gacaacgatcggaggaccgaaggagctaaccgcttttttgcacaacatgggggatcatgtaactcgccttgatcgttgggaacc
ggagctgaatgaagccataccaaacgacgagcgtgacaccacgatgcctgtagcaatggcaacaacgttgcgcaaactattaa
ctggcgaactacttactctagcttcccggcaacaattaatagactggatggaggcggataaagttgcaggaccacttctgcgctcg
gcccttccggctggctggtttattgctgataaatctggagccggtgagcgtgggtctcgcggtatcattgcagcactggggccag
atggtaagccctcccgtatcgtagttatctacacgacggggagtcaggcaactatggatgaacgaaatagacagatcgctgagat
aggtgcctcactgattaagcattggtaactgtcagaccaagtttactcatatatactttagattgatttaaaacttcatttttaatttaaaa
ggatctaggtgaagatcctttttgataatctcatgaccaaaatcccttaacgtgagttttcgttccactgagcgtcagaccccgtaga
aaagatcaaaggatcttcttgagatcctttttttctgcgcgtaatctgctgcttgcaaacaaaaaaaccaccgctaccagcggtggt
ttgtttgccggatcaagagctaccaactctttttccgaaggtaactggcttcagcagagcgcagataccaaatactgtccttctagtgt
agccgtagttaggccaccacttcaagaactctgtagcaccgcctacatacctcgctctgctaatcctgttaccagtggctgctgcca
gtggcgataagtcgtgtcttaccgggttggactcaagacgatagttaccggataaggcgcagcggtcgggctgaacggggggt
tcgtgcacacagcccagcttggagcgaacgacctacaccgaactgagatacctacagcgtgagctatgagaaagcgccacgct
tcccgaagggagaaaggcggacaggtatccggtaagcggcagggtcggaacaggagagcgcacgagggagcttccaggg
ggaaacgcctggtatctttatagtcctgtcgggtttcgccacctctgacttgagcgtcgatttttgtgatgctcgtcaggggggcgg
agcctatggaaaaacgccagcaacgcggcctttttacggttcctggccttttgctggccttttgctcacatgt
**pRC120 AAV Two Vector dCas12a/dCpf1 (SEQ ID NO: 15)** 
cctgcaggcagctgcgcgctcgctcgctcactgaggccgcccgggcaaagcccgggcgtcgggcgacctttggtcgcccgg
cctcagtgagcgagcgagcgcgcagagagggagtggccaactccatcactaggggttcctgcggccgcacgcgttctaggtc
ttgaaaggagtgggaattggctccggtgcccgtcagtgggcagagcgcacatcgcccacagtccccgagaagttggggggag
gggtcggcaattgatccggtgcctagagaaggtggcgcggggtaaactgggaaagtgatgtcgtgtactggctccgcctttttcc
cgagggtgggggagaaccgtatataagtgcagtagtcgccgtgaacgttctttttcgcaacgggtttgccgccagaacacaggT
GTCGTGACGCGcGTACGgccaccatgtcaaagctggagaaattcaccaactgttatagcctgtctaagaccctgc
gcttcaaggcaatcccagtgggcaagacacaagagaacattgacaacaaacggctcctggtggaggatgagaagagggctga
agattacaagggcgttaagaagctgctggataggtactatctgtcattcatcaacgatgtcctccacagtatcaagctgaagaatct
gaacaattacatttctctgttccggaagaagacacggaccgagaaggagaacaaagagctggagaatctggagatcaacctga
ggaaagaaatagctaaggctttcaaagggaacgagggttacaagtccctgttcaagaaagacattatcgagactattctgcctga
 
gttcctggacgataaagatgagatcgccctcgtcaattccttcaatgggtttaccacagcctttaccggcttcttcgacaatagaga
gaatatgttctctgaagaggccaaatccactagcatcgcctttcgctgcataaacgagaacctgactaggtacatcagcaatatgg
acatctttgagaaagtcgatgccatattcgacaaacatgaggtgcaggagattaaggagaagatcctgaactcagattacgatgtc
gaagatttcttcgagggagagttcttcaacttcgtgctcacacaagagggcattgatgtgtacaatgcaatcattggagggttcgtg
acagagagtggcgagaagataaagggcctgaacgagtatatcaacctctacaaccagaaaaccaagcagaaactgcctaagtt
caagccactgtacaaacaagtgctctcagatagggaaagcctgagcttctacggtgaagggtatacatcagatgaagaagtgct
cgaagtgttccgcaacaccctcaataagaacagtgaaatcttctcttcaatcaagaagctggagaaactgttcaagaatttcgatga
gtactcctctgccggaatctttgtgaagaatggccctgcaatatccactattagcaaagacatctttggcgagtggaacgttatcag
ggataagtggaatgccgagtacgatgatattcatctcaagaagaaagccgtggttacagagaaatacgaggatgatagacgcaa
gagctttaagaagattggtagcttctctctcgaacagctgcaggagtacgccgacgctgacctgtcagtcgtggagaaactcaag
gagatcataatccagaaggtggatgaaatctacaaagtgtatggaagctctgagaaactcttcgatgcagactttgttctggagaa
gagtctgaagaagaacgacgcagtggttgctatcatgaaggacctgctggattctgttaagtctttcgagaattacattaaggcatt
ctttggtgaagggaaggagacaaatagggacgagagcttctatggcgactttgttctggcctacgacatcctcctcaaggttgacc
acatctatgacgctatacggaattacgttacccagaagccctatagcaaagacaagttcaagctgtatttccagaatccacagtttat
gggtgggtgggataaagacaaagaaacagattacagggccactatcctgcggtacggcagcaaatactatctggctatcatgga
taagaagtacgccaaatgcctccagaagatcgacaaggacgacgtgaacggtaactacgagaagatcaattacaagctcctgc
caggacctaacaagatgctgcccaaggtgttcttctccaagaaatggatggcctactataacccaagcgaggacattcagaagat
atacaagaatgggacattcaagaagggcgatatgttcaacctcaacgactgccacaagctgattgatttcttcaaggatagcatttc
tcgctatcccaagtggtctaatgcatacgatttcaacttcagcgagactgagaagtacaaagacatcgctggcttctaccgggagg
tggaagagcaaggctataaggtgtcattcgaatccgcttctaagaaggaagtggataagctcgtggaagagggtaagctgtacat
gttccagatatacaacaaagacttcagcgataagagccacggcactccaaacctccatactatgtatttcaagctgctgtttgacga
gaacaaccacggacagattaggctgtcaggaggcgcagaactcttcatgcgcagagcttcactgaagaaggaggaactcgttg
tccacccagccaatagccctatagccaataagaatccagacaatcctaagaaaaccactactctgtcttacgatgtgtataaggat
aagagattctctgaagatcagtacgaactgcacatacccattgccattaacaagtgccctaagaacatcttcaagattaacacaga
ggttagagtgctcctgaaacacgacgataacccttatgttataggcattgctcgcggagagagaaacctgctgtacatcgtcgtgg
tggacggcaaaggcaacatcgtggaacagtacagtctcaatgaaatcattaacaatttcaacggaatccgcattaagaccgacta
ccattctctcctcgacaagaaggagaaagaaaggttcgaagcaagacagaattggacaagtatagagaatatcaaagaactgaa
ggctgggtacatctctcaggttgtgcacaagatatgtgagctggtggagaagtacgacgctgttatcgccctcgcggacctgaat
agcggcttcaagaactccagggtgaaggtggagaagcaggtgtatcagaagttcgagaagatgctgatcgacaagctcaactat
atggtggacaagaaatccaatccttgcgctactggtggagccctgaagggctatcaaatcaccaataagttcgaatctttcaagtct
atgagcacccagaatggcttcatcttctacatacccgcatggctgacatccaagattgatccctctaccggatttgttaatctgctca
agactaagtacacctctattgctgactcaaagaagttcatatcatcatttgaccgcatcatgtacgtgccagaagaggacctgttcg
agtttgccctggattacaagaatttctctcggactgacgccgactacatcaagaagtggaagctctactcttatggtaatcggattcg
catattccgcaatcccaagaagaataacgtgttcgattgggaggaagtttgcctcaccagcgcttacaaggagctgttcaataagt
atgggattaactaccagcagggcgacataagagccctgctgtgcgaacaatctgataaggcattctattcctctttcatggcactga
tgtcactgatgctgcaaatgcgcaattccatcaccggaagaacagacgtggcctttctgatctctcctgtcaagaactcagatggc
atcttctacgattcccgcaactatgaagcacaggagaatgctatcctgcctaagaatgccgatgcaaatggagcctataacatcgc
cagaaaggtcctctgggccataggacaattcaagaaagctgaagatgagaagctggacaaggtgaagatcgccatttcaaaca
aagagtggctcgaatatgctcagacctcagtgaagcataaaaggccggcggccacgaaaaaggccggccaggcaaaaaaga
aaaagGGCAGCGGTAGTGGGGAGGAGCTTCTTTCTAAAAATTATCATCTTGAAA
ATGAAGTGGCACGACTCAAGAAAGGCTCTGGGTCCGGTGAGGAACTCCTGTC
CAAGAACTATCATCTTGAAAACGAAGTCGCCCGCCTTAAAAAAGGCTCAGGC
TCAGGTGAAGAATTGTTGAGCAAAAACTATCACCTTGAGAATGAGGTTGCCC
GGCTCAAAAAAGGGTCTGGCAGCGGCGAGGAACTCCTTTCAAAGAACTACCA
TCTCGAAAATGAAGTTGCTCGGTTGAAGAAGGGTTCAGGATCTGGCGAGGAG
CTTCTGTCCAAGAACTACCATTTGGAGAACGAGGTCGCAAGACTTAAAAAAtg
aAATAAAAGATCTTTATTTTCATTAGATCTGTGTGTTGGTTTTTTGTGTGcggacc
gagcggccgcaggaacccctagtgatggagttggccactccctctctgcgcgctcgctcgctcactgaggccgggcgaccaa
aggtcgcccgacgcccgggctttgcccgggcggcctcagtgagcgagcgagcgcgcagctgcctgcaggggcgcctgatg
cggtattttctccttacgcatctgtgcggtatttcacaccgcatacgtcaaagcaaccatagtacgcgccctgtagcggcgcattaa
 
gcgcggcgggtgtggtggttacgcgcagcgtgaccgctacacttgccagcgccctagcgcccgctcctttcgctttcttcccttcc
tttctcgccacgttcgccggctttccccgtcaagctctaaatcgggggctccctttagggttccgatttagtgctttacggcacctcg
accccaaaaaacttgatttgggtgatggttcacgtagtgggccatcgccctgatagacggtttttcgccctttgacgttggagtcca
cgttctttaatagtggactcttgttccaaactggaacaacactcaaccctatctcgggctattcttttgatttataagggattttgccgatt
tcggcctattggttaaaaaatgagctgatttaacaaaaatttaacgcgaattttaacaaaatattaacgtttacaattttatggtgcactc
tcagtacaatctgctctgatgccgcatagttaagccagccccgacacccgccaacacccgctgacgcgccctgacgggcttgtc
tgctcccggcatccgcttacagacaagctgtgaccgtctccgggagctgcatgtgtcagaggttttcaccgtcatcaccgaaacg
cgcgagacgaaagggcctcgtgatacgcctatttttataggttaatgtcatgataataatggtttcttagacgtcaggtggcacttttc
ggggaaatgtgcgcggaacccctatttgtttatttttctaaatacattcaaatatgtatccgctcatgagacaataaccctgataaatg
cttcaataatattgaaaaaggaagagtatgagtattcaacatttccgtgtcgcccttattcccttttttgcggcattttgccttcctgttttt
gctcacccagaaacgctggtgaaagtaaaagatgctgaagatcagttgggtgcacgagtgggttacatcgaactggatctcaac
agcggtaagatccttgagagttttcgccccgaagaacgttttccaatgatgagcacttttaaagttctgctatgtggcgcggtattatc
ccgtattgacgccgggcaagagcaactcggtcgccgcatacactattctcagaatgacttggttgagtactcaccagtcacagaa
aagcatcttacggatggcatgacagtaagagaattatgcagtgctgccataaccatgagtgataacactgcggccaacttacttct
gacaacgatcggaggaccgaaggagctaaccgcttttttgcacaacatgggggatcatgtaactcgccttgatcgttgggaacc
ggagctgaatgaagccataccaaacgacgagcgtgacaccacgatgcctgtagcaatggcaacaacgttgcgcaaactattaa
ctggcgaactacttactctagcttcccggcaacaattaatagactggatggaggcggataaagttgcaggaccacttctgcgctcg
gcccttccggctggctggtttattgctgataaatctggagccggtgagcgtgggtctcgcggtatcattgcagcactggggccag
atggtaagccctcccgtatcgtagttatctacacgacggggagtcaggcaactatggatgaacgaaatagacagatcgctgagat
aggtgcctcactgattaagcattggtaactgtcagaccaagtttactcatatatactttagattgatttaaaacttcatttttaatttaaaa
ggatctaggtgaagatcctttttgataatctcatgaccaaaatcccttaacgtgagttttcgttccactgagcgtcagaccccgtaga
aaagatcaaaggatcttcttgagatcctttttttctgcgcgtaatctgctgcttgcaaacaaaaaaaccaccgctaccagcggtggtt
tgtttgccggatcaagagctaccaactctttttccgaaggtaactggcttcagcagagcgcagataccaaatactgtccttctagtgt
agccgtagttaggccaccacttcaagaactctgtagcaccgcctacatacctcgctctgctaatcctgttaccagtggctgctgcca
gtggcgataagtcgtgtcttaccgggttggactcaagacgatagttaccggataaggcgcagcggtcgggctgaacggggggt
tcgtgcacacagcccagcttggagcgaacgacctacaccgaactgagatacctacagcgtgagctatgagaaagcgccacgct
tcccgaagggagaaaggcggacaggtatccggtaagcggcagggtcggaacaggagagcgcacgagggagcttccaggg
ggaaacgcctggtatctttatagtcctgtcgggtttcgccacctctgacttgagcgtcgatttttgtgatgctcgtcaggggggcgg
agcctatggaaaaacgccagcaacgcggcctttttacggttcctggccttttgctggccttttgctcacatgt
**pRC121b AAV Two Vector dCas12a/dCpf1 (SEQ ID NO: 16)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atgGGCCCAGCCGCCAAACGAGTCAAGCTGGATGGCCCCGATATCGTCATGAC
TCAATCACCCTCATCACTGTCAGCCAGCGTCGGTGACCGGGTAACAATTACTT
GTAGATCATCAACTGGGGCCGTCACTACCTCCAACTATGCCAGTTGGGTTCAA
GAGAAACCCGGCAAGCTCTTTAAGGGACTGATCGGTGGGACCAATAATCGGG
CCCCCGGTGTGCCTTCTCGCTTTAGCGGGAGTTTGATAGGCGATAAAGCCACA
TTGACAATATCATCTCTTCAACCCGAGGACTTCGCTACCTATTTTTGTGCTTTG
 
TGGTATAGTAACCATTGGGTATTTGGCCAAGGAACTAAGGTAGAGTTGAAGCG
GGGGGGCGGCGGCTCTGGTGGAGGAGGTAGTGGGGGCGGTGGCTCTTCCGG
AGGGGGTTCCGAAGTAAAGCTTTTGGAGAGCGGCGGCGGCCTGGTCCAGCCT
GGAGGATCTCTCAAGCTTAGCTGCGCCGTCAGCGGCTTTTCCCTCACTGATTA
TGGTGTAAATTGGGTGAGGCAGGCTCCAGGCAGGGGGTTGGAGTGGATAGGG
GTGATATGGGGGGATGGCATCACAGATTATAATTCTGCACTTAAGGATAGGT
TTATTATCAGTAAGGACAACGGTAAGAACACCGTTTATCTTCAGATGTCTAAG
GTGCGGTCCGATGATACTGCCCTCTATTATTGTGTAACCGGCCTTTTCGATTAC
TGGGGCCAAGGGACTTTGGTAACCGTGTCATCATATCCCTATGACGTCCCTGA
CTACGCAGGGGGAGGAGGGGGATCCGGTGGCGGTGGTAGTGGAGGAGGTGG
ATCTGGAGGAGGGGGAAGCggtaccCCAGCTGCGAAACGAGTTAAATTGGATGG
TGGTGGCGGGTCCTCAGGTTTGCCCAATGGATTGGATGGTGATGAAGACTTCT
CAGACATCGCAGATATGGACTTTAGTGCCTTGCTGAGCCAAATCTCATCTGGC
TCATCAGGTCTTCCCAACGGATTGGATGGCGATGAGGACTTTTCCGACATCGC
AGACATGGACTTTTCCGCTCTGTTGAGCCAGATAAGTTCTGGCTCCTCCGGAC
TCCCTAATGGCTTGGATGGAGATGAGGATTTTAGCGATATAGCAGACATGGA
TTTTAGCGCATTGCTCTCACAAATCTCCAGTGGCGGGGGTGGTAGTGGATTTT
CAGTGGATACTTCCGCACTTCTCGATTTGTTTTCTCCCAGTGTCACCGTCCCAG
ACATGAGTCTTCCTGACCTCGACTCTTCTCTCGCCAGTATTCAGGAACTGCTC
AGTCCACAGGAGCCTCCTCGCCCTCCTGAAGCAGAGAATAGTTCCCCAGATT
CAGGAAAGCAGCTGGTGCACTACACAGCACAGCCACTGTTCCTCTTGGACCC
TGGGAGTGTAGACACAGGCTCAAACGATCTCCCCGTTCTTTTTGAACTTGGCG
AAGGTTCCTATTTTAGCGAGGGGGACGGATTCGCCGAGGACCCTACTATCAG
TCTCCTTACAGGTAGCGAGCCACCCAAAGCTAAAGACCCCACCGTGAGCTAG
TGActcgagGATTCGTCAGTAGGGTTGTAAAGGTTTTTCTTTTCCTGAGAAAACA
ACCTTTTGTTTTCTCAGGTTTTGCTTTTTGGCCTTTCCCTAGCTTTAAAAAAAA
AAAAGCAAAACTCACCGAGGCAGTTCCATAGGATGGCtagcAAGATCCTGGTA
TTGGTCTGCGAgaagagcgagctcttcaatcaacctctggattacaaaatttgtgaaagattgactggtattcttaactatg
ttgctccttttacgctatgtggatacgctgctttaatgcctttgtatcatgctattgcttcccgtatggctttcattttctcctccttgtataaa
tcctggttgctgtctctttatgaggagttgtggcccgttgtcaggcaacgtggcgtggtgtgcactgtgtttgctgacgcaaccccc
actggttggggcattgccaccacctgtcagctcctttccgggactttcgctttccccctccctattgccacggcggaactcatcgcc
gcctgccttgcccgctgctggacaggggctcggctgttgggcactgacaattccgtggtgttgtcggggaaatcatcgtcctttcc
ttggctgctcgcctgtgttgccacctggattctgcgcgggacgtccttctgctacgtcccttcggccctcaatccagcggaccttcc
ttcccgcggcctgctgccggctctgcggcctcttccgcgtcttcgccttcgccctcagacgagtcggatctccctttgggccgcct
ccccgcgtcgactttaagaccaatCGGCCGCAGGAACCCCTAGTGATGGAGTTGGCCACTCC
CTCTCTGCGCGCTCGCTCGCTCACTGAGGCCGGGCGACCAAAGGTCGCCCGA
CGCCCGGGCTTTGCCCGGGCGGCCTCAGTGAGCGAGCGAGCGCGCAGCTGCC
TGCAGGGGCGCCTGATGCGGTATTTTCTCCTTACGCATCTGTGCGGTATTTCA
CACCGCATACGTCAAAGCAACCATAGTACGCGCCCTGTAGCGGCGCATTAAG
CGCGGCGGGTGTGGTGGTTACGCGCAGCGTGACCGCTACACTTGCCAGCGCC
TTAGCGCCCGCTCCTTTCGCTTTCTTCCCTTCCTTTCTCGCCACGTTCGCCGGC
TTTCCCCGTCAAGCTCTAAATCGGGGGCTCCCTTTAGGGTTCCGATTTAGTGC
TTTACGGCACCTCGACCCCAAAAAACTTGATTTGGGTGATGGTTCACGTAGTG
GGCCATCGCCCTGATAGACGGTTTTTCGCCCTTTGACGTTGGAGTCCACGTTC
TTTAATAGTGGACTCTTGTTCCAAACTGGAACAACACTCAACTCTATCTCGGG
CTATTCTTTTGATTTATAAGGGATTTTGCCGATTTCGGTCTATTGGTTAAAAAA
TGAGCTGATTTAACAAAAATTTAACGCGAATTTTAACAAAATATTAACGTTTA
CAATTTTATGGTGCACTCTCAGTACAATCTGCTCTGATGCCGCATAGTTAAGC
CAGCCCCGACACCCGCCAACACCCGCTGACGCGCCCTGACGGGCTTGTCTGC
TCCCGGCATCCGCTTACAGACAAGCTGTGACCGTCTCCGGGAGCTGCATGTGT
 
CAGAGGTTTTCACCGTCATCACCGAAACGCGCGAGACGAAAGGGCCTCGTGA
TACGCCTATTTTTATAGGTTAATGTCATGATAATAATGGTTTCTTAGACGTCA
GGTGGCACTTTTCGGGGAAATGTGCGCGGAACCCCTATTTGTTTATTTTTCTA
AATACATTCAAATATGTATCCGCTCATGAGACAATAACCCTGATAAATGCTTC
AATAATATTGAAAAAGGAAGAGTATGAGTATTCAACATTTCCGTGTCGCCCTT
ATTCCCTTTTTTGCGGCATTTTGCCTTCCTGTTTTTGCTCACCCAGAAACGCTG
GTGAAAGTAAAAGATGCTGAAGATCAGTTGGGTGCACGAGTGGGTTACATCG
AACTGGATCTCAACAGCGGTAAGATCCTTGAGAGTTTTCGCCCCGAAGAACG
TTTTCCAATGATGAGCACTTTTAAAGTTCTGCTATGTGGCGCGGTATTATCCC
GTATTGACGCCGGGCAAGAGCAACTCGGTCGCCGCATACACTATTCTCAGAA
TGACTTGGTTGAGTACTCACCAGTCACAGAAAAGCATCTTACGGATGGCATG
ACAGTAAGAGAATTATGCAGTGCTGCCATAACCATGAGTGATAACACTGCGG
CCAACTTACTTCTGACAACGATCGGAGGACCGAAGGAGCTAACCGCTTTTTTG
CACAACATGGGGGATCATGTAACTCGCCTTGATCGTTGGGAACCGGAGCTGA
ATGAAGCCATACCAAACGACGAGCGTGACACCACGATGCCTGTAGCAATGGC
AACAACGTTGCGCAAACTATTAACTGGCGAACTACTTACTCTAGCTTCCCGGC
AACAATTAATAGACTGGATGGAGGCGGATAAAGTTGCAGGACCACTTCTGCG
CTCGGCCCTTCCGGCTGGCTGGTTTATTGCTGATAAATCTGGAGCCGGTGAGC
GTGGAAGCCGCGGTATCATTGCAGCACTGGGGCCAGATGGTAAGCCCTCCCG
TATCGTAGTTATCTACACGACGGGGAGTCAGGCAACTATGGATGAACGAAAT
AGACAGATCGCTGAGATAGGTGCCTCACTGATTAAGCATTGGTAACTGTCAG
ACCAAGTTTACTCATATATACTTTAGATTGATTTAAAACTTCATTTTTAATTTA
AAAGGATCTAGGTGAAGATCCTTTTTGATAATCTCATGACCAAAATCCCTTAA
CGTGAGTTTTCGTTCCACTGAGCGTCAGACCCCGTAGAAAAGATCAAAGGAT
CTTCTTGAGATCCTTTTTTTCTGCGCGTAATCTGCTGCTTGCAAACAAAAAAA
CCACCGCTACCAGCGGTGGTTTGTTTGCCGGATCAAGAGCTACCAACTCTTTT
TCCGAAGGTAACTGGCTTCAGCAGAGCGCAGATACCAAATACTGTTCTTCTAG
TGTAGCCGTAGTTAGGCCACCACTTCAAGAACTCTGTAGCACCGCCTACATAC
CTCGCTCTGCTAATCCTGTTACCAGTGGCTGCTGCCAGTGGCGATAAGTCGTG
TCTTACCGGGTTGGACTCAAGACGATAGTTACCGGATAAGGCGCAGCGGTCG
GGCTGAACGGGGGGTTCGTGCACACAGCCCAGCTTGGAGCGAACGACCTACA
CCGAACTGAGATACCTACAGCGTGAGCTATGAGAAAGCGCCACGCTTCCCGA
AGGGAGAAAGGCGGACAGGTATCCGGTAAGCGGCAGGGTCGGAACAGGAGA
GCGCACGAGGGAGCTTCCAGGGGGAAACGCCTGGTATCTTTATAGTCCTGTC
GGGTTTCGCCACCTCTGACTTGAGCGTCGATTTTTGTGATGCTCGTCAGGGGG
GCGGAGCCTATGGAAAAACGCCAGCAACGCGGCCTTTTTACGGTTCCTGGCC
TTTTGCTGGCCTTTTGCTCACATGT
**pRC124 AAV Two Vector dCas12a/dCpf1 (SEQ ID NO: 17)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
 
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atgCCCGCTGCGAAACGAGTTAAATTGGATggatcctcaaagctggagaaattcaccaactgttatagc
ctgtctaagaccctgcgcttcaaggcaatcccagtgggcaagacacaagagaacattgacaacaaacggctcctggtggagga
tgagaagagggctgaagattacaagggcgttaagaagctgctggataggtactatctgtcattcatcaacgatgtcctccacagta
tcaagctgaagaatctgaacaattacatttctctgttccggaagaagacacggaccgagaaggagaacaaagagctggagaatc
tggagatcaacctgaggaaagaaatagctaaggctttcaaagggaacgagggttacaagtccctgttcaagaaagacattatcg
agactattctgcctgagttcctggacgataaagatgagatcgccctcgtcaattccttcaatgggtttaccacagcctttaccggctt
cttcgacaatagagagaatatgttctctgaagaggccaaatccactagcatcgcctttcgctgcataaacgagaacctgactaggt
acatcagcaatatggacatctttgagaaagtcgatgccatattcgacaaacatgaggtgcaggagattaaggagaagatcctgaa
ctcagattacgatgtcgaagatttcttcgagggagagttcttcaacttcgtgctcacacaagagggcattgatgtgtacaatgcaatc
attggagggttcgtgacagagagtggcgagaagataaagggcctgaacgagtatatcaacctctacaaccagaaaaccaagca
gaaactgcctaagttcaagccactgtacaaacaagtgctctcagatagggaaagcctgagcttctacggtgaagggtatacatca
gatgaagaagtgctcgaagtgttccgcaacaccctcaataagaacagtgaaatcttctcttcaatcaagaagctggagaaactgtt
caagaatttcgatgagtactcctctgccggaatctttgtgaagaatggccctgcaatatccactattagcaaagacatctttggcga
gtggaacgttatcagggataagtggaatgccgagtacgatgatattcatctcaagaagaaagccgtggttacagagaaatacga
ggatgatagacgcaagagctttaagaagattggtagcttctctctcgaacagctgcaggagtacgccgacgctgacctgtcagtc
gtggagaaactcaaggagatcataatccagaaggtggatgaaatctacaaagtgtatggaagctctgagaaactcttcgatgcag
actttgttctggagaagagtctgaagaagaacgacgcagtggttgctatcatgaaggacctgctggattctgttaagtctttcgaga
attacattaaggcattctttggtgaagggaaggagacaaatagggacgagagcttctatggcgactttgttctggcctacgacatc
ctcctcaaggttgaccacatctatgacgctatacggaattacgttacccagaagccctatagcaaagacaagttcaagctgtatttc
cagaatccacagtttatgggtgggtgggataaagacaaagaaacagattacagggccactatcctgcggtacggcagcaaatac
tatctggctatcatggataagaagtacgccaaatgcctccagaagatcgacaaggacgacgtgggtaccagtCCAGCTG
CGAAACGAGTTAAATTGGATGGTGGTGGCGGGTCCTCAGGTTTGCCCAATGG
ATTGGATGGTGATGAAGACTTCTCAGACATCGCAGATATGGACTTTAGTGCCT
TGCTGAGCCAAATCTCATCTGGCTCATCAGGTCTTCCCAACGGATTGGATGGC
GATGAGGACTTTTCCGACATCGCAGACATGGACTTTTCCGCTCTGTTGAGCCA
GATAAGTTCTGGCTCCTCCGGACTCCCTAATGGCTTGGATGGAGATGAGGATT
TTAGCGATATAGCAGACATGGATTTTAGCGCATTGCTCTCACAAATCTCCAGT
GGCGGGGGTGGTAGTGGATTTTCAGTGGATACTTCCGCACTTCTCGATTTGTT
TTCTCCCAGTGTCACCGTCCCAGACATGAGTCTTCCTGACCTCGACTCTTCTCT
CGCCAGTATTCAGGAACTGCTCAGTCCACAGGAGCCTCCTCGCCCTCCTGAAG
CAGAGAATAGTTCCCCAGATTCAGGAAAGCAGCTGGTGCACTACACAGCACA
GCCACTGTTCCTCTTGGACCCTGGGAGTGTAGACACAGGCTCAAACGATCTCC
CCGTTCTTTTTGAACTTGGCGAAGGTTCCTATTTTAGCGAGGGGGACGGATTC
GCCGAGGACCCTACTATCAGTCTCCTTACAGGTAGCGAGCCACCCAAAGCTA
AAGACCCCACCGTGAGCTAGTGActcgagGATTCGTCAGTAGGGTTGTAAAGGT
TTTTCTTTTCCTGAGAAAACAACCTTTTGTTTTCTCAGGTTTTGCTTTTTGGCCT
TTCCCTAGCTTTAAAAAAAAAAAAGCAAAACTCACCGAGGCAGTTCCATAGG
ATGGCtagcAAGATCCTGGTATTGGTCTGCGAgaagagcgagctcttcaatcaacctctggattacaa
aatttgtgaaagattgactggtattcttaactatgttgctccttttacgctatgtggatacgctgctttaatgcctttgtatcatgctattgct
tcccgtatggctttcattttctcctccttgtataaatcctggttgctgtctctttatgaggagttgtggcccgttgtcaggcaacgtggcg
tggtgtgcactgtgtttgctgacgcaacccccactggttggggcattgccaccacctgtcagctcctttccgggactttcgctttccc
cctccctattgccacggcggaactcatcgccgcctgccttgcccgctgctggacaggggctcggctgttgggcactgacaattcc
gtggtgttgtcggggaaatcatcgtcctttccttggctgctcgcctgtgttgccacctggattctgcgcgggacgtccttctgctacg
tcccttcggccctcaatccagcggaccttccttcccgcggcctgctgccggctctgcggcctcttccgcgtcttcgccttcgccctc
agacgagtcggatctccctttgggccgcctccccgcCGGCCGCAGGAACCCCTAGTGATGGAGTTG
GCCACTCCCTCTCTGCGCGCTCGCTCGCTCACTGAGGCCGGGCGACCAAAGGT
CGCCCGACGCCCGGGCTTTGCCCGGGCGGCCTCAGTGAGCGAGCGAGCGCGC
AGCTGCCTGCAGGGGCGCCTGATGCGGTATTTTCTCCTTACGCATCTGTGCGG
 
TATTTCACACCGCATACGTCAAAGCAACCATAGTACGCGCCCTGTAGCGGCG
CATTAAGCGCGGCGGGTGTGGTGGTTACGCGCAGCGTGACCGCTACACTTGC
CAGCGCCTTAGCGCCCGCTCCTTTCGCTTTCTTCCCTTCCTTTCTCGCCACGTT
CGCCGGCTTTCCCCGTCAAGCTCTAAATCGGGGGCTCCCTTTAGGGTTCCGAT
TTAGTGCTTTACGGCACCTCGACCCCAAAAAACTTGATTTGGGTGATGGTTCA
CGTAGTGGGCCATCGCCCTGATAGACGGTTTTTCGCCCTTTGACGTTGGAGTC
CACGTTCTTTAATAGTGGACTCTTGTTCCAAACTGGAACAACACTCAACTCTA
TCTCGGGCTATTCTTTTGATTTATAAGGGATTTTGCCGATTTCGGTCTATTGGT
TAAAAAATGAGCTGATTTAACAAAAATTTAACGCGAATTTTAACAAAATATT
AACGTTTACAATTTTATGGTGCACTCTCAGTACAATCTGCTCTGATGCCGCAT
AGTTAAGCCAGCCCCGACACCCGCCAACACCCGCTGACGCGCCCTGACGGGC
TTGTCTGCTCCCGGCATCCGCTTACAGACAAGCTGTGACCGTCTCCGGGAGCT
GCATGTGTCAGAGGTTTTCACCGTCATCACCGAAACGCGCGAGACGAAAGGG
CCTCGTGATACGCCTATTTTTATAGGTTAATGTCATGATAATAATGGTTTCTTA
GACGTCAGGTGGCACTTTTCGGGGAAATGTGCGCGGAACCCCTATTTGTTTAT
TTTTCTAAATACATTCAAATATGTATCCGCTCATGAGACAATAACCCTGATAA
ATGCTTCAATAATATTGAAAAAGGAAGAGTATGAGTATTCAACATTTCCGTGT
CGCCCTTATTCCCTTTTTTGCGGCATTTTGCCTTCCTGTTTTTGCTCACCCAGA
AACGCTGGTGAAAGTAAAAGATGCTGAAGATCAGTTGGGTGCACGAGTGGGT
TACATCGAACTGGATCTCAACAGCGGTAAGATCCTTGAGAGTTTTCGCCCCGA
AGAACGTTTTCCAATGATGAGCACTTTTAAAGTTCTGCTATGTGGCGCGGTAT
TATCCCGTATTGACGCCGGGCAAGAGCAACTCGGTCGCCGCATACACTATTCT
CAGAATGACTTGGTTGAGTACTCACCAGTCACAGAAAAGCATCTTACGGATG
GCATGACAGTAAGAGAATTATGCAGTGCTGCCATAACCATGAGTGATAACAC
TGCGGCCAACTTACTTCTGACAACGATCGGAGGACCGAAGGAGCTAACCGCT
TTTTTGCACAACATGGGGGATCATGTAACTCGCCTTGATCGTTGGGAACCGGA
GCTGAATGAAGCCATACCAAACGACGAGCGTGACACCACGATGCCTGTAGCA
ATGGCAACAACGTTGCGCAAACTATTAACTGGCGAACTACTTACTCTAGCTTC
CCGGCAACAATTAATAGACTGGATGGAGGCGGATAAAGTTGCAGGACCACTT
CTGCGCTCGGCCCTTCCGGCTGGCTGGTTTATTGCTGATAAATCTGGAGCCGG
TGAGCGTGGAAGCCGCGGTATCATTGCAGCACTGGGGCCAGATGGTAAGCCC
TCCCGTATCGTAGTTATCTACACGACGGGGAGTCAGGCAACTATGGATGAAC
GAAATAGACAGATCGCTGAGATAGGTGCCTCACTGATTAAGCATTGGTAACT
GTCAGACCAAGTTTACTCATATATACTTTAGATTGATTTAAAACTTCATTTTTA
ATTTAAAAGGATCTAGGTGAAGATCCTTTTTGATAATCTCATGACCAAAATCC
CTTAACGTGAGTTTTCGTTCCACTGAGCGTCAGACCCCGTAGAAAAGATCAAA
GGATCTTCTTGAGATCCTTTTTTTCTGCGCGTAATCTGCTGCTTGCAAACAAAA
AAACCACCGCTACCAGCGGTGGTTTGTTTGCCGGATCAAGAGCTACCAACTCT
TTTTCCGAAGGTAACTGGCTTCAGCAGAGCGCAGATACCAAATACTGTTCTTC
TAGTGTAGCCGTAGTTAGGCCACCACTTCAAGAACTCTGTAGCACCGCCTACA
TACCTCGCTCTGCTAATCCTGTTACCAGTGGCTGCTGCCAGTGGCGATAAGTC
GTGTCTTACCGGGTTGGACTCAAGACGATAGTTACCGGATAAGGCGCAGCGG
TCGGGCTGAACGGGGGGTTCGTGCACACAGCCCAGCTTGGAGCGAACGACCT
ACACCGAACTGAGATACCTACAGCGTGAGCTATGAGAAAGCGCCACGCTTCC
CGAAGGGAGAAAGGCGGACAGGTATCCGGTAAGCGGCAGGGTCGGAACAGG
AGAGCGCACGAGGGAGCTTCCAGGGGGAAACGCCTGGTATCTTTATAGTCCT
GTCGGGTTTCGCCACCTCTGACTTGAGCGTCGATTTTTGTGATGCTCGTCAGG
GGGGCGGAGCCTATGGAAAAACGCCAGCAACGCGGCCTTTTTACGGTTCCTG
GCCTTTTGCTGGCCTTTTGCTCACATGT
**pRC126 AAV Two Vector dCas12a/dCpf1 (SEQ ID NO: 18)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
ATGCCCGCTGCGAAACGAGTTAAATTGGATGGTGGCGGGTCCTCAGGTT
TGCCCAATGGATTGGATGGTGATGAAGACTTCTCAGACATCGCAGATATGGA
CTTTAGTGCCTTGCTGAGCCAAATCTCATCTGGCTCATCAGGTCTTCCCAACG
GATTGGATGGCGATGAGGACTTTTCCGACATCGCAGACATGGACTTTTCCGCT
CTGTTGAGCCAGATAAGTTCTGGCTCCTCCGGACTCCCTAATGGCTTGGATGG
AGATGAGGATTTTAGCGATATAGCAGACATGGATTTTAGCGCATTGCTCTCAC
AAATCTCCAGTGGCGGGGGTGGTAGTGGATTTTCAGTGGATACTTCCGCACTT
CTCGATTTGTTTTCTCCCAGTGTCACCGTCCCAGACATGAGTCTTCCTGACCTC
GACTCTTCTCTCGCCAGTATTCAGGAACTGCTCAGTCCACAGGAGCCTCCTCG
CCCTCCTGAAGCAGAGAATAGTTCCCCAGATTCAGGAAAGCAGCTGGTGCAC
TACACAGCACAGCCACTGTTCCTCTTGGACCCTGGGAGTGTAGACACAGGCTC
AAACGATCTCCCCGTTCTTTTTGAACTTGGCGAAGGTTCCTATTTTAGCGAGG
GGGACGGATTCGCCGAGGACCCTACTATCAGTCTCCTTACAGGTAGCGAGCC
ACCCAAAGCTAAAGACCCCACCGTGAGCGGTGGTGGCGGGTCCGGTaacggtaact
acgagaagatcaattacaagctcctgccaggacctaacaagatgctgcccaaggtgttcttctccaagaaatggatggcctactat
aacccaagcgaggacattcagaagatatacaagaatgggacattcaagaagggcgatatgttcaacctcaacgactgccacaa
gctgattgatttcttcaaggatagcatttctcgctatcccaagtggtctaatgcatacgatttcaacttcagcgagactgagaagtaca
aagacatcgctggcttctaccgggaggtggaagagcaaggctataaggtgtcattcgaatccgcttctaagaaggaagtggata
agctcgtggaagagggtaagctgtacatgttccagatatacaacaaagacttcagcgataagagccacggcactccaaacctcc
atactatgtatttcaagctgctgtttgacgagaacaaccacggacagattaggctgtcaggaggcgcagaactcttcatgcgcaga
gcttcactgaagaaggaggaactcgttgtccacccagccaatagccctatagccaataagaatccagacaatcctaagaaaacc
actactctgtcttacgatgtgtataaggataagagattctctgaagatcagtacgaactgcacatacccattgccattaacaagtgcc
ctaagaacatcttcaagattaacacagaggttagagtgctcctgaaacacgacgataacccttatgttataggcattgctcgcgga
gagagaaacctgctgtacatcgtcgtggtggacggcaaaggcaacatcgtggaacagtacagtctcaatgaaatcattaacaatt
tcaacggaatccgcattaagaccgactaccattctctcctcgacaagaaggagaaagaaaggttcgaagcaagacagaattgga
caagtatagagaatatcaaagaactgaaggctgggtacatctctcaggttgtgcacaagatatgtgagctggtggagaagtacga
cgctgttatcgccctcgcggacctgaatagcggcttcaagaactccagggtgaaggtggagaagcaggtgtatcagaagttcga
gaagatgctgatcgacaagctcaactatatggtggacaagaaatccaatccttgcgctactggtggagccctgaagggctatcaa
atcaccaataagttcgaatctttcaagtctatgagcacccagaatggcttcatcttctacatacccgcatggctgacatccaagattg
atccctctaccggatttgttaatctgctcaagactaagtacacctctattgctgactcaaagaagttcatatcatcatttgaccgcatca
tgtacgtgccagaagaggacctgttcgagtttgccctggattacaagaatttctctcggactgacgccgactacatcaagaagtgg
aagctctactcttatggtaatcggattcgcatattccgcaatcccaagaagaataacgtgttcgattgggaggaagtttgcctcacc
agcgcttacaaggagctgttcaataagtatgggattaactaccagcagggcgacataagagccctgctgtgcgaacaatctgata
aggcattctattcctctttcatggcactgatgtcactgatgctgcaaatgcgcaattccatcaccggaagaacagacgtggcctttct
gatctctcctgtcaagaactcagatggcatcttctacgattcccgcaactatgaagcacaggagaatgctatcctgcctaagaatgc
cgatgcaaatggagcctataacatcgccagaaaggtcctctgggccataggacaattcaagaaagctgaagatgagaagctgg
 
acaaggtgaagatcgccatttcaaacaaagagtggctcgaatatgctcagacctcagtgaagcatggtaccCCCGCTGC
GAAACGAGTTAAATTGGATGGTGGTGGCGGGTCCTCAGGTTTGCCCAATGGA
TTGGATGGTGATGAAGACTTCTCAGACATCGCAGATATGGACTTTAGTGCCTT
GCTGAGCCAAATCTCATCTGGCTCATCAGGTCTTCCCAACGGATTGGATGGCG
ATGAGGACTTTTCCGACATCGCAGACATGGACTTTTCCGCTCTGTTGAGCCAG
ATAAGTTCTGGCTCCTCCGGACTCCCTAATGGCTTGGATGGAGATGAGGATTT
TAGCGATATAGCAGACATGGATTTTAGCGCATTGCTCTCACAAATCTCCAGTG
GCGGGGGTGGTAGTGGATTTTCAGTGGATACTTCCGCACTTCTCGATTTGTTT
TCTCCCAGTGTCACCGTCCCAGACATGAGTCTTCCTGACCTCGACTCTTCTCTC
GCCAGTATTCAGGAACTGCTCAGTCCACAGGAGCCTCCGCCCTCCTGAAGC
AGAGAATAGTTCCCCAGATTCAGGAAAGCAGCTGGTGCACTACACAGCACAG
CCACTGTTCCTCTTGGACCCTGGGAGTGTAGACACAGGCTCAAACGATCTCCC
CGTTCTTTTTGAACTTGGCGAAGGTTCCTATTTTAGCGAGGGGGACGGATTCG
CCGAGGACCCTACTATCAGTCTCCTTACAGGTAGCGAGCCACCCAAAGCTAA
AGACCCCACCGTGAGCTAGTGActcgagAATAAAAGATCTTTATTTTCATTAGAT
CTGTGTGTTGGTTTTTTGTGTCGGCCGCAGGAACCCCTAGTGATGGAGTTGGC
CACTCCCTCTCTGCGCGCTCGCTCGCTCACTGAGGCCGGGCGACCAAAGGTCG
CCCGACGCCCGGGCTTTGCCCGGGCGGCCTCAGTGAGCGAGCGAGCGCGCAG
CTGCCTGCAGGGGCGCCTGATGCGGTATTTTCTCCTTACGCATCTGTGCGGTA
TTTCACACCGCATACGTCAAAGCAACCATAGTACGCGCCCTGTAGCGGCGCA
TTAAGCGCGGCGGGTGTGGTGGTTACGCGCAGCGTGACCGCTACACTTGCCA
GCGCCTTAGCGCCCGCTCCTTTCGCTTTCTTCCCTTCCTTTCTCGCCACGTTCG
CCGGCTTTCCCCGTCAAGCTCTAAATCGGGGGCTCCCTTTAGGGTTCCGATTT
AGTGCTTTACGGCACCTCGACCCCAAAAAACTTGATTTGGGTGATGGTTCACG
TAGTGGGCCATCGCCCTGATAGACGGTTTTTCGCCCTTTGACGTTGGAGTCCA
CGTTCTTTAATAGTGGACTCTTGTTCCAAACTGGAACAACACTCAACTCTATC
TCGGGCTATTCTTTTGATTTATAAGGGATTTTGCCGATTTCGGTCTATTGGTTA
AAAAATGAGCTGATTTAACAAAAATTTAACGCGAATTTTAACAAAATATTAA
CGTTTACAATTTTATGGTGCACTCTCAGTACAATCTGCTCTGATGCCGCATAG
TTAAGCCAGCCCCGACACCCGCCAACACCCGCTGACGCGCCCTGACGGGCTT
GTCTGCTCCCGGCATCCGCTTACAGACAAGCTGTGACCGTCTCCGGGAGCTGC
ATGTGTCAGAGGTTTTCACCGTCATCACCGAAACGCGCGAGACGAAAGGGCC
TCGTGATACGCCTATTTTTATAGGTTAATGTCATGATAATAATGGTTTCTTAGA
CGTCAGGTGGCACTTTTCGGGGAAATGTGCGCGGAACCCCTATTTGTTTATTT
TTCTAAATACATTCAAATATGTATCCGCTCATGAGACAATAACCCTGATAAAT
GCTTCAATAATATTGAAAAAGGAAGAGTATGAGTATTCAACATTTCCGTGTCG
CCCTTATTCCCTTTTTTGCGGCATTTTGCCTTCCTGTTTTTGCTCACCCAGAAA
CGCTGGTGAAAGTAAAAGATGCTGAAGATCAGTTGGGTGCACGAGTGGGTTA
CATCGAACTGGATCTCAACAGCGGTAAGATCCTTGAGAGTTTTCGCCCCGAA
GAACGTTTTCCAATGATGAGCACTTTTAAAGTTCTGCTATGTGGCGCGGTATT
ATCCCGTATTGACGCCGGGCAAGAGCAACTCGGTCGCCGCATACACTATTCTC
AGAATGACTTGGTTGAGTACTCACCAGTCACAGAAAAGCATCTTACGGATGG
CATGACAGTAAGAGAATTATGCAGTGCTGCCATAACCATGAGTGATAACACT
GCGGCCAACTTACTTCTGACAACGATCGGAGGACCGAAGGAGCTAACCGCTT
TTTTGCACAACATGGGGGATCATGTAACTCGCCTTGATCGTTGGGAACCGGAG
CTGAATGAAGCCATACCAAACGACGAGCGTGACACCACGATGCCTGTAGCAA
TGGCAACAACGTTGCGCAAACTATTAACTGGCGAACTACTTACTCTAGCTTCC
CGGCAACAATTAATAGACTGGATGGAGGCGGATAAAGTTGCAGGACCACTTC
TGCGCTCGGCCCTTCCGGCTGGCTGGTTTATTGCTGATAAATCTGGAGCCGGT
GAGCGTGGAAGCCGCGGTATCATTGCAGCACTGGGGCCAGATGGTAAGCCCT
 
CCCGTATCGTAGTTATCTACACGACGGGGAGTCAGGCAACTATGGATGAACG
AAATAGACAGATCGCTGAGATAGGTGCCTCACTGATTAAGCATTGGTAACTG
TCAGACCAAGTTTACTCATATATACTTTAGATTGATTTAAAACTTCATTTTTAA
TTTAAAAGGATCTAGGTGAAGATCCTTTTTGATAATCTCATGACCAAAATCCC
TTAACGTGAGTTTTCGTTCCACTGAGCGTCAGACCCCGTAGAAAAGATCAAA
GGATCTTCTTGAGATCCTTTTTTTCTGCGCGTAATCTGCTGCTTGCAAACAAAA
AAACCACCGCTACCAGCGGTGGTTTGTTTGCCGGATCAAGAGCTACCAACTCT
TTTTCCGAAGGTAACTGGCTTCAGCAGAGCGCAGATACCAAATACTGTTCTTC
TAGTGTAGCCGTAGTTAGGCCACCACTTCAAGAACTCTGTAGCACCGCCTACA
TACCTCGCTCTGCTAATCCTGTTACCAGTGGCTGCTGCCAGTGGCGATAAGTC
GTGTCTTACCGGGTTGGACTCAAGACGATAGTTACCGGATAAGGCGCAGCGG
TCGGGCTGAACGGGGGGTTCGTGCACACAGCCCAGCTTGGAGCGAACGACCT
ACACCGAACTGAGATACCTACAGCGTGAGCTATGAGAAAGCGCCACGCTTCC
CGAAGGGAGAAAGGCGGACAGGTATCCGGTAAGCGGCAGGGTCGGAACAGG
AGAGCGCACGAGGGAGCTTCCAGGGGGAAACGCCTGGTATCTTTATAGTCCT
GTCGGGTTTCGCCACCTCTGACTTGAGCGTCGATTTTTGTGATGCTCGTCAGG
GGGGCGGAGCCTATGGAAAAACGCCAGCAACGCGGCCTTTTTACGGTTCCTG
GCCTTTTGCTGGCCTTTTGCTCACATGT
**pGW071-pAAV_EFSLTR-Cd80-T2A-light-P2A-cxcl10-E2A-41bb-F2A-Il2-sPA (SEQ ID NO: 24)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atggcttgcaattgtcagttgatgcaggatacaccactcctcaagtttccatgtccaaggctcattcttctctttgtgctgctgattcgtc
tttcacaagtgtcttcagatgttgatgaacaactgtccaagtcagtgaaagataaggtattgctgccttgccgttacaactctcctcat
gaagatgagtctgaagaccgaatctactggcaaaaacatgacaaagtggtgctgtctgtcattgTtgggaaactaaaagtgtggc
ccgagtataagaaccggactttatatgacaacactacctactctcttatcatcctgggcctggtcctttcagaccggggcacataca
gctgtgtcgttcaaaagaaggaaagaggaacgtatgaagttaaacacttggctttagtaaagttgtccatcaaagctgacttctcta
cccccaacataactgagtctggaaacccatctgcagacactaaaaggattacctgctttgcttccgggggtttcccaaagcctcgc
ttctcttggttggaaaatggaagagaattacctggcatcaatacgacaatttcccaggatcctgaatctgaattgtacaccattagta
gccaactagatttcaatacgactcgcaaccacaccattaagtgtctcattaaatatggagatgctcacgtgtcagaggacttcacct
gggaaaaacccccagaagaccctcctgatagcaagaacacacttgtgctctttggggcaggattcggcgcagtaataacagtcg
tcgtcatcgttgtcatcatcaaatgcttctgtaagcacagaagctgtttcagaagaaatgaggcaagcagagaaacaaacaacag
ccttaccttcgggcctgaagaagcattagctgaacagaccgtcttccttcgtacgGGCAGTGGAGAGGGCAGA
GGAAGTCTGCTAACATGCGGTGACGTCGAGGAGAATCCTGGCCCAatggagagtgt
ggtacagccttcagtgtttgtggtggatggacagacggacatcccattcaggcggctggaacagaaccaccggagacggcgct
gtggcactgtccaggtcagcctggccctggtgctgctgctaggtgctgggctggccactcagggctggtttctcctgagactgca
tcaacgtcttggagacatagtagctcatctgccagatggaggcaaaggctcctgggagaagctgatacaagatcaacgatctca
ccaggccaacccagcagcacatcttacaggagccaacgccagcttgataggtattggtggacctctgttatgggagacacgact
 
tggcctggccttcttgaggggcttgacgtatcatgatggggccctggtgaccatggagcccggttactactatgtgtactccaaag
tgcagctgagcggcgtgggctgcccccaggggctggccaatggcctccccatcacccatggactatacaagcgcacatcccg
ctacccgaaggagttagaactgctggtcagtcggcggtcaccctgtggccgggccaacagctcccgagtctggtgggacagca
gcttcctgggcggcgtggtacatctggaggctggggaagaggtggtggtccgcgtgcctggaaaccgcctggtcagaccacgt
gacggcaccaggtcctatttcggagctttcatggtcactagtGGATCCGGCGCAACAAACTTCTCTCTG
CTGAAACAAGCCGGAGATGTCGAAGAGAATCCTGGACCGatgaacccaagtgctgccgtc
attttctgcctcatcctgctgggtctgagtgggactcaagggatccctctcgcaaggacggtccgctgcaactgcatccatatcgat
gacgggccagtgagaatgagggccatagggaagcttgaaatcatccctgcgagcctatcctgcccacgtgttgagatcattgcc
acgatgaaaaagaatgatgagcagagatgtctgaatccggaatctaagaccatcaagaatttaatgaaagcgtttagccaaaaaa
ggtctaaaagggctcctgctagcagcggtacccagtgcaccaactacgccctgctgaagctggccggcgatgtggagagcaa
ccccgggcccatggaccagcacacacttgatgtggaggataccgcggatgccagacatccagcaggtacttcgtgcccctcgg
atgcggcgctcctcagagataccgggctcctcgcggacgctgcgctcctctcagatactgtgcgccccacaaatgccgcgctcc
ccacggatgctgcctaccctgcggttaatgttcgggatcgcgaggccgcgtggccgcctgcactgaacttctgttcccgccaccc
aaagctctatggcctagtcgctttggttttgctgcttctgatcgccgcctgtgttcctatcttcacccgcaccgagcctcggccagcg
ctcacaatcaccacctcgcccaacctgggtacccgagagaataatgcagaccaggtcacccctgtttcccacattggctgcccca
acactacacaacagggctctcctgtgttcgccaagctactggctaaaaaccaagcatcgttgtgcaatacaactctgaactggcac
agccaagatggagctgggagctcatacctatctcaaggtctgaggtacgaagaagacaaaaaggagttggtggtagacagtcc
cgggctctactacgtatttttggaactgaagctcagtccaacattcacaaacacaggccacaaggtgcagggctgggtctctcttg
ttttgcaagcaaagcctcaggtagatgactttgacaacttggccctgacagtggaactgttcccttgctccatggagaacaagttag
tggaccgttcctggagtcaactgttgctcctgaaggctggccaccgcctcagtgtgggtctgagggcttatctgcatggagccca
ggatgcatacagagactgggagctgtcttatcccaacaccaccagctttggactctttcttgtgaaacccgacaacccatgggaa
ggcatatgcggtaccgtgaagcagaccctgaacttcgatctgctgaagctggccggcgatgtggagagcaaccccgggcccat
gtacagcatgcagctcgcatcctgtgtcacattgacacttgtgctccttgtcaacagcgcacccacttcaagctccacttcaagctct
acagcggaagcacagcagcagcagcagcagcagcagcagcagcagcagcagcacctggagcagctgttgatggacctacagga
gctcctgagcaggatggagaattacaggaacctgaaactccccaggatgctcaccttcaaattttacttgcccaagcaggccaca
gaattgaaagatcttcagtgcctagaagatgaacttggacctctgcggcatgttctggatttgactcaaagcaaaagctttcaattgg
aagatgctgagaatttcatcagcaatatcagagtaactgttgtaaaactaaagggctctgacaacacatttgagtgccaattcgatg
atgagtcagcaactgtggtggactttctgaggagatggatagccttctgtcaaagcatcatctcaacaagccctcaataaGAAT
TCAATAAAAGATCTTTATTTTCATTAGATCTGTGTGTTGGTTTTTTGTGTGCGG
CCGCAGGAACCCCTAGTGATGGAGTTGGCCACTCCCTCTCTGCGCGCTCGCTC
GCTCACTGAGGCCGGGCGACCAAAGGTCGCCCGACGCCCGGGCTTTGCCCGG
GCGGCCTCAGTGAGCGAGCGAGCGCGCAGCTGCCTGCAGGGGCGCCTGATGC
GGTATTTTCTCCTTACGCATCTGTGCGGTATTTCACACCGCATACGTCAAAGC
AACCATAGTACGCGCCCTGTAGCGGCGCATTAAGCGCGGCGGGTGTGGTGGT
TACGCGCAGCGTGACCGCTACACTTGCCAGCGCCTTAGCGCCCGCTCCTTTCG
CTTTCTTCCCTTCCTTTCTCGCCACGTTCGCCGGCTTTCCCCGTCAAGCTCTAA
ATCGGGGGCTCCCTTTAGGGTTCCGATTTAGTGCTTTACGGCACCTCGACCCC
AAAAAACTTGATTTGGGTGATGGTTCACGTAGTGGGCCATCGCCCTGATAGA
CGGTTTTTCGCCCTTTGACGTTGGAGTCCACGTTCTTTAATAGTGGACTCTTGT
TCCAAACTGGAACAACACTCAACTCTATCTCGGGCTATTCTTTTGATTTATAA
GGGATTTTGCCGATTTCGGTCTATTGGTTAAAAAATGAGCTGATTTAACAAAA
ATTTAACGCGAATTTTAACAAAATATTAACGTTTACAATTTTATGGTGCACTC
TCAGTACAATCTGCTCTGATGCCGCATAGTTAAGCCAGCCCCGACACCCGCCA
ACACCCGCTGACGCGCCCTGACGGGCTTGTCTGCTCCCGGCATCCGCTTACAG
ACAAGCTGTGACCGTCTCCGGGAGCTGCATGTGTCAGAGGTTTTCACCGTCAT
CACCGAAACGCGCGAGACGAAAGGGCCTCGTGATACGCCTATTTTTATAGGT
TAATGTCATGATAATAATGGTTTCTTAGACGTCAGGTGGCACTTTTCGGGGAA
ATGTGCGCGGAACCCCTATTTGTTTATTTTTCTAAATACATTCAAATATGTATC
CGCTCATGAGACAATAACCCTGATAAATGCTTCAATAATATTGAAAAAGGAA
GAGTATGAGTATTCAACATTTCCGTGTCGCCCTTATTCCCTTTTTTGCGGCATT
 
TTGCCTTCCTGTTTTTGCTCACCCAGAAACGCTGGTGAAAGTAAAAGATGCTG
AAGATCAGTTGGGTGCACGAGTGGGTTACATCGAACTGGATCTCAACAGCGG
TAAGATCCTTGAGAGTTTTCGCCCCGAAGAACGTTTTCCAATGATGAGCACTT
TTAAAGTTCTGCTATGTGGCGCGGTATTATCCCGTATTGACGCCGGGCAAGAG
CAACTCGGTCGCCGCATACACTATTCTCAGAATGACTTGGTTGAGTACTCACC
AGTCACAGAAAAGCATCTTACGGATGGCATGACAGTAAGAGAATTATGCAGT
GCTGCCATAACCATGAGTGATAACACTGCGGCCAACTTACTTCTGACAACGAT
CGGAGGACCGAAGGAGCTAACCGCTTTTTTGCACAACATGGGGGATCATGTA
ACTCGCCTTGATCGTTGGGAACCGGAGCTGAATGAAGCCATACCAAACGACG
AGCGTGACACCACGATGCCTGTAGCAATGGCAACAACGTTGCGCAAACTATT
AACTGGCGAACTACTTACTCTAGCTTCCCGGCAACAATTAATAGACTGGATGG
AGGCGGATAAAGTTGCAGGACCACTTCTGCGCTCGGCCCTTCCGGCTGGCTG
GTTTATTGCTGATAAATCTGGAGCCGGTGAGCGTGGAAGCCGCGGTATCATTG
CAGCACTGGGGCCAGATGGTAAGCCCTCCCGTATCGTAGTTATCTACACGAC
GGGGAGTCAGGCAACTATGGATGAACGAAATAGACAGATCGCTGAGATAGG
TGCCTCACTGATTAAGCATTGGTAACTGTCAGACCAAGTTTACTCATATATAC
TTTAGATTGATTTAAAACTTCATTTTTAATTTAAAAGGATCTAGGTGAAGATC
CTTTTTGATAATCTCATGACCAAAATCCCTTAACGTGAGTTTTCGTTCCACTGA
GCGTCAGACCCCGTAGAAAAGATCAAAGGATCTTCTTGAGATCCTTTTTTTCT
GCGCGTAATCTGCTGCTTGCAAACAAAAAAACCACCGCTACCAGCGGTGGTT
TGTTTGCCGGATCAAGAGCTACCAACTCTTTTTCCGAAGGTAACTGGCTTCAG
CAGAGCGCAGATACCAAATACTGTTCTTCTAGTGTAGCCGTAGTTAGGCCACC
ACTTCAAGAACTCTGTAGCACCGCCTACATACCTCGCTCTGCTAATCCTGTTA
CCAGTGGCTGCTGCCAGTGGCGATAAGTCGTGTCTTACCGGGTTGGACTCAAG
ACGATAGTTACCGGATAAGGCGCAGCGGTCGGGCTGAACGGGGGGTTCGTGC
ACACAGCCCAGCTTGGAGCGAACGACCTACACCGAACTGAGATACCTACAGC
GTGAGCTATGAGAAAGCGCCACGCTTCCCGAAGGGAGAAAGGCGGACAGGT
ATCCGGTAAGCGGCAGGGTCGGAACAGGAGAGCGCACGAGGGAGCTTCCAG
GGGGAAACGCCTGGTATCTTTATAGTCCTGTCGGGTTTCGCCACCTCTGACTT
GAGCGTCGATTTTTGTGATGCTCGTCAGGGGGGCGGAGCCTATGGAAAAACG
CCAGCAACGCGGCCTTTTTACGGTTCCTGGCCTTTTGCTGGCCTTTTGCTCACA
TGT
**pGW072-pAAV_EFSLTR-IFNg-sPA (SEQ ID NO: 25)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atgaacgctacacactgcatcttggctttgcagctcttcctcatggctgtttctggctgttactgccacggcacagtcattgaaagcct
agaaagtctgaataactattttaactcaagtggcatagatgtggaagaaaagagtctcttcttggatatctggaggaactggcaaaa
ggatggtgacatgaaaatcctgcagagccagattatctctttctacctcagactctttgaagtcttgaaagacaatcaggccatcag
 
caacaacataagcgtcattgaatcacacctgattactaccttcttcagcaacagcaaggcgaaaaaggatgcattcatgagtattg
ccaagtttgaggtcaacaacccacaggtccagcgccaagcattcaatgagctcatccgagtggtccaccagctgttgccggaat
ccagcctcaggaagcggaaaaggagtcgctgctgaGAATTCAATAAAAGATCTTTATTTTCATTA
GATCTGTGTGTTGGTTTTTTGTGTGCGGCCGCAGGAACCCCTAGTGATGGAGT
TGGCCACTCCCTCTCTGCGCGCTCGCTCGCTCACTGAGGCCGGGCGACCAAAG
GTCGCCCGACGCCCGGGCTTTGCCCGGGCGGCCTCAGTGAGCGAGCGAGCGC
GCAGCTGCCTGCAGGGGCGCCTGATGCGGTATTTTCTCCTTACGCATCTGTGC
GGTATTTCACACCGCATACGTCAAAGCAACCATAGTACGCGCCCTGTAGCGG
CGCATTAAGCGCGGCGGGTGTGGTGGTTACGCGCAGCGTGACCGCTACACTT
GCCAGCGCCTTAGCGCCCGCTCCTTTCGCTTTCTTCCCTTCCTTTCTCGCCACG
TTCGCCGGCTTTCCCCGTCAAGCTCTAAATCGGGGGCTCCCTTTAGGGTTCCG
ATTTAGTGCTTTACGGCACCTCGACCCCAAAAAACTTGATTTGGGTGATGGTT
CACGTAGTGGGCCATCGCCCTGATAGACGGTTTTTCGCCCTTTGACGTTGGAG
TCCACGTTCTTTAATAGTGGACTCTTGTTCCAAACTGGAACAACACTCAACTC
TATCTCGGGCTATTCTTTTGATTTATAAGGGATTTTGCCGATTTCGGTCTATTG
GTTAAAAAATGAGCTGATTTAACAAAAATTTAACGCGAATTTTAACAAAATA
TTAACGTTTACAATTTTATGGTGCACTCTCAGTACAATCTGCTCTGATGCCGC
ATAGTTAAGCCAGCCCCGACACCCGCCAACACCCGCTGACGCGCCCTGACGG
GCTTGTCTGCTCCCGGCATCCGCTTACAGACAAGCTGTGACCGTCTCCGGGAG
CTGCATGTGTCAGAGGTTTTCACCGTCATCACCGAAACGCGCGAGACGAAAG
GGCCTCGTGATACGCCTATTTTTATAGGTTAATGTCATGATAATAATGGTTTCT
TAGACGTCAGGTGGCACTTTTCGGGGAAATGTGCGCGGAACCCCTATTTGTTT
ATTTTTCTAAATACATTCAAATATGTATCCGCTCATGAGACAATAACCCTGAT
AAATGCTTCAATAATATTGAAAAAGGAAGAGTATGAGTATTCAACATTTCCG
TGTCGCCCTTATTCCCTTTTTTGCGGCATTTTGCCTTCCTGTTTTTGCTCACCCA
GAAACGCTGGTGAAAGTAAAAGATGCTGAAGATCAGTTGGGTGCACGAGTGG
GTTACATCGAACTGGATCTCAACAGCGGTAAGATCCTTGAGAGTTTTCGCCCC
GAAGAACGTTTTCCAATGATGAGCACTTTTAAAGTTCTGCTATGTGGCGCGGT
ATTATCCCGTATTGACGCCGGGCAAGAGCAACTCGGTCGCCGCATACACTATT
CTCAGAATGACTTGGTTGAGTACTCACCAGTCACAGAAAAGCATCTTACGGA
TGGCATGACAGTAAGAGAATTATGCAGTGCTGCCATAACCATGAGTGATAAC
ACTGCGGCCAACTTACTTCTGACAACGATCGGAGGACCGAAGGAGCTAACCG
CTTTTTTGCACAACATGGGGGATCATGTAACTCGCCTTGATCGTTGGGAACCG
GAGCTGAATGAAGCCATACCAAACGACGAGCGTGACACCACGATGCCTGTAG
CAATGGCAACAACGTTGCGCAAACTATTAACTGGCGAACTACTTACTCTAGCT
TCCCGGCAACAATTAATAGACTGGATGGAGGCGGATAAAGTTGCAGGACCAC
TTCTGCGCTCGGCCCTTCCGGCTGGCTGGTTTATTGCTGATAAATCTGGAGCC
GGTGAGCGTGGAAGCCGCGGTATCATTGCAGCACTGGGGCCAGATGGTAAGC
CCTCCCGTATCGTAGTTATCTACACGACGGGGAGTCAGGCAACTATGGATGA
ACGAAATAGACAGATCGCTGAGATAGGTGCCTCACTGATTAAGCATTGGTAA
CTGTCAGACCAAGTTTACTCATATATACTTTAGATTGATTTAAAACTTCATTTT
TAATTTAAAAGGATCTAGGTGAAGATCCTTTTTGATAATCTCATGACCAAAAT
CCCTTAACGTGAGTTTTCGTTCCACTGAGCGTCAGACCCCGTAGAAAAGATCA
AAGGATCTTCTTGAGATCCTTTTTTTCTGCGCGTAATCTGCTGCTTGCAAACAA
AAAAACCACCGCTACCAGCGGTGGTTTGTTTGCCGGATCAAGAGCTACCAAC
TCTTTTTCCGAAGGTAACTGGCTTCAGCAGAGCGCAGATACCAAATACTGTTC
TTCTAGTGTAGCCGTAGTTAGGCCACCACTTCAAGAACTCTGTAGCACCGCCT
ACATACCTCGCTCTGCTAATCCTGTTACCAGTGGCTGCTGCCAGTGGCGATAA
GTCGTGTCTTACCGGGTTGGACTCAAGACGATAGTTACCGGATAAGGCGCAG
CGGTCGGGCTGAACGGGGGGTTCGTGCACACAGCCCAGCTTGGAGCGAACGA
 
CCTACACCGAACTGAGATACCTACAGCGTGAGCTATGAGAAAGCGCCACGCT
TCCCGAAGGGAGAAAGGCGGACAGGTATCCGGTAAGCGGCAGGGTCGGAAC
AGGAGAGCGCACGAGGGAGCTTCCAGGGGGAAACGCCTGGTATCTTTATAGT
CCTGTCGGGTTTCGCCACCTCTGACTTGAGCGTCGATTTTTGTGATGCTCGTCA
GGGGGGCGGAGCCTATGGAAAAACGCCAGCAACGCGGCCTTTTTACGGTTCC
TGGCCTTTTGCTGGCCTTTTGCTCACATGT
**pGW073-pAAV_EFSLTR-Cd80-T2A-light-P2A-cxcl10-E2A-41bb-F2A-Gitrl (SEQ ID NO: 26)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atggcttgcaattgtcagttgatgcaggatacaccactcctcaagtttccatgtccaaggctcattcttctctttgtgctgctgattcgtc
tttcacaagtgtcttcagatgttgatgaacaactgtccaagtcagtgaaagataaggtattgctgccttgccgttacaactctcctcat
gaagatgagtctgaagaccgaatctactggcaaaaacatgacaaagtggtgctgtctgtcattgTtgggaaactaaaagtgtggc
ccgagtataagaaccggactttatatgacaacactacctactctcttatcatcctgggcctggtcctttcagaccggggcacataca
gctgtgtcgttcaaaagaaggaaagaggaacgtatgaagttaaacacttggctttagtaaagttgtccatcaaagctgacttctcta
cccccaacataactgagtctggaaacccatctgcagacactaaaaggattacctgctttgcttccgggggtttcccaaagcctcgc
ttctcttggttggaaaatggaagagaattacctggcatcaatacgacaatttcccaggatcctgaatctgaattgtacaccattagta
gccaactagatttcaatacgactcgcaaccacaccattaagtgtctcattaaatatggagatgctcacgtgtcagaggacttcacct
gggaaaaacccccagaagaccctcctgatagcaagaacacacttgtgctctttggggcaggattcggcgcagtaataacagtcg
tcgtcatcgttgtcatcatcaaatgcttctgtaagcacagaagctgtttcagaagaaatgaggcaagcagagaaacaaacaacag
ccttaccttcgggcctgaagaagcattagctgaacagaccgtcttccttcgtacgGGCAGTGGAGAGGGCAGA
GGAAGTCTGCTAACATGCGGTGACGTCGAGGAGAATCCTGGCCCAatggagagtgt
ggtacagccttcagtgtttgtggtggatggacagacggacatcccattcaggcggctggaacagaaccaccggagacggcgct
gtggcactgtccaggtcagcctggccctggtgctgctgctaggtgctgggctggccactcagggctggtttctcctgagactgca
tcaacgtcttggagacatagtagctcatctgccagatggaggcaaaggctcctgggagaagctgatacaagatcaacgatctca
ccaggccaacccagcagcacatcttacaggagccaacgccagcttgataggtattggtggacctctgttatgggagacacgact
tggcctggccttcttgaggggcttgacgtatcatgatggggccctggtgaccatggagcccggttactactatgtgtactccaaag
tgcagctgagcggcgtgggctgcccccaggggctggccaatggcctccccatcacccatggactatacaagcgcacatcccg
ctacccgaaggagttagaactgctggtcagtcggcggtcaccctgtggccgggccaacagctcccgagtctggtgggacagca
gcttcctgggcggcgtggtacatctggaggctggggaagaggtggtggtccgcgtgcctggaaaccgcctggtcagaccacgt
gacggcaccaggtcctatttcggagctttcatggtcactagtGGATCCGGCGCAACAAACTTCTCTCTG
CTGAAACAAGCCGGAGATGTCGAAGAGAATCCTGGACCGatgaacccaagtgctgccgtc
attttctgcctcatcctgctgggtctgagtgggactcaagggatccctctcgcaaggacggtccgctgcaactgcatccatatcgat
gacgggccagtgagaatgagggccatagggaagcttgaaatcatccctgcgagcctatcctgcccacgtgttgagatcattgcc
acgatgaaaaagaatgatgagcagagatgtctgaatccggaatctaagaccatcaagaatttaatgaaagcgtttagccaaaaaa
ggtctaaaagggctcctgctagcagcggtacccagtgcaccaactacgccctgctgaagctggccggcgatgtggagagcaa
ccccgggcccatggaccagcacacacttgatgtggaggataccgcggatgccagacatccagcaggtacttcgtgcccctcgg
atgcggcgctcctcagagataccgggctcctcgcggacgctgcgctcctctcagatactgtgcgccccacaaatgccgcgctcc
 
ccacggatgctgcctaccctgcggttaatgttcgggatcgcgaggccgcgtggccgcctgcactgaacttctgttcccgccaccc
aaagctctatggcctagtcgctttggttttgctgcttctgatcgccgcctgtgttcctatcttcacccgcaccgagcctcggccagcg
ctcacaatcaccacctcgcccaacctgggtacccgagagaataatgcagaccaggtcacccctgtttcccacattggctgcccca
acactacacaacagggctctcctgtgttcgccaagctactggctaaaaaccaagcatcgttgtgcaatacaactctgaactggcac
agccaagatggagctgggagctcatacctatctcaaggtctgaggtacgaagaagacaaaaaggagttggtggtagacagtcc
cgggctctactacgtatttttggaactgaagctcagtccaacattcacaaacacaggccacaaggtgcagggctgggtctctcttg
ttttgcaagcaaagcctcaggtagatgactttgacaacttggccctgacagtggaactgttcccttgctccatggagaacaagttag
tggaccgttcctggagtcaactgttgctcctgaaggctggccaccgcctcagtgtgggtctgagggcttatctgcatggagccca
ggatgcatacagagactgggagctgtcttatcccaacaccaccagctttggactctttcttgtgaaacccgacaacccatgggaa
ggcatatgcggtaccgtgaagcagaccctgaacttcgatctgctgaagctggccggcgatgtggagagcaaccccgggcccat
ggaggaaatgcctttgagagagtcaagtcctcaaagggcagagaggtgcaagaagtcatggctcttgtgcatagtggctctgtta
ctgatgctgctctgttctttgggtacactgatctatacttcactcaagccaactgccatcgagtcctgcatggttaagtttgaactatca
tcctcaaaatggcacatgacatctcccaaacctcactgtgtgaatacgacatctgatgggaagctgaagatactgcagagtggca
catatttaatctacggccaagtgattcctgtggataagaaatacataaaagacaatgcccccttcgtagtacagatatataaaaaga
atgatgtcctacaaactctaatgaatgattttcaaatcttgcctataggaggggtttatgaactgcatgctggagataacatatatctg
aagttcaactctaaagaccatattcagaaaaataacacatactgggggatcatcttaatgcctgatctaccattcatctcttagGAA
TTCAATAAAAGATCTTTATTTTCATTAGATCTGTGTGTTGGTTTTTTGTGTGCG
GCCGCAGGAACCCCTAGTGATGGAGTTGGCCACTCCCTCTCTGCGCGCTCGCT
CGCTCACTGAGGCCGGGCGACCAAAGGTCGCCCGACGCCCGGGCTTTGCCCG
GGCGGCCTCAGTGAGCGAGCGAGCGCGCAGCTGCCTGCAGGGGCGCCTGATG
CGGTATTTTCTCCTTACGCATCTGTGCGGTATTTCACACCGCATACGTCAAAG
CAACCATAGTACGCGCCCTGTAGCGGCGCATTAAGCGCGGCGGGTGTGGTGG
TTACGCGCAGCGTGACCGCTACACTTGCCAGCGCCTTAGCGCCCGCTCCTTTC
GCTTTCTTCCCTTCCTTTCTCGCCACGTTCGCCGGCTTTCCCCGTCAAGCTCTA
AATCGGGGGCTCCCTTTAGGGTTCCGATTTAGTGCTTTACGGCACCTCGACCC
CAAAAAACTTGATTTGGGTGATGGTTCACGTAGTGGGCCATCGCCCTGATAG
ACGGTTTTTCGCCCTTTGACGTTGGAGTCCACGTTCTTTAATAGTGGACTCTTG
TTCCAAACTGGAACAACACTCAACTCTATCTCGGGCTATTCTTTTGATTTATA
AGGGATTTTGCCGATTTCGGTCTATTGGTTAAAAAATGAGCTGATTTAACAAA
AATTTAACGCGAATTTTAACAAAATATTAACGTTTACAATTTTATGGTGCACT
CTCAGTACAATCTGCTCTGATGCCGCATAGTTAAGCCAGCCCCGACACCCGCC
AACACCCGCTGACGCGCCCTGACGGGCTTGTCTGCTCCCGGCATCCGCTTACA
GACAAGCTGTGACCGTCTCCGGGAGCTGCATGTGTCAGAGGTTTTCACCGTCA
TCACCGAAACGCGCGAGACGAAAGGGCCTCGTGATACGCCTATTTTTATAGG
TTAATGTCATGATAATAATGGTTTCTTAGACGTCAGGTGGCACTTTTCGGGGA
AATGTGCGCGGAACCCCTATTTGTTTATTTTTCTAAATACATTCAAATATGTAT
CCGCTCATGAGACAATAACCCTGATAAATGCTTCAATAATATTGAAAAAGGA
AGAGTATGAGTATTCAACATTTCCGTGTCGCCCTTATTCCCTTTTTTGCGGCAT
TTTGCCTTCCTGTTTTTGCTCACCCAGAAACGCTGGTGAAAGTAAAAGATGCT
GAAGATCAGTTGGGTGCACGAGTGGGTTACATCGAACTGGATCTCAACAGCG
GTAAGATCCTTGAGAGTTTTCGCCCCGAAGAACGTTTTCCAATGATGAGCACT
TTTAAAGTTCTGCTATGTGGCGCGGTATTATCCCGTATTGACGCCGGGCAAGA
GCAACTCGGTCGCCGCATACACTATTCTCAGAATGACTTGGTTGAGTACTCAC
CAGTCACAGAAAAGCATCTTACGGATGGCATGACAGTAAGAGAATTATGCAG
TGCTGCCATAACCATGAGTGATAACACTGCGGCCAACTTACTTCTGACAACGA
TCGGAGGACCGAAGGAGCTAACCGCTTTTTTGCACAACATGGGGGATCATGT
AACTCGCCTTGATCGTTGGGAACCGGAGCTGAATGAAGCCATACCAAACGAC
GAGCGTGACACCACGATGCCTGTAGCAATGGCAACAACGTTGCGCAAACTAT
TAACTGGCGAACTACTTACTCTAGCTTCCCGGCAACAATTAATAGACTGGATG
GAGGCGGATAAAGTTGCAGGACCACTTCTGCGCTCGGCCCTTCCGGCTGGCT
 
GGTTTATTGCTGATAAATCTGGAGCCGGTGAGCGTGGAAGCCGCGGTATCATT
GCAGCACTGGGGCCAGATGGTAAGCCCTCCCGTATCGTAGTTATCTACACGA
CGGGGAGTCAGGCAACTATGGATGAACGAAATAGACAGATCGCTGAGATAG
GTGCCTCACTGATTAAGCATTGGTAACTGTCAGACCAAGTTTACTCATATATA
CTTTAGATTGATTTAAAACTTCATTTTTAATTTAAAAGGATCTAGGTGAAGAT
CCTTTTTGATAATCTCATGACCAAAATCCCTTAACGTGAGTTTTCGTTCCACTG
AGCGTCAGACCCCGTAGAAAAGATCAAAGGATCTTCTTGAGATCCTTTTTTTC
TGCGCGTAATCTGCTGCTTGCAAACAAAAAAACCACCGCTACCAGCGGTGGT
TTGTTTGCCGGATCAAGAGCTACCAACTCTTTTTCCGAAGGTAACTGGCTTCA
GCAGAGCGCAGATACCAAATACTGTTCTTCTAGTGTAGCCGTAGTTAGGCCAC
CACTTCAAGAACTCTGTAGCACCGCCTACATACCTCGCTCTGCTAATCCTGTT
ACCAGTGGCTGCTGCCAGTGGCGATAAGTCGTGTCTTACCGGGTTGGACTCAA
GACGATAGTTACCGGATAAGGCGCAGCGGTCGGGCTGAACGGGGGGTTCGTG
CACACAGCCCAGCTTGGAGCGAACGACCTACACCGAACTGAGATACCTACAG
CGTGAGCTATGAGAAAGCGCCACGCTTCCCGAAGGGAGAAAGGCGGACAGG
TATCCGGTAAGCGGCAGGGTCGGAACAGGAGAGCGCACGAGGGAGCTTCCA
GGGGGAAACGCCTGGTATCTTTATAGTCCTGTCGGGTTTCGCCACCTCTGACT
TGAGCGTCGATTTTTGTGATGCTCGTCAGGGGGGCGGAGCCTATGGAAAAAC
GCCAGCAACGCGGCCTTTTTACGGTTCCTGGCCTTTTGCTGGCCTTTTGCTCAC
ATGT
**pGW078-pAAV_EFSLTR-AgeI&EcoRI-sPA (SEQ ID NO: 27)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
tgaGAATTCAATAAAAGATCTTTATTTTCATTAGATCTGTGTGTTGGTTTTTTGT
GTGCGGCCGCAGGAACCCCTAGTGATGGAGTTGGCCACTCCCTCTCTGCGCG
CTCGCTCGCTCACTGAGGCCGGGCGACCAAAGGTCGCCCGACGCCCGGGCTT
TGCCCGGGCGGCCTCAGTGAGCGAGCGAGCGCGCAGCTGCCTGCAGGGGCGC
CTGATGCGGTATTTTCTCCTTACGCATCTGTGCGGTATTTCACACCGCATACGT
CAAAGCAACCATAGTACGCGCCCTGTAGCGGCGCATTAAGCGCGGCGGGTGT
GGTGGTTACGCGCAGCGTGACCGCTACACTTGCCAGCGCCTTAGCGCCCGCTC
CTTTCGCTTTCTTCCCTTCCTTTCTCGCCACGTTCGCCGGCTTTCCCCGTCAAG
CTCTAAATCGGGGGCTCCCTTTAGGGTTCCGATTTAGTGCTTTACGGCACCTC
GACCCCAAAAAACTTGATTTGGGTGATGGTTCACGTAGTGGGCCATCGCCCTG
ATAGACGGTTTTTCGCCCTTTGACGTTGGAGTCCACGTTCTTTAATAGTGGAC
TCTTGTTCCAAACTGGAACAACACTCAACTCTATCTCGGGCTATTCTTTTGATT
TATAAGGGATTTTGCCGATTTCGGTCTATTGGTTAAAAAATGAGCTGATTTAA
CAAAAATTTAACGCGAATTTTAACAAAATATTAACGTTTACAATTTTATGGTG
CACTCTCAGTACAATCTGCTCTGATGCCGCATAGTTAAGCCAGCCCCGACACC
 
CGCCAACACCCGCTGACGCGCCCTGACGGGCTTGTCTGCTCCCGGCATCCGCT
TACAGACAAGCTGTGACCGTCTCCGGGAGCTGCATGTGTCAGAGGTTTTCACC
GTCATCACCGAAACGCGCGAGACGAAAGGGCCTCGTGATACGCCTATTTTTA
TAGGTTAATGTCATGATAATAATGGTTTCTTAGACGTCAGGTGGCACTTTTCG
GGGAAATGTGCGCGGAACCCCTATTTGTTTATTTTTCTAAATACATTCAAATA
TGTATCCGCTCATGAGACAATAACCCTGATAAATGCTTCAATAATATTGAAAA
AGGAAGAGTATGAGTATTCAACATTTCCGTGTCGCCCTTATTCCCTTTTTTGCG
GCATTTTGCCTTCCTGTTTTTGCTCACCCAGAAACGCTGGTGAAAGTAAAAGA
TGCTGAAGATCAGTTGGGTGCACGAGTGGGTTACATCGAACTGGATCTCAAC
AGCGGTAAGATCCTTGAGAGTTTTCGCCCCGAAGAACGTTTTCCAATGATGAG
CACTTTTAAAGTTCTGCTATGTGGCGCGGTATTATCCCGTATTGACGCCGGGC
AAGAGCAACTCGGTCGCCGCATACACTATTCTCAGAATGACTTGGTTGAGTAC
TCACCAGTCACAGAAAAGCATCTTACGGATGGCATGACAGTAAGAGAATTAT
GCAGTGCTGCCATAACCATGAGTGATAACACTGCGGCCAACTTACTTCTGACA
ACGATCGGAGGACCGAAGGAGCTAACCGCTTTTTTGCACAACATGGGGGATC
ATGTAACTCGCCTTGATCGTTGGGAACCGGAGCTGAATGAAGCCATACCAAA
CGACGAGCGTGACACCACGATGCCTGTAGCAATGGCAACAACGTTGCGCAAA
CTATTAACTGGCGAACTACTTACTCTAGCTTCCCGGCAACAATTAATAGACTG
GATGGAGGCGGATAAAGTTGCAGGACCACTTCTGCGCTCGGCCCTTCCGGCT
GGCTGGTTTATTGCTGATAAATCTGGAGCCGGTGAGCGTGGAAGCCGCGGTA
TCATTGCAGCACTGGGGCCAGATGGTAAGCCCTCCCGTATCGTAGTTATCTAC
ACGACGGGGAGTCAGGCAACTATGGATGAACGAAATAGACAGATCGCTGAG
ATAGGTGCCTCACTGATTAAGCATTGGTAACTGTCAGACCAAGTTTACTCATA
TATACTTTAGATTGATTTAAAACTTCATTTTTAATTTAAAAGGATCTAGGTGA
AGATCCTTTTTGATAATCTCATGACCAAAATCCCTTAACGTGAGTTTTCGTTCC
ACTGAGCGTCAGACCCCGTAGAAAAGATCAAAGGATCTTCTTGAGATCCTTTT
TTTCTGCGCGTAATCTGCTGCTTGCAAACAAAAAAACCACCGCTACCAGCGGT
GGTTTGTTTGCCGGATCAAGAGCTACCAACTCTTTTTCCGAAGGTAACTGGCT
TCAGCAGAGCGCAGATACCAAATACTGTTCTTCTAGTGTAGCCGTAGTTAGGC
CACCACTTCAAGAACTCTGTAGCACCGCCTACATACCTCGCTCTGCTAATCCT
GTTACCAGTGGCTGCTGCCAGTGGCGATAAGTCGTGTCTTACCGGGTTGGACT
CAAGACGATAGTTACCGGATAAGGCGCAGCGGTCGGGCTGAACGGGGGGTTC
GTGCACACAGCCCAGCTTGGAGCGAACGACCTACACCGAACTGAGATACCTA
CAGCGTGAGCTATGAGAAAGCGCCACGCTTCCCGAAGGGAGAAAGGCGGAC
AGGTATCCGGTAAGCGGCAGGGTCGGAACAGGAGAGCGCACGAGGGAGCTT
CCAGGGGGAAACGCCTGGTATCTTTATAGTCCTGTCGGGTTTCGCCACCTCTG
ACTTGAGCGTCGATTTTTGTGATGCTCGTCAGGGGGGCGGAGCCTATGGAAA
AACGCCAGCAACGCGGCCTTTTTACGGTTCCTGGCCTTTTGCTGGCCTTTTGCT
CACATGT
**pGW090-pAAV_EFSLTR-Cd80-T2A-light-P2A-IL2-E2A-41bb-F2A-Ifng (SEQ ID NO: 28)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
 
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atggcttgcaattgtcagttgatgcaggatacaccactcctcaagtttccatgtccaaggctcattcttctctttgtgctgctgattcgtc
tttcacaagtgtcttcagatgttgatgaacaactgtccaagtcagtgaaagataaggtattgctgccttgccgttacaactctcctcat
gaagatgagtctgaagaccgaatctactggcaaaaacatgacaaagtggtgctgtctgtcattgTtgggaaactaaaagtgtggc
ccgagtataagaaccggactttatatgacaacactacctactctcttatcatcctgggcctggtcctttcagaccggggcacataca
gctgtgtcgttcaaaagaaggaaagaggaacgtatgaagttaaacacttggctttagtaaagttgtccatcaaagctgacttctcta
cccccaacataactgagtctggaaacccatctgcagacactaaaaggattacctgctttgcttccgggggtttcccaaagcctcgc
ttctcttggttggaaaatggaagagaattacctggcatcaatacgacaatttcccaggatcctgaatctgaattgtacaccattagta
gccaactagatttcaatacgactcgcaaccacaccattaagtgtctcattaaatatggagatgctcacgtgtcagaggacttcacct
gggaaaaacccccagaagaccctcctgatagcaagaacacacttgtgctctttggggcaggattcggcgcagtaataacagtcg
tcgtcatcgttgtcatcatcaaatgcttctgtaagcacagaagctgtttcagaagaaatgaggcaagcagagaaacaaacaacag
ccttaccttcgggcctgaagaagcattagctgaacagaccgtcttccttcgtacgGGCAGTGGAGAGGGCAGA
GGAAGTCTGCTAACATGCGGTGACGTCGAGGAGAATCCTGGCCCAatggagagtgt
ggtacagccttcagtgtttgtggtggatggacagacggacatcccattcaggcggctggaacagaaccaccggagacggcgct
gtggcactgtccaggtcagcctggccctggtgctgctgctaggtgctgggctggccactcagggctggtttctcctgagactgca
tcaacgtcttggagacatagtagctcatctgccagatggaggcaaaggctcctgggagaagctgatacaagatcaacgatctca
ccaggccaacccagcagcacatcttacaggagccaacgccagcttgataggtattggtggacctctgttatgggagacacgact
tggcctggccttcttgaggggcttgacgtatcatgatggggccctggtgaccatggagcccggttactactatgtgtactccaaag
tgcagctgagcggcgtgggctgcccccaggggctggccaatggcctccccatcacccatggactatacaagcgcacatcccg
ctacccgaaggagttagaactgctggtcagtcggcggtcaccctgtggccgggccaacagctcccgagtctggtgggacagca
gcttcctgggcggcgtggtacatctggaggctggggaagaggtggtggtccgcgtgcctggaaaccgcctggtcagaccacgt
gacggcaccaggtcctatttcggagctttcatggtcactagtGGATCCGGCGCAACAAACTTCTCTCTG
CTGAAACAAGCCGGAGATGTCGAAGAGAATCCTGGACCGatgtacagcatgcagctcgca
tcctgtgtcacattgacacttgtgctccttgtcaacagcgcacccacttcaagctccacttcaagctctacagcggaagcacagca
gcagcagcagcagcagcagcagcagcagcagcacctggagcagctgttgatggacctacaggagctcctgagcaggatgga
gaattacaggaacctgaaactccccaggatgctcaccttcaaattttacttgcccaagcaggccacagaattgaaagatcttcagt
gcctagaagatgaacttggacctctgcggcatgttctggatttgactcaaagcaaaagctttcaattggaagatgctgagaatttcat
cagcaatatcagagtaactgttgtaaaactaaagggctctgacaacacatttgagtgccaattcgatgatgagtcagcaactgtggt
ggactttctgaggagatggatagccttctgtcaaagcatcatctcaacaagccctcaagctagcagcggtacccagtgcaccaac
tacgccctgctgaagctggccggcgatgtggagagcaaccccgggcccatggaccagcacacacttgatgtggaggataccg
cggatgccagacatccagcaggtacttcgtgcccctcggatgcggcgctcctcagagataccgggctcctcgcggacgctgcg
ctcctctcagatactgtgcgccccacaaatgccgcgctccccacggatgctgcctaccctgcggttaatgttcgggatcgcgagg
ccgcgtggccgcctgcactgaacttctgttcccgccacccaaagctctatggcctagtcgctttggttttgctgcttctgatcgccgc
ctgtgttcctatcttcacccgcaccgagcctcggccagcgctcacaatcaccacctcgcccaacctgggtacccgagagaataat
gcagaccaggtcacccctgtttcccacattggctgccccaacactacacaacagggctctcctgtgttcgccaagctactggcta
aaaaccaagcatcgttgtgcaatacaactctgaactggcacagccaagatggagctgggagctcatacctatctcaaggtctgag
gtacgaagaagacaaaaaggagttggtggtagacagtcccgggctctactacgtatttttggaactgaagctcagtccaacattca
caaacacaggccacaaggtgcagggctgggtctctcttgttttgcaagcaaagcctcaggtagatgactttgacaacttggccct
gacagtggaactgttcccttgctccatggagaacaagttagtggaccgttcctggagtcaactgttgctcctgaaggctggccacc
gcctcagtgtgggtctgagggcttatctgcatggagcccaggatgcatacagagactgggagctgtcttatcccaacaccacca
gctttggactctttcttgtgaaacccgacaacccatgggaaggcatatgcggtaccgtgaagcagaccctgaacttcgatctgctg
aagctggccggcgatgtggagagcaaccccgggcccatgaacgctacacactgcatcttggctttgcagctcttcctcatggctg
tttctggctgttactgccacggcacagtcattgaaagcctagaaagtctgaataactattttaactcaagtggcatagatgtggaaga
aaagagtctcttcttggatatctggaggaactggcaaaaggatggtgacatgaaaatcctgcagagccagattatctctttctacct
cagactctttgaagtcttgaaagacaatcaggccatcagcaacaacataagcgtcattgaatcacacctgattactaccttcttcag
caacagcaaggcgaaaaaggatgcattcatgagtattgccaagtttgaggtcaacaacccacaggtccagcgccaagcattcaa
 
tgagctcatccgagtggtccaccagctgttgccggaatccagcctcaggaagcggaaaaggagtcgctgctgaGAATTC
AATAAAAGATCTTTATTTTCATTAGATCTGTGTGTTGGTTTTTTGTGTGCGGCC
GCAGGAACCCCTAGTGATGGAGTTGGCCACTCCCTCTCTGCGCGCTCGCTCGC
TCACTGAGGCCGGGCGACCAAAGGTCGCCCGACGCCCGGGCTTTGCCCGGGC
GGCCTCAGTGAGCGAGCGAGCGCGCAGCTGCCTGCAGGGGCGCCTGATGCGG
TATTTTCTCCTTACGCATCTGTGCGGTATTTCACACCGCATACGTCAAAGCAA
CCATAGTACGCGCCCTGTAGCGGCGCATTAAGCGCGGCGGGTGTGGTGGTTA
CGCGCAGCGTGACCGCTACACTTGCCAGCGCCTTAGCGCCCGCTCCTTTCGCT
TTCTTCCCTTCCTTTCTCGCCACGTTCGCCGGCTTTCCCCGTCAAGCTCTAAAT
CGGGGGCTCCCTTTAGGGTTCCGATTTAGTGCTTTACGGCACCTCGACCCCAA
AAAACTTGATTTGGGTGATGGTTCACGTAGTGGGCCATCGCCCTGATAGACG
GTTTTTCGCCCTTTGACGTTGGAGTCCACGTTCTTTAATAGTGGACTCTTGTTC
CAAACTGGAACAACACTCAACTCTATCTCGGGCTATTCTTTTGATTTATAAGG
GATTTTGCCGATTTCGGTCTATTGGTTAAAAAATGAGCTGATTTAACAAAAAT
TTAACGCGAATTTTAACAAAATATTAACGTTTACAATTTTATGGTGCACTCTC
AGTACAATCTGCTCTGATGCCGCATAGTTAAGCCAGCCCCGACACCCGCCAA
CACCCGCTGACGCGCCCTGACGGGCTTGTCTGCTCCCGGCATCCGCTTACAGA
CAAGCTGTGACCGTCTCCGGGAGCTGCATGTGTCAGAGGTTTTCACCGTCATC
ACCGAAACGCGCGAGACGAAAGGGCCTCGTGATACGCCTATTTTTATAGGTT
AATGTCATGATAATAATGGTTTCTTAGACGTCAGGTGGCACTTTTCGGGGAAA
TGTGCGCGGAACCCCTATTTGTTTATTTTTCTAAATACATTCAAATATGTATCC
GCTCATGAGACAATAACCCTGATAAATGCTTCAATAATATTGAAAAAGGAAG
AGTATGAGTATTCAACATTTCCGTGTCGCCCTTATTCCCTTTTTTGCGGCATTT
TGCCTTCCTGTTTTTGCTCACCCAGAAACGCTGGTGAAAGTAAAAGATGCTGA
AGATCAGTTGGGTGCACGAGTGGGTTACATCGAACTGGATCTCAACAGCGGT
AAGATCCTTGAGAGTTTTCGCCCCGAAGAACGTTTTCCAATGATGAGCACTTT
TAAAGTTCTGCTATGTGGCGCGGTATTATCCCGTATTGACGCCGGGCAAGAGC
AACTCGGTCGCCGCATACACTATTCTCAGAATGACTTGGTTGAGTACTCACCA
GTCACAGAAAAGCATCTTACGGATGGCATGACAGTAAGAGAATTATGCAGTG
CTGCCATAACCATGAGTGATAACACTGCGGCCAACTTACTTCTGACAACGATC
GGAGGACCGAAGGAGCTAACCGCTTTTTTGCACAACATGGGGGATCATGTAA
CTCGCCTTGATCGTTGGGAACCGGAGCTGAATGAAGCCATACCAAACGACGA
GCGTGACACCACGATGCCTGTAGCAATGGCAACAACGTTGCGCAAACTATTA
ACTGGCGAACTACTTACTCTAGCTTCCCGGCAACAATTAATAGACTGGATGGA
GGCGGATAAAGTTGCAGGACCACTTCTGCGCTCGGCCCTTCCGGCTGGCTGGT
TTATTGCTGATAAATCTGGAGCCGGTGAGCGTGGAAGCCGCGGTATCATTGC
AGCACTGGGGCCAGATGGTAAGCCCTCCCGTATCGTAGTTATCTACACGACG
GGGAGTCAGGCAACTATGGATGAACGAAATAGACAGATCGCTGAGATAGGT
GCCTCACTGATTAAGCATTGGTAACTGTCAGACCAAGTTTACTCATATATACT
TTAGATTGATTTAAAACTTCATTTTTAATTTAAAAGGATCTAGGTGAAGATCC
TTTTTGATAATCTCATGACCAAAATCCCTTAACGTGAGTTTTCGTTCCACTGAG
CGTCAGACCCCGTAGAAAAGATCAAAGGATCTTCTTGAGATCCTTTTTTTCTG
CGCGTAATCTGCTGCTTGCAAACAAAAAAACCACCGCTACCAGCGGTGGTTT
GTTTGCCGGATCAAGAGCTACCAACTCTTTTTCCGAAGGTAACTGGCTTCAGC
AGAGCGCAGATACCAAATACTGTTCTTCTAGTGTAGCCGTAGTTAGGCCACCA
CTTCAAGAACTCTGTAGCACCGCCTACATACCTCGCTCTGCTAATCCTGTTAC
CAGTGGCTGCTGCCAGTGGCGATAAGTCGTGTCTTACCGGGTTGGACTCAAG
ACGATAGTTACCGGATAAGGCGCAGCGGTCGGGCTGAACGGGGGGTTCGTGC
ACACAGCCCAGCTTGGAGCGAACGACCTACACCGAACTGAGATACCTACAGC
GTGAGCTATGAGAAAGCGCCACGCTTCCCGAAGGGAGAAAGGCGGACAGGT
 
ATCCGGTAAGCGGCAGGGTCGGAACAGGAGAGCGCACGAGGGAGCTTCCAG
GGGGAAACGCCTGGTATCTTTATAGTCCTGTCGGGTTTCGCCACCTCTGACTT
GAGCGTCGATTTTTGTGATGCTCGTCAGGGGGGCGGAGCCTATGGAAAAACG
CCAGCAACGCGGCCTTTTTACGGTTCCTGGCCTTTTGCTGGCCTTTTGCTCACA
TGT
**pGW091-pAAV_EFSLTR-Cd80-T2A-light-P2A-GITRL-E2A-41bb-F2A-Ifng (SEQ ID NO: 29)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atggcttgcaattgtcagttgatgcaggatacaccactcctcaagtttccatgtccaaggctcattcttctctttgtgctgctgattcgtc
tttcacaagtgtcttcagatgttgatgaacaactgtccaagtcagtgaaagataaggtattgctgccttgccgttacaactctcctcat
gaagatgagtctgaagaccgaatctactggcaaaaacatgacaaagtggtgctgtctgtcattgTtgggaaactaaaagtgtggc
ccgagtataagaaccggactttatatgacaacactacctactctcttatcatcctgggcctggtcctttcagaccggggcacataca
gctgtgtcgttcaaaagaaggaaagaggaacgtatgaagttaaacacttggctttagtaaagttgtccatcaaagctgacttctcta
cccccaacataactgagtctggaaacccatctgcagacactaaaaggattacctgctttgcttccgggggtttcccaaagcctcgc
ttctcttggttggaaaatggaagagaattacctggcatcaatacgacaatttcccaggatcctgaatctgaattgtacaccattagta
gccaactagatttcaatacgactcgcaaccacaccattaagtgtctcattaaatatggagatgctcacgtgtcagaggacttcacct
gggaaaaacccccagaagaccctcctgatagcaagaacacacttgtgctctttggggcaggattcggcgcagtaataacagtcg
tcgtcatcgttgtcatcatcaaatgcttctgtaagcacagaagctgtttcagaagaaatgaggcaagcagagaaacaaacaacag
ccttaccttcgggcctgaagaagcattagctgaacagaccgtcttccttcgtacgGGCAGTGGAGAGGGCAGA
GGAAGTCTGCTAACATGCGGTGACGTCGAGGAGAATCCTGGCCCAatggagagtgt
ggtacagccttcagtgtttgtggtggatggacagacggacatcccattcaggcggctggaacagaaccaccggagacggcgct
gtggcactgtccaggtcagcctggccctggtgctgctgctaggtgctgggctggccactcagggctggtttctcctgagactgca
tcaacgtcttggagacatagtagctcatctgccagatggaggcaaaggctcctgggagaagctgatacaagatcaacgatctca
ccaggccaacccagcagcacatcttacaggagccaacgccagcttgataggtattggtggacctctgttatgggagacacgact
tggcctggccttcttgaggggcttgacgtatcatgatggggccctggtgaccatggagcccggttactactatgtgtactccaaag
tgcagctgagcggcgtgggctgcccccaggggctggccaatggcctccccatcacccatggactatacaagcgcacatcccg
ctacccgaaggagttagaactgctggtcagtcggcggtcaccctgtggccgggccaacagctcccgagtctggtgggacagca
gcttcctgggcggcgtggtacatctggaggctggggaagaggtggtggtccgcgtgcctggaaaccgcctggtcagaccacgt
gacggcaccaggtcctatttcggagctttcatggtcactagtGGATCCGGCGCAACAAACTTCTCTCTG
CTGAAACAAGCCGGAGATGTCGAAGAGAATCCTGGACCGatggaggaaatgcctttgaga
gagtcaagtcctcaaagggcagagaggtgcaagaagtcatggctcttgtgcatagtggctctgttactgatgctgctctgttctttg
ggtacactgatctatacttcactcaagccaactgccatcgagtcctgcatggttaagtttgaactatcatcctcaaaatggcacatga
catctcccaaacctcactgtgtgaatacgacatctgatgggaagctgaagatactgcagagtggcacatatttaatctacggccaa
gtgattcctgtggataagaaatacataaaagacaatgcccccttcgtagtacagatatataaaaagaatgatgtcctacaaactcta
atgaatgattttcaaatcttgcctataggaggggtttatgaactgcatgctggagataacatatatctgaagttcaactctaaagacca
tattcagaaaaataacacatactgggggatcatcttaatgcctgatctaccattcatctctgctagcagcggtacccagtgcaccaa
ctacgccctgctgaagctggccggcgatgtggagagcaaccccgggcccatggaccagcacacacttgatgtggaggatacc
 
gcggatgccagacatccagcaggtacttcgtgcccctcggatgcggcgctcctcagagataccgggctcctcgcggacgctgc
gctcctctcagatactgtgcgccccacaaatgccgcgctccccacggatgctgcctaccctgcggttaatgttcgggatcgcgag
gccgcgtggccgcctgcactgaacttctgttcccgccacccaaagctctatggcctagtcgctttggttttgctgcttctgatcgcc
gcctgtgttcctatcttcacccgcaccgagcctcggccagcgctcacaatcaccacctcgcccaacctgggtacccgagagaat
aatgcagaccaggtcacccctgtttcccacattggctgccccaacactacacaacagggctctcctgtgttcgccaagctactgg
ctaaaaaccaagcatcgttgtgcaatacaactctgaactggcacagccaagatggagctgggagctcatacctatctcaaggtct
gaggtacgaagaagacaaaaaggagttggtggtagacagtcccgggctctactacgtatttttggaactgaagctcagtccaaca
ttcacaaacacaggccacaaggtgcagggctgggtctctcttgttttgcaagcaaagcctcaggtagatgactttgacaacttggc
cctgacagtggaactgttcccttgctccatggagaacaagttagtggaccgttcctggagtcaactgttgctcctgaaggctggcc
accgcctcagtgtgggtctgagggcttatctgcatggagcccaggatgcatacagagactgggagctgtcttatcccaacaccac
cagctttggactctttcttgtgaaacccgacaacccatgggaaggcatatgcggtaccgtgaagcagaccctgaacttcgatctgc
tgaagctggccggcgatgtggagagcaaccccgggcccatgaacgctacacactgcatcttggctttgcagctcttcctcatggc
tgtttctggctgttactgccacggcacagtcattgaaagcctagaaagtctgaataactattttaactcaagtggcatagatgtggaa
gaaaagagtctcttcttggatatctggaggaactggcaaaaggatggtgacatgaaaatcctgcagagccagattatctctttctac
ctcagactctttgaagtcttgaaagacaatcaggccatcagcaacaacataagcgtcattgaatcacacctgattactaccttcttca
gcaacagcaaggcgaaaaaggatgcattcatgagtattgccaagtttgaggtcaacaacccacaggtccagcgccaagcattca
atgagctcatccgagtggtccaccagctgttgccggaatccagcctcaggaagcggaaaaggagtcgctgctgaGAATTC
AATAAAAGATCTTTATTTTCATTAGATCTGTGTGTTGGTTTTTTGTGTGCGGCC
GCAGGAACCCCTAGTGATGGAGTTGGCCACTCCCTCTCTGCGCGCTCGCTCGC
TCACTGAGGCCGGGCGACCAAAGGTCGCCCGACGCCCGGGCTTTGCCCGGGC
GGCCTCAGTGAGCGAGCGAGCGCGCAGCTGCCTGCAGGGGCGCCTGATGCGG
TATTTTCTCCTTACGCATCTGTGCGGTATTTCACACCGCATACGTCAAAGCAA
CCATAGTACGCGCCCTGTAGCGGCGCATTAAGCGCGGCGGGTGTGGTGGTTA
CGCGCAGCGTGACCGCTACACTTGCCAGCGCCTTAGCGCCCGCTCCTTTCGCT
TTCTTCCCTTCCTTTCTCGCCACGTTCGCCGGCTTTCCCCGTCAAGCTCTAAAT
CGGGGGCTCCCTTTAGGGTTCCGATTTAGTGCTTTACGGCACCTCGACCCCAA
AAAACTTGATTTGGGTGATGGTTCACGTAGTGGGCCATCGCCCTGATAGACG
GTTTTTCGCCCTTTGACGTTGGAGTCCACGTTCTTTAATAGTGGACTCTTGTTC
CAAACTGGAACAACACTCAACTCTATCTCGGGCTATTCTTTTGATTTATAAGG
GATTTTGCCGATTTCGGTCTATTGGTTAAAAAATGAGCTGATTTAACAAAAAT
TTAACGCGAATTTTAACAAAATATTAACGTTTACAATTTTATGGTGCACTCTC
AGTACAATCTGCTCTGATGCCGCATAGTTAAGCCAGCCCCGACACCCGCCAA
CACCCGCTGACGCGCCCTGACGGGCTTGTCTGCTCCCGGCATCCGCTTACAGA
CAAGCTGTGACCGTCTCCGGGAGCTGCATGTGTCAGAGGTTTTCACCGTCATC
ACCGAAACGCGCGAGACGAAAGGGCCTCGTGATACGCCTATTTTTATAGGTT
AATGTCATGATAATAATGGTTTCTTAGACGTCAGGTGGCACTTTTCGGGGAAA
TGTGCGCGGAACCCCTATTTGTTTATTTTTCTAAATACATTCAAATATGTATCC
GCTCATGAGACAATAACCCTGATAAATGCTTCAATAATATTGAAAAAGGAAG
AGTATGAGTATTCAACATTTCCGTGTCGCCCTTATTCCCTTTTTTGCGGCATTT
TGCCTTCCTGTTTTTGCTCACCCAGAAACGCTGGTGAAAGTAAAAGATGCTGA
AGATCAGTTGGGTGCACGAGTGGGTTACATCGAACTGGATCTCAACAGCGGT
AAGATCCTTGAGAGTTTTCGCCCCGAAGAACGTTTTCCAATGATGAGCACTTT
TAAAGTTCTGCTATGTGGCGCGGTATTATCCCGTATTGACGCCGGGCAAGAGC
AACTCGGTCGCCGCATACACTATTCTCAGAATGACTTGGTTGAGTACTCACCA
GTCACAGAAAAGCATCTTACGGATGGCATGACAGTAAGAGAATTATGCAGTG
CTGCCATAACCATGAGTGATAACACTGCGGCCAACTTACTTCTGACAACGATC
GGAGGACCGAAGGAGCTAACCGCTTTTTTGCACAACATGGGGGATCATGTAA
CTCGCCTTGATCGTTGGGAACCGGAGCTGAATGAAGCCATACCAAACGACGA
GCGTGACACCACGATGCCTGTAGCAATGGCAACAACGTTGCGCAAACTATTA
ACTGGCGAACTACTTACTCTAGCTTCCCGGCAACAATTAATAGACTGGATGGA
 
GGCGGATAAAGTTGCAGGACCACTTCTGCGCTCGGCCCTTCCGGCTGGCTGGT
TTATTGCTGATAAATCTGGAGCCGGTGAGCGTGGAAGCCGCGGTATCATTGC
AGCACTGGGGCCAGATGGTAAGCCCTCCCGTATCGTAGTTATCTACACGACG
GGGAGTCAGGCAACTATGGATGAACGAAATAGACAGATCGCTGAGATAGGT
GCCTCACTGATTAAGCATTGGTAACTGTCAGACCAAGTTTACTCATATATACT
TTAGATTGATTTAAAACTTCATTTTTAATTTAAAAGGATCTAGGTGAAGATCC
TTTTTGATAATCTCATGACCAAAATCCCTTAACGTGAGTTTTCGTTCCACTGAG
CGTCAGACCCCGTAGAAAAGATCAAAGGATCTTCTTGAGATCCTTTTTTTCTG
CGCGTAATCTGCTGCTTGCAAACAAAAAAACCACCGCTACCAGCGGTGGTTT
GTTTGCCGGATCAAGAGCTACCAACTCTTTTTCCGAAGGTAACTGGCTTCAGC
AGAGCGCAGATACCAAATACTGTTCTTCTAGTGTAGCCGTAGTTAGGCCACCA
CTTCAAGAACTCTGTAGCACCGCCTACATACCTCGCTCTGCTAATCCTGTTAC
CAGTGGCTGCTGCCAGTGGCGATAAGTCGTGTCTTACCGGGTTGGACTCAAG
ACGATAGTTACCGGATAAGGCGCAGCGGTCGGGCTGAACGGGGGGTTCGTGC
ACACAGCCCAGCTTGGAGCGAACGACCTACACCGAACTGAGATACCTACAGC
GTGAGCTATGAGAAAGCGCCACGCTTCCCGAAGGGAGAAAGGCGGACAGGT
ATCCGGTAAGCGGCAGGGTCGGAACAGGAGAGCGCACGAGGGAGCTTCCAG
GGGGAAACGCCTGGTATCTTTATAGTCCTGTCGGGTTTCGCCACCTCTGACTT
GAGCGTCGATTTTTGTGATGCTCGTCAGGGGGGCGGAGCCTATGGAAAAACG
CCAGCAACGCGGCCTTTTTACGGTTCCTGGCCTTTTGCTGGCCTTTTGCTCACA
TGT
**pGW096-pAAV_EFSLTR-Ifng-P2A-Cd80-T2A-light-P2A-IL23-E2A-41BBL-sPA (SEQ ID NO: 30)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atgaacgctacacactgcatcttggctttgcagctcttcctcatggctgtttctggctgttactgccacggcacagtcattgaaagcct
agaaagtctgaataactattttaactcaagtggcatagatgtggaagaaaagagtctcttcttggatatctggaggaactggcaaaa
ggatggtgacatgaaaatcctgcagagccagattatctctttctacctcagactctttgaagtcttgaaagacaatcaggccatcag
caacaacataagcgtcattgaatcacacctgattactaccttcttcagcaacagcaaggcgaaaaaggatgcattcatgagtattg
ccaagtttgaggtcaacaacccacaggtccagcgccaagcattcaatgagctcatccgagtggtccaccagctgttgccggaat
ccagcctcaggaagcggaaaaggagtcgctgcGGATCCGGCGCAACAAACTTCTCTCTGCTGAA
ACAAGCCGGAGATGTCGAAGAGAATCCTGGACCGatggcttgcaattgtcagttgatgcaggat
acaccactcctcaagtttccatgtccaaggctcattcttctctttgtgctgctgattcgtctttcacaagtgtcttcagatgttgatgaac
aactgtccaagtcagtgaaagataaggtattgctgccttgccgttacaactctcctcatgaagatgagtctgaagaccgaatctact
ggcaaaaacatgacaaagtggtgctgtctgtcattgTtgggaaactaaaagtgtggcccgagtataagaaccggactttatatga
caacactacctactctcttatcatcctgggcctggtcctttcagaccggggcacatacagctgtgtcgttcaaaagaaggaaagag
gaacgtatgaagttaaacacttggctttagtaaagttgtccatcaaagctgacttctctacccccaacataactgagtctggaaaccc
atctgcagacactaaaaggattacctgctttgcttccgggggtttcccaaagcctcgcttctcttggttggaaaatggaagagaatta
 
cctggcatcaatacgacaatttcccaggatcctgaatctgaattgtacaccattagtagccaactagatttcaatacgactcgcaac
cacaccattaagtgtctcattaaatatggagatgctcacgtgtcagaggacttcacctgggaaaaacccccagaagaccctcctg
atagcaagaacacacttgtgctctttggggcaggattcggcgcagtaataacagtcgtcgtcatcgttgtcatcatcaaatgcttctg
taagcacagaagctgtttcagaagaaatgaggcaagcagagaaacaaacaacagccttaccttcgggcctgaagaagcattag
ctgaacagaccgtcttccttcgtacgGGCAGTGGAGAGGGCAGAGGAAGTCTGCTAACATGC
GGTGACGTCGAGGAGAATCCTGGCCCAatggagagtgtggtacagccttcagtgtttgtggtggatgga
cagacggacatcccattcaggcggctggaacagaaccaccggagacggcgctgtggcactgtccaggtcagcctggccctgg
tgctgctgctaggtgctgggctggccactcagggctggtttctcctgagactgcatcaacgtcttggagacatagtagctcatctgc
cagatggaggcaaaggctcctgggagaagctgatacaagatcaacgatctcaccaggccaacccagcagcacatcttacagg
agccaacgccagcttgataggtattggtggacctctgttatgggagacacgacttggcctggccttcttgaggggcttgacgtatc
atgatggggccctggtgaccatggagcccggttactactatgtgtactccaaagtgcagctgagcggcgtgggctgcccccagg
ggctggccaatggcctccccatcacccatggactatacaagcgcacatcccgctacccgaaggagttagaactgctggtcagtc
ggcggtcaccctgtggccgggccaacagctcccgagtctggtgggacagcagcttcctgggcggcgtggtacatctggaggc
tggggaagaggtggtggtccgcgtgcctggaaaccgcctggtcagaccacgtgacggcaccaggtcctatttcggagctttcat
ggtcactagtGGATCCGGCGCAACAAACTTCTCTCTGCTGAAACAAGCCGGAGATG
TCGAAGAGAATCCTGGACCGATGTGTCCTCAGAAGCTAACCATCTCCTGGTTT
GCCATCGTTTTGCTGGTGTCTCCACTCATGGCCATGTGGGAGCTGGAGAAAGA
CGTTTATGTTGTAGAGGTGGACTGGACTCCCGATGCCCCTGGAGAAACAGTG
AACCTCACCTGTGACACGCCTGAAGAAGATGACATCACCTGGACCTCAGACC
AGAGACATGGAGTCATAGGCTCTGGAAAGACCCTGACCATCACTGTCAAAGA
GTTTCTAGATGCTGGCCAGTACACCTGCCACAAAGGAGGCGAGACTCTGAGC
CACTCACATCTGCTGCTCCACAAGAAGGAAAATGGAATTTGGTCCACTGAAA
TTTTAAAAAATTTCAAAAACAAGACTTTCCTGAAGTGTGAAGCACCAAATTAC
TCCGGACGGTTCACGTGCTCATGGCTGGTGCAAAGAAACATGGACTTGAAGT
TCAACATCAAGAGCAGTAGCAGTTCCCCTGACTCTCGGGCAGTGACATGTGG
AATGGCGTCTCTGTCTGCAGAGAAGGTCACACTGGACCAAAGGGACTATGAG
AAGTATTCAGTGTCCTGCCAGGAGGATGTCACCTGCCCAACTGCCGAGGAGA
CCCTGCCCATTGAACTGGCGTTGGAAGCACGGCAGCAGAATAAATATGAGAA
CTACAGCACCAGCTTCTTCATCAGGGACATCATCAAACCAGACCCGCCCAAG
AACTTGCAGATGAAGCCTTTGAAGAACTCACAGGTGGAGGTCAGCTGGGAGT
ACCCTGACTCCTGGAGCACTCCCCATTCCTACTTCTCCCTCAAGTTCTTTGTTC
GAATCCAGCGCAAGAAAGAAAAGATGAAGGAGACAGAGGAGGGGTGTAACC
AGAAAGGTGCGTTCCTCGTAGAGAAGACATCTACCGAAGTCCAATGCAAAGG
CGGGAATGTCTGCGTGCAAGCTCAGGATCGCTATTACAATTCCTCATGCAGCA
AGTGGGCATGTGTTCCCTGCAGGGTCCGATCCgctagcagcggtacccagtgcaccaactacgc
cctgctgaagctggccggcgatgtggagagcaaccccgggcccatggaccagcacacacttgatgtggaggataccgcggat
gccagacatccagcaggtacttcgtgcccctcggatgcggcgctcctcagagataccgggctcctcgcggacgctgcgctcct
ctcagatactgtgcgccccacaaatgccgcgctccccacggatgctgcctaccctgcggttaatgttcgggatcgcgaggccgc
gtggccgcctgcactgaacttctgttcccgccacccaaagctctatggcctagtcgctttggttttgctgcttctgatcgccgcctgt
gttcctatcttcacccgcaccgagcctcggccagcgctcacaatcaccacctcgcccaacctgggtacccgagagaataatgca
gaccaggtcacccctgtttcccacattggctgccccaacactacacaacagggctctcctgtgttcgccaagctactggctaaaaa
ccaagcatcgttgtgcaatacaactctgaactggcacagccaagatggagctgggagctcatacctatctcaaggtctgaggtac
gaagaagacaaaaaggagttggtggtagacagtcccgggctctactacgtatttttggaactgaagctcagtccaacattcacaa
acacaggccacaaggtgcagggctgggtctctcttgttttgcaagcaaagcctcaggtagatgactttgacaacttggccctgac
agtggaactgttcccttgctccatggagaacaagttagtggaccgttcctggagtcaactgttgctcctgaaggctggccaccgcc
tcagtgtgggtctgagggcttatctgcatggagcccaggatgcatacagagactgggagctgtcttatcccaacaccaccagcttt
ggactctttcttgtgaaacccgacaacccatgggaatgaGAATTCAATAAAAGATCTTTATTTTCATT
AGATCTGTGTGTTGGTTTTTTGTGTGCGGCCGCAGGAACCCCTAGTGATGGAG
TTGGCCACTCCCTCTCTGCGCGCTCGCTCGCTCACTGAGGCCGGGCGACCAAA
GGTCGCCCGACGCCCGGGCTTTGCCCGGGCGGCCTCAGTGAGCGAGCGAGCG
 
CGCAGCTGCCTGCAGGGGCGCCTGATGCGGTATTTTCTCCTTACGCATCTGTG
CGGTATTTCACACCGCATACGTCAAAGCAACCATAGTACGCGCCCTGTAGCG
GCGCATTAAGCGCGGCGGGTGTGGTGGTTACGCGCAGCGTGACCGCTACACT
TGCCAGCGCCTTAGCGCCCGCTCCTTTCGCTTTCTTCCCTTCCTTTCTCGCCAC
GTTCGCCGGCTTTCCCCGTCAAGCTCTAAATCGGGGGCTCCCTTTAGGGTTCC
GATTTAGTGCTTTACGGCACCTCGACCCCAAAAAACTTGATTTGGGTGATGGT
TCACGTAGTGGGCCATCGCCCTGATAGACGGTTTTTCGCCCTTTGACGTTGGA
GTCCACGTTCTTTAATAGTGGACTCTTGTTCCAAACTGGAACAACACTCAACT
CTATCTCGGGCTATTCTTTTGATTTATAAGGGATTTTGCCGATTTCGGTCTATT
GGTTAAAAAATGAGCTGATTTAACAAAAATTTAACGCGAATTTTAACAAAAT
ATTAACGTTTACAATTTTATGGTGCACTCTCAGTACAATCTGCTCTGATGCCG
CATAGTTAAGCCAGCCCCGACACCCGCCAACACCCGCTGACGCGCCCTGACG
GGCTTGTCTGCTCCCGGCATCCGCTTACAGACAAGCTGTGACCGTCTCCGGGA
GCTGCATGTGTCAGAGGTTTTCACCGTCATCACCGAAACGCGCGAGACGAAA
GGGCCTCGTGATACGCCTATTTTTATAGGTTAATGTCATGATAATAATGGTTT
CTTAGACGTCAGGTGGCACTTTTCGGGGAAATGTGCGCGGAACCCCTATTTGT
TTATTTTTCTAAATACATTCAAATATGTATCCGCTCATGAGACAATAACCCTG
ATAAATGCTTCAATAATATTGAAAAAGGAAGAGTATGAGTATTCAACATTTC
CGTGTCGCCCTTATTCCCTTTTTTGCGGCATTTTGCCTTCCTGTTTTTGCTCACC
CAGAAACGCTGGTGAAAGTAAAAGATGCTGAAGATCAGTTGGGTGCACGAGT
GGGTTACATCGAACTGGATCTCAACAGCGGTAAGATCCTTGAGAGTTTTCGCC
CCGAAGAACGTTTTCCAATGATGAGCACTTTTAAAGTTCTGCTATGTGGCGCG
GTATTATCCCGTATTGACGCCGGGCAAGAGCAACTCGGTCGCCGCATACACT
ATTCTCAGAATGACTTGGTTGAGTACTCACCAGTCACAGAAAAGCATCTTACG
GATGGCATGACAGTAAGAGAATTATGCAGTGCTGCCATAACCATGAGTGATA
ACACTGCGGCCAACTTACTTCTGACAACGATCGGAGGACCGAAGGAGCTAAC
CGCTTTTTTGCACAACATGGGGGATCATGTAACTCGCCTTGATCGTTGGGAAC
CGGAGCTGAATGAAGCCATACCAAACGACGAGCGTGACACCACGATGCCTGT
AGCAATGGCAACAACGTTGCGCAAACTATTAACTGGCGAACTACTTACTCTA
GCTTCCCGGCAACAATTAATAGACTGGATGGAGGCGGATAAAGTTGCAGGAC
CACTTCTGCGCTCGGCCCTTCCGGCTGGCTGGTTTATTGCTGATAAATCTGGA
GCCGGTGAGCGTGGAAGCCGCGGTATCATTGCAGCACTGGGGCCAGATGGTA
AGCCCTCCCGTATCGTAGTTATCTACACGACGGGGAGTCAGGCAACTATGGA
TGAACGAAATAGACAGATCGCTGAGATAGGTGCCTCACTGATTAAGCATTGG
TAACTGTCAGACCAAGTTTACTCATATATACTTTAGATTGATTTAAAACTTCA
TTTTTAATTTAAAAGGATCTAGGTGAAGATCCTTTTTGATAATCTCATGACCA
AAATCCCTTAACGTGAGTTTTCGTTCCACTGAGCGTCAGACCCCGTAGAAAAG
ATCAAAGGATCTTCTTGAGATCCTTTTTTTCTGCGCGTAATCTGCTGCTTGCAA
ACAAAAAAACCACCGCTACCAGCGGTGGTTTGTTTGCCGGATCAAGAGCTAC
CAACTCTTTTTCCGAAGGTAACTGGCTTCAGCAGAGCGCAGATACCAAATACT
GTTCTTCTAGTGTAGCCGTAGTTAGGCCACCACTTCAAGAACTCTGTAGCACC
GCCTACATACCTCGCTCTGCTAATCCTGTTACCAGTGGCTGCTGCCAGTGGCG
ATAAGTCGTGTCTTACCGGGTTGGACTCAAGACGATAGTTACCGGATAAGGC
GCAGCGGTCGGGCTGAACGGGGGGTTCGTGCACACAGCCCAGCTTGGAGCGA
ACGACCTACACCGAACTGAGATACCTACAGCGTGAGCTATGAGAAAGCGCCA
CGCTTCCCGAAGGGAGAAAGGCGGACAGGTATCCGGTAAGCGGCAGGGTCG
GAACAGGAGAGCGCACGAGGGAGCTTCCAGGGGGAAACGCCTGGTATCTTTA
TAGTCCTGTCGGGTTTCGCCACCTCTGACTTGAGCGTCGATTTTTGTGATGCTC
GTCAGGGGGGCGGAGCCTATGGAAAAACGCCAGCAACGCGGCCTTTTTACGG
TTCCTGGCCTTTTGCTGGCCTTTTGCTCACATGT
**pGW097-pAAV_EFSLTR-IL23-sPA (SEQ ID NO: 31)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccac
ATGTGTCCTCAGAAGCTAACCATCTCCTGGTTTGCCATCGTTTTGCTGGTGTCT
CCACTCATGGCCATGTGGGAGCTGGAGAAAGACGTTTATGTTGTAGAGGTGG
ACTGGACTCCCGATGCCCCTGGAGAAACAGTGAACCTCACCTGTGACACGCC
TGAAGAAGATGACATCACCTGGACCTCAGACCAGAGACATGGAGTCATAGGC
TCTGGAAAGACCCTGACCATCACTGTCAAAGAGTTTCTAGATGCTGGCCAGTA
CACCTGCCACAAAGGAGGCGAGACTCTGAGCCACTCACATCTGCTGCTCCAC
AAGAAGGAAAATGGAATTTGGTCCACTGAAATTTTAAAAAATTTCAAAAACA
AGACTTTCCTGAAGTGTGAAGCACCAAATTACTCCGGACGGTTCACGTGCTCA
TGGCTGGTGCAAAGAAACATGGACTTGAAGTTCAACATCAAGAGCAGTAGCA
GTTCCCCTGACTCTCGGGCAGTGACATGTGGAATGGCGTCTCTGTCTGCAGAG
AAGGTCACACTGGACCAAAGGGACTATGAGAAGTATTCAGTGTCCTGCCAGG
AGGATGTCACCTGCCCAACTGCCGAGGAGACCCTGCCCATTGAACTGGCGTT
GGAAGCACGGCAGCAGAATAAATATGAGAACTACAGCACCAGCTTCTTCATC
AGGGACATCATCAAACCAGACCCGCCCAAGAACTTGCAGATGAAGCCTTTGA
AGAACTCACAGGTGGAGGTCAGCTGGGAGTACCCTGACTCCTGGAGCACTCC
CCATTCCTACTTCTCCCTCAAGTTCTTTGTTCGAATCCAGCGCAAGAAAGAAA
AGATGAAGGAGACAGAGGAGGGGTGTAACCAGAAAGGTGCGTTCCTCGTAG
AGAAGACATCTACCGAAGTCCAATGCAAAGGCGGGAATGTCTGCGTGCAAGC
TCAGGATCGCTATTACAATTCCTCATGCAGCAAGTGGGCATGTGTTCCCTGCA
GGGTCCGATCCTAGGAATTCAATAAAAGATCTTTATTTTCATTAGATCTGTGT
GTTGGTTTTTTGTGTGCGGCCGCAGGAACCCCTAGTGATGGAGTTGGCCACTC
CCTCTCTGCGCGCTCGCTCGCTCACTGAGGCCGGGCGACCAAAGGTCGCCCG
ACGCCCGGGCTTTGCCCGGGCGGCCTCAGTGAGCGAGCGAGCGCGCAGCTGC
CTGCAGGGGCGCCTGATGCGGTATTTTCTCCTTACGCATCTGTGCGGTATTTC
ACACCGCATACGTCAAAGCAACCATAGTACGCGCCCTGTAGCGGCGCATTAA
GCGCGGCGGGTGTGGTGGTTACGCGCAGCGTGACCGCTACACTTGCCAGCGC
CTTAGCGCCCGCTCCTTTCGCTTTCTTCCCTTCCTTTCTCGCCACGTTCGCCGG
CTTTCCCCGTCAAGCTCTAAATCGGGGGCTCCCTTTAGGGTTCCGATTTAGTG
CTTTACGGCACCTCGACCCCAAAAAACTTGATTTGGGTGATGGTTCACGTAGT
GGGCCATCGCCCTGATAGACGGTTTTTCGCCCTTTGACGTTGGAGTCCACGTT
CTTTAATAGTGGACTCTTGTTCCAAACTGGAACAACACTCAACTCTATCTCGG
GCTATTCTTTTGATTTATAAGGGATTTTGCCGATTTCGGTCTATTGGTTAAAAA
ATGAGCTGATTTAACAAAAATTTAACGCGAATTTTAACAAAATATTAACGTTT
ACAATTTTATGGTGCACTCTCAGTACAATCTGCTCTGATGCCGCATAGTTAAG
CCAGCCCCGACACCCGCCAACACCCGCTGACGCGCCCTGACGGGCTTGTCTG
 
CTCCCGGCATCCGCTTACAGACAAGCTGTGACCGTCTCCGGGAGCTGCATGTG
TCAGAGGTTTTCACCGTCATCACCGAAACGCGCGAGACGAAAGGGCCTCGTG
ATACGCCTATTTTTATAGGTTAATGTCATGATAATAATGGTTTCTTAGACGTC
AGGTGGCACTTTTCGGGGAAATGTGCGCGGAACCCCTATTTGTTTATTTTTCT
AAATACATTCAAATATGTATCCGCTCATGAGACAATAACCCTGATAAATGCTT
CAATAATATTGAAAAAGGAAGAGTATGAGTATTCAACATTTCCGTGTCGCCCT
TATTCCCTTTTTTGCGGCATTTTGCCTTCCTGTTTTTGCTCACCCAGAAACGCT
GGTGAAAGTAAAAGATGCTGAAGATCAGTTGGGTGCACGAGTGGGTTACATC
GAACTGGATCTCAACAGCGGTAAGATCCTTGAGAGTTTTCGCCCCGAAGAAC
GTTTTCCAATGATGAGCACTTTTAAAGTTCTGCTATGTGGCGCGGTATTATCC
CGTATTGACGCCGGGCAAGAGCAACTCGGTCGCCGCATACACTATTCTCAGA
ATGACTTGGTTGAGTACTCACCAGTCACAGAAAAGCATCTTACGGATGGCAT
GACAGTAAGAGAATTATGCAGTGCTGCCATAACCATGAGTGATAACACTGCG
GCCAACTTACTTCTGACAACGATCGGAGGACCGAAGGAGCTAACCGCTTTTTT
GCACAACATGGGGGATCATGTAACTCGCCTTGATCGTTGGGAACCGGAGCTG
AATGAAGCCATACCAAACGACGAGCGTGACACCACGATGCCTGTAGCAATGG
CAACAACGTTGCGCAAACTATTAACTGGCGAACTACTTACTCTAGCTTCCCGG
CAACAATTAATAGACTGGATGGAGGCGGATAAAGTTGCAGGACCACTTCTGC
GCTCGGCCCTTCCGGCTGGCTGGTTTATTGCTGATAAATCTGGAGCCGGTGAG
CGTGGAAGCCGCGGTATCATTGCAGCACTGGGGCCAGATGGTAAGCCCTCCC
GTATCGTAGTTATCTACACGACGGGGAGTCAGGCAACTATGGATGAACGAAA
TAGACAGATCGCTGAGATAGGTGCCTCACTGATTAAGCATTGGTAACTGTCA
GACCAAGTTTACTCATATATACTTTAGATTGATTTAAAACTTCATTTTTAATTT
AAAAGGATCTAGGTGAAGATCCTTTTTGATAATCTCATGACCAAAATCCCTTA
ACGTGAGTTTTCGTTCCACTGAGCGTCAGACCCCGTAGAAAAGATCAAAGGA
TCTTCTTGAGATCCTTTTTTTCTGCGCGTAATCTGCTGCTTGCAAACAAAAAAA
CCACCGCTACCAGCGGTGGTTTGTTTGCCGGATCAAGAGCTACCAACTCTTTT
TCCGAAGGTAACTGGCTTCAGCAGAGCGCAGATACCAAATACTGTTCTTCTAG
TGTAGCCGTAGTTAGGCCACCACTTCAAGAACTCTGTAGCACCGCCTACATAC
CTCGCTCTGCTAATCCTGTTACCAGTGGCTGCTGCCAGTGGCGATAAGTCGTG
TCTTACCGGGTTGGACTCAAGACGATAGTTACCGGATAAGGCGCAGCGGTCG
GGCTGAACGGGGGGTTCGTGCACACAGCCCAGCTTGGAGCGAACGACCTACA
CCGAACTGAGATACCTACAGCGTGAGCTATGAGAAAGCGCCACGCTTCCCGA
AGGGAGAAAGGCGGACAGGTATCCGGTAAGCGGCAGGGTCGGAACAGGAGA
GCGCACGAGGGAGCTTCCAGGGGGAAACGCCTGGTATCTTTATAGTCCTGTC
GGGTTTCGCCACCTCTGACTTGAGCGTCGATTTTTGTGATGCTCGTCAGGGGG
GCGGAGCCTATGGAAAAACGCCAGCAACGCGGCCTTTTTACGGTTCCTGGCC
TTTTGCTGGCCTTTTGCTCACATGT
**pGW098-pAAV_EFSLTR-Ifng-P2A-Cd80-T2A-light-P2A-IL23-sPA (SEQ ID NO: 32)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
 
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atgaacgctacacactgcatcttggctttgcagctcttcctcatggctgtttctggctgttactgccacggcacagtcattgaaagcct
agaaagtctgaataactattttaactcaagtggcatagatgtggaagaaaagagtctcttcttggatatctggaggaactggcaaaa
ggatggtgacatgaaaatcctgcagagccagattatctctttctacctcagactctttgaagtcttgaaagacaatcaggccatcag
caacaacataagcgtcattgaatcacacctgattactaccttcttcagcaacagcaaggcgaaaaaggatgcattcatgagtattg
ccaagtttgaggtcaacaacccacaggtccagcgccaagcattcaatgagctcatccgagtggtccaccagctgttgccggaat
ccagcctcaggaagcggaaaaggagtcgctgcGGATCCGGCGCAACAAACTTCTCTCTGCTGAA
ACAAGCCGGAGATGTCGAAGAGAATCCTGGACCGatggcttgcaattgtcagttgatgcaggat
acaccactcctcaagtttccatgtccaaggctcattcttctctttgtgctgctgattcgtctttcacaagtgtcttcagatgttgatgaac
aactgtccaagtcagtgaaagataaggtattgctgccttgccgttacaactctcctcatgaagatgagtctgaagaccgaatctact
ggcaaaaacatgacaaagtggtgctgtctgtcattgTtgggaaactaaaagtgtggcccgagtataagaaccggactttatatga
caacactacctactctcttatcatcctgggcctggtcctttcagaccggggcacatacagctgtgtcgttcaaaagaaggaaagag
gaacgtatgaagttaaacacttggctttagtaaagttgtccatcaaagctgacttctctacccccaacataactgagtctggaaaccc
atctgcagacactaaaaggattacctgctttgcttccgggggtttcccaaagcctcgcttctcttggttggaaaatggaagagaatta
cctggcatcaatacgacaatttcccaggatcctgaatctgaattgtacaccattagtagccaactagatttcaatacgactcgcaac
cacaccattaagtgtctcattaaatatggagatgctcacgtgtcagaggacttcacctgggaaaaacccccagaagaccctcctg
atagcaagaacacacttgtgctctttggggcaggattcggcgcagtaataacagtcgtcgtcatcgttgtcatcatcaaatgcttctg
taagcacagaagctgtttcagaagaaatgaggcaagcagagaaacaaacaacagccttaccttcgggcctgaagaagcattag
ctgaacagaccgtcttccttcgtacgGGCAGTGGAGAGGGCAGAGGAAGTCTGCTAACATGC
GGTGACGTCGAGGAGAATCCTGGCCCAatggagagtgtggtacagccttcagtgtttgtggtggatgga
cagacggacatcccattcaggcggctggaacagaaccaccggagacggcgctgtggcactgtccaggtcagcctggccctgg
tgctgctgctaggtgctgggctggccactcagggctggtttctcctgagactgcatcaacgtcttggagacatagtagctcatctgc
cagatggaggcaaaggctcctgggagaagctgatacaagatcaacgatctcaccaggccaacccagcagcacatcttacagg
agccaacgccagcttgataggtattggtggacctctgttatgggagacacgacttggcctggccttcttgaggggcttgacgtatc
atgatggggccctggtgaccatggagcccggttactactatgtgtactccaaagtgcagctgagcggcgtgggctgcccccagg
ggctggccaatggcctccccatcacccatggactatacaagcgcacatcccgctacccgaaggagttagaactgctggtcagtc
ggcggtcaccctgtggccgggccaacagctcccgagtctggtgggacagcagcttcctgggcggcgtggtacatctggaggc
tggggaagaggtggtggtccgcgtgcctggaaaccgcctggtcagaccacgtgacggcaccaggtcctatttcggagctttcat
ggtcactagtGGATCCGGCGCAACAAACTTCTCTCTGCTGAAACAAGCCGGAGATG
TCGAAGAGAATCCTGGACCGATGTGTCCTCAGAAGCTAACCATCTCCTGGTTT
GCCATCGTTTTGCTGGTGTCTCCACTCATGGCCATGTGGGAGCTGGAGAAAGA
CGTTTATGTTGTAGAGGTGGACTGGACTCCCGATGCCCCTGGAGAAACAGTG
AACCTCACCTGTGACACGCCTGAAGAAGATGACATCACCTGGACCTCAGACC
AGAGACATGGAGTCATAGGCTCTGGAAAGACCCTGACCATCACTGTCAAAGA
GTTTCTAGATGCTGGCCAGTACACCTGCCACAAAGGAGGCGAGACTCTGAGC
CACTCACATCTGCTGCTCCACAAGAAGGAAAATGGAATTTGGTCCACTGAAA
TTTTAAAAAATTTCAAAAACAAGACTTTCCTGAAGTGTGAAGCACCAAATTAC
TCCGGACGGTTCACGTGCTCATGGCTGGTGCAAAGAAACATGGACTTGAAGT
TCAACATCAAGAGCAGTAGCAGTTCCCCTGACTCTCGGGCAGTGACATGTGG
AATGGCGTCTCTGTCTGCAGAGAAGGTCACACTGGACCAAAGGGACTATGAG
AAGTATTCAGTGTCCTGCCAGGAGGATGTCACCTGCCCAACTGCCGAGGAGA
CCCTGCCCATTGAACTGGCGTTGGAAGCACGGCAGCAGAATAAATATGAGAA
CTACAGCACCAGCTTCTTCATCAGGGACATCATCAAACCAGACCCGCCCAAG
AACTTGCAGATGAAGCCTTTGAAGAACTCACAGGTGGAGGTCAGCTGGGAGT
ACCCTGACTCCTGGAGCACTCCCCATTCCTACTTCTCCCTCAAGTTCTTTGTTC
GAATCCAGCGCAAGAAAGAAAAGATGAAGGAGACAGAGGAGGGGTGTAACC
AGAAAGGTGCGTTCCTCGTAGAGAAGACATCTACCGAAGTCCAATGCAAAGG
 
CGGGAATGTCTGCGTGCAAGCTCAGGATCGCTATTACAATTCCTCATGCAGCA
AGTGGGCATGTGTTCCCTGCAGGGTCCGATCCtgaGAATTCAATAAAAGATCTT
TATTTTCATTAGATCTGTGTGTTGGTTTTTTGTGTGCGGCCGCAGGAACCCCTA
GTGATGGAGTTGGCCACTCCCTCTCTGCGCGCTCGCTCGCTCACTGAGGCCGG
GCGACCAAAGGTCGCCCGACGCCCGGGCTTTGCCCGGGCGGCCTCAGTGAGC
GAGCGAGCGCGCAGCTGCCTGCAGGGGCGCCTGATGCGGTATTTTCTCCTTAC
GCATCTGTGCGGTATTTCACACCGCATACGTCAAAGCAACCATAGTACGCGCC
CTGTAGCGGCGCATTAAGCGCGGCGGGTGTGGTGGTTACGCGCAGCGTGACC
GCTACACTTGCCAGCGCCTTAGCGCCCGCTCCTTTCGCTTTCTTCCCTTCCTTT
CTCGCCACGTTCGCCGGCTTTCCCCGTCAAGCTCTAAATCGGGGGCTCCCTTT
AGGGTTCCGATTTAGTGCTTTACGGCACCTCGACCCCAAAAAACTTGATTTGG
GTGATGGTTCACGTAGTGGGCCATCGCCCTGATAGACGGTTTTTCGCCCTTTG
ACGTTGGAGTCCACGTTCTTTAATAGTGGACTCTTGTTCCAAACTGGAACAAC
ACTCAACTCTATCTCGGGCTATTCTTTTGATTTATAAGGGATTTTGCCGATTTC
GGTCTATTGGTTAAAAAATGAGCTGATTTAACAAAAATTTAACGCGAATTTTA
ACAAAATATTAACGTTTACAATTTTATGGTGCACTCTCAGTACAATCTGCTCT
GATGCCGCATAGTTAAGCCAGCCCCGACACCCGCCAACACCCGCTGACGCGC
CCTGACGGGCTTGTCTGCTCCCGGCATCCGCTTACAGACAAGCTGTGACCGTC
TCCGGGAGCTGCATGTGTCAGAGGTTTTCACCGTCATCACCGAAACGCGCGA
GACGAAAGGGCCTCGTGATACGCCTATTTTTATAGGTTAATGTCATGATAATA
ATGGTTTCTTAGACGTCAGGTGGCACTTTTCGGGGAAATGTGCGCGGAACCCC
TATTTGTTTATTTTTCTAAATACATTCAAATATGTATCCGCTCATGAGACAATA
ACCCTGATAAATGCTTCAATAATATTGAAAAAGGAAGAGTATGAGTATTCAA
CATTTCCGTGTCGCCCTTATTCCCTTTTTTGCGGCATTTTGCCTTCCTGTTTTTG
CTCACCCAGAAACGCTGGTGAAAGTAAAAGATGCTGAAGATCAGTTGGGTGC
ACGAGTGGGTTACATCGAACTGGATCTCAACAGCGGTAAGATCCTTGAGAGT
TTTCGCCCCGAAGAACGTTTTCCAATGATGAGCACTTTTAAAGTTCTGCTATG
TGGCGCGGTATTATCCCGTATTGACGCCGGGCAAGAGCAACTCGGTCGCCGC
ATACACTATTCTCAGAATGACTTGGTTGAGTACTCACCAGTCACAGAAAAGC
ATCTTACGGATGGCATGACAGTAAGAGAATTATGCAGTGCTGCCATAACCAT
GAGTGATAACACTGCGGCCAACTTACTTCTGACAACGATCGGAGGACCGAAG
GAGCTAACCGCTTTTTTGCACAACATGGGGGATCATGTAACTCGCCTTGATCG
TTGGGAACCGGAGCTGAATGAAGCCATACCAAACGACGAGCGTGACACCACG
ATGCCTGTAGCAATGGCAACAACGTTGCGCAAACTATTAACTGGCGAACTAC
TTACTCTAGCTTCCCGGCAACAATTAATAGACTGGATGGAGGCGGATAAAGT
TGCAGGACCACTTCTGCGCTCGGCCCTTCCGGCTGGCTGGTTTATTGCTGATA
AATCTGGAGCCGGTGAGCGTGGAAGCCGCGGTATCATTGCAGCACTGGGGCC
AGATGGTAAGCCCTCCCGTATCGTAGTTATCTACACGACGGGGAGTCAGGCA
ACTATGGATGAACGAAATAGACAGATCGCTGAGATAGGTGCCTCACTGATTA
AGCATTGGTAACTGTCAGACCAAGTTTACTCATATATACTTTAGATTGATTTA
AAACTTCATTTTTAATTTAAAAGGATCTAGGTGAAGATCCTTTTTGATAATCT
CATGACCAAAATCCCTTAACGTGAGTTTTCGTTCCACTGAGCGTCAGACCCCG
TAGAAAAGATCAAAGGATCTTCTTGAGATCCTTTTTTTCTGCGCGTAATCTGC
TGCTTGCAAACAAAAAAACCACCGCTACCAGCGGTGGTTTGTTTGCCGGATC
AAGAGCTACCAACTCTTTTTCCGAAGGTAACTGGCTTCAGCAGAGCGCAGAT
ACCAAATACTGTTCTTCTAGTGTAGCCGTAGTTAGGCCACCACTTCAAGAACT
CTGTAGCACCGCCTACATACCTCGCTCTGCTAATCCTGTTACCAGTGGCTGCT
GCCAGTGGCGATAAGTCGTGTCTTACCGGGTTGGACTCAAGACGATAGTTAC
CGGATAAGGCGCAGCGGTCGGGCTGAACGGGGGGTTCGTGCACACAGCCCAG
CTTGGAGCGAACGACCTACACCGAACTGAGATACCTACAGCGTGAGCTATGA
 
GAAAGCGCCACGCTTCCCGAAGGGAGAAAGGCGGACAGGTATCCGGTAAGC
GGCAGGGTCGGAACAGGAGAGCGCACGAGGGAGCTTCCAGGGGGAAACGCC
TGGTATCTTTATAGTCCTGTCGGGTTTCGCCACCTCTGACTTGAGCGTCGATTT
TTGTGATGCTCGTCAGGGGGGCGGAGCCTATGGAAAAACGCCAGCAACGCGG
CCTTTTTACGGTTCCTGGCCTTTTGCTGGCCTTTTGCTCACATGT
**pGW099-pAAV_EFSLTR-IFNg-P2A-Cd80-T2A-light-P2A-cxcl10 (SEQ ID NO: 33)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atgaacgctacacactgcatcttggctttgcagctcttcctcatggctgtttctggctgttactgccacggcacagtcattgaaagcct
agaaagtctgaataactattttaactcaagtggcatagatgtggaagaaaagagtctcttcttggatatctggaggaactggcaaaa
ggatggtgacatgaaaatcctgcagagccagattatctctttctacctcagactctttgaagtcttgaaagacaatcaggccatcag
caacaacataagcgtcattgaatcacacctgattactaccttcttcagcaacagcaaggcgaaaaaggatgcattcatgagtattg
ccaagtttgaggtcaacaacccacaggtccagcgccaagcattcaatgagctcatccgagtggtccaccagctgttgccggaat
ccagcctcaggaagcggaaaaggagtcgctgcGGATCCGGCGCAACAAACTTCTCTCTGCTGAA
ACAAGCCGGAGATGTCGAAGAGAATCCTGGACCGatggcttgcaattgtcagttgatgcaggat
acaccactcctcaagtttccatgtccaaggctcattcttctctttgtgctgctgattcgtctttcacaagtgtcttcagatgttgatgaac
aactgtccaagtcagtgaaagataaggtattgctgccttgccgttacaactctcctcatgaagatgagtctgaagaccgaatctact
ggcaaaaacatgacaaagtggtgctgtctgtcattgTtgggaaactaaaagtgtggcccgagtataagaaccggactttatatga
caacactacctactctcttatcatcctgggcctggtcctttcagaccggggcacatacagctgtgtcgttcaaaagaaggaaagag
gaacgtatgaagttaaacacttggctttagtaaagttgtccatcaaagctgacttctctacccccaacataactgagtctggaaaccc
atctgcagacactaaaaggattacctgctttgcttccgggggtttcccaaagcctcgcttctcttggttggaaaatggaagagaatta
cctggcatcaatacgacaatttcccaggatcctgaatctgaattgtacaccattagtagccaactagatttcaatacgactcgcaac
cacaccattaagtgtctcattaaatatggagatgctcacgtgtcagaggacttcacctgggaaaaacccccagaagaccctcctg
atagcaagaacacacttgtgctctttggggcaggattcggcgcagtaataacagtcgtcgtcatcgttgtcatcatcaaatgcttctg
taagcacagaagctgtttcagaagaaatgaggcaagcagagaaacaaacaacagccttaccttcgggcctgaagaagcattag
ctgaacagaccgtcttccttcgtacgGGCAGTGGAGAGGGCAGAGGAAGTCTGCTAACATGC
GGTGACGTCGAGGAGAATCCTGGCCCAatggagagtgtggtacagccttcagtgtttgtggtggatgga
cagacggacatcccattcaggcggctggaacagaaccaccggagacggcgctgtggcactgtccaggtcagcctggccctgg
tgctgctgctaggtgctgggctggccactcagggctggtttctcctgagactgcatcaacgtcttggagacatagtagctcatctgc
cagatggaggcaaaggctcctgggagaagctgatacaagatcaacgatctcaccaggccaacccagcagcacatcttacagg
agccaacgccagcttgataggtattggtggacctctgttatgggagacacgacttggcctggccttcttgaggggcttgacgtatc
atgatggggccctggtgaccatggagcccggttactactatgtgtactccaaagtgcagctgagcggcgtgggctgcccccagg
ggctggccaatggcctccccatcacccatggactatacaagcgcacatcccgctacccgaaggagttagaactgctggtcagtc
ggcggtcaccctgtggccgggccaacagctcccgagtctggtgggacagcagcttcctgggcggcgtggtacatctggaggc
tggggaagaggtggtggtccgcgtgcctggaaaccgcctggtcagaccacgtgacggcaccaggtcctatttcggagctttcat
ggtcactagtGGATCCGGCGCAACAAACTTCTCTCTGCTGAAACAAGCCGGAGATG
TCGAAGAGAATCCTGGACCGatgaacccaagtgctgccgtcattttctgcctcatcctgctgggtctgagtggg
actcaagggatccctctcgcaaggacggtccgctgcaactgcatccatatcgatgacgggccagtgagaatgagggccatagg
 
gaagcttgaaatcatccctgcgagcctatcctgcccacgtgttgagatcattgccacgatgaaaaagaatgatgagcagagatgt
ctgaatccggaatctaagaccatcaagaatttaatgaaagcgtttagccaaaaaaggtctaaaagggctccttgaGAATTCA
ATAAAAGATCTTTATTTTCATTAGATCTGTGTGTTGGTTTTTTGTGTGCGGCCG
CAGGAACCCCTAGTGATGGAGTTGGCCACTCCCTCTCTGCGCGCTCGCTCGCT
CACTGAGGCCGGGCGACCAAAGGTCGCCCGACGCCCGGGCTTTGCCCGGGCG
GCCTCAGTGAGCGAGCGAGCGCGCAGCTGCCTGCAGGGGCGCCTGATGCGGT
ATTTTCTCCTTACGCATCTGTGCGGTATTTCACACCGCATACGTCAAAGCAAC
CATAGTACGCGCCCTGTAGCGGCGCATTAAGCGCGGCGGGTGTGGTGGTTAC
GCGCAGCGTGACCGCTACACTTGCCAGCGCCTTAGCGCCCGCTCCTTTCGCTT
TCTTCCCTTCCTTTCTCGCCACGTTCGCCGGCTTTCCCCGTCAAGCTCTAAATC
GGGGGCTCCCTTTAGGGTTCCGATTTAGTGCTTTACGGCACCTCGACCCCAAA
AAACTTGATTTGGGTGATGGTTCACGTAGTGGGCCATCGCCCTGATAGACGGT
TTTTCGCCCTTTGACGTTGGAGTCCACGTTCTTTAATAGTGGACTCTTGTTCCA
AACTGGAACAACACTCAACTCTATCTCGGGCTATTCTTTTGATTTATAAGGGA
TTTTGCCGATTTCGGTCTATTGGTTAAAAAATGAGCTGATTTAACAAAAATTT
AACGCGAATTTTAACAAAATATTAACGTTTACAATTTTATGGTGCACTCTCAG
TACAATCTGCTCTGATGCCGCATAGTTAAGCCAGCCCCGACACCCGCCAACA
CCCGCTGACGCGCCCTGACGGGCTTGTCTGCTCCCGGCATCCGCTTACAGACA
AGCTGTGACCGTCTCCGGGAGCTGCATGTGTCAGAGGTTTTCACCGTCATCAC
CGAAACGCGCGAGACGAAAGGGCCTCGTGATACGCCTATTTTTATAGGTTAA
TGTCATGATAATAATGGTTTCTTAGACGTCAGGTGGCACTTTTCGGGGAAATG
TGCGCGGAACCCCTATTTGTTTATTTTTCTAAATACATTCAAATATGTATCCGC
TCATGAGACAATAACCCTGATAAATGCTTCAATAATATTGAAAAAGGAAGAG
TATGAGTATTCAACATTTCCGTGTCGCCCTTATTCCCTTTTTTGCGGCATTTTG
CCTTCCTGTTTTTGCTCACCCAGAAACGCTGGTGAAAGTAAAAGATGCTGAAG
ATCAGTTGGGTGCACGAGTGGGTTACATCGAACTGGATCTCAACAGCGGTAA
GATCCTTGAGAGTTTTCGCCCCGAAGAACGTTTTCCAATGATGAGCACTTTTA
AAGTTCTGCTATGTGGCGCGGTATTATCCCGTATTGACGCCGGGCAAGAGCA
ACTCGGTCGCCGCATACACTATTCTCAGAATGACTTGGTTGAGTACTCACCAG
TCACAGAAAAGCATCTTACGGATGGCATGACAGTAAGAGAATTATGCAGTGC
TGCCATAACCATGAGTGATAACACTGCGGCCAACTTACTTCTGACAACGATCG
GAGGACCGAAGGAGCTAACCGCTTTTTTGCACAACATGGGGGATCATGTAAC
TCGCCTTGATCGTTGGGAACCGGAGCTGAATGAAGCCATACCAAACGACGAG
CGTGACACCACGATGCCTGTAGCAATGGCAACAACGTTGCGCAAACTATTAA
CTGGCGAACTACTTACTCTAGCTTCCCGGCAACAATTAATAGACTGGATGGAG
GCGGATAAAGTTGCAGGACCACTTCTGCGCTCGGCCCTTCCGGCTGGCTGGTT
TATTGCTGATAAATCTGGAGCCGGTGAGCGTGGAAGCCGCGGTATCATTGCA
GCACTGGGGCCAGATGGTAAGCCCTCCCGTATCGTAGTTATCTACACGACGG
GGAGTCAGGCAACTATGGATGAACGAAATAGACAGATCGCTGAGATAGGTGC
CTCACTGATTAAGCATTGGTAACTGTCAGACCAAGTTTACTCATATATACTTT
AGATTGATTTAAAACTTCATTTTTAATTTAAAAGGATCTAGGTGAAGATCCTT
TTTGATAATCTCATGACCAAAATCCCTTAACGTGAGTTTTCGTTCCACTGAGC
GTCAGACCCCGTAGAAAAGATCAAAGGATCTTCTTGAGATCCTTTTTTTCTGC
GCGTAATCTGCTGCTTGCAAACAAAAAAACCACCGCTACCAGCGGTGGTTTG
TTTGCCGGATCAAGAGCTACCAACTCTTTTTCCGAAGGTAACTGGCTTCAGCA
GAGCGCAGATACCAAATACTGTTCTTCTAGTGTAGCCGTAGTTAGGCCACCAC
TTCAAGAACTCTGTAGCACCGCCTACATACCTCGCTCTGCTAATCCTGTTACC
AGTGGCTGCTGCCAGTGGCGATAAGTCGTGTCTTACCGGGTTGGACTCAAGA
CGATAGTTACCGGATAAGGCGCAGCGGTCGGGCTGAACGGGGGGTTCGTGCA
CACAGCCCAGCTTGGAGCGAACGACCTACACCGAACTGAGATACCTACAGCG
 
TGAGCTATGAGAAAGCGCCACGCTTCCCGAAGGGAGAAAGGCGGACAGGTA
TCCGGTAAGCGGCAGGGTCGGAACAGGAGAGCGCACGAGGGAGCTTCCAGG
GGGAAACGCCTGGTATCTTTATAGTCCTGTCGGGTTTCGCCACCTCTGACTTG
AGCGTCGATTTTTGTGATGCTCGTCAGGGGGGCGGAGCCTATGGAAAAACGC
CAGCAACGCGGCCTTTTTACGGTTCCTGGCCTTTTGCTGGCCTTTTGCTCACAT
GT
**pGW100-pAAV_EFSLTR-Ifng-P2A-Cd80-T2A-light-P2A-Cxcl10-E2A-41BBL-sPA (SEQ ID NO: 34)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atgaacgctacacactgcatcttggctttgcagctcttcctcatggctgtttctggctgttactgccacggcacagtcattgaaagcct
agaaagtctgaataactattttaactcaagtggcatagatgtggaagaaaagagtctcttcttggatatctggaggaactggcaaaa
ggatggtgacatgaaaatcctgcagagccagattatctctttctacctcagactctttgaagtcttgaaagacaatcaggccatcag
caacaacataagcgtcattgaatcacacctgattactaccttcttcagcaacagcaaggcgaaaaaggatgcattcatgagtattg
ccaagtttgaggtcaacaacccacaggtccagcgccaagcattcaatgagctcatccgagtggtccaccagctgttgccggaat
ccagcctcaggaagcggaaaaggagtcgctgcGGATCCGGCGCAACAAACTTCTCTCTGCTGAA
ACAAGCCGGAGATGTCGAAGAGAATCCTGGACCGatggcttgcaattgtcagttgatgcaggat
acaccactcctcaagtttccatgtccaaggctcattcttctctttgtgctgctgattcgtctttcacaagtgtcttcagatgttgatgaac
aactgtccaagtcagtgaaagataaggtattgctgccttgccgttacaactctcctcatgaagatgagtctgaagaccgaatctact
ggcaaaaacatgacaaagtggtgctgtctgtcattgTtgggaaactaaaagtgtggcccgagtataagaaccggactttatatga
caacactacctactctcttatcatcctgggcctggtcctttcagaccggggcacatacagctgtgtcgttcaaaagaaggaaagag
gaacgtatgaagttaaacacttggctttagtaaagttgtccatcaaagctgacttctctacccccaacataactgagtctggaaaccc
atctgcagacactaaaaggattacctgctttgcttccgggggtttcccaaagcctcgcttctcttggttggaaaatggaagagaatta
cctggcatcaatacgacaatttcccaggatcctgaatctgaattgtacaccattagtagccaactagatttcaatacgactcgcaac
cacaccattaagtgtctcattaaatatggagatgctcacgtgtcagaggacttcacctgggaaaaacccccagaagaccctcctg
atagcaagaacacacttgtgctctttggggcaggattcggcgcagtaataacagtcgtcgtcatcgttgtcatcatcaaatgcttctg
taagcacagaagctgtttcagaagaaatgaggcaagcagagaaacaaacaacagccttaccttcgggcctgaagaagcattag
ctgaacagaccgtcttccttcgtacgGGCAGTGGAGAGGGCAGAGGAAGTCTGCTAACATGC
GGTGACGTCGAGGAGAATCCTGGCCCAatggagagtgtggtacagccttcagtgtttgtggtggatgga
cagacggacatcccattcaggcggctggaacagaaccaccggagacggcgctgtggcactgtccaggtcagcctggccctgg
tgctgctgctaAgtgctgggctggccactcagggctggtttctcctgagactgcatcaacgtcttggagacatagtagctcatctg
ccagatggaggcaaaggctcctgggagaagctgatacaagatcaacgatctcaccaggccaacccagcagcacatcttacag
gagccaacgccagcttgataggtattggtggacctctgttatgggagacacgacttggcctggccttcttgaggggcttgacgtat
catgatggggccctggtgaccatggagcccggttactactatgtgtactccaaagtgcagctgagcggcgtgggctgcccccag
gggctggccaatggcctccccatcacccatggactatacaagcgcacatcccgctacccgaaggagttagaactgctggtcagt
cggcggtcaccctgtggccgggccaacagctcccgagtctggtgggacagcagcttcctgggcggcgtggtacatctggagg
ctggggaagaggtggtggtccgcgtgcctggaaaccgcctggtcagaccacgtgacggcaccaggtcctatttcggagctttca
tggtcactagtGGATCCGGCGCAACAAACTTCTCTCTGCTGAAACAAGCCGGAGATG
 
TCGAAGAGAATCCTGGACCGatgaacccaagtgctgccgtcattttctgcctcatcctgctgggtctgagtggg
actcaagggatccctctcgcaaggacggtccgctgcaactgcatccatatcgatgacgggccagtgagaatgagggccatagg
gaagcttgaaatcatccctgcgagcctatcctgcccacgtgttgagatcattgccacgatgaaaaagaatgatgagcagagatgt
ctgaatccggaatctaagaccatcaagaatttaatgaaagcgtttagccaaaaaaggtctaaaagggctcctgctagcagcggta
cccagtgcaccaactacgccctgctgaagctggccggcgatgtggagagcaaccccgggcccatggaccagcacacacttga
tgtggaggataccgcggatgccagacatccagcaggtacttcgtgcccctcggatgcggcgctcctcagagataccgggctcct
cgcggacgctgcgctcctctcagatactgtgcgccccacaaatgccgcgctccccacggatgctgcctaccctgcggttaatgtt
cgggatcgcgaggccgcgtggccgcctgcactgaacttctgttcccgccacccaaagctctatggcctagtcgctttggttttgct
gcttctgatcgccgcctgtgttcctatcttcacccgcaccgagcctcggccagcgctcacaatcaccacctcgcccaacctgggt
acccgagagaataatgcagaccaggtcacccctgtttcccacattggctgccccaacactacacaacagggctctcctgtgttcg
ccaagctactggctaaaaaccaagcatcgttgtgcaatacaactctgaactggcacagccaagatggagctgggagctcatacc
tatctcaaggtctgaggtacgaagaagacaaaaaggagttggtggtagacagtcccgggctctactacgtatttttggaactgaag
ctcagtccaacattcacaaacacaggccacaaggtgcagggctgggtctctcttgttttgcaagcaaagcctcaggtagatgactt
tgacaacttggccctgacagtggaactgttcccttgctccatggagaacaagttagtggaccgttcctggagtcaactgttgctcct
gaaggctggccaccgcctcagtgtgggtctgagggcttatctgcatggagcccaggatgcatacagagactgggagctgtctta
tcccaacaccaccagctttggactctttcttgtgaaacccgacaacccatgggaatgaGAATTCAATAAAAGATC
TTTATTTTCATTAGATCTGTGTGTTGGTTTTTTGTGTGCGGCCGCAGGAACCCC
TAGTGATGGAGTTGGCCACTCCCTCTCTGCGCGCTCGCTCGCTCACTGAGGCC
GGGCGACCAAAGGTCGCCCGACGCCCGGGCTTTGCCCGGGCGGCCTCAGTGA
GCGAGCGAGCGCGCAGCTGCCTGCAGGGGCGCCTGATGCGGTATTTTCTCCTT
ACGCATCTGTGCGGTATTTCACACCGCATACGTCAAAGCAACCATAGTACGC
GCCCTGTAGCGGCGCATTAAGCGCGGCGGGTGTGGTGGTTACGCGCAGCGTG
ACCGCTACACTTGCCAGCGCCTTAGCGCCCGCTCCTTTCGCTTTCTTCCCTTCC
TTTCTCGCCACGTTCGCCGGCTTTCCCCGTCAAGCTCTAAATCGGGGGCTCCC
TTTAGGGTTCCGATTTAGTGCTTTACGGCACCTCGACCCCAAAAAACTTGATT
TGGGTGATGGTTCACGTAGTGGGCCATCGCCCTGATAGACGGTTTTTCGCCCT
TTGACGTTGGAGTCCACGTTCTTTAATAGTGGACTCTTGTTCCAAACTGGAAC
AACACTCAACTCTATCTCGGGCTATTCTTTTGATTTATAAGGGATTTTGCCGAT
TTCGGTCTATTGGTTAAAAAATGAGCTGATTTAACAAAAATTTAACGCGAATT
TTAACAAAATATTAACGTTTACAATTTTATGGTGCACTCTCAGTACAATCTGC
TCTGATGCCGCATAGTTAAGCCAGCCCCGACACCCGCCAACACCCGCTGACG
CGCCCTGACGGGCTTGTCTGCTCCCGGCATCCGCTTACAGACAAGCTGTGACC
GTCTCCGGGAGCTGCATGTGTCAGAGGTTTTCACCGTCATCACCGAAACGCGC
GAGACGAAAGGGCCTCGTGATACGCCTATTTTTATAGGTTAATGTCATGATAA
TAATGGTTTCTTAGACGTCAGGTGGCACTTTTCGGGGAAATGTGCGCGGAACC
CCTATTTGTTTATTTTTCTAAATACATTCAAATATGTATCCGCTCATGAGACAA
TAACCCTGATAAATGCTTCAATAATATTGAAAAAGGAAGAGTATGAGTATTC
AACATTTCCGTGTCGCCCTTATTCCCTTTTTTGCGGCATTTTGCCTTCCTGTTTT
TGCTCACCCAGAAACGCTGGTGAAAGTAAAAGATGCTGAAGATCAGTTGGGT
GCACGAGTGGGTTACATCGAACTGGATCTCAACAGCGGTAAGATCCTTGAGA
GTTTTCGCCCCGAAGAACGTTTTCCAATGATGAGCACTTTTAAAGTTCTGCTA
TGTGGCGCGGTATTATCCCGTATTGACGCCGGGCAAGAGCAACTCGGTCGCC
GCATACACTATTCTCAGAATGACTTGGTTGAGTACTCACCAGTCACAGAAAA
GCATCTTACGGATGGCATGACAGTAAGAGAATTATGCAGTGCTGCCATAACC
ATGAGTGATAACACTGCGGCCAACTTACTTCTGACAACGATCGGAGGACCGA
AGGAGCTAACCGCTTTTTTGCACAACATGGGGGATCATGTAACTCGCCTTGAT
CGTTGGGAACCGGAGCTGAATGAAGCCATACCAAACGACGAGCGTGACACCA
CGATGCCTGTAGCAATGGCAACAACGTTGCGCAAACTATTAACTGGCGAACT
ACTTACTCTAGCTTCCCGGCAACAATTAATAGACTGGATGGAGGCGGATAAA
GTTGCAGGACCACTTCTGCGCTCGGCCCTTCCGGCTGGCTGGTTTATTGCTGA
 
TAAATCTGGAGCCGGTGAGCGTGGAAGCCGCGGTATCATTGCAGCACTGGGG
CCAGATGGTAAGCCCTCCCGTATCGTAGTTATCTACACGACGGGGAGTCAGG
CAACTATGGATGAACGAAATAGACAGATCGCTGAGATAGGTGCCTCACTGAT
TAAGCATTGGTAACTGTCAGACCAAGTTTACTCATATATACTTTAGATTGATT
TAAAACTTCATTTTTAATTTAAAAGGATCTAGGTGAAGATCCTTTTTGATAAT
CTCATGACCAAAATCCCTTAACGTGAGTTTTCGTTCCACTGAGCGTCAGACCC
CGTAGAAAAGATCAAAGGATCTTCTTGAGATCCTTTTTTTCTGCGCGTAATCT
GCTGCTTGCAAACAAAAAAACCACCGCTACCAGCGGTGGTTTGTTTGCCGGA
TCAAGAGCTACCAACTCTTTTTCCGAAGGTAACTGGCTTCAGCAGAGCGCAG
ATACCAAATACTGTTCTTCTAGTGTAGCCGTAGTTAGGCCACCACTTCAAGAA
CTCTGTAGCACCGCCTACATACCTCGCTCTGCTAATCCTGTTACCAGTGGCTG
CTGCCAGTGGCGATAAGTCGTGTCTTACCGGGTTGGACTCAAGACGATAGTTA
CCGGATAAGGCGCAGCGGTCGGGCTGAACGGGGGGTTCGTGCACACAGCCCA
GCTTGGAGCGAACGACCTACACCGAACTGAGATACCTACAGCGTGAGCTATG
AGAAAGCGCCACGCTTCCCGAAGGGAGAAAGGCGGACAGGTATCCGGTAAG
CGGCAGGGTCGGAACAGGAGAGCGCACGAGGGAGCTTCCAGGGGGAAACGC
CTGGTATCTTTATAGTCCTGTCGGGTTTCGCCACCTCTGACTTGAGCGTCGATT
TTTGTGATGCTCGTCAGGGGGGCGGAGCCTATGGAAAAACGCCAGCAACGCG
GCCTTTTTACGGTTCCTGGCCTTTTGCTGGCCTTTTGCTCACATGT
**pGW101-pAAV_EFSLTR-Ifng-P2A-Cd80-T2A-light-E2A-41BBL-sPA (SEQ ID NO: 35)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atgaacgctacacactgcatcttggctttgcagctcttcctcatggctgtttctggctgttactgccacggcacagtcattgaaagcct
agaaagtctgaataactattttaactcaagtggcatagatgtggaagaaaagagtctcttcttggatatctggaggaactggcaaaa
ggatggtgacatgaaaatcctgcagagccagattatctctttctacctcagactctttgaagtcttgaaagacaatcaggccatcag
caacaacataagcgtcattgaatcacacctgattactaccttcttcagcaacagcaaggcgaaaaaggatgcattcatgagtattg
ccaagtttgaggtcaacaacccacaggtccagcgccaagcattcaatgagctcatccgagtggtccaccagctgttgccggaat
ccagcctcaggaagcggaaaaggagtcgctgcGGATCCGGCGCAACAAACTTCTCTCTGCTGAA
ACAAGCCGGAGATGTCGAAGAGAATCCTGGACCGatggcttgcaattgtcagttgatgcaggat
acaccactcctcaagtttccatgtccaaggctcattcttctctttgtgctgctgattcgtctttcacaagtgtcttcagatgttgatgaac
aactgtccaagtcagtgaaagataaggtattgctgccttgccgttacaactctcctcatgaagatgagtctgaagaccgaatctact
ggcaaaaacatgacaaagtggtgctgtctgtcattgTtgggaaactaaaagtgtggcccgagtataagaaccggactttatatga
caacactacctactctcttatcatcctgggcctggtcctttcagaccggggcacatacagctgtgtcgttcaaaagaaggaaagag
gaacgtatgaagttaaacacttggctttagtaaagttgtccatcaaagctgacttctctacccccaacataactgagtctggaaaccc
atctgcagacactaaaaggattacctgctttgcttccgggggtttcccaaagcctcgcttctcttggttggaaaatggaagagaatta
cctggcatcaatacgacaatttcccaggatcctgaatctgaattgtacaccattagtagccaactagatttcaatacgactcgcaac
cacaccattaagtgtctcattaaatatggagatgctcacgtgtcagaggacttcacctgggaaaaacccccagaagaccctcctg
 
atagcaagaacacacttgtgctctttggggcaggattcggcgcagtaataacagtcgtcgtcatcgttgtcatcatcaaatgcttctg
taagcacagaagctgtttcagaagaaatgaggcaagcagagaaacaaacaacagccttaccttcgggcctgaagaagcattag
ctgaacagaccgtcttccttcgtacgGGCAGTGGAGAGGGCAGAGGAAGTCTGCTAACATGC
GGTGACGTCGAGGAGAATCCTGGCCCAatggagagtgtggtacagccttcagtgtttgtggtggatgga
cagacggacatcccattcaggcggctggaacagaaccaccggagacggcgctgtggcactgtccaggtcagcctggccctgg
tgctgctgctaggtgctgggctggccactcagggctggtttctcctgagactgcatcaacgtcttggagacatagtagctcatctgc
cagatggaggcaaaggctcctgggagaagctgatacaagatcaacgatctcaccaggccaacccagcagcacatcttacagg
agccaacgccagcttgataggtattggtggacctctgttatgggagacacgacttggcctggccttcttgaggggcttgacgtatc
atgatggggccctggtgaccatggagcccggttactactatgtgtactccaaagtgcagctgagcggcgtgggctgcccccagg
ggctggccaatggcctccccatcacccatggactatacaagcgcacatcccgctacccgaaggagttagaactgctggtcagtc
ggcggtcaccctgtggccgggccaacagctcccgagtctggtgggacagcagcttcctgggcggcgtggtacatctggaggc
tggggaagaggtggtggtccgcgtgcctggaaaccgcctggtcagaccacgtgacggcaccaggtcctatttcggagctttcat
ggtcactagcagcggtacccagtgcaccaactacgccctgctgaagctggccggcgatgtggagagcaaccccgggcccatg
gaccagcacacacttgatgtggaggataccgcggatgccagacatccagcaggtacttcgtgcccctcggatgcggcgctcct
cagagataccgggctcctcgcggacgctgcgctcctctcagatactgtgcgccccacaaatgccgcgctccccacggatgctg
cctaccctgcggttaatgttcgggatcgcgaggccgcgtggccgcctgcactgaacttctgttcccgccacccaaagctctatgg
cctagtcgctttggttttgctgcttctgatcgccgcctgtgttcctatcttcacccgcaccgagcctcggccagcgctcacaatcacc
acctcgcccaacctgggtacccgagagaataatgcagaccaggtcacccctgtttcccacattggctgccccaacactacacaa
cagggctctcctgtgttcgccaagctactggctaaaaaccaagcatcgttgtgcaatacaactctgaactggcacagccaagatg
gagctgggagctcatacctatctcaaggtctgaggtacgaagaagacaaaaaggagttggtggtagacagtcccgggctctact
acgtatttttggaactgaagctcagtccaacattcacaaacacaggccacaaggtgcagggctgggtctctcttgttttgcaagcaa
agcctcaggtagatgactttgacaacttggccctgacagtggaactgttcccttgctccatggagaacaagttagtggaccgttcct
ggagtcaactgttgctcctgaaggctggccaccgcctcagtgtgggtctgagggcttatctgcatggagcccaggatgcataca
gagactgggagctgtcttatcccaacaccaccagctttggactctttcttgtgaaacccgacaacccatgggaatgaGAATT
CAATAAAAGATCTTTATTTTCATTAGATCTGTGTGTTGGTTTTTTGTGTGCGGC
CGCAGGAACCCCTAGTGATGGAGTTGGCCACTCCCTCTCTGCGCGCTCGCTCG
CTCACTGAGGCCGGGCGACCAAAGGTCGCCCGACGCCCGGGCTTTGCCCGGG
CGGCCTCAGTGAGCGAGCGAGCGCGCAGCTGCCTGCAGGGGCGCCTGATGCG
GTATTTTCTCCTTACGCATCTGTGCGGTATTTCACACCGCATACGTCAAAGCA
ACCATAGTACGCGCCCTGTAGCGGCGCATTAAGCGCGGCGGGTGTGGTGGTT
ACGCGCAGCGTGACCGCTACACTTGCCAGCGCCTTAGCGCCCGCTCCTTTCGC
TTTCTTCCCTTCCTTTCTCGCCACGTTCGCCGGCTTTCCCCGTCAAGCTCTAAA
TCGGGGGCTCCCTTTAGGGTTCCGATTTAGTGCTTTACGGCACCTCGACCCCA
AAAAACTTGATTTGGGTGATGGTTCACGTAGTGGGCCATCGCCCTGATAGAC
GGTTTTTCGCCCTTTGACGTTGGAGTCCACGTTCTTTAATAGTGGACTCTTGTT
CCAAACTGGAACAACACTCAACTCTATCTCGGGCTATTCTTTTGATTTATAAG
GGATTTTGCCGATTTCGGTCTATTGGTTAAAAAATGAGCTGATTTAACAAAAA
TTTAACGCGAATTTTAACAAAATATTAACGTTTACAATTTTATGGTGCACTCT
CAGTACAATCTGCTCTGATGCCGCATAGTTAAGCCAGCCCCGACACCCGCCA
ACACCCGCTGACGCGCCCTGACGGGCTTGTCTGCTCCCGGCATCCGCTTACAG
ACAAGCTGTGACCGTCTCCGGGAGCTGCATGTGTCAGAGGTTTTCACCGTCAT
CACCGAAACGCGCGAGACGAAAGGGCCTCGTGATACGCCTATTTTTATAGGT
TAATGTCATGATAATAATGGTTTCTTAGACGTCAGGTGGCACTTTTCGGGGAA
ATGTGCGCGGAACCCCTATTTGTTTATTTTTCTAAATACATTCAAATATGTATC
CGCTCATGAGACAATAACCCTGATAAATGCTTCAATAATATTGAAAAAGGAA
GAGTATGAGTATTCAACATTTCCGTGTCGCCCTTATTCCCTTTTTTGCGGCATT
TTGCCTTCCTGTTTTTGCTCACCCAGAAACGCTGGTGAAAGTAAAAGATGCTG
AAGATCAGTTGGGTGCACGAGTGGGTTACATCGAACTGGATCTCAACAGCGG
TAAGATCCTTGAGAGTTTTCGCCCCGAAGAACGTTTTCCAATGATGAGCACTT
TTAAAGTTCTGCTATGTGGCGCGGTATTATCCCGTATTGACGCCGGGCAAGAG
 
CAACTCGGTCGCCGCATACACTATTCTCAGAATGACTTGGTTGAGTACTCACC
AGTCACAGAAAAGCATCTTACGGATGGCATGACAGTAAGAGAATTATGCAGT
GCTGCCATAACCATGAGTGATAACACTGCGGCCAACTTACTTCTGACAACGAT
CGGAGGACCGAAGGAGCTAACCGCTTTTTTGCACAACATGGGGGATCATGTA
ACTCGCCTTGATCGTTGGGAACCGGAGCTGAATGAAGCCATACCAAACGACG
AGCGTGACACCACGATGCCTGTAGCAATGGCAACAACGTTGCGCAAACTATT
AACTGGCGAACTACTTACTCTAGCTTCCCGGCAACAATTAATAGACTGGATGG
AGGCGGATAAAGTTGCAGGACCACTTCTGCGCTCGGCCCTTCCGGCTGGCTG
GTTTATTGCTGATAAATCTGGAGCCGGTGAGCGTGGAAGCCGCGGTATCATTG
CAGCACTGGGGCCAGATGGTAAGCCCTCCCGTATCGTAGTTATCTACACGAC
GGGGAGTCAGGCAACTATGGATGAACGAAATAGACAGATCGCTGAGATAGG
TGCCTCACTGATTAAGCATTGGTAACTGTCAGACCAAGTTTACTCATATATAC
TTTAGATTGATTTAAAACTTCATTTTTAATTTAAAAGGATCTAGGTGAAGATC
CTTTTTGATAATCTCATGACCAAAATCCCTTAACGTGAGTTTTCGTTCCACTGA
GCGTCAGACCCCGTAGAAAAGATCAAAGGATCTTCTTGAGATCCTTTTTTTCT
GCGCGTAATCTGCTGCTTGCAAACAAAAAAACCACCGCTACCAGCGGTGGTT
TGTTTGCCGGATCAAGAGCTACCAACTCTTTTTCCGAAGGTAACTGGCTTCAG
CAGAGCGCAGATACCAAATACTGTTCTTCTAGTGTAGCCGTAGTTAGGCCACC
ACTTCAAGAACTCTGTAGCACCGCCTACATACCTCGCTCTGCTAATCCTGTTA
CCAGTGGCTGCTGCCAGTGGCGATAAGTCGTGTCTTACCGGGTTGGACTCAAG
ACGATAGTTACCGGATAAGGCGCAGCGGTCGGGCTGAACGGGGGGTTCGTGC
ACACAGCCCAGCTTGGAGCGAACGACCTACACCGAACTGAGATACCTACAGC
GTGAGCTATGAGAAAGCGCCACGCTTCCCGAAGGGAGAAAGGCGGACAGGT
ATCCGGTAAGCGGCAGGGTCGGAACAGGAGAGCGCACGAGGGAGCTTCCAG
GGGGAAACGCCTGGTATCTTTATAGTCCTGTCGGGTTTCGCCACCTCTGACTT
GAGCGTCGATTTTTGTGATGCTCGTCAGGGGGGCGGAGCCTATGGAAAAACG
CCAGCAACGCGGCCTTTTTACGGTTCCTGGCCTTTTGCTGGCCTTTTGCTCACA
TGT
**pGW102-pAAV_EFSLTR-Cd80-sPA (SEQ ID NO: 36)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atggcttgcaattgtcagttgatgcaggatacaccactcctcaagtttccatgtccaaggctcattcttctctttgtgctgctgattcgtc
tttcacaagtgtcttcagatgttgatgaacaactgtccaagtcagtgaaagataaggtattgctgccttgccgttacaactctcctcat
gaagatgagtctgaagaccgaatctactggcaaaaacatgacaaagtggtgctgtctgtcattgTtgggaaactaaaagtgtggc
ccgagtataagaaccggactttatatgacaacactacctactctcttatcatcctgggcctggtcctttcagaccggggcacataca
gctgtgtcgttcaaaagaaggaaagaggaacgtatgaagttaaacacttggctttagtaaagttgtccatcaaagctgacttctcta
cccccaacataactgagtctggaaacccatctgcagacactaaaaggattacctgctttgcttccgggggtttcccaaagcctcgc
ttctcttggttggaaaatggaagagaattacctggcatcaatacgacaatttcccaggatcctgaatctgaattgtacaccattagta
 
gccaactagatttcaatacgactcgcaaccacaccattaagtgtctcattaaatatggagatgctcacgtgtcagaggacttcacct
gggaaaaacccccagaagaccctcctgatagcaagaacacacttgtgctctttggggcaggattcggcgcagtaataacagtcg
tcgtcatcgttgtcatcatcaaatgcttctgtaagcacagaagctgtttcagaagaaatgaggcaagcagagaaacaaacaacag
ccttaccttcgggcctgaagaagcattagctgaacagaccgtcttccttcgttagGAATTCAATAAAAGATCTT
TATTTTCATTAGATCTGTGTGTTGGTTTTTTGTGTGCGGCCGCAGGAACCCCTA
GTGATGGAGTTGGCCACTCCCTCTCTGCGCGCTCGCTCGCTCACTGAGGCCGG
GCGACCAAAGGTCGCCCGACGCCCGGGCTTTGCCCGGGCGGCCTCAGTGAGC
GAGCGAGCGCGCAGCTGCCTGCAGGGGCGCCTGATGCGGTATTTTCTCCTTAC
GCATCTGTGCGGTATTTCACACCGCATACGTCAAAGCAACCATAGTACGCGCC
CTGTAGCGGCGCATTAAGCGCGGCGGGTGTGGTGGTTACGCGCAGCGTGACC
GCTACACTTGCCAGCGCCTTAGCGCCCGCTCCTTTCGCTTTCTTCCCTTCCTTT
CTCGCCACGTTCGCCGGCTTTCCCCGTCAAGCTCTAAATCGGGGGCTCCCTTT
AGGGTTCCGATTTAGTGCTTTACGGCACCTCGACCCCAAAAAACTTGATTTGG
GTGATGGTTCACGTAGTGGGCCATCGCCCTGATAGACGGTTTTTCGCCCTTTG
ACGTTGGAGTCCACGTTCTTTAATAGTGGACTCTTGTTCCAAACTGGAACAAC
ACTCAACTCTATCTCGGGCTATTCTTTTGATTTATAAGGGATTTTGCCGATTTC
GGTCTATTGGTTAAAAAATGAGCTGATTTAACAAAAATTTAACGCGAATTTTA
ACAAAATATTAACGTTTACAATTTTATGGTGCACTCTCAGTACAATCTGCTCT
GATGCCGCATAGTTAAGCCAGCCCCGACACCCGCCAACACCCGCTGACGCGC
CCTGACGGGCTTGTCTGCTCCCGGCATCCGCTTACAGACAAGCTGTGACCGTC
TCCGGGAGCTGCATGTGTCAGAGGTTTTCACCGTCATCACCGAAACGCGCGA
GACGAAAGGGCCTCGTGATACGCCTATTTTTATAGGTTAATGTCATGATAATA
ATGGTTTCTTAGACGTCAGGTGGCACTTTTCGGGGAAATGTGCGCGGAACCCC
TATTTGTTTATTTTTCTAAATACATTCAAATATGTATCCGCTCATGAGACAATA
ACCCTGATAAATGCTTCAATAATATTGAAAAAGGAAGAGTATGAGTATTCAA
CATTTCCGTGTCGCCCTTATTCCCTTTTTTGCGGCATTTTGCCTTCCTGTTTTTG
CTCACCCAGAAACGCTGGTGAAAGTAAAAGATGCTGAAGATCAGTTGGGTGC
ACGAGTGGGTTACATCGAACTGGATCTCAACAGCGGTAAGATCCTTGAGAGT
TTTCGCCCCGAAGAACGTTTTCCAATGATGAGCACTTTTAAAGTTCTGCTATG
TGGCGCGGTATTATCCCGTATTGACGCCGGGCAAGAGCAACTCGGTCGCCGC
ATACACTATTCTCAGAATGACTTGGTTGAGTACTCACCAGTCACAGAAAAGC
ATCTTACGGATGGCATGACAGTAAGAGAATTATGCAGTGCTGCCATAACCAT
GAGTGATAACACTGCGGCCAACTTACTTCTGACAACGATCGGAGGACCGAAG
GAGCTAACCGCTTTTTTGCACAACATGGGGGATCATGTAACTCGCCTTGATCG
TTGGGAACCGGAGCTGAATGAAGCCATACCAAACGACGAGCGTGACACCACG
ATGCCTGTAGCAATGGCAACAACGTTGCGCAAACTATTAACTGGCGAACTAC
TTACTCTAGCTTCCCGGCAACAATTAATAGACTGGATGGAGGCGGATAAAGT
TGCAGGACCACTTCTGCGCTCGGCCCTTCCGGCTGGCTGGTTTATTGCTGATA
AATCTGGAGCCGGTGAGCGTGGAAGCCGCGGTATCATTGCAGCACTGGGGCC
AGATGGTAAGCCCTCCCGTATCGTAGTTATCTACACGACGGGGAGTCAGGCA
ACTATGGATGAACGAAATAGACAGATCGCTGAGATAGGTGCCTCACTGATTA
AGCATTGGTAACTGTCAGACCAAGTTTACTCATATATACTTTAGATTGATTTA
AAACTTCATTTTTAATTTAAAAGGATCTAGGTGAAGATCCTTTTTGATAATCT
CATGACCAAAATCCCTTAACGTGAGTTTTCGTTCCACTGAGCGTCAGACCCCG
TAGAAAAGATCAAAGGATCTTCTTGAGATCCTTTTTTTCTGCGCGTAATCTGC
TGCTTGCAAACAAAAAAACCACCGCTACCAGCGGTGGTTTGTTTGCCGGATC
AAGAGCTACCAACTCTTTTTCCGAAGGTAACTGGCTTCAGCAGAGCGCAGAT
ACCAAATACTGTTCTTCTAGTGTAGCCGTAGTTAGGCCACCACTTCAAGAACT
CTGTAGCACCGCCTACATACCTCGCTCTGCTAATCCTGTTACCAGTGGCTGCT
GCCAGTGGCGATAAGTCGTGTCTTACCGGGTTGGACTCAAGACGATAGTTAC
 
CGGATAAGGCGCAGCGGTCGGGCTGAACGGGGGGTTCGTGCACACAGCCCAG
CTTGGAGCGAACGACCTACACCGAACTGAGATACCTACAGCGTGAGCTATGA
GAAAGCGCCACGCTTCCCGAAGGGAGAAAGGCGGACAGGTATCCGGTAAGC
GGCAGGGTCGGAACAGGAGAGCGCACGAGGGAGCTTCCAGGGGGAAACGCC
TGGTATCTTTATAGTCCTGTCGGGTTTCGCCACCTCTGACTTGAGCGTCGATTT
TTGTGATGCTCGTCAGGGGGGCGGAGCCTATGGAAAAACGCCAGCAACGCGG
CCTTTTTACGGTTCCTGGCCTTTTGCTGGCCTTTTGCTCACATGT
**pGW103-pAAV_EFSLTR-Light-sPA (SEQ ID NO: 37)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atggagagtgtggtacagccttcagtgtttgtggtggatggacagacggacatcccattcaggcggctggaacagaaccaccgg
agacggcgctgtggcactgtccaggtcagcctggccctggtgctgctgctaggtgctgggctggccactcagggctggtttctc
ctgagactgcatcaacgtcttggagacatagtagctcatctgccagatggaggcaaaggctcctgggagaagctgatacaagat
caacgatctcaccaggccaacccagcagcacatcttacaggagccaacgccagcttgataggtattggtggacctctgttatggg
agacacgacttggcctggccttcttgaggggcttgacgtatcatgatggggccctggtgaccatggagcccggttactactatgtg
tactccaaagtgcagctgagcggcgtgggctgcccccaggggctggccaatggcctccccatcacccatggactatacaagcg
cacatcccgctacccgaaggagttagaactgctggtcagtcggcggtcaccctgtggccgggccaacagctcccgagtctggt
gggacagcagcttcctgggcggcgtggtacatctggaggctggggaagaggtggtggtccgcgtgcctggaaaccgcctggt
cagaccacgtgacggcaccaggtcctatttcggagctttcatggtctgaGAATTCAATAAAAGATCTTTATT
TTCATTAGATCTGTGTGTTGGTTTTTTGTGTGCGGCCGCAGGAACCCCTAGTG
ATGGAGTTGGCCACTCCCTCTCTGCGCGCTCGCTCGCTCACTGAGGCCGGGCG
ACCAAAGGTCGCCCGACGCCCGGGCTTTGCCCGGGCGGCCTCAGTGAGCGAG
CGAGCGCGCAGCTGCCTGCAGGGGCGCCTGATGCGGTATTTTCTCCTTACGCA
TCTGTGCGGTATTTCACACCGCATACGTCAAAGCAACCATAGTACGCGCCCTG
TAGCGGCGCATTAAGCGCGGCGGGTGTGGTGGTTACGCGCAGCGTGACCGCT
ACACTTGCCAGCGCCTTAGCGCCCGCTCCTTTCGCTTTCTTCCCTTCCTTTCTC
GCCACGTTCGCCGGCTTTCCCCGTCAAGCTCTAAATCGGGGGCTCCCTTTAGG
GTTCCGATTTAGTGCTTTACGGCACCTCGACCCCAAAAAACTTGATTTGGGTG
ATGGTTCACGTAGTGGGCCATCGCCCTGATAGACGGTTTTTCGCCCTTTGACG
TTGGAGTCCACGTTCTTTAATAGTGGACTCTTGTTCCAAACTGGAACAACACT
CAACTCTATCTCGGGCTATTCTTTTGATTTATAAGGGATTTTGCCGATTTCGGT
CTATTGGTTAAAAAATGAGCTGATTTAACAAAAATTTAACGCGAATTTTAACA
AAATATTAACGTTTACAATTTTATGGTGCACTCTCAGTACAATCTGCTCTGAT
GCCGCATAGTTAAGCCAGCCCCGACACCCGCCAACACCCGCTGACGCGCCCT
GACGGGCTTGTCTGCTCCCGGCATCCGCTTACAGACAAGCTGTGACCGTCTCC
GGGAGCTGCATGTGTCAGAGGTTTTCACCGTCATCACCGAAACGCGCGAGAC
GAAAGGGCCTCGTGATACGCCTATTTTTATAGGTTAATGTCATGATAATAATG
GTTTCTTAGACGTCAGGTGGCACTTTTCGGGGAAATGTGCGCGGAACCCCTAT
 
TTGTTTATTTTTCTAAATACATTCAAATATGTATCCGCTCATGAGACAATAACC
CTGATAAATGCTTCAATAATATTGAAAAAGGAAGAGTATGAGTATTCAACAT
TTCCGTGTCGCCCTTATTCCCTTTTTTGCGGCATTTTGCCTTCCTGTTTTTGCTC
ACCCAGAAACGCTGGTGAAAGTAAAAGATGCTGAAGATCAGTTGGGTGCACG
AGTGGGTTACATCGAACTGGATCTCAACAGCGGTAAGATCCTTGAGAGTTTTC
GCCCCGAAGAACGTTTTCCAATGATGAGCACTTTTAAAGTTCTGCTATGTGGC
GCGGTATTATCCCGTATTGACGCCGGGCAAGAGCAACTCGGTCGCCGCATAC
ACTATTCTCAGAATGACTTGGTTGAGTACTCACCAGTCACAGAAAAGCATCTT
ACGGATGGCATGACAGTAAGAGAATTATGCAGTGCTGCCATAACCATGAGTG
ATAACACTGCGGCCAACTTACTTCTGACAACGATCGGAGGACCGAAGGAGCT
AACCGCTTTTTTGCACAACATGGGGGATCATGTAACTCGCCTTGATCGTTGGG
AACCGGAGCTGAATGAAGCCATACCAAACGACGAGCGTGACACCACGATGC
CTGTAGCAATGGCAACAACGTTGCGCAAACTATTAACTGGCGAACTACTTACT
CTAGCTTCCCGGCAACAATTAATAGACTGGATGGAGGCGGATAAAGTTGCAG
GACCACTTCTGCGCTCGGCCCTTCCGGCTGGCTGGTTTATTGCTGATAAATCT
GGAGCCGGTGAGCGTGGAAGCCGCGGTATCATTGCAGCACTGGGGCCAGATG
GTAAGCCCTCCCGTATCGTAGTTATCTACACGACGGGGAGTCAGGCAACTAT
GGATGAACGAAATAGACAGATCGCTGAGATAGGTGCCTCACTGATTAAGCAT
TGGTAACTGTCAGACCAAGTTTACTCATATATACTTTAGATTGATTTAAAACT
TCATTTTTAATTTAAAAGGATCTAGGTGAAGATCCTTTTTGATAATCTCATGA
CCAAAATCCCTTAACGTGAGTTTTCGTTCCACTGAGCGTCAGACCCCGTAGAA
AAGATCAAAGGATCTTCTTGAGATCCTTTTTTTCTGCGCGTAATCTGCTGCTTG
CAAACAAAAAAACCACCGCTACCAGCGGTGGTTTGTTTGCCGGATCAAGAGC
TACCAACTCTTTTTCCGAAGGTAACTGGCTTCAGCAGAGCGCAGATACCAAAT
ACTGTTCTTCTAGTGTAGCCGTAGTTAGGCCACCACTTCAAGAACTCTGTAGC
ACCGCCTACATACCTCGCTCTGCTAATCCTGTTACCAGTGGCTGCTGCCAGTG
GCGATAAGTCGTGTCTTACCGGGTTGGACTCAAGACGATAGTTACCGGATAA
GGCGCAGCGGTCGGGCTGAACGGGGGGTTCGTGCACACAGCCCAGCTTGGAG
CGAACGACCTACACCGAACTGAGATACCTACAGCGTGAGCTATGAGAAAGCG
CCACGCTTCCCGAAGGGAGAAAGGCGGACAGGTATCCGGTAAGCGGCAGGG
TCGGAACAGGAGAGCGCACGAGGGAGCTTCCAGGGGGAAACGCCTGGTATCT
TTATAGTCCTGTCGGGTTTCGCCACCTCTGACTTGAGCGTCGATTTTTGTGATG
CTCGTCAGGGGGGCGGAGCCTATGGAAAAACGCCAGCAACGCGGCCTTTTTA
CGGTTCCTGGCCTTTTGCTGGCCTTTTGCTCACATGT
**pGW104-pAAV_EFSLTR-Cxcl10-sPA (SEQ ID NO: 38)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atgaacccaagtgctgccgtcattttctgcctcatcctgctgggtctgagtgggactcaagggatccctctcgcaaggacggtccg
 
ctgcaactgcatccatatcgatgacgggccagtgagaatgagggccatagggaagcttgaaatcatccctgcgagcctatcctg
cccacgtgttgagatcattgccacgatgaaaaagaatgatgagcagagatgtctgaatccggaatctaagaccatcaagaatttaa
tgaaagcgtttagccaaaaaaggtctaaaagggctccttgaGAATTCAATAAAAGATCTTTATTTTCAT
TAGATCTGTGTGTTGGTTTTTTGTGTGCGGCCGCAGGAACCCCTAGTGATGGA
GTTGGCCACTCCCTCTCTGCGCGCTCGCTCGCTCACTGAGGCCGGGCGACCAA
AGGTCGCCCGACGCCCGGGCTTTGCCCGGGCGGCCTCAGTGAGCGAGCGAGC
GCGCAGCTGCCTGCAGGGGCGCCTGATGCGGTATTTTCTCCTTACGCATCTGT
GCGGTATTTCACACCGCATACGTCAAAGCAACCATAGTACGCGCCCTGTAGC
GGCGCATTAAGCGCGGCGGGTGTGGTGGTTACGCGCAGCGTGACCGCTACAC
TTGCCAGCGCCTTAGCGCCCGCTCCTTTCGCTTTCTTCCCTTCCTTTCTCGCCA
CGTTCGCCGGCTTTCCCCGTCAAGCTCTAAATCGGGGGCTCCCTTTAGGGTTC
CGATTTAGTGCTTTACGGCACCTCGACCCCAAAAAACTTGATTTGGGTGATGG
TTCACGTAGTGGGCCATCGCCCTGATAGACGGTTTTTCGCCCTTTGACGTTGG
AGTCCACGTTCTTTAATAGTGGACTCTTGTTCCAAACTGGAACAACACTCAAC
TCTATCTCGGGCTATTCTTTTGATTTATAAGGGATTTTGCCGATTTCGGTCTAT
TGGTTAAAAAATGAGCTGATTTAACAAAAATTTAACGCGAATTTTAACAAAA
TATTAACGTTTACAATTTTATGGTGCACTCTCAGTACAATCTGCTCTGATGCCG
CATAGTTAAGCCAGCCCCGACACCCGCCAACACCCGCTGACGCGCCCTGACG
GGCTTGTCTGCTCCCGGCATCCGCTTACAGACAAGCTGTGACCGTCTCCGGGA
GCTGCATGTGTCAGAGGTTTTCACCGTCATCACCGAAACGCGCGAGACGAAA
GGGCCTCGTGATACGCCTATTTTTATAGGTTAATGTCATGATAATAATGGTTT
CTTAGACGTCAGGTGGCACTTTTCGGGGAAATGTGCGCGGAACCCCTATTTGT
TTATTTTTCTAAATACATTCAAATATGTATCCGCTCATGAGACAATAACCCTG
ATAAATGCTTCAATAATATTGAAAAAGGAAGAGTATGAGTATTCAACATTTC
CGTGTCGCCCTTATTCCCTTTTTTGCGGCATTTTGCCTTCCTGTTTTTGCTCACC
CAGAAACGCTGGTGAAAGTAAAAGATGCTGAAGATCAGTTGGGTGCACGAGT
GGGTTACATCGAACTGGATCTCAACAGCGGTAAGATCCTTGAGAGTTTTCGCC
CCGAAGAACGTTTTCCAATGATGAGCACTTTTAAAGTTCTGCTATGTGGCGCG
GTATTATCCCGTATTGACGCCGGGCAAGAGCAACTCGGTCGCCGCATACACT
ATTCTCAGAATGACTTGGTTGAGTACTCACCAGTCACAGAAAAGCATCTTACG
GATGGCATGACAGTAAGAGAATTATGCAGTGCTGCCATAACCATGAGTGATA
ACACTGCGGCCAACTTACTTCTGACAACGATCGGAGGACCGAAGGAGCTAAC
CGCTTTTTTGCACAACATGGGGGATCATGTAACTCGCCTTGATCGTTGGGAAC
CGGAGCTGAATGAAGCCATACCAAACGACGAGCGTGACACCACGATGCCTGT
AGCAATGGCAACAACGTTGCGCAAACTATTAACTGGCGAACTACTTACTCTA
GCTTCCCGGCAACAATTAATAGACTGGATGGAGGCGGATAAAGTTGCAGGAC
CACTTCTGCGCTCGGCCCTTCCGGCTGGCTGGTTTATTGCTGATAAATCTGGA
GCCGGTGAGCGTGGAAGCCGCGGTATCATTGCAGCACTGGGGCCAGATGGTA
AGCCCTCCCGTATCGTAGTTATCTACACGACGGGGAGTCAGGCAACTATGGA
TGAACGAAATAGACAGATCGCTGAGATAGGTGCCTCACTGATTAAGCATTGG
TAACTGTCAGACCAAGTTTACTCATATATACTTTAGATTGATTTAAAACTTCA
TTTTTAATTTAAAAGGATCTAGGTGAAGATCCTTTTTGATAATCTCATGACCA
AAATCCCTTAACGTGAGTTTTCGTTCCACTGAGCGTCAGACCCCGTAGAAAAG
ATCAAAGGATCTTCTTGAGATCCTTTTTTTCTGCGCGTAATCTGCTGCTTGCAA
ACAAAAAAACCACCGCTACCAGCGGTGGTTTGTTTGCCGGATCAAGAGCTAC
CAACTCTTTTTCCGAAGGTAACTGGCTTCAGCAGAGCGCAGATACCAAATACT
GTTCTTCTAGTGTAGCCGTAGTTAGGCCACCACTTCAAGAACTCTGTAGCACC
GCCTACATACCTCGCTCTGCTAATCCTGTTACCAGTGGCTGCTGCCAGTGGCG
ATAAGTCGTGTCTTACCGGGTTGGACTCAAGACGATAGTTACCGGATAAGGC
GCAGCGGTCGGGCTGAACGGGGGGTTCGTGCACACAGCCCAGCTTGGAGCGA
 
ACGACCTACACCGAACTGAGATACCTACAGCGTGAGCTATGAGAAAGCGCCA
CGCTTCCCGAAGGGAGAAAGGCGGACAGGTATCCGGTAAGCGGCAGGGTCG
GAACAGGAGAGCGCACGAGGGAGCTTCCAGGGGGAAACGCCTGGTATCTTTA
TAGTCCTGTCGGGTTTCGCCACCTCTGACTTGAGCGTCGATTTTTGTGATGCTC
GTCAGGGGGGCGGAGCCTATGGAAAAACGCCAGCAACGCGGCCTTTTTACGG
TTCCTGGCCTTTTGCTGGCCTTTTGCTCACATGT
**pGW105-pAAV_EFSLTR-41bbL-sPA (SEQ ID NO: 39)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atggaccagcacacacttgatgtggaggataccgcggatgccagacatccagcaggtacttcgtgcccctcggatgcggcgct
cctcagagataccgggctcctcgcggacgctgcgctcctctcagatactgtgcgccccacaaatgccgcgctccccacggatg
ctgcctaccctgcggttaatgttcgggatcgcgaggccgcgtggccgcctgcactgaacttctgttcccgccacccaaagctctat
ggcctagtcgctttggttttgctgcttctgatcgccgcctgtgttcctatcttcacccgcaccgagcctcggccagcgctcacaatca
ccacctcgcccaacctgggtacccgagagaataatgcagaccaggtcacccctgtttcccacattggctgccccaacactacac
aacagggctctcctgtgttcgccaagctactggctaaaaaccaagcatcgttgtgcaatacaactctgaactggcacagccaaga
tggagctgggagctcatacctatctcaaggtctgaggtacgaagaagacaaaaaggagttggtggtagacagtcccgggctcta
ctacgtatttttggaactgaagctcagtccaacattcacaaacacaggccacaaggtgcagggctgggtctctcttgttttgcaagc
aaagcctcaggtagatgactttgacaacttggccctgacagtggaactgttcccttgctccatggagaacaagttagtggaccgtt
cctggagtcaactgttgctcctgaaggctggccaccgcctcagtgtgggtctgagggcttatctgcatggagcccaggatgcata
cagagactgggagctgtcttatcccaacaccaccagctttggactctttcttgtgaaacccgacaacccatgggaatgaGAAT
TCAATAAAAGATCTTTATTTTCATTAGATCTGTGTGTTGGTTTTTTGTGTGCGG
CCGCAGGAACCCCTAGTGATGGAGTTGGCCACTCCCTCTCTGCGCGCTCGCTC
GCTCACTGAGGCCGGGCGACCAAAGGTCGCCCGACGCCCGGGCTTTGCCCGG
GCGGCCTCAGTGAGCGAGCGAGCGCGCAGCTGCCTGCAGGGGCGCCTGATGC
GGTATTTTCTCCTTACGCATCTGTGCGGTATTTCACACCGCATACGTCAAAGC
AACCATAGTACGCGCCCTGTAGCGGCGCATTAAGCGCGGCGGGTGTGGTGGT
TACGCGCAGCGTGACCGCTACACTTGCCAGCGCCTTAGCGCCCGCTCCTTTCG
CTTTCTTCCCTTCCTTTCTCGCCACGTTCGCCGGCTTTCCCCGTCAAGCTCTAA
ATCGGGGGCTCCCTTTAGGGTTCCGATTTAGTGCTTTACGGCACCTCGACCCC
AAAAAACTTGATTTGGGTGATGGTTCACGTAGTGGGCCATCGCCCTGATAGA
CGGTTTTTCGCCCTTTGACGTTGGAGTCCACGTTCTTTAATAGTGGACTCTTGT
TCCAAACTGGAACAACACTCAACTCTATCTCGGGCTATTCTTTTGATTTATAA
GGGATTTTGCCGATTTCGGTCTATTGGTTAAAAAATGAGCTGATTTAACAAAA
ATTTAACGCGAATTTTAACAAAATATTAACGTTTACAATTTTATGGTGCACTC
TCAGTACAATCTGCTCTGATGCCGCATAGTTAAGCCAGCCCCGACACCCGCCA
ACACCCGCTGACGCGCCCTGACGGGCTTGTCTGCTCCCGGCATCCGCTTACAG
ACAAGCTGTGACCGTCTCCGGGAGCTGCATGTGTCAGAGGTTTTCACCGTCAT
CACCGAAACGCGCGAGACGAAAGGGCCTCGTGATACGCCTATTTTTATAGGT
 
TAATGTCATGATAATAATGGTTTCTTAGACGTCAGGTGGCACTTTTCGGGGAA
ATGTGCGCGGAACCCCTATTTGTTTATTTTTCTAAATACATTCAAATATGTATC
CGCTCATGAGACAATAACCCTGATAAATGCTTCAATAATATTGAAAAAGGAA
GAGTATGAGTATTCAACATTTCCGTGTCGCCCTTATTCCCTTTTTTGCGGCATT
TTGCCTTCCTGTTTTTGCTCACCCAGAAACGCTGGTGAAAGTAAAAGATGCTG
AAGATCAGTTGGGTGCACGAGTGGGTTACATCGAACTGGATCTCAACAGCGG
TAAGATCCTTGAGAGTTTTCGCCCCGAAGAACGTTTTCCAATGATGAGCACTT
TTAAAGTTCTGCTATGTGGCGCGGTATTATCCCGTATTGACGCCGGGCAAGAG
CAACTCGGTCGCCGCATACACTATTCTCAGAATGACTTGGTTGAGTACTCACC
AGTCACAGAAAAGCATCTTACGGATGGCATGACAGTAAGAGAATTATGCAGT
GCTGCCATAACCATGAGTGATAACACTGCGGCCAACTTACTTCTGACAACGAT
CGGAGGACCGAAGGAGCTAACCGCTTTTTTGCACAACATGGGGGATCATGTA
ACTCGCCTTGATCGTTGGGAACCGGAGCTGAATGAAGCCATACCAAACGACG
AGCGTGACACCACGATGCCTGTAGCAATGGCAACAACGTTGCGCAAACTATT
AACTGGCGAACTACTTACTCTAGCTTCCCGGCAACAATTAATAGACTGGATGG
AGGCGGATAAAGTTGCAGGACCACTTCTGCGCTCGGCCCTTCCGGCTGGCTG
GTTTATTGCTGATAAATCTGGAGCCGGTGAGCGTGGAAGCCGCGGTATCATTG
CAGCACTGGGGCCAGATGGTAAGCCCTCCCGTATCGTAGTTATCTACACGAC
GGGGAGTCAGGCAACTATGGATGAACGAAATAGACAGATCGCTGAGATAGG
TGCCTCACTGATTAAGCATTGGTAACTGTCAGACCAAGTTTACTCATATATAC
TTTAGATTGATTTAAAACTTCATTTTTAATTTAAAAGGATCTAGGTGAAGATC
CTTTTTGATAATCTCATGACCAAAATCCCTTAACGTGAGTTTTCGTTCCACTGA
GCGTCAGACCCCGTAGAAAAGATCAAAGGATCTTCTTGAGATCCTTTTTTTCT
GCGCGTAATCTGCTGCTTGCAAACAAAAAAACCACCGCTACCAGCGGTGGTT
TGTTTGCCGGATCAAGAGCTACCAACTCTTTTTCCGAAGGTAACTGGCTTCAG
CAGAGCGCAGATACCAAATACTGTTCTTCTAGTGTAGCCGTAGTTAGGCCACC
ACTTCAAGAACTCTGTAGCACCGCCTACATACCTCGCTCTGCTAATCCTGTTA
CCAGTGGCTGCTGCCAGTGGCGATAAGTCGTGTCTTACCGGGTTGGACTCAAG
ACGATAGTTACCGGATAAGGCGCAGCGGTCGGGCTGAACGGGGGGTTCGTGC
ACACAGCCCAGCTTGGAGCGAACGACCTACACCGAACTGAGATACCTACAGC
GTGAGCTATGAGAAAGCGCCACGCTTCCCGAAGGGAGAAAGGCGGACAGGT
ATCCGGTAAGCGGCAGGGTCGGAACAGGAGAGCGCACGAGGGAGCTTCCAG
GGGGAAACGCCTGGTATCTTTATAGTCCTGTCGGGTTTCGCCACCTCTGACTT
GAGCGTCGATTTTTGTGATGCTCGTCAGGGGGGCGGAGCCTATGGAAAAACG
CCAGCAACGCGGCCTTTTTACGGTTCCTGGCCTTTTGCTGGCCTTTTGCTCACA
TGT
**pGW110-pAAV_EFS-LTR-Cd80-T2A-Light-P2A-Cxcl10-E2A-IFNg-WPRE (SEQ ID NO: 40)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
 
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atggcttgcaattgtcagttgatgcaggatacaccactcctcaagtttccatgtccaaggctcattcttctctttgtgctgctgattcgtc
tttcacaagtgtcttcagatgttgatgaacaactgtccaagtcagtgaaagataaggtattgctgccttgccgttacaactctcctcat
gaagatgagtctgaagaccgaatctactggcaaaaacatgacaaagtggtgctgtctgtcattgctgggaaactaaaagtgtggc
ccgagtataagaaccggactttatatgacaacactacctactctcttatcatcctgggcctggtcctttcagaccggggcacataca
gctgtgtcgttcaaaagaaggaaagaggaacgtatgaagttaaacacttggctttagtaaagttgtccatcaaagctgacttctcta
cccccaacataactgagtctggaaacccatctgcagacactaaaaggattacctgctttgcttccgggggtttcccaaagcctcgc
ttctcttggttggaaaatggaagagaattacctggcatcaatacgacaatttcccaggatcctgaatctgaattgtacaccattagta
gccaactagatttcaatacgactcgcaaccacaccattaagtgtctcattaaatatggagatgctcacgtgtcagaggacttcacct
gggaaaaacccccagaagaccctcctgatagcaagaacacacttgtgctctttggggcaggattcggcgcagtaataacagtcg
tcgtcatcgttgtcatcatcaaatgcttctgtaagcacagaagctgtttcagaagaaatgaggcaagcagagaaacaaacaacag
ccttaccttcgggcctgaagaagcattagctgaacagaccgtcttccttcgtacgGGCAGTGGAGAGGGCAGA
GGAAGTCTGCTAACATGCGGTGACGTCGAGGAGAATCCTGGCCCAatggagagtgt
ggtacagccttcagtgtttgtggtggatggacagacggacatcccattcaggcggctggaacagaaccaccggagacggcgct
gtggcactgtccaggtcagcctggccctggtgctgctgctaAgtgctgggctggccactcagggctggtttctcctgagactgc
atcaacgtcttggagacatagtagctcatctgccagatggaggcaaaggctcctgggagaagctgatacaagatcaacgatctc
accaggccaacccagcagcacatcttacaggagccaacgccagcttgataggtattggtggacctctgttatgggagacacgac
ttggcctggccttcttgaggggcttgacgtatcatgatggggccctggtgaccatggagcccggttactactatgtgtactccaaa
gtgcagctgagcggcgtgggctgcccccaggggctggccaatggcctccccatcacccatggactatacaagcgcacatccc
gctacccgaaggagttagaactgctggtcagtcggcggtcaccctgtggccgggccaacagctcccgagtctggtgggacag
cagcttcctgggcggcgtggtacatctggaggctggggaagaggtggtggtccgcgtgcctggaaaccgcctggtcagacca
cgtgacggcaccaggtcctatttcggagctttcatggtcactagtGGATCCGGCGCAACAAACTTCTCTCT
GCTGAAACAAGCCGGAGATGTCGAAGAGAATCCTGGACCGatgaacccaagtgctgccg
tcattttctgcctcatcctgctgggtctgagtgggactcaagggatccctctcgcaaggacggtccgctgcaactgcatccatatcg
atgacgggccagtgagaatgagggccatagggaagcttgaaatcatccctgcgagcctatcctgcccacgtgttgagatcattg
ccacgatgaaaaagaatgatgagcagagatgtctgaatccggaatctaagaccatcaagaatttaatgaaagcgtttagccaaaa
aaggtctaaaagggctcctagcggtacccagtgcaccaactacgccctgctgaagctggccggcgatgtggagagcaacccc
gggcccgctagcatgaacgctacacactgcatcttggctttgcagctcttcctcatggctgtttctggctgttactgccacggcaca
gtcattgaaagcctagaaagtctgaataactattttaactcaagtggcatagatgtggaagaaaagagtctcttcttggatatctgga
ggaactggcaaaaggatggtgacatgaaaatcctgcagagccagattatctctttctacctcagactctttgaagtcttgaaagaca
atcaggccatcagcaacaacataagcgtcattgaatcacacctgattactaccttcttcagcaacagcaaggcgaaaaaggatgc
attcatgagtattgccaagtttgaggtcaacaacccacaggtgcagcgccaagcattcaatgagctcatccgagtggtccaccag
ctgttgccggaatccagcctcaggaagcggaaaaggagtcgctgctgaGaattcacgcgttaagtcgacaatcaacctctggat
tacaaaatttgtgaaagattgactggtattcttaactatgttgctccttttacgctatgtggatacgctgctttaatgcctttgtatcatgct
attgcttcccgtatggctttcattttctcctccttgtataaatcctggttgctgtctctttatgaggagttgtggcccgttgtcaggcaacg
tggcgtggtgtgcactgtgtttgctgacgcaacccccactggttggggcattgccaccacctgtcagctcctttccgggactttcgc
tttccccctccctattgccacggcggaactcatcgccgcctgccttgcccgctgctggacaggggctcggctgttgggcactgac
aattccgtggtgttgtcggggaaatcatcgtcctttccttggctgctcgcctgtgttgccacctggattctgcgcgggacgtccttct
gctacgtcccttcggccctcaatccagcggaccttccttcccgcggcctgctgccggctctgcggcctcttccgcgtcttcgccttc
gccctcagacgagtcggatctccctttgggccgcctccccgcgtcgactttaagaccaatgacGGCCGCAGGAACC
CCTAGTGATGGAGTTGGCCACTCCCTCTCTGCGCGCTCGCTCGCTCACTGAGG
CCGGGCGACCAAAGGTCGCCCGACGCCCGGGCTTTGCCCGGGCGGCCTCAGT
GAGCGAGCGAGCGCGCAGCTGCCTGCAGGGGCGCCTGATGCGGTATTTTCTC
CTTACGCATCTGTGCGGTATTTCACACCGCATACGTCAAAGCAACCATAGTAC
GCGCCCTGTAGCGGCGCATTAAGCGCGGCGGGTGTGGTGGTTACGCGCAGCG
TGACCGCTACACTTGCCAGCGCCTTAGCGCCCGCTCCTTTCGCTTTCTTCCCTT
CCTTTCTCGCCACGTTCGCCGGCTTTCCCCGTCAAGCTCTAAATCGGGGGCTC
CCTTTAGGGTTCCGATTTAGTGCTTTACGGCACCTCGACCCCAAAAAACTTGA
TTTGGGTGATGGTTCACGTAGTGGGCCATCGCCCTGATAGACGGTTTTTCGCC
 
CTTTGACGTTGGAGTCCACGTTCTTTAATAGTGGACTCTTGTTCCAAACTGGA
ACAACACTCAACTCTATCTCGGGCTATTCTTTTGATTTATAAGGGATTTTGCCG
ATTTCGGTCTATTGGTTAAAAAATGAGCTGATTTAACAAAAATTTAACGCGAA
TTTTAACAAAATATTAACGTTTACAATTTTATGGTGCACTCTCAGTACAATCT
GCTCTGATGCCGCATAGTTAAGCCAGCCCCGACACCCGCCAACACCCGCTGA
CGCGCCCTGACGGGCTTGTCTGCTCCCGGCATCCGCTTACAGACAAGCTGTGA
CCGTCTCCGGGAGCTGCATGTGTCAGAGGTTTTCACCGTCATCACCGAAACGC
GCGAGACGAAAGGGCCTCGTGATACGCCTATTTTTATAGGTTAATGTCATGAT
AATAATGGTTTCTTAGACGTCAGGTGGCACTTTTCGGGGAAATGTGCGCGGA
ACCCCTATTTGTTTATTTTTCTAAATACATTCAAATATGTATCCGCTCATGAGA
CAATAACCCTGATAAATGCTTCAATAATATTGAAAAAGGAAGAGTATGAGTA
TTCAACATTTCCGTGTCGCCCTTATTCCCTTTTTTGCGGCATTTTGCCTTCCTGT
TTTTGCTCACCCAGAAACGCTGGTGAAAGTAAAAGATGCTGAAGATCAGTTG
GGTGCACGAGTGGGTTACATCGAACTGGATCTCAACAGCGGTAAGATCCTTG
AGAGTTTTCGCCCCGAAGAACGTTTTCCAATGATGAGCACTTTTAAAGTTCTG
CTATGTGGCGCGGTATTATCCCGTATTGACGCCGGGCAAGAGCAACTCGGTC
GCCGCATACACTATTCTCAGAATGACTTGGTTGAGTACTCACCAGTCACAGAA
AAGCATCTTACGGATGGCATGACAGTAAGAGAATTATGCAGTGCTGCCATAA
CCATGAGTGATAACACTGCGGCCAACTTACTTCTGACAACGATCGGAGGACC
GAAGGAGCTAACCGCTTTTTTGCACAACATGGGGGATCATGTAACTCGCCTTG
ATCGTTGGGAACCGGAGCTGAATGAAGCCATACCAAACGACGAGCGTGACAC
CACGATGCCTGTAGCAATGGCAACAACGTTGCGCAAACTATTAACTGGCGAA
CTACTTACTCTAGCTTCCCGGCAACAATTAATAGACTGGATGGAGGCGGATA
AAGTTGCAGGACCACTTCTGCGCTCGGCCCTTCCGGCTGGCTGGTTTATTGCT
GATAAATCTGGAGCCGGTGAGCGTGGAAGCCGCGGTATCATTGCAGCACTGG
GGCCAGATGGTAAGCCCTCCCGTATCGTAGTTATCTACACGACGGGGAGTCA
GGCAACTATGGATGAACGAAATAGACAGATCGCTGAGATAGGTGCCTCACTG
ATTAAGCATTGGTAACTGTCAGACCAAGTTTACTCATATATACTTTAGATTGA
TTTAAAACTTCATTTTTAATTTAAAAGGATCTAGGTGAAGATCCTTTTTGATA
ATCTCATGACCAAAATCCCTTAACGTGAGTTTTCGTTCCACTGAGCGTCAGAC
CCCGTAGAAAAGATCAAAGGATCTTCTTGAGATCCTTTTTTTCTGCGCGTAAT
CTGCTGCTTGCAAACAAAAAAACCACCGCTACCAGCGGTGGTTTGTTTGCCGG
ATCAAGAGCTACCAACTCTTTTTCCGAAGGTAACTGGCTTCAGCAGAGCGCA
GATACCAAATACTGTTCTTCTAGTGTAGCCGTAGTTAGGCCACCACTTCAAGA
ACTCTGTAGCACCGCCTACATACCTCGCTCTGCTAATCCTGTTACCAGTGGCT
GCTGCCAGTGGCGATAAGTCGTGTCTTACCGGGTTGGACTCAAGACGATAGTT
ACCGGATAAGGCGCAGCGGTCGGGCTGAACGGGGGGTTCGTGCACACAGCCC
AGCTTGGAGCGAACGACCTACACCGAACTGAGATACCTACAGCGTGAGCTAT
GAGAAAGCGCCACGCTTCCCGAAGGGAGAAAGGCGGACAGGTATCCGGTAA
GCGGCAGGGTCGGAACAGGAGAGCGCACGAGGGAGCTTCCAGGGGGAAACG
CCTGGTATCTTTATAGTCCTGTCGGGTTTCGCCACCTCTGACTTGAGCGTCGAT
TTTTGTGATGCTCGTCAGGGGGGCGGAGCCTATGGAAAAACGCCAGCAACGC
GGCCTTTTTACGGTTCCTGGCCTTTTGCTGGCCTTTTGCTCACATGT
**pGW111-pAAV_EFS-LTR-Light-P2A-Cxcl10-E2A-IFNg-WPRE (SEQ ID NO: 41)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
 
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atggagagtgtggtacagccttcagtgtttgtggtggatggacagacggacatcccattcaggcggctggaacagaaccaccgg
agacggcgctgtggcactgtccaggtcagcctggccctggtgctgctgctaAgtgctgggctggccactcagggctggtttctc
ctgagactgcatcaacgtcttggagacatagtagctcatctgccagatggaggcaaaggctcctgggagaagctgatacaagat
caacgatctcaccaggccaacccagcagcacatcttacaggagccaacgccagcttgataggtattggtggacctctgttatggg
agacacgacttggcctggccttcttgaggggcttgacgtatcatgatggggccctggtgaccatggagcccggttactactatgtg
tactccaaagtgcagctgagcggcgtgggctgcccccaggggctggccaatggcctccccatcacccatggactatacaagcg
cacatcccgctacccgaaggagttagaactgctggtcagtcggcggtcaccctgtggccgggccaacagctcccgagtctggt
gggacagcagcttcctgggcggcgtggtacatctggaggctggggaagaggtggtggtccgcgtgcctggaaaccgcctggt
cagaccacgtgacggcaccaggtcctatttcggagctttcatggtcactagtGGATCCGGCGCAACAAACTTC
TCTCTGCTGAAACAAGCCGGAGATGTCGAAGAGAATCCTGGACCGatgaacccaagt
gctgccgtcattttctgcctcatcctgctgggtctgagtgggactcaagggatccctctcgcaaggacggtccgctgcaactgcat
ccatatcgatgacgggccagtgagaatgagggccatagggaagcttgaaatcatccctgcgagcctatcctgcccacgtgttga
gatcattgccacgatgaaaaagaatgatgagcagagatgtctgaatccggaatctaagaccatcaagaatttaatgaaagcgttta
gccaaaaaaggtctaaaagggctcctagcggtacccagtgcaccaactacgccctgctgaagctggccggcgatgtggagag
caaccccgggcccgctagcatgaacgctacacactgcatcttggctttgcagctcttcctcatggctgtttctggctgttactgcca
cggcacagtcattgaaagcctagaaagtctgaataactattttaactcaagtggcatagatgtggaagaaaagagtctcttcttgga
tatctggaggaactggcaaaaggatggtgacatgaaaatcctgcagagccagattatctctttctacctcagactctttgaagtcttg
aaagacaatcaggccatcagcaacaacataagcgtcattgaatcacacctgattactaccttcttcagcaacagcaaggcgaaaa
aggatgcattcatgagtattgccaagtttgaggtcaacaacccacaggtccagcgccaagcattcaatgagctcatccgagtggt
ccaccagctgttgccggaatccagcctcaggaagcggaaaaggagtcgctgctgaGaattcacgcgttaagtcgacaatcaac
ctctggattacaaaatttgtgaaagattgactggtattcttaactatgttgctccttttacgctatgtggatacgctgctttaatgcctttgt
atcatgctattgcttcccgtatggctttcattttctcctccttgtataaatcctggttgctgtctctttatgaggagttgtggcccgttgtca
ggcaacgtggcgtggtgtgcactgtgtttgctgacgcaacccccactggttggggcattgccaccacctgtcagctcctttccgg
gactttcgctttccccctccctattgccacggcggaactcatcgccgcctgccttgcccgctgctggacaggggctcggctgttgg
gcactgacaattccgtggtgttgtcggggaaatcatcgtcctttccttggctgctcgcctgtgttgccacctggattctgcgcggga
cgtccttctgctacgtcccttcggccctcaatccagcggaccttccttcccgcggcctgctgccggctctgcggcctcttccgcgtc
ttcgccttcgccctcagacgagtcggatctccctttgggccgcctccccgcgtcgactttaagaccaatgacGGCCGCAG
GAACCCCTAGTGATGGAGTTGGCCACTCCCTCTCTGCGCGCTCGCTCGCTCAC
TGAGGCCGGGCGACCAAAGGTCGCCCGACGCCCGGGCTTTGCCCGGGCGGCC
TCAGTGAGCGAGCGAGCGCGCAGCTGCCTGCAGGGGCGCCTGATGCGGTATT
TTCTCCTTACGCATCTGTGCGGTATTTCACACCGCATACGTCAAAGCAACCAT
AGTACGCGCCCTGTAGCGGCGCATTAAGCGCGGCGGGTGTGGTGGTTACGCG
CAGCGTGACCGCTACACTTGCCAGCGCCTTAGCGCCCGCTCCTTTCGCTTTCT
TCCCTTCCTTTCTCGCCACGTTCGCCGGCTTTCCCCGTCAAGCTCTAAATCGGG
GGCTCCCTTTAGGGTTCCGATTTAGTGCTTTACGGCACCTCGACCCCAAAAAA
CTTGATTTGGGTGATGGTTCACGTAGTGGGCCATCGCCCTGATAGACGGTTTT
TCGCCCTTTGACGTTGGAGTCCACGTTCTTTAATAGTGGACTCTTGTTCCAAAC
TGGAACAACACTCAACTCTATCTCGGGCTATTCTTTTGATTTATAAGGGATTTT
GCCGATTTCGGTCTATTGGTTAAAAAATGAGCTGATTTAACAAAAATTTAACG
CGAATTTTAACAAAATATTAACGTTTACAATTTTATGGTGCACTCTCAGTACA
ATCTGCTCTGATGCCGCATAGTTAAGCCAGCCCCGACACCCGCCAACACCCG
CTGACGCGCCCTGACGGGCTTGTCTGCTCCCGGCATCCGCTTACAGACAAGCT
 
GTGACCGTCTCCGGGAGCTGCATGTGTCAGAGGTTTTCACCGTCATCACCGAA
ACGCGCGAGACGAAAGGGCCTCGTGATACGCCTATTTTTATAGGTTAATGTCA
TGATAATAATGGTTTCTTAGACGTCAGGTGGCACTTTTCGGGGAAATGTGCGC
GGAACCCCTATTTGTTTATTTTTCTAAATACATTCAAATATGTATCCGCTCATG
AGACAATAACCCTGATAAATGCTTCAATAATATTGAAAAAGGAAGAGTATGA
GTATTCAACATTTCCGTGTCGCCCTTATTCCCTTTTTTGCGGCATTTTGCCTTCC
TGTTTTTGCTCACCCAGAAACGCTGGTGAAAGTAAAAGATGCTGAAGATCAG
TTGGGTGCACGAGTGGGTTACATCGAACTGGATCTCAACAGCGGTAAGATCC
TTGAGAGTTTTCGCCCCGAAGAACGTTTTCCAATGATGAGCACTTTTAAAGTT
CTGCTATGTGGCGCGGTATTATCCCGTATTGACGCCGGGCAAGAGCAACTCG
GTCGCCGCATACACTATTCTCAGAATGACTTGGTTGAGTACTCACCAGTCACA
GAAAAGCATCTTACGGATGGCATGACAGTAAGAGAATTATGCAGTGCTGCCA
TAACCATGAGTGATAACACTGCGGCCAACTTACTTCTGACAACGATCGGAGG
ACCGAAGGAGCTAACCGCTTTTTTGCACAACATGGGGGATCATGTAACTCGC
CTTGATCGTTGGGAACCGGAGCTGAATGAAGCCATACCAAACGACGAGCGTG
ACACCACGATGCCTGTAGCAATGGCAACAACGTTGCGCAAACTATTAACTGG
CGAACTACTTACTCTAGCTTCCCGGCAACAATTAATAGACTGGATGGAGGCG
GATAAAGTTGCAGGACCACTTCTGCGCTCGGCCCTTCCGGCTGGCTGGTTTAT
TGCTGATAAATCTGGAGCCGGTGAGCGTGGAAGCCGCGGTATCATTGCAGCA
CTGGGGCCAGATGGTAAGCCCTCCCGTATCGTAGTTATCTACACGACGGGGA
GTCAGGCAACTATGGATGAACGAAATAGACAGATCGCTGAGATAGGTGCCTC
ACTGATTAAGCATTGGTAACTGTCAGACCAAGTTTACTCATATATACTTTAGA
TTGATTTAAAACTTCATTTTTAATTTAAAAGGATCTAGGTGAAGATCCTTTTTG
ATAATCTCATGACCAAAATCCCTTAACGTGAGTTTTCGTTCCACTGAGCGTCA
GACCCCGTAGAAAAGATCAAAGGATCTTCTTGAGATCCTTTTTTTCTGCGCGT
AATCTGCTGCTTGCAAACAAAAAAACCACCGCTACCAGCGGTGGTTTGTTTGC
CGGATCAAGAGCTACCAACTCTTTTTCCGAAGGTAACTGGCTTCAGCAGAGC
GCAGATACCAAATACTGTTCTTCTAGTGTAGCCGTAGTTAGGCCACCACTTCA
AGAACTCTGTAGCACCGCCTACATACCTCGCTCTGCTAATCCTGTTACCAGTG
GCTGCTGCCAGTGGCGATAAGTCGTGTCTTACCGGGTTGGACTCAAGACGAT
AGTTACCGGATAAGGCGCAGCGGTCGGGCTGAACGGGGGGTTCGTGCACACA
GCCCAGCTTGGAGCGAACGACCTACACCGAACTGAGATACCTACAGCGTGAG
CTATGAGAAAGCGCCACGCTTCCCGAAGGGAGAAAGGCGGACAGGTATCCG
GTAAGCGGCAGGGTCGGAACAGGAGAGCGCACGAGGGAGCTTCCAGGGGGA
AACGCCTGGTATCTTTATAGTCCTGTCGGGTTTCGCCACCTCTGACTTGAGCG
TCGATTTTTGTGATGCTCGTCAGGGGGGCGGAGCCTATGGAAAAACGCCAGC
AACGCGGCCTTTTTACGGTTCCTGGCCTTTTGCTGGCCTTTTGCTCACATGT
**pGW112-pAAV_EFS-LTR-41BBL-T2A-Light-P2A-Cxcl10-E2A-IFNg-WPRE (SEQ ID NO: 42)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
 
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atggaccagcacacacttgatgtggaggataccgcggatgccagacatccagcaggtacttcgtgcccctcggatgcggcgct
cctcagagataccgggctcctcgcggacgctgcgctcctctcagatactgtgcgccccacaaatgccgcgctccccacggatg
ctgcctaccctgcggttaatgttcgggatcgcgaggccgcgtggccgcctgcactgaacttctgttcccgccacccaaagctctat
ggcctagtcgctttggttttgctgcttctgatcgccgcctgtgttcctatcttcacccgcaccgagcctcggccagcgctcacaatca
ccacctcgcccaacctgggtacccgagagaataatgcagaccaggtcacccctgtttcccacattggctgccccaacactacac
aacagggctctcctgtgttcgccaagctactggctaaaaaccaagcatcgttgtgcaatacaactctgaactggcacagccaaga
tggagctgggagctcatacctatctcaaggtctgaggtacgaagaagacaaaaaggagttggtggtagacagtcccgggctcta
ctacgtatttttggaactgaagctcagtccaacattcacaaacacaggccacaaggtgcagggctgggtctctcttgttttgcaagc
aaagcctcaggtagatgactttgacaacttggccctgacagtggaactgttcccttgctccatggagaacaagttagtggaccgtt
cctggagtcaactgttgctcctgaaggctggccaccgcctcagtgtgggtctgagggcttatctgcatggagcccaggatgcata
cagagactgggagctgtcttatcccaacaccaccagctttggactctttcttgtgaaacccgacaacccatgggaaGGCAGT
GGAGAGGGCAGAGGAAGTCTGCTAACATGCGGTGACGTCGAGGAGAATCCT
GGCCCAatggagagtgtggtacagccttcagtgtttgtggtggatggacagacggacatcccattcaggcggctggaaca
gaaccaccggagacggcgctgtggcactgtccaggtcagcctggccctggtgctgctgctaAgtgctgggctggccactcag
ggctggtttctcctgagactgcatcaacgtcttggagacatagtagctcatctgccagatggaggcaaaggctcctgggagaagc
tgatacaagatcaacgatctcaccaggccaacccagcagcacatcttacaggagccaacgccagcttgataggtattggtggac
ctctgttatgggagacacgacttggcctggccttcttgaggggcttgacgtatcatgatggggccctggtgaccatggagcccgg
ttactactatgtgtactccaaagtgcagctgagcggcgtgggctgcccccaggggctggccaatggcctccccatcacccatgg
actatacaagcgcacatcccgctacccgaaggagttagaactgctggtcagtcggcggtcaccctgtggccgggccaacagct
cccgagtctggtgggacagcagcttcctgggcggcgtggtacatctggaggctggggaagaggtggtggtccgcgtgcctgg
aaaccgcctggtcagaccacgtgacggcaccaggtcctatttcggagctttcatggtcactagtGGATCCGGCGCAA
CAAACTTCTCTCTGCTGAAACAAGCCGGAGATGTCGAAGAGAATCCTGGACC
Gatgaacccaagtgctgccgtcattttctgcctcatcctgctgggtctgagtgggactcaagggatccctctcgcaaggacggtc
cgctgcaactgcatccatatcgatgacgggccagtgagaatgagggccatagggaagcttgaaatcatccctgcgagcctatcc
tgcccacgtgttgagatcattgccacgatgaaaaagaatgatgagcagagatgtctgaatccggaatctaagaccatcaagaattt
aatgaaagcgtttagccaaaaaaggtctaaaagggctcctagcggtacccagtgcaccaactacgccctgctgaagctggccg
gcgatgtggagagcaaccccgggcccgctagcatgaacgctacacactgcatcttggctttgcagctcttcctcatggctgtttct
ggctgttactgccacggcacagtcattgaaagcctagaaagtctgaataactattttaactcaagtggcatagatgtggaagaaaa
gagtctcttcttggatatctggaggaactggcaaaaggatggtgacatgaaaatcctgcagagccagattatctctttctacctcag
actctttgaagtcttgaaagacaatcaggccatcagcaacaacataagcgtcattgaatcacacctgattactaccttcttcagcaac
agcaaggcgaaaaaggatgcattcatgagtattgccaagtttgaggtcaacaacccacaggtccagcgccaagcattcaatgag
ctcatccgagtggtccaccagctgttgccggaatccagcctcaggaagcggaaaaggagtcgctgctgaGaattcacgcgtta
agtcgacaatcaacctctggattacaaaatttgtgaaagattgactggtattcttaactatgttgctccttttacgctatgtggatacgct
gctttaatgcctttgtatcatgctattgcttcccgtatggctttcattttctcctccttgtataaatcctggttgctgtctctttatgaggagtt
gtggcccgttgtcaggcaacgtggcgtggtgtgcactgtgtttgctgacgcaacccccactggttggggcattgccaccacctgt
cagctcctttccgggactttcgctttccccctccctattgccacggcggaactcatcgccgcctgccttgcccgctgctggacagg
ggctcggctgttgggcactgacaattccgtggtgttgtcggggaaatcatcgtcctttccttggctgctcgcctgtgttgccacctg
gattctgcgcgggacgtccttctgctacgtcccttcggccctcaatccagcggaccttccttcccgcggcctgctgccggctctgc
ggcctcttccgcgtcttcgccttcgccctcagacgagtcggatctccctttgggccgcctccccgcgtcgactttaagaccaatga
cGGCCGCAGGAACCCCTAGTGATGGAGTTGGCCACTCCCTCTCTGCGCGCTCG
CTCGCTCACTGAGGCCGGGCGACCAAAGGTCGCCCGACGCCCGGGCTTTGCC
CGGGCGGCCTCAGTGAGCGAGCGAGCGCGCAGCTGCCTGCAGGGGCGCCTGA
TGCGGTATTTTCTCCTTACGCATCTGTGCGGTATTTCACACCGCATACGTCAA
AGCAACCATAGTACGCGCCCTGTAGCGGCGCATTAAGCGCGGCGGGTGTGGT
GGTTACGCGCAGCGTGACCGCTACACTTGCCAGCGCCTTAGCGCCCGCTCCTT
TCGCTTTCTTCCCTTCCTTTCTCGCCACGTTCGCCGGCTTTCCCCGTCAAGCTC
TAAATCGGGGGCTCCCTTTAGGGTTCCGATTTAGTGCTTTACGGCACCTCGAC
 
CCCAAAAAACTTGATTTGGGTGATGGTTCACGTAGTGGGCCATCGCCCTGATA
GACGGTTTTTCGCCCTTTGACGTTGGAGTCCACGTTCTTTAATAGTGGACTCTT
GTTCCAAACTGGAACAACACTCAACTCTATCTCGGGCTATTCTTTTGATTTAT
AAGGGATTTTGCCGATTTCGGTCTATTGGTTAAAAAATGAGCTGATTTAACAA
AAATTTAACGCGAATTTTAACAAAATATTAACGTTTACAATTTTATGGTGCAC
TCTCAGTACAATCTGCTCTGATGCCGCATAGTTAAGCCAGCCCCGACACCCGC
CAACACCCGCTGACGCGCCCTGACGGGCTTGTCTGCTCCCGGCATCCGCTTAC
AGACAAGCTGTGACCGTCTCCGGGAGCTGCATGTGTCAGAGGTTTTCACCGTC
ATCACCGAAACGCGCGAGACGAAAGGGCCTCGTGATACGCCTATTTTTATAG
GTTAATGTCATGATAATAATGGTTTCTTAGACGTCAGGTGGCACTTTTCGGGG
AAATGTGCGCGGAACCCCTATTTGTTTATTTTTCTAAATACATTCAAATATGT
ATCCGCTCATGAGACAATAACCCTGATAAATGCTTCAATAATATTGAAAAAG
GAAGAGTATGAGTATTCAACATTTCCGTGTCGCCCTTATTCCCTTTTTTGCGGC
ATTTTGCCTTCCTGTTTTTGCTCACCCAGAAACGCTGGTGAAAGTAAAAGATG
CTGAAGATCAGTTGGGTGCACGAGTGGGTTACATCGAACTGGATCTCAACAG
CGGTAAGATCCTTGAGAGTTTTCGCCCCGAAGAACGTTTTCCAATGATGAGCA
CTTTTAAAGTTCTGCTATGTGGCGCGGTATTATCCCGTATTGACGCCGGGCAA
GAGCAACTCGGTCGCCGCATACACTATTCTCAGAATGACTTGGTTGAGTACTC
ACCAGTCACAGAAAAGCATCTTACGGATGGCATGACAGTAAGAGAATTATGC
AGTGCTGCCATAACCATGAGTGATAACACTGCGGCCAACTTACTTCTGACAAC
GATCGGAGGACCGAAGGAGCTAACCGCTTTTTTGCACAACATGGGGGATCAT
GTAACTCGCCTTGATCGTTGGGAACCGGAGCTGAATGAAGCCATACCAAACG
ACGAGCGTGACACCACGATGCCTGTAGCAATGGCAACAACGTTGCGCAAACT
ATTAACTGGCGAACTACTTACTCTAGCTTCCCGGCAACAATTAATAGACTGGA
TGGAGGCGGATAAAGTTGCAGGACCACTTCTGCGCTCGGCCCTTCCGGCTGG
CTGGTTTATTGCTGATAAATCTGGAGCCGGTGAGCGTGGAAGCCGCGGTATC
ATTGCAGCACTGGGGCCAGATGGTAAGCCCTCCCGTATCGTAGTTATCTACAC
GACGGGGAGTCAGGCAACTATGGATGAACGAAATAGACAGATCGCTGAGAT
AGGTGCCTCACTGATTAAGCATTGGTAACTGTCAGACCAAGTTTACTCATATA
TACTTTAGATTGATTTAAAACTTCATTTTTAATTTAAAAGGATCTAGGTGAAG
ATCCTTTTTGATAATCTCATGACCAAAATCCCTTAACGTGAGTTTTCGTTCCAC
TGAGCGTCAGACCCCGTAGAAAAGATCAAAGGATCTTCTTGAGATCCTTTTTT
TCTGCGCGTAATCTGCTGCTTGCAAACAAAAAAACCACCGCTACCAGCGGTG
GTTTGTTTGCCGGATCAAGAGCTACCAACTCTTTTTCCGAAGGTAACTGGCTT
CAGCAGAGCGCAGATACCAAATACTGTTCTTCTAGTGTAGCCGTAGTTAGGCC
ACCACTTCAAGAACTCTGTAGCACCGCCTACATACCTCGCTCTGCTAATCCTG
TTACCAGTGGCTGCTGCCAGTGGCGATAAGTCGTGTCTTACCGGGTTGGACTC
AAGACGATAGTTACCGGATAAGGCGCAGCGGTCGGGCTGAACGGGGGGTTCG
TGCACACAGCCCAGCTTGGAGCGAACGACCTACACCGAACTGAGATACCTAC
AGCGTGAGCTATGAGAAAGCGCCACGCTTCCCGAAGGGAGAAAGGCGGACA
GGTATCCGGTAAGCGGCAGGGTCGGAACAGGAGAGCGCACGAGGGAGCTTC
CAGGGGGAAACGCCTGGTATCTTTATAGTCCTGTCGGGTTTCGCCACCTCTGA
CTTGAGCGTCGATTTTTGTGATGCTCGTCAGGGGGGCGGAGCCTATGGAAAA
ACGCCAGCAACGCGGCCTTTTTACGGTTCCTGGCCTTTTGCTGGCCTTTTGCTC
ACATGT
**pGW113-pAAV_EFS-LTR-IL23-T2A-Light-P2A-Cxcl10-E2A-IFNg-WPRE (SEQ ID NO: 43)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
 
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
ATGTGTCCTCAGAAGCTAACCATCTCCTGGTTTGCCATCGTTTTGCTGGTGTCT
CCACTCATGGCCATGTGGGAGCTGGAGAAAGACGTTTATGTTGTAGAGGTGG
ACTGGACTCCCGATGCCCCTGGAGAAACAGTGAACCTCACCTGTGACACGCC
TGAAGAAGATGACATCACCTGGACCTCAGACCAGAGACATGGAGTCATAGGC
TCTGGAAAGACCCTGACCATCACTGTCAAAGAGTTTCTAGATGCTGGCCAGTA
CACCTGCCACAAAGGAGGCGAGACTCTGAGCCACTCACATCTGCTGCTCCAC
AAGAAGGAAAATGGAATTTGGTCCACTGAAATTTTAAAAAATTTCAAAAACA
AGACTTTCCTGAAGTGTGAAGCACCAAATTACTCCGGACGGTTCACGTGCTCA
TGGCTGGTGCAAAGAAACATGGACTTGAAGTTCAACATCAAGAGCAGTAGCA
GTTCCCCTGACTCTCGGGCAGTGACATGTGGAATGGCGTCTCTGTCTGCAGAG
AAGGTCACACTGGACCAAAGGGACTATGAGAAGTATTCAGTGTCCTGCCAGG
AGGATGTCACCTGCCCAACTGCCGAGGAGACCCTGCCCATTGAACTGGCGTT
GGAAGCACGGCAGCAGAATAAATATGAGAACTACAGCACCAGCTTCTTCATC
AGGGACATCATCAAACCAGACCCGCCCAAGAACTTGCAGATGAAGCCTTTGA
AGAACTCACAGGTGGAGGTCAGCTGGGAGTACCCTGACTCCTGGAGCACTCC
CCATTCCTACTTCTCCCTCAAGTTCTTTGTTCGAATCCAGCGCAAGAAAGAAA
AGATGAAGGAGACAGAGGAGGGGTGTAACCAGAAAGGTGCGTTCCTCGTAG
AGAAGACATCTACCGAAGTCCAATGCAAAGGCGGGAATGTCTGCGTGCAAGC
TCAGGATCGCTATTACAATTCCTCATGCAGCAAGTGGGCATGTGTTCCCTGCA
GGGTCCGATCCGGCAGTGGAGAGGGCAGAGGAAGTCTGCTAACATGCGGTGA
CGTCGAGGAGAATCCTGGCCCAatggagagtgtggtacagccttcagtgtttgtggtggatggacagacgg
acatcccattcaggcggctggaacagaaccaccggagacggcgctgtggcactgtccaggtcagcctggccctggtgctgctg
ctaAgtgctgggctggccactcagggctggtttctcctgagactgcatcaacgtcttggagacatagtagctcatctgccagatg
gaggcaaaggctcctgggagaagctgatacaagatcaacgatctcaccaggccaacccagcagcacatcttacaggagccaa
cgccagcttgataggtattggtggacctctgttatgggagacacgacttggcctggccttcttgaggggcttgacgtatcatgatgg
ggccctggtgaccatggagcccggttactactatgtgtactccaaagtgcagctgagcggcgtgggctgcccccaggggctgg
ccaatggcctccccatcacccatggactatacaagcgcacatcccgctacccgaaggagttagaactgctggtcagtcggcggt
caccctgtggccgggccaacagctcccgagtctggtgggacagcagcttcctgggcggcgtggtacatctggaggctgggga
agaggtggtggtccgcgtgcctggaaaccgcctggtcagaccacgtgacggcaccaggtcctatttcggagctttcatggtcact
agtGGATCCGGCGCAACAAACTTCTCTCTGCTGAAACAAGCCGGAGATGTCGA
AGAGAATCCTGGACCGatgaacccaagtgctgccgtcattttctgcctcatcctgctgggtctgagtgggactcaa
gggatccctctcgcaaggacggtccgctgcaactgcatccatatcgatgacgggccagtgagaatgagggccatagggaagct
tgaaatcatccctgcgagcctatcctgcccacgtgttgagatcattgccacgatgaaaaagaatgatgagcagagatgtctgaatc
cggaatctaagaccatcaagaatttaatgaaagcgtttagccaaaaaaggtctaaaagggctcctagcggtacccagtgcaccaa
ctacgccctgctgaagctggccggcgatgtggagagcaaccccgggcccgctagcatgaacgctacacactgcatcttggcttt
gcagctcttcctcatggctgtttctggctgttactgccacggcacagtcattgaaagcctagaaagtctgaataactattttaactcaa
gtggcatagatgtggaagaaaagagtctcttcttggatatctggaggaactggcaaaaggatggtgacatgaaaatcctgcagag
ccagattatctctttctacctcagactctttgaagtcttgaaagacaatcaggccatcagcaacaacataagcgtcattgaatcacac
ctgattactaccttcttcagcaacagcaaggcgaaaaaggatgcattcatgagtattgccaagtttgaggtcaacaacccacaggt
 
ccagcgccaagcattcaatgagctcatccgagtggtccaccagctgttgccggaatccagcctcaggaagcggaaaaggagtc
gctgctgaGaattcacgcgttaagtcgacaatcaacctctggattacaaaatttgtgaaagattgactggtattcttaactatgttgct
ccttttacgctatgtggatacgctgctttaatgcctttgtatcatgctattgcttcccgtatggctttcattttctcctccttgtataaatcct
ggttgctgtctctttatgaggagttgtggcccgttgtcaggcaacgtggcgtggtgtgcactgtgtttgctgacgcaacccccactg
gttggggcattgccaccacctgtcagctcctttccgggactttcgctttccccctccctattgccacggcggaactcatcgccgcct
gccttgcccgctgctggacaggggctcggctgttgggcactgacaattccgtggtgttgtcggggaaatcatcgtcctttccttgg
ctgctcgcctgtgttgccacctggattctgcgcgggacgtccttctgctacgtcccttcggccctcaatccagcggaccttccttcc
cgcggcctgctgccggctctgcggcctcttccgcgtcttcgccttcgccctcagacgagtcggatctccctttgggccgcctccc
cgcgtcgactttaagaccaatgacGGCCGCAGGAACCCCTAGTGATGGAGTTGGCCACTCC
CTCTCTGCGCGCTCGCTCGCTCACTGAGGCCGGGCGACCAAAGGTCGCCCGA
CGCCCGGGCTTTGCCCGGGCGGCCTCAGTGAGCGAGCGAGCGCGCAGCTGCC
TGCAGGGGCGCCTGATGCGGTATTTTCTCCTTACGCATCTGTGCGGTATTTCA
CACCGCATACGTCAAAGCAACCATAGTACGCGCCCTGTAGCGGCGCATTAAG
CGCGGCGGGTGTGGTGGTTACGCGCAGCGTGACCGCTACACTTGCCAGCGCC
TTAGCGCCCGCTCCTTTCGCTTTCTTCCCTTCCTTTCTCGCCACGTTCGCCGGC
TTTCCCCGTCAAGCTCTAAATCGGGGGCTCCCTTTAGGGTTCCGATTTAGTGC
TTTACGGCACCTCGACCCCAAAAAACTTGATTTGGGTGATGGTTCACGTAGTG
GGCCATCGCCCTGATAGACGGTTTTTCGCCCTTTGACGTTGGAGTCCACGTTC
TTTAATAGTGGACTCTTGTTCCAAACTGGAACAACACTCAACTCTATCTCGGG
CTATTCTTTTGATTTATAAGGGATTTTGCCGATTTCGGTCTATTGGTTAAAAAA
TGAGCTGATTTAACAAAAATTTAACGCGAATTTTAACAAAATATTAACGTTTA
CAATTTTATGGTGCACTCTCAGTACAATCTGCTCTGATGCCGCATAGTTAAGC
CAGCCCCGACACCCGCCAACACCCGCTGACGCGCCCTGACGGGCTTGTCTGC
TCCCGGCATCCGCTTACAGACAAGCTGTGACCGTCTCCGGGAGCTGCATGTGT
CAGAGGTTTTCACCGTCATCACCGAAACGCGCGAGACGAAAGGGCCTCGTGA
TACGCCTATTTTTATAGGTTAATGTCATGATAATAATGGTTTCTTAGACGTCA
GGTGGCACTTTTCGGGGAAATGTGCGCGGAACCCCTATTTGTTTATTTTTCTA
AATACATTCAAATATGTATCCGCTCATGAGACAATAACCCTGATAAATGCTTC
AATAATATTGAAAAAGGAAGAGTATGAGTATTCAACATTTCCGTGTCGCCCTT
ATTCCCTTTTTTGCGGCATTTTGCCTTCCTGTTTTTGCTCACCCAGAAACGCTG
GTGAAAGTAAAAGATGCTGAAGATCAGTTGGGTGCACGAGTGGGTTACATCG
AACTGGATCTCAACAGCGGTAAGATCCTTGAGAGTTTTCGCCCCGAAGAACG
TTTTCCAATGATGAGCACTTTTAAAGTTCTGCTATGTGGCGCGGTATTATCCC
GTATTGACGCCGGGCAAGAGCAACTCGGTCGCCGCATACACTATTCTCAGAA
TGACTTGGTTGAGTACTCACCAGTCACAGAAAAGCATCTTACGGATGGCATG
ACAGTAAGAGAATTATGCAGTGCTGCCATAACCATGAGTGATAACACTGCGG
CCAACTTACTTCTGACAACGATCGGAGGACCGAAGGAGCTAACCGCTTTTTTG
CACAACATGGGGGATCATGTAACTCGCCTTGATCGTTGGGAACCGGAGCTGA
ATGAAGCCATACCAAACGACGAGCGTGACACCACGATGCCTGTAGCAATGGC
AACAACGTTGCGCAAACTATTAACTGGCGAACTACTTACTCTAGCTTCCCGGC
AACAATTAATAGACTGGATGGAGGCGGATAAAGTTGCAGGACCACTTCTGCG
CTCGGCCCTTCCGGCTGGCTGGTTTATTGCTGATAAATCTGGAGCCGGTGAGC
GTGGAAGCCGCGGTATCATTGCAGCACTGGGGCCAGATGGTAAGCCCTCCCG
TATCGTAGTTATCTACACGACGGGGAGTCAGGCAACTATGGATGAACGAAAT
AGACAGATCGCTGAGATAGGTGCCTCACTGATTAAGCATTGGTAACTGTCAG
ACCAAGTTTACTCATATATACTTTAGATTGATTTAAAACTTCATTTTTAATTTA
AAAGGATCTAGGTGAAGATCCTTTTTGATAATCTCATGACCAAAATCCCTTAA
CGTGAGTTTTCGTTCCACTGAGCGTCAGACCCCGTAGAAAAGATCAAAGGAT
CTTCTTGAGATCCTTTTTTTCTGCGCGTAATCTGCTGCTTGCAAACAAAAAAA
CCACCGCTACCAGCGGTGGTTTGTTTGCCGGATCAAGAGCTACCAACTCTTTT
 
TCCGAAGGTAACTGGCTTCAGCAGAGCGCAGATACCAAATACTGTTCTTCTAG
TGTAGCCGTAGTTAGGCCACCACTTCAAGAACTCTGTAGCACCGCCTACATAC
CTCGCTCTGCTAATCCTGTTACCAGTGGCTGCTGCCAGTGGCGATAAGTCGTG
TCTTACCGGGTTGGACTCAAGACGATAGTTACCGGATAAGGCGCAGCGGTCG
GGCTGAACGGGGGGTTCGTGCACACAGCCCAGCTTGGAGCGAACGACCTACA
CCGAACTGAGATACCTACAGCGTGAGCTATGAGAAAGCGCCACGCTTCCCGA
AGGGAGAAAGGCGGACAGGTATCCGGTAAGCGGCAGGGTCGGAACAGGAGA
GCGCACGAGGGAGCTTCCAGGGGGAAACGCCTGGTATCTTTATAGTCCTGTC
GGGTTTCGCCACCTCTGACTTGAGCGTCGATTTTTGTGATGCTCGTCAGGGGG
GCGGAGCCTATGGAAAAACGCCAGCAACGCGGCCTTTTTACGGTTCCTGGCC
TTTTGCTGGCCTTTTGCTCACATGT
**pGW114-pAAV_EFS-LTR-Cd80-T2A-Light-P2A-41BBL-E2A-IFNg-WPRE (SEQ ID NO: 44)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atggcttgcaattgtcagttgatgcaggatacaccactcctcaagtttccatgtccaaggctcattcttctctttgtgctgctgattcgtc
tttcacaagtgtcttcagatgttgatgaacaactgtccaagtcagtgaaagataaggtattgctgccttgccgttacaactctcctcat
gaagatgagtctgaagaccgaatctactggcaaaaacatgacaaagtggtgctgtctgtcattgctgggaaactaaaagtgtggc
ccgagtataagaaccggactttatatgacaacactacctactctcttatcatcctgggcctggtcctttcagaccggggcacataca
gctgtgtcgttcaaaagaaggaaagaggaacgtatgaagttaaacacttggctttagtaaagttgtccatcaaagctgacttctcta
cccccaacataactgagtctggaaacccatctgcagacactaaaaggattacctgctttgcttccgggggtttcccaaagcctcgc
ttctcttggttggaaaatggaagagaattacctggcatcaatacgacaatttcccaggatcctgaatctgaattgtacaccattagta
gccaactagatttcaatacgactcgcaaccacaccattaagtgtctcattaaatatggagatgctcacgtgtcagaggacttcacct
gggaaaaacccccagaagaccctcctgatagcaagaacacacttgtgctctttggggcaggattcggcgcagtaataacagtcg
tcgtcatcgttgtcatcatcaaatgcttctgtaagcacagaagctgtttcagaagaaatgaggcaagcagagaaacaaacaacag
ccttaccttcgggcctgaagaagcattagctgaacagaccgtcttccttcgtacgGGCAGTGGAGAGGGCAGA
GGAAGTCTGCTAACATGCGGTGACGTCGAGGAGAATCCTGGCCCAatggagagtgt
ggtacagccttcagtgtttgtggtggatggacagacggacatcccattcaggcggctggaacagaaccaccggagacggcgct
gtggcactgtccaggtcagcctggccctggtgctgctgctaAgtgctgggctggccactcagggctggtttctcctgagactgc
atcaacgtcttggagacatagtagctcatctgccagatggaggcaaaggctcctgggagaagctgatacaagatcaacgatctc
accaggccaacccagcagcacatcttacaggagccaacgccagcttgataggtattggtggacctctgttatgggagacacgac
ttggcctggccttcttgaggggcttgacgtatcatgatggggccctggtgaccatggagcccggttactactatgtgtactccaaa
gtgcagctgagcggcgtgggctgcccccaggggctggccaatggcctccccatcacccatggactatacaagcgcacatccc
gctacccgaaggagttagaactgctggtcagtcggcggtcaccctgtggccgggccaacagctcccgagtctggtgggacag
cagcttcctgggcggcgtggtacatctggaggctggggaagaggtggtggtccgcgtgcctggaaaccgcctggtcagacca
cgtgacggcaccaggtcctatttcggagctttcatggtcactagtGGATCCGGCGCAACAAACTTCTCTCT
GCTGAAACAAGCCGGAGATGTCGAAGAGAATCCTGGACCGatggaccagcacacacttg
atgtggaggataccgcggatgccagacatccagcaggtacttcgtgcccctcggatgcggcgctcctcagagataccgggctc
 
ctcgcggacgctgcgctcctctcagatactgtgcgccccacaaatgccgcgctccccacggatgctgcctaccctgcggttaatg
ttcgggatcgcgaggccgcgtggccgcctgcactgaacttctgttcccgccacccaaagctctatggcctagtcgctttggttttg
ctgcttctgatcgccgcctgtgttcctatcttcacccgcaccgagcctcggccagcgctcacaatcaccacctcgcccaacctgg
gtacccgagagaataatgcagaccaggtcacccctgtttcccacattggctgccccaacactacacaacagggctctcctgtgtt
cgccaagctactggctaaaaaccaagcatcgttgtgcaatacaactctgaactggcacagccaagatggagctgggagctcata
cctatctcaaggtctgaggtacgaagaagacaaaaaggagttggtggtagacagtcccgggctctactacgtatttttggaactga
agctcagtccaacattcacaaacacaggccacaaggtgcagggctgggtctctcttgttttgcaagcaaagcctcaggtagatga
ctttgacaacttggccctgacagtggaactgttcccttgctccatggagaacaagttagtggaccgttcctggagtcaactgttgct
cctgaaggctggccaccgcctcagtgtgggtctgagggcttatctgcatggagcccaggatgcatacagagactgggagctgtc
ttatcccaacaccaccagctttggactctttcttgtgaaacccgacaacccatgggaaagcggtacccagtgcaccaactacgcc
ctgctgaagctggccggcgatgtggagagcaaccccgggcccgctagcatgaacgctacacactgcatcttggctttgcagctc
ttcctcatggctgtttctggctgttactgccacggcacagtcattgaaagcctagaaagtctgaataactattttaactcaagtggcat
agatgtggaagaaaagagtctcttcttggatatctggaggaactggcaaaaggatggtgacatgaaaatcctgcagagccagatt
atctctttctacctcagactctttgaagtcttgaaagacaatcaggccatcagcaacaacataagcgtcattgaatcacacctgatta
ctaccttcttcagcaacagcaaggcgaaaaaggatgcattcatgagtattgccaagtttgaggtcaacaacccacaggtgcagcg
ccaagcattcaatgagctcatccgagtggtccaccagctgttgccggaatccagcctcaggaagcggaaaaggagtcgctgct
gaGaattcacgcgttaagtcgacaatcaacctctggattacaaaatttgtgaaagattgactggtattcttaactatgttgctcctttta
cgctatgtggatacgctgctttaatgcctttgtatcatgctattgcttcccgtatggctttcattttctcctccttgtataaatcctggttgct
gtctctttatgaggagttgtggcccgttgtcaggcaacgtggcgtggtgtgcactgtgtttgctgacgcaacccccactggttggg
gcattgccaccacctgtcagctcctttccgggactttcgctttccccctccctattgccacggcggaactcatcgccgcctgccttg
cccgctgctggacaggggctcggctgttgggcactgacaattccgtggtgttgtcggggaaatcatcgtcctttccttggctgctc
gcctgtgttgccacctggattctgcgcgggacgtccttctgctacgtcccttcggccctcaatccagcggaccttccttcccgcgg
cctgctgccggctctgcggcctcttccgcgtcttcgccttcgccctcagacgagtcggatctccctttgggccgcctccccgcgtc
gactttaagaccaatgacGGCCGCAGGAACCCCTAGTGATGGAGTTGGCCACTCCCTCTC
TGCGCGCTCGCTCGCTCACTGAGGCCGGGCGACCAAAGGTCGCCCGACGCCC
GGGCTTTGCCCGGGCGGCCTCAGTGAGCGAGCGAGCGCGCAGCTGCCTGCAG
GGGCGCCTGATGCGGTATTTTCTCCTTACGCATCTGTGCGGTATTTCACACCG
CATACGTCAAAGCAACCATAGTACGCGCCCTGTAGCGGCGCATTAAGCGCGG
CGGGTGTGGTGGTTACGCGCAGCGTGACCGCTACACTTGCCAGCGCCTTAGC
GCCCGCTCCTTTCGCTTTCTTCCCTTCCTTTCTCGCCACGTTCGCCGGCTTTCCC
CGTCAAGCTCTAAATCGGGGGCTCCCTTTAGGGTTCCGATTTAGTGCTTTACG
GCACCTCGACCCCAAAAAACTTGATTTGGGTGATGGTTCACGTAGTGGGCCAT
CGCCCTGATAGACGGTTTTTCGCCCTTTGACGTTGGAGTCCACGTTCTTTAATA
GTGGACTCTTGTTCCAAACTGGAACAACACTCAACTCTATCTCGGGCTATTCT
TTTGATTTATAAGGGATTTTGCCGATTTCGGTCTATTGGTTAAAAAATGAGCT
GATTTAACAAAAATTTAACGCGAATTTTAACAAAATATTAACGTTTACAATTT
TATGGTGCACTCTCAGTACAATCTGCTCTGATGCCGCATAGTTAAGCCAGCCC
CGACACCCGCCAACACCCGCTGACGCGCCCTGACGGGCTTGTCTGCTCCCGG
CATCCGCTTACAGACAAGCTGTGACCGTCTCCGGGAGCTGCATGTGTCAGAG
GTTTTCACCGTCATCACCGAAACGCGCGAGACGAAAGGGCCTCGTGATACGC
CTATTTTTATAGGTTAATGTCATGATAATAATGGTTTCTTAGACGTCAGGTGG
CACTTTTCGGGGAAATGTGCGCGGAACCCCTATTTGTTTATTTTTCTAAATAC
ATTCAAATATGTATCCGCTCATGAGACAATAACCCTGATAAATGCTTCAATAA
TATTGAAAAAGGAAGAGTATGAGTATTCAACATTTCCGTGTCGCCCTTATTCC
CTTTTTTGCGGCATTTTGCCTTCCTGTTTTTGCTCACCCAGAAACGCTGGTGAA
AGTAAAAGATGCTGAAGATCAGTTGGGTGCACGAGTGGGTTACATCGAACTG
GATCTCAACAGCGGTAAGATCCTTGAGAGTTTTCGCCCCGAAGAACGTTTTCC
AATGATGAGCACTTTTAAAGTTCTGCTATGTGGCGCGGTATTATCCCGTATTG
ACGCCGGGCAAGAGCAACTCGGTCGCCGCATACACTATTCTCAGAATGACTT
GGTTGAGTACTCACCAGTCACAGAAAAGCATCTTACGGATGGCATGACAGTA
 
AGAGAATTATGCAGTGCTGCCATAACCATGAGTGATAACACTGCGGCCAACT
TACTTCTGACAACGATCGGAGGACCGAAGGAGCTAACCGCTTTTTTGCACAA
CATGGGGGATCATGTAACTCGCCTTGATCGTTGGGAACCGGAGCTGAATGAA
GCCATACCAAACGACGAGCGTGACACCACGATGCCTGTAGCAATGGCAACAA
CGTTGCGCAAACTATTAACTGGCGAACTACTTACTCTAGCTTCCCGGCAACAA
TTAATAGACTGGATGGAGGCGGATAAAGTTGCAGGACCACTTCTGCGCTCGG
CCCTTCCGGCTGGCTGGTTTATTGCTGATAAATCTGGAGCCGGTGAGCGTGGA
AGCCGCGGTATCATTGCAGCACTGGGGCCAGATGGTAAGCCCTCCCGTATCG
TAGTTATCTACACGACGGGGAGTCAGGCAACTATGGATGAACGAAATAGACA
GATCGCTGAGATAGGTGCCTCACTGATTAAGCATTGGTAACTGTCAGACCAA
GTTTACTCATATATACTTTAGATTGATTTAAAACTTCATTTTTAATTTAAAAGG
ATCTAGGTGAAGATCCTTTTTGATAATCTCATGACCAAAATCCCTTAACGTGA
GTTTTCGTTCCACTGAGCGTCAGACCCCGTAGAAAAGATCAAAGGATCTTCTT
GAGATCCTTTTTTTCTGCGCGTAATCTGCTGCTTGCAAACAAAAAAACCACCG
CTACCAGCGGTGGTTTGTTTGCCGGATCAAGAGCTACCAACTCTTTTTCCGAA
GGTAACTGGCTTCAGCAGAGCGCAGATACCAAATACTGTTCTTCTAGTGTAGC
CGTAGTTAGGCCACCACTTCAAGAACTCTGTAGCACCGCCTACATACCTCGCT
CTGCTAATCCTGTTACCAGTGGCTGCTGCCAGTGGCGATAAGTCGTGTCTTAC
CGGGTTGGACTCAAGACGATAGTTACCGGATAAGGCGCAGCGGTCGGGCTGA
ACGGGGGGTTCGTGCACACAGCCCAGCTTGGAGCGAACGACCTACACCGAAC
TGAGATACCTACAGCGTGAGCTATGAGAAAGCGCCACGCTTCCCGAAGGGAG
AAAGGCGGACAGGTATCCGGTAAGCGGCAGGGTCGGAACAGGAGAGCGCAC
GAGGGAGCTTCCAGGGGGAAACGCCTGGTATCTTTATAGTCCTGTCGGGTTTC
GCCACCTCTGACTTGAGCGTCGATTTTTGTGATGCTCGTCAGGGGGGCGGAGC
CTATGGAAAAACGCCAGCAACGCGGCCTTTTTACGGTTCCTGGCCTTTTGCTG
GCCTTTTGCTCACATGT
**pGW115-pAAV_EFS-LTR-IL23-T2A-Light-P2A-41BBL-E2A-IFNg-WPRE (SEQ ID NO: 45)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
ATGTGTCCTCAGAAGCTAACCATCTCCTGGTTTGCCATCGTTTTGCTGGTGTCT
CCACTCATGGCCATGTGGGAGCTGGAGAAAGACGTTTATGTTGTAGAGGTGG
ACTGGACTCCCGATGCCCCTGGAGAAACAGTGAACCTCACCTGTGACACGCC
TGAAGAAGATGACATCACCTGGACCTCAGACCAGAGACATGGAGTCATAGGC
TCTGGAAAGACCCTGACCATCACTGTCAAAGAGTTTCTAGATGCTGGCCAGTA
CACCTGCCACAAAGGAGGCGAGACTCTGAGCCACTCACATCTGCTGCTCCAC
AAGAAGGAAAATGGAATTTGGTCCACTGAAATTTTAAAAAATTTCAAAAACA
AGACTTTCCTGAAGTGTGAAGCACCAAATTACTCCGGACGGTTCACGTGCTCA
 
TGGCTGGTGCAAAGAAACATGGACTTGAAGTTCAACATCAAGAGCAGTAGCA
GTTCCCCTGACTCTCGGGCAGTGACATGTGGAATGGCGTCTCTGTCTGCAGAG
AAGGTCACACTGGACCAAAGGGACTATGAGAAGTATTCAGTGTCCTGCCAGG
AGGATGTCACCTGCCCAACTGCCGAGGAGACCCTGCCCATTGAACTGGCGTT
GGAAGCACGGCAGCAGAATAAATATGAGAACTACAGCACCAGCTTCTTCATC
AGGGACATCATCAAACCAGACCCGCCCAAGAACTTGCAGATGAAGCCTTTGA
AGAACTCACAGGTGGAGGTCAGCTGGGAGTACCCTGACTCCTGGAGCACTCC
CCATTCCTACTTCTCCCTCAAGTTCTTTGTTCGAATCCAGCGCAAGAAAGAAA
AGATGAAGGAGACAGAGGAGGGGTGTAACCAGAAAGGTGCGTTCCTCGTAG
AGAAGACATCTACCGAAGTCCAATGCAAAGGCGGGAATGTCTGCGTGCAAGC
TCAGGATCGCTATTACAATTCCTCATGCAGCAAGTGGGCATGTGTTCCCTGCA
GGGTCCGATCCGGCAGTGGAGAGGGCAGAGGAAGTCTGCTAACATGCGGTGA
CGTCGAGGAGAATCCTGGCCCAatggagagtgtggtacagccttcagtgtttgtggtggatggacagacgg
acatcccattcaggcggctggaacagaaccaccggagacggcgctgtggcactgtccaggtcagcctggccctggtgctgctg
ctaAgtgctgggctggccactcagggctggtttctcctgagactgcatcaacgtcttggagacatagtagctcatctgccagatg
gaggcaaaggctcctgggagaagctgatacaagatcaacgatctcaccaggccaacccagcagcacatcttacaggagccaa
cgccagcttgataggtattggtggacctctgttatgggagacacgacttggcctggccttcttgaggggcttgacgtatcatgatgg
ggccctggtgaccatggagcccggttactactatgtgtactccaaagtgcagctgagcggcgtgggctgcccccaggggctgg
ccaatggcctccccatcacccatggactatacaagcgcacatcccgctacccgaaggagttagaactgctggtcagtcggcggt
caccctgtggccgggccaacagctcccgagtctggtgggacagcagcttcctgggcggcgtggtacatctggaggctgggga
agaggtggtggtccgcgtgcctggaaaccgcctggtcagaccacgtgacggcaccaggtcctatttcggagctttcatggtcact
agtGGATCCGGCGCAACAAACTTCTCTCTGCTGAAACAAGCCGGAGATGTCGA
AGAGAATCCTGGACCGatggaccagcacacacttgatgtggaggataccgcggatgccagacatccagcaggt
acttcgtgcccctcggatgcggcgctcctcagagataccgggctcctcgcggacgctgcgctcctctcagatactgtgcgcccc
acaaatgccgcgctccccacggatgctgcctaccctgcggttaatgttcgggatcgcgaggccgcgtggccgcctgcactgaa
cttctgttcccgccacccaaagctctatggcctagtcgctttggttttgctgcttctgatcgccgcctgtgttcctatcttcacccgcac
cgagcctcggccagcgctcacaatcaccacctcgcccaacctgggtacccgagagaataatgcagaccaggtcacccctgttt
cccacattggctgccccaacactacacaacagggctctcctgtgttcgccaagctactggctaaaaaccaagcatcgttgtgcaat
acaactctgaactggcacagccaagatggagctgggagctcatacctatctcaaggtctgaggtacgaagaagacaaaaagga
gttggtggtagacagtcccgggctctactacgtatttttggaactgaagctcagtccaacattcacaaacacaggccacaaggtgc
agggctgggtctctcttgttttgcaagcaaagcctcaggtagatgactttgacaacttggccctgacagtggaactgttcccttgctc
catggagaacaagttagtggaccgttcctggagtcaactgttgctcctgaaggctggccaccgcctcagtgtgggtctgagggct
tatctgcatggagcccaggatgcatacagagactgggagctgtcttatcccaacaccaccagctttggactctttcttgtgaaaccc
gacaacccatgggaaagcggtacccagtgcaccaactacgccctgctgaagctggccggcgatgtggagagcaaccccggg
cccgctagcatgaacgctacacactgcatcttggctttgcagctcttcctcatggctgtttctggctgttactgccacggcacagtca
ttgaaagcctagaaagtctgaataactattttaactcaagtggcatagatgtggaagaaaagagtctcttcttggatatctggaggaa
ctggcaaaaggatggtgacatgaaaatcctgcagagccagattatctctttctacctcagactctttgaagtcttgaaagacaatca
ggccatcagcaacaacataagcgtcattgaatcacacctgattactaccttcttcagcaacagcaaggcgaaaaaggatgcattc
atgagtattgccaagtttgaggtcaacaacccacaggtccagcgccaagcattcaatgagctcatccgagtggtccaccagctgt
tgccggaatccagcctcaggaagcggaaaaggagtcgctgctgaGaattcacgcgttaagtcgacaatcaacctctggattaca
aaatttgtgaaagattgactggtattcttaactatgttgctccttttacgctatgtggatacgctgctttaatgcctttgtatcatgctattg
cttcccgtatggctttcattttctcctccttgtataaatcctggttgctgtctctttatgaggagttgtggcccgttgtcaggcaacgtgg
cgtggtgtgcactgtgtttgctgacgcaacccccactggttggggcattgccaccacctgtcagctcctttccgggactttcgctttc
cccctccctattgccacggcggaactcatcgccgcctgccttgcccgctgctggacaggggctcggctgttgggcactgacaatt
ccgtggtgttgtcggggaaatcatcgtcctttccttggctgctcgcctgtgttgccacctggattctgcgcgggacgtccttctgcta
cgtcccttcggccctcaatccagcggaccttccttcccgcggcctgctgccggctctgcggcctcttccgcgtcttcgccttcgcc
ctcagacgagtcggatctccctttgggccgcctccccgcgtcgactttaagaccaatgacGGCCGCAGGAACCCC
TAGTGATGGAGTTGGCCACTCCCTCTCTGCGCGCTCGCTCGCTCACTGAGGCC
GGGCGACCAAAGGTCGCCCGACGCCCGGGCTTTGCCCGGGCGGCCTCAGTGA
GCGAGCGAGCGCGCAGCTGCCTGCAGGGGCGCCTGATGCGGTATTTTCTCCTT
 
ACGCATCTGTGCGGTATTTCACACCGCATACGTCAAAGCAACCATAGTACGC
GCCCTGTAGCGGCGCATTAAGCGCGGCGGGTGTGGTGGTTACGCGCAGCGTG
ACCGCTACACTTGCCAGCGCCTTAGCGCCCGCTCCTTTCGCTTTCTTCCCTTCC
TTTCTCGCCACGTTCGCCGGCTTTCCCCGTCAAGCTCTAAATCGGGGGCTCCC
TTTAGGGTTCCGATTTAGTGCTTTACGGCACCTCGACCCCAAAAAACTTGATT
TGGGTGATGGTTCACGTAGTGGGCCATCGCCCTGATAGACGGTTTTTCGCCCT
TTGACGTTGGAGTCCACGTTCTTTAATAGTGGACTCTTGTTCCAAACTGGAAC
AACACTCAACTCTATCTCGGGCTATTCTTTTGATTTATAAGGGATTTTGCCGAT
TTCGGTCTATTGGTTAAAAAATGAGCTGATTTAACAAAAATTTAACGCGAATT
TTAACAAAATATTAACGTTTACAATTTTATGGTGCACTCTCAGTACAATCTGC
TCTGATGCCGCATAGTTAAGCCAGCCCCGACACCCGCCAACACCCGCTGACG
CGCCCTGACGGGCTTGTCTGCTCCCGGCATCCGCTTACAGACAAGCTGTGACC
GTCTCCGGGAGCTGCATGTGTCAGAGGTTTTCACCGTCATCACCGAAACGCGC
GAGACGAAAGGGCCTCGTGATACGCCTATTTTTATAGGTTAATGTCATGATAA
TAATGGTTTCTTAGACGTCAGGTGGCACTTTTCGGGGAAATGTGCGCGGAACC
CCTATTTGTTTATTTTTCTAAATACATTCAAATATGTATCCGCTCATGAGACAA
TAACCCTGATAAATGCTTCAATAATATTGAAAAAGGAAGAGTATGAGTATTC
AACATTTCCGTGTCGCCCTTATTCCCTTTTTTGCGGCATTTTGCCTTCCTGTTTT
TGCTCACCCAGAAACGCTGGTGAAAGTAAAAGATGCTGAAGATCAGTTGGGT
GCACGAGTGGGTTACATCGAACTGGATCTCAACAGCGGTAAGATCCTTGAGA
GTTTTCGCCCCGAAGAACGTTTTCCAATGATGAGCACTTTTAAAGTTCTGCTA
TGTGGCGCGGTATTATCCCGTATTGACGCCGGGCAAGAGCAACTCGGTCGCC
GCATACACTATTCTCAGAATGACTTGGTTGAGTACTCACCAGTCACAGAAAA
GCATCTTACGGATGGCATGACAGTAAGAGAATTATGCAGTGCTGCCATAACC
ATGAGTGATAACACTGCGGCCAACTTACTTCTGACAACGATCGGAGGACCGA
AGGAGCTAACCGCTTTTTTGCACAACATGGGGGATCATGTAACTCGCCTTGAT
CGTTGGGAACCGGAGCTGAATGAAGCCATACCAAACGACGAGCGTGACACCA
CGATGCCTGTAGCAATGGCAACAACGTTGCGCAAACTATTAACTGGCGAACT
ACTTACTCTAGCTTCCCGGCAACAATTAATAGACTGGATGGAGGCGGATAAA
GTTGCAGGACCACTTCTGCGCTCGGCCCTTCCGGCTGGCTGGTTTATTGCTGA
TAAATCTGGAGCCGGTGAGCGTGGAAGCCGCGGTATCATTGCAGCACTGGGG
CCAGATGGTAAGCCCTCCCGTATCGTAGTTATCTACACGACGGGGAGTCAGG
CAACTATGGATGAACGAAATAGACAGATCGCTGAGATAGGTGCCTCACTGAT
TAAGCATTGGTAACTGTCAGACCAAGTTTACTCATATATACTTTAGATTGATT
TAAAACTTCATTTTTAATTTAAAAGGATCTAGGTGAAGATCCTTTTTGATAAT
CTCATGACCAAAATCCCTTAACGTGAGTTTTCGTTCCACTGAGCGTCAGACCC
CGTAGAAAAGATCAAAGGATCTTCTTGAGATCCTTTTTTTCTGCGCGTAATCT
GCTGCTTGCAAACAAAAAAACCACCGCTACCAGCGGTGGTTTGTTTGCCGGA
TCAAGAGCTACCAACTCTTTTTCCGAAGGTAACTGGCTTCAGCAGAGCGCAG
ATACCAAATACTGTTCTTCTAGTGTAGCCGTAGTTAGGCCACCACTTCAAGAA
CTCTGTAGCACCGCCTACATACCTCGCTCTGCTAATCCTGTTACCAGTGGCTG
CTGCCAGTGGCGATAAGTCGTGTCTTACCGGGTTGGACTCAAGACGATAGTTA
CCGGATAAGGCGCAGCGGTCGGGCTGAACGGGGGGTTCGTGCACACAGCCCA
GCTTGGAGCGAACGACCTACACCGAACTGAGATACCTACAGCGTGAGCTATG
AGAAAGCGCCACGCTTCCCGAAGGGAGAAAGGCGGACAGGTATCCGGTAAG
CGGCAGGGTCGGAACAGGAGAGCGCACGAGGGAGCTTCCAGGGGGAAACGC
CTGGTATCTTTATAGTCCTGTCGGGTTTCGCCACCTCTGACTTGAGCGTCGATT
TTTGTGATGCTCGTCAGGGGGGCGGAGCCTATGGAAAAACGCCAGCAACGCG
GCCTTTTTACGGTTCCTGGCCTTTTGCTGGCCTTTTGCTCACATGT
**pGW118-pAAV_EFSLTR-Ifng-P2A-Cd80-T2A-light-E2A-41BBL-sPA (SEQ ID NO: 46)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atgaacgctacacactgcatcttggctttgcagctcttcctcatggctgtttctggctgttactgccacggcacagtcattgaaagcct
agaaagtctgaataactattttaactcaagtggcatagatgtggaagaaaagagtctcttcttggatatctggaggaactggcaaaa
ggatggtgacatgaaaatcctgcagagccagattatctctttctacctcagactctttgaagtcttgaaagacaatcaggccatcag
caacaacataagcgtcattgaatcacacctgattactaccttcttcagcaacagcaaggcgaaaaaggatgcattcatgagtattg
ccaagtttgaggtcaacaacccacaggtccagcgccaagcattcaatgagctcatccgagtggtccaccagctgttgccggaat
ccagcctcaggaagcggaaaaggagtcgctgcGGATCCGGCGCAACAAACTTCTCTCTGCTGAA
ACAAGCCGGAGATGTCGAAGAGAATCCTGGACCGatggcttgcaattgtcagttgatgcaggat
acaccactcctcaagtttccatgtccaaggctcattcttctctttgtgctgctgattcgtctttcacaagtgtcttcagatgttgatgaac
aactgtccaagtcagtgaaagataaggtattgctgccttgccgttacaactctcctcatgaagatgagtctgaagaccgaatctact
ggcaaaaacatgacaaagtggtgctgtctgtcattgTtgggaaactaaaagtgtggcccgagtataagaaccggactttatatga
caacactacctactctcttatcatcctgggcctggtcctttcagaccggggcacatacagctgtgtcgttcaaaagaaggaaagag
gaacgtatgaagttaaacacttggctttagtaaagttgtccatcaaagctgacttctctacccccaacataactgagtctggaaaccc
atctgcagacactaaaaggattacctgctttgcttccgggggtttcccaaagcctcgcttctcttggttggaaaatggaagagaatta
cctggcatcaatacgacaatttcccaggatcctgaatctgaattgtacaccattagtagccaactagatttcaatacgactcgcaac
cacaccattaagtgtctcattaaatatggagatgctcacgtgtcagaggacttcacctgggaaaaacccccagaagaccctcctg
atagcaagaacacacttgtgctctttggggcaggattcggcgcagtaataacagtcgtcgtcatcgttgtcatcatcaaatgcttctg
taagcacagaagctgtttcagaagaaatgaggcaagcagagaaacaaacaacagccttaccttcgggcctgaagaagcattag
ctgaacagaccgtcttccttcgtacgGGCAGTGGAGAGGGCAGAGGAAGTCTGCTAACATGC
GGTGACGTCGAGGAGAATCCTGGCCCAatggagagtgtggtacagccttcagtgtttgtggtggatgga
cagacggacatcccattcaggcggctggaacagaaccaccggagacggcgctgtggcactgtccaggtcagcctggccctgg
tgctgctgctaggtgctgggctggccactcagggctggtttctcctgagactgcatcaacgtcttggagacatagtagctcatctgc
cagatggaggcaaaggctcctgggagaagctgatacaagatcaacgatctcaccaggccaacccagcagcacatcttacagg
agccaacgccagcttgataggtattggtggacctctgttatgggagacacgacttggcctggccttcttgaggggcttgacgtatc
atgatggggccctggtgaccatggagcccggttactactatgtgtactccaaagtgcagctgagcggcgtgggctgcccccagg
ggctggccaatggcctccccatcacccatggactatacaagcgcacatcccgctacccgaaggagttagaactgctggtcagtc
ggcggtcaccctgtggccgggccaacagctcccgagtctggtgggacagcagcttcctgggcggcgtggtacatctggaggc
tggggaagaggtggtggtccgcgtgcctggaaaccgcctggtcagaccacgtgacggcaccaggtcctatttcggagctttcat
ggtcactagcagcggtacccagtgcaccaactacgccctgctgaagctggccggcgatgtggagagcaaccccgggcccatg
gaccagcacacacttgatgtggaggataccgcggatgccagacatccagcaggtacttcgtgcccctcggatgcggcgctcct
cagagataccgggctcctcgcggacgctgcgctcctctcagatactgtgcgccccacaaatgccgcgctccccacggatgctg
cctaccctgcggttaatgttcgggatcgcgaggccgcgtggccgcctgcactgaacttctgttcccgccacccaaagctctatgg
cctagtcgctttggttttgctgcttctgatcgccgcctgtgttcctatcttcacccgcaccgagcctcggccagcgctcacaatcacc
acctcgcccaacctgggtacccgagagaataatgcagaccaggtcacccctgtttcccacattggctgccccaacactacacaa
cagggctctcctgtgttcgccaagctactggctaaaaaccaagcatcgttgtgcaatacaactctgaactggcacagccaagatg
gagctgggagctcatacctatctcaaggtctgaggtacgaagaagacaaaaaggagttggtggtagacagtcccgggctctact
 
acgtatttttggaactgaagctcagtccaacattcacaaacacaggccacaaggtgcagggctgggtctctcttgttttgcaagcaa
agcctcaggtagatgactttgacaacttggccctgacagtggaactgttcccttgctccatggagaacaagttagtggaccgttcct
ggagtcaactgttgctcctgaaggctggccaccgcctcagtgtgggtctgagggcttatctgcatggagcccaggatgcataca
gagactgggagctgtcttatcccaacaccaccagctttggactctttcttgtgaaacccgacaacccatgggaatgaGAATT
CAATAAAAGATCTTTATTTTCATTAGATCTGTGTGTTGGTTTTTTGTGTGCGGC
CGCAGGAACCCCTAGTGATGGAGTTGGCCACTCCCTCTCTGCGCGCTCGCTCG
CTCACTGAGGCCGGGCGACCAAAGGTCGCCCGACGCCCGGGCTTTGCCCGGG
CGGCCTCAGTGAGCGAGCGAGCGCGCAGCTGCCTGCAGGGGCGCCTGATGCG
GTATTTTCTCCTTACGCATCTGTGCGGTATTTCACACCGCATACGTCAAAGCA
ACCATAGTACGCGCCCTGTAGCGGCGCATTAAGCGCGGCGGGTGTGGTGGTT
ACGCGCAGCGTGACCGCTACACTTGCCAGCGCCTTAGCGCCCGCTCCTTTCGC
TTTCTTCCCTTCCTTTCTCGCCACGTTCGCCGGCTTTCCCCGTCAAGCTCTAAA
TCGGGGGCTCCCTTTAGGGTTCCGATTTAGTGCTTTACGGCACCTCGACCCCA
AAAAACTTGATTTGGGTGATGGTTCACGTAGTGGGCCATCGCCCTGATAGAC
GGTTTTTCGCCCTTTGACGTTGGAGTCCACGTTCTTTAATAGTGGACTCTTGTT
CCAAACTGGAACAACACTCAACTCTATCTCGGGCTATTCTTTTGATTTATAAG
GGATTTTGCCGATTTCGGTCTATTGGTTAAAAAATGAGCTGATTTAACAAAAA
TTTAACGCGAATTTTAACAAAATATTAACGTTTACAATTTTATGGTGCACTCT
CAGTACAATCTGCTCTGATGCCGCATAGTTAAGCCAGCCCCGACACCCGCCA
ACACCCGCTGACGCGCCCTGACGGGCTTGTCTGCTCCCGGCATCCGCTTACAG
ACAAGCTGTGACCGTCTCCGGGAGCTGCATGTGTCAGAGGTTTTCACCGTCAT
CACCGAAACGCGCGAGACGAAAGGGCCTCGTGATACGCCTATTTTTATAGGT
TAATGTCATGATAATAATGGTTTCTTAGACGTCAGGTGGCACTTTTCGGGGAA
ATGTGCGCGGAACCCCTATTTGTTTATTTTTCTAAATACATTCAAATATGTATC
CGCTCATGAGACAATAACCCTGATAAATGCTTCAATAATATTGAAAAAGGAA
GAGTATGAGTATTCAACATTTCCGTGTCGCCCTTATTCCCTTTTTTGCGGCATT
TTGCCTTCCTGTTTTTGCTCACCCAGAAACGCTGGTGAAAGTAAAAGATGCTG
AAGATCAGTTGGGTGCACGAGTGGGTTACATCGAACTGGATCTCAACAGCGG
TAAGATCCTTGAGAGTTTTCGCCCCGAAGAACGTTTTCCAATGATGAGCACTT
TTAAAGTTCTGCTATGTGGCGCGGTATTATCCCGTATTGACGCCGGGCAAGAG
CAACTCGGTCGCCGCATACACTATTCTCAGAATGACTTGGTTGAGTACTCACC
AGTCACAGAAAAGCATCTTACGGATGGCATGACAGTAAGAGAATTATGCAGT
GCTGCCATAACCATGAGTGATAACACTGCGGCCAACTTACTTCTGACAACGAT
CGGAGGACCGAAGGAGCTAACCGCTTTTTTGCACAACATGGGGGATCATGTA
ACTCGCCTTGATCGTTGGGAACCGGAGCTGAATGAAGCCATACCAAACGACG
AGCGTGACACCACGATGCCTGTAGCAATGGCAACAACGTTGCGCAAACTATT
AACTGGCGAACTACTTACTCTAGCTTCCCGGCAACAATTAATAGACTGGATGG
AGGCGGATAAAGTTGCAGGACCACTTCTGCGCTCGGCCCTTCCGGCTGGCTG
GTTTATTGCTGATAAATCTGGAGCCGGTGAGCGTGGAAGCCGCGGTATCATTG
CAGCACTGGGGCCAGATGGTAAGCCCTCCCGTATCGTAGTTATCTACACGAC
GGGGAGTCAGGCAACTATGGATGAACGAAATAGACAGATCGCTGAGATAGG
TGCCTCACTGATTAAGCATTGGTAACTGTCAGACCAAGTTTACTCATATATAC
TTTAGATTGATTTAAAACTTCATTTTTAATTTAAAAGGATCTAGGTGAAGATC
CTTTTTGATAATCTCATGACCAAAATCCCTTAACGTGAGTTTTCGTTCCACTGA
GCGTCAGACCCCGTAGAAAAGATCAAAGGATCTTCTTGAGATCCTTTTTTTCT
GCGCGTAATCTGCTGCTTGCAAACAAAAAAACCACCGCTACCAGCGGTGGTT
TGTTTGCCGGATCAAGAGCTACCAACTCTTTTTCCGAAGGTAACTGGCTTCAG
CAGAGCGCAGATACCAAATACTGTTCTTCTAGTGTAGCCGTAGTTAGGCCACC
ACTTCAAGAACTCTGTAGCACCGCCTACATACCTCGCTCTGCTAATCCTGTTA
CCAGTGGCTGCTGCCAGTGGCGATAAGTCGTGTCTTACCGGGTTGGACTCAAG
 
ACGATAGTTACCGGATAAGGCGCAGCGGTCGGGCTGAACGGGGGGTTCGTGC
ACACAGCCCAGCTTGGAGCGAACGACCTACACCGAACTGAGATACCTACAGC
GTGAGCTATGAGAAAGCGCCACGCTTCCCGAAGGGAGAAAGGCGGACAGGT
ATCCGGTAAGCGGCAGGGTCGGAACAGGAGAGCGCACGAGGGAGCTTCCAG
GGGGAAACGCCTGGTATCTTTATAGTCCTGTCGGGTTTCGCCACCTCTGACTT
GAGCGTCGATTTTTGTGATGCTCGTCAGGGGGGCGGAGCCTATGGAAAAACG
CCAGCAACGCGGCCTTTTTACGGTTCCTGGCCTTTTGCTGGCCTTTTGCTCACA
TGT
**pGW119-pAAV_EFSLTR-Ifng-STOP-P2A-Cd80-T2A-light-P2A-E2A-41BBL-sPA (SEQ ID NO: 47)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atgaacgctacacactgcatcttggctttgcagctcttcctcatggctgtttctggctgttactgccacggcacagtcattgaaagcct
agaaagtctgaataactattttaactcaagtggcatagatgtggaagaaaagagtctcttcttggatatctggaggaactggcaaaa
ggatggtgacatgaaaatcctgcagagccagattatctctttctacctcagactctttgaagtcttgaaagacaatcaggccatcag
caacaacataagcgtcattgaatcacacctgattactaccttcttcagcaacagcaaggcgaaaaaggatgcattcatgagtattg
ccaagtttgaggtcaacaacccacaggtccagcgccaagcattcaatgagctcatccgagtggtccaccagctgttgccggaat
ccagcctcaggaagcggaaaaggagtcgctgcTGAATCCGGCGCAACAAACTTCTCTCTGCTGA
AACAAGCCGGAGATGTCGAAGAGAATCCTGGACCGatggcttgcaattgtcagttgatgcagg
atacaccactcctcaagtttccatgtccaaggctcattcttctctttgtgctgctgattcgtctttcacaagtgtcttcagatgttgatgaa
caactgtccaagtcagtgaaagataaggtattgctgccttgccgttacaactctcctcatgaagatgagtctgaagaccgaatctac
tggcaaaaacatgacaaagtggtgctgtctgtcattgTtgggaaactaaaagtgtggcccgagtataagaaccggactttatatg
acaacactacctactctcttatcatcctgggcctggtcctttcagaccggggcacatacagctgtgtcgttcaaaagaaggaaaga
ggaacgtatgaagttaaacacttggctttagtaaagttgtccatcaaagctgacttctctacccccaacataactgagtctggaaac
ccatctgcagacactaaaaggattacctgctttgcttccgggggtttcccaaagcctcgcttctcttggttggaaaatggaagagaa
ttacctggcatcaatacgacaatttcccaggatcctgaatctgaattgtacaccattagtagccaactagatttcaatacgactcgca
accacaccattaagtgtctcattaaatatggagatgctcacgtgtcagaggacttcacctgggaaaaacccccagaagaccctcct
gatagcaagaacacacttgtgctctttggggcaggattcggcgcagtaataacagtcgtcgtcatcgttgtcatcatcaaatgcttct
gtaagcacagaagctgtttcagaagaaatgaggcaagcagagaaacaaacaacagccttaccttcgggcctgaagaagcatta
gctgaacagaccgtcttccttcgtacgGGCAGTGGAGAGGGCAGAGGAAGTCTGCTAACATG
CGGTGACGTCGAGGAGAATCCTGGCCCAatggagagtgtggtacagccttcagtgtttgtggtggatg
gacagacggacatcccattcaggcggctggaacagaaccaccggagacggcgctgtggcactgtccaggtcagcctggccct
ggtgctgctgctaggtgctgggctggccactcagggctggtttctcctgagactgcatcaacgtcttggagacatagtagctcatc
tgccagatggaggcaaaggctcctgggagaagctgatacaagatcaacgatctcaccaggccaacccagcagcacatcttaca
ggagccaacgccagcttgataggtattggtggacctctgttatgggagacacgacttggcctggccttcttgaggggcttgacgt
atcatgatggggccctggtgaccatggagcccggttactactatgtgtactccaaagtgcagctgagcggcgtgggctgccccc
aggggctggccaatggcctccccatcacccatggactatacaagcgcacatcccgctacccgaaggagttagaactgctggtc
agtcggcggtcaccctgtggccgggccaacagctcccgagtctggtgggacagcagcttcctgggcggcgtggtacatctgga
 
ggctggggaagaggtggtggtccgcgtgcctggaaaccgcctggtcagaccacgtgacggcaccaggtcctatttcggagctt
tcatggtcactagcagcggtacccagtgcaccaactacgccctgctgaagctggccggcgatgtggagagcaaccccgggcc
catggaccagcacacacttgatgtggaggataccgcggatgccagacatccagcaggtacttcgtgcccctcggatgcggcgc
tcctcagagataccgggctcctcgcggacgctgcgctcctctcagatactgtgcgccccacaaatgccgcgctccccacggatg
ctgcctaccctgcggttaatgttcgggatcgcgaggccgcgtggccgcctgcactgaacttctgttcccgccacccaaagctctat
ggcctagtcgctttggttttgctgcttctgatcgccgcctgtgttcctatcttcacccgcaccgagcctcggccagcgctcacaatca
ccacctcgcccaacctgggtacccgagagaataatgcagaccaggtcacccctgtttcccacattggctgccccaacactacac
aacagggctctcctgtgttcgccaagctactggctaaaaaccaagcatcgttgtgcaatacaactctgaactggcacagccaaga
tggagctgggagctcatacctatctcaaggtctgaggtacgaagaagacaaaaaggagttggtggtagacagtcccgggctcta
ctacgtatttttggaactgaagctcagtccaacattcacaaacacaggccacaaggtgcagggctgggtctctcttgttttgcaagc
aaagcctcaggtagatgactttgacaacttggccctgacagtggaactgttcccttgctccatggagaacaagttagtggaccgtt
cctggagtcaactgttgctcctgaaggctggccaccgcctcagtgtgggtctgagggcttatctgcatggagcccaggatgcata
cagagactgggagctgtcttatcccaacaccaccagctttggactctttcttgtgaaacccgacaacccatgggaatgaGAAT
TCAATAAAAGATCTTTATTTTCATTAGATCTGTGTGTTGGTTTTTTGTGTGCGG
CCGCAGGAACCCCTAGTGATGGAGTTGGCCACTCCCTCTCTGCGCGCTCGCTC
GCTCACTGAGGCCGGGCGACCAAAGGTCGCCCGACGCCCGGGCTTTGCCCGG
GCGGCCTCAGTGAGCGAGCGAGCGCGCAGCTGCCTGCAGGGGCGCCTGATGC
GGTATTTTCTCCTTACGCATCTGTGCGGTATTTCACACCGCATACGTCAAAGC
AACCATAGTACGCGCCCTGTAGCGGCGCATTAAGCGCGGCGGGTGTGGTGGT
TACGCGCAGCGTGACCGCTACACTTGCCAGCGCCTTAGCGCCCGCTCCTTTCG
CTTTCTTCCCTTCCTTTCTCGCCACGTTCGCCGGCTTTCCCCGTCAAGCTCTAA
ATCGGGGGCTCCCTTTAGGGTTCCGATTTAGTGCTTTACGGCACCTCGACCCC
AAAAAACTTGATTTGGGTGATGGTTCACGTAGTGGGCCATCGCCCTGATAGA
CGGTTTTTCGCCCTTTGACGTTGGAGTCCACGTTCTTTAATAGTGGACTCTTGT
TCCAAACTGGAACAACACTCAACTCTATCTCGGGCTATTCTTTTGATTTATAA
GGGATTTTGCCGATTTCGGTCTATTGGTTAAAAAATGAGCTGATTTAACAAAA
ATTTAACGCGAATTTTAACAAAATATTAACGTTTACAATTTTATGGTGCACTC
TCAGTACAATCTGCTCTGATGCCGCATAGTTAAGCCAGCCCCGACACCCGCCA
ACACCCGCTGACGCGCCCTGACGGGCTTGTCTGCTCCCGGCATCCGCTTACAG
ACAAGCTGTGACCGTCTCCGGGAGCTGCATGTGTCAGAGGTTTTCACCGTCAT
CACCGAAACGCGCGAGACGAAAGGGCCTCGTGATACGCCTATTTTTATAGGT
TAATGTCATGATAATAATGGTTTCTTAGACGTCAGGTGGCACTTTTCGGGGAA
ATGTGCGCGGAACCCCTATTTGTTTATTTTTCTAAATACATTCAAATATGTATC
CGCTCATGAGACAATAACCCTGATAAATGCTTCAATAATATTGAAAAAGGAA
GAGTATGAGTATTCAACATTTCCGTGTCGCCCTTATTCCCTTTTTTGCGGCATT
TTGCCTTCCTGTTTTTGCTCACCCAGAAACGCTGGTGAAAGTAAAAGATGCTG
AAGATCAGTTGGGTGCACGAGTGGGTTACATCGAACTGGATCTCAACAGCGG
TAAGATCCTTGAGAGTTTTCGCCCCGAAGAACGTTTTCCAATGATGAGCACTT
TTAAAGTTCTGCTATGTGGCGCGGTATTATCCCGTATTGACGCCGGGCAAGAG
CAACTCGGTCGCCGCATACACTATTCTCAGAATGACTTGGTTGAGTACTCACC
AGTCACAGAAAAGCATCTTACGGATGGCATGACAGTAAGAGAATTATGCAGT
GCTGCCATAACCATGAGTGATAACACTGCGGCCAACTTACTTCTGACAACGAT
CGGAGGACCGAAGGAGCTAACCGCTTTTTTGCACAACATGGGGGATCATGTA
ACTCGCCTTGATCGTTGGGAACCGGAGCTGAATGAAGCCATACCAAACGACG
AGCGTGACACCACGATGCCTGTAGCAATGGCAACAACGTTGCGCAAACTATT
AACTGGCGAACTACTTACTCTAGCTTCCCGGCAACAATTAATAGACTGGATGG
AGGCGGATAAAGTTGCAGGACCACTTCTGCGCTCGGCCCTTCCGGCTGGCTG
GTTTATTGCTGATAAATCTGGAGCCGGTGAGCGTGGAAGCCGCGGTATCATTG
CAGCACTGGGGCCAGATGGTAAGCCCTCCCGTATCGTAGTTATCTACACGAC
GGGGAGTCAGGCAACTATGGATGAACGAAATAGACAGATCGCTGAGATAGG
 
TGCCTCACTGATTAAGCATTGGTAACTGTCAGACCAAGTTTACTCATATATAC
TTTAGATTGATTTAAAACTTCATTTTTAATTTAAAAGGATCTAGGTGAAGATC
CTTTTTGATAATCTCATGACCAAAATCCCTTAACGTGAGTTTTCGTTCCACTGA
GCGTCAGACCCCGTAGAAAAGATCAAAGGATCTTCTTGAGATCCTTTTTTTCT
GCGCGTAATCTGCTGCTTGCAAACAAAAAAACCACCGCTACCAGCGGTGGTT
TGTTTGCCGGATCAAGAGCTACCAACTCTTTTTCCGAAGGTAACTGGCTTCAG
CAGAGCGCAGATACCAAATACTGTTCTTCTAGTGTAGCCGTAGTTAGGCCACC
ACTTCAAGAACTCTGTAGCACCGCCTACATACCTCGCTCTGCTAATCCTGTTA
CCAGTGGCTGCTGCCAGTGGCGATAAGTCGTGTCTTACCGGGTTGGACTCAAG
ACGATAGTTACCGGATAAGGCGCAGCGGTCGGGCTGAACGGGGGGTTCGTGC
ACACAGCCCAGCTTGGAGCGAACGACCTACACCGAACTGAGATACCTACAGC
GTGAGCTATGAGAAAGCGCCACGCTTCCCGAAGGGAGAAAGGCGGACAGGT
ATCCGGTAAGCGGCAGGGTCGGAACAGGAGAGCGCACGAGGGAGCTTCCAG
GGGGAAACGCCTGGTATCTTTATAGTCCTGTCGGGTTTCGCCACCTCTGACTT
GAGCGTCGATTTTTGTGATGCTCGTCAGGGGGGCGGAGCCTATGGAAAAACG
CCAGCAACGCGGCCTTTTTACGGTTCCTGGCCTTTTGCTGGCCTTTTGCTCACA
TGT
**pGW122-pAAV_EFS-LTR-IL23-T2A-41BBL-P2A-IFNg-WPRE (SEQ ID NO: 48)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
ATGTGTCCTCAGAAGCTAACCATCTCCTGGTTTGCCATCGTTTTGCTGGTGTCT
CCACTCATGGCCATGTGGGAGCTGGAGAAAGACGTTTATGTTGTAGAGGTGG
ACTGGACTCCCGATGCCCCTGGAGAAACAGTGAACCTCACCTGTGACACGCC
TGAAGAAGATGACATCACCTGGACCTCAGACCAGAGACATGGAGTCATAGGC
TCTGGAAAGACCCTGACCATCACTGTCAAAGAGTTTCTAGATGCTGGCCAGTA
CACCTGCCACAAAGGAGGCGAGACTCTGAGCCACTCACATCTGCTGCTCCAC
AAGAAGGAAAATGGAATTTGGTCCACTGAAATTTTAAAAAATTTCAAAAACA
AGACTTTCCTGAAGTGTGAAGCACCAAATTACTCCGGACGGTTCACGTGCTCA
TGGCTGGTGCAAAGAAACATGGACTTGAAGTTCAACATCAAGAGCAGTAGCA
GTTCCCCTGACTCTCGGGCAGTGACATGTGGAATGGCGTCTCTGTCTGCAGAG
AAGGTCACACTGGACCAAAGGGACTATGAGAAGTATTCAGTGTCCTGCCAGG
AGGATGTCACCTGCCCAACTGCCGAGGAGACCCTGCCCATTGAACTGGCGTT
GGAAGCACGGCAGCAGAATAAATATGAGAACTACAGCACCAGCTTCTTCATC
AGGGACATCATCAAACCAGACCCGCCCAAGAACTTGCAGATGAAGCCTTTGA
AGAACTCACAGGTGGAGGTCAGCTGGGAGTACCCTGACTCCTGGAGCACTCC
CCATTCCTACTTCTCCCTCAAGTTCTTTGTTCGAATCCAGCGCAAGAAAGAAA
AGATGAAGGAGACAGAGGAGGGGTGTAACCAGAAAGGTGCGTTCCTCGTAG
AGAAGACATCTACCGAAGTCCAATGCAAAGGCGGGAATGTCTGCGTGCAAGC
 
TCAGGATCGCTATTACAATTCCTCATGCAGCAAGTGGGCATGTGTTCCCTGCA
GGGTCCGATCCGGCAGTGGAGAGGGCAGAGGAAGTCTGCTAACATGCGGTGA
CGTCGAGGAGAATCCTGGCCCAatggaccagcacacacttgatgtggaggataccgcggatgccagacat
ccagcaggtacttcgtgcccctcggatgcggcgctcctcagagataccgggctcctcgcggacgctgcgctcctctcagatact
gtgcgccccacaaatgccgcgctccccacggatgctgcctaccctgcggttaatgttcgggatcgcgaggccgcgtggccgcc
tgcactgaacttctgttcccgccacccaaagctctatggcctagtcgctttggttttgctgcttctgatcgccgcctgtgttcctatcttc
acccgcaccgagcctcggccagcgctcacaatcaccacctcgcccaacctgggtacccgagagaataatgcagaccaggtca
cccctgtttcccacattggctgccccaacactacacaacagggctctcctgtgttcgccaagctactggctaaaaaccaagcatcg
ttgtgcaatacaactctgaactggcacagccaagatggagctgggagctcatacctatctcaaggtctgaggtacgaagaagac
aaaaaggagttggtggtagacagtcccgggctctactacgtatttttggaactgaagctcagtccaacattcacaaacacaggcc
acaaggtgcagggctgggtctctcttgttttgcaagcaaagcctcaggtagatgactttgacaacttggccctgacagtggaactg
ttcccttgctccatggagaacaagttagtggaccgttcctggagtcaactgttgctcctgaaggctggccaccgcctcagtgtggg
tctgagggcttatctgcatggagcccaggatgcatacagagactgggagctgtcttatcccaacaccaccagctttggactctttct
tgtgaaacccgacaacccatgggaaGGATCCGGCGCAACAAACTTCTCTCTGCTGAAACAA
GCCGGAGATGTCGAAGAGAATCCTGGACCGgctagcatgaacgctacacactgcatcttggctttgc
agctcttcctcatggctgtttctggctgttactgccacggcacagtcattgaaagcctagaaagtctgaataactattttaactcaagt
ggcatagatgtggaagaaaagagtctcttcttggatatctggaggaactggcaaaaggatggtgacatgaaaatcctgcagagc
cagattatctctttctacctcagactctttgaagtcttgaaagacaatcaggccatcagcaacaacataagcgtcattgaatcacacc
tgattactaccttcttcagcaacagcaaggcgaaaaaggatgcattcatgagtattgccaagtttgaggtcaacaacccacaggtc
cagcgccaagcattcaatgagctcatccgagtggtccaccagctgttgccggaatccagcctcaggaagcggaaaaggagtcg
ctgctgaGaattcacgcgttaagtcgacaatcaacctctggattacaaaatttgtgaaagattgactggtattcttaactatgttgctc
cttttacgctatgtggatacgctgctttaatgcctttgtatcatgctattgcttcccgtatggctttcattttctcctccttgtataaatcctg
gttgctgtctctttatgaggagttgtggcccgttgtcaggcaacgtggcgtggtgtgcactgtgtttgctgacgcaacccccactgg
ttggggcattgccaccacctgtcagctcctttccgggactttcgctttccccctccctattgccacggcggaactcatcgccgcctg
ccttgcccgctgctggacaggggctcggctgttgggcactgacaattccgtggtgttgtcggggaaatcatcgtcctttccttggct
gctcgcctgtgttgccacctggattctgcgcgggacgtccttctgctacgtcccttcggccctcaatccagcggaccttccttcccg
cggcctgctgccggctctgcggcctcttccgcgtcttcgccttcgccctcagacgagtcggatctccctttgggccgcctccccg
cgtcgactttaagaccaatgacGGCCGCAGGAACCCCTAGTGATGGAGTTGGCCACTCCCT
CTCTGCGCGCTCGCTCGCTCACTGAGGCCGGGCGACCAAAGGTCGCCCGACG
CCCGGGCTTTGCCCGGGCGGCCTCAGTGAGCGAGCGAGCGCGCAGCTGCCTG
CAGGGGCGCCTGATGCGGTATTTTCTCCTTACGCATCTGTGCGGTATTTCACA
CCGCATACGTCAAAGCAACCATAGTACGCGCCCTGTAGCGGCGCATTAAGCG
CGGCGGGTGTGGTGGTTACGCGCAGCGTGACCGCTACACTTGCCAGCGCCTT
AGCGCCCGCTCCTTTCGCTTTCTTCCCTTCCTTTCTCGCCACGTTCGCCGGCTT
TCCCCGTCAAGCTCTAAATCGGGGGCTCCCTTTAGGGTTCCGATTTAGTGCTT
TACGGCACCTCGACCCCAAAAAACTTGATTTGGGTGATGGTTCACGTAGTGG
GCCATCGCCCTGATAGACGGTTTTTCGCCCTTTGACGTTGGAGTCCACGTTCTT
TAATAGTGGACTCTTGTTCCAAACTGGAACAACACTCAACTCTATCTCGGGCT
ATTCTTTTGATTTATAAGGGATTTTGCCGATTTCGGTCTATTGGTTAAAAAATG
AGCTGATTTAACAAAAATTTAACGCGAATTTTAACAAAATATTAACGTTTACA
ATTTTATGGTGCACTCTCAGTACAATCTGCTCTGATGCCGCATAGTTAAGCCA
GCCCCGACACCCGCCAACACCCGCTGACGCGCCCTGACGGGCTTGTCTGCTCC
CGGCATCCGCTTACAGACAAGCTGTGACCGTCTCCGGGAGCTGCATGTGTCA
GAGGTTTTCACCGTCATCACCGAAACGCGCGAGACGAAAGGGCCTCGTGATA
CGCCTATTTTTATAGGTTAATGTCATGATAATAATGGTTTCTTAGACGTCAGG
TGGCACTTTTCGGGGAAATGTGCGCGGAACCCCTATTTGTTTATTTTTCTAAAT
ACATTCAAATATGTATCCGCTCATGAGACAATAACCCTGATAAATGCTTCAAT
AATATTGAAAAAGGAAGAGTATGAGTATTCAACATTTCCGTGTCGCCCTTATT
CCCTTTTTTGCGGCATTTTGCCTTCCTGTTTTTGCTCACCCAGAAACGCTGGTG
AAAGTAAAAGATGCTGAAGATCAGTTGGGTGCACGAGTGGGTTACATCGAAC
 
TGGATCTCAACAGCGGTAAGATCCTTGAGAGTTTTCGCCCCGAAGAACGTTTT
CCAATGATGAGCACTTTTAAAGTTCTGCTATGTGGCGCGGTATTATCCCGTAT
TGACGCCGGGCAAGAGCAACTCGGTCGCCGCATACACTATTCTCAGAATGAC
TTGGTTGAGTACTCACCAGTCACAGAAAAGCATCTTACGGATGGCATGACAG
TAAGAGAATTATGCAGTGCTGCCATAACCATGAGTGATAACACTGCGGCCAA
CTTACTTCTGACAACGATCGGAGGACCGAAGGAGCTAACCGCTTTTTTGCACA
ACATGGGGGATCATGTAACTCGCCTTGATCGTTGGGAACCGGAGCTGAATGA
AGCCATACCAAACGACGAGCGTGACACCACGATGCCTGTAGCAATGGCAACA
ACGTTGCGCAAACTATTAACTGGCGAACTACTTACTCTAGCTTCCCGGCAACA
ATTAATAGACTGGATGGAGGCGGATAAAGTTGCAGGACCACTTCTGCGCTCG
GCCCTTCCGGCTGGCTGGTTTATTGCTGATAAATCTGGAGCCGGTGAGCGTGG
AAGCCGCGGTATCATTGCAGCACTGGGGCCAGATGGTAAGCCCTCCCGTATC
GTAGTTATCTACACGACGGGGAGTCAGGCAACTATGGATGAACGAAATAGAC
AGATCGCTGAGATAGGTGCCTCACTGATTAAGCATTGGTAACTGTCAGACCA
AGTTTACTCATATATACTTTAGATTGATTTAAAACTTCATTTTTAATTTAAAAG
GATCTAGGTGAAGATCCTTTTTGATAATCTCATGACCAAAATCCCTTAACGTG
AGTTTTCGTTCCACTGAGCGTCAGACCCCGTAGAAAAGATCAAAGGATCTTCT
TGAGATCCTTTTTTTCTGCGCGTAATCTGCTGCTTGCAAACAAAAAAACCACC
GCTACCAGCGGTGGTTTGTTTGCCGGATCAAGAGCTACCAACTCTTTTTCCGA
AGGTAACTGGCTTCAGCAGAGCGCAGATACCAAATACTGTTCTTCTAGTGTAG
CCGTAGTTAGGCCACCACTTCAAGAACTCTGTAGCACCGCCTACATACCTCGC
TCTGCTAATCCTGTTACCAGTGGCTGCTGCCAGTGGCGATAAGTCGTGTCTTA
CCGGGTTGGACTCAAGACGATAGTTACCGGATAAGGCGCAGCGGTCGGGCTG
AACGGGGGGTTCGTGCACACAGCCCAGCTTGGAGCGAACGACCTACACCGAA
CTGAGATACCTACAGCGTGAGCTATGAGAAAGCGCCACGCTTCCCGAAGGGA
GAAAGGCGGACAGGTATCCGGTAAGCGGCAGGGTCGGAACAGGAGAGCGCA
CGAGGGAGCTTCCAGGGGGAAACGCCTGGTATCTTTATAGTCCTGTCGGGTTT
CGCCACCTCTGACTTGAGCGTCGATTTTTGTGATGCTCGTCAGGGGGGCGGAG
CCTATGGAAAAACGCCAGCAACGCGGCCTTTTTACGGTTCCTGGCCTTTTGCT
GGCCTTTTGCTCACATGT
**pGW127b-pAAV_EFS-LTR-hIFNg-P2A-hIL23-T2A-h41BBL-WPRE (SEQ ID NO: 49)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
ATGAAATATACAAGTTATATCTTGGCTTTTCAGCTCTGCATCGTTTTGGGTTCT
CTTGGCTGTTACTGCCAGGACCCATATGTAAAAGAAGCAGAAAACCTTAAGA
AATATTTTAATGCAGGTCATTCAGATGTAGCGGATAATGGAACTCTTTTCTTA
GGCATTTTGAAGAATTGGAAAGAGGAGAGTGACAGAAAAATAATGCAGAGC
 
CAAATTGTCTCCTTTTACTTCAAACTTTTTAAAAACTTTAAAGATGACCAGAG
CATCCAAAAGAGTGTGGAGACCATCAAGGAAGACATGAATGTCAAGTTTTTC
AATAGCAACAAAAAGAAACGAGATGACTTCGAAAAGCTGACTAATTATTCGG
TAACTGACTTGAATGTCCAACGCAAAGCAATACATGAACTCATCCAAGTGAT
GGCTGAACTGTCGCCAGCAGCTAAAACAGGGAAGCGAAAAAGGAGTCAGAT
GCTGTTTCGAGGTCGAAGAGCATCCCAGggcggaGGATCCGGCGCAACAAACTT
CTCTCTGCTGAAACAAGCCGGAGATGTCGAAGAGAATCCTGGACCGATGTGT
CACCAGCAGTTGGTCATCTCTTGGTTTTCCCTGGTTTTTCTGGCATCTCCCCTC
GTGGCCATATGGGAACTGAAGAAAGATGTTTATGTCGTAGAATTGGATTGGT
ATCCGGATGCCCCTGGAGAAATGGTGGTCCTCACCTGTGACACCCCTGAAGA
AGATGGTATCACCTGGACCTTGGACCAGAGCAGTGAGGTCTTAGGCTCTGGC
AAAACCCTGACCATCCAAGTCAAAGAGTTTGGAGATGCTGGCCAGTACACCT
GTCACAAAGGAGGCGAGGTTCTAAGCCATTCGCTCCTGCTGCTTCACAAAAA
GGAAGATGGAATTTGGTCCACTGATATTTTAAAGGACCAGAAAGAACCCAAA
AATAAGACCTTTCTAAGATGCGAGGCCAAGAATTATTCTGGACGTTTCACCTG
CTGGTGGCTGACGACAATCAGTACTGATTTGACATTCAGTGTCAAAAGCAGC
AGAGGCTCTTCTGACCCCCAAGGGGTGACGTGCGGAGCTGCTACACTCTCTGC
AGAGAGAGTCAGAGGGGACAACAAGGAGTATGAGTACTCAGTGGAGTGCCA
GGAGGACAGTGCCTGCCCAGCTGCTGAGGAGAGTCTGCCCATTGAGGTCATG
GTGGATGCCGTTCACAAGCTCAAGTATGAAAACTACACCAGCAGCTTCTTCAT
CAGGGACATCATCAAACCTGACCCACCCAAGAACTTGCAGCTGAAGCCATTA
AAGAATTCTCGGCAGGTGGAGGTCAGCTGGGAGTACCCTGACACCTGGAGTA
CTCCACATTCCTACTTCTCCCTGACATTCTGCGTTCAGGTCCAGGGCAAGAGC
AAGAGAGAAAAGAAAGATAGAGTCTTCACGGACAAGACCTCAGCCACGGTC
ATCTGCCGCAAAAATGCCAGCATTAGCGTGCGGGCCCAGGACCGCTACTATA
GCTCATCTTGGAGCGAATGGGCATCTGTGCCCTGCAGTGGCactAGTGGAGAG
GGCAGAGGAAGTCTGCTAACATGCGGTGACGTCGAGGAGAATCCTGGCCCAA
TGGAATACGCCTCTGACGCTTCACTGGACCCCGAAGCCCCGTGGCCTCCCGCG
CCCCGCGCTCGCGCCTGCCGCGTACTGCCTTGGGCCCTGGTCGCGGGGCTGCT
GCTGCTGCTGCTGCTCGCTGCCGCCTGCGCCGTCTTCCTCGCCTGCCCCTGGG
CCGTGTCCGGGGCTCGCGCCTCGCCCGGCTCCGCGGCCAGCCCGAGACTCCG
CGAGGGTCCCGAGCTTTCGCCCGACGATCCCGCCGGCCTCTTGGACCTGCGGC
AGGGCATGTTTGCGCAGCTGGTGGCCCAAAATGTTCTGCTGATCGATGGGCCC
CTGAGCTGGTACAGTGACCCAGGCCTGGCAGGCGTGTCCCTGACGGGGGGCC
TGAGCTACAAAGAGGACACGAAGGAGCTGGTGGTGGCCAAGGCTGGAGTCT
ACTATGTCTTCTTTCAACTAGAGCTGCGGCGCGTGGTGGCCGGCGAGGGCTCA
GGCTCCGTTTCACTTGCGCTGCACCTGCAGCCACTGCGCTCTGCTGCTGGGGC
CGCCGCCCTGGCTTTGACCGTGGACCTGCCACCCGCCTCCTCCGAGGCTCGGA
ACTCGGCCTTCGGTTTCCAGGGCCGCTTGCTGCACCTGAGTGCCGGCCAGCGC
CTGGGCGTCCATCTTCACACTGAGGCCAGGGCACGCCATGCCTGGCAGCTTA
CCCAGGGCGCCACAGTCTTGGGACTCTTCCGGGTGACCCCCGAAATCCCAGC
CGGACTCCCTTCACCGAGGTCGGAATAAGaattcacgcgttaagtcgacaatcaacctctggattaca
aaatttgtgaaagattgactggtattcttaactatgttgctccttttacgctatgtggatacgctgctttaatgcctttgtatcatgctattg
cttcccgtatggctttcattttctcctccttgtataaatcctggttgctgtctctttatgaggagttgtggcccgttgtcaggcaacgtgg
cgtggtgtgcactgtgtttgctgacgcaacccccactggttggggcattgccaccacctgtcagctcctttccgggactttcgctttc
cccctccctattgccacggcggaactcatcgccgcctgccttgcccgctgctggacaggggctcggctgttgggcactgacaatt
ccgtggtgttgtcggggaaatcatcgtcctttccttggctgctcgcctgtgttgccacctggattctgcgcgggacgtccttctgcta
cgtcccttcggccctcaatccagcggaccttccttcccgcggcctgctgccggctctgcggcctcttccgcgtcttcgccttcgcc
ctcagacgagtcggatctccctttgggccgcctccccgcgtcgactttaagaccaatgacGGCCGCAGGAACCCC
TAGTGATGGAGTTGGCCACTCCCTCTCTGCGCGCTCGCTCGCTCACTGAGGCC
 
GGGCGACCAAAGGTCGCCCGACGCCCGGGCTTTGCCCGGGCGGCCTCAGTGA
GCGAGCGAGCGCGCAGCTGCCTGCAGGGGCGCCTGATGCGGTATTTTCTCCTT
ACGCATCTGTGCGGTATTTCACACCGCATACGTCAAAGCAACCATAGTACGC
GCCCTGTAGCGGCGCATTAAGCGCGGCGGGTGTGGTGGTTACGCGCAGCGTG
ACCGCTACACTTGCCAGCGCCTTAGCGCCCGCTCCTTTCGCTTTCTTCCCTTCC
TTTCTCGCCACGTTCGCCGGCTTTCCCCGTCAAGCTCTAAATCGGGGGCTCCC
TTTAGGGTTCCGATTTAGTGCTTTACGGCACCTCGACCCCAAAAAACTTGATT
TGGGTGATGGTTCACGTAGTGGGCCATCGCCCTGATAGACGGTTTTTCGCCCT
TTGACGTTGGAGTCCACGTTCTTTAATAGTGGACTCTTGTTCCAAACTGGAAC
AACACTCAACTCTATCTCGGGCTATTCTTTTGATTTATAAGGGATTTTGCCGAT
TTCGGTCTATTGGTTAAAAAATGAGCTGATTTAACAAAAATTTAACGCGAATT
TTAACAAAATATTAACGTTTACAATTTTATGGTGCACTCTCAGTACAATCTGC
TCTGATGCCGCATAGTTAAGCCAGCCCCGACACCCGCCAACACCCGCTGACG
CGCCCTGACGGGCTTGTCTGCTCCCGGCATCCGCTTACAGACAAGCTGTGACC
GTCTCCGGGAGCTGCATGTGTCAGAGGTTTTCACCGTCATCACCGAAACGCGC
GAGACGAAAGGGCCTCGTGATACGCCTATTTTTATAGGTTAATGTCATGATAA
TAATGGTTTCTTAGACGTCAGGTGGCACTTTTCGGGGAAATGTGCGCGGAACC
CCTATTTGTTTATTTTTCTAAATACATTCAAATATGTATCCGCTCATGAGACAA
TAACCCTGATAAATGCTTCAATAATATTGAAAAAGGAAGAGTATGAGTATTC
AACATTTCCGTGTCGCCCTTATTCCCTTTTTTGCGGCATTTTGCCTTCCTGTTTT
TGCTCACCCAGAAACGCTGGTGAAAGTAAAAGATGCTGAAGATCAGTTGGGT
GCACGAGTGGGTTACATCGAACTGGATCTCAACAGCGGTAAGATCCTTGAGA
GTTTTCGCCCCGAAGAACGTTTTCCAATGATGAGCACTTTTAAAGTTCTGCTA
TGTGGCGCGGTATTATCCCGTATTGACGCCGGGCAAGAGCAACTCGGTCGCC
GCATACACTATTCTCAGAATGACTTGGTTGAGTACTCACCAGTCACAGAAAA
GCATCTTACGGATGGCATGACAGTAAGAGAATTATGCAGTGCTGCCATAACC
ATGAGTGATAACACTGCGGCCAACTTACTTCTGACAACGATCGGAGGACCGA
AGGAGCTAACCGCTTTTTTGCACAACATGGGGGATCATGTAACTCGCCTTGAT
CGTTGGGAACCGGAGCTGAATGAAGCCATACCAAACGACGAGCGTGACACCA
CGATGCCTGTAGCAATGGCAACAACGTTGCGCAAACTATTAACTGGCGAACT
ACTTACTCTAGCTTCCCGGCAACAATTAATAGACTGGATGGAGGCGGATAAA
GTTGCAGGACCACTTCTGCGCTCGGCCCTTCCGGCTGGCTGGTTTATTGCTGA
TAAATCTGGAGCCGGTGAGCGTGGAAGCCGCGGTATCATTGCAGCACTGGGG
CCAGATGGTAAGCCCTCCCGTATCGTAGTTATCTACACGACGGGGAGTCAGG
CAACTATGGATGAACGAAATAGACAGATCGCTGAGATAGGTGCCTCACTGAT
TAAGCATTGGTAACTGTCAGACCAAGTTTACTCATATATACTTTAGATTGATT
TAAAACTTCATTTTTAATTTAAAAGGATCTAGGTGAAGATCCTTTTTGATAAT
CTCATGACCAAAATCCCTTAACGTGAGTTTTCGTTCCACTGAGCGTCAGACCC
CGTAGAAAAGATCAAAGGATCTTCTTGAGATCCTTTTTTTCTGCGCGTAATCT
GCTGCTTGCAAACAAAAAAACCACCGCTACCAGCGGTGGTTTGTTTGCCGGA
TCAAGAGCTACCAACTCTTTTTCCGAAGGTAACTGGCTTCAGCAGAGCGCAG
ATACCAAATACTGTTCTTCTAGTGTAGCCGTAGTTAGGCCACCACTTCAAGAA
CTCTGTAGCACCGCCTACATACCTCGCTCTGCTAATCCTGTTACCAGTGGCTG
CTGCCAGTGGCGATAAGTCGTGTCTTACCGGGTTGGACTCAAGACGATAGTTA
CCGGATAAGGCGCAGCGGTCGGGCTGAACGGGGGGTTCGTGCACACAGCCCA
GCTTGGAGCGAACGACCTACACCGAACTGAGATACCTACAGCGTGAGCTATG
AGAAAGCGCCACGCTTCCCGAAGGGAGAAAGGCGGACAGGTATCCGGTAAG
CGGCAGGGTCGGAACAGGAGAGCGCACGAGGGAGCTTCCAGGGGGAAACGC
CTGGTATCTTTATAGTCCTGTCGGGTTTCGCCACCTCTGACTTGAGCGTCGATT
 
TTTGTGATGCTCGTCAGGGGGGCGGAGCCTATGGAAAAACGCCAGCAACGCG
GCCTTTTTACGGTTCCTGGCCTTTTGCTGGCCTTTTGCTCACATGT
**pGW128b-pAAV_EFS-LTR-hIFNg-P2A-hIL23-T2A-h41BBL-E2A-hLight-WPRE (SEQ ID NO: 50)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
ATGAAATATACAAGTTATATCTTGGCTTTTCAGCTCTGCATCGTTTTGGGTTCT
CTTGGCTGTTACTGCCAGGACCCATATGTAAAAGAAGCAGAAAACCTTAAGA
AATATTTTAATGCAGGTCATTCAGATGTAGCGGATAATGGAACTCTTTTCTTA
GGCATTTTGAAGAATTGGAAAGAGGAGAGTGACAGAAAAATAATGCAGAGC
CAAATTGTCTCCTTTTACTTCAAACTTTTTAAAAACTTTAAAGATGACCAGAG
CATCCAAAAGAGTGTGGAGACCATCAAGGAAGACATGAATGTCAAGTTTTTC
AATAGCAACAAAAAGAAACGAGATGACTTCGAAAAGCTGACTAATTATTCGG
TAACTGACTTGAATGTCCAACGCAAAGCAATACATGAACTCATCCAAGTGAT
GGCTGAACTGTCGCCAGCAGCTAAAACAGGGAAGCGAAAAAGGAGTCAGAT
GCTGTTTCGAGGTCGAAGAGCATCCCAGggcggaGGATCCGGCGCAACAAACTT
CTCTCTGCTGAAACAAGCCGGAGATGTCGAAGAGAATCCTGGACCGATGTGT
CACCAGCAGTTGGTCATCTCTTGGTTTTCCCTGGTTTTTCTGGCATCTCCCCTC
GTGGCCATATGGGAACTGAAGAAAGATGTTTATGTCGTAGAATTGGATTGGT
ATCCGGATGCCCCTGGAGAAATGGTGGTCCTCACCTGTGACACCCCTGAAGA
AGATGGTATCACCTGGACCTTGGACCAGAGCAGTGAGGTCTTAGGCTCTGGC
AAAACCCTGACCATCCAAGTCAAAGAGTTTGGAGATGCTGGCCAGTACACCT
GTCACAAAGGAGGCGAGGTTCTAAGCCATTCGCTCCTGCTGCTTCACAAAAA
GGAAGATGGAATTTGGTCCACTGATATTTTAAAGGACCAGAAAGAACCCAAA
AATAAGACCTTTCTAAGATGCGAGGCCAAGAATTATTCTGGACGTTTCACCTG
CTGGTGGCTGACGACAATCAGTACTGATTTGACATTCAGTGTCAAAAGCAGC
AGAGGCTCTTCTGACCCCCAAGGGGTGACGTGCGGAGCTGCTACACTCTCTGC
AGAGAGAGTCAGAGGGGACAACAAGGAGTATGAGTACTCAGTGGAGTGCCA
GGAGGACAGTGCCTGCCCAGCTGCTGAGGAGAGTCTGCCCATTGAGGTCATG
GTGGATGCCGTTCACAAGCTCAAGTATGAAAACTACACCAGCAGCTTCTTCAT
CAGGGACATCATCAAACCTGACCCACCCAAGAACTTGCAGCTGAAGCCATTA
AAGAATTCTCGGCAGGTGGAGGTCAGCTGGGAGTACCCTGACACCTGGAGTA
CTCCACATTCCTACTTCTCCCTGACATTCTGCGTTCAGGTCCAGGGCAAGAGC
AAGAGAGAAAAGAAAGATAGAGTCTTCACGGACAAGACCTCAGCCACGGTC
ATCTGCCGCAAAAATGCCAGCATTAGCGTGCGGGCCCAGGACCGCTACTATA
GCTCATCTTGGAGCGAATGGGCATCTGTGCCCTGCAGTGGCAGTGGAGAGGG
CAGAGGAAGTCTGCTAACATGCGGTGACGTCGAGGAGAATCCTGGCCCAATG
GAATACGCCTCTGACGCTTCACTGGACCCCGAAGCCCCGTGGCCTCCCGCGCC
 
CCGCGCTCGCGCCTGCCGCGTACTGCCTTGGGCCCTGGTCGCGGGGCTGCTGC
TGCTGCTGCTGCTCGCTGCCGCCTGCGCCGTCTTCCTCGCCTGCCCCTGGGCC
GTGTCCGGGGCTCGCGCCTCGCCCGGCTCCGCGGCCAGCCCGAGACTCCGCG
AGGGTCCCGAGCTTTCGCCCGACGATCCCGCCGGCCTCTTGGACCTGCGGCA
GGGCATGTTTGCGCAGCTGGTGGCCCAAAATGTTCTGCTGATCGATGGGCCCC
TGAGCTGGTACAGTGACCCAGGCCTGGCAGGCGTGTCCCTGACGGGGGGCCT
GAGCTACAAAGAGGACACGAAGGAGCTGGTGGTGGCCAAGGCTGGAGTCTA
CTATGTCTTCTTTCAACTAGAGCTGCGGCGCGTGGTGGCCGGCGAGGGCTCAG
GCTCCGTTTCACTTGCGCTGCACCTGCAGCCACTGCGCTCTGCTGCTGGGGCC
GCCGCCCTGGCTTTGACCGTGGACCTGCCACCCGCCTCCTCCGAGGCTCGGAA
CTCGGCCTTCGGTTTCCAGGGCCGCTTGCTGCACCTGAGTGCCGGCCAGCGCC
TGGGCGTCCATCTTCACACTGAGGCCAGGGCACGCCATGCCTGGCAGCTTAC
CCAGGGCGCCACAGTCTTGGGACTCTTCCGGGTGACCCCCGAAATCCCAGCC
GGACTCCCTTCACCGAGGTCGGAAGGTgctagcggtacccagtgcaccaactacgccctgctgaagct
ggccggcgatgtggagagcaaccccgggcccATGGAGGAGAGTGTCGTACGGCCCTCAGTGTT
TGTGGTGGATGGACAGACCGACATCCCATTCACGAGGCTGGGACGAAGCCAC
CGGAGACAGTCGTGCAGTGTGGCCCGGGTGGGTCTGGGTCTCTTGCTGTTGCT
GATGGGGGCCGGGCTGGCCGTCCAAGGCTGGTTCCTCCTGCAGCTGCACTGG
CGTCTAGGAGAGATGGTCACCCGCCTGCCTGACGGACCTGCAGGCTCCTGGG
AGCAGCTGATACAAGAGCGAAGGTCTCACGAGGTCAACCCAGCAGCGCATCT
CACAGGGGCCAACTCCAGCTTGACCGGCAGCGGGGGGCCGCTGTTATGGGAG
ACTCAGCTGGGCCTGGCCTTCCTGAGGGGCCTCAGCTACCACGATGGGGCCC
TTGTGGTCACCAAAGCTGGCTACTACTACATCTACTCCAAGGTGCAGCTGGGC
GGTGTGGGCTGCCCGCTGGGCCTGGCCAGCACCATCACCCACGGCCTCTACA
AGCGCACACCCCGCTACCCCGAGGAGCTGGAGCTGTTGGTCAGCCAGCAGTC
ACCCTGCGGACGGGCCACCAGCAGCTCCCGGGTCTGGTGGGACAGCAGCTTC
CTGGGTGGTGTGGTACACCTGGAGGCTGGGGAGAAGGTGGTCGTCCGTGTGC
TGGATGAACGCCTGGTTCGACTGCGTGATGGTACCCGGTCTTACTTCGGGGCT
TTCATGGTGGGTGGAGGCGGTTGCGGAAAGCGTAAGTGAGaattcacgcgttaagtcgac
aatcaacctctggattacaaaatttgtgaaagattgactggtattcttaactatgttgctccttttacgctatgtggatacgctgctttaat
gcctttgtatcatgctattgcttcccgtatggctttcattttctcctccttgtataaatcctggttgctgtctctttatgaggagttgtggcc
cgttgtcaggcaacgtggcgtggtgtgcactgtgtttgctgacgcaacccccactggttggggcattgccaccacctgtcagctc
ctttccgggactttcgctttccccctccctattgccacggcggaactcatcgccgcctgccttgcccgctgctggacaggggctcg
gctgttgggcactgacaattccgtggtgttgtcggggaaatcatcgtcctttccttggctgctcgcctgtgttgccacctggattctg
cgcgggacgtccttctgctacgtcccttcggccctcaatccagcggaccttccttcccgcggcctgctgccggctctgcggcctct
tccgcgtcttcgccttcgccctcagacgagtcggatctccctttgggccgcctccccgcgtcgactttaagaccaatgacGGC
CGCAGGAACCCCTAGTGATGGAGTTGGCCACTCCCTCTCTGCGCGCTCGCTCG
CTCACTGAGGCCGGGCGACCAAAGGTCGCCCGACGCCCGGGCTTTGCCCGGG
CGGCCTCAGTGAGCGAGCGAGCGCGCAGCTGCCTGCAGGGGCGCCTGATGCG
GTATTTTCTCCTTACGCATCTGTGCGGTATTTCACACCGCATACGTCAAAGCA
ACCATAGTACGCGCCCTGTAGCGGCGCATTAAGCGCGGCGGGTGTGGTGGTT
ACGCGCAGCGTGACCGCTACACTTGCCAGCGCCTTAGCGCCCGCTCCTTTCGC
TTTCTTCCCTTCCTTTCTCGCCACGTTCGCCGGCTTTCCCCGTCAAGCTCTAAA
TCGGGGGCTCCCTTTAGGGTTCCGATTTAGTGCTTTACGGCACCTCGACCCCA
AAAAACTTGATTTGGGTGATGGTTCACGTAGTGGGCCATCGCCCTGATAGAC
GGTTTTTCGCCCTTTGACGTTGGAGTCCACGTTCTTTAATAGTGGACTCTTGTT
CCAAACTGGAACAACACTCAACTCTATCTCGGGCTATTCTTTTGATTTATAAG
GGATTTTGCCGATTTCGGTCTATTGGTTAAAAAATGAGCTGATTTAACAAAAA
TTTAACGCGAATTTTAACAAAATATTAACGTTTACAATTTTATGGTGCACTCT
CAGTACAATCTGCTCTGATGCCGCATAGTTAAGCCAGCCCCGACACCCGCCA
 
ACACCCGCTGACGCGCCCTGACGGGCTTGTCTGCTCCCGGCATCCGCTTACAG
ACAAGCTGTGACCGTCTCCGGGAGCTGCATGTGTCAGAGGTTTTCACCGTCAT
CACCGAAACGCGCGAGACGAAAGGGCCTCGTGATACGCCTATTTTTATAGGT
TAATGTCATGATAATAATGGTTTCTTAGACGTCAGGTGGCACTTTTCGGGGAA
ATGTGCGCGGAACCCCTATTTGTTTATTTTTCTAAATACATTCAAATATGTATC
CGCTCATGAGACAATAACCCTGATAAATGCTTCAATAATATTGAAAAAGGAA
GAGTATGAGTATTCAACATTTCCGTGTCGCCCTTATTCCCTTTTTTGCGGCATT
TTGCCTTCCTGTTTTTGCTCACCCAGAAACGCTGGTGAAAGTAAAAGATGCTG
AAGATCAGTTGGGTGCACGAGTGGGTTACATCGAACTGGATCTCAACAGCGG
TAAGATCCTTGAGAGTTTTCGCCCCGAAGAACGTTTTCCAATGATGAGCACTT
TTAAAGTTCTGCTATGTGGCGCGGTATTATCCCGTATTGACGCCGGGCAAGAG
CAACTCGGTCGCCGCATACACTATTCTCAGAATGACTTGGTTGAGTACTCACC
AGTCACAGAAAAGCATCTTACGGATGGCATGACAGTAAGAGAATTATGCAGT
GCTGCCATAACCATGAGTGATAACACTGCGGCCAACTTACTTCTGACAACGAT
CGGAGGACCGAAGGAGCTAACCGCTTTTTTGCACAACATGGGGGATCATGTA
ACTCGCCTTGATCGTTGGGAACCGGAGCTGAATGAAGCCATACCAAACGACG
AGCGTGACACCACGATGCCTGTAGCAATGGCAACAACGTTGCGCAAACTATT
AACTGGCGAACTACTTACTCTAGCTTCCCGGCAACAATTAATAGACTGGATGG
AGGCGGATAAAGTTGCAGGACCACTTCTGCGCTCGGCCCTTCCGGCTGGCTG
GTTTATTGCTGATAAATCTGGAGCCGGTGAGCGTGGAAGCCGCGGTATCATTG
CAGCACTGGGGCCAGATGGTAAGCCCTCCCGTATCGTAGTTATCTACACGAC
GGGGAGTCAGGCAACTATGGATGAACGAAATAGACAGATCGCTGAGATAGG
TGCCTCACTGATTAAGCATTGGTAACTGTCAGACCAAGTTTACTCATATATAC
TTTAGATTGATTTAAAACTTCATTTTTAATTTAAAAGGATCTAGGTGAAGATC
CTTTTTGATAATCTCATGACCAAAATCCCTTAACGTGAGTTTTCGTTCCACTGA
GCGTCAGACCCCGTAGAAAAGATCAAAGGATCTTCTTGAGATCCTTTTTTTCT
GCGCGTAATCTGCTGCTTGCAAACAAAAAAACCACCGCTACCAGCGGTGGTT
TGTTTGCCGGATCAAGAGCTACCAACTCTTTTTCCGAAGGTAACTGGCTTCAG
CAGAGCGCAGATACCAAATACTGTTCTTCTAGTGTAGCCGTAGTTAGGCCACC
ACTTCAAGAACTCTGTAGCACCGCCTACATACCTCGCTCTGCTAATCCTGTTA
CCAGTGGCTGCTGCCAGTGGCGATAAGTCGTGTCTTACCGGGTTGGACTCAAG
ACGATAGTTACCGGATAAGGCGCAGCGGTCGGGCTGAACGGGGGGTTCGTGC
ACACAGCCCAGCTTGGAGCGAACGACCTACACCGAACTGAGATACCTACAGC
GTGAGCTATGAGAAAGCGCCACGCTTCCCGAAGGGAGAAAGGCGGACAGGT
ATCCGGTAAGCGGCAGGGTCGGAACAGGAGAGCGCACGAGGGAGCTTCCAG
GGGGAAACGCCTGGTATCTTTATAGTCCTGTCGGGTTTCGCCACCTCTGACTT
GAGCGTCGATTTTTGTGATGCTCGTCAGGGGGGCGGAGCCTATGGAAAAACG
CCAGCAACGCGGCCTTTTTACGGTTCCTGGCCTTTTGCTGGCCTTTTGCTCACA
TGT
**pGW145-pAAV_EFS-LTR-hIFNg-WPRE-sPA (SEQ ID NO: 51)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
 
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
ATGAAATATACAAGTTATATCTTGGCTTTTCAGCTCTGCATCGTTTTGGGTTCT
CTTGGCTGTTACTGCCAGGACCCATATGTAAAAGAAGCAGAAAACCTTAAGA
AATATTTTAATGCAGGTCATTCAGATGTAGCGGATAATGGAACTCTTTTCTTA
GGCATTTTGAAGAATTGGAAAGAGGAGAGTGACAGAAAAATAATGCAGAGC
CAAATTGTCTCCTTTTACTTCAAACTTTTTAAAAACTTTAAAGATGACCAGAG
CATCCAAAAGAGTGTGGAGACCATCAAGGAAGACATGAATGTCAAGTTTTTC
AATAGCAACAAAAAGAAACGAGATGACTTCGAAAAGCTGACTAATTATTCGG
TAACTGACTTGAATGTCCAACGCAAAGCAATACATGAACTCATCCAAGTGAT
GGCTGAACTGTCGCCAGCAGCTAAAACAGGGAAGCGAAAAAGGAGTCAGAT
GCTGTTTCGAGGTCGAAGAGCATCCCAGTGAGaattcacgcgttaagtcgacaatcaacctctgga
ttacaaaatttgtgaaagattgactggtattcttaactatgttgctccttttacgctatgtggatacgctgctttaatgcctttgtatcatgc
tattgcttcccgtatggctttcattttctcctccttgtataaatcctggttgctgtctctttatgaggagttgtggcccgttgtcaggcaac
gtggcgtggtgtgcactgtgtttgctgacgcaacccccactggttggggcattgccaccacctgtcagctcctttccgggactttcg
ctttccccctccctattgccacggcggaactcatcgccgcctgccttgcccgctgctggacaggggctcggctgttgggcactga
caattccgtggtgttgtcggggaaatcatcgtcctttccttggctgctcgcctgtgttgccacctggattctgcgcgggacgtccttct
gctacgtcccttcggccctcaatccagcggaccttccttcccgcggcctgctgccggctctgcggcctcttccgcgtcttcgccttc
gccctcagacgagtcggatctccctttgggccgcctccccgcgtcgactttaagaccaatgacGGCCGaataaaagatcttta
ttttcattagatctgtgtgttggttttttgtgtgcggccgCAGGAACCCCTAGTGATGGAGTTGGCCACT
CCCTCTCTGCGCGCTCGCTCGCTCACTGAGGCCGGGCGACCAAAGGTCGCCC
GACGCCCGGGCTTTGCCCGGGCGGCCTCAGTGAGCGAGCGAGCGCGCAGCTG
CCTGCAGGGGCGCCTGATGCGGTATTTTCTCCTTACGCATCTGTGCGGTATTT
CACACCGCATACGTCAAAGCAACCATAGTACGCGCCCTGTAGCGGCGCATTA
AGCGCGGCGGGTGTGGTGGTTACGCGCAGCGTGACCGCTACACTTGCCAGCG
CCTTAGCGCCCGCTCCTTTCGCTTTCTTCCCTTCCTTTCTCGCCACGTTCGCCG
GCTTTCCCCGTCAAGCTCTAAATCGGGGGCTCCCTTTAGGGTTCCGATTTAGT
GCTTTACGGCACCTCGACCCCAAAAAACTTGATTTGGGTGATGGTTCACGTAG
TGGGCCATCGCCCTGATAGACGGTTTTTCGCCCTTTGACGTTGGAGTCCACGT
TCTTTAATAGTGGACTCTTGTTCCAAACTGGAACAACACTCAACTCTATCTCG
GGCTATTCTTTTGATTTATAAGGGATTTTGCCGATTTCGGTCTATTGGTTAAAA
AATGAGCTGATTTAACAAAAATTTAACGCGAATTTTAACAAAATATTAACGTT
TACAATTTTATGGTGCACTCTCAGTACAATCTGCTCTGATGCCGCATAGTTAA
GCCAGCCCCGACACCCGCCAACACCCGCTGACGCGCCCTGACGGGCTTGTCT
GCTCCCGGCATCCGCTTACAGACAAGCTGTGACCGTCTCCGGGAGCTGCATGT
GTCAGAGGTTTTCACCGTCATCACCGAAACGCGCGAGACGAAAGGGCCTCGT
GATACGCCTATTTTTATAGGTTAATGTCATGATAATAATGGTTTCTTAGACGT
CAGGTGGCACTTTTCGGGGAAATGTGCGCGGAACCCCTATTTGTTTATTTTTC
TAAATACATTCAAATATGTATCCGCTCATGAGACAATAACCCTGATAAATGCT
TCAATAATATTGAAAAAGGAAGAGTATGAGTATTCAACATTTCCGTGTCGCCC
TTATTCCCTTTTTTGCGGCATTTTGCCTTCCTGTTTTTGCTCACCCAGAAACGC
TGGTGAAAGTAAAAGATGCTGAAGATCAGTTGGGTGCACGAGTGGGTTACAT
CGAACTGGATCTCAACAGCGGTAAGATCCTTGAGAGTTTTCGCCCCGAAGAA
CGTTTTCCAATGATGAGCACTTTTAAAGTTCTGCTATGTGGCGCGGTATTATC
CCGTATTGACGCCGGGCAAGAGCAACTCGGTCGCCGCATACACTATTCTCAG
AATGACTTGGTTGAGTACTCACCAGTCACAGAAAAGCATCTTACGGATGGCA
TGACAGTAAGAGAATTATGCAGTGCTGCCATAACCATGAGTGATAACACTGC
GGCCAACTTACTTCTGACAACGATCGGAGGACCGAAGGAGCTAACCGCTTTT
 
TTGCACAACATGGGGGATCATGTAACTCGCCTTGATCGTTGGGAACCGGAGC
TGAATGAAGCCATACCAAACGACGAGCGTGACACCACGATGCCTGTAGCAAT
GGCAACAACGTTGCGCAAACTATTAACTGGCGAACTACTTACTCTAGCTTCCC
GGCAACAATTAATAGACTGGATGGAGGCGGATAAAGTTGCAGGACCACTTCT
GCGCTCGGCCCTTCCGGCTGGCTGGTTTATTGCTGATAAATCTGGAGCCGGTG
AGCGTGGAAGCCGCGGTATCATTGCAGCACTGGGGCCAGATGGTAAGCCCTC
CCGTATCGTAGTTATCTACACGACGGGGAGTCAGGCAACTATGGATGAACGA
AATAGACAGATCGCTGAGATAGGTGCCTCACTGATTAAGCATTGGTAACTGT
CAGACCAAGTTTACTCATATATACTTTAGATTGATTTAAAACTTCATTTTTAAT
TTAAAAGGATCTAGGTGAAGATCCTTTTTGATAATCTCATGACCAAAATCCCT
TAACGTGAGTTTTCGTTCCACTGAGCGTCAGACCCCGTAGAAAAGATCAAAG
GATCTTCTTGAGATCCTTTTTTTCTGCGCGTAATCTGCTGCTTGCAAACAAAAA
AACCACCGCTACCAGCGGTGGTTTGTTTGCCGGATCAAGAGCTACCAACTCTT
TTTCCGAAGGTAACTGGCTTCAGCAGAGCGCAGATACCAAATACTGTTCTTCT
AGTGTAGCCGTAGTTAGGCCACCACTTCAAGAACTCTGTAGCACCGCCTACAT
ACCTCGCTCTGCTAATCCTGTTACCAGTGGCTGCTGCCAGTGGCGATAAGTCG
TGTCTTACCGGGTTGGACTCAAGACGATAGTTACCGGATAAGGCGCAGCGGT
CGGGCTGAACGGGGGGTTCGTGCACACAGCCCAGCTTGGAGCGAACGACCTA
CACCGAACTGAGATACCTACAGCGTGAGCTATGAGAAAGCGCCACGCTTCCC
GAAGGGAGAAAGGCGGACAGGTATCCGGTAAGCGGCAGGGTCGGAACAGGA
GAGCGCACGAGGGAGCTTCCAGGGGGAAACGCCTGGTATCTTTATAGTCCTG
TCGGGTTTCGCCACCTCTGACTTGAGCGTCGATTTTTGTGATGCTCGTCAGGG
GGGCGGAGCCTATGGAAAAACGCCAGCAACGCGGCCTTTTTACGGTTCCTGG
CCTTTTGCTGGCCTTTTGCTCACATGT
**pGW146-pAAV_EFS-LTR-hIL23-WPRE-sPA (SEQ ID NO: 52)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
ATGTGTCACCAGCAGTTGGTCATCTCTTGGTTTTCCCTGGTTTTTCTGGCATCT
CCCCTCGTGGCCATATGGGAACTGAAGAAAGATGTTTATGTCGTAGAATTGG
ATTGGTATCCGGATGCCCCTGGAGAAATGGTGGTCCTCACCTGTGACACCCCT
GAAGAAGATGGTATCACCTGGACCTTGGACCAGAGCAGTGAGGTCTTAGGCT
CTGGCAAAACCCTGACCATCCAAGTCAAAGAGTTTGGAGATGCTGGCCAGTA
CACCTGTCACAAAGGAGGCGAGGTTCTAAGCCATTCGCTCCTGCTGCTTCACA
AAAAGGAAGATGGAATTTGGTCCACTGATATTTTAAAGGACCAGAAAGAACC
CAAAAATAAGACCTTTCTAAGATGCGAGGCCAAGAATTATTCTGGACGTTTC
ACCTGCTGGTGGCTGACGACAATCAGTACTGATTTGACATTCAGTGTCAAAAG
CAGCAGAGGCTCTTCTGACCCCCAAGGGGTGACGTGCGGAGCTGCTACACTC
TCTGCAGAGAGAGTCAGAGGGGACAACAAGGAGTATGAGTACTCAGTGGAG
 
TGCCAGGAGGACAGTGCCTGCCCAGCTGCTGAGGAGAGTCTGCCCATTGAGG
TCATGGTGGATGCCGTTCACAAGCTCAAGTATGAAAACTACACCAGCAGCTT
CTTCATCAGGGACATCATCAAACCTGACCCACCCAAGAACTTGCAGCTGAAG
CCATTAAAGAATTCTCGGCAGGTGGAGGTCAGCTGGGAGTACCCTGACACCT
GGAGTACTCCACATTCCTACTTCTCCCTGACATTCTGCGTTCAGGTCCAGGGC
AAGAGCAAGAGAGAAAAGAAAGATAGAGTCTTCACGGACAAGACCTCAGCC
ACGGTCATCTGCCGCAAAAATGCCAGCATTAGCGTGCGGGCCCAGGACCGCT
ACTATAGCTCATCTTGGAGCGAATGGGCATCTGTGCCCTGCAGTTGAGaattcacg
cgttaagtcgacaatcaacctctggattacaaaatttgtgaaagattgactggtattcttaactatgttgctccttttacgctatgtggat
acgctgctttaatgcctttgtatcatgctattgcttcccgtatggctttcattttctcctccttgtataaatcctggttgctgtctctttatgag
gagttgtggcccgttgtcaggcaacgtggcgtggtgtgcactgtgtttgctgacgcaacccccactggttggggcattgccacca
cctgtcagctcctttccgggactttcgctttccccctccctattgccacggcggaactcatcgccgcctgccttgcccgctgctgga
caggggctcggctgttgggcactgacaattccgtggtgttgtcggggaaatcatcgtcctttccttggctgctcgcctgtgttgcca
cctggattctgcgcgggacgtccttctgctacgtcccttcggccctcaatccagcggaccttccttcccgcggcctgctgccggct
ctgcggcctcttccgcgtcttcgccttcgccctcagacgagtcggatctccctttgggccgcctccccgcgtcgactttaagacca
atgacGGCCGaataaaagatctttattttcattagatctgtgtgttggttttttgtgtgcggccgCAGGAACCCCTAG
TGATGGAGTTGGCCACTCCCTCTCTGCGCGCTCGCTCGCTCACTGAGGCCGGG
CGACCAAAGGTCGCCCGACGCCCGGGCTTTGCCCGGGCGGCCTCAGTGAGCG
AGCGAGCGCGCAGCTGCCTGCAGGGGCGCCTGATGCGGTATTTTCTCCTTACG
CATCTGTGCGGTATTTCACACCGCATACGTCAAAGCAACCATAGTACGCGCCC
TGTAGCGGCGCATTAAGCGCGGCGGGTGTGGTGGTTACGCGCAGCGTGACCG
CTACACTTGCCAGCGCCTTAGCGCCCGCTCCTTTCGCTTTCTTCCCTTCCTTTC
TCGCCACGTTCGCCGGCTTTCCCCGTCAAGCTCTAAATCGGGGGCTCCCTTTA
GGGTTCCGATTTAGTGCTTTACGGCACCTCGACCCCAAAAAACTTGATTTGGG
TGATGGTTCACGTAGTGGGCCATCGCCCTGATAGACGGTTTTTCGCCCTTTGA
CGTTGGAGTCCACGTTCTTTAATAGTGGACTCTTGTTCCAAACTGGAACAACA
CTCAACTCTATCTCGGGCTATTCTTTTGATTTATAAGGGATTTTGCCGATTTCG
GTCTATTGGTTAAAAAATGAGCTGATTTAACAAAAATTTAACGCGAATTTTAA
CAAAATATTAACGTTTACAATTTTATGGTGCACTCTCAGTACAATCTGCTCTG
ATGCCGCATAGTTAAGCCAGCCCCGACACCCGCCAACACCCGCTGACGCGCC
CTGACGGGCTTGTCTGCTCCCGGCATCCGCTTACAGACAAGCTGTGACCGTCT
CCGGGAGCTGCATGTGTCAGAGGTTTTCACCGTCATCACCGAAACGCGCGAG
ACGAAAGGGCCTCGTGATACGCCTATTTTTATAGGTTAATGTCATGATAATAA
TGGTTTCTTAGACGTCAGGTGGCACTTTTCGGGGAAATGTGCGCGGAACCCCT
ATTTGTTTATTTTTCTAAATACATTCAAATATGTATCCGCTCATGAGACAATAA
CCCTGATAAATGCTTCAATAATATTGAAAAAGGAAGAGTATGAGTATTCAAC
ATTTCCGTGTCGCCCTTATTCCCTTTTTTGCGGCATTTTGCCTTCCTGTTTTTGC
TCACCCAGAAACGCTGGTGAAAGTAAAAGATGCTGAAGATCAGTTGGGTGCA
CGAGTGGGTTACATCGAACTGGATCTCAACAGCGGTAAGATCCTTGAGAGTT
TTCGCCCCGAAGAACGTTTTCCAATGATGAGCACTTTTAAAGTTCTGCTATGT
GGCGCGGTATTATCCCGTATTGACGCCGGGCAAGAGCAACTCGGTCGCCGCA
TACACTATTCTCAGAATGACTTGGTTGAGTACTCACCAGTCACAGAAAAGCAT
CTTACGGATGGCATGACAGTAAGAGAATTATGCAGTGCTGCCATAACCATGA
GTGATAACACTGCGGCCAACTTACTTCTGACAACGATCGGAGGACCGAAGGA
GCTAACCGCTTTTTTGCACAACATGGGGGATCATGTAACTCGCCTTGATCGTT
GGGAACCGGAGCTGAATGAAGCCATACCAAACGACGAGCGTGACACCACGA
TGCCTGTAGCAATGGCAACAACGTTGCGCAAACTATTAACTGGCGAACTACTT
ACTCTAGCTTCCCGGCAACAATTAATAGACTGGATGGAGGCGGATAAAGTTG
CAGGACCACTTCTGCGCTCGGCCCTTCCGGCTGGCTGGTTTATTGCTGATAAA
TCTGGAGCCGGTGAGCGTGGAAGCCGCGGTATCATTGCAGCACTGGGGCCAG
 
ATGGTAAGCCCTCCCGTATCGTAGTTATCTACACGACGGGGAGTCAGGCAAC
TATGGATGAACGAAATAGACAGATCGCTGAGATAGGTGCCTCACTGATTAAG
CATTGGTAACTGTCAGACCAAGTTTACTCATATATACTTTAGATTGATTTAAA
ACTTCATTTTTAATTTAAAAGGATCTAGGTGAAGATCCTTTTTGATAATCTCAT
GACCAAAATCCCTTAACGTGAGTTTTCGTTCCACTGAGCGTCAGACCCCGTAG
AAAAGATCAAAGGATCTTCTTGAGATCCTTTTTTTCTGCGCGTAATCTGCTGC
TTGCAAACAAAAAAACCACCGCTACCAGCGGTGGTTTGTTTGCCGGATCAAG
AGCTACCAACTCTTTTTCCGAAGGTAACTGGCTTCAGCAGAGCGCAGATACCA
AATACTGTTCTTCTAGTGTAGCCGTAGTTAGGCCACCACTTCAAGAACTCTGT
AGCACCGCCTACATACCTCGCTCTGCTAATCCTGTTACCAGTGGCTGCTGCCA
GTGGCGATAAGTCGTGTCTTACCGGGTTGGACTCAAGACGATAGTTACCGGA
TAAGGCGCAGCGGTCGGGCTGAACGGGGGGTTCGTGCACACAGCCCAGCTTG
GAGCGAACGACCTACACCGAACTGAGATACCTACAGCGTGAGCTATGAGAAA
GCGCCACGCTTCCCGAAGGGAGAAAGGCGGACAGGTATCCGGTAAGCGGCA
GGGTCGGAACAGGAGAGCGCACGAGGGAGCTTCCAGGGGGAAACGCCTGGT
ATCTTTATAGTCCTGTCGGGTTTCGCCACCTCTGACTTGAGCGTCGATTTTTGT
GATGCTCGTCAGGGGGGCGGAGCCTATGGAAAAACGCCAGCAACGCGGCCTT
TTTACGGTTCCTGGCCTTTTGCTGGCCTTTTGCTCACATGT
**pGW147-pAAV_EFS-LTR-h41BBL-WPRE-sPA (SEQ ID NO: 53)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
ATGGAATACGCCTCTGACGCTTCACTGGACCCCGAAGCCCCGTGGCCTCCCGC
GCCCCGCGCTCGCGCCTGCCGCGTACTGCCTTGGGCCCTGGTCGCGGGGCTGC
TGCTGCTGCTGCTGCTCGCTGCCGCCTGCGCCGTCTTCCTCGCCTGCCCCTGG
GCCGTGTCCGGGGCTCGCGCCTCGCCCGGCTCCGCGGCCAGCCCGAGACTCC
GCGAGGGTCCCGAGCTTTCGCCCGACGATCCCGCCGGCCTCTTGGACCTGCG
GCAGGGCATGTTTGCGCAGCTGGTGGCCCAAAATGTTCTGCTGATCGATGGG
CCCCTGAGCTGGTACAGTGACCCAGGCCTGGCAGGCGTGTCCCTGACGGGG
GGCCTGAGCTACAAAGAGGACACGAAGGAGCTGGTGGTGGCCAAGGCTGGAG
TCTACTATGTCTTCTTTCAACTAGAGCTGCGGCGCGTGGTGGCCGGCGAGGGC
TCAGGCTCCGTTTCACTTGCGCTGCACCTGCAGCCACTGCGCTCTGCTGCTGG
GGCCGCCGCCCTGGCTTTGACCGTGGACCTGCCACCCGCCTCCTCCGAGGCTC
GGAACTCGGCCTTCGGTTTCCAGGGCCGCTTGCTGCACCTGAGTGCCGGCCAG
CGCCTGGGCGTCCATCTTCACACTGAGGCCAGGGCACGCCATGCCTGGCAGC
TTACCCAGGGCGCCACAGTCTTGGGACTCTTCCGGGTGACCCCCGAAATCCCA
GCCGGACTCCCTTCACCGAGGTCGGAATGAGaattcacgcgttaagtcgacaatcaacctctggatt
acaaaatttgtgaaagattgactggtattcttaactatgttgctccttttacgctatgtggatacgctgctttaatgcctttgtatcatgcta
ttgcttcccgtatggctttcattttctcctccttgtataaatcctggttgctgtctctttatgaggagttgtggcccgttgtcaggcaacgt
 
ggcgtggtgcactgtgtttgctgacgcaacccccactggttggggcattgccaccacctgtcagctcctttccgggactttcgct
ttccccctccctattgccacggcggaactcatcgccgcctgccttgcccgctgctggacaggggctcggctgttgggcactgaca
attccgtggtgttgtcggggaaatcatcgtcctttccttggctgctcgcctgtgttgccacctggattctgcgcgggacgtccttctg
ctacgtcccttcggccctcaatccagcggaccttccttcccgcggcctgctgccggctctgcggcctcttccgcgtcttcgccttcg
ccctcagacgagtcggatctccctttgggccgcctccccgcgtcgactttaagaccaatgacGGCCGaataaaagatctttatt
ttcattagatctgtgtgttggttttttgtgtgcggccgCAGGAACCCCTAGTGATGGAGTTGGCCACTC
CCTCTCTGCGCGCTCGCTCGCTCACTGAGGCCGGGCGACCAAAGGTCGCCCG
ACGCCCGGGCTTTGCCCGGGCGGCCTCAGTGAGCGAGCGAGCGCGCAGCTGC
CTGCAGGGGCGCCTGATGCGGTATTTTCTCCTTACGCATCTGTGCGGTATTTC
ACACCGCATACGTCAAAGCAACCATAGTACGCGCCCTGTAGCGGCGCATTAA
GCGCGGCGGGTGTGGTGGTTACGCGCAGCGTGACCGCTACACTTGCCAGCGC
CTTAGCGCCCGCTCCTTTCGCTTTCTTCCCTTCCTTTCTCGCCACGTTCGCCGG
CTTTCCCCGTCAAGCTCTAAATCGGGGGCTCCCTTTAGGGTTCCGATTTAGTG
CTTTACGGCACCTCGACCCCAAAAAACTTGATTTGGGTGATGGTTCACGTAGT
GGGCCATCGCCCTGATAGACGGTTTTTCGCCCTTTGACGTTGGAGTCCACGTT
CTTTAATAGTGGACTCTTGTTCCAAACTGGAACAACACTCAACTCTATCTCGG
GCTATTCTTTTGATTTATAAGGGATTTTGCCGATTTCGGTCTATTGGTTAAAAA
ATGAGCTGATTTAACAAAAATTTAACGCGAATTTTAACAAAATATTAACGTTT
ACAATTTTATGGTGCACTCTCAGTACAATCTGCTCTGATGCCGCATAGTTAAG
CCAGCCCCGACACCCGCCAACACCCGCTGACGCGCCCTGACGGGCTTGTCTG
CTCCCGGCATCCGCTTACAGACAAGCTGTGACCGTCTCCGGGAGCTGCATGTG
TCAGAGGTTTTCACCGTCATCACCGAAACGCGCGAGACGAAAGGGCCTCGTG
ATACGCCTATTTTTATAGGTTAATGTCATGATAATAATGGTTTCTTAGACGTC
AGGTGGCACTTTTCGGGGAAATGTGCGCGGAACCCCTATTTGTTTATTTTTCT
AAATACATTCAAATATGTATCCGCTCATGAGACAATAACCCTGATAAATGCTT
CAATAATATTGAAAAAGGAAGAGTATGAGTATTCAACATTTCCGTGTCGCCCT
TATTCCCTTTTTTGCGGCATTTTGCCTTCCTGTTTTTGCTCACCCAGAAACGCT
GGTGAAAGTAAAAGATGCTGAAGATCAGTTGGGTGCACGAGTGGGTTACATC
GAACTGGATCTCAACAGCGGTAAGATCCTTGAGAGTTTTCGCCCCGAAGAAC
GTTTTCCAATGATGAGCACTTTTAAAGTTCTGCTATGTGGCGCGGTATTATCC
CGTATTGACGCCGGGCAAGAGCAACTCGGTCGCCGCATACACTATTCTCAGA
ATGACTTGGTTGAGTACTCACCAGTCACAGAAAAGCATCTTACGGATGGCAT
GACAGTAAGAGAATTATGCAGTGCTGCCATAACCATGAGTGATAACACTGCG
GCCAACTTACTTCTGACAACGATCGGAGGACCGAAGGAGCTAACCGCTTTTTT
GCACAACATGGGGGATCATGTAACTCGCCTTGATCGTTGGGAACCGGAGCTG
AATGAAGCCATACCAAACGACGAGCGTGACACCACGATGCCTGTAGCAATGG
CAACAACGTTGCGCAAACTATTAACTGGCGAACTACTTACTCTAGCTTCCCGG
CAACAATTAATAGACTGGATGGAGGCGGATAAAGTTGCAGGACCACTTCTGC
GCTCGGCCCTTCCGGCTGGCTGGTTTATTGCTGATAAATCTGGAGCCGGTGAG
CGTGGAAGCCGCGGTATCATTGCAGCACTGGGGCCAGATGGTAAGCCCTCCC
GTATCGTAGTTATCTACACGACGGGGAGTCAGGCAACTATGGATGAACGAAA
TAGACAGATCGCTGAGATAGGTGCCTCACTGATTAAGCATTGGTAACTGTCA
GACCAAGTTTACTCATATATACTTTAGATTGATTTAAAACTTCATTTTTAATTT
AAAAGGATCTAGGTGAAGATCCTTTTTGATAATCTCATGACCAAAATCCCTTA
ACGTGAGTTTTCGTTCCACTGAGCGTCAGACCCCGTAGAAAAGATCAAAGGA
TCTTCTTGAGATCCTTTTTTTCTGCGCGTAATCTGCTGCTTGCAAACAAAAAAA
CCACCGCTACCAGCGGTGGTTTGTTTGCCGGATCAAGAGCTACCAACTCTTTT
TCCGAAGGTAACTGGCTTCAGCAGAGCGCAGATACCAAATACTGTTCTTCTAG
TGTAGCCGTAGTTAGGCCACCACTTCAAGAACTCTGTAGCACCGCCTACATAC
CTCGCTCTGCTAATCCTGTTACCAGTGGCTGCTGCCAGTGGCGATAAGTCGTG
 
TCTTACCGGGTTGGACTCAAGACGATAGTTACCGGATAAGGCGCAGCGGTCG
GGCTGAACGGGGGGTTCGTGCACACAGCCCAGCTTGGAGCGAACGACCTACA
CCGAACTGAGATACCTACAGCGTGAGCTATGAGAAAGCGCCACGCTTCCCGA
AGGGAGAAAGGCGGACAGGTATCCGGTAAGCGGCAGGGTCGGAACAGGAGA
GCGCACGAGGGAGCTTCCAGGGGGAAACGCCTGGTATCTTTATAGTCCTGTC
GGGTTTCGCCACCTCTGACTTGAGCGTCGATTTTTGTGATGCTCGTCAGGGGG
GCGGAGCCTATGGAAAAACGCCAGCAACGCGGCCTTTTTACGGTTCCTGGCC
TTTTGCTGGCCTTTTGCTCACATGT
**pGW149-pAAV_EFS-LTR-hLIGHT(Remove-EQLI)-RSR-WPRE-sPA (SEQ ID NO: 54)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
ATGGAGGAGAGTGTCGTACGGCCCTCAGTGTTTGTGGTGGATGGACAGACCG
ACATCCCATTCACGAGGCTGGGACGAAGCCACCGGAGACAGTCGTGCAGTGT
GGCCCGGGTGGGTCTGGGTCTCTTGCTGTTGCTGATGGGGGCCGGGCTGGCCG
TCCAAGGCTGGTTCCTCCTGCAGCTGCACTGGCGTCTAGGAGAGATGGTCACC
CGCCTGCCTGACGGACCTGCAGGCTCCTGGCAAGAGCGAAGGTCTCACGAGG
TCAACCCAGCAGCGCATCTCACAGGGGCCAACTCCAGCTTGACCGGCAGCGG
GGGGCCGCTGTTATGGGAGACTCAGCTGGGCCTGGCCTTCCTGAGGGGCCTC
AGCTACCACGATGGGGCCCTTGTGGTCACCAAAGCTGGCTACTACTACATCTA
CTCCAAGGTGCAGCTGGGCGGTGTGGGCTGCCCGCTGGGCCTGGCCAGCACC
ATCACCCACGGCCTCTACAAGCGCACACCCCGCTACCCCGAGGAGCTGGAGC
TGTTGGTCAGCCAGCAGTCACCCTGCGGACGGGCCACCAGCAGCTCCCGGGT
CTGGTGGGACAGCAGCTTCCTGGGTGGTGTGGTACACCTGGAGGCTGGGGAG
AAGGTGGTCGTCCGTGTGCTGGATGAACGCCTGGTTCGACTGCGTGATGGTAC
CCGGTCTTACTTCGGGGCTTTCATGGTGggtggaggcggttgcggaaagcgtaagTGAGaattca
cgcgttaagtcgacaatcaacctctggattacaaaatttgtgaaagattgactggtattcttaactatgttgctccttttacgctatgtgg
atacgctgctttaatgcctttgtatcatgctattgcttcccgtatggctttcattttctcctccttgtataaatcctggttgctgtctctttatg
aggagttgtggcccgttgtcaggcaacgtggcgtggtgtgcactgtgtttgctgacgcaacccccactggttggggcattgccac
cacctgtcagctcctttccgggactttcgctttccccctccctattgccacggcggaactcatcgccgcctgccttgcccgctgctg
gacaggggctcggctgttgggcactgacaattccgtggtgttgtcggggaaatcatcgtcctttccttggctgctcgcctgtgttgc
cacctggattctgcgcgggacgtccttctgctacgtcccttcggccctcaatccagcggaccttccttcccgcggcctgctgccgg
ctctgcggcctcttccgcgtcttcgccttcgccctcagacgagtcggatctccctttgggccgcctccccgcgtcgactttaagacc
aatgacGGCCGaataaaagatctttattttcattagatctgtgtgttggttttttgtgtgcggccgCAGGAACCCCTAG
TGATGGAGTTGGCCACTCCCTCTCTGCGCGCTCGCTCGCTCACTGAGGCCGGG
CGACCAAAGGTCGCCCGACGCCCGGGCTTTGCCCGGGCGGCCTCAGTGAGCG
AGCGAGCGCGCAGCTGCCTGCAGGGGCGCCTGATGCGGTATTTTCTCCTTACG
CATCTGTGCGGTATTTCACACCGCATACGTCAAAGCAACCATAGTACGCGCCC
 
TGTAGCGGCGCATTAAGCGCGGCGGGTGTGGTGGTTACGCGCAGCGTGACCG
CTACACTTGCCAGCGCCTTAGCGCCCGCTCCTTTCGCTTTCTTCCCTTCCTTTC
TCGCCACGTTCGCCGGCTTTCCCCGTCAAGCTCTAAATCGGGGGCTCCCTTTA
GGGTTCCGATTTAGTGCTTTACGGCACCTCGACCCCAAAAAACTTGATTTGGG
TGATGGTTCACGTAGTGGGCCATCGCCCTGATAGACGGTTTTTCGCCCTTTGA
CGTTGGAGTCCACGTTCTTTAATAGTGGACTCTTGTTCCAAACTGGAACAACA
CTCAACTCTATCTCGGGCTATTCTTTTGATTTATAAGGGATTTTGCCGATTTCG
GTCTATTGGTTAAAAAATGAGCTGATTTAACAAAAATTTAACGCGAATTTTAA
CAAAATATTAACGTTTACAATTTTATGGTGCACTCTCAGTACAATCTGCTCTG
ATGCCGCATAGTTAAGCCAGCCCCGACACCCGCCAACACCCGCTGACGCGCC
CTGACGGGCTTGTCTGCTCCCGGCATCCGCTTACAGACAAGCTGTGACCGTCT
CCGGGAGCTGCATGTGTCAGAGGTTTTCACCGTCATCACCGAAACGCGCGAG
ACGAAAGGGCCTCGTGATACGCCTATTTTTATAGGTTAATGTCATGATAATAA
TGGTTTCTTAGACGTCAGGTGGCACTTTTCGGGGAAATGTGCGCGGAACCCCT
ATTTGTTTATTTTTCTAAATACATTCAAATATGTATCCGCTCATGAGACAATAA
CCCTGATAAATGCTTCAATAATATTGAAAAAGGAAGAGTATGAGTATTCAAC
ATTTCCGTGTCGCCCTTATTCCCTTTTTTGCGGCATTTTGCCTTCCTGTTTTTGC
TCACCCAGAAACGCTGGTGAAAGTAAAAGATGCTGAAGATCAGTTGGGTGCA
CGAGTGGGTTACATCGAACTGGATCTCAACAGCGGTAAGATCCTTGAGAGTT
TTCGCCCCGAAGAACGTTTTCCAATGATGAGCACTTTTAAAGTTCTGCTATGT
GGCGCGGTATTATCCCGTATTGACGCCGGGCAAGAGCAACTCGGTCGCCGCA
TACACTATTCTCAGAATGACTTGGTTGAGTACTCACCAGTCACAGAAAAGCAT
CTTACGGATGGCATGACAGTAAGAGAATTATGCAGTGCTGCCATAACCATGA
GTGATAACACTGCGGCCAACTTACTTCTGACAACGATCGGAGGACCGAAGGA
GCTAACCGCTTTTTTGCACAACATGGGGGATCATGTAACTCGCCTTGATCGTT
GGGAACCGGAGCTGAATGAAGCCATACCAAACGACGAGCGTGACACCACGA
TGCCTGTAGCAATGGCAACAACGTTGCGCAAACTATTAACTGGCGAACTACTT
ACTCTAGCTTCCCGGCAACAATTAATAGACTGGATGGAGGCGGATAAAGTTG
CAGGACCACTTCTGCGCTCGGCCCTTCCGGCTGGCTGGTTTATTGCTGATAAA
TCTGGAGCCGGTGAGCGTGGAAGCCGCGGTATCATTGCAGCACTGGGGCCAG
ATGGTAAGCCCTCCCGTATCGTAGTTATCTACACGACGGGGAGTCAGGCAAC
TATGGATGAACGAAATAGACAGATCGCTGAGATAGGTGCCTCACTGATTAAG
CATTGGTAACTGTCAGACCAAGTTTACTCATATATACTTTAGATTGATTTAAA
ACTTCATTTTTAATTTAAAAGGATCTAGGTGAAGATCCTTTTTGATAATCTCAT
GACCAAAATCCCTTAACGTGAGTTTTCGTTCCACTGAGCGTCAGACCCCGTAG
AAAAGATCAAAGGATCTTCTTGAGATCCTTTTTTTCTGCGCGTAATCTGCTGC
TTGCAAACAAAAAAACCACCGCTACCAGCGGTGGTTTGTTTGCCGGATCAAG
AGCTACCAACTCTTTTTCCGAAGGTAACTGGCTTCAGCAGAGCGCAGATACCA
AATACTGTTCTTCTAGTGTAGCCGTAGTTAGGCCACCACTTCAAGAACTCTGT
AGCACCGCCTACATACCTCGCTCTGCTAATCCTGTTACCAGTGGCTGCTGCCA
GTGGCGATAAGTCGTGTCTTACCGGGTTGGACTCAAGACGATAGTTACCGGA
TAAGGCGCAGCGGTCGGGCTGAACGGGGGGTTCGTGCACACAGCCCAGCTTG
GAGCGAACGACCTACACCGAACTGAGATACCTACAGCGTGAGCTATGAGAAA
GCGCCACGCTTCCCGAAGGGAGAAAGGCGGACAGGTATCCGGTAAGCGGCA
GGGTCGGAACAGGAGAGCGCACGAGGGAGCTTCCAGGGGGAAACGCCTGGT
ATCTTTATAGTCCTGTCGGGTTTCGCCACCTCTGACTTGAGCGTCGATTTTTGT
GATGCTCGTCAGGGGGGCGGAGCCTATGGAAAAACGCCAGCAACGCGGCCTT
TTTACGGTTCCTGGCCTTTTGCTGGCCTTTTGCTCACATGT
**pGW036-EFS-dSaCas9-VP64 (SEQ ID NO: 55)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atggccccaaagaagaagcggaaggtcggtatccacggagtcccagcagccaagcggaactacatcctgggcctggccatc
ggcatcaccagcgtgggctacggcatcatcgactacgagacacgggacgtgatcgatgccggcgtgcggctgttcaaagagg
ccaacgtggaaaacaacgagggcaggcggagcaagagaggcgccagaaggctgaagcggcggaggcggcatagaatcc
agagagtgaagaagctgctgttcgactacaacctgctgaccgaccacagcgagctgagcggcatcaacccctacgaggccag
agtgaagggcctgagccagaagctgagcgaggaagagttctctgccgccctgctgcacctggccaagagaagaggcgtgca
caacgtgaacgaggtggaagaggacaccggcaacgagctgtccaccaaagagcagatcagccggaacagcaaggccctgg
aagagaaatacgtggccgaactgcagctggaacggctgaagaaagacggcgaagtgcggggcagcatcaacagattcaaga
ccagcgactacgtgaaagaagccaaacagctgctgaaggtgcagaaggcctaccaccagctggaccagagcttcatcgacac
ctacatcgacctgctggaaacccggcggacctactatgagggacctggcgagggcagccccttcggctggaaggacatcaaa
gaatggtacgagatgctgatgggccactgcacctacttccccgaggaactgcggagcgtgaagtacgcctacaacgccgacct
gtacaacgccctgaacgacctgaacaatctcgtgatcaccagggacgagaacgagaagctggaatattacgagaagttccaga
tcatcgagaacgtgttcaagcagaagaagaagcccaccctgaagcagatcgccaaagaaatcctcgtgaacgaagaggatatt
aagggctacagagtgaccagcaccggcaagcccgagttcaccaacctgaaggtgtaccacgacatcaaggacattaccgccc
ggaaagagattattgagaacgccgagctgctggatcagattgccaagatcctgaccatctaccagagcagcgaggacatccag
gaagaactgaccaatctgaactccgagctgacccaggaagagatcgagcagatctctaatctgaagggctataccggcaccca
caacctgagcctgaaggccatcaacctgatcctggacgagctgtggcacaccaacgacaaccagatcgctatcttcaaccggct
gaagctggtgcccaagaaggtggacctgtcccagcagaaagagatccccaccaccctggtggacgacttcatcctgagcccc
gtcgtgaagagaagcttcatccagagcatcaaagtgatcaacgccatcatcaagaagtacggcctgcccaacgacatcattatcg
agctggcccgcgagaagaactccaaggacgcccagaaaatgatcaacgagatgcagaagcggaaccggcagaccaacgag
cggatcgaggaaatcatccggaccaccggcaaagagaacgccaagtacctgatcgagaagatcaagctgcacgacatgcag
gaaggcaagtgcctgtacagcctggaagccatccctctggaagatctgctgaacaaccccttcaactatgaggtggaccacatc
atccccagaagcgtgtccttcgacaacagcttcaacaacaaggtgctcgtgaagcaggaagaagccagcaagaagggcaacc
ggaccccattccagtacctgagcagcagcgacagcaagatcagctacgaaaccttcaagaagcacatcctgaatctggccaag
ggcaagggcagaatcagcaagaccaagaaagagtatctgctggaagaacgggacatcaacaggttctccgtgcagaaagact
tcatcaaccggaacctggtggataccagatacgccaccagaggcctgatgaacctgctgcggagctacttcagagtgaacaac
ctggacgtgaaagtgaagtccatcaatggcggcttcaccagctttctgcggcggaagtggaagtttaagaaagagcggaacaa
ggggtacaagcaccacgccgaggacgccctgatcattgccaacgccgatttcatcttcaaagagtggaagaaactggacaagg
ccaaaaaagtgatggaaaaccagatgttcgaggaaaagcaggccgagagcatgcccgagatcgaaaccgagcaggagtaca
aagagatcttcatcaccccccaccagatcaagcacattaaggacttcaaggactacaagtacagccaccgggtggacaagaag
cctaatagagagctgattaacgacaccctgtactccacccggaaggacgacaagggcaacaccctgatcgtgaacaatctgaa
cggcctgtacgacaaggacaatgacaagctgaaaaagctgatcaacaagagccccgaaaagctgctgatgtaccaccacgac
ccccagacctaccagaaactgaagctgattatggaacagtacggcgacgagaagaatcccctgtacaagtactacgaggaaac
cgggaactacctgaccaagtactccaaaaaggacaacggccccgtgatcaagaagattaagtattacggcaacaaactgaacg
cccatctggacatcaccgacgactaccccaacagcagaaacaaggtcgtgaagctgtccctgaagccctacagattcgacgtgt
acctggacaatggcgtgtacaagttcgtgaccgtgaagaatctggatgtgatcaaaaaagaaaactactacgaagtgaatagcaa
gtgctatgaggaagctaagaagctgaagaagatcagcaaccaggccgagtttatcgcctccttctacaacaacgatctgatcaag
atcaacggcgagctgtatagagtgatcggcgtgaacaacgacctgctgaaccggatcgaagtgaacatgatcgacatcacctac
 
cgcgagtacctggaaaacatgaacgacaagaggccccccaggatcattaagacaatcgcctccaagacccagagcattaaga
agtacagcacagacattctgggcaacctgtatgaagtgaaatctaagaagcaccctcagatcatcaaaaagggcaaaaggccg
gcggccacgaaaaaggccggccaggcaaaaaagaaaaagggatccGGACGGGCTGACGCATTGGACG
ATTTTGATCTGGATATGCTGGGAAGTGACGCCCTCGATGATTTTGACCTTGAC
ATGCTTGGTTCGGATGCCCTTGATGACTTTGACCTCGACATGCTCGGCAGTGA
CGCCCTTGATGATTTCGACCTGGACATGCTGATTTAAGAATTCAATAAAAGAT
CTTTATTTTCATTAGATCTGTGTGTTGGTTTTTTGTGTGCGGCCGCAGGAACCC
CTAGTGATGGAGTTGGCCACTCCCTCTCTGCGCGCTCGCTCGCTCACTGAGGC
CGGGCGACCAAAGGTCGCCCGACGCCCGGGCTTTGCCCGGGCGGCCTCAGTG
AGCGAGCGAGCGCGCAGCTGCCTGCAGGGGCGCCTGATGCGGTATTTTCTCC
TTACGCATCTGTGCGGTATTTCACACCGCATACGTCAAAGCAACCATAGTACG
CGCCCTGTAGCGGCGCATTAAGCGCGGCGGGTGTGGTGGTTACGCGCAGCGT
GACCGCTACACTTGCCAGCGCCTTAGCGCCCGCTCCTTTCGCTTTCTTCCCTTC
CTTTCTCGCCACGTTCGCCGGCTTTCCCCGTCAAGCTCTAAATCGGGGGCTCC
CTTTAGGGTTCCGATTTAGTGCTTTACGGCACCTCGACCCCAAAAAACTTGAT
TTGGGTGATGGTTCACGTAGTGGGCCATCGCCCTGATAGACGGTTTTTCGCCC
TTTGACGTTGGAGTCCACGTTCTTTAATAGTGGACTCTTGTTCCAAACTGGAA
CAACACTCAACTCTATCTCGGGCTATTCTTTTGATTTATAAGGGATTTTGCCGA
TTTCGGTCTATTGGTTAAAAAATGAGCTGATTTAACAAAAATTTAACGCGAAT
TTTAACAAAATATTAACGTTTACAATTTTATGGTGCACTCTCAGTACAATCTG
CTCTGATGCCGCATAGTTAAGCCAGCCCCGACACCCGCCAACACCCGCTGAC
GCGCCCTGACGGGCTTGTCTGCTCCCGGCATCCGCTTACAGACAAGCTGTGAC
CGTCTCCGGGAGCTGCATGTGTCAGAGGTTTTCACCGTCATCACCGAAACGCG
CGAGACGAAAGGGCCTCGTGATACGCCTATTTTTATAGGTTAATGTCATGATA
ATAATGGTTTCTTAGACGTCAGGTGGCACTTTTCGGGGAAATGTGCGCGGAAC
CCCTATTTGTTTATTTTTCTAAATACATTCAAATATGTATCCGCTCATGAGACA
ATAACCCTGATAAATGCTTCAATAATATTGAAAAAGGAAGAGTATGAGTATT
CAACATTTCCGTGTCGCCCTTATTCCCTTTTTTGCGGCATTTTGCCTTCCTGTTT
TTGCTCACCCAGAAACGCTGGTGAAAGTAAAAGATGCTGAAGATCAGTTGGG
TGCACGAGTGGGTTACATCGAACTGGATCTCAACAGCGGTAAGATCCTTGAG
AGTTTTCGCCCCGAAGAACGTTTTCCAATGATGAGCACTTTTAAAGTTCTGCT
ATGTGGCGCGGTATTATCCCGTATTGACGCCGGGCAAGAGCAACTCGGTCGC
CGCATACACTATTCTCAGAATGACTTGGTTGAGTACTCACCAGTCACAGAAAA
GCATCTTACGGATGGCATGACAGTAAGAGAATTATGCAGTGCTGCCATAACC
ATGAGTGATAACACTGCGGCCAACTTACTTCTGACAACGATCGGAGGACCGA
AGGAGCTAACCGCTTTTTTGCACAACATGGGGGATCATGTAACTCGCCTTGAT
CGTTGGGAACCGGAGCTGAATGAAGCCATACCAAACGACGAGCGTGACACCA
CGATGCCTGTAGCAATGGCAACAACGTTGCGCAAACTATTAACTGGCGAACT
ACTTACTCTAGCTTCCCGGCAACAATTAATAGACTGGATGGAGGCGGATAAA
GTTGCAGGACCACTTCTGCGCTCGGCCCTTCCGGCTGGCTGGTTTATTGCTGA
TAAATCTGGAGCCGGTGAGCGTGGAAGCCGCGGTATCATTGCAGCACTGGGG
CCAGATGGTAAGCCCTCCCGTATCGTAGTTATCTACACGACGGGGAGTCAGG
CAACTATGGATGAACGAAATAGACAGATCGCTGAGATAGGTGCCTCACTGAT
TAAGCATTGGTAACTGTCAGACCAAGTTTACTCATATATACTTTAGATTGATT
TAAAACTTCATTTTTAATTTAAAAGGATCTAGGTGAAGATCCTTTTTGATAAT
CTCATGACCAAAATCCCTTAACGTGAGTTTTCGTTCCACTGAGCGTCAGACCC
CGTAGAAAAGATCAAAGGATCTTCTTGAGATCCTTTTTTTCTGCGCGTAATCT
GCTGCTTGCAAACAAAAAAACCACCGCTACCAGCGGTGGTTTGTTTGCCGGA
TCAAGAGCTACCAACTCTTTTTCCGAAGGTAACTGGCTTCAGCAGAGCGCAG
ATACCAAATACTGTTCTTCTAGTGTAGCCGTAGTTAGGCCACCACTTCAAGAA
 
CTCTGTAGCACCGCCTACATACCTCGCTCTGCTAATCCTGTTACCAGTGGCTG
CTGCCAGTGGCGATAAGTCGTGTCTTACCGGGTTGGACTCAAGACGATAGTTA
CCGGATAAGGCGCAGCGGTCGGGCTGAACGGGGGGTTCGTGCACACAGCCCA
GCTTGGAGCGAACGACCTACACCGAACTGAGATACCTACAGCGTGAGCTATG
AGAAAGCGCCACGCTTCCCGAAGGGAGAAAGGCGGACAGGTATCCGGTAAG
CGGCAGGGTCGGAACAGGAGAGCGCACGAGGGAGCTTCCAGGGGGAAACGC
CTGGTATCTTTATAGTCCTGTCGGGTTTCGCCACCTCTGACTTGAGCGTCGATT
TTTGTGATGCTCGTCAGGGGGGCGGAGCCTATGGAAAAACGCCAGCAACGCG
GCCTTTTTACGGTTCCTGGCCTTTTGCTGGCCTTTTGCTCACATGT
**pGW011b_pAAV-EFS-dCAS9-spA (SEQ ID NO: 56)** 
cctgcaggcagctgcgcgctcgctcgctcactgaggccgcccgggcaaagcccgggcgtcgggcgacctttggtcgcccgg
cctcagtgagcgagcgagcgcgcagagagggagtggccaactccatcactaggggttcctgcggccgcacgcgttctaggtc
ttgaaaggagtgggaattggctccggtgcccgtcagtgggcagagcgcacatcgcccacagtccccgagaagttggggggag
gggtcggcaattgatccggtgcctagagaaggtggcgcggggtaaactgggaaagtgatgtcgtgtactggctccgcctttttcc
cgagggtgggggagaaccgtatataagtgcagtagtcgccgtgaacgttctttttcgcaacgggtttgccgccagaacacaggT
GTCGTGACGCGcGTACGtaatacgactcactatagggccgccaccATGAAAAGGCCGGCGGCC
ACGAAAAAGGCCGGCCAGGCAAAAAAGAAAAAGGACAAGAAGTACAGCATC
GGCCTGGCCATCGGCACCAACTCTGTGGGCTGGGCCGTGATCACCGACGAGT
ACAAGGTGCCCAGCAAGAAATTCAAGGTGCTGGGCAACACCGACCGGCACA
GCATCAAGAAGAACCTGATCGGAGCCCTGCTGTTCGACAGCGGCGAAACAGC
CGAGGCCACCCGGCTGAAGAGAACCGCCAGAAGAAGATACACCAGACGGAA
GAACCGGATCTGCTATCTGCAAGAGATCTTCAGCAACGAGATGGCCAAGGTG
GACGACAGCTTCTTCCACAGACTGGAAGAGTCCTTCCTGGTGGAAGAGGATA
AGAAGCACGAGCGGCACCCCATCTTCGGCAACATCGTGGACGAGGTGGCCTA
CCACGAGAAGTACCCCACCATCTACCACCTGAGAAAGAAACTGGTGGACAGC
ACCGACAAGGCCGACCTGCGGCTGATCTATCTGGCCCTGGCCCACATGATCA
AGTTCCGGGGCCACTTCCTGATCGAGGGCGACCTGAACCCCGACAACAGCGA
CGTGGACAAGCTGTTCATCCAGCTGGTGCAGACCTACAACCAGCTGTTCGAG
GAAAACCCCATCAACGCCAGCGGCGTGGACGCCAAGGCCATCCTGTCTGCCA
GACTGAGCAAGAGCAGACGGCTGGAAAATCTGATCGCCCAGCTGCCCGGCGA
GAAGAAGAATGGCCTGTTCGGCAACCTGATTGCCCTGAGCCTGGGCCTGACC
CCCAACTTCAAGAGCAACTTCGACCTGGCCGAGGATGCCAAACTGCAGCTGA
GCAAGGACACCTACGACGACGACCTGGACAACCTGCTGGCCCAGATCGGCGA
CCAGTACGCCGACCTGTTTCTGGCCGCCAAGAACCTGTCCGACGCCATCCTGC
TGAGCGACATCCTGAGAGTGAACACCGAGATCACCAAGGCCCCCCTGAGCGC
CTCTATGATCAAGAGATACGACGAGCACCACCAGGACCTGACCCTGCTGAAA
GCTCTCGTGCGGCAGCAGCTGCCTGAGAAGTACAAAGAGATTTTCTTCGACC
AGAGCAAGAACGGCTACGCCGGCTACATTGACGGCGGAGCCAGCCAGGAAG
AGTTCTACAAGTTCATCAAGCCCATCCTGGAAAAGATGGACGGCACCGAGGA
ACTGCTCGTGAAGCTGAACAGAGAGGACCTGCTGCGGAAGCAGCGGACCTTC
GACAACGGCAGCATCCCCCACCAGATCCACCTGGGAGAGCTGCACGCCATTC
TGCGGCGGCAGGAAGATTTTTACCCATTCCTGAAGGACAACCGGGAAAAGAT
CGAGAAGATCCTGACCTTCCGCATCCCCTACTACGTGGGCCCTCTGGCCAGGG
GAAACAGCAGATTCGCCTGGATGACCAGAAAGAGCGAGGAAACCATCACCC
CCTGGAACTTCGAGGAAGTGGTGGACAAGGGCGCTTCCGCCCAGAGCTTCAT
CGAGCGGATGACCAACTTCGATAAGAACCTGCCCAACGAGAAGGTGCTGCCC
AAGCACAGCCTGCTGTACGAGTACTTCACCGTGTATAACGAGCTGACCAAAG
TGAAATACGTGACCGAGGGAATGAGAAAGCCCGCCTTCCTGAGCGGCGAGCA
GAAAAAGGCCATCGTGGACCTGCTGTTCAAGACCAACCGGAAAGTGACCGTG
 
AAGCAGCTGAAAGAGGACTACTTCAAGAAAATCGAGTGCTTCGACTCCGTGG
AAATCTCCGGCGTGGAAGATCGGTTCAACGCCTCCCTGGGCACATACCACGA
TCTGCTGAAAATTATCAAGGACAAGGACTTCCTGGACAATGAGGAAAACGAG
GACATTCTGGAAGATATCGTGCTGACCCTGACACTGTTTGAGGACAGAGAGA
TGATCGAGGAACGGCTGAAAACCTATGCCCACCTGTTCGACGACAAAGTGAT
GAAGCAGCTGAAGCGGCGGAGATACACCGGCTGGGGCAGGCTGAGCCGGAA
GCTGATCAACGGCATCCGGGACAAGCAGTCCGGCAAGACAATCCTGGATTTC
CTGAAGTCCGACGGCTTCGCCAACAGAAACTTCATGCAGCTGATCCACGACG
ACAGCCTGACCTTTAAAGAGGACATCCAGAAAGCCCAGGTGTCCGGCCAGGG
CGATAGCCTGCACGAGCACATTGCCAATCTGGCCGGCAGCCCCGCCATTAAG
AAGGGCATCCTGCAGACAGTGAAGGTGGTGGACGAGCTCGTGAAAGTGATGG
GCCGGCACAAGCCCGAGAACATCGTGATCGAAATGGCCAGAGAGAACCAGA
CCACCCAGAAGGGACAGAAGAACAGCCGCGAGAGAATGAAGCGGATCGAAG
AGGGCATCAAAGAGCTGGGCAGCCAGATCCTGAAAGAACACCCCGTGGAAA
ACACCCAGCTGCAGAACGAGAAGCTGTACCTGTACTACCTGCAGAATGGGCG
GGATATGTACGTGGACCAGGAACTGGACATCAACCGGCTGTCCGACTACGAT
GTGGACCACATCGTGCCTCAGAGCTTTCTGAAGGACGACTCCATCGACAACA
AGGTGCTGACCAGAAGCGACAAGGCCCGGGGCAAGAGCGACAACGTGCCCT
CCGAAGAGGTCGTGAAGAAGATGAAGAACTACTGGCGGCAGCTGCTGAACG
CCAAGCTGATTACCCAGAGAAAGTTCGACAATCTGACCAAGGCCGAGAGAGG
CGGCCTGAGCGAACTGGATAAGGCCGGCTTCATCAAGAGACAGCTGGTGGAA
ACCCGGCAGATCACAAAGCACGTGGCACAGATCCTGGACTCCCGGATGAACA
CTAAGTACGACGAGAATGACAAGCTGATCCGGGAAGTGAAAGTGATCACCCT
GAAGTCCAAGCTGGTGTCCGATTTCCGGAAGGATTTCCAGTTTTACAAAGTGC
GCGAGATCAACAACTACCACCACGCCCACGACGCCTACCTGAACGCCGTCGT
GGGAACCGCCCTGATCAAAAAGTACCCTAAGCTGGAAAGCGAGTTCGTGTAC
GGCGACTACAAGGTGTACGACGTGCGGAAGATGATCGCCAAGAGCGAGCAG
GAAATCGGCAAGGCTACCGCCAAGTACTTCTTCTACAGCAACATCATGAACTT
TTTCAAGACCGAGATTACCCTGGCCAACGGCGAGATCCGGAAGCGGCCTCTG
ATCGAGACAAACGGCGAAACCGGGGAGATCGTGTGGGATAAGGGCCGGGAT
TTTGCCACCGTGCGGAAAGTGCTGAGCATGCCCCAAGTGAATATCGTGAAAA
AGACCGAGGTGCAGACAGGCGGCTTCAGCAAAGAGTCTATCCTGCCCAAGAG
GAACAGCGATAAGCTGATCGCCAGAAAGAAGGACTGGGACCCTAAGAAGTA
CGGCGGCTTCGACAGCCCCACCGTGGCCTATTCTGTGCTGGTGGTGGCCAAAG
TGGAAAAGGGCAAGTCCAAGAAACTGAAGAGTGTGAAAGAGCTGCTGGGGA
TCACCATCATGGAAAGAAGCAGCTTCGAGAAGAATCCCATCGACTTTCTGGA
AGCCAAGGGCTACAAAGAAGTGAAAAAGGACCTGATCATCAAGCTGCCTAA
GTACTCCCTGTTCGAGCTGGAAAACGGCCGGAAGAGAATGCTGGCCTCTGCC
GGCGAACTGCAGAAGGGAAACGAACTGGCCCTGCCCTCCAAATATGTGAACT
TCCTGTACCTGGCCAGCCACTATGAGAAGCTGAAGGGCTCCCCCGAGGATAA
TGAGCAGAAACAGCTGTTTGTGGAACAGCACAAGCACTACCTGGACGAGATC
ATCGAGCAGATCAGCGAGTTCTCCAAGAGAGTGATCCTGGCCGACGCTAATC
TGGACAAAGTGCTGTCCGCCTACAACAAGCACCGGGATAAGCCCATCAGAGA
GCAGGCCGAGAATATCATCCACCTGTTTACCCTGACCAATCTGGGAGCCCCTG
CCGCCTTCAAGTACTTTGACACCACCATCGACCGGAAGAGGTACACCAGCAC
CAAAGAGGTGCTGGACGCCACCCTGATCCACCAGAGCATCACCGGCCTGTAC
GAGACACGGATCGACCTGTCTCAGCTGGGAGGCGACagccctaagaaaaagaggaaggtg
AGCtgagaattcAATAAATAAAAGATCTTTATTTTCATTAGATCTGTGTGTTGGTTT
TTTGTGTGcggaccgagcggccgcaggaacccctagtgatggagttggccactccctctctgcgcgctcgctcgctcac
tgaggccgggcgaccaaaggtcgcccgacgcccgggctttgcccgggcggcctcagtgagcgagcgagcgcgcagctgcc
 
tgcaggggcgcctgatgcggtattttctccttacgcatctgtgcggtatttcacaccgcatacgtcaaagcaaccatagtacgcgc
cctgtagcggcgcattaagcgcggcgggtgtggtggttacgcgcagcgtgaccgctacacttgccagcgccctagcgcccgct
cctttcgctttcttcccttcctttctcgccacgttcgccggctttccccgtcaagctctaaatcgggggctccctttagggttccgattta
gtgctttacggcacctcgaccccaaaaaacttgatttgggtgatggttcacgtagtgggccatcgccctgatagacggtttttcgcc
ctttgacgttggagtccacgttctttaatagtggactcttgttccaaactggaacaacactcaaccctatctcgggctattcttttgattt
ataagggattttgccgatttcggcctattggttaaaaaatgagctgatttaacaaaaatttaacgcgaattttaacaaaatattaacgttt
acaattttatggtgcactctcagtacaatctgctctgatgccgcatagttaagccagccccgacacccgccaacacccgctgacgc
gccctgacgggcttgtctgctcccggcatccgcttacagacaagctgtgaccgtctccgggagctgcatgtgtcagaggttttcac
cgtcatcaccgaaacgcgcgagacgaaagggcctcgtgatacgcctatttttataggttaatgtcatgataataatggtttcttagac
gtcaggtggcacttttcggggaaatgtgcgcggaacccctatttgtttatttttctaaatacattcaaatatgtatccgctcatgagaca
ataaccctgataaatgcttcaataatattgaaaaaggaagagtatgagtattcaacatttccgtgtcgcccttattcccttttttgcggc
attttgccttcctgtttttgctcacccagaaacgctggtgaaagtaaaagatgctgaagatcagttgggtgcacgagtgggttacatc
gaactggatctcaacagcggtaagatccttgagagttttcgccccgaagaacgttttccaatgatgagcacttttaaagttctgctat
gtggcgcggtattatcccgtattgacgccgggcaagagcaactcggtcgccgcatacactattctcagaatgacttggttgagtac
tcaccagtcacagaaaagcatcttacggatggcatgacagtaagagaattatgcagtgctgccataaccatgagtgataacactg
cggccaacttacttctgacaacgatcggaggaccgaaggagctaaccgcttttttgcacaacatgggggatcatgtaactcgcctt
gatcgttgggaaccggagctgaatgaagccataccaaacgacgagcgtgacaccacgatgcctgtagcaatggcaacaacgtt
gcgcaaactattaactggcgaactacttactctagcttcccggcaacaattaatagactggatggaggcggataaagttgcagga
ccacttctgcgctcggcccttccggctggctggtttattgctgataaatctggagccggtgagcgtgggtctcgcggtatcattgca
gcactggggccagatggtaagccctcccgtatcgtagttatctacacgacggggagtcaggcaactatggatgaacgaaataga
cagatcgctgagataggtgcctcactgattaagcattggtaactgtcagaccaagtttactcatatatactttagattgatttaaaactt
catttttaatttaaaaggatctaggtgaagatcctttttgataatctcatgaccaaaatcccttaacgtgagttttcgttccactgagcgt
cagaccccgtagaaaagatcaaaggatcttcttgagatcctttttttctgcgcgtaatctgctgcttgcaaacaaaaaaaccaccgct
accagcggtggtttgtttgccggatcaagagctaccaactctttttccgaaggtaactggcttcagcagagcgcagataccaaata
ctgtccttctagtgtagccgtagttaggccaccacttcaagaactctgtagcaccgcctacatacctcgctctgctaatcctgttacc
agtggctgctgccagtggcgataagtcgtgtcttaccgggttggactcaagacgatagttaccggataaggcgcagcggtcggg
ctgaacggggggttcgtgcacacagcccagcttggagcgaacgacctacaccgaactgagatacctacagcgtgagctatgag
aaagcgccacgcttcccgaagggagaaaggcggacaggtatccggtaagcggcagggtcggaacaggagagcgcacgag
ggagcttccagggggaaacgcctggtatctttatagtcctgtcgggtttcgccacctctgacttgagcgtcgatttttgtgatgctcg
tcaggggggcggagcctatggaaaaacgccagcaacgcggcctttttacggttcctggccttttgctggccttttgctcacatgt
**pGW089-pAAV-EFSLTR-IL2-sPA (SEQ ID NO: 57)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccaca
tgtacagcatgcagctcgcatcctgtgtcacattgacacttgtgctccttgtcaacagcgcacccacttcaagctccacttcaagctc
tacagcggaagcacagcagcagcagcagcagcagcagcagcagcagcagcacctggagcagctgttgatggacctacagg
agctcctgagcaggatggagaattacaggaacctgaaactccccaggatgctcaccttcaaattttacttgcccaagcaggccac
agaattgaaagatcttcagtgcctagaagatgaacttggacctctgcggcatgttctggatttgactcaaagcaaaagctttcaattg
gaagatgctgagaatttcatcagcaatatcagagtaactgttgtaaaactaaagggctctgacaacacatttgagtgccaattcgat
 
gatgagtcagcaactgtggtggactttctgaggagatggatagccttctgtcaaagcatcatctcaacaagccctcaataaGAA
TTCAATAAAAGATCTTTATTTTCATTAGATCTGTGTGTTGGTTTTTTGTGTGCG
GCCGCAGGAACCCCTAGTGATGGAGTTGGCCACTCCCTCTCTGCGCGCTCGCT
CGCTCACTGAGGCCGGGCGACCAAAGGTCGCCCGACGCCCGGGCTTTGCCCG
GGCGGCCTCAGTGAGCGAGCGAGCGCGCAGCTGCCTGCAGGGGCGCCTGATG
CGGTATTTTCTCCTTACGCATCTGTGCGGTATTTCACACCGCATACGTCAAAG
CAACCATAGTACGCGCCCTGTAGCGGCGCATTAAGCGCGGCGGGTGTGGTGG
TTACGCGCAGCGTGACCGCTACACTTGCCAGCGCCTTAGCGCCCGCTCCTTTC
GCTTTCTTCCCTTCCTTTCTCGCCACGTTCGCCGGCTTTCCCCGTCAAGCTCTA
AATCGGGGGCTCCCTTTAGGGTTCCGATTTAGTGCTTTACGGCACCTCGACCC
CAAAAAACTTGATTTGGGTGATGGTTCACGTAGTGGGCCATCGCCCTGATAG
ACGGTTTTTCGCCCTTTGACGTTGGAGTCCACGTTCTTTAATAGTGGACTCTTG
TTCCAAACTGGAACAACACTCAACTCTATCTCGGGCTATTCTTTTGATTTATA
AGGGATTTTGCCGATTTCGGTCTATTGGTTAAAAAATGAGCTGATTTAACAAA
AATTTAACGCGAATTTTAACAAAATATTAACGTTTACAATTTTATGGTGCACT
CTCAGTACAATCTGCTCTGATGCCGCATAGTTAAGCCAGCCCCGACACCCGCC
AACACCCGCTGACGCGCCCTGACGGGCTTGTCTGCTCCCGGCATCCGCTTACA
GACAAGCTGTGACCGTCTCCGGGAGCTGCATGTGTCAGAGGTTTTCACCGTCA
TCACCGAAACGCGCGAGACGAAAGGGCCTCGTGATACGCCTATTTTTATAGG
TTAATGTCATGATAATAATGGTTTCTTAGACGTCAGGTGGCACTTTTCGGGGA
AATGTGCGCGGAACCCCTATTTGTTTATTTTTCTAAATACATTCAAATATGTAT
CCGCTCATGAGACAATAACCCTGATAAATGCTTCAATAATATTGAAAAAGGA
AGAGTATGAGTATTCAACATTTCCGTGTCGCCCTTATTCCCTTTTTTGCGGCAT
TTTGCCTTCCTGTTTTTGCTCACCCAGAAACGCTGGTGAAAGTAAAAGATGCT
GAAGATCAGTTGGGTGCACGAGTGGGTTACATCGAACTGGATCTCAACAGCG
GTAAGATCCTTGAGAGTTTTCGCCCCGAAGAACGTTTTCCAATGATGAGCACT
TTTAAAGTTCTGCTATGTGGCGCGGTATTATCCCGTATTGACGCCGGGCAAGA
GCAACTCGGTCGCCGCATACACTATTCTCAGAATGACTTGGTTGAGTACTCAC
CAGTCACAGAAAAGCATCTTACGGATGGCATGACAGTAAGAGAATTATGCAG
TGCTGCCATAACCATGAGTGATAACACTGCGGCCAACTTACTTCTGACAACGA
TCGGAGGACCGAAGGAGCTAACCGCTTTTTTGCACAACATGGGGGATCATGT
AACTCGCCTTGATCGTTGGGAACCGGAGCTGAATGAAGCCATACCAAACGAC
GAGCGTGACACCACGATGCCTGTAGCAATGGCAACAACGTTGCGCAAACTAT
TAACTGGCGAACTACTTACTCTAGCTTCCCGGCAACAATTAATAGACTGGATG
GAGGCGGATAAAGTTGCAGGACCACTTCTGCGCTCGGCCCTTCCGGCTGGCT
GGTTTATTGCTGATAAATCTGGAGCCGGTGAGCGTGGAAGCCGCGGTATCATT
GCAGCACTGGGGCCAGATGGTAAGCCCTCCCGTATCGTAGTTATCTACACGA
CGGGGAGTCAGGCAACTATGGATGAACGAAATAGACAGATCGCTGAGATAG
GTGCCTCACTGATTAAGCATTGGTAACTGTCAGACCAAGTTTACTCATATATA
CTTTAGATTGATTTAAAACTTCATTTTTAATTTAAAAGGATCTAGGTGAAGAT
CCTTTTTGATAATCTCATGACCAAAATCCCTTAACGTGAGTTTTCGTTCCACTG
AGCGTCAGACCCCGTAGAAAAGATCAAAGGATCTTCTTGAGATCCTTTTTTTC
TGCGCGTAATCTGCTGCTTGCAAACAAAAAAACCACCGCTACCAGCGGTGGT
TTGTTTGCCGGATCAAGAGCTACCAACTCTTTTTCCGAAGGTAACTGGCTTCA
GCAGAGCGCAGATACCAAATACTGTTCTTCTAGTGTAGCCGTAGTTAGGCCAC
CACTTCAAGAACTCTGTAGCACCGCCTACATACCTCGCTCTGCTAATCCTGTT
ACCAGTGGCTGCTGCCAGTGGCGATAAGTCGTGTCTTACCGGGTTGGACTCAA
GACGATAGTTACCGGATAAGGCGCAGCGGTCGGGCTGAACGGGGGGTTCGTG
CACACAGCCCAGCTTGGAGCGAACGACCTACACCGAACTGAGATACCTACAG
CGTGAGCTATGAGAAAGCGCCACGCTTCCCGAAGGGAGAAAGGCGGACAGG
 
TATCCGGTAAGCGGCAGGGTCGGAACAGGAGAGCGCACGAGGGAGCTTCCA
GGGGGAAACGCCTGGTATCTTTATAGTCCTGTCGGGTTTCGCCACCTCTGACT
TGAGCGTCGATTTTTGTGATGCTCGTCAGGGGGGCGGAGCCTATGGAAAAAC
GCCAGCAACGCGGCCTTTTTACGGTTCCTGGCCTTTTGCTGGCCTTTTGCTCAC
ATGT
**pGW094-pAAV_EFSLTR-Ifng-P2A-Cd80-T2A-light-P2A-Gitrl-E2A-41BBL-sPA (SEQ ID NO: 58)** 
CCTGCAGGCAGCTGCGCGCTCGCTCGCTCACTGAGGCCGCCCGGGCGTCGGG
CGACCTTTGGTCGCCCGGCCTCAGTGAGCGAGCGAGCGCGCAGAGAGGGAGT
GGCCAACTCCATCACTAGGGGTTCCTGCGGCCTCTAGAAAGGATCTGCGATC
GCTCCGGTGCCCGTCAGTGGGCAGAGCGCACATCGCCCACAGTCCCCGAGAA
GTTGGGGGGAGGGGTCGGCAATTGAACGGGTGCCTAGAGAAGGTGGCGCGG
GGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAAC
GGGTTTGCCGCCAGAACACAGCTGAAGCTTCGAGGGGCTCGCATCTCTCCTTC
ACGCGCCCGCCGCCCTACCTGAGGCCGCCATCCACGCCGGTTGAGTCGCGTTC
TGCCGCCTCCCGCCTGTGGTGCCTCCTGAACTGCGTCCGCCGTCTAGGTAAGT
TTAAAGCTCAGGTCGAGACCGGGCCTTTGTCCGGCGCTCCCTTGGAGCCTACC
TAGACTCAGCCGGCTCTCCACGCTTTGCCTGACCCTGCTTGCTCAACTCTACG
TCTTTGTTTCGTTTTCTGTTCTGCGCCGTTACAGATCTGGCTAACTAccggtgccacc
atgaacgctacacactgcatcttggctttgcagctcttcctcatggctgtttctggctgttactgccacggcacagtcattgaaagcct
agaaagtctgaataactattttaactcaagtggcatagatgtggaagaaaagagtctcttcttggatatctggaggaactggcaaaa
ggatggtgacatgaaaatcctgcagagccagattatctctttctacctcagactctttgaagtcttgaaagacaatcaggccatcag
caacaacataagcgtcattgaatcacacctgattactaccttcttcagcaacagcaaggcgaaaaaggatgcattcatgagtattg
ccaagtttgaggtcaacaacccacaggtccagcgccaagcattcaatgagctcatccgagtggtccaccagctgttgccggaat
ccagcctcaggaagcggaaaaggagtcgctgcGGATCCGGCGCAACAAACTTCTCTCTGCTGAA
ACAAGCCGGAGATGTCGAAGAGAATCCTGGACCGatggcttgcaattgtcagttgatgcaggat
acaccactcctcaagtttccatgtccaaggctcattcttctctttgtgctgctgattcgtctttcacaagtgtcttcagatgttgatgaac
aactgtccaagtcagtgaaagataaggtattgctgccttgccgttacaactctcctcatgaagatgagtctgaagaccgaatctact
ggcaaaaacatgacaaagtggtgctgtctgtcattgTtgggaaactaaaagtgtggcccgagtataagaaccggactttatatga
caacactacctactctcttatcatcctgggcctggtcctttcagaccggggcacatacagctgtgtcgttcaaaagaaggaaagag
gaacgtatgaagttaaacacttggctttagtaaagttgtccatcaaagctgacttctctacccccaacataactgagtctggaaaccc
atctgcagacactaaaaggattacctgctttgcttccgggggtttcccaaagcctcgcttctcttggttggaaaatggaagagaatta
cctggcatcaatacgacaatttcccaggatcctgaatctgaattgtacaccattagtagccaactagatttcaatacgactcgcaac
cacaccattaagtgtctcattaaatatggagatgctcacgtgtcagaggacttcacctgggaaaaacccccagaagaccctcctg
atagcaagaacacacttgtgctctttggggcaggattcggcgcagtaataacagtcgtcgtcatcgttgtcatcatcaaatgcttctg
taagcacagaagctgtttcagaagaaatgaggcaagcagagaaacaaacaacagccttaccttcgggcctgaagaagcattag
ctgaacagaccgtcttccttcgtacgGGCAGTGGAGAGGGCAGAGGAAGTCTGCTAACATGC
GGTGACGTCGAGGAGAATCCTGGCCCAatggagagtgtggtacagccttcagtgtttgtggtggatgga
cagacggacatcccattcaggcggctggaacagaaccaccggagacggcgctgtggcactgtccaggtcagcctggccctgg
tgctgctgctaggtgctgggctggccactcagggctggtttctcctgagactgcatcaacgtcttggagacatagtagctcatctgc
cagatggaggcaaaggctcctgggagaagctgatacaagatcaacgatctcaccaggccaacccagcagcacatcttacagg
agccaacgccagcttgataggtattggtggacctctgttatgggagacacgacttggcctggccttcttgaggggcttgacgtatc
atgatggggccctggtgaccatggagcccggttactactatgtgtactccaaagtgcagctgagcggcgtgggctgcccccagg
ggctggccaatggcctccccatcacccatggactatacaagcgcacatcccgctacccgaaggagttagaactgctggtcagtc
ggcggtcaccctgtggccgggccaacagctcccgagtctggtgggacagcagcttcctgggcggcgtggtacatctggaggc
tggggaagaggtggtggtccgcgtgcctggaaaccgcctggtcagaccacgtgacggcaccaggtcctatttcggagctttcat
ggtcactagtGGATCCGGCGCAACAAACTTCTCTCTGCTGAAACAAGCCGGAGATG
TCGAAGAGAATCCTGGACCGatggaggaaatgcctttgagagagtcaagtcctcaaagggcagagaggtgc
 
aagaagtcatggctcttgtgcatagtggctctgttactgatgctgctctgttctttgggtacactgatctatacttcactcaagccaact
gccatcgagtcctgcatggttaagtttgaactatcatcctcaaaatggcacatgacatctcccaaacctcactgtgtgaatacgacat
ctgatgggaagctgaagatactgcagagtggcacatatttaatctacggccaagtgattcctgtggataagaaatacataaaagac
aatgcccccttcgtagtacagatatataaaaagaatgatgtcctacaaactctaatgaatgattttcaaatcttgcctataggaggggt
ttatgaactgcatgctggagataacatatatctgaagttcaactctaaagaccatattcagaaaaataacacatactgggggatcatc
ttaatgcctgatctaccattcatctctgctagcagcggtacccagtgcaccaactacgccctgctgaagctggccggcgatgtgga
gagcaaccccgggcccatggaccagcacacacttgatgtggaggataccgcggatgccagacatccagcaggtacttcgtgc
ccctcggatgcggcgctcctcagagataccgggctcctcgcggacgctgcgctcctctcagatactgtgcgccccacaaatgcc
gcgctccccacggatgctgcctaccctgcggttaatgttcgggatcgcgaggccgcgtggccgcctgcactgaacttctgttccc
gccacccaaagctctatggcctagtcgctttggttttgctgcttctgatcgccgcctgtgttcctatcttcacccgcaccgagcctcg
gccagcgctcacaatcaccacctcgcccaacctgggtacccgagagaataatgcagaccaggtcacccctgtttcccacattgg
ctgccccaacactacacaacagggctctcctgtgttcgccaagctactggctaaaaaccaagcatcgttgtgcaatacaactctga
actggcacagccaagatggagctgggagctcatacctatctcaaggtctgaggtacgaagaagacaaaaaggagttggtggta
gacagtcccgggctctactacgtatttttggaactgaagctcagtccaacattcacaaacacaggccacaaggtgcagggctgg
gtctctcttgttttgcaagcaaagcctcaggtagatgactttgacaacttggccctgacagtggaactgttcccttgctccatggaga
acaagttagtggaccgttcctggagtcaactgttgctcctgaaggctggccaccgcctcagtgtgggtctgagggcttatctgcat
ggagcccaggatgcatacagagactgggagctgtcttatcccaacaccaccagctttggactctttcttgtgaaacccgacaacc
catgggaatgaGAATTCAATAAAAGATCTTTATTTTCATTAGATCTGTGTGTTGGTTT
TTTGTGTGCGGCCGCAGGAACCCCTAGTGATGGAGTTGGCCACTCCCTCTCTG
CGCGCTCGCTCGCTCACTGAGGCCGGGCGACCAAAGGTCGCCCGACGCCCGG
GCTTTGCCCGGGCGGCCTCAGTGAGCGAGCGAGCGCGCAGCTGCCTGCAGGG
GCGCCTGATGCGGTATTTTCTCCTTACGCATCTGTGCGGTATTTCACACCGCAT
ACGTCAAAGCAACCATAGTACGCGCCCTGTAGCGGCGCATTAAGCGCGGCGG
GTGTGGTGGTTACGCGCAGCGTGACCGCTACACTTGCCAGCGCCTTAGCGCCC
GCTCCTTTCGCTTTCTTCCCTTCCTTTCTCGCCACGTTCGCCGGCTTTCCCCGTC
AAGCTCTAAATCGGGGGCTCCCTTTAGGGTTCCGATTTAGTGCTTTACGGCAC
CTCGACCCCAAAAAACTTGATTTGGGTGATGGTTCACGTAGTGGGCCATCGCC
CTGATAGACGGTTTTTCGCCCTTTGACGTTGGAGTCCACGTTCTTTAATAGTG
GACTCTTGTTCCAAACTGGAACAACACTCAACTCTATCTCGGGCTATTCTTTT
GATTTATAAGGGATTTTGCCGATTTCGGTCTATTGGTTAAAAAATGAGCTGAT
TTAACAAAAATTTAACGCGAATTTTAACAAAATATTAACGTTTACAATTTTAT
GGTGCACTCTCAGTACAATCTGCTCTGATGCCGCATAGTTAAGCCAGCCCCGA
CACCCGCCAACACCCGCTGACGCGCCCTGACGGGCTTGTCTGCTCCCGGCATC
CGCTTACAGACAAGCTGTGACCGTCTCCGGGAGCTGCATGTGTCAGAGGTTTT
CACCGTCATCACCGAAACGCGCGAGACGAAAGGGCCTCGTGATACGCCTATT
TTTATAGGTTAATGTCATGATAATAATGGTTTCTTAGACGTCAGGTGGCACTT
TTCGGGGAAATGTGCGCGGAACCCCTATTTGTTTATTTTTCTAAATACATTCA
AATATGTATCCGCTCATGAGACAATAACCCTGATAAATGCTTCAATAATATTG
AAAAAGGAAGAGTATGAGTATTCAACATTTCCGTGTCGCCCTTATTCCCTTTT
TTGCGGCATTTTGCCTTCCTGTTTTTGCTCACCCAGAAACGCTGGTGAAAGTA
AAAGATGCTGAAGATCAGTTGGGTGCACGAGTGGGTTACATCGAACTGGATC
TCAACAGCGGTAAGATCCTTGAGAGTTTTCGCCCCGAAGAACGTTTTCCAATG
ATGAGCACTTTTAAAGTTCTGCTATGTGGCGCGGTATTATCCCGTATTGACGC
CGGGCAAGAGCAACTCGGTCGCCGCATACACTATTCTCAGAATGACTTGGTT
GAGTACTCACCAGTCACAGAAAAGCATCTTACGGATGGCATGACAGTAAGAG
AATTATGCAGTGCTGCCATAACCATGAGTGATAACACTGCGGCCAACTTACTT
CTGACAACGATCGGAGGACCGAAGGAGCTAACCGCTTTTTTGCACAACATGG
GGGATCATGTAACTCGCCTTGATCGTTGGGAACCGGAGCTGAATGAAGCCAT
ACCAAACGACGAGCGTGACACCACGATGCCTGTAGCAATGGCAACAACGTTG
CGCAAACTATTAACTGGCGAACTACTTACTCTAGCTTCCCGGCAACAATTAAT
 
AGACTGGATGGAGGCGGATAAAGTTGCAGGACCACTTCTGCGCTCGGCCCTT
CCGGCTGGCTGGTTTATTGCTGATAAATCTGGAGCCGGTGAGCGTGGAAGCC
GCGGTATCATTGCAGCACTGGGGCCAGATGGTAAGCCCTCCCGTATCGTAGTT
ATCTACACGACGGGGAGTCAGGCAACTATGGATGAACGAAATAGACAGATCG
CTGAGATAGGTGCCTCACTGATTAAGCATTGGTAACTGTCAGACCAAGTTTAC
TCATATATACTTTAGATTGATTTAAAACTTCATTTTTAATTTAAAAGGATCTAG
GTGAAGATCCTTTTTGATAATCTCATGACCAAAATCCCTTAACGTGAGTTTTC
GTTCCACTGAGCGTCAGACCCCGTAGAAAAGATCAAAGGATCTTCTTGAGAT
CCTTTTTTTCTGCGCGTAATCTGCTGCTTGCAAACAAAAAAACCACCGCTACC
AGCGGTGGTTTGTTTGCCGGATCAAGAGCTACCAACTCTTTTTCCGAAGGTAA
CTGGCTTCAGCAGAGCGCAGATACCAAATACTGTTCTTCTAGTGTAGCCGTAG
TTAGGCCACCACTTCAAGAACTCTGTAGCACCGCCTACATACCTCGCTCTGCT
AATCCTGTTACCAGTGGCTGCTGCCAGTGGCGATAAGTCGTGTCTTACCGGGT
TGGACTCAAGACGATAGTTACCGGATAAGGCGCAGCGGTCGGGCTGAACGGG
GGGTTCGTGCACACAGCCCAGCTTGGAGCGAACGACCTACACCGAACTGAGA
TACCTACAGCGTGAGCTATGAGAAAGCGCCACGCTTCCCGAAGGGAGAAAGG
CGGACAGGTATCCGGTAAGCGGCAGGGTCGGAACAGGAGAGCGCACGAGGG
AGCTTCCAGGGGGAAACGCCTGGTATCTTTATAGTCCTGTCGGGTTTCGCCAC
CTCTGACTTGAGCGTCGATTTTTGTGATGCTCGTCAGGGGGGCGGAGCCTATG
GAAAAACGCCAGCAACGCGGCCTTTTTACGGTTCCTGGCCTTTTGCTGGCCTT
TTGCTCACATGT
**Ori (SEQ ID NO: 59)** 
TTGAGATCCTTTTTTTCTGCGCGTAATCTGCTGCTTGCAAACAAAAAAACCAC
CGCTACCAGCGGTGGTTTGTTTGCCGGATCAAGAGCTACCAACTCTTTTTCCG
AAGGTAACTGGCTTCAGCAGAGCGCAGATACCAAATACTGTTCTTCTAGTGTA
GCCGTAGTTAGGCCACCACTTCAAGAACTCTGTAGCACCGCCTACATACCTCG
CTCTGCTAATCCTGTTACCAGTGGCTGCTGCCAGTGGCGATAAGTCGTGTCTT
ACCGGGTTGGACTCAAGACGATAGTTACCGGATAAGGCGCAGCGGTCGGGCT
GAACGGGGGGTTCGTGCACACAGCCCAGCTTGGAGCGAACGACCTACACCGA
ACTGAGATACCTACAGCGTGAGCTATGAGAAAGCGCCACGCTTCCCGAAGGG
AGAAAGGCGGACAGGTATCCGGTAAGCGGCAGGGTCGGAACAGGAGAGCGC
ACGAGGGAGCTTCCAGGGGGAAACGCCTGGTATCTTTATAGTCCTGTCGGGTT
TCGCCACCTCTGACTTGAGCGTCGATTTTTGTGATGCTCGTCAGGGGGGCGGA
GCCTATGGAAA
**flOri (SEQ ID NO: 60)** 
ACGCGCCCTGTAGCGGCGCATTAAGCGCGGCGGGTGTGGTGGTTACGCGCAG
CGTGACCGCTACACTTGCCAGCGCCTTAGCGCCCGCTCCTTTCGCTTTCTTCCC
TTCCTTTCTCGCCACGTTCGCCGGCTTTCCCCGTCAAGCTCTAAATCGGGGGCT
CCCTTTAGGGTTCCGATTTAGTGCTTTACGGCACCTCGACCCCAAAAAACTTG
ATTTGGGTGATGGTTCACGTAGTGGGCCATCGCCCTGATAGACGGTTTTTCGC
CCTTTGACGTTGGAGTCCACGTTCTTTAATAGTGGACTCTTGTTCCAAACTGG
AACAACACTCAACTCTATCTCGGGCTATTCTTTTGATTTATAAGGGATTTTGCC
GATTTCGGTCTATTGGTTAAAAAATGAGCTGATTTAACAAAAATTTAACGCGA
ATTTTAACAAAATATTAACGTTTACAATTT
**AmpR Promoter (SEQ ID NO: 61)** 
CGCGGAACCCCTATTTGTTTATTTTTCTAAATACATTCAAATATGTATCCGCTC
ATGAGACAATAACCCTGATAAATGCTTCAATAATATTGAAAAAGGAAGAGT
**AmpR (SEQ ID NO: 62)** 
ATGAGTATTCAACATTTCCGTGTCGCCCTTATTCCCTTTTTTGCGGCATTTTGC
CTTCCTGTTTTTGCTCACCCAGAAACGCTGGTGAAAGTAAAAGATGCTGAAGA
TCAGTTGGGTGCACGAGTGGGTTACATCGAACTGGATCTCAACAGCGGTAAG
ATCCTTGAGAGTTTTCGCCCCGAAGAACGTTTTCCAATGATGAGCACTTTTAA
AGTTCTGCTATGTGGCGCGGTATTATCCCGTATTGACGCCGGGCAAGAGCAAC
TCGGTCGCCGCATACACTATTCTCAGAATGACTTGGTTGAGTACTCACCAGTC
ACAGAAAAGCATCTTACGGATGGCATGACAGTAAGAGAATTATGCAGTGCTG
CCATAACCATGAGTGATAACACTGCGGCCAACTTACTTCTGACAACGATCGG
AGGACCGAAGGAGCTAACCGCTTTTTTGCACAACATGGGGGATCATGTAACT
CGCCTTGATCGTTGGGAACCGGAGCTGAATGAAGCCATACCAAACGACGAGC
GTGACACCACGATGCCTGTAGCAATGGCAACAACGTTGCGCAAACTATTAAC
TGGCGAACTACTTACTCTAGCTTCCCGGCAACAATTAATAGACTGGATGGAG
GCGGATAAAGTTGCAGGACCACTTCTGCGCTCGGCCCTTCCGGCTGGCTGGTT
TATTGCTGATAAATCTGGAGCCGGTGAGCGTGGAAGCCGCGGTATCATTGCA
GCACTGGGGCCAGATGGTAAGCCCTCCCGTATCGTAGTTATCTACACGACGG
GGAGTCAGGCAACTATGGATGAACGAAATAGACAGATCGCTGAGATAGGTGC
CTCACTGATTAAGCATTGGTAA
**AAV-ITR (SEQ ID NO: 63)** 
AGGAACCCCTAGTGATGGAGTTGGCCACTCCCTCTCTGCGCGCTCGCTCGCTC
ACTGAGGCCGGGCGACCAAAGGTCGCCCGACGCCCGGGCTTTGCCCGGGCGG
CCTCAGTGAGCGAGCGAGCGCGCAGCTGCCTGCAGG
**EFS core promoter (SEQ ID NO: 64)** 
GGGCAGAGCGCACATCGCCCACAGTCCCCGAGAAGTTGGGGGGAGGGGTCG
GCAATTGAACGGGTGCCTAGAGAAGGTGGCGCGGGGTAAACTGGGAAAGTG
ATGTCGTGTACTGGCTCCGCCTTTTTCCCGAGGGTGGGGGAGAACCGTATATA
AGTGCAGTAGTCGCCGTGAACGTTCTTTTTCGCAACGGGTTTGCCGCCAGAAC
ACAG
**LTR promoter (SEQ ID NO: 65)** 
GGGGCTCGCATCTCTCCTTCACGCGCCCGCCGCCCTACCTGAGGCCGCCATCC
ACGCCGGTTGAGTCGCGTTCTGCCGCCTCCCGCCTGTGGTGCCTCCTGAACTG
CGTCCGCCGTCTAGGTAAGTTTAAAGCTCAGGTCGAGACCGGGCCTTTGTCCG
GCGCTCCCTTGGAGCCTACCTAGACTCAGCCGGCTCTCCACGCTTTGCCTGAC
CCTGCTTGCTCAACTCTACGTCTTTGTTTCGTTTTCTGTTCTGCGCCGTTACAG
ATC
**EFS-LTR promoter (SEQ ID NO: 66)** 
GGGCAGAGCGCACATCGCCCACAGTCCCCGAGAAGTTGGGGGGAGGGGTCG
GCAATTGAACGGGTGCCTAGAGAAGGTGGCGCGGGGTAAACTGGGAAAGTG
ATGTCGTGTACTGGCTCCGCCTTTTTCCCGAGGGTGGGGGAGAACCGTATATA
AGTGCAGTAGTCGCCGTGAACGTTCTTTTTCGCAACGGGTTTGCCGCCAGAAC
ACAGCTGAAGCTTCGAGGGGCTCGCATCTCTCCTTCACGCGCCCGCCGCCCTA
CCTGAGGCCGCCATCCACGCCGGTTGAGTCGCGTTCTGCCGCCTCCCGCCTGT
GGTGCCTCCTGAACTGCGTCCGCCGTCTAGGTAAGTTTAAAGCTCAGGTCGAG
ACCGGGCCTTTGTCCGGCGCTCCCTTGGAGCCTACCTAGACTCAGCCGGCTCT
 
CCACGCTTTGCCTGACCCTGCTTGCTCAACTCTACGTCTTTGTTTCGTTTTCTG
TTCTGCGCCGTTACAGATC
**T2A (SEQ ID NO: 67)** 
GAGGAAGTCTGCTAACATGCGGTGACGTCGAGGAGAATCCTGGCCCAatggagag
tgtggtacagcct
**P2A (SEQ ID NO: 68)** 
actagtGGATCCGGCGCAACAAACTTCTCTCTGCTGAAACAAGCCGGAGATGTCG
AAGAGAATCCTGGACCG
**E2A (SEQ ID NO: 69)**
   gctagcagcggtacccagtgcaccaactacgccctgctgaagctggccggcgatgtggagagcaaccccgggccc
**F2A (SEQ ID NO: 70)**
   ggcatatgcggtaccgtgaagcagaccctgaacttcgatctgctgaagctggccggcgatgtggagagcaaccccgggccc
**WPRE (SEQ ID NO: 71)** 
aatcaacctctggattacaaaatttgtgaaagattgactggtattcttaactatgttgctccttttacgctatgtggatacgctgctttaat
gcctttgtatcatgctattgcttcccgtatggctttcattttctcctccttgtataaatcctggttgctgtctctttatgaggagttgtggcc
cgttgtcaggcaacgtggcgtggtgtgcactgtgtttgctgacgcaacccccactggttggggcattgccaccacctgtcagctc
ctttccgggactttcgctttccccctccctattgccacggcggaactcatcgccgcctgccttgcccgctgctggacaggggctcg
gctgttgggcactgacaattccgtggtgttgtcggggaaatcatcgtcctttccttggctgctcgcctgtgttgccacctggattctg
cgcgggacgtccttctgctacgtcccttcggccctcaatccagcggaccttccttcccgcggcctgctgccggctctgcggcctct
tccgcgtcttcgccttcgccctcagacgagtcggatctccctttgggccgcctccccgc
**short-PolyA (SEQ ID NO: 72)**
   AATAAAAGATCTTTATTTTCATTAGATCTGTGTGTTGGTTTTTTGTGTG
**ORF-Cd80 (SEQ ID NO: 73)** 
atggcttgcaattgtcagttgatgcaggatacaccactcctcaagtttccatgtccaaggctcattcttctctttgtgctgctgattcgtc
tttcacaagtgtcttcagatgttgatgaacaactgtccaagtcagtgaaagataaggtattgctgccttgccgttacaactctcctcat
gaagatgagtctgaagaccgaatctactggcaaaaacatgacaaagtggtgctgtctgtcattgctgggaaactaaaagtgtggc
ccgagtataagaaccggactttatatgacaacactacctactctcttatcatcctgggcctggtcctttcagaccggggcacataca
gctgtgtcgttcaaaagaaggaaagaggaacgtatgaagttaaacacttggctttagtaaagttgtccatcaaagctgacttctcta
cccccaacataactgagtctggaaacccatctgcagacactaaaaggattacctgctttgcttccgggggtttcccaaagcctcgc
ttctcttggttggaaaatggaagagaattacctggcatcaatacgacaatttcccaggatcctgaatctgaattgtacaccattagta
gccaactagatttcaatacgactcgcaaccacaccattaagtgtctcattaaatatggagatgctcacgtgtcagaggacttcacct
gggaaaaacccccagaagaccctcctgatagcaagaacacacttgtgctctttggggcaggattcggcgcagtaataacagtcg
tcgtcatcgttgtcatcatcaaatgcttctgtaagcacagaagctgtttcagaagaaatgaggcaagcagagaaacaaacaacag
ccttaccttcgggcctgaagaagcattagctgaacagaccgtcttcctt
**ORF-Light (SEQ ID NO: 74)** 
atggagagtgtggtacagccttcagtgtttgtggtggatggacagacggacatcccattcaggcggctggaacagaaccaccgg
agacggcgctgtggcactgtccaggtcagcctggccctggtgctgctgctaggtgctgggctggccactcagggctggtttctc
ctgagactgcatcaacgtcttggagacatagtagctcatctgccagatggaggcaaaggctcctgggagaagctgatacaagat
caacgatctcaccaggccaacccagcagcacatcttacaggagccaacgccagcttgataggtattggtggacctctgttatggg
agacacgacttggcctggccttcttgaggggcttgacgtatcatgatggggccctggtgaccatggagcccggttactactatgtg
tactccaaagtgcagctgagcggcgtgggctgcccccaggggctggccaatggcctccccatcacccatggactatacaagcg
cacatcccgctacccgaaggagttagaactgctggtcagtcggcggtcaccctgtggccgggccaacagctcccgagtctggt
 
gggacagcagcttcctgggcggcgtggtacatctggaggctggggaagaggtggtggtccgcgtgcctggaaaccgcctggt
cagaccacgtgacggcaccaggtcctatttcggagctttcatggtc
**ORF-Cxcl10 (SEQ ID NO: 75)** 
atgaacccaagtgctgccgtcattttctgcctcatcctgctgggtctgagtgggactcaagggatccctctcgcaaggacggtccg
ctgcaactgcatccatatcgatgacgggccagtgagaatgagggccatagggaagcttgaaatcatccctgcgagcctatcctg
cccacgtgttgagatcattgccacgatgaaaaagaatgatgagcagagatgtctgaatccggaatctaagaccatcaagaatttaa
tgaaagcgtttagccaaaaaaggtctaaaagggctcct
**ORF-41BBL (SEQ ID NO: 76)** 
atggaccagcacacacttgatgtggaggataccgcggatgccagacatccagcaggtacttcgtgcccctcggatgcggcgct
cctcagagataccgggctcctcgcggacgctgcgctcctctcagatactgtgcgccccacaaatgccgcgctccccacggatg
ctgcctaccctgcggttaatgttcgggatcgcgaggccgcgtggccgcctgcactgaacttctgttcccgccacccaaagctctat
ggcctagtcgctttggttttgctgcttctgatcgccgcctgtgttcctatcttcacccgcaccgagcctcggccagcgctcacaatca
ccacctcgcccaacctgggtacccgagagaataatgcagaccaggtcacccctgtttcccacattggctgccccaacactacac
aacagggctctcctgtgttcgccaagctactggctaaaaaccaagcatcgttgtgcaatacaactctgaactggcacagccaaga
tggagctgggagctcatacctatctcaaggtctgaggtacgaagaagacaaaaaggagttggtggtagacagtcccgggctcta
ctacgtatttttggaactgaagctcagtccaacattcacaaacacaggccacaaggtgcagggctgggtctctcttgttttgcaagc
aaagcctcaggtagatgactttgacaacttggccctgacagtggaactgttcccttgctccatggagaacaagttagtggaccgtt
cctggagtcaactgttgctcctgaaggctggccaccgcctcagtgtgggtctgagggcttatctgcatggagcccaggatgcata
cagagactgggagctgtcttatcccaacaccaccagctttggactctttcttAtgaaacccgacaacccatgggaa
**ORF-IFNg (SEQ ID NO: 77)** 
atgaacgctacacactgcatcttggctttgcagctcttcctcatggctgtttctggctgttactgccacggcacagtcattgaaagcct
agaaagtctgaataactattttaactcaagtggcatagatgtggaagaaaagagtctcttcttggatatctggaggaactggcaaaa
ggatggtgacatgaaaatcctgcagagccagattatctctttctacctcagactctttgaagtcttgaaagacaatcaggccatcag
caacaacataagcgtcattgaatcacacctgattactaccttcttcagcaacagcaaggcgaaaaaggatgcattcatgagtattg
ccaagtttgaggtcaacaacccacaggtccagcgccaagcattcaatgagctcatccgagtggtccaccagctgttgccggaat
ccagcctcaggaagcggaaaaggagtcgctgctga
**ORF-112 (SEQ ID NO: 78)** 
atgtacagcatgcagctcgcatcctgtgtcacattgacacttgtgctccttgtcaacagcgcacccacttcaagctccacttcaagct
ctacagcggaagcacagcagcagcagcagcagcagcagcagcagcagcacctggagcagctgttgatggacctacag
gagctcctgagcaggatggagaattacaggaacctgaaactccccaggatgctcaccttcaaattttacttgcccaagcaggcca
cagaattgaaagatcttcagtgcctagaagatgaacttggacctctgcggcatgttctggatttgactcaaagcaaaagctttcaatt
ggaagatgctgagaatttcatcagcaatatcagagtaactgttgtaaaactaaagggctctgacaacacatttgagtgccaattcga
tgatgagtcagcaactgtggtggactttctgaggagatggatagccttctgtcaaagcatcatctcaacaagccctcaataaGA
ATTC
ORF-Gitrl (SEQ ID NO: 79) 
atggaggaaatgcctttgagagagtcaagtcctcaaagggcagagaggtgcaagaagtcatggctcttgtgcatagtggctctgt
tactgatgctgctctgttctttgggtacactgatctatacttcactcaagccaactgccatcgagtcctgcatggttaagtttgaactat
catcctcaaaatggcacatgacatctcccaaacctcactgtgtgaatacgacatctgatgggaagctgaagatactgcagagtgg
cacatatttaatctacggccaagtgattcctgtggataagaaatacataaaagacaatgcccccttcgtagtacagatatataaaaa
gaatgatgtcctacaaactctaatgaatgattttcaaatcttgcctataggaggggtttatgaactgcatgctggagataacatatatc
tgaagttcaactctaaagaccatattcagaaaaataacacatactgggggatcatcttaatgcctgatctaccattcatctct
**ORF-II23 (SEQ ID NO: 80)** 
ATGTGTCCTCAGAAGCTAACCATCTCCTGGTTTGCCATCGTTTTGCTGGTGTCT
CCACTCATGGCCATGTGGGAGCTGGAGAAAGACGTTTATGTTGTAGAGGTGG
 
ACTGGACTCCCGATGCCCCTGGAGAAACAGTGAACCTCACCTGTGACACGCC
TGAAGAAGATGACATCACCTGGACCTCAGACCAGAGACATGGAGTCATAGGC
TCTGGAAAGACCCTGACCATCACTGTCAAAGAGTTTCTAGATGCTGGCCAGTA
CACCTGCCACAAAGGAGGCGAGACTCTGAGCCACTCACATCTGCTGCTCCAC
AAGAAGGAAAATGGAATTTGGTCCACTGAAATTTTAAAAAATTTCAAAAACA
AGACTTTCCTGAAGTGTGAAGCACCAAATTACTCCGGACGGTTCACGTGCTCA
TGGCTGGTGCAAAGAAACATGGACTTGAAGTTCAACATCAAGAGCAGTAGCA
GTTCCCCTGACTCTCGGGCAGTGACATGTGGAATGGCGTCTCTGTCTGCAGAG
AAGGTCACACTGGACCAAAGGGACTATGAGAAGTATTCAGTGTCCTGCCAGG
AGGATGTCACCTGCCCAACTGCCGAGGAGACCCTGCCCATTGAACTGGCGTT
GGAAGCACGGCAGCAGAATAAATATGAGAACTACAGCACCAGCTTCTTCATC
AGGGACATCATCAAACCAGACCCGCCCAAGAACTTGCAGATGAAGCCTTTGA
AGAACTCACAGGTGGAGGTCAGCTGGGAGTACCCTGACTCCTGGAGCACTCC
CCATTCCTACTTCTCCCTCAAGTTCTTTGTTCGAATCCAGCGCAAGAAAGAA
AAGATGAAGGAGACAGAGGAGGGGTGTAACCAGAAAGGTGCGTTCCTCGTAG
AGAAGACATCTACCGAAGTCCAATGCAAAGGCGGGAATGTCTGCGTGCAAGC
TCAGGATCGCTATTACAATTCCTCATGCAGCAAGTGGGCATGTGTTCCCTGCA
GGGTCCGATCCTAG
**ORF-hIFNg (SEQ ID NO: 81)** 
ATGAAATATACAAGTTATATCTTGGCTTTTCAGCTCTGCATCGTTTTGGGTTCT
CTTGGCTGTTACTGCCAGGACCCATATGTAAAAGAAGCAGAAAACCTTAAGA
AATATTTTAATGCAGGTCATTCAGATGTAGCGGATAATGGAACTCTTTTCTTA
GGCATTTTGAAGAATTGGAAAGAGGAGAGTGACAGAAAAATAATGCAGAGC
CAAATTGTCTCCTTTTACTTCAAACTTTTTAAAAACTTTAAAGATGACCAGAG
CATCCAAAAGAGTGTGGAGACCATCAAGGAAGACATGAATGTCAAGTTTTTC
AATAGCAACAAAAAGAAACGAGATGACTTCGAAAAGCTGACTAATTATTCGG
TAACTGACTTGAATGTCCAACGCAAAGCAATACATGAACTCATCCAAGTGAT
GGCTGAACTGTCGCCAGCAGCTAAAACAGGGAAGCGAAAAAGGAGTCAGAT
GCTGTTTCGAGGTCGAAGAGCATCCCAG
**ORF-hIL23 (SEQ ID NO: 82)** 
TGTGTCACCAGCAGTTGGTCATCTCTTGGTTTTCCCTGGTTTTTCTGGCATCTC
CCCTCGTGGCCATATGGGAACTGAAGAAAGATGTTTATGTCGTAGAATTGGA
TTGGTATCCGGATGCCCCTGGAGAAATGGTGGTCCTCACCTGTGACACCCCTG
AAGAAGATGGTATCACCTGGACCTTGGACCAGAGCAGTGAGGTCTTAGGCTC
TGGCAAAACCCTGACCATCCAAGTCAAAGAGTTTGGAGATGCTGGCCAGTAC
ACCTGTCACAAAGGAGGCGAGGTTCTAAGCCATTCGCTCCTGCTGCTTCACAA
AAAGGAAGATGGAATTTGGTCCACTGATATTTTAAAGGACCAGAAAGAACCC
AAAAATAAGACCTTTCTAAGATGCGAGGCCAAGAATTATTCTGGACGTTTCA
CCTGCTGGTGGCTGACGACAATCAGTACTGATTTGACATTCAGTGTCAAAAGC
AGCAGAGGCTCTTCTGACCCCCAAGGGGTGACGTGCGGAGCTGCTACACTCT
CTGCAGAGAGAGTCAGAGGGGACAACAAGGAGTATGAGTACTCAGTGGAGT
GCCAGGAGGACAGTGCCTGCCCAGCTGCTGAGGAGAGTCTGCCCATTGAGGT
CATGGTGGATGCCGTTCACAAGCTCAAGTATGAAAACTACACCAGCAGCTTC
TTCATCAGGGACATCATCAAACCTGACCCACCCAAGAACTTGCAGCTGAAGC
CATTAAAGAATTCTCGGCAGGTGGAGGTCAGCTGGGAGTACCCTGACACCTG
GAGTACTCCACATTCCTACTTCTCCCTGACATTCTGCGTTCAGGTCCAGGGCA
AGAGCAAGAGAGAAAAGAAAGATAGAGTCTTCACGGACAAGACCTCAGCCA
 
CGGTCATCTGCCGCAAAAATGCCAGCATTAGCGTGCGGGCCCAGGACCGCTA
CTATAGCTCATCTTGGAGCGAATGGGCATCTGTGCCCTGCAGT
**ORF-h41BBL (SEQ ID NO: 83)** 
ATGGAATACGCCTCTGACGCTTCACTGGACCCCGAAGCCCCGTGGCCTCCCGC
GCCCCGCGCTCGCGCCTGCCGCGTACTGCCTTGGGCCCTGGTCGCGGGGCTGC
TGCTGCTGCTGCTGCTCGCTGCCGCCTGCGCCGTCTTCCTCGCCTGCCCCTGG
GCCGTGTCCGGGGCTCGCGCCTCGCCCGGCTCCGCGGCCAGCCCGAGACTCC
GCGAGGGTCCCGAGCTTTCGCCCGACGATCCCGCCGGCCTCTTGGACCTGCG
GCAGGGCATGTTTGCGCAGCTGGTGGCCCAAAATGTTCTGCTGATCGATGGG
CCCCTGAGCTGGTACAGTGACCCAGGCCTGGCAGGCGTGTCCCTGACGGGGG
GCCTGAGCTACAAAGAGGACACGAAGGAGCTGGTGGTGGCCAAGGCTGGAG
TCTACTATGTCTTCTTTCAACTAGAGCTGCGGCGCGTGGTGGCCGGCGAGGGC
TCAGGCTCCGTTTCACTTGCGCTGCACCTGCAGCCACTGCGCTCTGCTGCTGG
GGCCGCCGCCCTGGCTTTGACCGTGGACCTGCCACCCGCCTCCTCCGAGGCTC
GGAACTCGGCCTTCGGTTTCCAGGGCCGCTTGCTGCACCTGAGTGCCGGCCAG
CGCCTGGGCGTCCATCTTCACACTGAGGCCAGGGCACGCCATGCCTGGCAGC
TTACCCAGGGCGCCACAGTCTTGGGACTCTTCCGGGTGACCCCCGAAATCCCA
GCCGGACTCCCTTCACCGAGGTCGGAA
**ORF-hLIGHT(remove-EQLI) (SEQ ID NO: 84)** 
ATGGAGGAGAGTGTCGTACGGCCCTCAGTGTTTGTGGTGGATGGACAGACCG
ACATCCCATTCACGAGGCTGGGACGAAGCCACCGGAGACAGTCGTGCAGTGT
GGCCCGGGTGGGTCTGGGTCTCTTGCTGTTGCTGATGGGGGCCGGGCTGGCCG
TCCAAGGCTGGTTCCTCCTGCAGCTGCACTGGCGTCTAGGAGAGATGGTCACC
CGCCTGCCTGACGGACCTGCAGGCTCCTGGCAAGAGCGAAGGTCTCACGAGG
TCAACCCAGCAGCGCATCTCACAGGGGCCAACTCCAGCTTGACCGGCAGCGG
GGGGCCGCTGTTATGGGAGACTCAGCTGGGCCTGGCCTTCCTGAGGGGCCTC
AGCTACCACGATGGGGCCCTTGTGGTCACCAAAGCTGGCTACTACTACATCTA
CTCCAAGGTGCAGCTGGGCGGTGTGGGCTGCCCGCTGGGCCTGGCCAGCACC
ATCACCCACGGCCTCTACAAGCGCACACCCCGCTACCCCGAGGAGCTGGAGC
TGTTGGTCAGCCAGCAGTCACCCTGCGGACGGGCCACCAGCAGCTCCCGGGT
CTGGTGGGACAGCAGCTTCCTGGGTGGTGTGGTACACCTGGAGGCTGGGGAG
AAGGTGGTCGTCCGTGTGCTGGATGAACGCCTGGTTCGACTGCGTGATGGTAC
CCGGTCTTACTTCGGGGCTTTCATGGTG
**CGKRK (SEQ ID NO: 85)**
   tgcggaaagcgtaag

The results of the experiments are now described.

### Example 1: CRISPRa screen assisted rational design of an off the shelf viral immune gene therapy

In order to develop an improved off-the-shelf viral immune gene therapy that is both simpler and more potent, an *in vivo* CRISPRa-based screen was devised using a sgRNA library to identify the key genes that are effective in Multiplexed Activation of Endogenous Genes as an Immunotherapy (MAEGI) or direct ORF-based viral immune gene therapy settings (Fig. 1A). By performing a CRISPRa *in vivo* depletion screen in the syngeneic systemic cancer model, a small number of genes that are depleted in the metastatic tumors in the lung were identified.

An APCM sgRNA library (SEQ ID NOs. 86-192) (Fig. 7) was introduced into E0771, a triple-negative breast cancer cell line, by infection with a lentiviral CRISPRa vector at M.O.I = 0.2-0.3. After 7 days of *in vitro* culture under puromycin, 2*10⁶ of APCM library transduced cells were intravenously injected into immune-competent C57BL/6J mice (Fig. 1A). Mouse survival was then followed for 30 days (Fig. 1B). The metastatic tumors formed in the lungs of each animal were collected, and the immune-mediated sgRNA depletion and enrichment was readout by illumina sequencing (Fig. 1C). The abundance of sgRNAs within tumor samples and cell samples was then determined by mapping the sequenced reads to the library. The depleted genes were chosen in combinations (e.g. CLC4, CLC4G, CLC4I) as screen-rationalized, off-the-shelf viral immune gene therapy, either in the form of o-MAEGI or concatenated ORF (Fig. 1D).

### Example 2: In vivo anti-tumor efficacy of AAV-o-MAEGI-CLC4, AAV-ORF-CLC4 and AAV-ORF-CLC4I in a syngeneic triple-negative breast cancer model

Taking the top hits from the analysis of depleted gene expression in APCM-transduced tumors from Example 1, three off-the-shelf therapies were developed: CLC4 (CD80-Light-CXCL10-4-1BBL), CLC4G (CD80-Light-CXCL10-4-1BBL-Gitrl) and CLC4I (CD80-Light-CXCL10-4-1BBL-IFNG). These therapies were developed in the form of MAEGI (CRISPRa-based pooled activation) and direct ORF-based viral immune gene therapy (AAV expressing concatenated ORFs).

The therapeutic effects of intratumoral activation of CLC4, CLC4G and CLC4I were assessed by injecting the AAV constructs into established E077 tumors. Mice were treated with PBS, AAV-dCas9 + Vector, AAV-dCas9 + CLC4 (dual AAV delivered CRISPRa of sgRNA library targeting CLC4G, *i.e.* o-MAEGI-CLC4G), AAV-orf-CLC4 (AAV delivered open reading frame expression of CD80-Light-CXCL10-4-1BBL), or AAV-orf-CLC4I (AAV delivered open reading frame expression of CD80-Light-CXCL10-4-1BBL-IFNG). Tumor growth was then followed over the next 27 days (Fig. 2). Tumors treated with empty vector plus the dCas9 AAV (Vector + 11b) had slightly reduced but not significant growth compared to PBS-alone controls. Treatment with CLC4 (pGW066) resulted in a significant reduction in tumor volume. Treatment with CLC4G+11b (o-MAEGI) or CLC4I (pGW067) resulted in a highly significant reduction in tumor volume. The data demonstrated the *in vivo* therapeutic efficacy of these treatments.

### Example 3: Design and characterization of adenovirus-based one-vector CRISPRa systems

Adenoviral-based dSpCas9 CRISPRa systems were designed herein that utilize a single construct to deliver all CRISPRa components. The constructs, pGW029 (SEQ ID NO: 4), pGW035 (SEQ ID NO: 5), and pGW063 (SEQ ID NO: 6), are illustrated in Fig. 3A. In order to test this system *in vitro,* an sgRNA that targed Ox40L was cloned into these constructs, and their activation efficacies were tested. Four days post-transfection, expression levels of Ox40L were determined by quantitative RT-PCR (Fig. 3B). The cytotoxic effects in HEK293FT cells caused by adenovirus production were then observed (Fig. 3C). Cells were transfected with AdTrack (adenoviral CMV-EGFP) constructs, and the expression of GFP and cytopathic effects were observed (Fig. 3C top row). In a similar study, the CRISPRa system was cloned into adenoviral constructs (U6-sgRNA-EFS-LTR-dSpCas9-p65-HSF1-sPA) and the adenoviruses were rescued. 4-6 days post-infection of the adenovirus, cytopathic effects on HEK293FT were observed (Fig. 3C, bottom row). These results demonstrated the robustness of the dSpCas9 CRISPRa adenoviral system.

### Example 4: Design and testing of CRISPRa AAV-based vector systems

Additional vector systems were designed utilizing AAV to deliver CRISPRa components to target cells. The first was a dSaCas9-based AIO (all-in-one) CRISPRa system, which uses a single AAV vector (pZB3: SEQ ID NO: 10) to deliver all CRISPRa components (Fig. 4A). For *in vitro* testing of this system, Pmel was targeted with the AIO CRISPRa system. Four days post-infection, expression levels of Pmel were determined by quantitative RT-PCR, which revealed up-regulation of Pmel expression by the AAV-based (pZB3) vector (Fig. 4B). A lentiviral based version was used for comparison.

An AAV-based two-vector system with dSaCas9 was designed for use in endogenous gene activation and MAEGI. In this case, the dSaCas9 enzyme was delivered on a separate construct from the other CRISPRa components (pGW060: SEQ ID NO: 12 and pGW047: SEQ ID NO:11, respectively) (Fig. 5A). For *in vitro* testing, Pmel targeted sgRNA was cloned into the dual AAV-CRISPRa system. Expression levels of Pmel were determined by quantitative RT-PCR four days after infection (Fig. 5B). A significant increase in Pmel expression was observed in cells transfected with the two-vector dSaCas9 system.

Another two-vector system was also designed, which utilized the dSpCas9 endonuclease. A schematic of the two constructs used in this dSpCas9-based dual-AAV delivery system is depicted in Fig. 6A (pGW045: SEQ ID NO: 13). For *in vitro* testing, Pmel targeted sgRNA was cloned into cells with the dual AAV-CRISPRa system (Fig. 6B). Four days post-infection, expression levels of Pmel were determined by quantitative RT-PCR, which demonstrated significant upregulation of Pmel transcript as compared to a non-sgRNA containing control.

### Example 5: An AAV-based two-vector system with dCas12a/dCpf1

AAV-based two-vector systems were generated that utilize dCas12a/dCpf1 to facilitate endogenous gene activation and MAEGI. Vectors comprising Cas12 (formerly known as Cpf1) derived from *Lachnospiraceae bacterium ND2006* (dLbCas12a/ dLbCpf1) or *Acidaminococcus sp.* (dAsCas12a/dAsCpf1) were generated. Vectors used in this system include: pRC119: pRC119 AAV EFS-denAsCas12a-SunTag3x-1 (SEQ ID NO: 14); pRC120: pRC120 AAV EFS-dLbCas12a-SunTag5x-1 (SEQ ID NO: 15); pRC121b: pRC121b AAV-EFS-LTR-scFV_Gcn4-Activ-Triplex-SapI-WPRE (SEQ ID NO: 16); pRC124: pRC124 AAV-EFS-LTR-NLS-dLbCpf1_574-NLS-Activ-Triplex-SapI-WPRE-1 (SEQ ID NO: 17); and pRC126: pRC126 AAV-EFS-LTR-NLS-Activ-dLbCpf1_1229-NLS-Activ-sPA-1(SEQ ID NO: 18).

### Example 6: Multiplexed activation of endogenous genes by APCM as an immune-gene therapy approach

Syngeneic orthotopic breast tumors were induced in mice by E0771 cells in C57BL/6 mice (Figs. 9A-9D). These animals were treated with PBS, AAV-Vector, or AAV-APCM sgRNA library by intratumoral administration at indicated times (arrows), using either a single vector carrying the library alone into dCas9-VP64 expressing cells (Fig. 9A, against single tumor; and Fig.9C, against both local and distant tumor), or using a dual-AAV approach (Fig. 9B). Survival of these animals treated with PBS, AAV-Vector, or AAV-APCM sgRNA library showed that AAV-APCM treatment extended the overall survival of treated animals (Fig. 9D). Syngeneic pancreatic tumors were induced in mice by Pan02 cells in C57BL/6 mice (Fig. 9E). These animals were treated with PBS, AAV-Vector, AAV-p-MAEGI(Pan02) or AAV-APCM sgRNA library by intratumoral administration with two doses per week, total of 3-4 doses starting at end of week 1.

### Example 7: Molecular characterization and therapeutic effect of multiplexed activation of Cd80, Light, Cxcl10, Gitrl, 41bbl, and IFNg in tumor cells by AAV-delivered CRISPRa or AAV-ORFs (polycistronic open-reading-frames)

Fig. 10A left panel shows AAV-CRISPRa-mediated transcriptional activation of Cd80, Light, Cxcl10, Gitrl, 41bbl, and IFNg, normalized to vector-transduced controls; Right panel shows Co-transfection of or Dual AAV-CRISPRa-mediated transcriptional activation of Cd80, Light, Cxcl10, Gitrl, 41bbl, and IFNg, normalized to control. The results showed expression of CD80, 41BBL, and Gitrl in dual-AAV-CRISPRa infected cells assessed by flow cytometry (Fig.10B). Therapeutic effects of intratumoral injected AAV-CRISPRa with dual-AAV mediated activation of Cd80, light/Tnfsf14, Cxcl10, 4-1BBL/Tnfsf9, and Ifng (CLC4I) are shown in Fig. 10C. Growth curves showed the time course sizes of E0771 tumors treated with PBS, AAV-dCas9 + Vector, AAV-dCas9 + CLCG4 (dual AAV delivered CRISRPa of Clcg)(Fig. 10C). QPCR results showed successful overexpression of Cd80, Light, Cxcl10, 41bbl, or/and IFNg using AAV polycistronic ORFs (Fig. 10D). Both transfection and AAV infection led to high levels of expression of these molecules. Results showed significant therapeutic effects of intratumoral injected AAV-ORFs expressing Cd80, Light, Cxcl10, 41bbl (orfClc4), or AAV expressing Cd80, Light, Cxcl10, 41bbl, Ifng (orfClc4I) (Fig. 10E). Significant therapeutic effects of intratumoral injected AAV-ORFs expressing Cd80, Light, Cxcl10, 41bbl, Ifng (orfClc4I) were also demonstrated (Fig. 10F).

### Example 8: Immune characterization of APCM treated tumors

Immune profiling was performed to characterize AAV-mediated costimulatory molecule expression that promotes tumor immune infiltration (Figs. 11A-11F). Results showed flow cytometry quantification of CD45+ immune cells in tumor microenvironment at DPI = 30, showing percentages of CD45+ immune cells out of total cells in tumors treated with PBS, dual-AAV-vector, dual-AAV-Clcg4, AAV-orfClc4, AAV-orfClc4I (Fig. 11A). The percentages of CD8+ T cells out of total cells in tumors treated with PBS, dual-AAV-vector, dual-AAV-Clcg4, AAV-orfClc4, AAV-orfClc4I are shown in Fig. 11B. The percentages of of CD4+ T cells out of total cells in tumors treated with PBS, dual-AAV-vector, dual-AAV-Clcg4, AAV-orfClc4, AAV-orfClc4I are shown in Fig. 11C. Flow cytometry quantification of PD1+CD8+ T cells out of total CD8+ T cells in tumors treated with PBS, dual-AAV-vector, dual-AAV-Clcg4, AAV-orfClc4, AAV-orfClc4I is shown in Fig. 11D.) Flow cytometry quantification of monocytes out of total cells in tumors treated with PBS, dual-AAV-vector, dual-AAV-Clcg4, AAV-orfClc4, AAV-orfClc4I is shown in Fig. 11E. Flow cytometry quantification of monocytes out of total cells in tumors treated with PBS, dual-AAV-vector, dual-AAV-Clcg4, AAV-orfClc4, AAV-orfClc4I is shown in Fig. 11F.

### Example 9: Rational design of off-the-shelf immune gene therapy by composition subtraction ("minus-one experiment")

Composition subtraction and testing were performed to optimize the combinations of immune-stimulating molecules for tumor immune-gene therapy (Figs. 12A-12F). Results demonstrated the therapeutic effects of intra-tumoral injected AAV-CRISPR activating Cd80, light, Cxcl10, 4-1BBL, Gitrl and Ifng (Clcg4I) (Fig. 12A). Growth curves of E0771 tumors treated with PBS (n = 5), AAV- Vector (n = 5), AAV-Clcg4I are shown in Figs. 12B-12C. The "minus-one" experiment further optimized the combinatorial genes pool by removing one gene in each pool. Results showed the differential therapeutic efficacy of various different constructs of the full set and the "minus-one" constructs, with growth curves of E0771 tumors treated with PBS (n = 4), AAV-Vector (n = 4), AAV-Clcg4I (n = 4), or AAV-Clcg4Iminus1 (n = 4) (Fig. 12B). Growth curves of E0771 tumors treated with AAV-Clcg4I (n = 4), or AAV-Clcg4Iminus1 (n = 4) are shown in Fig. 12C. Growth curves of B16F10 melanoma treated with PBS (n = 4), AAV-Vector (n = 5), AAV-p67:Clc4I (Cd80, Light, Cxcl10, 41bbl, Ifng; n = 5), AAV-P72:IFNg (n = 4) are shown in Fig. 12D. Growth curves of E0771 tumors treated with PBS (n = 6), AAV-Vector (n = 6), AAV-P72:IFNg (n = 6), AAV-p94:IClG4 (Ifng, Cd80, Light, Gitrl, 41bbl; n = 6) are shown in Fig. 12E. Growth curves of E0771 tumors treated with AAV-Vector (n = 5), AAV-polycistronic P119:IFNg-STOP-CD80-Light-Cxcl10-41BBL (n = 5), AAV-polycistronic P118:IFNg-CD80-Light-Cxcl10-41BBL (n = 5), AAV-polycistronic P100:IFNg-CD80-Light-Cxcl10 (n = 5), AAV-P67: CD80-Light-Cxcl10-41BBL-IFNg (n = 5) are shown in Fig. 12F.

### Example 10: Analysis of expression levels of IFNg and CD80 in mammalian cells using different polycistronic AAV-ORFs

AAV-ORFs-mediated expression of IFNg was demonstrated in different AAV constructs transfected into E0771 cells (Fig. 13A). The percentage of IFNg expressing cells in different constructs-transfected E0771 cells is shown in Fig. 13A, lower panel left. Fig. 13A, low panel (right) shows the median fluorescent intensity (MFI) of IFNg-APC in different constructs-transfected E0771 cells. Fig. 13B, upper panel shows representative flow cytometry plots demonstrating AAV-ORFs-mediated expression of CD80 in different AAV constructs transfected E0771 cells. Fig. 13B lower panel (left) shows the percentage of CD80+ cells in different constructs-transfected E0771 cells and Fig. 13B lower panel (right) shows the median fluorescent intensity (MFI) of CD80-PE in different constructs-transfected E0771 cells.

### Example 11: Rational design of single AAV vectors for tumor immune gene therapies

The costimulatory molecule combinations in single AAV vectors as tumor immune gene therapies were optimized (Figs. 14A-14F). Fig. 14A-14B upper panels show representative flow cytometry plots demonstrating AAV-ORFs-mediated expression of CD80, 41BBL, IL23 and IFNg in AAV-infected E0771 or MA1NC cells. Figs. 14A-14B lower panels show the percentage of CD80, 41BBL, IL23, and IFNg positive cells in different AAV-infected E0771 and MA1NC cells. Result showed significant efficacy of indicated single AAV vectors with various defined combinations of transgenes expressing immune molecules as tumor immune gene therapies, where the tumor growth curves show the dynamics of E0771 tumors treated with PBS (n = 5), AAV-Vector (n = 10), AAV-IL23 (n = 5), AAV-IFNg (n = 6), Pooled AAVs(IFNg + CD80 + LIGHT + CXCL10 + 41BBL; n = 5) (Fig. 14C). Significant efficacy of indicated single AAV vectors was also demonstrated with various defined combinations of transgenes expressing immune molecules as tumor immune gene therapies, where the tumor growth curves show the dynamics of tumor growth curves of E0771 tumors treated AAV-Vector (n =5), AAV-IFNg (n = 4), AAV-IFNg+AAV-IL23 (n = 5), pooled AAVs (IFNg + CXCL10 + 41BBL; n = 5), pooled AAVs (IFNg + LIGHT + 41BBL; n = 5), pooled AAV(IFNg + LIGHT + CXCL10 + 41BBL; n = 5) (Fig. 14D). Significant efficacy of indicated single AAV vectors was demonstrated with various defined combinations of transgenes expressing immune molecules as tumor immune gene therapies, where the tumor growth curves show the dynamics of E0771 tumors treated AAV-Vector (n =11), AAV-IFNg (n = 10), AAV-IL23 (n = 10), AAV-IFNg+AAV-IL23 (n = 4) (Fig. 14E). Tumor growth curves of E0771 tumors treated AAV-Vector (n = 5), AAV-IFNg (n = 5), AAV-IL23 (n = 5), AAV-IFNg+AAV-IL23+AAV-41BBL (n = 5) are shown in Fig. 14F.

### Other Embodiments

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

It is to be understood that wherever values and ranges are provided herein, all values and ranges encompassed by these values and ranges, are meant to be encompassed within the scope of the present invention. Moreover, all values that fall within these ranges, as well as the upper or lower limits of a range of values, are also contemplated by the present application. While this invention has been disclosed with reference to specific embodiments, it is apparent that other embodiments and variations of this invention may be devised by others skilled in the art

## Claims

1. A method of developing a cancer immunotherapy, the method comprising:
a) administering a CRISPR activation (CRISPRa) system comprising an sgRNA library to a cancer cell, thereby generating a modified cell,
b) administering the modified cell to an immunocompetent non-human mammal whereby the mammal develops cancer,
c) determining the sgRNAs and thereby the targeted genes that are depleted in the cancer, and
d) designing a cancer immunotherapy that targets the depleted genes,
wherein the mammal is a mouse.

2. The method of claim 1, wherein the sgRNA library comprises the nucleotide sequences set forth in any one of SEQ ID NOs: 86-192 or SEQ ID NOs: 193-411.

3. The method of claim 1, wherein the CRISPRa system comprises a vector comprising the nucleotide sequence set forth in SEQ ID NO: 1.

4. The method of claim 1, wherein determining the genes that are depleted in the cancer comprises nucleotide sequencing and analysis.

5. The method of claim 1, wherein designing a cancer therapy that targets the depleted genes comprises packaging the open reading frames (ORFs) of the depleted genes in a vector.

6. The method of claim 5, wherein the vector is an adeno-associated viral (AAV) vector, or an adenoviral vector.

## Patentansprüche

1. Verfahren zur Entwicklung einer Krebsimmuntherapie, wobei das Verfahren umfasst:
a) Verabreichung eines CRISPR-Aktivierungssystems (CRISPRa), das eine sgRNA-Bibliothek umfasst, an eine Krebszelle, wodurch eine modifizierte Zelle erzeugt wird,
b) Verabreichen der modifizierten Zelle an ein immunkompetentes nichtmenschliches Säugetier, wodurch das Säugetier Krebs entwickelt,
c) Bestimmung der sgRNAs und damit der Zielgene, die in dem Krebs abgereichert sind, und
d) Entwerfen einer Krebsimmuntherapie, die auf die abgereicherten Gene abzielt,
wobei das Säugetier eine Maus ist.

2. Verfahren nach Anspruch 1, wobei die sgRNA-Bibliothek die Nukleotidsequenzen umfasst, die in beliebigen der SEQ ID NOs: 86-192 oder SEQ ID NOs: 193-411 dargelegt sind.

3. Verfahren nach Anspruch 1, wobei das CRISPRa-System einen Vektor umfasst, der die in SEQ ID NO: 1 dargelegte Nukleotidsequenz umfasst.

4. Verfahren nach Anspruch 1, wobei die Bestimmung der Gene, die in dem Krebs abgereichert sind, die Nukleotidsequenzierung und -analyse umfasst.

5. Verfahren nach Anspruch 1, wobei das Entwerfen einer Krebstherapie, die auf die abgereicherten Gene abzielt, das Verpacken der offenen Leserahmen (ORFs) der abgereicherten Gene in einen Vektor umfasst.

6. Verfahren nach Anspruch 5, wobei der Vektor ein Adeno-assoziierter viraler (AAV) Vektor oder ein adenoviraler Vektor ist.

## Revendications

1. Procédé de mise au point d'une immunothérapie anticancéreuse, le procédé comprenant :
a) l'administration, à une cellule cancéreuse, d'un système d'activation CRISPR (CRISPRa) comprenant une bibliothèque d'ARNg courts, ce qui permet de générer une cellule modifiée,
b) l'administration de la cellule modifiée à un mammifère immunocompétent non humain, ce qui amène le mammifère à développer un cancer,
c) la détermination des ARNg courts et, par conséquent, des gènes ciblés qui sont sous-exprimés dans le cancer, et
d) la conception d'une immunothérapie anticancéreuse qui cible les gènes sous-exprimés,
dans lequel le mammifère est une souris.

2. Procédé selon la revendication 1, dans lequel la bibliothèque d'ARNg courts comprend les séquences nucléotidiques présentées dans l'une quelconque des SEQ ID No : 86 à 192 ou SEQ ID No : 193 à 411.

3. Procédé selon la revendication 1, dans lequel le système CRISPRa comprend un vecteur comprenant la séquence nucléotidique présentée dans la SEQ ID No : 1.

4. Procédé selon la revendication 1, dans lequel la détermination des gènes qui sont sous-exprimés dans le cancer comprend le séquençage et l'analyse de nucléotides.

5. Procédé selon la revendication 1, dans lequel la conception d'une thérapie anticancéreuse qui cible les gènes sous-exprimés comprend l'encapsulation des cadres ouverts de lecture (ORF) des gènes sous-exprimés dans un vecteur.

6. Procédé selon la revendication 5, dans lequel le vecteur est un vecteur viral adéno-associé (AAV), ou un vecteur adénoviral.
